(19) Europäisches Patentamt
European Patent Office
Office européen des brevets

(11) EP 0 863 875 B1

(12) **EUROPÄISCHE PATENTSCHRIFT**

(45) Veröffentlichungstag und Bekanntmachung des Hinweises auf die Patenterteilung:
**04.06.2003 Patentblatt 2003/23**

(21) Anmeldenummer: **96927715.1**

(22) Anmeldetag: **29.08.1996**

(51) Int Cl.$^7$: **C07D 211/42**, C07D 401/12, C07D 401/04, C07D 401/06, A61K 31/445

(86) Internationale Anmeldenummer:
**PCT/EP96/03803**

(87) Internationale Veröffentlichungsnummer:
**WO 97/009311 (13.03.1997 Gazette 1997/12)**

(54) **NEUE 4-(OXYALKOXYPHENYL)-3-OXY-PIPERIDINE ZUR BEHANDLUNG VON HERZ- UND NIERENINSUFFIZIENZ**

NEW 4-(OXYALKOXYPHENYL)-3-OXY-PIPERIDINES FOR TREATING HEART AND KIDNEY INSUFFICIENCY

NOUVELLES 4-(OXYALCOXYPHENYL)-3-OXY-PIPERIDINES UTILES POUR TRAITER L'INSUFFISANCE CARDIAQUE ET RENALE

(84) Benannte Vertragsstaaten:
**AT BE CH DE DK ES FI FR GB GR IE IT LI LU MC NL PT SE**

(30) Priorität: **07.09.1995 CH 254895**
**26.07.1996 CH 187696**

(43) Veröffentlichungstag der Anmeldung:
**16.09.1998 Patentblatt 1998/38**

(73) Patentinhaber: **F. HOFFMANN-LA ROCHE AG**
**4070 Basel (CH)**

(72) Erfinder:
- **BINGGELI, Alfred**
  **CH-4112 Flüh (CH)**
- **BREU, Volker**
  **D-79418 Schliengen (DE)**
- **BUR, Daniel**
  **CH-4052 Basel (CH)**
- **FISCHLI, Walter**
  **CH-4123 Allschwil (CH)**
- **GUELLER, Rolf**
  **CH-4310 Rheinfelden (CH)**
- **HIRTH, Georges**
  **F-68330 Huningue (FR)**
- **MAERKI, Hans-Peter**
  **CH-4059 Basel (CH)**
- **MUELLER, Marcel**
  **CH-4402 Frenkendorf (CH)**
- **OEFNER, Christian**
  **D-79108 Freiburg (DE)**
- **STADLER, Heinz**
  **CH-4310 Rheinfelden (CH)**
- **VIEIRA, Eric**
  **CH-4123 Allschwil (CH)**
- **WILHELM, Maurice**
  **F-68790 Morschwiller-le-Bas (FR)**
- **WOSTL, Wolfgang**
  **D-79639 Grenzach-Wyhlen (DE)**

(74) Vertreter: **Witte, Hubert, Dr. et al**
**124 Grenzacherstrasse**
**4070 Basel (CH)**

(56) Entgegenhaltungen:
**EP-A- 0 505 868** **US-A- 4 007 196**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 863 875 B1

Printed by Jouve, 75001 PARIS (FR)

## Beschreibung

Die vorliegende Erfindung betrifft neue Piperidinderivate, ihre Herstellung und Verwendung als Heilmittel. Insbesondere betrifft die Erfindung die neuen Piperidinderivate der allgemeinen Formel I

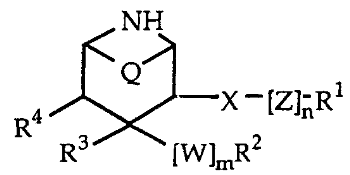

I

worin

| | |
|---|---|
| $R^1$ | Phenyl oder durch $C_{1-6}$-Alkyl, $C_{1-6}$-Alkenyl, $C_{1-6}$-Alkoxy, $C_{1-6}$-Alkylthio, Halogen, Hydroxy, Hydroxy-$C_{1-6}$-alkoxy, $C_{1-6}$-Alkoxy-$C_{1-6}$-alkoxy, $C_{1-6}$-Alkylsulfinyl, $C_{1-6}$-Alkylsulfonyl, Cyano, Trifluormethyl, Trifluormethoxy, Carboxy, Cyclobutylmethoxy-$C_{1-6}$-alkyl, $C_{1-6}$-Alkylendioxy, Phenyl, Phenoxy, $C_{1-6}$-Alkoxycarbonylphenyl, Hydroxy-$C_{1-6}$-alkylphenyl, 2,3-Dihydroxypropylaminocarbonylphenyl, Benzyloxy, Benzoyl, Pyridyl-$C_{1-6}$-alkoxy-$C_{1-6}$-alkyl oder Nicotinoylamino-$C_{1-6}$-alkyl substituiertes Phenyl ist; oder auch Naphthyl oder durch Hydroxy, Oxo, $C_{1-6}$-Alkoxy, $C_{1-6}$-Alkenyloxy, $C_{1-6}$-Alkoxy-$C_{1-6}$-alkoxy, Di-$C_{1-6}$Alkylamino, 2,3-Dihydroxypropoxy, 2,3-Dihydroxypropoxy-$C_{1-6}$-alkoxy, 2,3-Dimethoxypropoxy, $C_{1-6}$-Alkoxycarbonyl-$C_{1-6}$-alkoxy, Carbamoyl-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridylcarbamoyloxy-$C_{1-6}$-alkoxy, Morpholino-$C_{1-6}$-alkoxy, 3-Morpholino-2-hydroxypropoxy, N-Methylpiperazino-N-$C_{1-6}$-alkoxy, Benzoyloxy-$C_{1-6}$-alkoxy oder Picolyloxy substituiertes Naphthyl ist; oder auch Tetrahydronaphthyl oder durch Methyl substituiertes Tetrahydronaphthyl oder Indanyl ist; oder auch Pyridyl, Benzimidazolyl, Di-$C_{1-6}$-alkoxypyrimidinyl oder 2- und 5-Benzo-[b]thienyl, 6- und 7-Chinolyl, 6- und 7-Isochinolyl, 6- und 7-Tetrahydrochinolyl, 6- und 7-Tetrahydroisochinolinyl, 6-Chinoxalinyl, 6- und 7-Chinazolinyl oder durch Hydroxy, Oxo, $C_{1-6}$-Alkoxy, $C_{1-6}$-Alkenyloxy, $C_{1-6}$-Alkoxy-$C_{1-6}$-alkoxy, Di-$C_{1-6}$-Alkylamino, 2,3-Dihydroxypropoxy, 2,3-Dihydroxypropoxy-$C_{1-6}$-alkoxy, 2,3-Dimethoxypropoxy, $C_{1-6}$-Alkoxycarbonyl-$C_{1-6}$-alkoxy, Carbamoyl-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridylcarbamoyloxy-$C_{1-6}$-alkoxy, Morpholino-$C_{1-6}$-alkoxy, 3-Morpholino-2-hydroxypropoxy, N-Methylpiperazino-N-$C_{1-6}$-alkoxy, Benzoyloxy-$C_{1-6}$-alkoxy oder Picolyloxy substituiertes 6- und 7-Chinolyl, 6- und 7-Isochinolyl, 6- und 7-Tetrahydrochinolyl, 6-und 7-Tetrahydroisochinolinyl, 6-Chinoxalinyl oder 6- und 7-Chinazolinyl ist; |
| $R^2$ | Phenyl, Naphthyl, Acenaphthyl, Cyclohexyl,Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl oder Furyl, welche Reste durch 1-3 Halogen, Hydroxy, Cyano, Trifluormethyl, $C_{1-6}$-Alkyl, Halo-$C_{1-6}$-alkyl, Hydroxy-$C_{1-6}$-alkyl, $C_{1-6}$-Alkoxy-$C_{1-6}$-alkyl, Cyano-$C_{1-6}$-alkyl, Carboxy-$C_{1-6}$-alkyl, $C_{1-6}$-Alkanoyloxy-$C_{1-6}$-alkyl, $C_{1-6}$-Alkoxycarbonyloxy-$C_{1-6}$-alkyl, $C_{1-6}$-Alkoxycarbonyl, oder $C_{1-6}$-Alkoxy-gruppen, oder eine $C_{1-6}$-Alkylendioxygruppe, und/oder durch einen Rest $L^1$-$T^1$-$L^2$-$T^2$-$L^3$-$T^3$-$L^4$-$T^4$-$L^5$-U substituiert sein können, darstellen; |
| $L^1$, $L^2$, $L^3$, $L^4$ und $L^5$ | unabhängig voneinander eine Bindung, $C_{1-8}$-Alkylen, $C_{2-8}$- Alkenylen oder $C_{2-8}$- Alkinylen darstellen, oder abwesend sind; |
| $T^1$, $T^2$, $T^3$ und $T^4$ | unabhängig voneinander (a) eine Bindung darstellen, oder abwesend sind, oder eine der Gruppen (b) -CH(OH)-; (c) -CH($OR^6$)-; (d) -CH($NR^5R^6$)-; (e) -CO-; (f) -$CR^7R^8$-; (g) -O- oder -$NR^6$-, (h) -$S(O)_{0-2}$-; (i) -$SO_2NR^6$-; (j) -$NR^6$ $SO_2$-; (k) -$CONR^6$-; (l) -$NR^6$ CO-; (m) -O-CO-; (n) -CO-O-; (o) -O-CO-O-; (p) -O-CO-$NR^6$-; (q) -N($R^6$)-CO-N($R^6$)- oder (r) -N($R^6$)-CO-O- darstellen, wobei die von (b), (d), (e) und (g)-(r) ausgehenden Bindungen zu einem C-Atom der benachbarten Gruppe führen und dieses C-Atom gesättigt ist, falls die Bindung von einem Heteroatom ausgeht, und wobei nicht mehr als zwei Gruppen (b)-(f), drei Gruppen (g)-(h) und eine Gruppe (i) |

-(p) anwesend sind;

| | |
|---|---|
| $R^3$ | Wasserstoff, Hydroxy, $C_{1-6}$-Alkoxy oder $C_{1-6}$-Alkenyloxy; |
| $R^4$ | Wasserstoff, $C_{1-6}$-Alkyl, $C_{1-6}$-Alkenyl, $C_{1-6}$-Alkoxy, Hydroxy-$C_{1-6}$ alkyl, $C_{1-6}$-Alkoxy-$C_{1-6}$alkyl, Benzyl, Oxo, oder eine Gruppe $R^{4a}$-$Z^1$-$X^1$ - darstellen, wobei |
| $R^{4a}$ | eine Gruppe (a) H-; (b) $C_{1-6}$-Alkyl-; (c) $C_{1-6}$-Alkenyl-; (d) Hydroxy-$C_{1-6}$-Alkyl; (e) Polyhydroxy-$C_{1-6}$-alkyl-; (f) $C_{1-6}$-Alkyl-O-$C_{1-6}$-alkyl-; (g) Aryl-; (h) Heterocyclyl-; (i) Aryl-$C_{1-6}$-alkyl-; (j) Heterocyclyl-$C_{1-6}$-alkyl-; (k) Aryloxy-$C_{1-6}$-alkyl-; (l) Heterocyclyloxyl-$C_{1-6}$-alkyl-; (m) ($R^5$, $R^6$)-N-$(CH_2)_{1-3}$-; (n) ($R^5$, $R^6$)-N-; (o) $C_{1-6}$-Alkyl-$S(O)_{0-2}$-; (p) Aryl-$S(O)_{0-2}$-; (q) Heterocyclyl-$S(O)_{0-2}$-; (r) HO-$SO_3$- bzw. deren Salze; (s) $H_2N$-C(NH)-NH-; oder (t) NC- darstellt und die von (n) - (t) ausgehenden Bindungen zu einem C-Atom der benachbarten Gruppe führen und dieses C-Atom gesättigt ist, falls die Bindung von einem Heteroatom ausgeht; |
| $Z^1$ | (a) eine Bindung darstellt, abwesend ist, oder eine der Gruppen(b) $C_{1-6}$-Alkylen-; (c) $C_{1-6}$-Alkenylen-; (d) -O-, -N($R^{11}$)-, -$S(O)_{0-2}$-; (e) -CO-; (f) -O-CO-; (g) -O-CO-O-; (h) -O-CO-N($R^{11}$)-; (i) - N($R^{11}$)-CO-O-; (j) -CO-N($R^{11}$)-; (k) -N($R^{11}$)-CO-; (l) - N($R^{11}$)-CO-N($R^{11}$)- oder (m) -CH($OR^9$)- darstellt und die von (d) und (f) - (m) ausgehenden Bindungen zu einem C-Atom der benachbarten Gruppe führen und dieses C-Atom gesättigt ist, falls die Bindung von einem Heteroatom ausgeht; |
| $X^1$ | (a) eine Bindung darstellt, abwesend ist, oder eine der Gruppen (b) -O-; (c) -N($R^{11}$) -; (d) -$S(O)_{0-2}$-; oder (e) - $(CH_2)_{1-3}$; darstellt; oder |
| $R^3$ und $R^4$ | zusammen eine Bindung darstellen; |
| $R^5$ und $R^6$ | Wasserstoff, $C_{1-6}$-Alkyl, $C_{1-6}$-Alkenyl, Aryl-$C_{1-6}$-alkyl oder Acyl bedeuten, oder gemeinsam mit dem N-Atom, an das sie gebunden sind, einen 5- oder 6-gliedrigen heterocyclischen Ring bedeuten, der ein zusätzliches N-, O- oder S-Atom oder eine -SO- oder -$SO_2$- Gruppe enthalten kann, darstellen, wobei das zusätzliche N-Atom gegebenenfalls durch $C_{1-6}$-Alkyl-Reste substituiert sein kann; |
| $R^7$ und $R^8$ | gemeinsam mit dem C-Atom, an das sie gebunden sind, einen 3-7 - gliedrigen Ring darstellen, der ein oder zwei -O- oder -S-Atome oder -SO- oder -$SO_2$- Gruppen enthalten kann; |
| $R^9$ | Wasserstoff, $C_{1-6}$-Alkyl, Acyl oder Aryl-$C_{1-6}$-alkyl ist; |
| $R^{10}$ | Carboxy-$C_{1-6}$-alkyl, $C_{1-6}$-Alkoxycarbonyl-$C_{1-6}$-alkyl, $C_{1-6}$-Alkyl oder Wasserstoff ist; |
| $R^{11}$ | Wasserstoff oder $C_{1-6}$-Alkyl ist; |
| U | Wasserstoff, $C_{1-6}$-Alkyl, $C_{3-6}$-Cycloalkyl, Cyano, gegebenenfalls substituiertes $C_{3-6}$-Cycloalkyl, Aryl, oder Heterocyclyl darstellt: |
| Q | Aethylen darstellt oder abwesend ist; |
| X | eine Bindung, Sauerstoff, Schwefel oder eine Gruppe -$CHOR^9$- ist, -O-CO, -CO- oder C=$NOR^{10}$- darstellt, wobei die von einem Sauerstoff- oder Schwefelatom ausgehende Bindung zu einem gesättigten C-Atom der Gruppe Z oder zu $R^1$ führt; |
| W | Sauerstoff oder Schwefel darstellt; |
| Z | $C_{1-6}$-Alkylen, $C_{1-6}$-Alkenylen, Hydroxy-$C_{1-6}$-alkyliden-, -Alk-O- oder -Alk-S- ist, wobei Alk $C_{1-6}$-Alkylen bezeichnet; und wobei, falls X eine Bindung darstellt, Z $C_{1-6}$-Alkenylen darstellt, |
| n | 1 ist oder, wenn X -O-CO- ist, 0 oder 1 ist; |

m 0 oder 1 ist;

und pharmazeutisch anwendbare Salze davon;
mit Ausnahme der Verbindungen 4-(4-Fluorphenyl)-3-(3,4-methylendioxy-benzyloxy)piperidin, dessen Hydrochlorid und trans-3-(3- Chlorphenoxy)-4-(3, 4-dimethyl-phenoxy) piperidin sowie dessen pharmazeutisch verträgliche Salze; und wobei

"Acyl" $C_{1-6}$-Alkanoylreste oder Benzoyl darstellt;

"Aryl" allein oder in Zusammensetzungen Phenyl, Naphthyl oder Tetrahydronaphthyl darstellt, welche Gruppen ein- oder mehrfach durch $C_{1-6}$-Alkyl, Trifluormethyl, Nitro, Amino, $C_{1-6}$-Alkenyl, $C_{1-6}$-Alkoxy, $C_{1-6}$-Alkylcarbonyloxy, Hydroxy, Halogen, Cyano, Carbamoyl, $C_{1-6}$-Alkylendioxy, gegebenenfalls durch Halogen, $C_{1-6}$-Alkyl, $C_{1-6}$-Alkoxy oder Dihydroxy-$C_{1-6}$-alkylaminocarbonyl substituiertes Phenyl, Phenoxy, Phenylthio, Phenyl-$C_{1-6}$-alkyl, Phenyl-$C_{1-6}$-alkoxy, $C_{1-6}$-Alkoxycarbonylphenyl, Hydroxy-$C_{1-6}$-alkylphenyl, Benzyloxy, Pyridylcarbonylamino-$C_{1-6}$-alkyl, $C_{1-6}$-Alkenyloxy, $C_{1-6}$-Alkoxy-$C_{1-6}$alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$alkoxy, Hydroxy-$C_{1-6}$-alkoxy, Carbamoyloxy-$C_{1-6}$-alkoxy, Pyridylcarbamoyloxy-$C_{1-6}$-alkoxy, Benzoyloxy-$C_{1-6}$-alkoxy; sowie gegebenenfalls durch Halogen, $C_{1-6}$-Alkyl, $C_{1-6}$-Alkoxy oder Dihydroxy-$C_{1-6}$alkylaminocarbonyl substituiertes Pyridyl, Pyridyloxy, Pyridylthio, Pyridylamino, Pyridyl-$C_{1-6}$-alkyl, Pyridyl-$C_{1-6}$-alkoxy, Pyrimidinyl, Pyrimidinyloxy, Pyrimidinylthio, Pyrimidinylamino, Pyrimidinyl-$C_{1-6}$alkyl, Pyrimidinyl-$C_{1-6}$-alkoxy, Thienyl, Thienyl-$C_{1-6}$-alkyl, Thienyl-$C_{1-6}$-alkoxy, Furyl, Furyl-$C_{1-6}$-alkyl und/oder Furyl-$C_{1-6}$-alkoxy substituiert sein können;

"Heterocyclischer Ring" bzw. "Heterocyclyl" allein oder in Zusammensetzungen mono- oder bicyclische, gesättigte und ungesättigte heterocyclische Reste mit 1 bis 4 Stickstoff- und/oder 1 oder 2 Schwefel- oder Sauerstoffatomen bezeichnet, die ein- oder mehrfach, insbesondere durch (im Falle ungesättigter Heterocyclylreste) $C_{1-6}$-Alkyl, Hydroxy, $C_{1-6}$-Alkoxy, Nitro oder Halogen oder durch Substituenten, wie oben für "Aryl" definiert, substituiert sein können oder (im Falle gesättigter Heterocyclylreste) durch $C_{1-6}$-Alkyl oder $C_{1-6}$-Alkoxy substituiert sein können.

Der hier verwendete Ausdruck "$C_{1-6}$" bezeichnet Gruppen mit 1-6, vorzugsweise 1-4 C-Atomen. Beispiele von $C_{1-6}$-Alkyl- und Alkoxyreste sind Methyl, Aethyl, n-Propyl, Isopropyl, n-Butyl, Isobutyl, sec.-Butyl, tert-Butyl, Pentyl, Hexyl bzw. Methoxy, Aethoxy, Propoxy, Isopropoxy, Butoxy, Isobutoxy, sec.-Butoxy und tert-Butoxy. $C_{1-6}$-Alkylendioxyreste sind vorzugsweise Methylendioxy, Aethylendioxy und Propylendioxy. Beispiele von $C_{1-6}$-Alkanoylresten sind Acetyl, Propionyl und Butyryl. $C_{3-6}$-Cycloalkyl bedeutet einen gesättigten, cyclischen Kohlenwasserstoffrest mit 3-6 Kohlenstoffatomen, d.h. Cyclopropyl, Cyclobutyl, Cyclopentyl und Cyclohexyl. $C_{1-8}$-Alkylenreste sind z.B. Methylen, Äthylen, Propylen, 2-Methyl-propylen, Tetra-, Penta- und Hexamethylen; $C_{2-8}$-Alkenylenreste sind z.B. Vinylen und Propenylen; $C_{2-8}$-Alkinylenreste ist z.B. Aethinylen "Acyl" bedeutet $C_{1-6}$- Acylreste oder Benzoyl. "Aryl" bezeichnet allein oder in Zusammensetzungen Phenyl, Naphthyl oder Tetrahydronaphthyl, welche Gruppen ein- oder mehrfach durch $C_{1-6}$-Alkyl, Trifluormethyl, Nitro, Amino, $C_{1-6}$-Alkenyl, $C_{1-6}$-Alkoxy, $C_{1-6}$-Alkylcarbonyloxy, Hydroxy, Halogen, Cyano, Carbamoyl, $C_{1-6}$-Alkylendioxy, gegebenenfalls durch Halogen, $C_{1-6}$-Alkyl, $C_{1-6}$-Alkoxy oder Dihydroxy-$C_{1-6}$-alkylaminocarbonyl substituiertes Phenyl, Phenoxy, Phenylthio, Phenyl-$C_{1-6}$-alkyl, Phenyl-$C_{1-6}$-alkoxy, $C_{1-6}$-Alkoxycarbonylphenyl, Hydroxy-$C_{1-6}$-alkylphenyl, Benzyloxy, Pyridylcarbonylamino-$C_{1-6}$-alkyl, $C_{1-6}$-Alkenyloxy, $C_{1-6}$-Alkoxy-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridylcarbamoyloxy-$C_{1-6}$-alkoxy, Benzoyloxy-$C_{1-6}$-alkoxy; sowie gegebenenfalls durch Halogen, $C_{1-6}$-Alkyl, $C_{1-6}$-Alkoxy oder Dihydroxy-$C_{1-6}$-alkylaminocarbonyl substituiertes Pyridyl, Pyridyloxy, Pyridylthio, Pyridylamino, Pyridyl-$C_{1-6}$-alkyl, Pyridyl-$C_{1-6}$-alkoxy, Pyrimidinyl, Pyrimidinyloxy, Pyrimidinylthio, Pyrimidinylamino, Pyrimidinyl-$C_{1-6}$-alkyl, Pyrimidinyl-$C_{1-6}$-alkoxy, Thienyl, Thienyl-$C_{1-6}$alkyl, Thienyl-$C_{1-6}$-alkoxy, Furyl, Furyl-$C_{1-6}$-alkyl und/oder Furyl-$C_{1-6}$-alkoxy substituiert sein können.

Der Ausdruck "Heterocyclyl" bezeichnet allein oder in Zusammensetzungen mono- oder bicyclische, gesättigte und ungesättigte heterocyclische Reste mit 1 bis 4 Stickstoff- und/oder 1 oder 2 Schwefel- oder Sauerstoffatomen, die ein- oder mehrfach, insbesondere durch (im Falle ungesättigter Heterocyclylreste) $C_{1-6}$-Alkyl, Hydroxy, $C_{1-6}$-Alkoxy, Nitro oder Halogen oder durch Substituenten, wie oben für "Aryl" definiert, substituiert sein können oder (im Falle gesättigter Heterocyclylreste) durch $C_{1-6}$-Alkyl oder $C_{1-6}$-Alkoxy substituiert sein können. Beispiele für Heterocyclyl-Reste sind Pyridyl, Thienyl, Pyrazinyl, Triazolyl, Imidazolyl, Benzthiazolyl, Furyl, Pyrimidinyl, Morpholinyl, Chinazolinyl, Chinolyl, Chinoxalinyl, Isochinolyl, Benzo[b]thienyl, Isobenzofuranyl, Benzimidazolyl, 2-Oxo-benzimidazolyl oder Thiazolyl. Beispiele substituierter Heterocyclyl-Reste sind Nitrobenzthiazolyl, Phenyl-tetrazolyl, Phenyl-oxadiazolyl, Thienyl-oxadiazolyl, Furanyl-oxadiazolyl, Benzyl-oxadiazolyl, Phenyl-oxazolyl. Beispiele gesättigter Heterocyclyl-Reste sind Dioxolanyl, Dioxanyl, Dithiolanyl, Dithianyl, Pyrrolidinyl, Piperidinyl, Piperazinyl, 4-Methylpiperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl,

4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxoimidazolidinyl, 2-Oxo-oxazolidinyl, 2-Oxo-pyrrolidinyl, 2-Oxo-[1,3]oxazinyl, 2-Oxo-tetrahydro-pyrimidinyl und dergleichen.

Der Ausdruck "Heterocyclischer Ring" hat die gleiche Bedeutung wie "Heterocyclyl".

Beispiele für durch $NR^5R^6$ dargestellte 5- und 6-gliedrige heterocyclische Ringe sind Pyrrolidinyl, Piperidinyl, Piperazinyl, Morpholinyl, Thiomorpholinyl, 2-Hydroxymethylpyrrolidinyl, 3-Hydroxypyrrolidinyl, 3,4-Dihydroxypyrrolidinyl, 4-Hydroxypiperidinyl, 4-Oxopiperidinyl, 3,5-Dimethylmorpholinyl, 4,4-Dioxothiomorpholinyl, 4-Oxothiomorpholinyl, 2,6-Dimethylmorpholinyl, 2-Oxo-imidazolidinyl, 2-Oxo-oxazolidinyl, 2-Oxo-pyrrolidinyl, 2-Oxo-[1,3]-oxazinyl, 2-Oxo-tetrahydro-pyrimidinyl und dergleichen. Beispiele für durch $CR^7R^8$ dargestellte 3-7-gliedrige Ringe sind Cyclopentyl, Cyclohexyl, Cycloheptyl, 1,3-Dioxolanyl, 1,3-Dioxanyl,1,3-Dithiolanyl und 1,3-Dithianyl.

Der Ausdruck Polyhydroxy-alkyl bezeichnet $C_1$-$C_7$ - Alkylreste, die mit 2-6 Hydroxy-Gruppen substituiert sein können, wie z.B. Glyceryl, Arabityl, Sorbityl etc.

Die Verbindungen der Formel I besitzen mindestens zwei asymmetrische Kohlenstoffatome und können deshalb in Form von optisch reinen Diastereomeren; Diastereomerengemischen, diastereomeren Racematen oder Gemischen von diastereomeren Racematen vorliegen. Die Erfindung umfaßt alle diese Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugte erfindungsgemässe Verbindungen sind solche der allgemeinen Formel

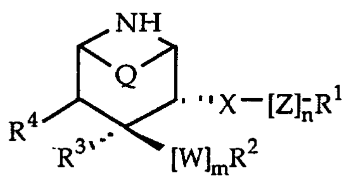

I A

worin $R^1$ - $R^4$, Q, W, X und Z, n und m die oben angegebene Bedeutung haben.

Eine weitere, bevorzugte Gruppe von Verbindungen der Formel I sind Verbindungen der Formel

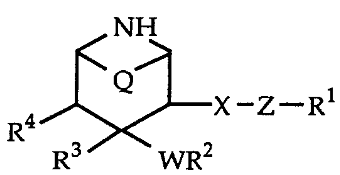

I - 1

worin

| | |
|---|---|
| $R^1$ | die oben gegebene Bedeutung hat; |
| $R^2$ | Phenyl, Cyclohexyl, durch Halogen, Hydroxy, Cyano, Trifluormethyl, $C_{1-6}$-Alkyl, Halo-$C_{1-6}$-alkyl, Hydroxy-$C_{1-6}$-alkyl, $C_{1-6}$-Alkoxy-$C_{1-6}$-alkyl, Cyano-$C_{1-6}$-alkyl, Carboxy-$C_{1-6}$-alkyl,$C_{1-6}$-Alkanoyloxy-$C_{1-6}$-alkyl, $C_{1-6}$-Alkoxycarbonyloxy-$C_{1-6}$-alkyl, $C_{1-6}$-Alkoxycarbonyl,$C_{1-6}$-Alkoxy, $C_{1-6}$-Alkylendioxy, oder durch einen Rest $L^1$-$T^1$-$L^2$-$T^2$-$L^3$-$T^3$-$L^4$-$T^4$-$L^5$-U substituiertes Phenyl oder Cyclohexyl; oder Naphthyl oder Acenaphthyl; |
| $L^1$, $L^2$, $L^3$, $L^4$ und $L^5$ | unabhängig voneinander eine Bindung, $C_{1-8}$-Alkylen, $C_{2-8}$- Alkenylen oder $C_{2-8}$- Alkinylen, oder abwesend sind; |
| $T^1$, $T^2$, $T^3$ und $T^4$ | unabhängig voneinander (a) eine Bindung darstellen oder abwesend sind, oder eine der Gruppen (b) -CH(OH)-; (c) -CH($OR^6$)-; (d) -CH($NR^5R^6$)-; (e) -CO-; (f) -$CR^7R^8$-; (g) -O- oder -$NR^6$-, (h) -$S(O)_{0-2}$-; (i)-$SO_2NR^6$ -; (j) -$NR^6$ $SO_2$-; (k) -$CONR^6$-; (l) -$NR^6$ CO-; (m) -O-CO-; (n) -CO-O-; (o) -O-CO-O- oder (p) -O-CO-$NR^6$- darstellen, wobei die von (b), (d), (e) und (g)-(p) ausgehenden Bindungen zu einem C-Atom der benachbarten Gruppe führen und dieses C-Atom gesättigt ist, falls die Bindung von einem Heteroatom ausgeht, und wobei nicht mehr als zwei Gruppen (b)-(f), drei Gruppen (g)-(h) und eine Gruppe (i)-(p) anwesend sind; |

| | |
|---|---|
| $R^3$ | Wasserstoff, Hydroxy, $C_{1-6}$-Alkoxy oder $C_{1-6}$-Alkenyloxy; |
| $R^4$ | Wasserstoff, $C_{1-6}$-Alkyl, $C_{1-6}$-Alkenyl, $C_{1-6}$-Alkoxy, Hydroxy-$C_{1-6}$-alkyl, $C_{1-6}$-Alkoxy-$C_{1-6}$-alkyl oder Benzyl ist; |
| $R^5$ und $R^6$ | Wasserstoff, $C_{1-6}$-Alkyl oder Acyl, oder gemeinsam mit dem N-Atom, an das sie gebunden sind, einen 5- oder 6-gliedrigen heterocyclischen Ring, der ein zusätzliches N-, O- oder S-Atom enthalten kann; |
| $R^7$ und $R^8$ | gemeinsam mit dem C-Atom, an das sie gebunden sind, einen 3-7 -gliedrigen Ring bedeuten, der ein oder zwei Sauerstoff- oder Schwefelatome enthalten kann; |
| U | Wasserstoff, $C_{1-6}$-Alkyl, $C_{3-6}$-Cycloalkyl, Cyano, Aryl oder Heterocyclyl; |
| Q | Aethylen oder abwesend ist; |
| X | Sauerstoff, Schwefel oder eine Gruppe, -CHOR$^9$-, oder -OCO- und $R^9$ Wasserstoff, $C_{1-6}$-Alkyl, Acyl oder Aryl-$C_{1-6}$-alkyl ist; |
| W | abwesend ist; oder Sauerstoff oder Schwefel darstellen kann, wenn $R^3$ Wasserstoff ist; |
| Z | $C_{1-6}$-Alkylen oder abwesend ist. |

Weiterhin sind Verbindungen der Formeln I und IA bevorzugt, in denen W abwesend ist, und solche, in denen Q abwesend ist. X ist vorzugsweise Sauerstoff, Schwefel oder -CO-; Z ist vorzugsweise Methylen.

Bevorzugte Reste $R^2$ sind Phenyl, durch Halogen, Hydroxy, Cyano, Trifluormethyl, $C_{1-6}$-Alkyl, Halo- $C_{1-6}$-alkyl, Hydroxy- $C_{1-6}$-alkyl, $C_{1-6}$-Alkoxy-$C_{1-6}$-alkyl, Cyano-$C_{1-6}$-alkyl, Carboxy-$C_{1-6}$-alkyl, $C_{1-6}$-Alkanoyloxy-$C_{1-6}$-alkyl, $C_{1-6}$-Alkoxycarbonyloxy- $C_{1-6}$-alkyl, $C_{1-6}$-Alkoxycarbonyl, $C_{1-6}$-Alkoxy oder $C_{1-6}$-Alkylendioxy substituiertes Phenyl.

Ebenfalls bevorzugte Reste $R^2$ sind durch einen Rest $L^1$-$T^1$-$L^2$-$T^2$-$L^3$-$T^3$-$L^4$-$T^4$-$L^5$-U substituiertes Phenyl, wobei $L^1$ und $L^2$ vorzugsweise abwesend oder $C_{1-8}$-Alkylen sind und $L^3$ abwesend ist und U Wasserstoff, $C_{1-6}$-Alkyl, $C_{3-6}$-Cycloalkyl, Phenyl, durch $C_{1-6}$-Alkyl, $C_{1-6}$-Alkoxy, $C_{1-6}$-Alkylthio, $C_{1-6}$-Alkylsulfinyl, $C_{1-6}$-Alkylendioxy, Halogen, Benzoyl- $C_{1-6}$-alkyl, Halogen- $C_{1-6}$-alkyl, $C_{1-6}$-Alkanoyloxy oder Hydroxy substituiertes Phenyl; oder Naphthyl; oder Pyridyl, Thienyl, Pyrazinyl, Triazolyl, Imidazolyl, Phenyl-oxadiazolyl, Thienyl-oxadiazolyl, Furyl-oxadiazolyl, Phenyl-oxazolyl, Benzthiazolyl, Furyl, Pyrimidinyl, Nitrobenzthiazolyl Phenyltetrazolyl oder Morpholinyl darstellt.

Bei den Guppen $T^1$-$T^4$ sind die Bedeutungen (a)-(c), (e)-(h), (m) und (n) bevorzugt.

Beispiele besonders bevorzugter Reste $R^2$ sind Phenyl oder Phenyl substituiert durch 2-Benzothiazolyl-thio-$C_{1-6}$-alkyl, 2-Benzyloxy-3-methoxypropoxy, 2-Benzoyloxy-3-methoxypropoxy, 2,3-Dihydroxypropoxy, 2-Hydroxy-3-benzylamino-propoxy, 2-Hydroxy-3-phenoxypropoxy, 2-Hydroxy-3-phenylthiopropoxy, 2-Methoxy-3-phenoxypropoxy, 2-Methoxy-3-benzyloxypropoxy, 2-Methyl-3-fluor-phenylbutyryloxy-$C_{1-6}$-alkoxy, 2-$C_{2-6}$-Alkenyloxy-4-phenylbutyl, 3,4,5-Trimethoxyphenyl-oxadiazolyl-$C_{1-6}$-alkoxy, 6-Nitro-2-benzothiazolyl-thio-$C_{1-6}$-alkyl, Benzamido-$C_{1-6}$-alkoxy, Benzamido-$C_{1-6}$-alkyl, Benzoyl-$C_{1-6}$-alkoxy und Ketale davon, Benzoyl-$C_{1-6}$-alkyl und Ketale davon, Benzoyl-$C_{1-6}$-alkyl-aminocarbonyl-$C_{1-6}$-alkyl, Benzoyl-$C_{1-6}$alkoxycarbonyl-$C_{1-6}$-alkyl, Benzoyl-$C_{1-6}$-alkylaminocarbonyl, Benzoyloxy, Benzoyloxy-$C_{1-6}$-alkyl-benzoyloxy-$C_{1-6}$-alkoxy, Benzoyloxy-$C_{1-6}$-alkoxy, Benzoyloxy-$C_{1-6}$-alkyl, Benzthiazolylhio-$C_{1-6}$-alkoxy, Benzthiazolylthio-$C_{1-6}$alkyl, Benzylcarbamoyl-$C_{1-6}$-alkoxy, Benzyloxy-$C_{1-6}$alkylcarbonyloxy-$C_{1-6}$alkyl, Benzyloxy-$C_{1-6}$-alkoxy, Benzylthio-$C_{1-6}$-alkoxy, Carbamoyloxy-$C_{1-6}$alkoxy, Carbamoyloxy-$C_{1-6}$-alkyl, Carboxy-$C_{1-6}$-alkoxy, Carboxy-$C_{1-6}$-alkyl, Cyano, Cyano-$C_{1-6}$-alkoxy, Cyano-$C_{1-6}$-alkyl, Cyanophenyl-$C_{1-6}$-alkoxy, Cyclohexylcarbonyloxy-$C_{1-6}$-alkyl, Cyclopropylcarbonyloxy-$C_{1-6}$-alkyl, Cyclopropyloxy-benzyloxy-$C_{1-6}$-alkoxy, Dioxolanyl-$C_{1-6}$-alkoxy, Furyloxadiazolyl-$C_{1-6}$-alkoxy, Furoyloxy-$C_{1-6}$-alkoxy, Halo-phenoxy-$C_{1-6}$-alkyl, Halobenzoyl-$C_{1-6}$-alkoxy, Halobenzoyloxy-$C_{1-6}$-alkyl, Halobenzoyloxy-$C_{1-6}$alkoxy, Halobenzyloxy-$C_{1-6}$-alkoxy, Halogen, Halogen-$C_{1-6}$-alkyl, Halophenoxy, Halophenyl-oxadiazolyl-$C_{1-6}$-alkoxy, Hydroxy, Hydroxybenzoyloxy-$C_{1-6}$-alkyl, Hydroxy-benzyloxy-$C_{1-6}$-alkoxy, Hydroxy-$C_{1-6}$-alkoxy, Hydroxy-$C_{1-6}$-alkyl, Imidazolylcarbonyloxy-$C_{1-6}$-alkyl, Methoxybenzoyl-$C_{1-6}$alkyl, Methoxybenzyloxy-$C_{1-6}$-alkoxy, Methylendioxybenzoyl-$C_{1-6}$-alkoxy, Morpholino-$C_{1-6}$-alkoxy, Morpholinocarbonyloxy-$C_{1-6}$-alkoxy, Morpholinocarbonyloxy-$C_{1-6}$-alkyl, N-Methylaminophenyl-carbonyloxy-$C_{1-6}$alkyl, N-Methyl-benzylamino-$C_{1-6}$-alkoxy, N-Methylpyrrolylcarbonyloxy-$C_{1-6}$-alkoxy, N-$C_{1-6}$-Alkylbenzamido-$C_{1-6}$-alkyl, Naphthyl-$C_{1-6}$-alkoxy, Nicotinoyloxy-$C_{1-6}$-alkoxy, Nicotinoyloxy-$C_{1-6}$-alkyl, $C_{1-6}$-Alkanoylbenzoyloxy-$C_{1-6}$-alkyl, $C_{1-6}$-Alkanoyloxy-$C_{1-6}$-alkoxy, $C_{1-6}$-Alkanoyloxy-$C_{1-6}$alkyl, $C_{1-6}$-Alkenyl-benzyloxy-$C_{1-6}$-alkoxy, $C_{1-6}$-Alkenyloxy, $C_{1-6}$-Alkenyloxybenzyloxy-$C_{1-6}$-alkoxy, $C_{1-6}$-Alkoxy, $C_{1-6}$-Alkoxy-benzoyloxy-$C_{1-6}$-alkyl, $C_{1-6}$-Alkoxy-carbonyl, $C_{1-6}$-Alkoxy-$C_{1-6}$-alkyl, $C_{1-6}$-Alkoxybenzoylamino-$C_{1-6}$-alkyl, $C_{1-6}$-Alkoxybenzylcarbonyloxy-$C_{1-6}$-alkyl, $C_{1-6}$-Alkoxy-benzyloxy-$C_{1-6}$-alkoxy, $C_{1-6}$-Alkoxy-ben-

zylthio-$C_{1-6}$-alkoxy, $C_{1-6}$-Alkoxycarbonyl, $C_{1-6}$-Alkoxycarbonyl-$C_{1-6}$-alkoxy, $C_{1-6}$-Alkoxycarbonyl-$C_{1-6}$-alkyl, $C_{1-6}$-Alkoxyphenyloxadiazolyl-$C_{1-6}$-alkoxy, $C_{1-6}$-Alkyl, $C_{1-6}$-Alkylbenzyloxy-$C_{1-6}$-alkoxy, $C_{1-6}$-Alkylendioxy, $C_{1-6}$-Alkylendioxybenzyloxy-$C_{1-6}$-alkoxy, $C_{1-6}$-Alkylsulfonylbenzoyl-$C_{1-6}$-alkoxy, $C_{1-6}$-Alkylthiobenzoyloxy-$C_{1-6}$-alkoxy, $C_{1-6}$Alkylthiobenzyloxy-$C_{1-6}$-alkoxy, Benzoyloxybenzyl-$C_{1-6}$-alkoxy, Hydroxybenzyl-$C_{1-6}$alkoxy, $C_{1-6}$-Alkoxybenzyl-$C_{1-6}$-alkoxy, $C_{1-6}$-Alkoxybenzylcarbonyloxyalkoxy, Phenoxy-benzyloxy-$C_{1-6}$-alkoxy, Phenoxycarbonyl-$C_{1-6}$-alkyl, Phenoxy-$C_{1-6}$-alkenyloxy, Phenoxy-$C_{1-6}$-alkinyloxy, Phenyl-$C_{1-6}$alkanoylamino-$C_{1-6}$-alkyl, Phenyl-$C_{1-6}$-alkenyloxy, Phenyl-$C_{1-6}$-alkoxy, Phenoxy-$C_{1-6}$-alkyl, Phenyl-$C_{1-6}$-alkylaminocarbonyl, Phenoxy-$C_{1-6}$alkylcarbonyl-$C_{1-6}$-alkoxy, Phenyl-$C_{1-6}$-alkylaminocarbonyl-$C_{1-6}$-alkyl, Phenylaminocarbonyloxy-$C_{1-6}$-alkoxy, Phenylaminocarbonyloxy-$C_{1-6}$-alkyl, Phenyl-hydroxy-$C_{1-6}$-alkyl, Phenyl-oxadiazolyl-$C_{1-6}$-alkoxy, Phenyl-oxadiazolyl-$C_{1-6}$-alkoxy, Phenyl-oxadiazolyl-$C_{1-6}$-alkyl, Phenyl-oxazolyl-$C_{1-6}$-alkoxy, Phenyloxy-$C_{1-6}$-alkoxy, Phenylsulfamoyl-$C_{1-6}$-alkyl, Phenylsulfinyl-$C_{1-6}$-alkyl, Phenylsulfonyl-$C_{1-6}$-alkoxy, Phenylsulfonyl-$C_{1-6}$-alkyl, Phenyltetrazolyl-thio-$C_{1-6}$-alkyl, Phenylthio-$C_{1-6}$-alkoxy, Phenylthio-$C_{1-6}$-alkyl, Pyrazinylcarbonyloxy-$C_{1-6}$-alkyl, Pyridylaminocarbonyloxy-$C_{1-6}$-alkoxy, Pyridylaminocarbonyloxy-$C_{1-6}$-alkyl, Pyridylcarbamoyloxy, Pyridyl-$C_{1-6}$-alkoxy-$C_{1-6}$-alkoxy, Pyridyl-$C_{1-6}$-alkoxy-$C_{1-6}$-alkyl, Pyridyl-oxadiazolyl-$C_{1-6}$-alkoxy, Pyridylthio-$C_{1-6}$-alkyl, Pyrimidinyloxy-$C_{1-6}$-alkoxy, Pyrimidinylthio-$C_{1-6}$-alkyl, Thenoyloxy-$C_{1-6}$-alkoxy, Thenoyloxy-$C_{1-6}$-alkyl, Thienyl-oxadiazolyl-$C_{1-6}$-alkoxy, Triazolyl-$C_{1-6}$-alkoxy, Trifluormethylbenzyloxy-$C_{1-6}$alkoxy oder Trifluormethyl.

Bevorzugte Reste $R^4$ sind 2-Oxo-imidazolidin-1-yl-$C_{1-6}$-alkyl, 4-Hydroxypiperidin- 1-yl-$C_{1-6}$-alkoxy, 4-Hydroxy-piperidin-1-yl-$C_{1-6}$-alkoxy-$C_{1-6}$-alkyl, 4-Methyl-piperazin-1-yl-$C_{1-6}$-alkoxy, 4-Methyl-piperazin-1-yl-$C_{1-6}$-alkoxy-$C_{1-6}$-alkyl, 4-Methyl-piperazin-1-yl-$C_{1-6}$-alkyl-carbamoyloxy-$C_{1-6}$-alkyl, 1,2,4-Triazolyl-$C_{1-6}$-alkyl, Amino, Amino-$C_{1-6}$-alkyl, Amino-$C_{1-6}$-alkyl-amino, Amino-$C_{1-6}$-alkyl-amino-$C_{1-6}$-alkyl, Amino-$C_{1-6}$-alkoxyl, Amino-$C_{1-6}$-alkoxyl-$C_{1-6}$-alkyl, Aminocarbonyloxy-$C_{1-6}$-alkyl, Benzyloxy oder durch $C_{1-6}$-Alkyl, $C_{1-6}$-Alkenyl, $C_{1-6}$-Alkoxy, Trifluormethoxy, $C_{1-6}$-Alkylthio, Hydroxy oder Halogen substituiertes Benzyloxy, Benzyloxy-$C_{1-6}$-alkyl oder durch $C_{1-6}$-Alkyl, $C_{1-6}$-Alkenyl, $C_{1-6}$-Alkoxy oder Halogen substituiertes Benzyloxy, Carbamoyloxy-$C_{1-6}$-alkyl, Cyano-$C_{1-6}$-alkyl, Di-$C_{1-6}$-Alkyl-amino, Di-$C_{1-6}$-Alkyl-amino-$C_{1-6}$-alkyl, Di-$C_{1-6}$-Alkyl-amino-$C_{1-6}$-alkyl-(N-$C_{1-6}$-alkyl)-amino-$C_{1-6}$-alkyl, Di-$C_{1-6}$-Alkyl-amino-$C_{1-6}$-alkyl-amino Di-$C_{1-6}$-Alkyl-amino-$C_{1-6}$alkyl-amino-$C_{1-6}$-alkyl, Di-$C_{1-6}$-Alkyl-amino-$C_{1-6}$-alkoxyl Di-$C_{1-6}$-Alkyl-amino-$C_{1-6}$-alkoxyl-$C_{1-6}$-alkyl, Dihydroxy-$C_{1-6}$-alkoxy, Dihydroxy-$C_{1-6}$-alkoxy-$C_{1-6}$alkyl, Dihydroxy-$C_{1-6}$-alkyl-amino, Dihydroxy-$C_{1-6}$-alkyl-amino-$C_{1-6}$-alkyl, Guanidinyl-$C_{1-6}$-alkoxy-$C_{1-6}$-alkyl, Guanidinyl-$C_{1-6}$-alkyl, Hydroxy, Hydroxy-$C_{1-6}$-alkyl, Sulfooxy-$C_{1-6}$-alkyl,Hydroxy-$C_{1-6}$-alkoxyl, Hydroxy-$C_{1-6}$-alkoxyl-$C_{1-6}$-alkyl, Morpholin-4-yl-$C_{1-6}$-alkoxy, Morpholin-4-yl-$C_{1-6}$-alkoxy-$C_{1-6}$-alkyl, Morpholin-4-yl-$C_{1-6}$-alkyl-carbamoyloxy-$C_{1-6}$-alkyl, Naphthyl-alkoxy oder durch $C_{1-6}$-Alkoxy substituiertes Naphthyl-alkoxy, $C_{1-6}$-Alkoxy, $C_{1-6}$-Alkoxy-$C_{1-6}$-alkoxy $C_{1-6}$-Alkoxy-$C_{1-6}$-alkoxy-$C_{1-6}$-alkyl, $C_{1-6}$-Alkoxy-$C_{1-6}$-alkyl, $C_{1-6}$-Alkyl, $C_{1-6}$-Alkylsulfonylamino-$C_{1-6}$-alkyl, Phenoxy-$C_{1-6}$-alkyl oder durch $C_{1-6}$-Alkyl, $C_{1-6}$-Alkoxy substituiertes Phenoxy-$C_{1-6}$-alkyl, Phenyl-thio-$C_{1-6}$-alkyl oder durch $C_{1-6}$-Alkyl, $C_{1-6}$-Alkoxy substituiertes Phenylthio-$C_{1-6}$alkyl, Piperazin-4-yl-$C_{1-6}$-alkoxy, Piperazin-4-yl-$C_{1-6}$-alkoxy-$C_{1-6}$-alkyl, Piperidin-1-yl-$C_{1-6}$-alkyl-carbamoyloxy-$C_{1-6}$-alkyl, Piperidin-4-yl-$C_{1-6}$-alkoxy, Piperidin-4-yl-$C_{1-6}$-alkoxy-$C_{1-6}$-alkyl, Pyridyl-$C_{1-6}$-alkoxyl, Pyridyl-$C_{1-6}$alkoxy-$C_{1-6}$-alkyl, Pyridylthio-$C_{1-6}$-alkyl, Pyrimidinyloxy-$C_{1-6}$-alkyl oder durch $C_{1-6}$-Alkoxy substituiertes Pyrimidinyloxy-$C_{1-6}$-alkyl, Tetrazolyl-$C_{1-6}$alkyl, Trifluormethylsulfonylamino-$C_{1-6}$-alkyl oder Wasserstoff.

Weitere bevorzugte Gruppen von Verbindungen der Formel I sind solche, in denen $R^2$ Cyclohexyl oder Benzoyloxycyclohexyl ist; solche, in denen $R^2$ Naphthyl, Tetrahydronaphthyl oder Acenaphthyl ist; solche, in denen $R^2$ Pyridyl oder Oxopyridyl, oder durch 3-H-2-Thioxo-benzthiazolyl, $C_{1-6}$-Alkoxyphenyl-$C_1$-6-alkoxy-$C_{1-6}$-alkoxy, Phenyl-$C_{1-6}$-alkoxy-$C_{1-6}$-alkoxy, Phenyl-$C_{1-6}$-alkyl, Cyclohexyl-$C_{1-6}$-alkoxy, Phenoxy-$C_{1-6}$-alkyl oder Phenyl-$C_{1-6}$-alkoxy-$C_{1-6}$-alkyl substituiertes Pyridyl oder Oxopyridyl ist; solche, in denen $R^2$ Pyrimidinyl oder durch Benzodioxanyl-$C_{1-6}$-alkoxy, Biphenylyloxy, Biphenylyl-$C_{1-6}$-alkoxy, Cyclohexyl-$C_{1-6}$-alkoxy, Cyclohexyloxy-$C_{1-6}$-alkoxy, Halophenyl-$C_{1-6}$-alkoxy, Halophenyl-oxadiazolyl-$C_{1-6}$-alkoxy, Indanyl-$C_{1-6}$-alkoxy, Naphthyl-$C_{1-6}$-alkoxy, Phenyl-$C_{1-6}$-alkyl, N-$C_{1-6}$-Alkyl-phenyl-$C_{1-6}$-alkoxy-$C_{1-6}$-alkylamino, N-$C_{1-6}$-Alkyl-phenyl-$C_{1-6}$-alkylamino, $C_{1-6}$-Alkythio, $C_{1-6}$-Alkoxy, $C_{1-6}$-Alkoxyphenyl-$C_{1-6}$-alkoxy-$C_{1-6}$-alkoxy, $C_{1-6}$-Alkoxyphenyl-$C_{1-6}$-alkylamino, $C_{1-6}$-Alkylphenyl-$C_{1-6}$alkylamino, Halophenyl-$C_{1-6}$-alkylamino, Halophenoxy-$C_{1-6}$-alkoxy, $C_{1-6}$-Alkylpyridyl-$C_{1-6}$-alkoxy, Phenyl-$C_{1-6}$-alkoxy-$C_{1-6}$-alkoxy, Phenyl-$C_{1-6}$-alkoxy-$C_{1-6}$-alkylthio, Phenyl-$C_{1-6}$-alkoxy-$C_{1-6}$-alkylamino, Phenoxy-phenyl-$C_{1-6}$-alkoxy, Phenoxy-phenoxy, Phenyl-$C_{1-6}$-alkinyloxy, Phenyl-$C_{1-6}$-alkoxy-$C_{1-6}$-alkoxy, Phenylthio-$C_{1-6}$-alkoxy, Phenyl-oxazolyl-$C_{1-6}$-alkoxy, Phenyl-$C_{1-6}$-alkinyloxy, Phenyl-$C_{1-6}$-alkenyloxy, Phenyl-$C_{1-6}$-alkylamino, Phenyl-pyridyl-$C_{1-6}$-alkoxy oder Phenylpyridyl-$C_{1-6}$-alkylaminosubstituiertes Pyrimidinyl ist; und schliesslich solche, in denen $R^2$ Halobenzoyl-$C_{1-6}$-alkyl-triazolyl, Phenyl-$C_{1-6}$-alkoxy-$C_{1-6}$alkyl-triazolyl oder Phenyl-$C_{1-6}$-alkoxy-$C_{1-6}$-alkoxy-$C_{1-6}$-alkyl-triazolyl ist.

Ganz besonders bevorzugt sind folgende Verbindungen:

4-[2-[7-[(3R,4R)-4-[4-(3-Benzyloxy-propoxy)-phenyl]-piperidin-3-yloxymethyl]-naphthalin-2-yloxy]-äthyl]-morpholin

(R)-3-[7-[(3R,4R)-4-[4-(3-Benzyloxy-propoxy)-phenyl]-piperidin-3-yloxymethyl]-naphthalin-2-yloxy]-propan-1,2-diol

(S)-3-[7-[(3R,4R)-4-[4-(3-Benzyloxy-propoxy)-phenyl]-piperidin-3-yloxymethyl]-naphthalin-2-yloxy]-propan-1,2-diol

(R)-3-[2-[7-[(3R,4R)-4-[4-(3-Benzyloxy-propoxy)-phenyl]-piperidin-3-yloxymethyl]-naphthalin-2-yloxy]-äthoxy]-propan-1,2-diol

(S)-3-[2-[7-[(3R,4R)-4-[4-(3-Benzyloxy-propoxy)-phenyl]-piperidin-3-yloxymethyl]-naphthalin-2-yloxy]-äthoxy]-propan-1,2-diol

1-[2-[7-[(3R,4R)-4-[4-(3-Benzyloxy-propoxy)-phenyl]-piperidin-3-yloxymethyl]-naphthalin-2-yloxy]-äthyl]-4-methyl-piperazin

1-[(3R,4S-4-[4-(3-Benzyloxy-propoxy)-phenyl]-piperidin-3-yl]-2-naphthalin-2-yl-äthanon

(3R,4S,5S)-4-[4-(3-Benzyloxy-propoxy)-phenyl]-3-(1,4-dimethoxy-naphthalin-2-ylmethoxy)-piperidin-5-ol

3R,4R)-4-[4-(3-Benzyloxy-propoxy)-phenyl]-3-[7-[(R)-2,3-dihydroxy-propoxymethyl]-naphthalin-2-ylmethoxy]-piperidin

(3R,4R)-4-[4-(3-Benzyloxy-propoxy)-phenyl]-3-[7-[(S)-2,3-dihydroxy-propoxymethyl]-naphthalin-2-ylmethoxy]-piperidin

(3R,4R)-4-[4-(3-Benzyloxy-propoxy)-phenyl]-3-[6-[(R)-2,3-dihydroxy-propoxymethyl]-naphthalin-2-ylmethoxy]-piperidin

(3R,4R)-4-[4-(3-Benzyloxy-propoxy)-phenyl]-3-[6-[(S)-2,3-dihydroxy-propoxymethyl]-naphthalin-2-ylmethoxy]-piperidin

4-[(3R,4S,5S)-4-[4-(3-Benzyloxy-propoxy)-phenyl]-3-(naphthalin-2-ylmethoxy)-piperidin-5-yloxy]-butan-1-ol

3-[(3R,4S,5S)-4-[4-(3-Benzyloxy-propoxy)-phenyl]-3-(naphthalin-2-ylmethoxy)-piperidin-5-yloxy]-propan-1-ol

1-{2-[(3R,4R,5S)-4-[4-(3-Benzyloxy-propoxy)-phenyl]-3-(naphthalin-2-ylmethoxy)-piperidin-5-yloxy]-äthyl}-4-methyl-piperazin

(3R,4R,5R)-4-[2-[4-[4(3-Benzyloxy-propoxy)-phenyl]-5-(naphthalin-2-ylmethoxy)-piperidin-3-ylmethoxy]-äthyl}-morpholin

(3R,4S,5S)-4-[4-(3-Benzyloxy-propoxy)-phenyl]-3-(4-methoxy-benzyloxy)-piperidin-5-ol

(3R,4s,5S)-4-[4-(3-Benzyloxy-propoxy)-phenyl]-3,5-bis-(4-methoxybenzyloxy)-piperidin

(3S,4R,5R)-4-[2-[4-[4-(3-Benzyloxy-propoxy)-phenyl]-5-(naphthalin-2-ylmethoxy)-piperidin-3-ylmethoxy]-äthyl]-morpholin

(3S,4R,5R)-4-[4-(3-Benzyloxy-propoxy)-phenyl]-3-methoxymethyl-5-(naphthalin-2-ylmethoxy)-piperidin

[3-(4-Methyl-piperazin-1-yl)-propyl]-carbaminsäure (3S,4R,5R)-4-[4-(3-benzyloxy-propoxy)-phenyl]-5-(naphthalin-2-ylmethoxy)-piperidin-3-ylmethylester

(3S,4R,5R)-4-[4-[4-(3-Benzyloxy-propoxy)-phenyl]-5-(naphthalin-2-ylmethoxy)-piperidin-3-ylmethylsulfanyl]-pyridin

2-(4-Cyclohexyl-butoxy)-5-[(3R,4R)-3-(1,4-dimethoxy-naphthalin-2-ylmethoxy)-piperidin-4-yl]-pyrimidin

(3'R,4'R)-6-(3-Cyclohexyl-propoxy)-3'-(1,4-dimethoxy-naphthalin-2-ylmethoxy)-1',2',3',4',5',6'-hexahydro-[3,4']bipyridin

(3S,4R,5R)-[4-[4-(3-Benzyloxy-propoxy)-phenyl]-5-(naphthalin-2-ylmethoxy)-piperidin-3-yl]-methanol

(3S,4R,5R)-N-[4-[4-(3-Benzyloxy-propoxy)-phenyl]-5-(naphthalin-2-ylmethoxy)-piperidin-3-ylmethyl]-N,N',N'-trimethyl-äthan-1,2-diamin

(3S,4R,5R)-[4-[4-(3-Benzyloxy-propoxy)-phenyl]-5-(naphthalin-2-ylmethoxy)-piperidin-3-ylmethyl]-diäthyl-amin

1-[(3R,4S,5S)-4-[4-(3-Benzyloxy-propoxy)-phenyl]-5-(2-morpholin-4-yl-äthoxymethyl)-piperidin-3-yl]-2-naphthalin-2-yl-äthanon

(3R,4R)-3-(1,4-Dimethoxy-naphthalin-2-ylmethoxy)-4-[4-[3-(2-methoxyphenoxy)-propoxy]-phenyl]-piperidin

(3R,4s,5S)-4-[4-(3-Benzyloxy-propoxy)-phenyl]-3,5-bis-(3,4,5-trimethoxybenzyloxy)-piperidin

(3R,4R,5S)-4-[4-(3-Benzyloxy-propoxy)-phenyl]-3-(naphthalin-2-ylmethoxy)-5-[1,2,4]triazol-1-ylmethyl-piperidin

(3R,4R)-4-[4-[3-(2-Methoxy-benzyloxy)-propoxy]-phenyl]-3-(quinolin-7-ylmethoxy)-piperidin

2-(7-{(3R,4R)-4-[4-(3-Benzyloxy-propoxy)-phenyl]-piperidin-3-yloxymethyl}naphthalin-2-ylmethoxy)-äthanol

7-{(3R,4R)-4-[4-(3-Benzyloxy-propoxy)-phenyl]-piperidin-3-yloxymethyl}naphthalin-2-ylmethyl)-dimethyl-amin

(3R,4R)-3-(4-Benzyloxy-naphthalin-2-ylmethoxy)-4-(4-fluor-phenyl)-piperidin

(3'R,4'R)-3'-(1,4-Dimethoxy-naphthalin-2-ylmethoxy)-6-[3-(2-methoxybenzyloxy)-propoxy]-1',2',3',4',5',6'-hexahydro-[3,4']bipyridin

(3R,4R)-3-(1,4-Dimethoxy-naphthalin-2-ylmethoxy)-4-[4-[3-(2-methoxybenzyloxy)-propoxy]-phenyl]-piperidin

(3S,4R,5R)-1-[4-[4-(3-Benzyloxy-propoxy)-phenyl]-5-(naphthalin-2-ylmethoxy)-piperidin-3-ylmethyl]-imidazolidin-2-on

(3R,4R)-4-[4-[3-(2-Methoxy-benzyloxy)-propoxy]-phenyl]-3-(2-oxo-1,2-dihydro-chinolin-7-ylmethoxy)-piperidin

(3R,4R)-3-(Isochinolin-7-ylmethoxy)-4-[4-[3-(2-methoxy-benzyloxy)-propoxy]-phenyl]-piperidin

(3R,4R)-4-[4-[3-(2-Methoxy-benzyloxy)-propoxy]-phenyl]-3-(1,2,3,4-tetrahydro-chinolin-7-ylmethoxy)-piperidin

1-[2-[7-[(3R,4R)-4-[4-[3-(2-Methoxy-benzyloxy)-propoxy]-piperidin-3-yloxymethyl]-naphthalin-2-yloxy] -äthyl]-4-methyl-piperazin

1-[2-[7-[(3R,4S,5S)-5-Hydroxy-4-[4-[-3-(2-methoxy-benzyloxy)-propoxy]-piperidin-3-yloxymethyl]-naphthalin-2-yloxy]-äthyl]-4-methyl-piperazin

(3R,4S,5S)-3-(1,4-Dimethoxy-naphthalin-2-ylmethoxy)-4-[4-[3-(2-methoxybenzyloxy)-propoxy]-phenyl]-piperidin-5-ol und

(3R,4R,5S)-3-(1,4-Dimethoxy-naphthalin-2-ylmethoxy)-4-{4-[3-(2-methoxybenzyloxy)-propoxy]-phenyl}-5-(1H-tetrazol-5-ylmethyl)-piperidin.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen.

Die Verbindungen der Formel I können erfindungsgemäss dadurch hergestellt werden, dass man aus einer Verbindung der Formel II

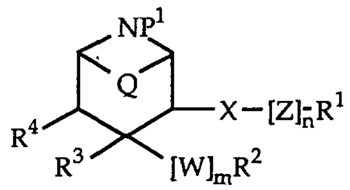

II

in der $P^1$ eine Schutzgruppe darstellt und die übrigen Symbole die oben angegebene Bedeutung haben und in $R^1$, $R^2$ und $R^4$ gegebenenfalls enthaltene Hydroxygruppen in geschützter Form vorliegen können, die Schutzgruppe(n) abspaltet, gewünschtenfalls in der so erhaltenen Verbindung der Formel I reaktionsfähige Gruppe funktionell abwandelt und/oder die Verbindung der Formel I in ein pharmazeutisch anwendbares Salz überführt.

Die Abspaltung einer Schutzgruppe $P^1$ und gegebenenfalls anwesender Hydroxyschutzgruppen kann in an sich bekannter Weise vorgenommen werden. Beispiele von Schutzgruppen $P^1$ sind übliche Aminoschutzgruppen wie tert-Butoxycarbonyl, Benzyloxycarbonyl, Vinyloxycarbonyl, Alkylsilylalkyloxycarbonyl wie 2-(Trimethylsilyl)äthoxycarbonyl, und Trichloräthoxycarbonyl. Beispiele von Hydroxyschutzgruppen sind Ätherschutzgruppen wie Tetrahydropyranyl, Allyl, 2-(Trimethylsilyl)äthoxymethyl, Trityl, tert-Butyldimethylsilyl oder Esterschutzgruppen wie Acetyl.

Die Abspaltung dieser Schutzgruppen erfolgt durch saure oder basische Hydrolyse, durch Hydrogenolyse, durch reduktive Methoden oder mittels Lewis-Säuren. Für die saure Hydrolyse verwendet man vorteilhafterweise eine Lösung einer Mineralsäure, wie Chlorwasserstoffsäure, Bromwasserstoffsäure, Schwefelsäure, Phosphorsäure und dergleichen, in einem inerten Lösungsmittel oder Lösungsmittelgemisch. Geeignete Lösungsmittel sind Alkohole, wie Methanol oder Äthanol, Äther, wie Tetrahydrofuran oder Dioxan, chlorierte Kohlenwasserstoffe, wie Methylenchlorid, und dergleichen. Für die basische Hydrolyse können Alkalimetallhydroxide und -carbonate, wie Kalium- oder Natriumhydroxid oder Kaliumoder Natriumcarbonat, organische Amine, wie Piperidin, und dergleichen verwendet werden. Inerte organische Lösungsmittel, wie sie oben für die saure Hydrolyse genannt sind, können als Lösungsvermittler eingesetzt werden. Die Reaktionstemperatur kann für die saure und basische Hydrolyse in einem Bereich von 0°C bis Rückflußtemperatur variiert werden, wobei man vorzugsweise zwischen etwa 0°C und der Raumtemperatur arbeitet. Der tert-Butoxycarbonylrest wird zweckmäßigerweise mit Salzsäure, mit Chlorwasserstoff, mit Trifluoressigsäure oder Ameisensäure in Gegenwart oder Abwesenheit eines inerten Lösungsmittels abgespalten. Der tert-Butoxycarbonylrest kann weiterhin mittels wasserfreiem Zinkbromid in Gegenwart eines inerten Lösungsmittels, vorzugsweise Methylenchlorid, abgespalten werden. Die Abspaltung der Trichloräthoxycarbonylgruppe kann vorteilhafterweise reduktiv mit Zink in Eisessig erfolgen. Die Reaktionstemperatur kann in einem Bereich von 0° C bis 40° C liegen, wobei man vorzugsweise bei Raumtemperatur arbeitet. Die Abspaltung des 2-(Trimethylsilyl)äthoxycarbonylrestes kann mittels Fluoridionen in Gegenwart eines inerten Lösungsmittels, wie Acetonitril, Dimethylsulfoxid, Dimethylformamid oder Tetrahydrofuran, vorzugsweise mittels Tetrabutylammoniumfluorid in Tetrahydrofuran, bei Temperaturen von etwa 0°C bis etwa Raumtemperatur erfolgen.

Die Verbindungen der Formel II sind neu und ebenfalls Gegenstand der Erfindung. Ihre Herstellung ist nachstehend in den Schemata 1 - 15 und in den Beispielen näher beschrieben.

**Schema 1**

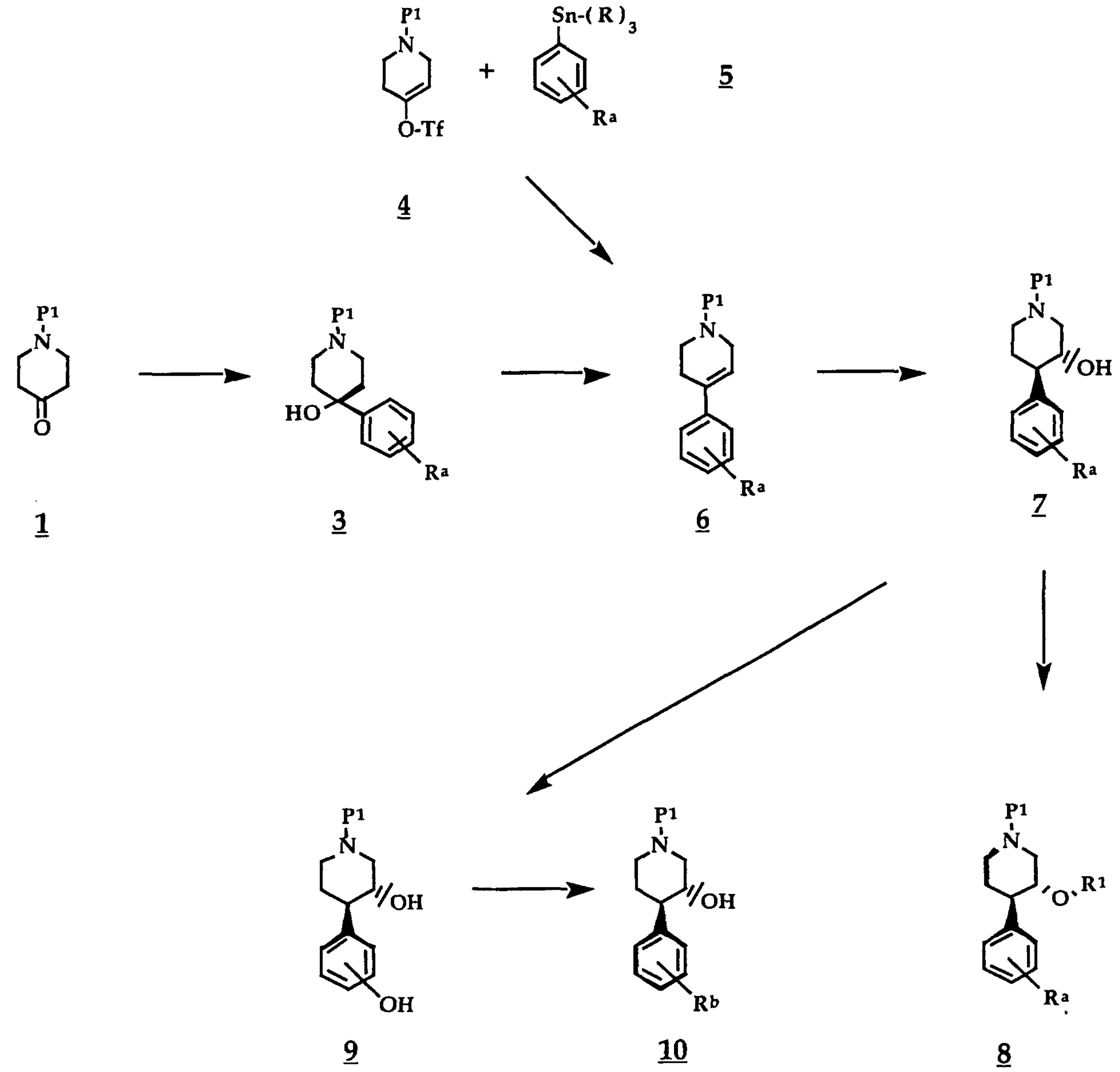

4 5

1 3 6 7

9 10 8

Gemäss Schema 1 können Verbindungen der allgemeinen Formel 3 erhalten werden, indem in an sich bekannter Weise Verbindungen der allgemeinen Formel 1 mit metallorganischen Derivaten, vorzugsweise Lithium- oder Magnesiumderivaten, hergestellt aus Verbindungen der allgemeinen Formel 2, umgesetzt werden,

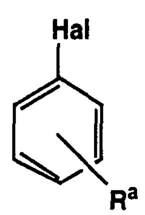

2

wobei $R^a$ Wasserstoff oder einen Substituenten darstellt, der unter den Reaktionsbedingungen inert ist oder in dem

reaktionsfähige Gruppen in geschützter Form vorliegen. Vorzugsweise geht man von Verbindungen 2 aus, in denen $R^a$ Halogen, $C_{1-6}$-Alkoxy oder Benzyloxy darstellt und benutzt diese Substituenten für den Aufbau eines anderen erwünschten Substituenten in einer geeigneten, späteren Stufe der Reaktionssequenz.

Die Umsetzung mit einer solchen metallorganischen Verbindung erfolgt nach an sich bekannten Methoden, beispielsweise in einem unter den Reaktionsbedingungen inerten Lösungsmittel, wie einem Äther, bei einer Temperatur zwischen etwa -78° C und 75° C. Daraus können in Gegenwart einer Säure oder anderer wasserabspaltender Reagenzien, gegebenenfalls in Anwesenheit einer Base, in einem organischen Lösungsmittel die Verbindungen der allgemeinen Formel 6 erhalten werden. Als Säuren kommen z.B. Salzsäure, Trifluoressig oder p-Toluolsulfonsäure zur Anwendung, als wasserabspaltendes Reagenz kann z.B. Phosphoroxytrichlorid in Pyridin eingesetzt werden. Die Reaktionstemperatur liegt zwischen 0 - 120° C, als Lösungsmittel können z.B. Toluol, Dimethylformamid oder Alkohole eingesetzt werden.

Ebenfalls zu Verbindungen der allgemeinen Formel 6 gelangt man direkt, ausgehend von einer Verbindung der allgemeinen Formel 4 , in der Tf eine aktivierende Gruppe, wie Trifluormethylsulfonyl (Triflat) ist, durch Umsetzung mit einer metallorganischen Verbindung, speziell einem Zinnderivat der allgemeinen Formel 5, worin R $C_{1-6}$-Alkyl, z. B. Butyl ist oder einem entsprechendem Arylborsäurederivat unter Verwendung eines geeigneten Katalysators wie z. B. tetrakis-Triphenylphosphinpalladium in einem inerten Lösungsmittel, wie Dioxan, Dimethoxyäthan oder Dimethylformamid, bei Temperaturen zwischen Raumtemperatur und 150 °C.

Verbindungen der allgemeinen Formel 7 können durch Hydroborierung und anschließende basische oxidative Aufarbeitung von Verbindungen der allgemeinen Formel 6 erhalten werden. Die Hydroborierung kann nach an sich bekannten Methoden erfolgen, beispielsweise in einem unter den Reaktionsbedingungen inerten Lösungsmittel, wie einem Äther, z.B. 1,2-Dimethoxyäthan, bei einer Temperatur zwischen etwa 0° C und 70° C, und mit einem Diboran enthaltenden oder freisetzenden Reagenz, wie z.B. Boran in Tetrahydrofuran oder eine Mischung von Natriumborhydrid und Bortrifluoridätherat. Die intermediär gebildeten Carboborane können mittels Umsetzung mit Basen, z.B. Kaliumhydroxid, und einem Oxidationsmittel, z.B. Wasserstoffperoxid, bei einer Temperatur zwischen etwa Raumtemperatur und 120° C, in die sekundären Alkohole der allgemeinen Formel 7 übergeführt werden.

Verbindungen der allgemeinen Formel 8 worin $R^a$ Halogen, Cyano, Trifluormethyl, $C_{1-6}$-Alkyl, $C_{1-6}$-Alkoxy-$C_{1-6}$-alkyl, $C_{1-6}$-Alkoxy oder $C_{1-6}$-Alkylendioxy ist, können aus 7 durch Alkylierung mit einer den Rest $R^1$ abgebenden Verbindung erhalten werden. Die Alkylierung des sekundären Alkohols erfolgt nach an sich bekannten Methoden, beispielsweise in einem unter den Reaktionsbedingungen inerten Lösungsmittel, wie einem Äther, z.B. Tetrahydrofuran oder 1,2-Dimethoxyäthan, oder Dimethylformamid, mit Hilfe einer alkoholatbildenden Base, z.B. Natriumhydrid, bei einer Temperatur zwischen etwa 0° C und 40° C und unter Verwendung eines Halogenids, vorzugsweise Chlorids oder Bromids, oder eines Sulfonsäureesters, z.B. eines Mesylats oder Tosylats als $R^1$ abgebende Verbindung. Verbindungen der allgemeinen Formel 7, worin $R^a$ $C_{1-6}$-Alkoxy ist, können durch eine Alkyl-Aryl-Ätherspaltung in Verbindungen der allgemeinen Formel 9 übergeführt werden. Die Ätherspaltung erfolgt nach an sich bekannten Methoden, indem vorzugsweise ausgehend von Verbindungen, worin $R^a$ die Bedeutung Methoxy hat, der Alkyl-Aryl-Äther mit Mineralsäuren, wie Bromwasserstoffsäure oder Jodwasserstoffsäure, oder vorzugsweise mit Lewis-Säuren, wie Bortrichlorid oder Bortribromid, in einem unter den Reaktionsbedingungen inerten Lösungsmittel, wie z.B. einem halogenierten Kohlenwasserstoff, bei einer Temperatur zwischen etwa -10° C und Raumtemperatur umgesetzt wird.

Verbindungen der allgemeinen Formel 9 können als Ausgangssubstanzen zur Herstellung von Verbindungen der allgemeinen Formel 10 dienen, worin $R^b$ ein Rest $-T^1- L^2 - T^2 - L^3- T^3- L^4- T^4-$ U, $T^1$ Sauerstoff, (m), (o) oder (p) ist und die weiteren Substituenten $L^{2,3,4}$, $T^{2,3,4}$ und U die eingangs erwähnten Bedeutungen haben können. Die Anknüpfung des Restes $-L^2- T^2 - L^3- T^3- L^4- T^4-$ U kann selektiv durch Umsetzung mit einem eine geeignete Abgangsgruppe tragenden Derivat des einzuführenden Restes erfolgen, wobei der gewünschte Rest auch stufenweise aufgebaut werden kann. Die selektive Verknüpfung mit dem phenolischen Alkohol erfolgt nach an sich bekannten Methoden der Alkylierung bzw. Acylierung in Gegenwart einer Base wie Kaliumcarbonat. Als Alkylierungsmittel kommen Chloride, Bromide, Jodide, Tosylate oder Mesylate in Frage. Die Reaktion erfolgt in einem unter den Reaktionsbedingungen inerten Lösungsmittel, wie z.B. einem Äther, wie Tetrahydrofuran, einem aromatischen Kohlenwasserstoff, wie z.B. Toluol, Pyridin, Aceton oder Methyläthylketon, bei einer Temperatur zwischen etwa 0° C und 100° C. Geeignete Acylierungsmittel sind aktivierte Derivate, wie gegebenenfalls aktivierte Ester, Säurehalogenide, Säureanhydride oder gemischte Säureanhydride. Die Reaktion erfolgt in einem unter den Reaktionsbedingungen inerten Lösungsmittel, wie z.B. einem Äther, wie Tetrahydrofuran, einem aromatischen Kohlenwasserstoff, wie z.B. Toluol, einem chlorierten Kohlenwasserstoff, wie Methylenchlorid oder Chloroform, und dergleichen bei einer Temperatur zwischen etwa 0° C und 50° C.

In dem einzuführenden Rest neben der Abgangsgruppe enthaltene(n) reaktive Gruppen, wie Keto- oder Hydroxygruppen, liegen dabei in geeigneter Weise geschützter Form vor, z.B. in Form von Acetalen, Estern, Carbamaten, Silylderivaten und dergleichen. Nach Abspaltung dieser Schutzgruppen kann ein stufenweiser Aufbau des Restes $-T^1- L^2- T^2- L^3-T^3- L^4- T^4-$ U fortgesetzt werden. Die im Piperidinring der so erhaltenen Verbindungen enthaltene Hydroxygruppe kann wie oben für die Umsetzung von 7 zu 8 beschrieben, alkyliert werden.

**Schema 2**

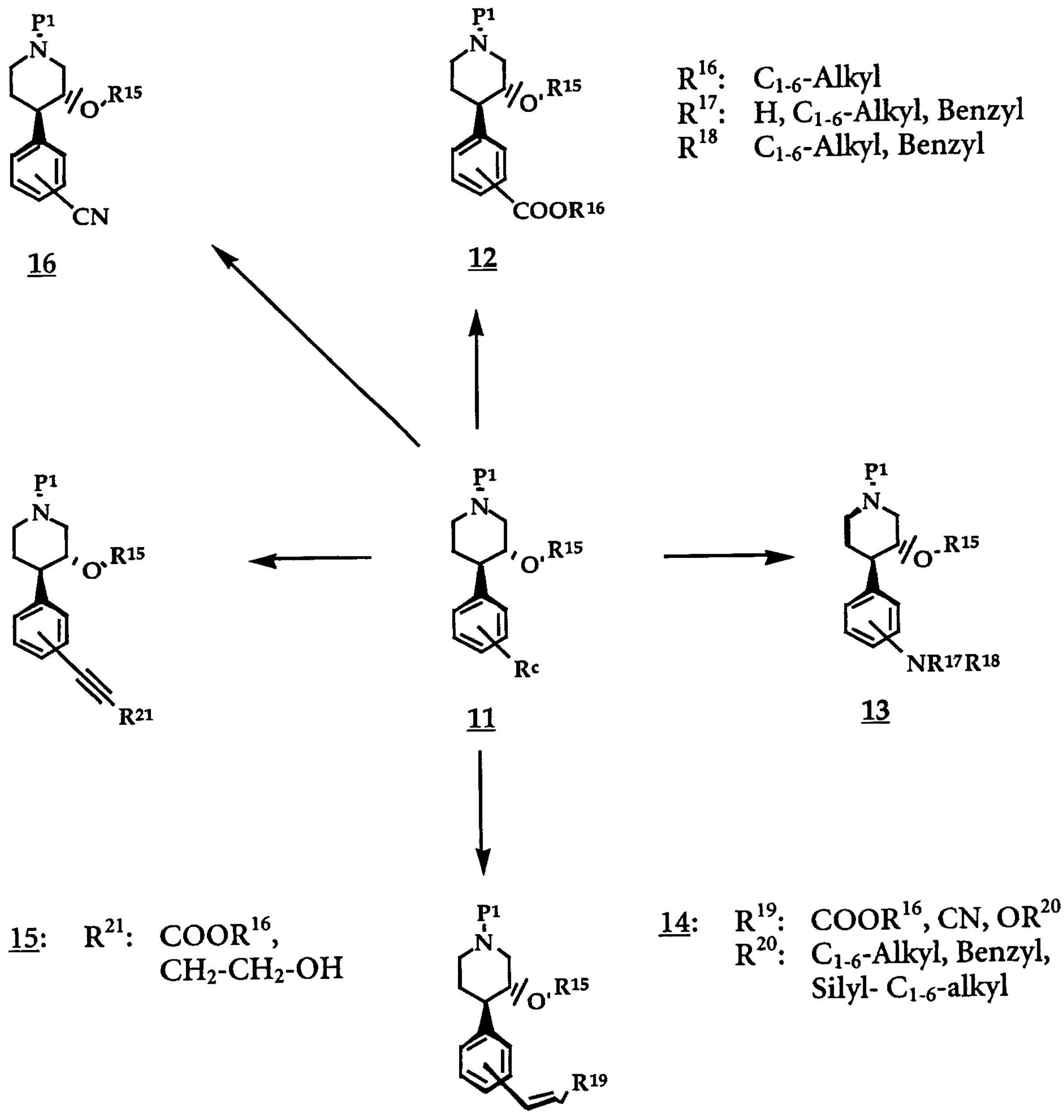


16 12 11 13 14

Gemäss Schema 2 können ausgehend von Verbindungen der allgemeinen Formel 11, worin $R^c$ Chlor, Brom, Jod sowie Hydroxy in Form eines aktivierten Derivates, wie z.B. des Triflates, und $R^{15}$ Wasserstoff oder ein unter den Reaktionsbedingungen inerter Rest $R^1$ ist, Verbindungen der Formeln 12-16 hergestellt werden, indem in an sich bekannter Weise Palladium-katalysierte Kopplungen mit Kohlenmonoxid, Cyaniden, Aminen oder Verbindungen der allgemeinen Formeln

$$H_2C{=}CH\text{-}R^{19}$$

oder

$$HC+C-R^{19}$$

wobei $R^{19}$ $-COOR^{16}$, -CN oder $-OR^{20}$ ist,
durchgeführt werden. Als Palladium-Katalysatoren können dabei in situ hergestellte Komplexe von z.B. $PdCl_2(CH_3CN)_2$ oder $Pd(OAc)_2$ mit 1,1'-Bis(diphenylphosphino)ferrocen, 1,3-Bis(diphenylphosphino)propan oder Tri(o-tolyl)phosphin verwendet werden. In den so erhaltenen Verbindungen können die Reste $-HC=CH-R^{19}$ oder $-C+C-R^{19}$ für einen weiteren stufenweisen Aufbau eines Substituenten $L^1$ - $T^1$- $L^2$ - $T^2$ - $L^3$- $T^3$- $L^4$- $T^4$- U derivatisiert werden. Eine C+C - Dreifachbindung kann in eine Doppelbindung, und letztere in eine Einfachbindung überführt werden. Eine Cyanogruppe kann in ein Amid, einen Aldehyd, eine Säure, einen Ester oder ein Amin übergeführt werden. Verbindungen der Formel 13 mit $R^{17,18}$ = Benzyl können debenzyliert und die dabei gebildete sek. bzw. prim. Aminogruppe kann ebenfalls für eine weitere Derivatisierung benutzt werden. Alle diese Umwandlungen oder Derivatisierungen, die nichtlimitierend in den Schemata 3-9 dargestellt sind, können nach an sich bekannten Methoden vorgenommen werden.

**Schema 3**

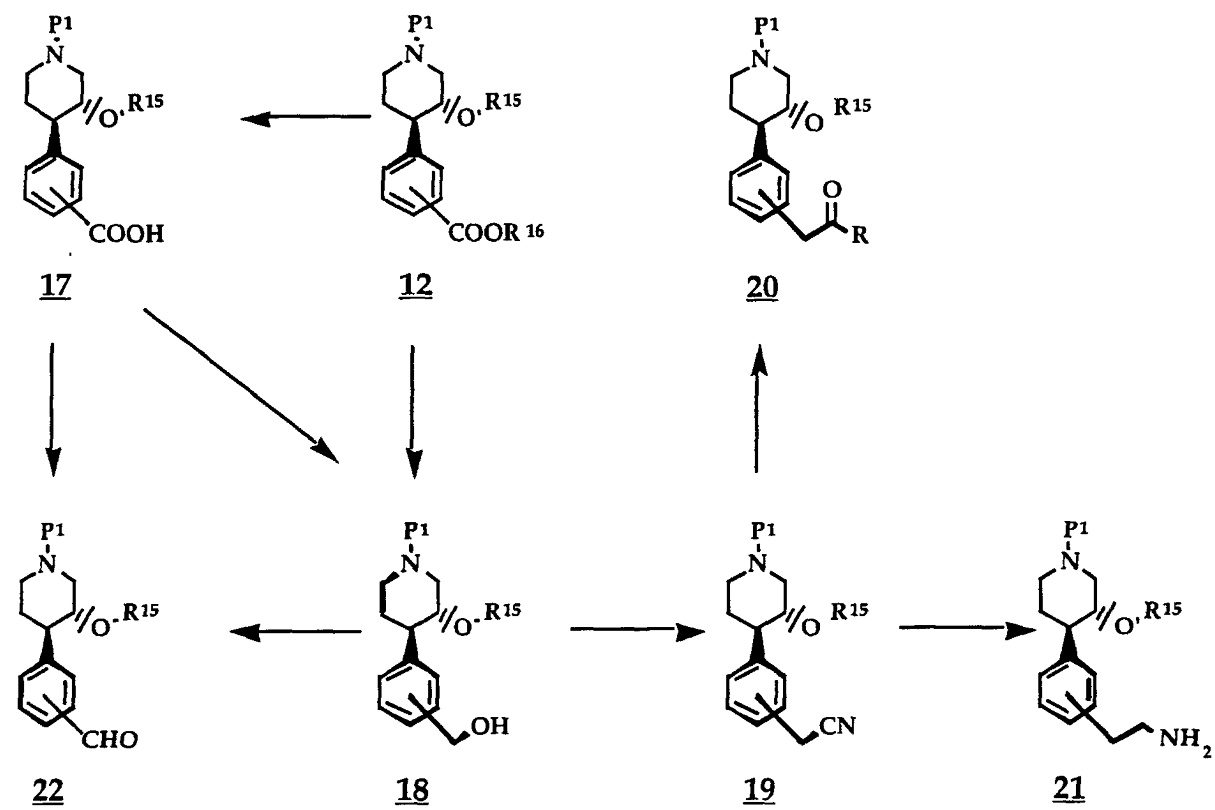

17 12 20

22 18 19 21

Gemäß Schema 3 können aus Verbindungen der allgemeinen Formel 12, erhalten durch Reaktion von Verbindungen der allgemeinen Formel 11 mit Kohlenmonoxid mittels Palladiumkatalyse, Verbindungen der allgemeinen Formeln 17-22 erhalten werden.

**Schema 4**

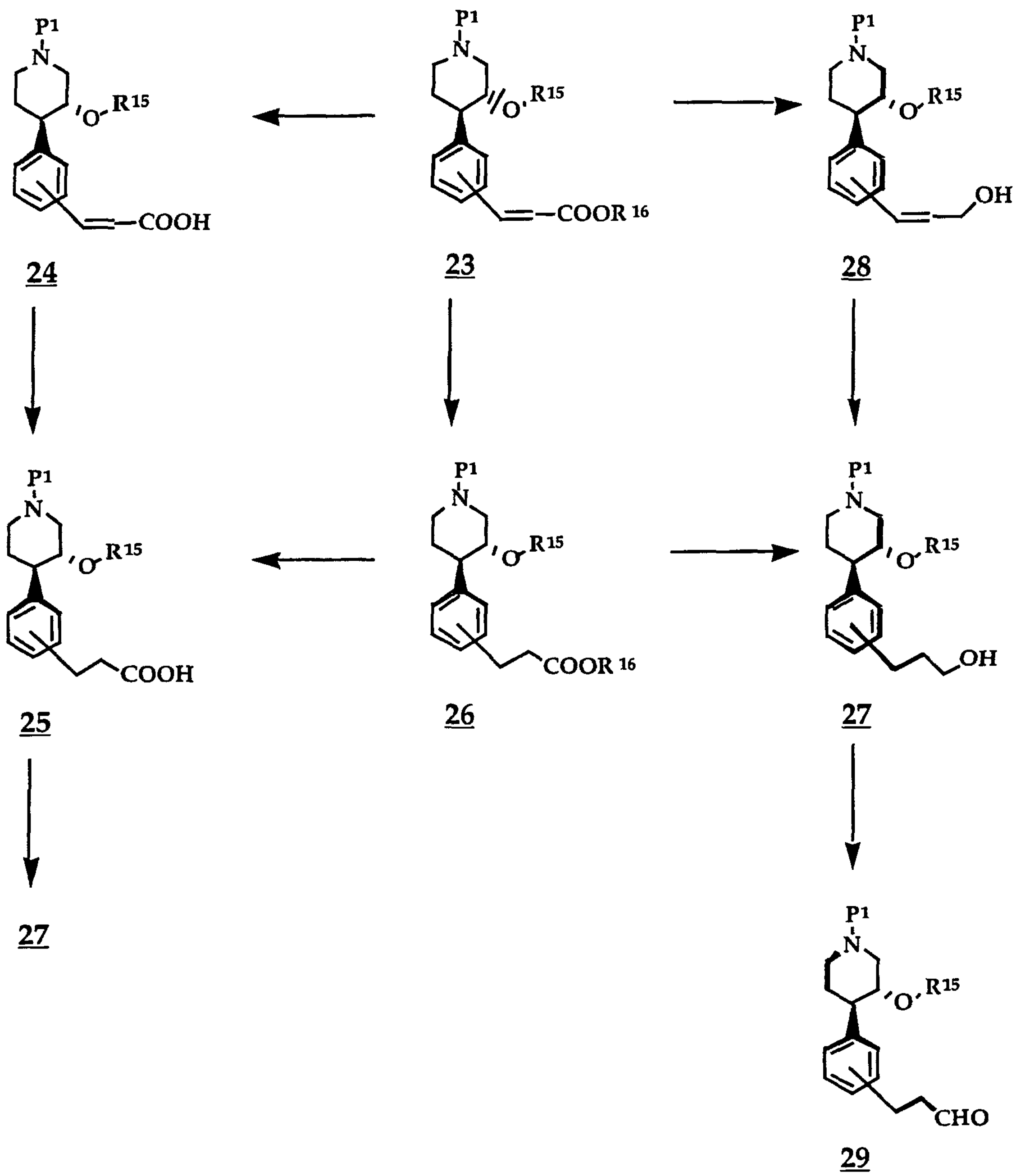

24 23 28

25 26 27

27

29

Gemäß Schema 4 können aus Verbindungen der allgemeinen Formel 23, erhalten durch Reaktion von Verbindungen der allgemeinen Formel 11 mit Acrylsäureestern mittels Palladiumkatalyse, Verbindungen der allgemeinen Formeln 24-29 erhalten werden.

**Schema 5**

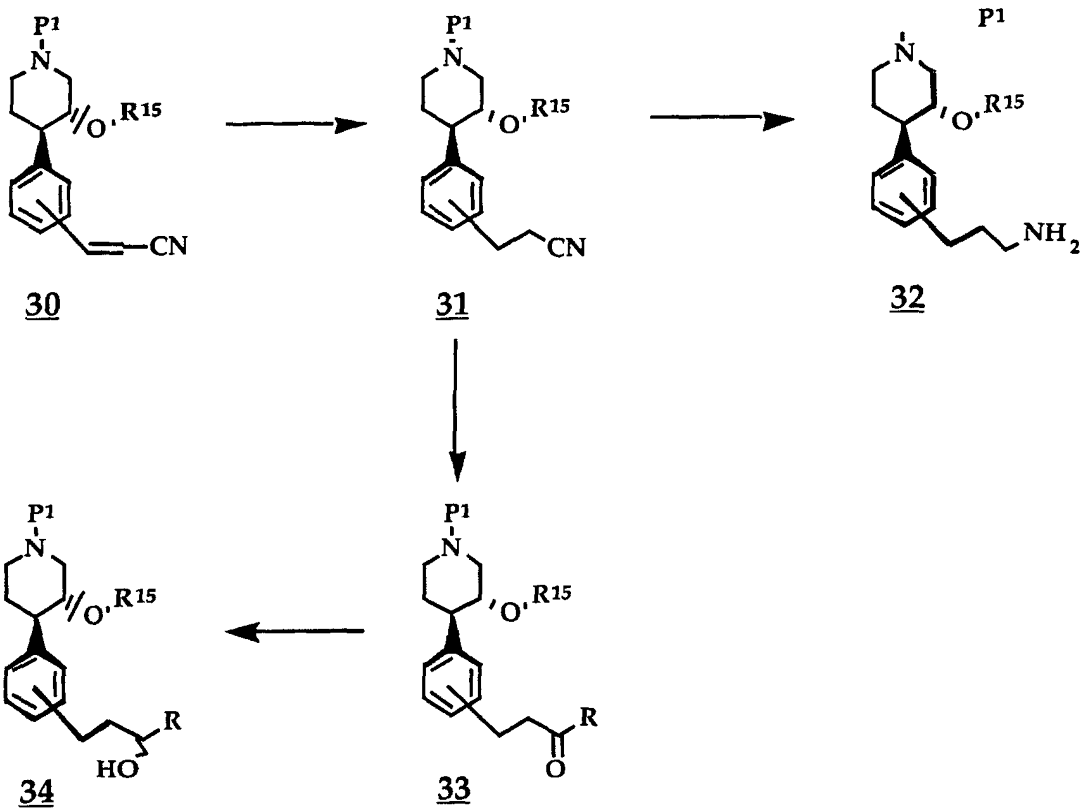

30 31 32

34 33

Gemäß Schema 5 können aus Verbindungen der allgemeinen Formel 30, erhalten durch Reaktion von Verbindungen der allgemeinen Formel 11 mit Acrylnitril mittels Palladiumkatalyse, Verbindungen der allgemeinen Formeln 31-34 erhalten werden.

**Schema 6**

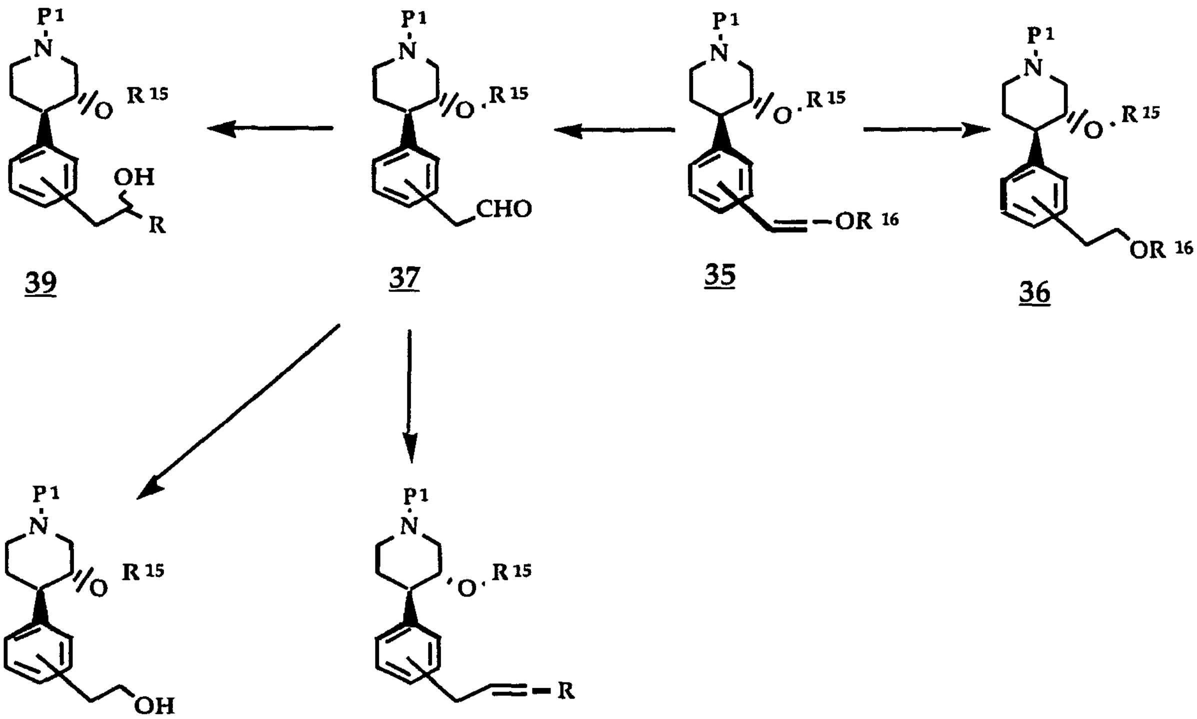

39 37 35 36

40 38

Gemäß Schema 6 können aus Verbindungen der allgemeinen Formel 35, erhalten durch Reaktion von Verbindungen der allgemeinen Formel 11 mit Vinyläthern mittels Palladiumkatalyse, Verbindungen der allgemeinen Formeln 36-40 erhalten werden.

Piperidone der allgemeinen Formel 41 können in analoger Weise wie für das Piperidon 1 beschrieben, als Ausgangsstoffe zur Synthese der Piperdinderivate I Verwendung finden. Sie können analog dem von A.H.Beckett et al. im Journal of Medicinal and Pharmaceutical Chemistry Vol 1(1), 37-58 (1959) beschriebenen Verfahren hergestellt werden. Die Piperidone der allgemeinen Formel 41 können durch intramolekularen Ringschluß eines Propionsäurederivates der

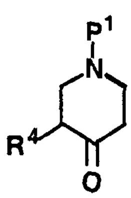 41

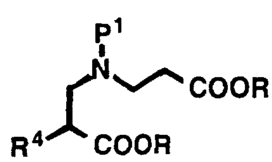 42

allgemeinen Formel 42 hergestellt werden. Dabei ist $R^4$ wie eingangs definiert oder stellt einen Substituenten dar, der unter den Reaktionsbedingungen inert ist oder in dem reaktionsfähige Gruppen in entsprechend geschützter Form vorliegen. Vorzugsweise wird $R^4$ so gewählt, daß gegebenfalls ein Aufbau eines anderen erwünschten Substituenten in einer geeigneten späteren Stufe der Reaktionssequenz möglich ist. $P^1$ hat die Bedeutung von Methyl oder Benzyl. Dieser Ringschluss erfolgt in Gegenwart einer Base, wie z.B. Natriumalkoholat, Natriumhydrid oder Natriumdispersion in Xylol. Die anschließende Decarbalkoxylierung einer so erhaltenen Verbindung der allgemeinen Formel 43 oder 44

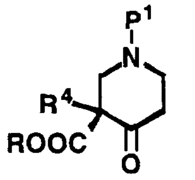
43

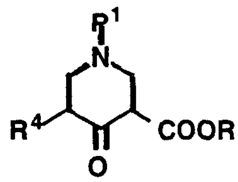
44

mittels Salzsäure führt zu Verbindungen der allgemeinen Formel 41.

Derivate der allgemeinen Formel 43, in denen $R^4$ Allyl oder Benzyl bedeutet, können auch auf direktem Wege durch C-Alkylierung des Natriumsalzes einer Verbindung der allgemeinen Formel 45

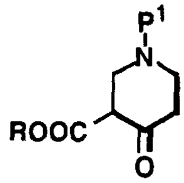
45

mittels Allyldimethylaniliniumbromid bzw. Benzyldimethylaniliniumbromid, analog dem von A.H.Beckett et al. (siehe oben) beschriebenen Verfahren, hergestellt werden.

Derivate der allgemeinen Formel 41, in denen $R^4$ an der Hydroxyfunktion geeignet geschütztes Hydroxymethyl bedeutet, können ebenso aus der Verbindung der allgemeinen Formel 45 durch Reduktion zum Diol, analog dem von E.Jaeger und J.H.Biel in J.Org.Chem. 30(3), 740-744 (1965) beschriebenen Verfahren, Einführung einer geeigneten Schutzgruppe für den primären Alkohol, z.B. Trityl, und Oxidation des sekundären Alkohols erhalten werden.

Weiterhin können Derivate der allgemeinen Formel 46 durch Hydroxymethylierung von Verbindungen der allgemeinen Formel 6 analog dem von K.Willcocks et al. im Journal of Labelled Compounds and Radiopharmaceuticals Vol. XXXIII, No.8, 783-794 (1993) beschriebenen Verfahren hergestellt werden.

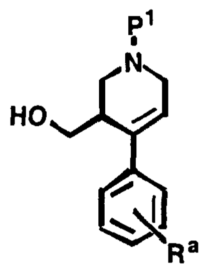
46

Verbindungen der allgemeinen Formel II, worin $R^3$ die Bedeutung Wasserstoff und W die Bedeutung Sauerstoff oder Schwefel haben, können ausgehend von Verbindungen der allgemeinen Formel 47

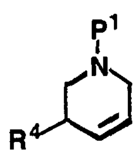
47

durch Epoxidierung erhalten werden. Verbindungen der allgemeinen Formel 47 können nach dem von M.Ferles und M.Jankovsky in Coll.Czechoslov.Chem.Commun. Vol, 35, 2802-2809 (1970) beschriebenen Verfahren hergestellt werden. Die Epoxide können dann durch Umsetzung mit geeigneten Thiophenolaten und Phenolaten, wie von R. Paioni in der Deutschen Offenlegungsschrift 2738477 beschrieben, geöffnet werden. Der weitere Aufbau zu Verbindungen der Formel II kann wie bereits vorgehend beschrieben erfolgen.

**Schema 7**

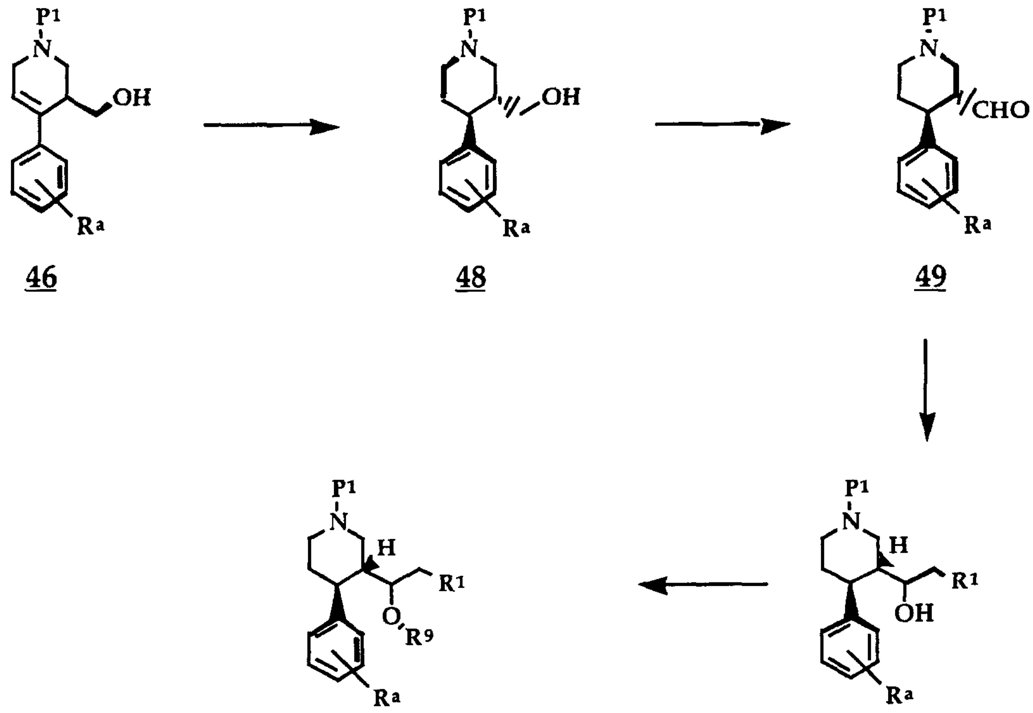

46 48 49

51 50

Gemäss Schema 7 können Verbindungen der allgemeinen Formel 51 aus Verbindungen der allgemeinen Formel 46 dadurch erhalten werden, daß zunächst die Doppelbindung unter Verwendung von komplexen Hydriden wie Lithiumaluminiumhydrid, wie von J.M. Lundbeck et al. in der Europäischen Patentanmeldung E.P. 0 374 674 beschrieben, oder Natrium dihydrido-bis-(2-methoxyäthoxy)aluminat in einem unter den Reaktionsbedingungen inerten Lösungsmittel, wie Tetrahydrofüran, Dioxan oder Toluol, bei Temperaturen zwischen Raumtemperatur und 110 °C reduziert oder unter Verwendung eines Katalysators mit Wasserstoff hydriert wird, wobei Verbindungen der allgemeinen Formel 48 erhalten werden. Verbindungen der allgemeinen Formel 48 lassen sich durch übliche Oxidationsverfahren, z.B. unter Verwendung von Oxalylchlorid und Dimethylsulfoxid wie von A.J. Mancuso und D. Swern in Synthesis 1981, 165 beschrieben, in die entsprechenden Aldehyde der allgemeinen Formel 49 überführen.

Die Kondensation der Aldehyde der allgemeinen Formel 49 mit Grignard- oder Lithiumverbindungen in einem unter den Reaktionsbedingungen inerten Lösungsmitteln wie Äther, Tetrahydrofuran oder Dioxan bei Temperaturen zwischen - 80 °C und Raumtemperatur führt zu Verbindungen der allgemeinen Formel 50, welche nach bekannten Verfahren in die entsprechenden Ester- oder Ätherverbindungen der allgemeinen Formel 51 übergeführt werden können.

**Schema 8**

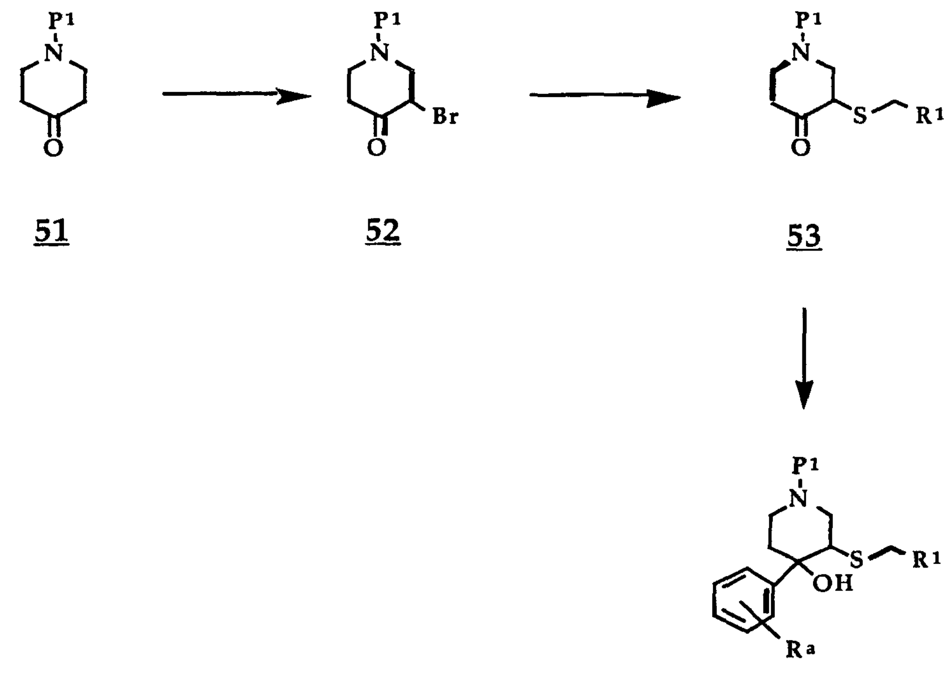

51 52 53

54

Gemäss Schema 8 können Verbindungen der allgemeinen Formel 54 dadurch erhalten werden, daß durch Bromierung von Verbindungen der allgemeinen Formel 1 in einem unter den Reaktionsbedingungen inerten Lösungsmittel, wie Chloroform oder Methylenchlorid, und unter Verwendung eines Puffersalzes wie Dinatriumhydrogenphosphat bei Temperaturen zwischen 0 °C und 50 °C zunächst Verbindungen der allgemeinen Formel 52 gebildet werden, deren Umsetzung mit einem Alkalimetallsalz eines Thiols in einem Lösungsmittel wie Aceton, Acetonitril oder Dimethylformamid bei Temperaturen zwischen Raumtemperatur und 100 °C Verbindungen der allgemeinen Formel 53 ergibt. Deren Kondensation mit Grignard- oder Lithiumverbindungen in inerten Lösungsmitteln wie Äther, Tetrahydrofuran oder Dioxan bei Temperaturen zwischen - 80 °C und Raumtemperatur liefert dann Verbindungen der allgemeinen Formel 54.

**Schema 9**

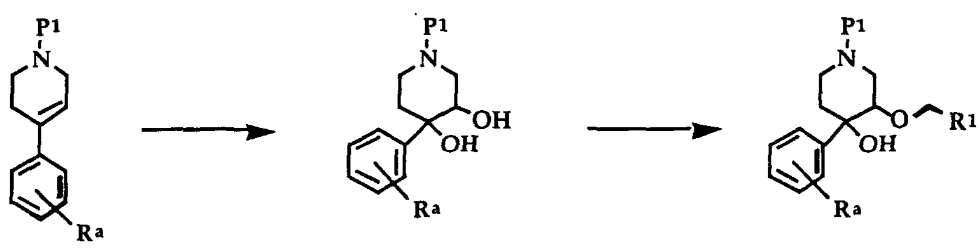

6 55 56

Gemäß Schema 9 können Verbindungen der allgemeinen Formel 56 erhalten werden, indem Verbindungen der allgemeinen Formel 6 zu Verbindungen der allgemeinen Formel 55 dihydroxyliert werden. Deren Alkylierung liefert,

nach bereits vorgehend beschriebenen Verfahren, Verbindungen der allgemeinen Formel 56. Die Dihydroxylierung kann nach an sich bekannten Verfahren erfolgen, beispielsweise in einem unter den Reaktionsbedingungen inerten Lösungsmittel, wie Aceton oder tert-Butanol, bei einer Temperatur zwischen 0 °C und 50 °C, vorzugsweise bei Raumtemperatur, mit einem hydroxylierenden Reagenz, wie z.B. einem Gemisch von Osmiumtetroxid und Wasserstoffperoxid.

Die gemäss Schema 2-9 hergestellten Verbindungen können wiederum als Ausgangsmaterial für einen weiteren Aufbau eines in $R^2$ gegebenenfalls anwesenden Substituenten

$-L^1 - T^1 - L^2 - T^2 - L^3 - T^3 - L^4 - T^4 - U$

wie weiter oben beschrieben verwendet werden.

Weiterhin können Verbindungen der allgemeinen Formel II mit anwesender Äthylenbrücke Q gemäß den in den Schemata 1 - 9 sowie den vorstehend und in den Beispielen näher beschriebenen Verfahren hergestellt werden.

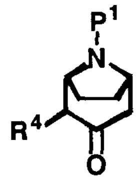

57

Die als Ausgangsstoffe verwendbaren Tropinone der allgemeinen Formel 57 können u.a. nach den von M. Lounasmaa und C.J.Johansson in Tetrahedron Letters, No.29, 2509 (1974) oder Ö.Kovács et al. in Helv.Chim.Acta Vol. XXXVII, 802 (1954) beschriebenen Verfahren hergestellt werden.

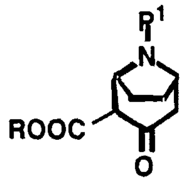

58

Derivate der allgemeinen Formel 57 können aus der Verbindung der allgemeinen Formel 58. analog dem von Ö.Kovács et al. in Helv.Chim.Acta Vol. XXXVII, 802 (1954) beschriebenen Verfahren, durch Reduktion zum Diol, Einführung einer geeigneten Schutzgruppe für den primären Alkohol, z.B. Trityl, und Oxidation des sekundären Alkohols erhalten werden. Dabei ist $R^4$ wie eingangs definiert oder stellt einen Substituenten dar, der unter den Reaktionsbedingungen inert ist oder in dem reaktionsfähige Gruppen in entsprechend geschützter Form vorliegen, vorzugsweise so gewählt, daß gegebenfalls ein Aufbau eines anderen erwünschten Substituenten in einer geeigneten späteren Stufe der Reaktionssequenz möglich ist.

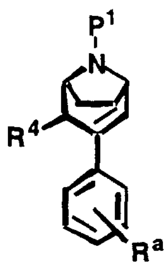

59

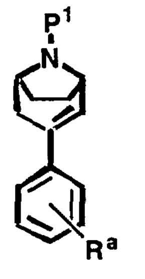

60

Weiterhin können Derivate der allgemeinen Formel 59, in denen $R^4$ Hydroxymethyl bedeutet, durch Hydroxymethylierung von Verbindungen der allgemeinen Formel 60 analog dem von K.Willcocks et al. in Journal of Labelled Compounds and Radiopharmaceuticals Vol. XXXIII, No.8, 783-794 (1993) beschriebenen Verfahren hergestellt werden.

Des weiteren besteht die Möglichkeit, Derivate der allgemeinen Formel 57 dadurch herzustellen, daß ausgehend von entsprechend substituierten Acetondicarbonsäurederivaten durch Umsetzung mit Succindialdehyd und einem Amin, analog zu literaturbekannten Verfahren, das entsprechende Tropinonderivat aufgebaut wird. Die als Ausgangsstoffe eingesetzten substituierten Acetondicarbonsäurederivate können z.B. nach den von I.Ito und S.I.Nagai in Chem.Pharm.Bull. 22(9) 2131 (1974) oder T.Arslan und S.A.Benner in J.Org.Chem. 58, 2260 (1993) beschriebenen Verfahren hergestellt werden.

**Schema 10**

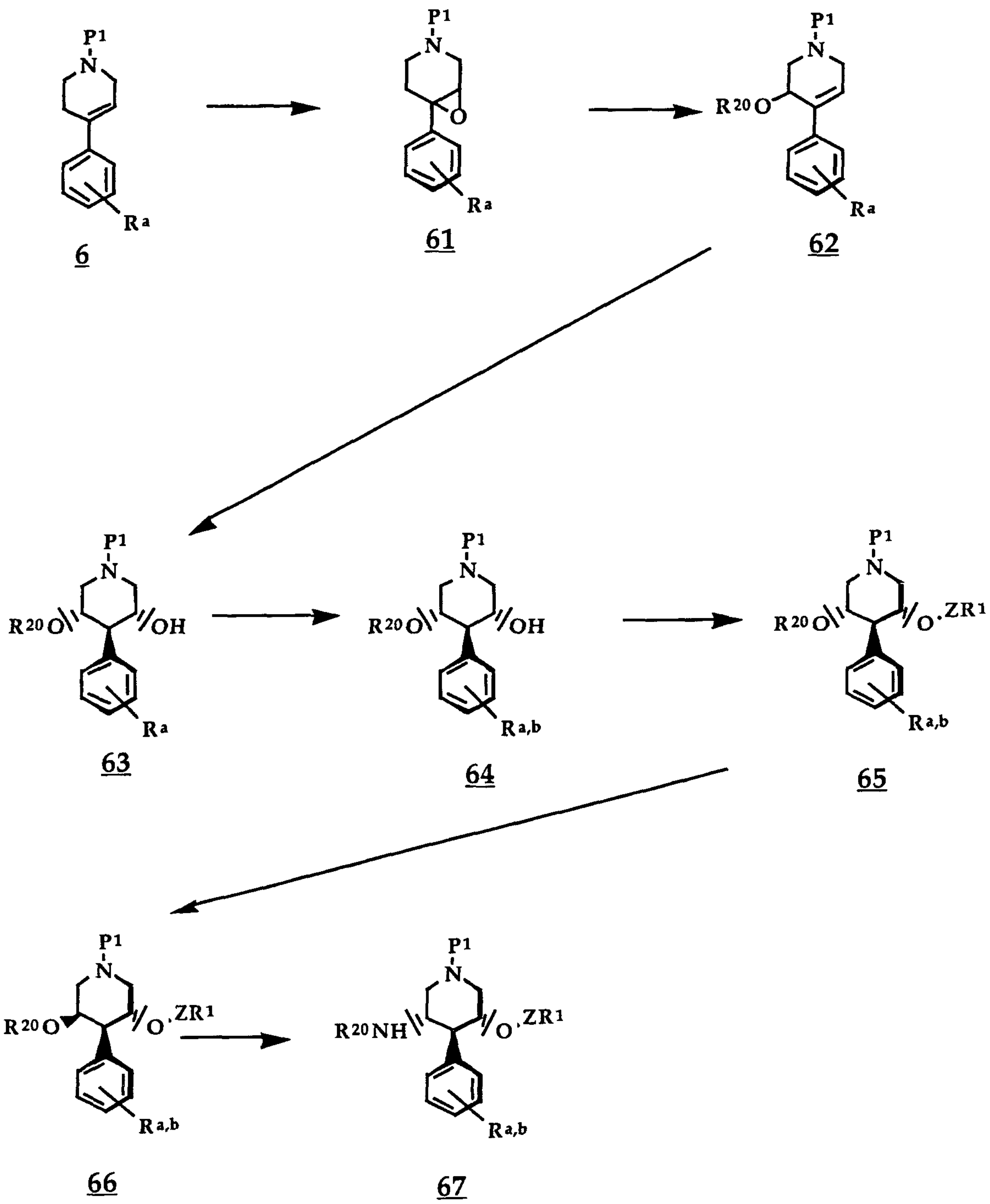

6 61 62

63 64 65

66 67

Ein möglicher synthetischer Aufbau von 4-Aryl-Piperidinen, die in Stellung 3 und 5 je mit O, resp. N Atomen substituiert sind, ist in Schema 10 dargestellt. Oxiran-Verbindungen der allgemeinen Formel 61 können aus den Olefinen 6 zum

Beispiel über ein intermediär gebildetes Bromhydrin gebildet werden, welches durch Anlagerung von Brom in wässrigem Dioxan erhalten werden kann. Das Bromhydrin kann dann anschliessend durch Zugabe von wässeriger Natronlauge zum Epoxid 61 geschlossen werden. Die Behandlung eines solchen Oxiranes mit Methyllithium, Butyllithium oder einem Lithiumamid in aprotischen Lösungmitteln wie Äther oder Tetrahydrofuran bei Temperaturen zwischen -80 °C und + 60 °C führt zu Allylalkoholen 62, die mit freiem OH oder nach Einführung einer Ätherfunktion $R^{20}$ weiter verarbeitet werden können. Die Hydroborierung dieser Allylalkoholderivate 62, wie schon für die Herstellung von Verbindungen der allgemeinen Formel 7 beschrieben, liefert die freien oder monofunktionalisierten Dihydroxyderivate 63. Zur strukturellen Variation der Reste $R^{20}$ ($R^{4a}Z^1$- oder H), -$ZR^1$ und $R^a$ können nun je nach angestrebtem Zielmolekül in unterschiedlicher Reihenfolge Reste $R^{20}$ und -$ZR^1$ eingeführt und Rest $R^a$ zu $R^b$, einem Rest der allgemeinen Formel -$T^1$- $L^2$ - $T^2$ - $L^3$ - $T^3$ - $L^4$ - $T^4$ - U, modifiziert werden. Je nach Aufbauschema kann es sinnvoll sein, eine der beiden OH Funktionen intermediär mit einer Schutzgruppe zu versehen und diese im späteren Verlauf des Aufbaues wieder abzuspalten oder die Reste -$ZR^1$ und $R^{20}$ so zu wählen, dass gegebenenfalls ein Aufbau eines anderen erwünschten Substituenten in einer geeigneten späteren Stufe der Reaktionssequenz möglich ist. Ausgehend von monoäthergeschützten Derivaten 65 kann die freie OH Funktion beispielsweise durch Reaktion mit Ameisensäure, Triphenylphosphin und einem Azodicarbonsäureester in einem inerten Lösungsmittel wie Tetrahydrofuran nach Mitsunobu [Synthesis 1981, 1] stereochemisch invertiert werden, wobei Verbindungen der allgemeinen Formel 66 erhalten werden. Die Verwendung von Diphenylphosphorylazid an Stelle von Ameisensäure unter ähnlichen Bedingungen stellt eine Möglichkeit dar, ausgehend von Verbindungen 66 durch eine erneute Inversion am gleichen Zentrum eine Azidofünktion einzuführen, die beispielsweise durch Reduktion mit Triphenylphosphin/Wasser in Tetrahydrofuran bei Temperaturen zwischen Raumtemperatur und 80° C [Synth. Commun. 17, 377 (1987)] in eine primäre Aminofunktion umgewandelt werden kann. Damit ergeben sich Verbindungen der allgemeinen Formel 67, die anschliessend alkyliert oder acyliert werden können.

Schema 11

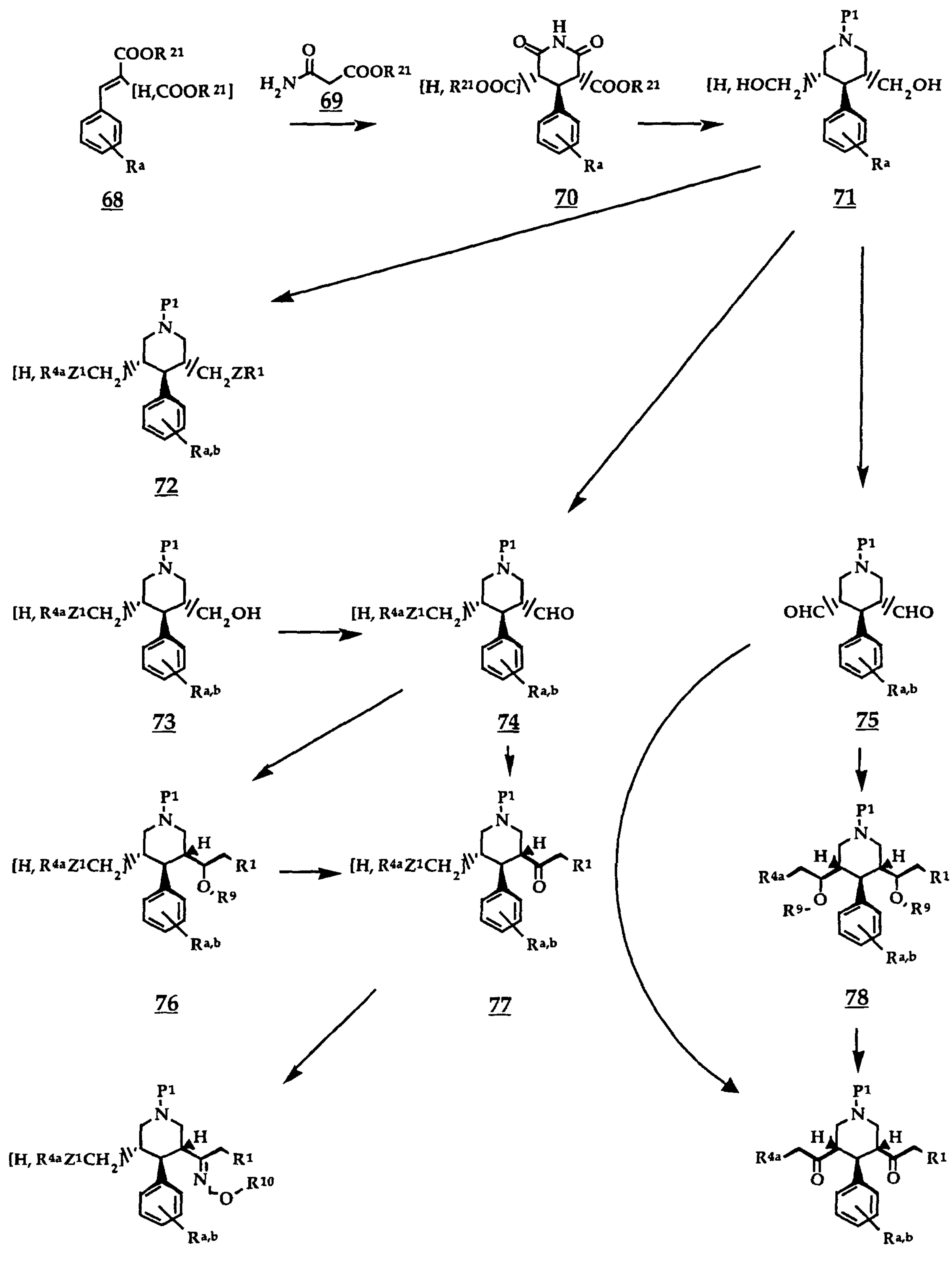

68, 69, 70, 71, 72, 73, 74, 75, 76, 77, 78, 79, 80

Gemäss Schema 11 können literaturbekannte Zimtsäurederivate 68 mit Malonsäure-monoester-monoamiden 69 in protischen Lösungsmitteln wie Äthanol oder Methanol oder aprotischen Lösungsmitteln wie N,N-Dimethylformamid, Dimethylsulfoxid, Tetrahydrofuran oder Acetonitril unter Verwendung von Basen wie zum Beispiel Kalium-tert-butylat oder Natriumhydrid bei Temperaturen zwischen Raumtemperatur und 130 °C zu cyclischen Imiden 70 umgesetzt werden. Reduktion dieser Imide 70 mit Hydrid-Reduktionsreagentien wie Lithiumaluminiumhydrid, Diisobutylaluminiumhydrid oder Natrium dihydrido-bis-(2-methoxyäthoxy)aluminat in aprotischen Lösungsmitteln wie Äther, Tetrahydrofuran oder Dioxan bei Temperaturen zwischen Raumtemperatur und 120 °C liefert die Piperidin Mono- und Dimethanole 71, die am Piperidinstickstoff mit einer geeigneten Schutzgruppe versehen werden können. Mono- und Dihydroxyverbindungen der allgemeinen Formel 71 können nun, in analoger Weise wie schon für Mono- und Dihydroxy Verbindungen 7, 10, 62, 63, 64 beschrieben oder durch Umwandlung in die entsprechenden Brom, Chlor, Iod-Verbindungen, Aryl- oder Alkylsulfonsäureester und anschließende nukleophile Substitution mit Alkoholaten, Phenolaten oder Thiophenolaten nach gängiger Methodik stufenweise selektiv fünktionalisiert werden. Dabei können $ZR^1$, $Z^1R^{4a}$ Substituenten darstellen, wie eingangs in der allgemeinen Formel I definiert oder geeignete Vorstufen, die unter den Reaktionsbedingungen inert sind oder in welchen reaktionsfähige Gruppen in entsprechend geschützter Form vorliegen, vorzugsweise so gewählt, daß gegebenenfalls ein Aufbau eines anderen erwünschten Substituenten in einer geeigneten späteren Stufe der Reaktionssequenz möglich ist. $R^a$ und $R^b$ entsprechen den oben aufgeführten Definitionen. Andererseits können Monohydroxyverbindungen 71, Dihydroxyverbindungen 71 oder Derivate 73 der Dihydroxyverbindungen 71, deren eine Hydroxy-Funktion strukturell modifiziert worden ist, durch Oxidation beispielsweise nach Swern (Dimethylsulfoxid, Oxalylchorid) [J. Org. Chem. 43, 2480 (1978) ] in die Aldehyde 74 überführt werden. Anlagerung einer Grignard oder Lithiumverbindungen nach bekannter Methodologie in einem inerten Lösungsmittel wie Tetrahydrofuran oder 1,2-Dimethoxyäthan bei Temperaturen zwischen -78 °C und Raumtemperatur liefert dann die Alkohole 76 ($R^9$ = H), die gegebenenfalls alkyliert, acyliert oder erneut zum Beispiel nach Swern oxidiert werden können und somit Derivate 76 ($R^9$, ungleich H), respektive Ketone 77 ergeben. Ausgehend von Dihydroxyverbindungen 71 kann durch gleichzeitige Umwandlung beider Hydroxyfunktionen analog wie oben beschrieben via Dialdehyde 75 zu Verbindungen der allgemeinen Formeln 78 und 80 gelangen. Die Transformation der Aldehyde 74, 75 in Ketone 77, 80 kann auch über eine Oxidation zur Säure [beispielsweise mit Natriumchlorit, Amidosulfonsäure und Isopropenylacetat in einem Lösungsmittel wie Aceton/Wasser bei 0 °C bis Raumtemperatur nach J. Am. Chem Soc. 110, 2242 (1988)], anschliessende Amidkupplung zu N-Methyl-N-methoxy Amiden mit N,O-Dimethylhydroxylamin nach bekannten Methoden sowie deren Umsetzung mit Organolithium oder Organomagnesium Verbindungen in einem inerten Lösungsmittel wie Tetrahydrofuran oder 1,2-Dimethoxyäthan bei Temperaturen zwischen -78 °C und Raumtemperatur wie beispielsweise in [Synthesis 1986, 944] beschrieben, erfolgen. Ketone 77 können mit gegebenenfalls substituierten Hydroxylaminderivaten durch Umsatz in einem Lösungsmittel wie Pyridin in Gegenwart von katalytischen oder stöchiometrischen Mengen einer starken Säure bei Temperaturen zwischen Raumtemperatur und 120 °C in die Oxime 79 überführt werden, wobei $R^{10}$ die in der allgemeinen Formel I definierte Bedeutung haben kann.

**Schema 12**

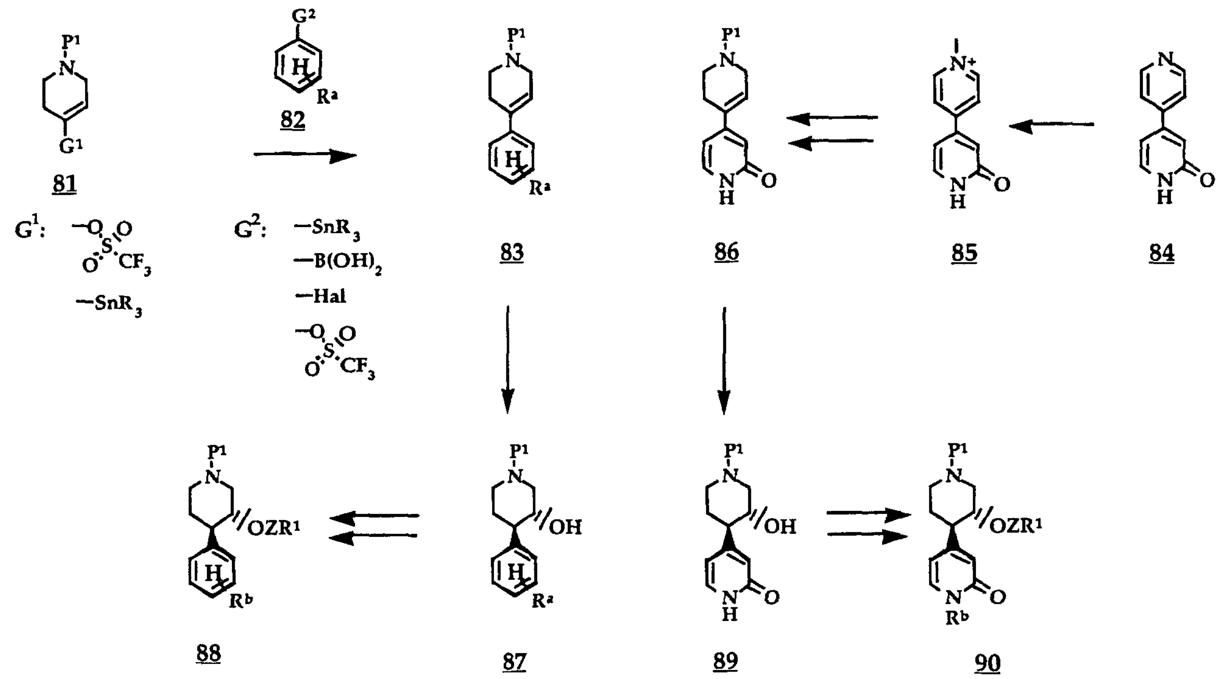

81 82 83 86 85 84

88 87 89 90

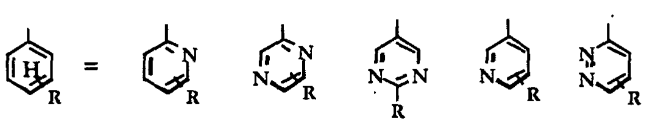

5

Gemäss Schema 12 können Verbindungen der allgemeinen Formeln 88 und 90, die heterocyclische Substituenten in 4 Stellung des Piperidin-Ringes enthalten, stufenweise z.B. wie im Folgenden beschrieben aufgebaut werden:

4-Heteroaryl-1,2,3,6-tetrahydro-pyridin Derivate 83 lassen sich beispielsweise aus in der Form eines Enol-Triflates aktivierten 1,2,3,6-Tetrahydro-pyridin Derivaten 81 durch Kondensation mit geeignet funktionalisierten, beispielsweise in Form von Zinnverbindungen aktivierten heteroaromatischen Verbindungen 82 erhalten. Kopplungsreaktionen dieses Typs werden vorzugsweise in einem inerten Lösungsmittel wie 1,2-Dimethoxyäthan, Tetrahydrofuran oder N,N-Dimethylformamid unter Verwendung eines Katalysators wie Tetrakis-(triphenylphosphin)-palladium bei Temperaturen bis zu 130 °C durchgeführt. An Stelle der Zinnverbindungen können auch analoge Borsäurederivate 82 unter vergleichbaren Reaktionsbedingungen umgesetzt werden oder eine zum Enol-Triflat 81 analoge Vinyl-Zinnverbindung 81 mit heterocyclischen Halogenverbindungen oder Triflaten 82 ebenso unter vergleichbaren Bedingungen zur Reaktion gebracht werden; in beiden Fällen führen die Reaktionen zu denselben Produkten. 4-Heteroaryl-1,2,3,6-tetrahydro-pyridin Derivate 86 können aber auch aus Pyridyl-heteroaryl-biaryl Derivaten wie 84 nach bekannten Methoden: N-Methylierung, Teilhydrierung des methylierten Pyridinringes mit einem geeigneten Hydridreagenz wie Natriumborhydrid und anschliessende Umwandlung der N-Methyl Funktion in eine geeignete Schutzgruppe durch demethylierende Carbamoylierung [wie zum Beispiel in J. Org. Chem. 49, 2081 (1984) beschrieben] erhalten werden.

Verbindungen der allgemeinen Formel 87 und 89 können durch Hydroborierung und anschliessende basische

oxidative Aufarbeitung von Verbindungen der allgemeinen Formel 83 und 86 erhalten werden. Die Hydroborierung kann nach an sich bekannten Methoden erfolgen, beispielsweise in einem unter den Reaktionsbedingungen inerten Lösungsmittel, wie einem Äther, z.B. 1,2-Dimethoxyäthan, bei einer Temperatur zwischen etwa 0 °C und 70 °C, und mit einem Diboran enthaltenden oder freisetzenden Reagenz, wie z.B. Boran in Tetrahydrofuran, Boran-Dimethylsulfid oder einer Mischung von Natriumborhydrid und Bortrifluoridätherat. Die intermediär gebildeten Carboborane können durch Umsetzung mit Basen, z.B. Kaliumhydroxid, und einem Oxidationsmittel, z.B. Wasserstoffperoxid, Natriumperborat oder mit Natriumpercarbonat, einer Kombination von Base und Oxidationsmittel oder mit Trimethylamin-N-oxid ohne Basenzusatz, bei einer Temperatur zwischen etwa Raumtemperatur und 120 °C, in die sekundären Alkohole der allgemeinen Formel 87 und 89 übergeführt werden.

Verbindungen der allgemeinen Formel 88 und 90, worin $-ZR^1$ ein eine Aryl oder eine Heteroarylfünktion enthaltender Substituent darstellt, können aus Verbindungen der allgemeinen Formeln 87 und 89 durch Alkylierung mit einer den Rest $-ZR^1$ abgebenden Verbindung erhalten werden. Die Alkylierung des sekundären Alkohols erfolgt nach an sich bekannten Methoden, beispielsweise in einem unter den Reaktionsbedingungen inerten Lösungsmittel, wie einem Äther, z.B. Tetrahydrofüran oder 1,2-Dimethoxyäthan, oder Dimethylformamid, mit Hilfe einer alkoholatbildenden Base, z.B. Natriumhydrid, bei einer Temperatur zwischen etwa 0° C und 40° C und unter Verwendung eines Halogenids, vorzugsweise Chlorids oder Bromids, oder eines Sulfonsäureesters, z.B. eines Mesylats oder Tosylats als $-ZR^1$ abgebende Verbindung. Der Rest $R^a$ kann dabei vor der oben beschriebenen Alkylierung strukturell modifiziert werden oder auch im Anschluss an diese Alkylierung. Modifizierungsreaktionen des Restes $R^a$ zu $R^b$, einem Rest der allgemeinen Formel $-T^1- L^2 - T^2 - L^3 - T^3- L^4- T^4- U$, beinhalten übliche Transformationsreaktionen wie Entfernung und wieder Einführung einer funktionellen Gruppe, Alkylierung und Acylierung von Alkohol und Aminfunktionen, Oxidationen von Sulfiden zu Sulfoxiden und Sulfonen sowie weitere in der Literatur gut dokumentierte Transformations-reaktionen. Beispiele für spezifische strukturelle Umwandlungen des Restes $R^a$ zu Resten $R^b$ sind in Schema 13 zusammengefasst:

**Schema 13**

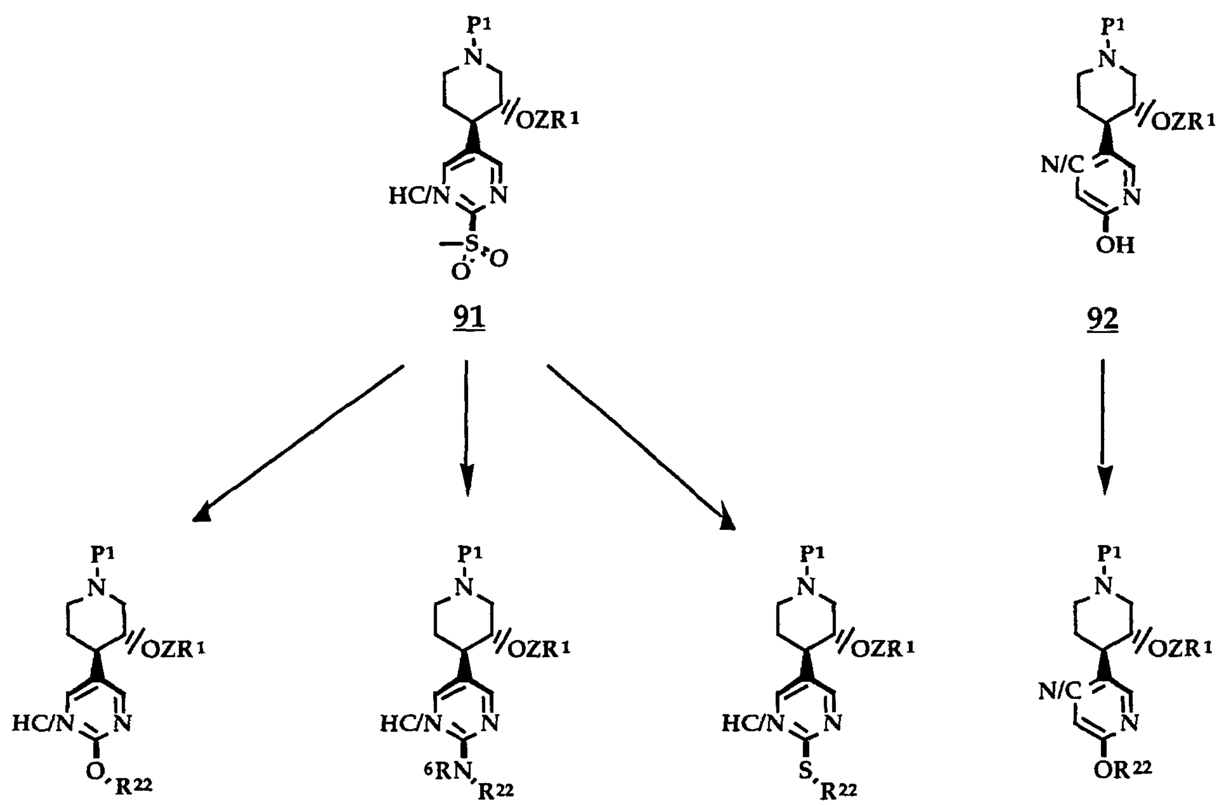

91 92

93 94 95 96

2-Methylsulfonyl Pyridin und Pyrimidin Derivate 91, die in 3 Stellung des Piperidins bereits geeignet substituiert sind, lassen sich mit Alkoholaten, Thiolaten und Aminen in Tetrahydrofuran, 1,2-Dimethoxyäthan, Dimethylformamid oder Dimethylsulfoxid bei Temperaturen zwischen Raumtemperatur und ca. 150 °C zu den entsprechenden substituierten Heteroaryl-Verbindungen umsetzen. Phenolische Pyridin oder Pyrazin-Derivate 92, die in 3-Stellung des Piperidines eine OH oder eine $OZR^1$ Funktion besitzen, können durch Verwendung einer Base und eines Alkylierungsmittels nach bekannten Methoden an der phenolischen O-Funktion alkyliert werden, wobei jeweils unterschiedliche Anteile an N-Alkylierungsprodukten mit entstehen können. Setzt man dagegen mit einem Alkohol in Gegenwart von Triphenylphosphin und eines Azodicarbonsäureesters in einem Lösungsmittel wie Tetrahydrofuran oder 1,2-Dimethoxyäthan nach Mitsunobu [Synthesis 1981, 1] um, so werden fast ausschliesslich O-Alkylierungsprodukte gebildet. In den Reaktionsprodukten der allgemeinen Formeln 93 - 96 stellen somit $-OR^{22}$, $-R^6NR^{22}$, $-SR^{22}$ je ein Rest $-T^1- L^2 - T^2 - L^3- T^3 - L^4 - T^4 - U$, mit $T^1$ = Sauerstoff, Stickstoff oder Schwefel dar.

**Schema 14**

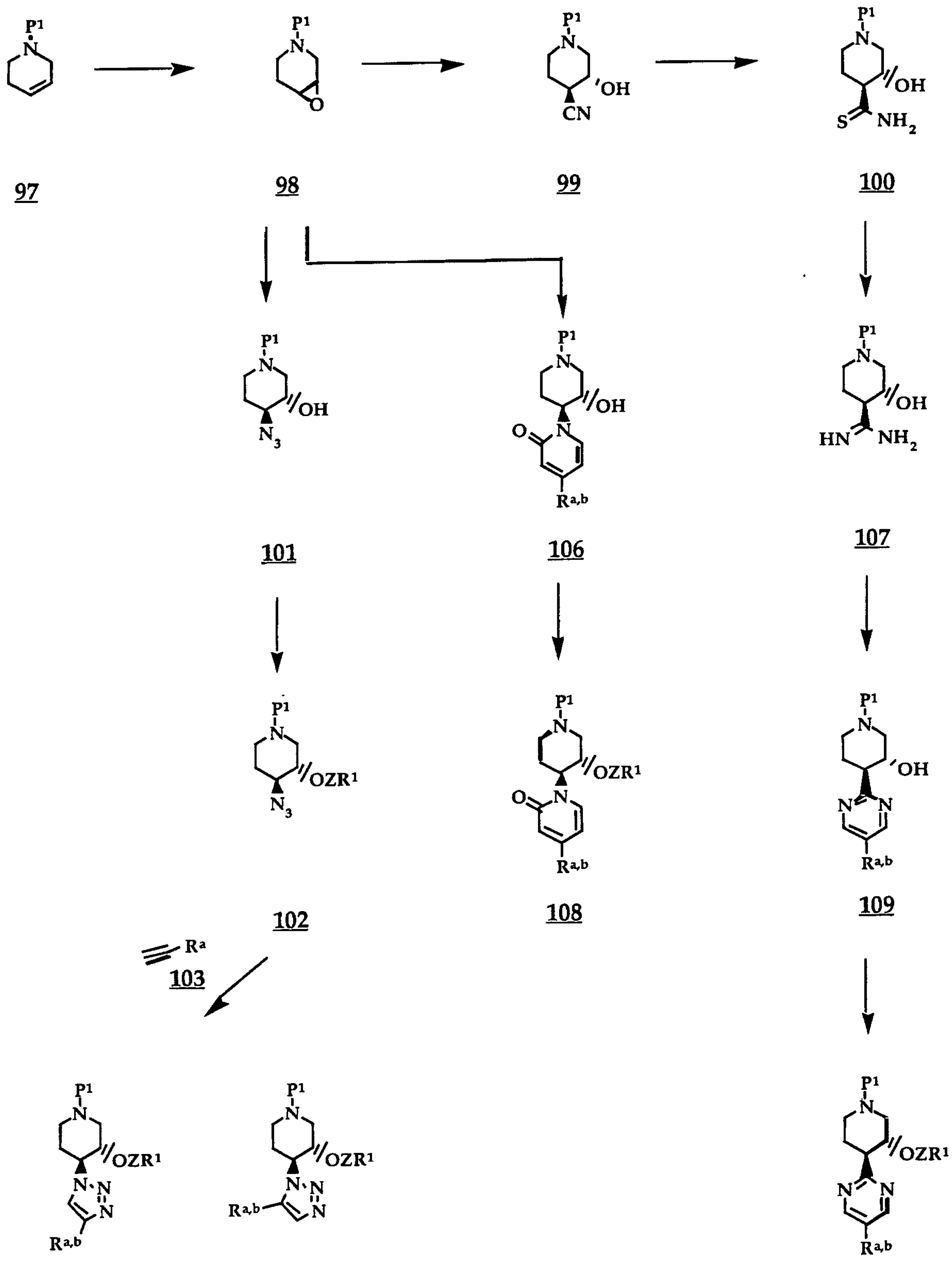

97 98 99 100

101 106 107

102 108 109

103

104 105 110

Gemäß Schema 14 können Verbindungen der allgemeinen Formel 104, 105, sowie 108 bis 110 aus Oxiran-Verbindungen 98 erhalten werden. Oxiran-Verbindungen 98 können durch Oxidation der entsprechenden Olefine mittels Persäuren, wie Peressigsäure oder Perbenzoesäure, vorzugsweise 3-Chlor-perbenzoesäure erhalten werden. Sie lassen sich mit Azid-Anionen in protischen Lösungsmitteln wie Äthanol oder Methanol oder in aprotischen Lösungsmitteln wie N,N,-Dimethylformamid, Acetonitril oder Dimethylsulfoxid mit oder ohne Zusatz von Lewis Säuren wie Lithiumperchlorat oder Magnesiumsulfat bei Temperaturen zwischen 50 °C und 150 °C mit unterschiedlichem Anteil an Epoxidöffnungsprodukten mit der Azido-Funktion in 3-Stellung und der OH-Funktion in 4-Stellung des Piperidinringes zu Azido-Verbindungen 101 umsetzen. Die unerwünschten isomeren Verbindungen mit Azido-Funktion in 3-Stellung des Piperidinringes lassen sich beispielsweise durch Chromatographie an Kieselgel abtrennen. Nach Einführung einer geeigneten Ätherfünktion in Stellung 3 können die Azidoverbindungen durch Kondensation mit einer geeigneten Acetylenverbindung 103, wie beispielsweise Propargylalkohol in einem apolaren Lösungsmittel wie Toluol oder Xylol bei Temperaturen zwischen 60 °C und 160 °C, zu den beiden isomeren N-Triazolyl-Verbindungen der allgemeinen Formeln 104 und 105 umgewandelt werden. Beide ergeben nach Einführung einer geeigneten Seitenkette am Substituenten des Triazol-Ringes durch Anwendung spezifischer Modifizierungsreaktionen des Restes $R^a$ zu $R^b$ wie Entfernung und wieder Einführung einer funktionellen Gruppe, Alkylierung und Acylierung von Alkohol und Aminfünktionen, Oxidationen von Sulfiden zu Sulfoxiden und Sulfonen sowie weitere in der Literatur gut dokumentierte Transformationsreaktionen und nach Abspaltung der Schutzgruppe am Stickstoff des Piperidinringes potente Reninhemmer. Verbindungen der allgemeinen Formel 106 können durch nucleophile Öffnung der Oxirane der allgemeinen Formel 98 mit dem aus dem substituierten Pyridon erhaltenen Anion synthetisiert werden. Die nucleophile Öffnung kann nach an sich bekannten Methoden erfolgen, beispielsweise in einem unter den Reaktionsbedingungen inerten Lösungsmittel, wie z.B. Acetonitril, 1,2-Dimethoxyäthan oder N,N-Dimethylformamid, bei einer Temperatur zwischen etwa Raumtemperatur und 120 °C und unter Verwendung eines Katalysators, z.B. Ammoniumchlorid oder Lithiumperchlorat, wobei das in unterschiedlichen Anteilen anfallende isomere Epoxidöffnungsprodukt zweckmäßigerweise durch eine Chromatographie an Kieselgel abgetrennt werden kann. Verbindungen der allgemeinen Formel 109 können ausgehend von Verbindungen der allgemeinen Formel 98 erhalten werden, indem zunächst eine nucleophile Öffnung der Oxirane mittels Cyanid-Ionen zu Verbindungen der Formel 99 stattfindet. Die nucleophile Öffnung kann nach an sich bekannten Methoden erfolgen, beispielsweise in einem unter den Reaktionsbedingungen inerten Lösungsmittel, wie z.B. Acetonitril, 1,2-Dimethoxyäthan oder N,N-Dimethylformamid, bei einer Temperatur zwischen etwa Raumtemperatur und 120 °C und unter Verwendung eines Katalysators, z.B. Ammoniumchlorid, Zinktrifluoracetat oder Lithiumtetrafluoroborat, speziell Lithiumperchlorat, wobei das in unterschiedlichen Anteilen anfallende isomere Epoxidöffnungsprodukt zweckmäßigerweise durch eine Chromatographie an Kieselgel abgetrennt wird. Verbindungen der allgemeinen Formel 107 können durch direkte Addition von Ammoniak oder über die entsprechenden Thioamide der allgemeinen Formel 100 aus Verbindungen der allgemeinen Formel 99 erhalten werden. Die Addition kann nach an sich bekannten Methoden erfolgen, beispielsweise indem das Nitril unter Druck mit Ammoniak und Ammoniumchlorid umgesetzt wird oder indem Schwefelwasserstoff, vorzugsweise in Form eines Hydrogensulfids, mit dem Nitril zum Thioamid der allgemeinen Formel 100 umgesetzt wird. Dieses kann wiederum, z.B. nach dem in Helv.Chim.Acta Vol. 69, 1224 (1986) beschriebenen Verfahren, durch Alkylierung mittels Methyliodid oder Äthyliodid in das entsprechende Sulfonium-Derivat überführt werden, dessen Ammonolyse, z.B. mit Ammoniumchlorid, zum Amidin der allgemeinen Formel 107 führt. Verbindungen der allgemeinen Formel 109 können durch eine Ringschlußreaktion des Amidins der allgemeinen Formel 107 mit einem entsprechenden Malondialdehyd hergestellt werden. Der Aufbau der Pyrimidineinheit kann nach an sich bekannten Verfahren erfolgen, beispielsweise durch Umsetzung des Amidins mit dem Diacetal oder Enamin des 2-substituierten Malondialdehyds in einem unter den Reaktionsbedingungen inerten Lösungsmittel, wie z.B. Methanol, bei einer Temperatur zwischen etwa Raumtemperatur und 120 °C.

Je nach angestrebtem Zielmolekül kann ausgehend von Verbindungen 106 und 109 zunächst $-ZR^1$ eingeführt und anschliessend der Rest $R^a$ zu $R^b$ modifiziert werden oder aber ein umgekehrtes Aufbauschema verwendet werden.

## Schema 15

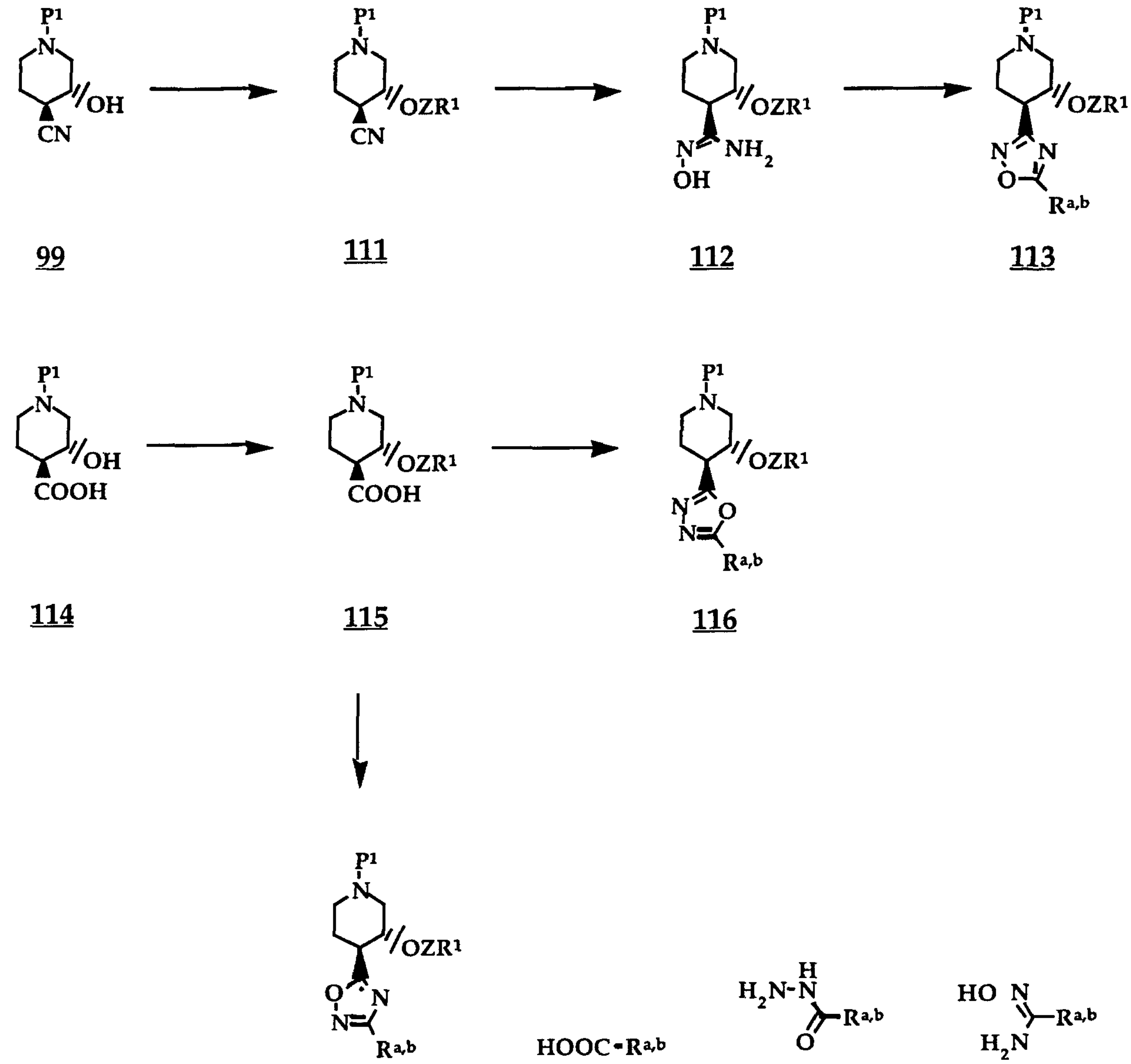

99 111 112 113

114 115 116

$HOOC-R^{a,b}$

117 118 119 120

Gemäß Schema 15 können Verbindungen der allgemeinen Formel 113 aus Verbindungen der allgemeinen Formel 99 dadurch erhalten werden, daß zunächst der sekundäre Alkohol mit einer den Rest Z-$R^1$ abgebenden Verbindung alkyliert wird, wobei gegebenenfalls der weitere Aufbau des gewünschten Substitutenten in einer späteren Stufe der Reaktionssequenz erfolgen kann. Die Alkylierung des sekundären Alkohols erfolgt nach an sich bekannten Verfahren, beispielsweise in einem unter den Reaktionsbedingungen inerten Lösungsmittel, wie einem Äther, z.B. Tetrahydrofuran oder 1,2-Dimethoxyäthan, oder Dimethylformamid, mit Hilfe einer alkoholatbildenden Base Base, z. B. Natriumhydrid, bei einer Temperatur zwischen etwa 0 °C und 40 °C und unter Verwendung eines Halogenids, vorzugsweise Chlorids oder Bromids, oder eines Sulfonsäureesters, z.B. eines Mesylats oder Tosylats als Z-$R^1$ abgebende Verbindung.

Die Umsetzung von Verbindungen der allgemeinen Formel 111 mit Hydroxylamin in Gegenwart einer Base, wie z.B. Natriummethylat, zweckmäßigerweise bei Temperaturen zwischen 40 °C und 100 °C, ergibt Amidoxime der allgemeinen Formel 112. Verbindungen der allgemeinen Formel 113 werden erhalten, indem reaktionsfähige funktionelle Derivate einer Carbonsäure der allgemeinen Formel 118 mit einem Amidoxim der Formel 112 umgesetzt werden.

Die Reaktion erfolgt zweckmäßigerweise durch mehrstündiges Erhitzen auf etwa 70 °C bis 130 °C in einem inerten Lösungsmittel, z.B. in Dimethylformamid. Das intermediär gebildete, nicht-cyclisierte Kondensationsprodukt cyclisiert spontan unter den gegebenen Reaktionsbedingungen. Als reaktionsfähige funktionelle Derivate der Carbonsäure der allgemeinen Formel 118 können die entsprechenden Imidazolide eingesetzt werden, welche nach an sich bekannten Verfahren aus den entsprechenden freien Carbonsäuren hergestellt werden können, z.B. durch Umsetzung mit 1,1'-Carbonyldiimidazol in einem inerten organischen Lösungsmittel, z.B. in Dimethylformamid. Weiterhin können als reaktionsfähige funktionelle Derivate der Carbonsäure auch Carbonsäurechloride eingesetzt werden, welche sich aus den entsprechenden freien Carbonsäuren mittels Thionylchlorid oder Oxalylchlorid herstellen lassen.

Gemäß Schema 15 können Verbindungen der allgemeinen Formel 116 aus Verbindungen der allgemeinen Formel 114 dadurch erhalten werden, daß zunächst, wie bereits oben erwähnt, durch Alkylierung Verbindungen der allgemeinen Formel 115 hergestellt werden. Diese werden dann in reaktionsfähige, funktionelle Derivate der Carbonsäure überführt und mit Hydraziden der allgemeinen Formel 119 umgesetzt. Die Reaktion erfolgt zweckmäßigerweise bei Raumtemperatur bis 50 °C in einem inerten organischen Lösungsmittel, z.B. in Dimethylformamid. Das dabei entstehende nicht-cyclisierte Kondensationsprodukt kann isoliert werden, um dann durch mehrstündiges Erhitzen mit Polyphosphorsäure bei ca. 100 °C zu Verbindungen der allgemeinen Formel 116 cyclisiert zu werden.

Gemäß Schema 15 können Verbindungen der allgemeinen Formel 117 aus Verbindungen der allgemeinen Formel 115 dadurch erhalten werden, daß reaktionsfähige, funktionelle Derivate der Carbonsäure mit Amidoximen der allgemeinen Formel 120 umgesetzt werden. Die Reaktion erfolgt zweckmäßigerweise durch mehrstündiges Erhitzen auf etwa 70 °C bis 130 °C in einem inerten Lösungsmittel, z.B. in Dimethylformamid. Das intermediär gebildete, nicht-cyclisierte Kondensationsprodukt cyclisiert spontan unter den gegebenen Reaktionsbedingungen.

Die Verbindungen der allgemeinen Formel 114, 118 - 120 gehören allgemein bekannten Verbindungsklassen an und sind daher für jeden Fachmann ohne weiteres zugänglich.

Im weiteren lassen sich Verbindung der allgemeinen Formel II, bei welchen $R^2$ die Bedeutung eines 5-gliederigen aromatischen Restes hat, auch in enger Anlehnung an die in Schema 12 beschriebene Aufbausequenz herstellen, wobei das spezfisch funktionalisierte 6-gliederige aromatische Synthon 82 durch ein entsprechendes 5-gliederiges aromatisches Synthon ersetzt werden muss.

Piperidine der allgemeinen Formel I können auch in optisch reiner Form dargestellt werden. Die Trennung in Antipoden kann nach an sich bekannten Methoden erfolgen entweder vorzugsweise auf einer synthetisch frühen Stufe durch Salzbildung mit einer optisch aktiven Säure wie zum Beispiel (+)- oder (-)-Mandelsäure und Trennung der diasteromeren Salze durch fraktionierte Kristallisation oder vorzugsweise auf einer eher späten Stufe durch Derivatisierung mit einem chiralen Hilfsbaustein, wie zum Beispiel (+)- oder (-)-Camphansäurechlorid, und Trennung der diastereomeren Produkte durch Chromatographie und/oder Kristallisation und anschließende Spaltung der Bindung zum chiralen Hilfsstoff. Die reinen diastereomeren Salze und Derivate können zur Bestimmung des absoluten Konfiguration des enthaltenen Piperidines mit gängigen spektroskopischen Methoden analysiert werden, wobei die X-Ray Spektroskopie an Einkristallen eine besonders geeignete Methode darstellt.

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.
Die in-vitro-Potenz von Renin-Hemmern kann, wie bereits von W.Fischli et al. in Hypertension Vol.18 (1), 22-31 (1991) oder in Hypertension Vol.22 (1), 9-17 (1993) beschrieben, experimentell mittels der nachstehend beschriebenen Tests gezeigt werden. Die Tests werden in analoger Weise zu denen von D.T.Pals et al. in Hypertension Vol.8, 1105-1112 (1986) oder J.Boger et al. in J.Med.Chem. 28 1779-1790 (1985) oder J.F.Dellaria et al. in J.Med.Chem. 30, 2137-2144 (1987) oder T.Kokubu et al. in Biochem.Biophys.Res.Commun. 118, 929-933 (1984) beschriebenen durchgeführt.

In vitro-Test mit reinem Human-Renin

Der Test wird in Eppendorf Röhrchen durchgeführt. Die Inkubationsmischung besteht aus (1) 100 ml Human-Renin in Puffer A (0.1M Natriumphosphatlösung, pH 7.4, enthaltend 0.1% Rinderserumalbumin, 0.1 % Natriumazid und 1 mM Aethylendiamintetraessigsäure), genügend für eine Renin-Aktivität von 2-3 ng Angiotensin I/ ml/Std.; (2) 145 ml Puffer A: (3) 30 ml von 10 mM humanem Tetradekapeptid-Reninsubstrat (hTD) in 10 mM Salzsäure: (4) 15 ml Dimethylsulfoxid mit bzw. ohne Hemmer und (5) 10 ml einer 0.03 molaren Lösung von Hydroxychinolinsulfat in Wasser.

Die Proben werden drei Stunden bei 37°C bzw. 4°C in Triplikaten inkubiert. 2 x 100 ml Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0.0013 %; hTD (Angiotensin I-Val-Ile-His-Ser-OH) 0.09 %. Die Produktion von Angiotensin I wird durch die Differenz zwischen dem Versuch bei 37°C und demjenigen bei 4°C bestimmt.
Folgende Kontrollen werden mitgeführt:

(a) Inkubation von hTD-Proben ohne Renin und ohne Hemmer bei 37°C und 4°C. Die Differenz zwischen diesen beiden Werten ergibt den Grundwert der Angiotensin I-Produktion.

(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.
Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.
Die in diesen Tests erhaltenen Resultate sind in der folgenden Tabelle zusammengefasst:

Tabelle

| Verbindung | $IC_{50}$-Werte in µMol/l |
|---|---|
| A | 0.011 |
| B | 0.026 |
| C | 0.070 |
| D | 0.040 |
| E | 0.041 |
| F | 0.057 |
| G | 0.033 |
| H | 0.073 |
| I | 0.317 |
| J | 0.017 |
| K | 2.600 |
| L | 3.080 |
| M | 0.008 |
| N | 0.012 |
| O | 0.017 |
| P | 0.006 |
| Q | 0.005 |
| R | 0.003 |
| S | 0.002 |
| T | 0.005 |
| U | 0.024 |
| V | 0.002 |
| W | 0.002 |
| X | 0.003 |
| Y | 0.003 |
| Z | 0.001 |
| AA | 0.001 |
| BB | 0.003 |
| CC | 0.002 |
| DD | 0.001 |
| EE | 0.0004 |

Tabelle (fortgesetzt)

| Verbindung | $IC_{50}$-Werte in µMol/l |
|---|---|
| FF | 0.0006 |
| GG | 0.001 |
| HH | 0.006 |
| II | 0.002 |
| JJ | 0.002 |
| KK | 0.012 |
| LL | 0.001 |
| MM | 0.0005 |
| NN | 0.001 |
| OO | 0.006 |
| PP | 0.002 |
| QQ | 0.002 |
| RR | 0.270 |
| SS | 132 |
| TT | 0.0005 |
| UU | 0.0001 |
| VV | 0.002 |
| WW | 0.009 |
| XX | 0.0008 |
| YY | 0.0005 |
| ZZ | 0.00003 |

A = Thiophen-2-carbonsäure (3RS,4RS)-2-[4-(3-Naphthalin-2-ylmethoxy-piperidin-4-yl)-phenoxy]-äthylester Hydrochlorid (Beispiel 58-4)

B = 2-Chlor-benzoesäure (3RS,4RS)-2-[4-(3-Napthalin-2-ylmethoxy-piperidin-4-yl)-phenoxy]-äthylester Hydrochlorid (Beispiel 54-2)

C = Benzoesäure (3RS,4RS)-2-[4-[3-[4-(2-Methoxy-benzyloxy)-naphthalin-2-ylmethoxy]-piperidin-4-yl]-phenoxy]-äthylester Hydrochlorid (Beispiel 55-2)

D = (3RS,4RS)-4-[4-(3-Benzyloxy-propoxy)-phenyl]-3-(2,3-dihydro-benzo[1,4]dioxin-6-ylmethoxy)-piperidin (Beispiel 86-54)

E = (3RS,4RS)-3-(Naphthalin-2-ylmethoxy)-4-[4-(3-phenyl-[1,2,4]oxadiazol-5-ylmethoxy)-phenyl]-piperidin Trifluoracetat (Beispiel 86-34)

F = (3RS,4RS)-3-(Naphthalin-2-ylmethoxy)-4-[4-(3-phenyl-isoxazol-5-ylmethoxy)-phenyl]-piperidin Trifluoracetat (Beispiel 86-36)

G = (3RS,4RS)-3-(Naphthalin-2-ylmethoxy)-4-[4-(3-phenylsulfanyl-propyl)-phenyl]-piperidin (Beispiel 86-19)

H = (3RS,4RS)-3-[4-[4-[2-(Benzothiazol-2-ylsulfanyl)-äthyl]-phenyl]-piperidin-3-yloxymethyl]-naphthalin-1-ol (Beispiel 86-23)

I = (3RS,4RS,5SR)-3-(4-Benzyloxy-naphthalin-2-ylmethoxy)-4-(4-fluor-phenyl)-5-propyl-piperidin (Beispiel 64)

J = (3SR,4RS,5RS)-4-(4-Benzyloxymethyl-phenyl)-3-methoxymethyl-5-(naphthalin-2-ylmethoxy)-piperidin (Beispiel 86-60)

K = Benzoesäure (SR)- oder (RS)-1-[(3RS,4SR)-4-(4-fluor-phenyl)-piperidin-3-yl]-2-naphthalin-2-yl-äthylester Hydrochlorid (Beispiel 75 b)

L = (1RS,2RS,3RS,5SR)-2-(4-Benzyloxy-naphthalin-2-ylmethoxy)-3-(4-fluor-phenyl)-8-aza-bicyclo[3.2.1]octan (Beispiel 84 e)

M = (3RS,4RS)-4-[4-(3-Benzyloxy-propoxy)-phenyl]-3-(naphthalin-2-ylmethoxy)-piperidin

N = 4-[2-[7-[(3RS,4RS)-4-[4-(3-Benzyloxy-propoxy)-phenyl]-piperidin-3-yloxymethyl]-naphthalin-2-yloxy]-äthyl]-morpholin Hydrochlorid (1:2) (Beispiel 90-07)

O = Gemisch des (RS)- und (SR)-3-[7-[(3RS,4RS)-4-[4-(3-Benzyloxy-propoxy)-phenyl]-piperidin-3-yloxymethyl]-naphthalin-2-yloxy]-propan-1,2-diols (Beispiel 90-08)

P = Gemisch des (RS)- und (SR)-3-[2-[7-[(3RS,4RS)-4-[4-(3-Benzyloxy-propoxy)-phenyl]-piperidin-3-yloxymethyl]-naphthalin-2-yloxy]-äthoxy]-propan-1,2-diol Hydrochlorides (1:1) (Beispiel 98)

Tabelle (fortgesetzt)

| Verbindung | $IC_{50}$-Werte in µMol/l |
|---|---|
| Q = 1-[2-[7-[(3RS,4RS)-4-[4-(3-Benzyloxy-propoxy)-phenyl]-piperidin-3-yloxymethyl]-naphthalin-2-yloxy]-äthyl]-4-methyl-piperazin Hydrochlorid (1:3) (Beispiel 90-13)<br>R = 1-[(3RS,4SR)-4-[4-(3-Benzyloxy-propoxy)-phenyl]-piperidin-3-yl]-2-naphthalin-2-yl-äthanon Hydrochlorid (1:1) (Beispiel 100)<br>S = (3RS,4SR,5SR)-4-[4-(3-Benzyloxy-propoxy)-phenyl]-3-(1,4-dimethoxy-naphthalin-2-ylmethoxy)-piperidin-5-ol (Beispiel 109-04)<br>T = Gemisch des (3RS,4RS)- und (3SR,4SR)-4-[4-(3-Benzyloxy-propoxy)-phenyl]-3-[7-[(RS)-2,3-dihydroxy-propoxymethyl]-naphthalin-2-ylmethoxy]-piperidins (Beispiel 106-02)<br>U = Gemisch des (3RS,4RS)- und (3SR,4SR)-4-[4-(3-Benzyloxy-propoxy)-phenyl]-3-[6-[(RS)-2,3-dihydroxy-propoxymethyl]-naphthalin-2-ylmethoxy]-piperidins (Beispiel 106-01)<br>V = 4-[(3RS,4SR,5SR)-4-[4-(3-Benzyloxy-propoxy)-phenyl]-3-(naphthalin-2-ylmethoxy)-piperidin-5-yloxy]-butan-1-ol (Beispiel 110-08)<br>W = 3-[(3RS,4SR,5SR)-4-[4-(3-Benzyloxy-propoxy)-phenyl]-3-(naphthalin-2-ylmethoxy)-piperidin-5-yloxy]-propan-1-ol (Beispiel 110-07)<br>X = 1-{2-[(3RS,4RS,5SR)-4-[4-(3-Benzyloxy-propoxy)-phenyl]-3-(naphthalin-2-ylmethoxy)-piperidin-5-yloxy]-äthyl}-4-methyl-piperazin (Beispiel 110-02)<br>Y = 4-{2-[(3RS,4RS,5SR)-4-[4-(3-Benzyloxy-propoxy)-phenyl]-3-(naphthalin-2-ylmethoxy)-piperidin-5-yloxy]-äthyl}-morpholin (Beispiel 110-09)<br>Z = (3RS,4SR,5SR)-4-[4-(3-Benzyloxy-propoxy)-phenyl]-3-(4-methoxy-benzyloxy)-piperidin-5-ol (Beispiel 109-28)<br>AA = (3R,4s,5S)-4-[4-(3-Benzyloxy-propoxy)-phenyl]-3,5-bis-(4-methoxy-benzyloxy)-piperidin (Beispiel 109-27)<br>BB = (3SR,4RS,5RS)-4-[2-[4-[4-(3-Benzyloxy-propoxy)-phenyl]-5-(naphthalin-2-ylmethoxy)-piperidin-3-ylmethoxy]-äthyl]-morpholin (Beispiel 149-04)<br>CC = (3SR,4RS,5RS)-4-[4-(3-Benzyloxy-propoxy)-phenyl]-3-methoxymethyl-5-(naphthalin-2-ylmethoxy)-piperidin (Beispiel 148)<br>DD = [3-(4-Methyl-piperazin-1-yl)-propyl]-carbaminsäure (3SR,4RS,5RS)-4-[4-(3-benzyloxy-propoxy)-phenyl]-5-(naphthalin-2-ylmethoxy)-piperidin-3-ylmethylester (Beispiel 150-01)<br>EE = (3SR,4RS,5RS)-4-[4-[4-(3-Benzyloxy-propoxy)-phenyl]-5-(naphthalin-2-ylmethoxy)-piperidin-3-ylmethylsulfanyl]-pyridin (Beispiel 149-02)<br>FF = 2-(4-Cyclohexyl-butoxy)-5-[(3RS,4RS)-3-(1,4-dimethoxy-naphthalin-2-ylmethoxy)-piperidin-4-yl]-pyrimidin (Beispiel 139-03)<br>GG = (3'RS,4'RS)-6-(3-Cyclohexyl-propoxy)-3'-(1,4-dimethoxy-naphthalin-2-ylmethoxy)-1',2',3',4',5',6'-hexahydro-[3,4']bipyridin (Beispiel 140-01)<br>HH = (3SR,4RS,5RS)-[4-[4-(3-Benzyloxy-propoxy)-phenyl]-5-(naphthalin-2-ylmethoxy)-piperidin-3-yl]-methanol Hydrochlorid (Beispiel 149-02)<br>II = (3SR,4RS,5RS)-N-[4-[4-(3-Benzyloxy-propoxy)-phenyl]-5-(naphthalin-2-ylmethoxy)-piperidin-3-ylmethyl]-N,N',N'-trimethyl-äthan-1,2-diamin (Beispiel 149-06)<br>JJ = (3SR,4RS,5RS)-[4-[4-(3-Benzyloxy-propoxy)-phenyl]-5-(naphthalin-2-ylmethoxy)-piperidin-3-ylmethyl]-diäthyl-amin (Beispiel 149-05)<br>KK = 1-[(3RS,4SR,5SR)-4-[4-(3-Benzyloxy-propoxy)-phenyl]-5-(2-morpholin-4-yl-äthoxymethyl)-piperidin-3-yl]-2-naphthalin-2-yl-äthanon (Beispiel 101)<br>LL = (3RS,4RS)-3-(1,4-Dimethoxy-naphthalin-2-ylmethoxy)-4-[4-[3-(2-methoxy-phenoxy)-propoxy]-phenyl]-piperidin (Beispiel 123-27)<br>MM =(3R,4s,5S)-4-[4-(3-Benzyloxy-propoxy)-phenyl]-3,5-bis-(3,4,5-trimethoxy-benzyloxy)-piperidin (Beispiel 109-29)<br>NN = (3RS,4RS,5SR)-4-[4-(3-Benzyloxy-propoxy)-phenyl]-3-(naphthalin-2-ylmethoxy)-5-[1,2,4]triazol-1-ylmethyl-piperidin Hydrochlorid (Beispiel 149-07)<br>OO = (3RS,4RS)-4-[4-[3-(2-Methoxy-benzyloxy)-propoxy]-phenyl]-3-(quinolin-7-ylmethoxy)-piperidin (Beispiel 120-07)<br>PP = 2-(7-{(3RS,4RS)-4-[4-(3-Benzyloxy-propoxy)-phenyl]-piperidin-3-yloxymethyl}-naphthalin-2-ylmethoxy)-äthanol (Beispiel 106-03) | |

Tabelle (fortgesetzt)

| Verbindung | $IC_{50}$-Werte in µMol/l |
|---|---|
| QQ= 7-{(3RS,4RS)-4-[4-(3-Benzyloxy-propoxy)-phenyl]-piperidin-3-yloxymethyl}-naphthalin-2-ylmethyl)-dimethyl-amin (Beispiel 106-03)<br>RR = (3R,4R)-3-(4-Benzyloxy-naphthalin-2-ylmethoxy)-4-(4-fluor-phenyl)-piperidin (Beispiel 154-06)<br>SS = (3S,4S)-3-(4-Benzyloxy-naphthalin-2-ylmethoxy)-4-(4-fluor-phenyl)-piperidin (Beispiel 154-07)<br>TT = (3'RS,4'RS)-3'-(1,4-Dimethoxy-naphthalin-2-ylmethoxy)-6-[3-(2-methoxybenzyloxy)-propoxy]-1',2',3',4',5',6'-hexahydro-[3,4']bipyridin (Beispiel 140-02)<br>UU = (3RS,4RS)-3-(1,4-Dimethoxy-naphthalin-2-ylmethoxy)-4-[4-[3-(2-methoxy-benzyloxy)-propoxy]-phenyl]-piperidin (Beispiel 123-32)<br>W = (3SR,4RS,5RS)-1-[4-[4-(3-Benzyloxy-propoxy)-phenyl]-5-(naphthalin-2-ylmethoxy)-piperidin-3-ylmethyl]-imidazolidin-2-on (Beispiel 149-08)<br>WW= (3RS,4RS)-4-[4-[3-(2-Methoxy-benzyloxy)-propoxy]-phenyl]-3-(2-oxo-1,2-dihydro-chinolin-7-ylmethoxy)-piperidin (Beispiel 120-10)<br>XX = (3RS,4RS)-3-(Isochinolin-7-ylmethoxy)-4-[4-[3-(2-methoxy-benzyloxy)-propoxy]-phenyl]-piperidin (Beispiel 120-11)<br>YY = (3RS,4RS)-4-[4-[3-(2-Methoxy-benzyloxy)-propoxy]-phenyl]-3-(1,2,3,4-tetrahydro-chinolin-7-ylmethoxy)-piperidin (Beispiel 120-12)<br>ZZ = (3RS,4SR,5SR)-3-(1,4-Dimethoxy-naphthalin-2-ylmethoxy)-4- [4-[3-(2-methoxy-benzyloxy)-propoxy]-phenyl]-piperidin-5-ol (Beispiel 112-11) | |

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser. Polyole, Sacharose, Invertzucker, Glukose etc.

Für Injektionslösungen eignen sich als Excipientien z.B. Wasser, Alkohole, Polyole, Glycerin, vegetabile Oele, Gallensäuren, Lecithin etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz, sowie Glaukom, Herzinfarkt, Niereninsuffizienz und Restenosen verwenden.

Die erfindungsgemässen Verbindungen können auch in Kombination mit einem oder mehreren cardiovasculär wirksamen Mitteln, z.B. α- und β-Blockern wie Phentolamin, Phenoxybenzamin, Prazosin, Terazosin, Tolazine, Atenolol, Metoprolol, Nadolol, Propranolol, Timolol, Carteolol etc.; Vasodilatatoren wie Hydralazin, Minoxidil, Diazoxid, Nitroprussid, Flosequinan etc.; Calcium- Antagonisten wie Amrinon, Bencyclan, Diltiazem, Fendilin, Flunarizin, Nicardipin, Nimodipin, Perhexilen, Verapamil, Gallopamil, Nifedipin etc.; ACE-Hemmern wie Cilazapril, Captopril, Enalapril, Lisinopril etc.; Kalium-Aktivatoren wie Pinacidil; anti-Serotoninergica wie Ketanserin; Thromboxan-Synthetasehemmern; Angiotensin II Antagonisten; sowie Diuretica wie Hydrochlorothiazid, Chlorothiazid, Acetazolamid, Amilorid, Bumetanid, Benzthiazid, Ethacrynsäure, Furosemid, Indacrinon, Metolazon, Spironolacton, Triamteren, Chlorthalidon etc.; Sympatholytica wie Methyldopa, Clonidin, Guanabenz, Reserpin; und anderen Mitteln, die für die Behandlung von Bluthochdruck, Herzinsuffizienz oder mit Diabetes oder Nierenerkrankungen wie akutem oder chronischem Nierenversagen verbundenen Gefässerkrankungen bei Mensch und Tier geeignet sind, verabreicht werden. Solche Kombinationen können getrennt oder in Präparaten, die mehrere Komponenten enthalten, angewandt werden.

Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro erwachsene Person (70 kg), verteilt auf vorzugsweise 1-3 Einzeldosen, die z.B. gleich gross sein können, angemessen sein, wobei aber die angegebene obere Grenze auch überschritten werden kann, wenn sich dies als angezeigt erweisen sollte. Ueblicherweise erhalten Kinder reduzierte Dosis ihrem Alter und Körpergewicht entsprechend geringere Dosis.

Ähnliche Verbindungen aus anderem pharmakologishcne Gebiet sind bekannt aus

US-A-4007196
US-A-4877799
US-A-5276042
Israelisches Patent 84021
Israelisches Patent 89903
Chilenisches Patent 34759.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:

BOC : tert-Butoxycarbonyl
DME: 1,2-Dimethoxyäthan
DMF: Dimethylformamid
TBAF: Tetrabutylammoniumfluorid
EDC : N-Aethyl-N'-(3-dimethylaminopropyl)-carbodiimid Hydrochlorid
THP: Tetrahydropyranyl
TROC : Trichloräthoxycarbonyl
TPTU: O-(1,2-Dihydro-2-oxo-1-pyridyl)-N,N,N'N'-tetramethyluronium-tetrafluoroborat
HBTU: O-(1H-Benzotriazol-1-yl)-N,N,N'N'-tetramethyluronium-hexafluorophosphat
SEM: 2-(Trimethylsilyl)-äthoxymethyl.

Beispiel 1

(a) Eine Lösung von 23.6 g (100 mMol) 1,3-Dibrombenzol in 250 ml absolutem Äther wurde auf -75° C abgekühlt. Es wurde innerhalb von 45 Minuten eine Lösung von 62.5 ml (100 mMol) n-Butyllithium (1.6 M in Hexan) tropfenweise zugegeben. Die entstandene Suspension wurde 2.5 Stunden lang bei -75° C gerührt. Anschließend wurde eine Lösung von 19.0 g (100 mMol) 1-Benzyl-4-piperidon in 100 ml absolutem Äther innerhalb von 30 Minuten bei -70° C bis -75° C tropfenweise zugegeben und danach 2 Stunden gerührt. Anschließend wurde das Gemisch zwischen Äther und gesättigter Ammoniumchlorid-Lösung verteilt, die organische Phase über Magnesiumsulfat getrocknet und schließlich das Lösungsmittel unter verminderten Druck entfernt. Das Rohprodukt wurde durch Chromatographie an Kieselgel mit einem 1:1-Gemisch von Methylenchlorid und Hexan als Eluierungsmittel gereinigt. Es wurden 28.3 g (82% d.Th.) 1-Benzyl-4-(3-Bromphenyl)-piperidin-4-ol als gelbes Öl erhalten; MS: 345, 347 $(M)^+$.

(b) Eine Lösung von 28.2 g (81.4 mMol) 4-(3-Bromphenyl)-piperidin-4-ol in 600 ml Toluol wurde mit 30 g (157 mMol) p-Toluolsulfonsäuremonohydrat versetzt und 4 Stunden am Wasserabscheider zum Rückfluß erhitzt. Anschließend wurde das Reaktionsgemisch auf Raumtemperatur abgekühlt und mit 3N Natronlauge auf pH 10 gestellt. Danach wurde zunächst dreimal mit 500 ml Methylenchlorid extrahiert. Die vereinigten organischen Phasen wurden dreimal mit je 200 ml Wasser gewaschen, über Magnesiumsulfat getrocknet und dann unter vermindertem Druck eingedampft. Das Rohprodukt wurde durch Chromatographie an Kieselgel mit einem 1:1-Gemisch von Methylenchlorid und Hexan als Eluierungsmittel gereinigt. Es wurden 9.5 g (36% d.Th.) 1-Benzyl-4-(3-bromphenyl)-1,2,3,6-tetrahydro-pyridin als leicht gelbes Öl erhalten; MS : 327, 329 $(M+H)^+$.

(c) Zu einer Suspension von 9.5 g (28.9 mMol) 1-Benzyl-4-(3-bromphenyl)-1,2,3,6-tetrahydro-pyridin in 65 ml absolutem Dimethoxyäthan (DME) wurden bei Raumtemperatur portionsweise 3.15 g (83.3 mMol) Natriumborhydrid gegeben. Danach wurde bei 15-20° C eine Lösung von 17.7 ml (20.0 g 140.9 mMol) Bortrifluorid-Ätherat in 11 ml

DME tropfenweise zugegeben und 2 Stunden bei Raumtemperatur gerührt. Anschließend wurde bei 20-25° C eine Lösung von 18.3 g (326 mMol) Kaliumhydroxid in 100 ml Wasser innerhalb von 30 Minuten zugetropft. Schließlich wurden bei 20-25° C innerhalb von 30 Minuten 55 ml 30%iger Wasserstoffperoxid-Lösung tropfenweise zugegeben. Das Gemisch wurde 30 Minuten bei Raumtemperatur gerührt und 3 Stunden zum Rückfluß erhitzt. Nach Abkühlen des Reaktionsgemisches wurde die ausgefallene Borsäure abfiltriert. Anschließend wurde das Filtrat zwischen Methylenchlorid und Wasser verteilt, die organische Phase über Magnesiumsulfat getrocknet, und das Lösungsmittel unter vermindertem Druck entfernt. Das Rohprodukt wurde durch Chromatographie an Kieselgel mit einem 1:1-Gemisch von Essigester und Methylenchlorid als Eluierungsmittel gereinigt. Es wurden 6.3 g (63% d.Th.) (3RS,4RS)-1-Benzyl-4-(3-bromphenyl)-piperidin-3-ol als farbloses Öl erhalten. MS: 345, 347 $(M)^+$.

(d) Eine Lösung von 691 mg (2.00 mMol) (3RS,4RS)-1-Benzyl-4-(3-bromphenyl)-piperidin-3-ol in 3 ml absolutem Tetrahydrofuran wurde mit 163 mg (2.20 mMol) Lithiumcarbonat versetzt und auf -50° C abgekühlt. Dazu wurde tropfenweise bei -50° C eine Lösung von 722 mg (4.00 mMol) Chlorameisensäure β-trimethylsilyläthylester [Synthesis 346 (1987)] in 4 ml Toluol gegeben. Das Reaktionsgemisch wurde auf Raumtemperatur erwärmt und 24 Stunden gerührt. Anschließend wurde das Gemisch zwischen Methylenchlorid und Wasser verteilt, die organische Phase über Magnesiumsulfat getrocknet und schließlich das Lösungsmittel unter verminderten Druck entfernt. Das Rohprodukt (0.8 g) wurde durch Chromatographie an Kieselgel mit Methylenchlorid als Eluierungsmittel gereinigt. Es wurden 470 mg (43% d.Th.) (3RS,4RS)-4-(3-Bromphenyl)-3-(2-trimethylsilyl-äthoxycarbonyloxy)-piperidin-1-carbonsäure 2-trimethylsilyläthylester als leicht gelbes Öl erhalten, das direkt in der nächsten Stufe eingesetzt wurde.

(e) Eine Lösung von 470 mg (0.863 mMol) (3RS,4RS)-4-(3-Bromphenyl)-3-(2-trimethylsilyl-äthoxycarbonyloxy)-piperidin-1-carbonsäure 2-trimethylsilyläthylester in 3 ml absolutem Tetrahydrofuran wurde mit 2.65 ml (2.91 mMol) Tetrabutylammoniumfluorid-Lösung (1.1M in THF) versetzt und 2.5 Stunden bei Raumtemperatur gerührt. Anschließend wurde das Gemisch zwischen Methylenchlorid und gesättigter Natriumcarbonat-Lösung verteilt, die organische Phase über Magnesiumsulfat getrocknet und schließlich das Lösungsmittel unter verminderten Druck entfernt. Das Rohprodukt (440 mg) wurde durch Chromatographie an Kieselgel mit einem 6.5:1:0.1-Gemisch von Methylenchlorid, MeOH und 25%igem Ammoniak als Eluierungsmittel gereinigt. Es wurden 180 mg (81% d.Th.) (3RS,4RS)-4-(3-Bromphenyl)-piperidin-3-ol als leicht gelbes Öl erhalten. MS: 255, 257 $(M)^+$.

(f) Eine Lösung von 180 mg (0.702 mMol) (3RS,4RS)-4-(3-Bromphenyl)-piperidin-3-ol in 1 ml absolutem Dimethylformamid wurde bei 0° C mit 0.1 ml (73 mg, 0.72 mMol) Triäthylamin versetzt. Dazu wurde bei 0° C eine Lösung von 167 mg (76.5 mMol) Di-tert-butyl-dicarbonat in 0.5 ml Dimethylformamid gegeben. Das Gemisch wurde auf Raumtemperatur erwärmt und 20 Stunden gerührt. Das Lösungmittel wurde bei 0.1 mm Hg bei 50-55° C abdestilliert. Anschließend wurde der erhaltene Rückstand zwischen Methylenchlorid und Wasser verteilt, die organische Phase über Magnesiumsulfat getrocknet und schließlich das Lösungsmittel unter verminderten Druck entfernt. Das Rohprodukt wurde durch Chromatographie an Kieselgel mit einem 4:1-Gemisch von Methylenchlorid und Essigester gereinigt. Es wurden 220 mg (92% d.Th.) (3RS,4RS)-4-(3-Bromphenyl)-3-hydroxy-piperidin-1-carbonsäure tert-butylester als farbloser Festkörper erhalten; MS: 299, 301 $(M-C_4H_8)^+$.

(g) Eine Lösung von 168 mg (0.47 mMol) (3RS,4RS)-4-(3-Bromphenyl)-3-hydroxy-piperidin-1-carbonsäure tert-butylester und 157 mg (0.71 mMol) 2-Brommethylnaphthalin in 2 ml Dimethylformamid wurde mit 28 mg (0.7 mMol) Natriumhydrid (60%ige Dispersion in Weißöl) versetzt und das Gemisch 3 Stunden lang bei Raumtemperatur gerührt. Anschließend wurde das Reaktionsgemisch zwischen Essigester und Wasser verteilt, die organische Phase über Magnesiumsulfat getrocknet und schließlich das Lösungsmittel unter verminderten Druck entfernt. Das Rohprodukt wurde durch Chromatographie an Kieselgel mit einem 1:4-Gemisch von Essigester und Hexan als Eluierungsmittel gereinigt. Es wurden 173 mg (74% d.Th.) (3RS,4RS)-4-(3-Bromphenyl)-3-(naphthalin-2-ylmethoxy)-piperidin-1-carbonsäure tert-butylester als leicht gelbes Öl erhalten; MS: 439, 441 $(M-C_4H_8)^+$.

(h) Eine Lösung von 173 mg (0.35 mMol) (3RS,4RS)-4-(3-Bromphenyl)-3-(naphthalin-2-ylmethoxy)-piperidin-1-carbonsäure tert-butylester in 6 ml Methanol wurde mit 6 ml einer 2 N Lösung von Chlorwasserstoff in MeOH versetzt und 4 Stunden bei 50° C gerührt. Anschließend wurde das Gemisch zwischen Essigester und einer 5%igen Natriumhydrogencarbonat-Lösung verteilt, die organische Phase über Magnesiumsulfat getrocknet und schließlich das Lösungsmittel unter verminderten Druck entfernt. Das Rohprodukt wurde durch Chromatographie an Kieselgel mit einem 10:1:0.1-Gemisch von Methylenchlorid, MeOH und 25%igem Ammoniak als Eluierungsmittel gereinigt. Es wurden 126 mg (91% d.Th.) (3RS,4RS)-4-(3-Bromphenyl)-3-(naphthalin-2-ylmethoxy)-piperidin als leicht gelbes Öl erhalten. MS: 396, 398 $(M+H)^+$.

Beispiel 2

In analoger Weise wie im Beispiel 1 (h) beschrieben, wurden durch Abspaltung der BOC-Gruppe die folgenden Verbindungen erhalten:

1) - Aus (3RS,4RS)-3-(4-Methoxy-benzyloxy)-4-phenyl-piperidin-1-carbonsäure tert-butylester das (3RS,4RS)-3-(4-Methoxy-benzyloxy)-4-phenyl-piperidin als leicht gelbes Öl; MS: 298 $(M+H)^+$;

2) - aus (3RS,4RS)-4-(4-Bromphenyl-3-(4-methoxy-benzyloxy)-piperidin-1-carbonsäure tert-butylester das (3RS, 4RS)-4-(4-Bromphenyl-3-(4-methoxy-benzyloxy)-piperidin als farloses Öl; MS: 376, 378 $(M+H)^+$;

3) - aus (3RS,4RS)-3-(4-Methoxy-benzyloxy)-4-(3-trifluormethylphenyl)-piperidin-1-carbonsäure tert-butylester das (3RS,4RS)-3-(4-Methoxy-benzyloxy)-4-(3-trifluormethylphenyl)-piperidin als farbloses Öl; MS: 366 $(M+H)^+$;

4) - aus (3RS,4RS)-3-(4-Methoxy-benzyloxy)-4-p-tolyl-piperidin-1-carbonsäure tert-butylester das (3RS,4RS)-3-(4-Methoxy-benzyloxy)-4-ptolyl-piperidin als farbloser Festkörper; MS: 312 $(M+H)^+$;

5) - aus (3RS,4RS)-3-(Naphthalin-2-ylmethoxy)-4-phenyl-piperidin-1-carbonsäure tert-butylester das (3RS,4RS)-3-(Naphthalin-2-ylmethoxy)-4-phenyl-piperidin als leicht gelbes Öl; MS: 318 $(M+H)^+$;

6) - aus (3RS,4RS)-4-(4-Bromphenyl)-3-(naphthalin-2-ylmethoxy)-piperidin-1-carbonsäure tert-butylester das (3RS,4RS)-4-(4-Bromphenyl)-3-(naphthalin-2-ylmethoxy)-piperidin als farbloser Festkörper; MS: 396, 398 $(M+H)^+$;

7) - aus (3RS,4RS)-3-(Naphthalin-2-ylmethoxy)-4-(3-trifluormethylphenyl)-piperidin-1-carbonsäure tert-butylester das (3RS,4RS)-3-(Naphthalin-2-ylmethoxy)-4-(3-trifluormethyl-phenyl)-piperidin als farbloses Öl; MS: 386 $(M+H)^+$;

8) - aus (3RS,4RS)-4-Cyclohexyl-3-(naphthalin-2-ylmethoxy)-piperidin-1-carbonsäure tert-butylester das (3RS, 4RS)-4-Cyclohexyl-3-(naphthalin-2-ylmethoxy)-piperidin als leicht gelbes Öl; MS: 324 $(M+H)^+$;

9) - aus (3RS,4RS)-3-(Naphthalin-2-ylmethoxy)-4-p-tolyl-piperidin-1-carbonsäure tert-butylester das (3RS,4RS)-3-(Naphthalin-2-ylmethoxy)-4-p-tolyl-piperidin als farbloser Festkörper; MS: 332 $(M+H)^+$;

10) - aus (3RS,4RS)-4-Naphthalin-2-yl-3-(naphthalin-2-ylmethoxy)-piperidin-1-carbonsäure tert-butylester das (3RS,4RS)-4-Naphthalin-2-yl-3-(naphthalin-2-ylmethoxy)-piperidin als farbloses Öl; MS: 367 $(M)^+$;

11) - aus (3RS,4RS)-3-(Naphthalin-2-ylmethoxy)-4-(5,6,7,8-tetrahydronaphthalin-2-yl)-piperidin-1-carbonsäure tert-butylester das (3RS,4RS)-3-(Naphthalin-2-ylmethoxy)-4-(5,6,7,8-tetrahydro-naphthalin-2-yl)-piperidin als farbloses Öl; MS: 372 $(M+H)^+$;

12) - aus (3RS,4RS)-4-Naphthalin-1-yl-3-(naphthalin-2-ylmethoxy)-piperidin-1-carbonsäure tert-butylester das (3RS,4RS)-4-Naphthalin-1-yl-3-(naphthalin-2-ylmethoxy)-piperidin als farbloser Festkörper; MS: 367 $(M)^+$;

13) - aus (3RS,4RS)-4-(3,4-Dimethoxy-phenyl)-3-(naphthalin-2-ylmethoxy)-piperidin-1-carbonsäure tert-butylester das (3RS,4RS)-4-(3,4-Dimethoxy-phenyl)-3-(naphthalin-2-ylmethoxy)-piperidin als farbloses Harz; MS: 377 $(M)^+$;

14) - aus (3RS,4RS)-4-Acenaphthen-5-yl-3-(naphthalin-2-ylmethoxy)-piperidin-1-carbonsäure tert-butylester das (3RS,4RS)-4-Acenaphthen-5-yl-3-(naphthalin-2-ylmethoxy)-piperidin als farbloser Festkörper; MS: 394 $(M+H)^+$;

15) - aus (3RS,4RS)-4-(4-Chlorphenyl)-3-(3-phenoxy-benzyloxy)-piperidin-1-carbonsäure tert-butylester das (3RS,4RS)-4-(4-Chlorphenyl)-3-(3-phenoxy-benzyloxy)-piperidin als farbloser Festkörper; MS: 394, 396 $(M+H)^+$;

16) - aus (3RS,4SR)-3-(Naphthalin-2-ylmethoxy)-4-phenyl-piperidin-1-carbonsäure tert-butylester das (3RS, 4SR)-3-(Naphthalin-2-ylmethoxy)-4-phenyl-piperidin Hydrochlorid als farbloses Pulver; MS: 318 $(M+H)^+$.

Die als Ausgangstoffe eingesetzten BOC-Verbindungen wurden wie folgt hergestellt:

In analoger Weise wie in Beispiel 1 (b)-(c) und (f)-(g) beschrieben, wurden die folgenden Verbindungen erhalten:

(a) Aus 4-Phenyl-piperidin-4-ol wurde durch Elimination das 4-Phenyl-1,2,3,6-tetrahydro-pyridin als leicht gelbes Öl erhalten; MS: 159 $(M)^+$. Die anschließende Hydroborierung ergab das (3RS,4RS)-4-Phenylpiperidin-3-ol als farblosen Festkörper; MS: 177 $(M)^+$. Die Einführung der BOC-Gruppe lieferte den (3RS,4RS)-3-Hydroxy-4-phenyl-piperidin-1-carbonsäure tert-butylester als farblosen Festkörper; MS: 277 $(M)^+$. Nach der Alkylierung mit 4-Methoxybenzylbromid wurde der (3RS,4RS)-3-(4-Methoxy-benzyloxy)-4-phenyl-piperidin-1-carbonsäure tert-butylester als farbloser Festkörper erhalten; MS: 340 $(M-C_4H_9)^+$.

(b) Aus 4-(4-Bromphenyl)-piperidin-4-ol wurde durch Elimination das 4-(4-Bromphenyl)-1,2,3,6-tetrahydro-pyridin als leicht gelber Festkörper erhalten; MS: 237, 239 $(M)^+$. Die anschließende Hydroborierung ergab das (3RS,4RS)-4-(4-Bromphenyl)-piperidin-3-ol als farblosen Festkörper; MS: 255, 257 $(M)^+$. Einführung der BOC-Gruppe lieferte den (3RS,4RS)-4-(4-Bromphenyl)-3-hydroxy-piperidin-1-carbonsäure tert-butylester als farblosen Festkörper; MS: 299, 301 $(M-C_4H_8)^+$. Nach der Alkylierung mit 4-Methoxybenzylbromid wurde der (3RS,4RS)-4-(4-Bromphenyl)-3-(4-Methoxy-benzyloxy)-piperidin-1-carbonsäure tert-butylester als leicht gelbes Öl erhalten; MS: 418, 420 $(M-C_4H_9)^+$.

(c) Aus 4-(3-Trifluormethylphenyl)-piperidin-4-ol wurde durch Elimination das 4-(3-Trifluormethylphenyl)-1,2,3,6-tetrahydro-pyridin als farbloser Festkörper erhalten; MS: 227 $(M)^+$. Die anschließende Hydroborierung ergab das (3RS,4RS)-4-(3-Trifluormethylphenyl)-piperidin-3-ol als farblosen Festkörper; MS: 245 $(M)^+$. Die Einführung der BOC-Gruppe lieferte den (3RS,4RS)-3-Hydroxy-4-(3-trifluormethylphenyl)-piperidin-1-carbonsäure tert-butylester als farblosen Festkörper; MS: 289 $(M-C_4H_8)^+$. Nach der Alkylierung mit 4-Methoxybenzylbromid wurde der (3RS,4RS)-3-(4-Methoxy-benzyloxy)-4-(3-trifluormethylphenyl)-piperidin-1-carbonsäure tert-butylester als leicht gelbes Öl erhalten; MS: 408 $(M-C_4H_9)^+$.

(d) Aus 1-Benzyl-4-(p-tolyl)-piperidin-4-ol wurde durch Elimination das 1-Benzyl-4-(p-tolyl)-1,2,3,6-tetrahydro-pyridin als leicht gelber Festkörper erhalten; MS: 263 $(M)^+$. Die anschließende Hydroborierung ergab das (3RS,4RS)-1-Benzyl-4-(p-tolyl)-piperidin-3-ol als farblosen Festkörper; MS: 281 $(M)^+$.

(e) Eine Lösung von 2.5 g (8.9 mMol) (3RS,4RS)-1-Benzyl-4-(p-tolyl)-piperidin-3-ol in 100 ml Methanol wurde bei Raumtemperatur während 18 Stunden unter Verwendung eines Palladium(10%ig)-Kohle-Katalysators bei 5 bar hydriert. Zur Aufarbeitung wurde der Katalysator abfiltriert, mit Methanol gewaschen und die erhaltene Lösung unter vermindertem Druck eingedampft. Der Rückstand wurde zur Reinigung an Kieselgel unter Verwendung eines 5:1:0.1-Gemisches von Methylenchlorid, Methanol und Ammoniak als Eluierungsmittel chromatographiert. Es wurden 1.15 g (68% d.Th.) (3RS,4RS)-4-(p-tolyl)-piperidin-3-ol als farbloser Festkörper erhalten; MS: 191 $(M)^+$.

(f) Durch Einführung der BOC-Gruppe wurde aus dem (3RS,4RS)-4-(ptolyl)-piperidin-3-ol der (3RS,4RS)-3-Hydroxy-4-(p-tolyl)-piperidin-1-carbonsäure tert-butylester als farbloser Festkörper erhalten; MS: 291 $(M)^+$. Nach der Alkylierung 4-Methoxybenzylbromid wurde der (3RS,4RS)-3-(4-Methoxy-benzyloxy)-4-(p-tolyl)-piperidin-1-carbonsäure tert-butylester als farbloses Öl erhalten; MS: 354 $(M-C_4H_9)^+$.

In analoger Weise wie in Beispiel 1 (g) beschrieben, wurden die folgenden Verbindungen erhalten:

(g) Durch Alkylierung des (3RS,4RS)-3-Hydroxy-4-phenyl-piperidin-1-carbonsäure tert-butylesters mit 2-Brommethylnaphthalin wurde der (3RS,4RS)-3-(Napththalin-2-ylmethoxy)-4-phenyl-piperidin-1-carbonsäure tert-butylester als leicht gelbes Öl erhalten; MS: 417 $(M)^+$.

(h) Durch Alkylierung des (3RS,4RS)-4-(4-Bromphenyl)-3-hydroxypiperidin-1-carbonsäure tert-butylesters mit 2-Brommethyl-naphthalin wurde der (3RS,4RS)-4-(4-Bromphenyl)-3-(naphthalin-2-ylmethoxy)-piperidin-1-carbonsäure tert-butylester als farbloser Festkörper erhalten; MS: 495, 497 $(M)^+$.

(i) Durch Alkylierung des (3RS,4RS)-3-Hydroxy-4-(3-Trifluormethylphenyl)-piperidin-1-carbonsäure tert-butylesters mit 2-Brommethyl-naphthalin wurde der (3RS,4RS)-3-(Naphthalin-2-ylmethoxy)-4-(3-trifluormethyl-phenyl)-piperidin-1-carbonsäure tert-butylester als leicht gelbes Öl erhalten; MS: 485 $(M)^+$.

(j) Eine Lösung von 4.0 g (13.8 mMol) (3RS,4RS)-3-Hydroxy-4-phenylpiperidin-1-carbonsäure tert-butylesters in 100 ml Methanol wurde bei 100° C während 18 Stunden unter Verwendung eines Rhodium(5%ig)-Aluminiumoxid-Katalysators bei 150 bar hydriert. Zur Aufarbeitung wurde der Katalysator abfiltriert, mit Methanol gewaschen und

die erhaltene Lösung unter vermindertem Druck eingedampft. Der Rückstand wurde zur Reinigung an Kieselgel unter Verwendung eines 4:1-Gemisches von Hexan und Essigester als Eluierungsmittel chromatographiert. Es wurden 2.32 g (59% d.Th.) (3RS,4RS)-4-Cyclohexyl-3-(naphthalin-2-ylmethoxy)-piperidin-1-carbonsäure tert-butylester als farbloser Festkörper erhalten; MS: 283 $(M)^+$.

(k) Durch Alkylierung des (3RS,4RS)-4-Cyclohexyl-3-(naphthalin-2-ylmethoxy)-piperidin-1-carbonsäure tert-butylesters mit 2-Brommethyl-naphthalin wurde der (3RS,4RS)-4-Cyclohexyl-3-(naphthalin-2-ylmethoxy)-piperidin-1-carbonsäure tert-butylester als leicht gelbes Öl erhalten; MS: 423 $(M)^+$.

(l) Durch Alkylierung des (3RS,4RS)-3-Hydroxy-4-(p-tolyl)-piperidin-1-carbonsäure tert-butylesters mit 2-Brommethylnaphthalin wurde der (3RS,4RS)-3-(Naphthalin-2-ylmethoxy)-4-p-tolyl-piperidin-1-carbonsäure tert-butylester als farbloses Öl erhalten; MS: 431 $(M)^+$.

Die weiteren Ausgangsstoffe wurden wie folgt erhalten:

(m) Aus 2-Bromnaphthalin und 1-Benzyl-4-piperidon wurde in analoger Weise zu Beispiel 1 (a) das 1-Benzyl-4-naphthalin-2-ylpiperidin-4-ol als leicht gelbes Öl erhalten; MS: 317 (M)+. Die Elimination, in analoger Weise wie im Beispiel 1 (b) beschrieben, lieferte das 1-Benzyl-4-naphthalin-2-yl-1,2,3,6-tetrahydro-pyridin als leicht braunes Öl; MS: 299 $(M)^+$. Die folgende Abspaltung der Benzylgruppe, analog zu Beispiel 1 (d), ergab den 4-Naphthalin-2-yl-1,2,3,6-tetrahydro-pyridin-1-carbonsäure 2-trimethylsilyläthylester als farblosen Festkörper; MS: 325 $(M-C_2H_4)^+$. Durch Behandlung mit Tetrabutylammoniumfluorid in Tetrahydrofuran, analog zu Beispiel 1 (e) wurde das 4-Naphthalin-2-yl-1,2,3,6-tetrahydro-pyridin als farbloser Festkörper erhalten; MS: 209 $(M)^+$. Die anschließende Hydroborierung, in analoger Weise wie Beispiel 1 (c) beschrieben, ergab das (3RS,4RS)-Naphthalin-2-yl-4-piperidin-3-ol als farblosen Festkörper; MS: 227 $(M)^+$. Die Einführung der BOC-Gruppe, in Analogie zu Beispiel 1 (f), lieferte den (3RS,4RS)-3-Hydroxy-4-naphthalin-2-yl-piperidin-1-carbonsäure tert-butylester als farbloses Öl; MS: 327 $(M)^+$. Nach der Alkylierung mit 2-Brommethylnaphthalin, in analoger Weise zu dem im Beispiel 1 (g) beschriebenen Verfahren, wurde der (3RS,4RS)-4-Naphthalin-2-yl-3-(naphthalin-2-ylmethoxy)-piperidin-1-carbonsäure tert-butylester als farbloses Öl erhalten; MS: 467 $(M)^+$.

(n) In analoger Weise wie in Beispiel 2 (e) beschrieben, wurde durch katalytische Hydrierung von (3RS,4RS)-1-Benzyl-4-naphthalin-2-ylpiperidin-3-ol das (3RS,4RS)-4-(5,6,7,8-Tetrahydro-naphthalin-2-yl)-piperidin-3-ol als farbloser Festkörper erhalten; MS: 231 $(M)^+$. Die Einführung der BOC-Gruppe, in analoger Weise wie Beispiel 1 (f) beschrieben, lieferte den (3RS,4RS)-3-Hydroxy-4-(5,6,7,8-tetrahydronaphthalin-2-yl)-piperidin-1-carbonsäure tert-butylester als farbloses Öl; MS: 331 $(M)^+$. Nach der Alkylierung mit 2-Brommethylnaphthalin, in analoger Weise zu dem im Beispiel 1 (g) beschriebenen Verfahren, wurde der (3RS,4RS)-3-(Naphthalin-2-ylmethoxy)-4-(5,6,7,8-tetrahydronaphthalin-2-yl)-piperidin-1-carbonsäure tert-butylester als farbloses Öl erhalten; MS: 471 $(M)^+$.

(o) Aus 1-Benzyl-4-naphthalin-1-yl-1,2,3,6-tetrahydro-pyridin (EP-A-372 776) wurde durch Hydroborierung, in analoger Weise wie Beispiel 1(c) beschrieben, das (3RS,4RS)-1-Benzyl-4-naphthalin-1-yl-piperidin-3-ol als farbloser Festkörper erhalten; MS: 317 $(M)^+$. In analoger Weise wie in Beispiel 2 (e) beschrieben, wurde die Benzylgruppe durch katalytische Hydrierung [Palladium(10%)-Kohle, Äthanol, 80° C, 24 Stunden, 50 bar, 21% d.Th.] entfernt. Das (3RS,4RS)-4-Naphthalin-1-yl-piperidin-3-ol wurde als beiger Festkörper erhalten; MS: 227 $(M)^+$. Die Einführung der BOC-Gruppe, in analoger Weise wie Beispiel 1 (f) beschrieben, lieferte den (3RS,4RS)-3-Hydroxy-4-naphthalin-1-yl-piperidin-1-carbonsäure tert-butylester als farblosen Festkörper; MS: 327 $(M)^+$. Nach der Alkylierung mit 2-Brommethylnaphthalin, in Analogie zu dem im Beispiel 1 (g) beschriebenen Verfahren, wurde der (3RS,4RS)-4-Naphthalin-1-yl-3-(naphthalin-2-ylmethoxy)-piperidin-1-carbonsäure tert-butylester als farbloser Festkörper erhalten; MS: 467 $(M)^+$.

(p) Aus 1-Benzyl-4-(3,4-dimethoxy-phenyl)-1,2,3,6-tetrahydro-pyridin (JP 60 146 872) wurde durch Hydroborierung, in analoger Weise wie Beispiel 1 (c) beschrieben, das (3RS,4RS)-1-Benzyl-(3,4-dimethoxyphenyl)-piperidin-3-ol als farbloser Festkörper erhalten; MS: 327 $(M)^+$. In analoger Weise wie in Beispiel 2 (e) beschrieben, wurde die Benzylgruppe durch katalytische Hydrierung [Palladium(10%)-Kohle, Methanol, Raumtemperatur, 18 Stunden, 5 bar, 81% d.Th.] entfernt. Das (3RS,4RS)-4-(3,4-Dimethoxy-phenyl)-piperidin-3-ol wurde als farbloser Festkörper erhalten; MS: 237 $(M)^+$. Die Einführung der BOC-Gruppe, in analoger Weise wie Beispiel 1(f) beschrieben, lieferte den (3RS,4RS)-3-Hydroxy-4-(3,4-dimethoxy-phenyl)-piperidin-1-carbonsäure tert-butylester als leicht gelbes Öl; MS: 337 $(M)^+$. Nach der Alkylierung mit 2-Brommethylnaphthalin, in Analogie zu dem im Beispiel 1 (g) beschriebenen Verfahren, wurde der (3RS,4RS)-4-(3,4-Dimethoxyphenyl)-3-(naphthalin-2-ylmethoxy)-piperidin-1-carbonsäure tert-butylester als leicht gelbes Öl erhalten; MS: 477 $(M)^+$.

(q) Aus 5-Brom-acenaphten und 1-Benzyl-4-piperidon wurde in analoger Weise zu Beispiel 1 (a) das 4-Acenaphten-5-yl-1-benzylpiperidin-4-ol als gelbes Öl erhalten; MS: 343 $(M)^+$. Die Elimination, in analoger Weise wie im Beispiel 1 (b) beschrieben, lieferte das 4-Acenaphten-5-yl-1-benzyl-1,2,3,6-tetrahydro-pyridin als leicht braunes Öl; MS: 325 $(M)^+$. Die anschließende Hydroborierung, in analoger Weise wie Beispiel 1 (c) beschrieben, ergab das (3RS,4RS)-1-Benzylacenaphten-5-yl-4-piperidin-3-ol als gelbes Öl; MS: 343 $(M)^+$. In analoger Weise wie in Beispiel 2 (e) beschrieben, wurde die Benzylgruppe durch katalytische Hydrierung [Palladium(10%)-Kohle, Methanol, Raumtemperatur, 18 Stunden, 5 bar, 95% d.Th.] entfernt. Das (3RS,4RS)-4-Acenaphten-5-yl-piperidin-3-ol wurde als farbloser Festkörper erhalten; MS: 253 $(M)^+$. Die Einführung der BOC-Gruppe, in analoger Weise wie Beispiel 1 (f) beschrieben, lieferte den (3RS,4RS)-4-Acenaphten-5-yl-3-hydroxy-piperidin-1-carbonsäure tert-butylester als farblosen Festkörper; MS: 353 $(M)^+$. Nach der Alkylierung mit 2-Brommethylnaphthalin, in Analogie zu dem im Beispiel 1(g) beschriebenen Verfahren, wurde der (3RS,4RS)-4-Acenaphten-5-yl-3-(naphthalin-2-ylmethoxy)-piperidin-1-carbonsäure tert-butylester als gelber Festkörper erhalten; MS: 493 $(M)^+$.

(r) Aus 4-(4-Chlorphenyl)-piperidin-4-ol wurde durch Elimination, in analoger Weise wie im Beispiel 1 (b) beschrieben, das 4-(4-Chlorphenyl)-1,2,3,6-tetrahydro-pyridin als leicht gelber Festkörper erhalten; MS: 193, 195 $(M)^+$. Die Hydroborierung in analoger Weise wie Beispiel 1 (c) ergab das (3RS,4RS)-4-(4-Chlorphenyl)-piperidin-3-ol als farblosen Festkörper; MS: 211, 213 $(M)^+$. Die Einführung der BOC-Gruppe, in analoger Weise wie Beispiel 1 (f) beschrieben, lieferte den (3RS,4RS)-4-(4-Chlorphenyl)-3-hydroxy-piperidin-1-carbonsäure tert-butylester als farblosen Festkörper; MS: 255, 257 $(M-C_4H_8)^+$. Nach der Alkylierung mit 4-Phenoxybenzylchlorid, in Analogie zu dem im Beispiel 1 (g) beschriebenen Verfahren, wurde der (3RS,4RS)-4-(4-Chlorphenyl)-3-(4-Phenoxy-benzyloxy)-piperidin-1-carbonsäure tert-butylester als farbloses Öl erhalten; MS: 437, 439 $(M-C_4H_9)^+$.

(s) Durch Einführung der BOC-Gruppe wurde aus (3RS,4SR)-4-Phenylpiperidin-3-ol [J.A.Gauthier et al., US 4132710] der (3RS,4SR)-3-Hydroxy-4-phenyl-piperidin-1-carbonsäure tert-butylester als farbloser Festkörper erhalten; Smp.: 134-134.5 °C. Die anschließende Alkylierung mit 2-Brommethylnaphthalin ergab den (3RS,4SR)-3-(Naphthalin-2-ylmethoxy)-4-phenyl-piperidin-1-carbonsäure tert-butylester als farblosen Festkörper; MS: 417 $(M)^+$.

Beispiel 3

130 mg (0.31 mMol) (3RS,4RS)-4-(4-Fluorphenyl)-3-(4-methoxybenzyloxy)-piperidin-1-carbonsäure tert-butylester wurden in 5 ml Methanol gelöst, mit 5 ml einer 2N Lösung von Chlorwasserstoff in Methanol versetzt und 4 Stunden bei 50° C gerührt. Anschließend wurde das Gemisch zwischen Essigester und wäßriger 5%iger Natriumhydrogencarbonat-Lösung verteilt, die organische Phase über Magnesiumsulfat getrocknet und schließlich das Lösungsmittel unter verminderten Druck abdestilliert. Zur Reinigung wurde das Rohprodukt an Kieselgel mit einem 10:1:0.1-Gemisch von Methylenchlorid, Methanol und Ammoniak als Eluierungsmittel chromatographiert. Es wurden 76 mg (78% d.Th.) (3RS,4RS)-4-(4-Fluorphenyl)-3-(4-methoxy-benzyloxy)-piperidin als farbloses Öl erhalten. MS: 316 $(M+H)^+$.

Der als Ausgangstoff eingesetzte (3RS,4RS)-4-(4-Fluorphenyl)-3-(4-methoxy-benzyloxy)-piperidin-1-carbonsäure tert-butylester wurde wie folgt hergestellt:

(a) 20.0 g (93.6 mMol) 4-(4-Fluorphenyl)-1,2,3,6-tetrahydropyridin Hydrochlorid wurden in 160 ml absolutem Dimethoxyäthan suspendiert. Es wurden portionsweise bei Raumtemperatur 10.6 g (280 mMol) Natriumborhydrid zugegeben. Danach wurde bei 15-20° C eine Lösung von 62 ml (500 mMol) Bortrifluorid-Ätherat in 30 ml Dimethoxyäthan tropfenweise zugegeben und 2.5 Stunden bei Raumtemperatur gerührt. Anschließend wurde bei 20-25° C eine Lösung von 65 g (1.16 Mol) Kaliumhydroxid in 340 ml Wasser innerhalb von 60 Minuten zugetropft. Es wurden dann bei 20-25° C innerhalb von 30 Minuten 55 ml Wasserstoffperoxid-Lösung (30%ig) tropfenweise zugegeben. Das Gemisch wurde 30 Minuten bei Raumtemperatur gerührt und 3 Stunden unter Rückfluß gekocht. Nach dem Abkühlen des Reaktionsgemisches wurde die ausgefallene Borsäure abfiltriert. Anschließend wurde das Filtrat zwischen Methylenchlorid und Wasser verteilt, die organische Phase über Magnesiumsulfat getrocknet und das Lösungsmittel unter verminderten Druck abdestilliert. Das Rohprodukt wurde durch Chromatographie an Kieselgel mit einem 3:1:0.1-Gemisch von Methylenchlorid, Methanol und Ammoniak als Eluierungsmittel gereinigt. Es wurden 9.1 g (50% d.Th.) (3RS,4RS)-4-(4-Fluorphenyl)-piperidin-3-ol als farbloses Öl erhalten. MS: 195 $(M)^+$.

(b) 4.10 g (21.0 mMol) (3RS,4RS)-4-(4-Fluorphenyl)-piperidin-3-ol wurden in 35 ml absolutem Dimethylformamid gelöst. Dazu wurden bei 0° C 3.2 ml (23.0 mMol) Triäthylamin und anschließend tropfenweise eine Lösung von 5.04 g (23.1 mMol) Di-tert-butyl-dicarbonat in 15 ml Dimethylformamid gegeben. Das Gemisch wurde auf Raumtemperatur erwärmt und 20 Stunden gerührt. Das Lösungsmittel wurde bei 0.1 mm Hg bei 50-55° C abdestilliert.

Anschließend wurde der erhaltene Rückstand zwischen Methylenchlorid und Wasser verteilt, die organische Phase über Magnesiumsulfat getrocknet und schließlich das Lösungsmittel unter verminderten Druck entfernt. Das Rohprodukt (7.09 g) wurde durch Chromatographie an Kieselgel mit einem 2:3-Gemisch von Essigester und Hexan als Eluierungsmittel gereinigt. Es wurden 5.45 mg (88% d. Th.) (3RS,4RS)-4-(4-Fluorphenyl)-3-hydroxypiperidin-1-carbonsäure tert-butylester erhalten; MS: 239 $(M - C_4H_8)^+$.

(c) 200 mg (0.68 mMol) (3RS,4RS)-4-(4-Fluorphenyl)-3-hydroxy-piperidin-1-carbonsäure tert-butylester und 159 mg (1.01 mMol) 4-Methoxybenzylchlorid wurden in 3 ml Dimethylformamid gelöst. Es wurden 40 mg (1.01 mMol) einer 60%igen Natriumhydridsuspension zugegeben, und das Gemisch wurde 3 Stunden bei Raumtemperatur gerührt. Anschließend wurde das Reaktionsgemisch zwischen Essigester und Wasser verteilt, die organische Phase über Magnesiumsulfat getrocknet und schließlich das Lösungsmittel unter verminderten Druck entfernt. Das Rohprodukt wurde durch Chromatographie an Kieselgel mit einem 1:3-Gemisch von Essigester und Hexan als Eluierungsmittel gereinigt. Es wurden 250 mg (90% d.Th.) (3RS,4RS)-4-(4-Fluorphenyl)-3-(4-methoxy-benzyloxy)-piperidin-1-carbonsäure tert-butylester als leicht gelbes Öl erhalten; MS: 358 $(M - C_4H_9)^+$.

Beispiel 4

In analoger Weise wie in Beispiel 3 beschrieben, wurden die folgenden Verbindungen hergestellt:

1) - Aus (3RS,4RS)-4-(4-Fluorphenyl)-3-(naphthalin-2-yl-methoxy)-piperidin-1-carbonsäure tert-butylester das (3RS,4RS)- 4-(4-Fluorphenyl)-3-(naphthalin-2-ylmethoxy)- piperidin als farbloses Öl; MS: 336 $(M+1)^+$;

2) - aus (3RS,4RS)-3-(3-Benzyloxy-benzyloxy)-4-(4-fluorphenyl)-piperidin-1-carbonsäure tert-butylester das (3RS,4RS)-3-(3-Benzyloxybenzyloxy)-4-(4-fluorphenyl)-piperidin als farbloser Festkörper; MS: 392 $(M+1)^+$;

3) - aus (3RS,4RS)-4-(4-Fluorphenyl)-3-(4-methoxy-chinazolin-2-ylmethoxy)-piperidin-1-carbonsäure tert-butylester das (3RS,4RS)-4-(4-Fluorphenyl)-3-(4-methoxy-chinazolin-2-ylmethoxy)-piperidin als farbloser Festkörper; MS: 368 $(M+1)^+$;

4) - aus (3RS,4RS)-3-(Benzo[b]thiophen-5-ylmethoxy)-4-(4-fluorphenyl)-piperidin-1-carbonsäure tert-butylester das (3RS,4RS)-3-(Benzo[b]thiophen-5-ylmethoxy)-4-(4-fluor-phenyl)-piperidin als farbloser Festkörper; MS: 342 $(M+1)^+$;

5) - aus (3RS,4RS)-4-(4-Fluorphenyl)-3-(indan-5-yl-methoxy)-piperidin-1-carbonsäure tert-butylester das (3RS, 4RS)-4-(4-Fluorphenyl)-3-(indan-5-ylmethoxy)-piperidin als farbloser Festkörper; MS: 326 $(M+1)^+$;

6) - aus (3RS,4RS)-4-(4-Fluorphenyl)-3-(5,6,7,8-tetra-hydronaphthalin-2-ylmethoxy)-piperidin-1-carbonsäure tert-butylester das (3RS,4RS)-4-(4-Fluor-phenyl)-3-(5,6,7,8-tetrahydro-naphthalin-2-ylmethoxy)-piperidin als farbloser Festkörper; MS: 340 $(M+1)^+$;

7) - aus (3RS,4RS)-4-(4-Fluor-phenyl)-3-(isochinolin-6-ylmethoxy)-piperidin-1-carbonsäure tert-butylester das (3RS,4RS)-4-(4-Fluorphenyl)-3-(isochinolin-6-ylmethoxy)-piperidin als farbloser Festkörper erhalten; MS: 337 $(M+1)^+$.

Die als Ausgangsstoffe eingesetzten BOC-Derivate wurden in analoger Weise zu dem in Beispiel 3 (c) beschriebenen Alkylierungsverfahren wie folgt erhalten:

- Aus (3RS,4RS)-4-(4-Fluorphenyl)-3-hydroxy-piperidin-1- carbonsäure tert-butylester und 2-Naphthylmethylbromid der (3RS,4RS)-4-(4-Fluorphenyl)-3-(naphthalin-2-yl-methoxy)-piperidin-1-carbonsäure tert-butylester als leicht gelbes Öl; MS: 436 $(M+1)^+$;

- aus (3RS,4RS)-4-(4-Fluorphenyl)-3-hydroxy-piperidin-1- carbonsäure tert-butylester und 3-Benzyloxy-benzylchlorid [J. Med. Chem. 31(3), 606 (1988)] der (3RS,4RS)-3-(3-Benzyloxy-benzyloxy)-4-(4-fluorphenyl)-piperidin-1-carbonsäure tert-butylester als farbloser Festkörper; MS: 492 $(M+1)^+$;

- aus (3RS,4RS)-4-(4-Fluorphenyl)-3-hydroxy-piperidin 1-carbonsäure tert-butylester und 2-Brommethyl-4-methoxy-chinazolin der (3RS,4RS)-4-(4-Fluorphenyl)-3-(4-methoxy-chinazolin-2-ylmethoxy)-piperidin-1-carbonsäure tert-butylester als farbloser Festkörper; MS: 492 $(M+1)^+$;

- aus (3RS,4RS)-4-(4-Fluorphenyl)-3-hydroxy-piperidin-1-carbonsäure tert-butylester und 5-Brommethyl-benzo[b]thiophen [J. Med. Chem. 34(1), 65(1991)] der (3RS,4RS)-3-(Benzo[b]thiophen-5-ylmethoxy)-4-(4-fluor-phenyl)-piperidin-1-carbonsäure tert-butylester als leicht gelbes Harz; MS: 442 $(M+1)^+$;

- aus (3RS,4RS)-4-(4-Fluorphenyl)-3-hydroxy-piperidin-1-carbonsäure tert-butylester und 5-Chlormethylindan [Recl. Trav. Chim. Pays-Bas 77, 792 (1988)] der (3RS,4RS)-4-(4-Fluorphenyl)-3-(indan-5-ylmethoxy)-piperidin-1-carbonsäure tert-butylester als farbloser Festkörper; MS: 426 $(M+1)^+$;

- aus (3RS,4RS)-4-(4-Fluorphenyl)-3-hydroxy-piperidin-1-carbonsäure tert-butylester und 6-Chlormethyl-1,2,3,4-tetrahydro-naphthalin [J. Chem. Soc. 684(1941)] der (3RS,4RS)-4-(4-Fluorphenyl)-3-(5,6,7,8-tetrahydro-naphthalin-2-ylmethoxy)-piperidin-1-carbonsäure tert-butylester als leicht gelbes Harz; MS: 440 $(M+1)^+$;

- aus (3RS,4RS)-4-(4-Fluorphenyl)-3-hydroxy-piperidin-1-carbonsäure tert-butylester und 6-Brommethylisochinolin Hydrobromid der (3RS,4RS)-4-(4-Fluor-phenyl)-3-(isochinolin-6-ylmethoxy)-piperidin-1-carbonsäure tert-butylester als leicht gelbes Harz; MS: 437 $(M+1)^+$.

Die folgenden, als Alkylierungsmittel eingesetzten Verbindungen wurden wie folgt hergestellt:

2-Brommethyl-4-methoxy-chinazolin

(a) In analoger Weise zu dem für die Herstellung von 6-Brommethyl-chinoxalin [J. Het. Chem. 11, 595(1974)] aus 6-Methylchinoxalin beschriebenen Verfahren, wurde durch Bromierung von 2-Methyl-4-methoxy-chinazolin [Recl. Trav. Chim. Pays-Bas 76, 401 (1957)] mit N-Bromsuccinimid in Tetrachlorkohlenstoff das 2-Brommethyl-4-methoxy-chinazolin als leicht gelber Festkörper erhalten; MS: 252, 254 $(M)^+$

6-Brommethyl-isochinolin Hydrobromid

(b) Aus Isochinolin-6-carbonsäure [J.Am.Chem.Soc. 61, 183(1939)] wurde durch Veresterung mit Äthanol/Schwefelsäure der Isochinolin-6-carbonsäureäthylester als farbloser Festkörper erhalten; MS: 201 $(M)^+$. Die folgende Reduktion lieferte das 6-Isochinolin-methanol als gelben Festkörper, der direkt in die nächste Stufe eingesetzt wurde.

(c) Eine Lösung von 190 mg (1.19 mMol) 6-Isochinolin-methanol in 1 ml Eisessig wurde mit 2 ml 30%iger HBr in Eisessig versetzt und 45 Minuten bei 70°C erhitzt. Das Reaktionsgemisch wurde abgekühlt, mit 20 ml Diäthyläther versetzt und 30 Min bei 0°C gerührt. Der entstandene Festkörper wurde abfiltriert, mit Diäthyläther gewaschen und am Hochvakuum getrocknet. Es wurde das 6-Brommethylisochinolin Hydrobromid (73% d. Th.) als leicht brauner Festkörper erhalten; MS: 221, 223 $(M)^+$.

Beispiel 5

70 mg (0.141 mMol) (3RS,4RS)-4-(4-Fluorphenyl)-3-(4-hydroxynaphthalin-2-ylmethoxy)-piperidin-1-carbonsäure β-trimethylsilyläthylester wurden in 1.0 ml Tetrabutylammoniumfluorid-Lösung (1M in Tetrahydrofuran) gelöst und eine Stunde bei Raumtemperatur gerührt. Anschließend wurde das Gemisch zwischen Methylenchlorid und wäßriger 5%iger Natriumhydrogencarbonat-Lösung verteilt, dann die organische Phase über Magnesiumsulfat getrocknet und schließlich das Lösungsmittel unter verminderten Druck abdestilliert. Das Rohprodukt (72 mg) wurde durch Chromatographie an Kieselgel mit einem 10:1:0.1-Gemisch von Methylenchlorid, Methanol und Ammoniak als Eluierungsmittel gereinigt. Es wurden 41 mg (83% d.Th.) (3RS,4RS)-4-(4-Fluorphenyl)-3-(4-hydroxy-naphthalin-2-ylmethoxy)-piperidin als farbloser Festkörper erhalten. MS: 352 $(M+H)^+$.

Der als Ausgangstoff eingesetzte (3RS,4RS)-4-(4-Fluorphenyl)-3-(4-hydroxy-naphthalin-2-ylmethoxy)-piperidin-1-carbonsäure β-trimethylsilyläthylester wurde wie folgt hergestellt:

(a) 17.87 g (82.64 mMol) 4-Hydroxy-naphthalin-2-carbonsäureäthylester [J.Agric.Chem Soc.Japan 24, 313 (1950)] wurden in 900 ml Methylenchlorid suspendiert, die Suspension auf 0-5° C abgekühlt und anschließend mit 17.9 ml (91.02 mMol) 2-(Trimethylsilyl)-äthoxymethylchlorid (SEM-Chlorid) und 28.3 ml (165.31 mMol) N-Äthyldiisopropylamin versetzt. Die gelbe Lösung wurde auf Raumtemperatur erwärmt und 2 Stunden gerührt. Das Lösungsmittel wurde unter verminderten Druck abdestilliert und das Rohprodukt (58 g) ohne weitere Aufarbeitung an Kieselgel unter Verwendung eines 3:2-Gemisches von Methylenchlorid und Hexan als Eluierungsmittel chromatographiert. Es wurden 15.81 g (99% d.Th.) 4-(2-Trimethylsilylethoxy-methoxy)-naphthalin-2-carbonsäureäthylester als leicht

gelbes Öl erhalten; MS: 322, 324 $(M)^+$.

(b) Zu einer Suspension von 3.29 g (86.69 mMol) Lithiumaluminiumhydrid in 230 ml Diäthyläther wurden unter Argon bei -5 bis 0° C eine Lösung von 28.31 g (81.70 mMol) 4-(2-Trimethylsilyl-äthoxymethoxy)-naphthalin-2-carbonsäureäthylester in 480 ml Diäthyläther innerhalb 90 Minuten tropfenweise zugegeben. Das Reaktionsgemisch wurde auf Raumtemperatur erwärmt und 2 Stunden gerührt. Zur Aufarbeitung wurde auf 0° C abgekühlt, tropfenweise mit 25 ml Essigester sowie 50 ml gesättigter Kalium-Natriumtartrat-Lösung versetzt. Es entstand eine leicht gelbliche Lösung mit weißem Niederschlag. Die Lösung wurde auf Raumtemperatur erwärmt, vom Niederschlag abdekantiert. Der Rückstand dreimal mit Diäthyläther aufgeschlämmt und jeweils das Lösungsmittel abdekantiert. Die vereinigten organischen Phasen wurden mit Wasser gewaschen, die organische Phase über Magnesiumsulfat getrocknet und schließlich das Lösungsmittel unter verminderten Druck entfernt. Das Rohprodukt (26.4 g) wurde an Kieselgel unter Verwendung eines 3:7-Gemisches von Essigester und Hexan chromatographiert. Es wurden 23.72 g (95% d.Th.) [4-(2-Trimethylsilyl-äthoxymethoxy)-naphthalin-2-yl]-methanol als leicht gelbes Öl erhalten; MS: 304 $(M)^+$.

(c) 23.72 g (77.91 mMol) [4-(2-Trimethylsilyl-äthoxymethoxy)-naphthalin-2-yl]-methanol wurden in 350 ml Tetrachlorkohlenstoff gelöst und die Lösung auf 0° C abgekühlt. Daraufhin wurden 350 ml Acetonitril und 26.54 g (101.2 mMol) Triphenylphosphin zugegeben. Die leicht gelbe Lösung wurde 30 Minuten bei 0° C gerührt, auf Raumtemperatur erwärmt und 2 Stunden weiter gerührt. Es wurden weitere 10.14 g (38.7 mMol) Triphenylphosphin zugegeben und das Reaktionsgemisch 90 Minuten bei Raumtemperatur nachgerührt. Anschließend wurde das Gemisch zwischen Methylenchlorid und Wasser verteilt, die organische Phase über Magnesiumsulfat getrocknet und schließlich das Lösungsmittel unter verminderten Druck entfernt. Das Rohprodukt wurde an Kieselgel unter Verwendung eines 3:7-Gemisches von Methylenchlorid und Hexan als Eluierungsmittel chromatographiert. Es wurden 15.81 g (63% d.Th.) 2-Chlormethyl-4-(β-trimethylsilyläthoxymethoxy)-naphthalin als leicht gelbes Öl erhalten; MS: 322, 324 $(M)^+$.

(d) Eine Lösung von 4.00 g (20.5 mMol) (3RS,4RS)-4-(4-Fluorphenyl)-piperidin-3-ol in 150 ml Äthanol wurde mit 2.80 g (26.4 mMol) Natriumcarbonat versetzt und zum Rückfluß gebracht. Es wurde innerhalb einer Stunde eine Lösung von 2.50 ml (21.1 mMol) Benzylbromid in 50 ml Äthanol tropfenweise zugegeben und danach 2 Stunden bei Rückflußtemperatur gehalten. Die blaßbräunliche Suspension wurde filtriert und das Filtrat unter vermindertem Druck aufkonzentriert. Anschließend wurde der Rückstand zwischen Methylenchlorid und Wasser verteilt, die organische Phase über Magnesiumsulfat getrocknet und schließlich das Lösungsmittel unter vermindertem Druck abdestilliert. Das Rohprodukt wurde an Kieselgel unter Verwendung eines 2:3-Gemisches von Essigester und Hexan als Eluierungsmittel chromatographiert. Es wurden 4.34 g (74% d.Th.) (3RS,4RS)-1-Benzyl-4-(4-fluor-phenyl)-piperidin-3-ol als farbloser Festkörper erhalten; MS: 285 $(M)^+$.

(e) In analoger Weise wie in Beispiel 1 (g) beschrieben, wurde durch Alkylierung von (3RS,4RS)-1-Benzyl-4-(4-fluoro-phenyl)-piperidin-3-ol mit 2-Chlormethyl-4-(β-trimethylsilyläthoxymethoxy)-naphthalin das (3RS,4RS)-1-Benzyl-4-(4-fluor-phenyl)-3-[4-(2-trimethylsilyl-äthoxymethoxy)-naphthalin-2-ylmethoxy]-piperidin als leicht gelbes Öl erhalten; MS: 572 $(M+H)^+$.

(f) In analoger Weise wie in Beispiel 1 (d) beschrieben, wurde durch Abspaltung der Benzyl-Gruppe mittels Chlorameisensäure β-trimethylsilyläthylester aus dem (3RS,4RS)-1-Benzyl-4-(4-fluor-phenyl)-3-[4-(2-trimethylsilyl-äthoxymethoxy)-naphthalin-2-ylmethoxy]-piperidin der (3RS,4RS)-4-(4-Fluorphenyl)-3-[4-(2-trimethylsilyl-äthoxymethoxy)-naphthalin-2-yl-methoxy]-piperidine-1-carbonsäure β-trimethylsilyläthylester als leicht gelbes Öl erhalten; MS: 626 $(M+H)^+$.

(g) 4.65 g (7.43 mMol) (3RS,4RS)-4-(4-Fluorphenyl)-3-[4-(2-trimethylsilyl-äthoxymethoxy)-naphthalin-2-ylmethoxy]-piperidin-1-carbonsäure β-trimethylsilyläthylester wurden in 40 ml Methanol gelöst, mit 40 ml einer 2 N Lösung von Chlorwasserstoff in Methanol versetzt und 90 Minuten bei 50° C gerührt. Anschließend wurde das Gemisch zwischen Methylenchlorid und wäßriger 5%iger Natriumhydrogencarbonat-Lösung verteilt, die organische Phase über Magnesiumsulfat getrocknet und schließlich das Lösungsmittel unter verminderten Druck abdestilliert. Das Rohprodukt (6.8 g) wurde durch Chromatographie an Kieselgel mit einem 3:7-Gemisch von Essigester und Hexan gereinigt. Es wurden 2.93 g (80% d.Th.) (3RS,4RS)-4-(4-Fluorphenyl)-3-(4-hydroxy-naphthalin-2-ylmethoxy)-piperidin-1-carbonsäure β-trimethyl- silyläthylester als farbloser Festkörper erhalten; MS: 496 $(M+H)^+$.

Beispiel 6

In analoger Weise wie in Beispiel 5 beschrieben, wurden die folgenden Verbindungen erhalten:

1) - Aus (3RS,4RS)-4-(4-Fluorphenyl)-3-(1-hydroxy-naphthalin-2-ylmethoxy)-piperidin-1-carbonsäure β-trimethylsilyläthylester das 4-(4-Fluor-phenyl)- 3-(1-hydroxy-naphthalin-2-ylmethoxy)- piperidin als leicht brauner Festkörper; MS : 351 $(M)^+$;

2) - aus (3RS,4RS)-4-(4-Fluorphenyl)-3-(5-hydroxy-naphthalin-2-ylmethoxy)-piperidin-1-carbonsäure β-trimethylsilyläthylester das 4-(4-Fluor-phenyl)-3-(5-hydroxy-naphthalin-2-yl-methoxy)-piperidin als farbloser Festkörper; MS: 351 $(M)^+$;

3) - aus (3RS,4RS)-4-(4-Fluorphenyl)-3-(6-hydroxy-naphthalin-2-ylmethoxy)-piperidin-1-carbonsäure β-trimethylsilyläthylester das 4-(4-Fluor-phenyl)-3-(6-hydroxy-naphthalin-2-ylmethoxy)-piperidin als farbloser Festkörper; MS: 351 $(M)^+$;

4) - aus (3RS,4RS)-4-(4-Fluorphenyl)-3-(7-hydroxy-naphthalin-2-ylmethoxy)-piperidin-1-carbonsäure β-trimethylsilyläthylester das 4-(4-Fluor-phenyl)-3-(7-hydroxy-naphthalin-2-yl-methoxy)-piperidin als leicht brauner Festkörper; MS : 351 $(M)^+$;

5) - aus (3RS,4RS)-4-(4-Fluorphenyl)-3-(8-hydroxy-naphthalin-2-ylmethoxy)-piperidin-1-carbonsäure β-trimethylsilyläthylester das 4-(4-Fluor-phenyl)-3-(8-hydroxy-naphthalin-2-yl-methoxy)-piperidin als leicht gelbes Harz; MS : 352 $(M+H)^+$.

Die als Ausgangsstoffe eingesetzten β-Trimethylsilyläthylcarbamate wurden in Analogie zu dem im Beispiel 5 (a)-(f) beschriebenen Verfahren wie folgt hergestellt:

(a) Aus 1-Hydroxy-naphthalin-2-carbonsäuremethylester [J. Chem. Soc. 309 (1948)] wurde durch Einführung der Schutzgruppe der 1-(2-Trimethylsilyläthoxymethoxy)-naphthalin-2-carbonsäuremethylester als leicht gelbes Öl erhalten; MS : 333 $(M+H)^+$.

(b) Die Reduktion von 1-(2-Trimethylsilyl-äthoxymethoxy)-naphthalin-2-carbonsäuremethylester ergab das [1-(2-Trimethyl-silyläthoxymethoxy)-naphthalin-2-yl]-methanol als leicht gelbes Öl; MS : 305 $(M+H)^+$.

(c) Die Chlorierung von [1-(2-Trimethylsilyläthoxy-methoxy)-naphthalin-2-yl]-methanol lieferte das 2-Chlor-methyl-1-(β-trimethylsilyläthoxymethoxy)-naphthalin als farbloses Öl; MS : 322, 324 $(M)^+$.

(d) Die Alkylierung von (3RS,4RS)-1-Benzyl-4-(4-fluoro-phenyl)-piperidin-3-ol mit 2-Chlor-methyl-1-(β-trimethylsilyläthoxy-methoxy)-naphthalin lieferte das (3RS,4RS)-1-Benzyl-3-(4-fluorphenyl)-3-[1-(2-trimethylsilyläthoxymethoxy)-naphthalin-2-ylmethoxy]-piperidin als leicht gelbes Öl; MS : 572 $(M+H)^+$.

(e) Die Abspaltung der N-Benzylgruppe des (3RS,4RS)-1-Benzyl-3-(4-fluorphenyl)-3-[1-(2-trimethylsilyläthoxymethoxy)-naphthalin-2-ylmethoxy]-piperidins mit Chlorameisensäure β-trimethylsilyl-äthylester ergab den 3RS, 4RS)-4-(4-Fluorphenyl)-3-[1-(2-trimethylsilyl-äthoxymethoxy)-naphthalin-2-ylmethoxy]-piperidine-1-carbonsäure β-trimethylsilyläthylester als farbloses Öl; MS : 626 $(M+H)^+$.

(f) Die Abspaltung der SEM-Gruppe des (3RS, 4RS)-4-(4-Fluorphenyl)-3-[1-(2-trimethylsilyl-äthoxymethoxy)-naphthalin-2-ylmethoxy]-piperidine-1-carbonsäure β-trimethylsilyläthylesters lieferte den (3RS,4RS)-4-(4-Fluorphenyl)-3-(1-hydroxy-naphthalin-2-ylmethoxy)-piperidin-1-carbonsäure β-trimethylsilyläthylester als farbloses Öl; MS: 494 $(M-H)^-$.

(g) Aus 5-Hydroxy-naphthalin-2-carbonsäure [Bull. Soc. Chim. Fr., 857 (1953)] wurde zunächst durch Veresterung mit Methanol/Schwefelsäure der 5-Hydroxy-naphthalin-2-carbonsäuremethylester als leicht gelber Festkörper erhalten; MS : 202 $(M)^+$. Durch Einführung der Schutzgruppe wurde der 5-(2-Trimethylsilyl-äthoxymethoxy)-naphthalin-2-carbonsäuremethylester als leicht gelbes Öl erhalten; MS : 333 $(M+H)^+$.

(h) Die Reduktion des 5-(2-Trimethylsilyl-äthoxymethoxy)-naphthalin-2-carbonsäuremethylesters ergab das [5-(2-Trimethylsilyl-äthoxymethoxy)-naphthalin-2-yl]-methanol als leicht gelbes Öl ; MS : 305 $(M+H)^+$.

(i) Die Chlorierung von [5-(2-Trimethylsilyl-äthoxymethoxy)-naphthalin-2-yl]-methanol lieferte das 2-Chlor-methyl-5-(β-trimethylsilyläthoxy-methoxy)-naphthalin als farbloses Öl; MS : 322, 324 $(M)^+$.

(j) Die Alkylierung von (3RS,4RS)-1-Benzyl-4-(4-fluoro-phenyl)-piperidin-3-ol mit 2-Chlor-methyl-5-(β-trimethyl-silyläthoxy-methoxy)-naphthalin lieferte das (3RS,4RS)-1-Benzyl-3-(4-fluor-phenyl)-3-[5-(2-tri-methylsilyl-äthoxy-methoxy)-naphthalin-2-ylmethoxy]- piperidin als leicht gelbes Öl; MS: 572 $(M+H)^+$.

(k) Die Abspaltung der N-Benzylgruppe des (3RS,4RS)-1-Benzyl-3-(4-fluor-phenyl)-3-[5-(2-tri-methylsilyl-äthoxy-methoxy)-naphthalin-2-ylmethoxy]- piperidins mit Chlorameisensäure β-trimethylsilyl-äthylester ergab den (3RS, 4RS)-4-(4-Fluorphenyl)-3-[5-(2-trimethylsilyl-äthoxymethoxy)-naphthalin-2-ylmethoxy]-piperidin-1-carbonsäure β-trimethylsilyläthylester als farbloses Öl; MS : 626 $(M+H)^+$.

(l) Die Abspaltung der SEM-Gruppe in (3RS,4RS)-4-(4-Fluorphenyl)-3-[5-(2-trimethylsilyl-äthoxymethoxy)-naphthalin-2-ylmethoxy]-piperidin-1-carbonsäure β-trimethylsilyläthylester lieferte den (3RS,4RS)-4-(4-Fluorphenyl)-3-(5-hydroxy-naphthalin-2-ylmethoxy)-piperidin-1-carbonsäure β-trimethylsilyläthylester als farbloses Öl; MS : 494 (M-H)-.

(m) Aus 6-Hydroxy-naphthalin-2-carbonsäureäthylester [J. Chem. Soc. 123, 1654 (1923)] wurde durch Einführung der Schutzgruppe der 6-(2-Trimethylsilyl-äthoxymethoxy)-naphthalin-2-carbonsäureäthylester als leicht gelbes Öl erhalten; MS : 346 $(M)^+$.

(n) Die Reduktion von 6-(2-Trimethylsilyl-äthoxymethoxy)-naphthalin-2-carbonsäureäthylester ergab das [6-(2-Trimethylsilyl-äthoxy-methoxy)-naphthalin-2-yl]-methanol als farblosen Festkörper; MS : 304 (M)+.

(o) Die Chlorierung von [6-(2-Trimethylsilyl-äthoxy-methoxy)-naphthalin-2-yl]-methanol lieferte das 6-Chlormethyl-2-(β-trimethylsilyläthoxymethoxy)-naphthalin als farbloses Öl; MS : 322, 324 (M)+.

(p) Die Alkylierung von (3RS,4RS)-1-Benzyl-4-(4-fluoro-phenyl)-piperidin-3-ol mit 6-Chlormethyl-2-(ß-trimethylsilyläthoxymethoxy)-naphthalin lieferte das (3RS,4RS)-1-Benzyl-3-(4-fluorphenyl)-3-[6-(2-trimethyl-silyläthoxy-methoxy)-naphthalin-2-ylmethoxy]-piperidin als leicht gelbes Öl; MS : 572 $(M+H)^+$.

(q) Die Abspaltung der N-Benzylgruppe des (3RS,4RS)-1-Benzyl-3-(4-fluorphenyl)-3-[6-(2-trimethyl-silyläthoxy-methoxy)-naphthalin-2-ylmethoxy]-piperidins mit Chlorameisensäure β-trimethylsilyl-äthylester ergab den (3RS, 4RS)-4-(4-Fluorphenyl)-3-[6-(2-trimethylsilyläthoxy-methoxy)- naphthalin- 2-yl-methoxy]- piperidin-1-carbonsäure β-trimethylsilyläthylester als farbloses Öl; MS : 626 $(M+H)^+$.

(r) Die Abspaltung der SEM-Gruppe des (3RS,4RS)-4-(4-Fluorphenyl)-3-[6-(2-trimethylsilyläthoxy-methoxy)-naphthalin- 2-yl-methoxy]-piperidin-1-carbonsäure β-trimethylsilyläthylesters lieferte den (3RS,4RS)-4-(4-Fluorphenyl)-3-(6-hydroxy-naphthalin-2-ylmethoxy)-piperidin-1-carbonsäure β-trimethylsilyläthylester als farbloses Harz; MS : 495 $(M)^+$.

(s) Aus 7-Hydroxy-naphthalin-2-carbonsäure [Bull. Soc. Chim. Fr., 573 (1952)] wurde zunächst durch Veresterung mit Methanol/Schwefelsäure der 7-Hydroxy-naphthalin-2-carbonsäuremethylester als farbloser Festkörper erhalten; MS : 202 $(M)^+$. Die Einführung der Schutzgruppe lieferte den 7-(2-Trimethylsilyl-äthoxymethoxy)-naphthalin-2-carbonsäuremethylester als leicht gelbes Öl; MS : 332 $(M)^+$.

(t) Die Reduktion des 7-(2-Trimethylsilyl-äthoxymethoxy)-naphthalin-2-carbonsäuremethylesters ergab das [7-(2-Trimethylsilyl-äthoxymethoxy)-naphthalin-2-yl]-methanol als leicht gelbes Öl ; MS : 304 $(M)^+$.

(u) Die Chlorierung von [7-(2-Trimethylsilyl-äthoxy-methoxy)-naphthalin-2-yl]-methanol lieferte das 2-Chlormethyl-7-(β-trimethylsilyläthoxymethoxy)-naphthalin als leicht gelbes Öl; MS : 322, 324 $(M)^+$.

(v) Die Alkylierung des (3RS,4RS)-1-Benzyl-4-(4-fluoro-phenyl)-piperidin-3-ols mit 2-Chlormethyl-7-(β-trimethylsilyläthoxymethoxy)-naphthalin lieferte das (3RS,4RS)-1-Benzyl-3-(4-fluor-phenyl)-3-[7-(2-trimethylsilyl-äthoxy-methoxy)-naphthalin-2-ylmethoxy]- piperidin als leicht gelbes Öl; MS : 572 $(M+H)^+$.

(w) Die Abspaltung der N-Benzylgruppe des (3RS,4RS)-1-Benzyl-3-(4-fluor-phenyl)-3-[7-(2-trimethylsilyl-äthoxy-methoxy)-naphthalin-2-ylmethoxy]- piperidins mit Chlorameisensäure β-trimethylsilyl-äthylester ergab den (3RS,

4RS)-4-(4-Fluorphenyl)-3-[7-(2-trimethylsilyl-äthoxymethoxy)-naphthalin-2-ylmethoxy]-piperidin- 1-carbonsäure β-trimethylsilyläthylester als farbloses Öl; MS : 626 (M+H)$^+$.

(x) Die Abspaltung der SEM-Gruppe des (3RS,4RS)-4-(4-Fluorphenyl)-3-[7-(2-trimethylsilyl-äthoxymethoxy)-naphthalin-2-ylmethoxy]-piperidin-1-carbonsäure β-trimethylsilyläthylesters lieferte den (3RS,4RS)-4-(4-Fluorphenyl)-3-(7-hydroxy-naphthalin-2-ylmethoxy)-piperidin 1-carbonsäure β-trimethylsilyläthylester als leicht gelbes Öl; MS : 495 (M)$^+$.

(y) Aus 8-Hydroxy-naphthalin-2-carbonsäure [Bull. Soc. Chim. Fr., 857 (1953)] wurde zunächst durch Veresterung mit Methanol/Schwefelsäure der 8-Hydroxy-naphthalin-2-carbonsäuremethylester als leicht gelber Festkörper erhalten; MS : 202 (M)$^+$. Die Einführung der Schutzgruppe lieferte den 8-(2-Trimethylsilyl-äthoxymethoxy)-naphthalin-2-carbonsäuremethylester als farblosen Festkörper; MS: 274 [M-($C_2H_4$+$CH_2O$)]$^+$.

(z) Die Reduktion des 8-(2-Trimethylsilyl-äthoxymethoxy)-naphthalin-2-carbonsäuremethylesters ergab das [8-(2-Trimethylsilyl-äthoxymethoxy)-naphthalin-2-yl]-methanol als farbloses Öl ; MS: 304 (M)$^+$.

(aa) Die Chlorierung von [8-(2-Trimethylsilyl-äthoxymethoxy)-naphthalin-2-yl]-methanol lieferte das 2-Chlormethyl-8-(2-trimethylsilyläthoxy-methoxy)-naphthalin als leicht gelbes Öl; MS: 322, 324 (M)$^+$.

(bb) Die Alkylierung des (3RS,4RS)-1-Benzyl-4-(4-fluoro-phenyl)-piperidin-3-ols mit 2-Chlormethyl-8-(2-trimethylsilyläthoxy-methoxy)-naphthalin lieferte das (3RS,4RS)-1-Benzyl-3-(4-fluor-phenyl)-3-[8-(2-trimethylsilyläthoxy-methoxy)-naphthalin-2-ylmethoxy]-piperidin als leicht gelbes Öl; MS : 572 (M+H)$^+$.

(cc) Die Abspaltung der N-Benzylgruppe des (3RS,4RS)-1-Benzyl-3-(4-fluor-phenyl)-3-[8-(2-trimethylsilyläthoxy-methoxy)-naphthalin-2-ylmethoxy]-piperidins mit Chlorameisensäure β-trimethylsilyl-äthylester ergab den (3RS, 4RS)-4-(4-Fluorphenyl)-3-[8-(2-trimethylsilyl-äthoxymethoxy)-naphthalin-2-yl-methoxy]-piperidin-1-carbonsäure β-trimethylsilyläthylester als farbloses Öl; MS : 626 (M+H)$^+$.

(dd) Die Abspaltung der SEM-Gruppe des (3RS,4RS)-4-(4-Fluorphenyl)-3-[8-(2-trimethylsilyl-äthoxymethoxy)-naphthalin-2-yl-methoxy]-piperidin-1-carbonsäure β-trimethylsilyläthylesters lieferte den (3RS,4RS)-4-(4-Fluorphenyl)-3-(8-hydroxy-naphthalin-2-ylmethoxy)-piperidin-1-carbonsäure β-trimethylsilyläthylester als farbloses Öl; MS : 494 (M-H)$^-$.

Beispiel 7

In analoger Weise wie in den Beispielen 3 bzw. 5 beschrieben, wurden die folgenden Verbindungen hergestellt:

1) (3RS,4RS)-4-(4-Fluorphenyl)-3-(3-hydroxy-naphthalin-2-ylmethoxy)-piperidin

In analoger Weise wie in den Beispielen 3 und 5 (g) beschrieben, wurde durch Abspaltung der beiden Schutzgruppen mit methanolischer Salzsäure aus (3RS,4RS)-4-(4-Fluorphenyl)-3-[3-(2-trimethylsilyl-äthoxymethoxy)-naphthalin-2-yl-methoxy]-piperidin-1-carbonsäure tert-butylester das (3RS,4RS)-4-(4-Fluorphenyl)-3-(3-hydroxy-naphthalin-2-yl-methoxy)-piperidin als farbloser Festkörper erhalten; MS : 351 (M)$^+$.

Der als Ausgangssubstanz eingesetzte (3RS,4RS)-4-(4-Fluorphenyl)-3-[3-(2-trimethylsilyl-äthoxymethoxy)-naphthalin-2-ylmethoxy]-piperidin-1-carbonsäure tert-butylester wurde wie folgt hergestellt:

(a) In analoger Weise wie in Beispiel 5 (a) beschrieben, wurde durch Einführung der Schutzgruppe aus dem 3-Hydroxy-naphthalin-2-carbonsäure methylester der 3-(2-Trimethylsilyl-äthoxy-methoxy)-naphthalin-2-carbonsäure methylester als leicht gelbes Öl erhalten; MS: 274 (M-($C_2H_4$+$CH_2O$)]$^+$.

(b) In analoger Weise wie in Beispiel 5 (b) beschrieben, ergab die Reduktion des 3-(2-Trimethylsilyl-äthoxy-methoxy)-naphthalin-2-carbonsäure methylesters das [3-(2-Trimethylsilyläthoxy-methoxy)-naphthalin-2-yl]-methanol als leicht gelbes Öl ; MS : 304 (M)$^+$.

(c) 400 mg (1.30 mMol) [3-(2-Trimethylsilyläthoxy-methoxy)-naphthalin-2-yl]-methanol und 462 mg (1.81 mMol) Tetrabromkohlenstoff wurden in 5 ml absolutem Acetonitril gelöst und die Lösung auf 0° C abgekühlt. Dazu wurde bei 0° C eine Lösung von 446 mg (1.68 mMol) Triphenylphosphin in 6 ml absolutem Acetonitril innerhalb von 10 Minuten getropft und danach 30 Minuten bei 0° C weitergerührt. Anschließend wurde das

Lösungsmittel unter vermindertem Druck abdestilliert und das Rohprodukt ohne weitere Aufarbeitung zur Reinigung an Kieselgel unter Verwendung eines 2:3-Gemisches von Methylenchlorid und Hexan als Eluierungsmittel chromatographiert. Es wurden 314 mg (65% d.Th.) 2-Brommethyl-3-(2-trimethylsilyläthoxymethoxy)-naphthalin als leicht gelbes Öl erhalten; MS : 366, 368 $(M)^+$.

(d) In analoger Weise wie in Beispiel 3 (c) beschrieben, wurde durch Alkylierung des (3RS,4RS)-4-(4-Fluorphenyl)-3-hydroxy-piperidin-1-carbonsäure tert-butylesters mit 2-Brommethyl-3-(2-trimethylsilyl-äthoxy-methoxy)-naphthalin der (3RS,4RS)-4-(4-Fluorphenyl)-3-[3-(2-trimethylsilyl-äthoxy-methoxy)-naphthalin-2-ylmethoxy]-piperidin-1-carbonsäure tert-butylester als leicht gelbes Öl erhalten; MS : 523 $[M-(C_2H_4 + CH_2O)]^+$.

2) (3RS,4RS)-4-(4-Fluorphenyl)-3-(1-methoxy-naphthalin-2-ylmethoxy)-piperidin

In analoger Weise wie in Beispiel 3 beschrieben, wurde durch Abspaltung der BOC-Schutzgruppe aus dem (3RS,4RS)-4-(4-Fluorphenyl)-3- [1-methoxy-naphthalin-2-ylmethoxy] -piperidin-1-carbonsäure tert-butylester das (3RS,4RS)-4-(4-Fluorphenyl)-3-(1-methoxy-naphthalin-2-yl-methoxy)-piperidin als leicht gelbes Öl erhalten; MS: 365 $(M)^+$.

Der als Ausgangsmaterial eingesetzte (3RS,4RS)-4-(4-Fluorphenyl)-3-[1-methoxy-naphthalin-2-ylmethoxy]-piperidin-1-carbonsäure tert-butylester wurde wie folgt erhalten:

(e) In analoger Weise wie in Beispiel 5 (b) beschrieben, wurde durch Reduktion von 1-Methoxy-naphthalin-2-carbonsäure methylester [J.Chem.Soc. 121,1657 (1922)] das [1-Methoxy)-naphthalin-2-yl]-methanol als farbloser Festkörper erhalten; MS : 188 $(M)^+$.

(f) In analoger Weise wie in Beispiel 7 (c) beschrieben, wurde durch Bromierung von [1-Methoxy)-naphthalin-2-yl]-methanol das 2-Brommethyl-1-methoxy-naphthalin als farbloser Festkörper erhalten; MS : 250, 252 $(M)^+$.

(g) In analoger Weise wie in Beispiel 3 (c) beschrieben, wurde durch Alkylierung des (3RS,4RS)-4-(4-Fluorphenyl)-3-hydroxy-piperidin-1-carbonsäure tert-butylesters [Beispiel 3 (b)] mit 2-Brommethyl-1-methoxy-naphthalin der (3RS,4RS)-4-(4-Fluorphenyl)-3-[1-methoxynaphthalin-2-yl-methoxy]-piperidin-1-carbonsäure tert-butylester als farbloses Harz erhalten; MS : 465 $(M)^+$.

3) (3RS,4RS)-4-(4-Fluorphenyl)-3-(3-methoxy-naphthalin-2-ylmethoxy)-piperidin

In analoger Weise wie in Beispiel 3 beschrieben, wurde durch Abspaltung der BOC-Schutzgruppe aus dem (3RS,4RS)-4-(4-Fluorphenyl)-3-[3-methoxy-naphthalin-2-ylmethoxy]-piperidin-1-carbonsäure tert-butylester das (3RS,4RS)-4-(4-Fluorphenyl)-3-(3-methoxy-naphthalin-2-yl-methoxy)-piperidin als leicht gelbes Öl erhalten; MS : 365 (M)+.

Der als Ausgangsmaterial eingesetzte (3RS,4RS)-4-(4-Fluorphenyl)-3-[3-methoxy-naphthalin-2-ylmethoxy]-piperidin-1-carbonsäure tert-butylester wurde wie folgt erhalten:

(h) In analoger Weise wie in Beispiel 5 (b) beschrieben, wurde durch Reduktion des 3-Methoxy-naphthalin-2-carbonsäure methylesters [J. Chem. Soc. 2351 (1950)] das [3-Methoxy)-naphthalin-2-yl]-methanol als farbloser Festkörper erhalten; MS : 188 $(M)^+$.

(i) In analoger Weise wie in Beispiel 7 (c) beschrieben, wurde durch Bromierung des [3-Methoxy)-naphthalin-2-yl]-methanols das 2-Brommethyl-3-methoxy-naphthalin als farbloser Festkörper erhalten; MS : 250, 252 $(M)^+$.

(j) In analoger Weise wie in Beispiel 3 (c) beschrieben, wurde durch Alkylierung des (3RS,4RS)-4-(4-Fluorphenyl)-3-hydroxy-piperidin-1-carbonsäure tert-butylesters [Beispiel 3 (b)] mit 2-Brommethyl-3-methoxy-naphthalin der (3RS,4RS)-4-(4-Fluorphenyl)-3-[3-methoxynaphthalin-2-yl-methoxy]-piperidin-1-carbonsäure tert-butylester als farbloses Harz erhalten; MS : 465 $(M)^+$.

Beispiel 8

(a) Zu 1.46 g Magnesium-Spänen, die zuvor mit Tetrahydrofuran überschichtet worden waren, wurde eine Lösung von 12.06 g (60 mMol) 5-Brom-benzo[1,3]dioxol in 30 ml absolutem Tetrahydrofuran getropft, gefolgt von der Zugabe von 11.35 g (60 mMol) 1-Benzyl-4-piperidon. Das Reaktionsgemisch wurde während 1 Stunde bei 50° C

gerührt, dann auf Eis und Ammoniumchlorid-Lösung gegossen. Das gebildete 4-Benzo[1,3]dioxol-5-yl-1-benzyl-piperidin-4-ol wurde mit Essigester extrahiert und kristallisierte beim Einengen der Lösung aus. Man erhielt 10.85 g (58 % d.Th.) weiße Kristalle; Smp.: 144° C.

(b) In analoger Weise wie in Beispiel 2 (e) beschrieben, wurde durch katalytische Hydrierung bei Normaldruck innerhalb von 4 Stunden aus dem 4-Benzo[1,3]dioxol-5-yl-1-benzyl-piperidin-4-ol das 4-Benzo[1,3]-dioxol-5-yl-piperidin-4-ol als farbloser Feststoff in quantitativer Ausbeute erhalten; MS : 221 $(M)^+$.

(c) In analoger Weise wie in Beispiel 1 (b) beschrieben, wurde durch Elimination aus dem 4-Benzo[1,3]dioxol-5-yl-piperidin-4-ol das 4-Benzo-[1,3]dioxol-5-yl-1,2,3,6-tetrahydro-pyridin als beigefarbener Feststoff erhalten; MS : 203 $(M)^+$.

(d) In analoger Weise wie in Beispiel 1 (f) beschrieben, wurde durch Einführung der BOC-Gruppe aus 4-Benzo [1,3]dioxol-5-yl-1,2,3,6-tetrahydro-pyridin der 4-Benzo[1,3]dioxol-5-yl-3,6-dihydro-2H-pyridin-1-carbonsäure tert-butylester als farbloses Öl erhalten; MS : 304 $(M+H)^+$.

(e) In analoger Weise wie in Beispiel 1 (c) beschrieben, wurde durch Hydroborierung des 4-Benzo(1,3]dioxol-5-yl-3,6-dihydro-2H-pyridin-1-carbonsäure tert-butylesters der (3RS,4RS)-4-Benzo[1,3]dioxol-5-yl-3-hydroxy-piperidin-1-carbonsäure tert-butylester als weiße Kristalle erhalten; Smp.: 112° C.

(f) In analoger Weise wie in Beispiel 1 (g) beschrieben, wurde durch Alkylierung des (3RS,4RS)-4-Benzo[1,3] dioxol-5-yl-3-hydroxy-piperidin-1-carbonsäure tert-butylesters mit 2-Brommethylnaphthalin der (3RS,4RS)-4-Benzo[1,3]dioxol-5-yl-3-(naphthalin-2-ylmethoxy)-piperidin-1-carbonsäure tert-butylester nach Kristallisation aus Hexan als weiße Kristalle erhalten; Smp.: 128-129° C.

(g) Eine Lösung von 190 mg (0.41 mMol) (3RS,4RS)-4-Benzo[1,3]dioxol-5-yl-3-(naphthalin-2-ylmethoxy)-piperidin-1-carbonsäure tert-butylester in einem Gemisch von 5 ml Methanol und 25%iger wäßriger Salzsäure wurde 1 Stunde lang unter Rückfluß erhitzt. Anschließend wurde unter vermindertem Druck das Lösungsmittelgemisch abdestilliert. Nach dem Umkristallisieren des Rückstandes aus einem Gemisch von Methanol und Äther wurden 130 mg (73% d.Th.) (3RS,4RS)-4-(1,3-Benzodioxol-5-yl)-3-naphthalin-2-ylmethoxy-piperidin Hydrochlorid als weißes Pulver erhalten; MS : 362 $(M+H)^+$.

Beispiel 9

In analoger Weise wie in Beispiel 8 (g) beschrieben, wurden durch Abspaltung der BOC-Gruppe mittels Säure die folgenden Verbindungen erhalten:

1) -Aus (3RS,4RS)-4-Phenyl-3-(2-{[(pyridin-3-carbonyl)-amino]-methyl}-benzyloxy)-piperidin-1-carbonsäure tert-butylester das Pyridin-3-carbonsäure (3RS,4RS)-2-(4-phenyl-piperidin-3-yloxymethyl)-benzylamid Hydrochlorid als beigefarbenes Pulver; MS : 402 $(M+H)^+$;

2) - aus (3RS,4RS)-4-Benzo[1,3]dioxol-5-yl-3-(2-carbamoyl-benzyloxy)-piperidin-1-carbonsäure tert-butylester das (3RS,4RS)-2-(4-[1,3]Benzodioxol-5-yl-piperidin-3-yloxymethyl)-benzamid Hydrochlorid als weißes Pulver; MS : 355 $(M+H)^+$;

Die als Ausgangsstoffe eingesetzten BOC-Derivate wurden wie folgt hergestellt:

(a) In analoger Weise wie in Beispiel 1 (g) beschrieben, wurde durch Alkylierung des (3RS,4RS)-3-Hydroxy-4-phenyl-piperidin-1-carbonsäure tert-butylesters [Beispiel 2 (a)] mit 2-Brommethylbenzonitril der (3RS,4RS)-3-(2-Cyano-benzyloxy)-4-phenyl-piperidin-1-carbonsäure tert-butylester als farbloses Öl erhalten; MS : 393 $(M+H)^+$.

(b) In Analogie zu dem von H.C.Brown et al. in Synthesis 1981, 605 beschriebenen Verfahren, wurden 528 mg (1.35 mMol) (3RS,4RS)-3-(2-Cyano-benzyloxy)-4-phenyl-piperidin-1-carbonsäure tert-butylester mit 0.3 ml Boran-Dimethylsulfid-Komplex reduziert. Es wurden 480 mg (90% d.Th.) (3RS,4RS)-3-(2-Aminomethyl-benzyloxy)-4-phenyl-piperidin-1-carbonsäure tert-butylester als farbloser Feststoff erhalten; 397 $(M+H)^+$.

(c) Eine Lösung von 150 mg (0.38 mMol) (3RS,4RS)-3-(2-Aminomethylbenzyloxy)-4-phenyl-piperidin-1-carbonsäure tert-butylester in 2 ml Methylenchlorid wurde mit 229 mg (2.26 mMol) Triäthylamin, 139 mg (1.05 mMol)

Nicotinsäure, 216 mg (1.13 mMol) EDC und 10 mg (0.08 mMol) 4-Dimethylaminopyridin versetzt und 24 Stunden lang bei Raumtemperatur gerührt. Danach wurde die Reaktionslösung mit Methylenchlorid verdünnt und mit einer gesättigten Natriumhydrogencarbonat-Lösung gewaschen. Die organische Phase wurde abgetrennt, über Natriumsulfat getrocknet und unter vermindertem Druck eingedampft. Der Rückstand wurde an Kieselgel unter Verwendung von Essigester als Eluierungsmittel chromatographiert. Man erhielt 100 mg (53% d.Th.) (3RS,4RS)-4-Phenyl-3-(2-{[(pyridin-3-carbonyl)-amino]-methyl}-benzyloxy)-piperidin-1-carbonsäure tert-butylester als farblosen Feststoff; MS : 502 $(M+H)^+$.

(d) In analoger Weise wie in Beispiel 1 (g) beschrieben, wurde durch Alkylierung des (3RS,4RS)-4-Benzo[1,3]dioxol-5-yl-3-(naphthalin-2-ylmethoxy)-piperidin-1-carbonsäure tert-butylesters [Beispiel 8 (f)] mit 2-Brommethylbenzonitril der (3RS,4RS)-4-Benzo[1,3]dioxol-5-yl-3-(2-cyano-benzyloxy)-piperidin-1-carbonsäure tert-butylester als farblose Kristalle erhalten; MS : 455 $(M+H)^+$.

(e) Zu einer Lösung von 236 mg (0.54 mMol) (3RS,4RS)-4-Benzo[1,3]dioxol-5-yl-3-(2-cyano-benzyloxy)-piperidin-1-carbonsäure tert-butylester in 5 ml Methanol wurden 0.5 ml Wasserstoffperoxid (33%ig) und 0.2 ml 2 N Natronlauge gegeben. Die Reaktionslösung wurde 2 Stunden lang unter Rückfluß erhitzt. Anschließend wurden nochmals die gleichen Mengen Wasserstoffperoxid und Natronlauge zugegeben und die Lösung weitere 2 Stunden erhitzt. Danach wurde abgekühlt und die Lösung unter vermindertem Druck eingedampft. Der Rückstand wurde zur Reinigung an Kieselgel unter Verwendung eines 9:1-Gemisches von Methylenchlorid und Äther als Eluierungsmittel chromatographiert. Es wurden 140 mg (57 % d.Th.) (3RS,4RS)-4-Benzo[1,3]dioxol-5-yl-3-(2-carbamoyl-benzyloxy)-piperidin-1-carbonsäure tert-butylester als farbloser Feststoff erhalten; MS: 455 $(M+H)^+$.

Beispiel 10

(a) In analoger Weise wie in Beispiel 1 (g) beschrieben, wurde durch Alkylierung des (3RS,4RS)-4-(4-Fluorphenyl)-3-hydroxy-piperidin-1-carbonsäure tert-butylesters mit 6-Chlormethyl-2,3-dihydrobenzo[1,4]dioxin [Brit. Pat. 566732 (1943)] der (3RS,4RS)-3-(2,3-Dihydrobenzo[1,4]dioxin-6-ylmethoxy)-4-(4-fluorphenyl)-piperidin-1-carbonsäure tert-butylester als farbloser Festkörper erhalten; MS: 444 $(M+H)^+$;

(b) Eine Lösung von 280 mg (0.63 mMol) (3RS,4RS)-3-(2,3-Dihydrobenzo[1,4]dioxin-6-ylmethoxy)-4-(4-fluorphenyl)-piperidin-1-carbonsäure tert-butylester in 5 ml trockenem Methylenchlorid wurde mit 808 mg (1.89 mMol) wasserfreiem Zinkbromid versetzt und das Gemisch 5 Stunden lang bei Raumtemperatur gerührt.Anschließend wurde das Lösungsmittel unter vermindertem Druck abdestilliert, der Rückstand in 10 ml Methanol aufgenommen, mit 2 ml 2 N Natronlauge versetzt und der Festkörper abgetrennt. Das Filtrat wurde unter vermindertem Druck eingedampft und der Rückstand zwischen Methylenchlorid und Wasser verteilt. Die organische Phase wurde abgetrennt und unter vermindertem Druck eingedampft. Es wurden 220 mg (98% d.Th.) (3RS,4RS)-3-(2,3-Dihydrobenzo[1,4]dioxin-6-yl-methoxy)-4-(4-fluorphenyl)-piperidin als gelblicher Feststoff erhalten; MS: 344 $(M+H)^+$.

Beispiel 11

In analoger Weise wie in Beispiel 10 (b) beschrieben, wurde aus (3RS,4RS)-3-(Benzo[b]furan-5-ylmethoxy)-4-(4-fluor-phenyl)-piperidin-1-carbonsäure tert-butylester das (3RS,4RS)-3-(Benzo[b]furan-5-ylmethoxy)-4-(4-fluor-phenyl)-piperidin als farbloser Festkörper erhalten; MS: 326 $(M+1)^+$;

Der als Ausgangsverbindung eingesetzte (3RS,4RS)-3-(Benzo[b]furan-5-ylmethoxy)-4-(4-fluor-phenyl)-piperidin-1-carbonsäure tert-butylester wurde analog zu dem in Beispiel 1 (g) beschriebenen Verfahren durch Alkylierung des (3RS,4RS)-4-(4-Fluorphenyl)-3-hydroxy-piperidin-1-carbonsäure tert-butylesters mit 5-Brommethylbenzo[b]furan als farbloser Festkörper erhalten; MS: 426 $(M+H)^+$.

Das als Alkylierungsmittel eingesetzte 5-Brommethylbenzo[b]furan wurde wie folgt hergestellt:

In analoger Weise zu dem für die Herstellung von 5-Brommethylbenzo[b]thiophen [J. Med. Chem. 34(1), 65 (1991)] aus 5-Methyl-benzo[b]-thiophen beschriebenen Verfahren, wurde durch Bromierung von 5-Methyl-benzo[b]furan [Synth. Commun. 19, 257(1989)] mit N-Bromsuccinimid in Tetrachlorkohlenstoff das 5-Brommethylbenzo[b]furan als leicht gelber Festkörper erhalten; MS: 210, 212 $(M)^+$.

Beispiel 12

(a) In analoger Weise wie in Beispiel 1 (c) beschrieben, wurde durch Hydroborierung mittels Boran in Tetrahydrofuran aus dem 4-(4-Chlorphenyl)-1-methyl-1,2,3,6-tetrahydropyridin [US Pat. 3 320 265] das (3RS,4RS)-4-(4-Chlor-phenyl)-1-methyl-piperidin-3-ol erhalten, das nach dem Umkristallisieren aus einem Gemisch von Methylenchlorid und Hexan als farblose Kristalle vom Smp.: 99-100° C vorlag.

(b) Zu einer Suspension von 0.264 g (5 mMol) Natriumhydrid (50%ige Dispersion in Weißöl) in 8 ml Tetrahydrofuran tropfte man eine Lösung von 1.12 g (5 mMol) (3RS,4RS)-4-(4-Chlor-phenyl)-1-methyl-piperidin-3-ol in 5 ml Tetrahydrofuran und rührte 60 Minuten bei 50° C. Anschließend wurde auf Raumtemperatur abgekühlt und mit 1.10 g (5 mMol) 2-Brommethylnaphthalin in 5 ml Tetrahydrofuran versetzt. Nach 2 Stunden bei 50° C wurde die Reaktionslösung auf 60 ml Eiswasser gegossen und dreimal mit je 25 ml Essigester extrahiert. Die organischen Phasen wurden mit gesättigter Kochsalzlösung gewaschen, über Magnesiumsulfat getrocknet, filtriert und eingedampft. Der Rückstand wurde an Kieselgel unter Verwendung eines 95:5-Gemisches von Methylenchlorid und Methanol als Eluierungsmittel chromatographiert. Es wurden 0.53 g (28 % d.Th.) (3RS,4RS)-4-(4-Chlor-phenyl)-1-methyl-3-(naphthalin-2-ylmethoxy)-piperidin als hellgelbes Öl erhalten; MS : 366 $(M)^+$.

(c) Eine Lösung von 0.526 g (1.43 mMol) (3RS,4RS)-4-(4-Chlor-phenyl)-1-methyl-3-(naphthalin-2-ylmethoxy)-piperidin in 12 ml Toluol wurde mit 100 mg Kaliumcarbonat versetzt und auf 100° C erhitzt. Anschließend wurden 0.423 g (0.288 ml, 2 mMol) Chlorameisensäure 2,2,2-trichloräthylester dazugegeben und 12 Stunden bei 100° gerührt. Die Reaktionslösung wurde eingedampft, in 50 ml Essigester aufgenommen und mit 20 ml Wasser und 20 ml gesättigter Natriumhydrogencarbonat-Lösung gewaschen. Trocknen über Magnesiumsulfat, Filtieren und Eindampfen lieferte ein farbloses Öl, welches an Kieselgel mittels eines 3:2-Gemisches von Hexan und Essigester als Eluierungsmittel chromatographiert wurde. Man erhielt 0.426 g (57 % d.Th.) 4-(4-Chlor-phenyl)-3-(naphthalin-2-ylmethoxy)-piperidin-1-carbonsäure 2,2,2-trichloräthylester als farbloses Öl; $R_f$: 0.31 (Kieselgel, Hexan/Essigester: 3/2).

(d) Eine Suspension von 0.420 g (0.8 mMol) (3RS,4RS)-4-(4-Chlorphenyl)-3-(naphthalin-2-ylmethoxy)-piperidin-1-carbonsäure 2,2,2-trichloräthylester und 300 mg Zink in 10 ml Essigsäure wurde 12 Stunden bei Raumtemperatur gerührt. Die Reaktionslösung wurde mit 40 ml Wasser verdünnt und viermal mit 30 ml Methylenchlorid extrahiert. Die organischen Phase wurden zweimal mit je 40 ml 1N Natronlauge gewaschen, über Natriumsulfat getrocknet, filtriert und eingedampft. Der Rückstand wurde an Kieselgel unter Verwendung eines 9:1-Gemisches von Methylenchlorid und Methanol als Eluierungsmittel chromatographiert. Es wurden 0.210 g (74% d.Th.) (3RS, 4RS)-4-(4-Chlor-phenyl)-3-(naphthalin-2-ylmethoxy)-piperidin, MS : 210 $(M - C_{11}H_9)^+$, erhalten, welches mit einer Lösung von Chlorwasserstoff in Methanol in das Hydrochlorid vom Smp. 159-161° C (Zers.) überführt wurde.

Beispiel 13

In analoger Weise wie in Beispiel 12 (b)-(d) beschrieben, wurden durch Alkylierung und nachfolgende Abspaltung der N-Methyl-Gruppe die folgenden Verbindungen hergestellt:

1) - Aus (3RS,4RS)-4-(4-Chlor-phenyl)-1-methyl-piperidin-3-ol und 1-Brommethylnaphthalin das (3RS,4RS)-4-(4-Chlor-phenyl)-3-(naphthalin-1-ylmethoxy)-piperidin, MS : 210 $(M-C_{11}H_9)^+$, welches mit einer Lösung von Chlorwasserstoff in Äthanol in das Hydrochlorid vom Schmp. 210-213° C (Zers.) überführt wurde.

2) - Aus (3RS,4RS)-4-(4-Chlor-phenyl)-1-methyl-piperidin-3-ol und 1-Brommethyl-4-tert-butylbenzol das (3RS, 4RS)-3-(4-tert.Butyl-benzyloxy)-4-(4-chlor-phenyl)-piperidin, MS : 358 $(M)^+$, welches mit einer Lösung von Chlorwasserstoff in Äthanol in das Hydrochlorid vom Schmp. 164-166° C (Zers.) überführt wurde.

3) - Aus (3RS,4RS)-4-(4-Chlor-phenyl)-1-methyl-piperidin-3-ol und 5-Chlormethyl-benzo[1.3]dioxol das (3RS, 4RS)-3-(Benzo[1.3]dioxol-5-ylmethoxy)-4-(4-chlor-phenyl)-piperidin, MS : 210 $(M-C_8H_7O_2)^+$, welches mit Methansulfonsäure in einem Gemisch von Dioxan und Wasser und anschließender Lyophilisation in das entsprechende Methansulfonat überfuhrt wurde; $R_f$: 0.45 (Kieselgel, Methylenchlorid/Methanol : 9/1).

4) - Aus (3RS,4RS)-4-(4-Chlor-phenyl)-1-methyl-piperidin-3-ol und 1,2-Dichlor-4-chlormethylbenzol das (3RS, 4RS)-4-(4-Chlor-phenyl)-3-(3,4-dichlorbenzyloxy)-piperidin, MS: 370 $(M)^+$, welches mit einer Lösung von Chlorwasserstoff in Äthanol in das Hydrochlorid vom Schmp. 156-158° C (Zers.) überführt wurde.

5) - Aus (3RS,4RS)-4-(4-Chlor-phenyl)-1-methyl-piperidin-3-ol und 2,4-Dichlor-1-chlormethylbenzol das (3RS, 4RS)-4-(4-Chlor-phenyl)-3-(2,4-dichloro-benzyloxy)-piperidin vom Schmp. 83-84° C; MS: 370 $(M)^+$.

6) - Aus (3RS,4RS)-4-(4-Chlor-phenyl)-1-methyl-piperidin-3-ol und 1-Chlor-4-chlormethylbenzol das (3RS,4RS)-3-(4-Chlorbenzyloxy)-4-(4-chlor-phenyl)-piperidin, MS : 210 $(M-C_7H_6Cl)^+$, welches mit einer Lösung von Chlorwasserstoff in Äthanol in das Hydrochlorid vom Schmp. 128-130° C (Zers.) überführt wurde.

7) - Aus (3RS,4RS)-4-(4-Chlor-phenyl)-1-methyl-piperidin-3-ol und 1-Chlormethyl-3-methoxy-benzol das (3RS, 4RS)-(4-Chlor-phenyl)-3-(2-methoxy-benzyloxy)-piperidin, MS: 332 $(M)^+$, welches mit einer Lösung von Chlorwasserstoff in Äthanol in das Hydrochlorid vom Schmp. 116-118° C (Zers.) überführt wurde.

8) - Aus (3RS,4RS)-4-(4-Chlor-phenyl)-1-methyl-piperidin-3-ol und 1-Chlor-2-chlormethyl-benzol das (3RS,4RS)-3-(2-Chlorbenzyloxy)-4-(4-chlor-phenyl)-piperidin, MS : 210 $(M-C_7H_6Cl)^+$ welches mit einer Lösung von Chlorwasserstoff in Äthanol in das Hydrochlorid vom Schmp. 145-147° C (Zers.) überführt wurde.

9) - Aus (3RS,4RS)-4-(4-Chlor-phenyl)-1-methyl-piperidin-3-ol und 4-Chlormethyl-biphenyl das (3RS,4RS)-3-(Biphenyl-4-ylmethoxy)-4-(4-chlor-phenyl)-piperidin, MS: 210 $(M-C_{13}H_{11})^+$, welches mit einer Lösung von Chlorwasserstoff in Äthanol in das Hydrochlorid vom Schmp. 177-180° C (Zers.) überführt wurde.

10) - Aus (3RS,4RS)-4-(4-Chlor-phenyl)-1-methyl-piperidin-3-ol und 2-Chlormethyl-chinolin das (3RS,4RS)-2-[4-(4-Chlor-phenyl)-piperidin-3-yloxy-methyl]chinolin, MS: 353 $(M)^+$ vom Schmp. 109-110° C.

11) - Aus (3RS,4RS)-4-(4-Chlor-phenyl)-1-methyl-piperidin-3-ol und 2-Chlormethyl-benzofuran [J.Am. Chem. Soc. 73, 4400 (1951)] das (3RS,4RS)-3-(Benzofuran-2-ylmethoxy)-4-(4-chlor-phenyl)-piperidin, MS: 341 $(M)^+$, welches mit einer Lösung von Chlorwasserstoff in Äthanol in das Hydrochlorid vom Schmp.144-146° C (Zers.) überführt wurde.

12) - Aus (3RS,4RS)-4-(4-Chlor-phenyl)-1-methyl-piperidin-3-ol und 2-Chlormethyl-benzo[b]thiophen [J. Am. Chem. Soc. 71, 2856 (1949)] das (3RS,4RS)-3-(Benzo[b]thiophen-2-ylmethoxy)-4-(4-Chlor-phenyl)-piperidin, MS: 210 $(M-C_8H_7S)^+$, welches mit einer Lösung von Chlorwasserstoff in Äthanol in das Hydrochlorid vom Schmp. 141-144° C (Zers.) überführt wurde.

13) - Aus (3RS,4RS)-4-(4-Chlor-phenyl)-1-methyl-piperidin-3-ol und 4'-Brommethyl-biphenyl-2-carbonsäuremethylester [J. Med.Chem. 34, 2525 (1991)] der (3RS,4RS)-4'-[4-(4-Chloro-phenyl)piperidin-3-yloxymethyl]-biphenyl-2-carbonsäuremethylester, MS: 436 $(M)^+$, welcher mit Salzsäure in Äthanol in das Hydrochlorid vom Schmp. 95-99° C (Zers.) überführt wurde.

14) - Aus (3RS,4RS)-4-(4-Chlor-phenyl)-1-methyl-piperidin-3-ol und 3-Chlormethyl-pyridin [J.Am. Chem. Soc. 77, 1054 (1955)] das (3RS,4RS)-3-[4-(4-Chlor-phenyl-piperidin-3-yloxymethyl]-pyridin, MS: 303 $(M)^+$, welches mit einer Lösung von Chlorwasserstoff in Äthanol in das Dihydrochlorid vom Schmp. 78-81° C (Zers.) überführt wurde.

15) - Aus (3RS,4RS)-4-(4-Chlor-phenyl)-1-methyl-piperidin-3-ol und 6-Chlormethyl-1,1,4,4,-tetramethyl-1,2,3,4-tetrahydronaphthalin das (3RS,4RS)-4-(4-Chlor-phenyl)-3-(5,5,8,8,-tetramethyl-5,6,7,8-tetrahydronaphthalin-2-ylmethoxy)-piperidin, MS: 412 $(M)^+$, welches mit einer Lösung von Chlorwasserstoff in Äthanol in das Hydrochlorid vom Schmp. 118-121° C (Zers.) überführt wurde.

16) - Aus (3RS,4RS)-4-(3-Chlor-phenyl)-1-methyl-piperidin-3-ol [US Pat. 4 132 710 (1976)] und 4-Methoxybenzylchlorid das (3RS,4RS)-3-(4-Methoxy-benzyloxy)-4-(3-chlor-phenyl)-piperidin; MS : 332 $(M)^+$.

Beispiel 14

In Analogie zu dem im Beispiel 1 (e) beschriebenen Verfahren, wurden durch Abspaltung der 2-Trimethylsilyläthoxycarbonyl-Gruppe mit Tetrabutylammoniumfluorid in Tetrahydrofuran die folgenden Verbindungen hergestellt:

1) - Aus (3RS,4RS)-4-(4-Fluorphenyl)-3-(4-methoxy-naphthalin-2-ylmethoxy)-piperidin-1-carbonsäure β-trimethyl-silyläthylester das (3RS,4RS)-4-(4-Fluorphenyl)-3-(4-methoxy-naphthalin-2-ylmethoxy)-piperidin als farbloser Festkörper; MS : 365 $(M)^+$;

2) - aus (3RS,4RS)-4-(4-Fluorphenyl)-3-(5-methoxy-naphthalin-2-ylmethoxy)-piperidin-1-carbonsäure β-trimethylsilyläthylester das (3RS,4RS)-4-(4-Fluorphenyl)-3-(5-methoxy-naphthalin-2-ylmethoxy)-piperidin als farbloser Festkörper; MS : 365 $(M)^+$;

3) - aus (3RS,4RS)-4-(4-Fluorphenyl)-3-(6-methoxy-naphthalin-2-ylmethoxy)-piperidin-1-carbonsäure β-trimethylsilyläthylester das (3RS,4RS)-4-(4-Fluorphenyl)-3-(6-methoxy-naphthalin-2-ylmethoxy)-piperidin als farbloser Festkörper; MS : 365 $(M)^+$;

4) - aus (3RS,4RS)-4-(4-Fluorphenyl)-3-(7-methoxy-naphthalin-2-ylmethoxy)-piperidin-1-carbonsäure β-trimethylsilyläthylester das (3RS,4RS)-4-(4-Fluorphenyl)-3-(7-methoxy-naphthalin-2-ylmethoxy)-piperidin als farbloser Festkörper; MS : 365 $(M)^+$;

5) - aus (3RS,4RS)-4-(4-Fluorphenyl)-3-(8-methoxy-naphthalin-2-ylmethoxy)-piperidin-1-carbonsäure β-trimethylsilyläthylester das (3RS,4RS)-4-(4-Fluorphenyl)-3-(8-methoxy-naphthalin-2-ylmethoxy)-piperidin als farbloses Harz; MS : 366 $(M+H)^+$;

6) - aus (3RS,4RS)-4-(4-Fluorphenyl)-3-[4-(pyridin-2-ylmethoxy)-naphthalin-2-ylmethoxy]-piperidin-1-carbonsäure β-trimethylsilyläthylester das (3RS,4RS)-4-(4-Fluorphenyl)-3-[4-(pyridin-2-ylmethoxy)-naphthalin-2-ylmethoxy]-piperidin als farbloser Festkörper; MS : 442 $(M)^+$;

7) - aus (3RS,4RS)-4-(4-Fluorphenyl)-3-[4-(pyridin-3-ylmethoxy)-naphthalin-2-ylmethoxy]-piperidin-1-carbonsäure β-trimethylsilyläthylester das (3RS,4RS)-4-(4-Fluorphenyl)-3-[4-(pyridin-3-ylmethoxy)-naphthalin-2-ylmethoxy]-piperidin als farbloser Festkörper; MS : 443 $(M+H)^+$;

8) - aus (3RS,4RS)-4-(4-Fluorphenyl)-3-[4-(pyridin-4-ylmethoxy)-naphthalin-2-ylmethoxy]-piperidin-1-carbonsäure β-trimethylsilyläthylester das (3RS,4RS)-4-(4-Fluorphenyl)-3-[4-(pyridin-4-ylmethoxy)-naphthalin-2-ylmethoxy]-piperidin als farbloser Festkörper; MS : 442 $(M)^+$;

9) - aus (3RS,4RS)-3-(4-Allyloxy-naphthalin-2-ylmethoxy)-4-(4-fluorphenyl)-piperidin- 1-carbonsäure β-trimethylsilyläthylester das (3RS,4RS)-3-(4-Allyloxy-naphthalin-2-ylmethoxy)-4-(4-fluorphenyl)-piperidin als farbloser Festkörper; MS : 391 $(M)^+$;

10) - aus (3RS,4RS)-3-(6-Allyloxy-naphthalin-2-ylmethoxy)-4-(4-fluorphenyl)-piperidin-1-carbonsäure β-trimethylsilyläthylester das (3RS,4RS)-3-(6-Allyloxy-naphthalin-2-ylmethoxy)-4-(4-fluorphenyl)-piperidin als farbloser Festkörper; MS : 391 $(M)^+$;

11) - aus (3RS,4RS)-4-(4-Fluorphenyl)-3-(4-isobutoxy-naphthalin-2-ylmethoxy)-piperidin-1-carbonsäure β-trimethyl-silyläthylester das (3RS,4RS)-4-(4-Fluorphenyl)-3-(4-isobutyloxy-naphthalin-2-ylmethoxy)-piperidin als farbloser Festkörper; MS : 407 $(M)^+$;

12) - aus (3RS,4RS)-3-(1-Benzyloxy-naphthalin-2-ylmethoxy)-4-(4-fluorphenyl)-piperidin-1-carbonsäure β-trimethyl-silyläthylester das (3RS,4RS)-3-(1-Benzyloxy-naphthalin-2-ylmethoxy)-4-(4-fluorphenyl)-piperidin als leicht gelbes Öl; MS : 441 $(M)^+$;

13) - aus (3RS,4RS)-3-(4-Benzyloxy-naphthalin-2-ylmethoxy)-4-(4-fluorphenyl)-piperidin-1-carbonsäure β-trimethyl-silyläthylester das (3RS,4RS)-3-(4-Benzyloxy-naphthalin-2-ylmethoxy)-4-(4-fluorphenyl)-piperidin als farbloser Festkörper; MS : 441 $(M)^+$;

14) - aus (3RS,4RS)-3-(5-Benzyloxy-naphthalin-2-ylmethoxy)-4-(4-fluorphenyl)-piperidin-1-carbonsäure β-trimethyl-silyläthylester das (3RS,4RS)-3-(5-Benzyloxy-naphthalin-2-ylmethoxy)-4-(4-fluorphenyl)-piperidin als leicht gelbes Öl; MS : 442 $(M+H)^+$;

15) - aus (3RS,4RS)-3-(7-Benzyloxy-naphthalin-2-ylmethoxy)-4-(4-fluorphenyl)-piperidin-1-carbonsäure β-trimethyl-silyläthylester das (3RS,4RS)-3-(7-Benzyloxy-naphthalin-2-ylmethoxy)-4-(4-fluorphenyl)-piperidin als farbloser Festkörper; MS : 441 $(M)^+$;

16) - aus (3RS,4RS)-3-(8-Benzyloxy-naphthalin-2-ylmethoxy)-4-(4-fluorphenyl)-piperidin-1-carbonsäure β-trimethyl-silyläthylester das (3RS,4RS)-3-(8-Benzyloxy-naphthalin-2-ylmethoxy)-4-(4-fluorphenyl)-piperidin als leicht gelbes Harz; MS : 442 $(M+H)^+$;

17) - aus (3RS,4RS)-4-(4-Fluorphenyl)-3-[4-(2-methoxy-äthoxy)-naphthalin-2-ylmethoxy]-piperidin-1-carbonsäure β-trimethylsilyläthylester das (3RS,4RS)-4-(4-Fluorphenyl)-3-[4-(2-methoxy-äthoxy)-naphthalin-2-ylmethoxy]-piperidin als farbloser Festkörper; MS : 410 $(M+H)^+$;

18) - aus 4-(4-Fluorphenyl)-3-[4-(3-methoxy-propoxy)-naphthalin-2-ylmethoxy]-piperidin-1-carbonsäure β-trimethyl-silyläthylester das 4-(4-Fluorphenyl)-3-[4-(3-methoxy-propoxy)-naphthalin-2-ylmethoxy]-piperidin als leicht gelben Festkörper; MS : 424 $(M+H)^+$;

19) - aus (3RS,4RS) 3-(4-Butoxy-naphthalin-2-ylmethoxy)-4-(4-fluorphenyl)-piperidin-1-carbonsäure β-trimethylsilyläthylester das (3RS,4RS) 3-(4-Butoxy-naphthalin-2-ylmethoxy)-4-(4-fluorphenyl)-piperidin als farbloser Festkörper; MS : 408 $(M+H)^+$;

20) - aus (3RS,4RS)-4-(4-Fluorphenyl)-3-(4-(2-methoxy-benzyloxy)-naphthalin-2-ylmethoxy)-piperidin-1-carbonsäure β-trimethylsilyläthylester das (3RS,4RS)-4-(4-Fluorphenyl)-3-(4-(2-methoxybenzyloxy)-naphthalin-2-ylmethoxy)-piperidin als farbloses Harz; MS : 472 $(M+H)^+$;

21) - aus (3RS,4RS)-4-(4-Fluorphenyl)-3-(4-(3-methoxy-benzyloxy)-naphthalin-2-ylmethoxy)-piperidin-1-carbonsäure β-trimethylsilyläthylester das (3RS,4RS)-4-(4-Fluorphenyl)-3-(4-(3-methoxybenzyloxy)-naphthalin-2-ylmethoxy)-piperidin als farbloser Festkörper; MS : 471 $(M)^+$;

22) - aus (3RS,4RS)-4-(4-Fluorphenyl)-3-(4-methoxy-benzyloxy)-naphthalin-2-ylmethoxy)-piperidin-1-carbonsäure β-trimethylsilyläthylester das (3RS,4RS)-4-(4-Fluorphenyl)-3-(4-(4-methoxybenzyloxy)-naphthalin-2-ylmethoxy)-piperidin als farbloses Öl; MS : 471 $(M)^+$;

23) - aus (3RS,4RS)-4-(4-Fluorphenyl)-3-(1-phenäthyloxy-naphthalin-2-ylmethoxy)-piperidin-1-carbonsäure β-trimethyl-silyläthylester das (3RS,4RS)-4-(4-Fluorphenyl)-3-(1-phenäthyloxy-naphthalin-2-ylmethoxy)-piperidin als leicht gelbes Öl; MS : 456 $(M+H)^+$;

24) - aus (3RS,4RS)-4-(4-Fluorphenyl)-3-(4-phenäthyloxy-naphthalin-2-ylmethoxy)-piperidin-1-carbonsäure β-trimethyl-silyläthylester das (3RS,4RS)-4-(4-Fluorphenyl)-3-(4-phenäthyloxy-naphthalin-2-ylmethoxy)-piperidin als farbloser Festkörper; MS : 456 $(M+H)^+$;

25) - aus (3RS,4RS)-3-[4-(2-[1,3]Dioxolan-2-yl-äthoxy)-naphthalin-2-ylmethoxy]-4-(4-fluorphenyl)-piperidin-1-carbonsäure β-trimethylsilyläthylester das (3RS,4RS)-3-[4-(2-[1,3]Dioxolan-2-yl-äthoxy)-naphthalin-2-ylmethoxy]-4-(4-fluorphenyl)-piperidin als leicht gelbes Öl; MS : 451 $(M)^+$;

26) - aus (3RS,4RS)-3-[1-(2-[1,3]Dioxolan-2-yl-äthoxy)-naphthalin-2-ylmethoxy]-4-(4-fluorphenyl)-piperidin-1-carbonsäure β-trimethylsilyläthylester das (3RS,4RS)-3-[1-(2-[1,3]Dioxolan-2-yl-äthoxy)-naphthalin-2-ylmethoxy]-4-(4-fluorphenyl)-piperidin als leicht gelbes Öl; MS : 452 $(M+H)^+$;

27) - aus (3RS,4RS)-3-[4-(Benzo[1,3]dioxol-5-ylmethoxy)-naphthalin-2-ylmethoxy]-4-(4-fluorphenyl)-piperidin-1-carbonsäure β-trimethylsilyläthylester das (3RS,4RS)-3-[4-(Benzo[1,3]dioxol-5-ylmethoxy)-naphthalin-2-ylmethoxy]-4-(4-fluorphenyl)-piperidin als farbloses Harz; MS : 485 $(M)^+$;

28) - aus (3RS,4RS)-3-[4-(2-Cyclopropyl-äthoxy)-naphthalin-2-ylmethoxy]-4-(4-fluorphenyl)-piperidin- 1-carbonsäure β-trimethylsilyläthylester das (3RS,4RS)-3-[4-(2-Cyclopropyl-äthoxy)-naphthalin-2-ylmethoxy]-4-(4-fluorphenyl)-piperidin als farbloser Festkörper; MS: 419 $(M)^+$;

29) - aus (3RS,4RS)-4-(4-Fluorphenyl)-3-[4-(2-hydroxy-äthoxy)-naphthalin-2-ylmethoxy]-piperidin-1-carbonsäure β-trimethylsilyläthylester das (3RS,4RS)-4-(4-Fluorphenyl)-3-[4-(2-hydroxy-äthoxy)-naphthalin-2-ylmethoxy]-piperidin als farbloser Festkörper; MS : 395 $(M)^+$;

Die als Ausgangssubstanzen eingesetzten Verbindungen wurden wie folgt hergestellt:

(a) 99 mg (0.20 mMol) (3RS,4RS)-4-(4-Fluorphenyl)-3-(4-hydroxynaphthalin-2-ylmethoxy)-piperidin-1-carbonsäure β-trimethylsilyläthylester wurden in 1 ml Dimethylformamid gelöst, mit 69 mg (0.50 mMol) wasserfreiem Kaliumcarbonat und 19 µl (43 mg, 0.30 mMol) Methyljodid versetzt und 4 Stunden bei Raumtemperatur gerührt. Anschließend wurde das Gemisch zwischen Methylenchlorid und Wasser verteilt, die organische Phase über Magnesiumsulfat getrocknet und schließlich das Lösungsmittel unter verminderten Druck abdestilliert. Zur Reinigung wurde das Rohprodukt an Kieselgel mit einem 4:1-Gemisch von Hexan und Methylenchlorid als Eluierungsmittel chromatographiert. Es wurden 85 mg (83% d.Th.) (3RS,4RS)-4-(4-Fluorphenyl)-3-(4-methoxy-naphthalin-2-ylmethoxy)-piperidin-1-carbonsäure β-trimethyl-silyläthylester als leicht gelbes Öl erhalten; MS : 509 $(M)^+$.

In analoger Weise zu dem vorstehend beschriebenen Verfahren wurden die folgenden Verbindungen hergestellt:

- Aus (3RS,4RS)-4-(4-Fluorphenyl)-3-(5-hydroxy-naphthalin-2-ylmethoxy)-piperidin-1-carbonsäure β-trimethylsilyläthylester und Methyljodid der (3RS,4RS)-4-(4-Fluorphenyl)-3-(5-methoxy-naphthalin-2-ylmethoxy)-piperidin-1-carbonsäure β-trimethyl-silyläthylester, der roh in die nächste Stufe eingesetzt wurde;

- aus (3RS,4RS)-4-(4-Fluorphenyl)-3-(6-hydroxy-naphthalin-2-ylmethoxy)-piperidin-1-carbonsäure β-trimethylsilyläthylester und Methyljodid der (3RS,4RS)-4-(4-Fluorphenyl)-3-(6-methoxy-naphthalin-2-ylmethoxy)-piperidin-1-carbonsäure β-trimethyl-silyläthylester als leicht gelbes Öl; MS : 509 $(M)^+$;

- aus (3RS,4RS)-4-(4-Fluorphenyl)-3-(7-hydroxy-naphthalin-2-ylmethoxy)-piperidin-1-carbonsäure β-trimethylsilyläthylester und Methyljodid der (3RS,4RS)-4-(4-Fluorphenyl)-3-(7-methoxy-naphthalin-2-ylmethoxy)-piperidin-1-carbonsäure β-trimethyl-silyläthylester, der roh in die nächste Stufe eingesetzt wurde;

- aus (3RS,4RS)-4-(4-Fluorphenyl)-3-(8-hydroxy-naphthalin-2-ylmethoxy)-piperidin-1-carbonsäure β-trimethylsilyläthylester und Methyljodid der (3RS,4RS)-4-(4-Fluorphenyl)-3-(8-methoxy-naphthalin-2-ylmethoxy)-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl-silyläthylester und 2-Pyridylmethylchlorid der (3RS,4RS)-4-(4-Fluorphenyl)-3-[4-(pyridin-2-ylmethoxy)-naphthalin-2-ylmethoxy]-piperidin-1-carbonsäure β-trimethyl-silyläthylester als leicht gelbes Öl; MS : 587 $(M+H)^+$;

- aus (3RS,4RS)-4-(4-Fluorphenyl)-3-(4-hydroxy-naphthalin-2-ylmethoxy)-piperidin-1-carbonsäure β-trimethyl-silyläthylester und 3-Pyridylmethylchlorid der (3RS,4RS)-4-(4-Fluorphenyl)-3-[4-(pyridin-3-ylmethoxy)-naphthalin-2-ylmethoxy]-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 4-Pyridylmethylchlorid der (3RS,4RS)-4-(4-Fluorphenyl)-3-[4-(pyridin-4-ylmethoxy)-naphthalin-2-ylmethoxy]-piperidin-1-carbonsäure β-trimethyl-silyläthylester als leicht gelbes Öl; MS : 587 $(M+H)^+$;

- aus (3RS,4RS)-4-(4-Fluorphenyl)-3-(4-hydroxy-naphthalin-2-ylmethoxy)-piperidin-1-carbonsäure β-trimethyl- silyläthylester und Allylbromid der (3RS,4RS)-3-(4-Allyloxy-naphthalin-2-ylmethoxy)-4-(4-fluorphenyl)-piperidin-1-carbonsäure β-trimethyl-silyläthylester, der roh in die nächste Stufe eingesetzt wurde;

- aus (3RS,4RS)-4-(4-Fluorphenyl)-3-(6-hydroxy-naphthalin-2-ylmethoxy)-piperidin-1-carbonsäure β-trimethylsilyläthylester und Allylbromid der (3RS,4RS)-3-(6-Allyloxy-naphthalin-2-ylmethoxy)-4-(4-fluorphenyl)-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und iso-Butylbromid der (3RS,4RS)-4-(4-Fluorphenyl)-3-(4-isobutoxy-naphthalin-2-ylmethoxy)-piperidin-1-carbonsäure β-trimethyl-silyläthylester der roh in die nächste Stufe eingesetzt wurde;

- aus (3RS,4RS)-4-(4-Fluorphenyl)-3-(1-hydroxy-naphthalin-2-ylmethoxy)-piperidin-1-carbonsäure β-trimethylsilyläthylester und Benzylbromid der (3RS,4RS)-4-(4-Fluorphenyl)-3-(1-benzyloxynaphthalin-2-ylmethoxy)-piperidin-1-carbonsäure β-trimethylsilyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und Benzylbromid der (3RS,4RS)-3-(4-Benzyloxy-naphthalin-2-ylmethoxy)-4-(4-fluorphenyl)-piperidin-1-carbonsäure β-trimethyl-silyläthylester als leicht gelbes Öl; MS : 586 $(M+H)^+$;

- aus (3RS,4RS)-4-(4-Fluorphenyl)-3-(7-hydroxy-naphthalin-2-ylmethoxy)-piperidin-1-carbonsäure β-trimethylsilyläthylester und Benzylbromid der (3RS,4RS)-3-(7-Benzyloxy-naphthalin-2-ylmethoxy)-4-(4-fluorphenyl)-piperidin-1-carbonsäure β-trimethyl-silyläthylester als leicht gelbes Öl; MS : 585 $(M)^+$;

- aus (3RS,4RS)-4-(4-Fluorphenyl)-3-(5-hydroxy-naphthalin-2-ylmethoxy)-piperidin-1-carbonsäure β-trimethylsilyläthylester und Benzylbromid der (3RS,4RS)-3-(5-Benzyloxy-naphthalin-2-ylmethoxy)-4-(4-fluor-phenyl)-piperidin-1-carbonsäure 2-trimethylsilanyl-äthyl ester als farbloses Öl; MS : 585 (M)+;

- aus (3RS,4RS)-4-(4-Fluorphenyl)-3-(8-hydroxy-naphthalin-2-ylmethoxy)-piperidin-1-carbonsäure β-trimethylsilyläthylester und Benzylbromid der (3RS,4RS)-3-(8-Benzyloxy-naphthalin-2-ylmethoxy)-4-(4-fluorphenyl)-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 2-Methoxyäthylbromid der (3RS,4RS)-4-(4-Fluor-phenyl)-3-[4-(2-methoxy-äthoxy)-naphthalin-2-ylmethoxy]-piperidin-1-carbonsäure 2-trimethylsilanyl-äthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 3-Methoxypropylchlorid [J.Org.Chem. 16, 704(1951)] der (3RS,4RS)-4-(4-Fluor-phenyl)-3-[4-(3-methoxy-propoxy)-naphthalin-2-ylmethoxy]-piperidin-1-carbonsäure 2-trimethylsilanyl-äthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und Butylbromid der (3RS,4RS)-3-(4-Butyl-naphthalin-2-ylmethoxy)-4-(4-fluor-phenyl)-piperidin-1-carbonsäure 2-trimethylsilanyl-äthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 2-Methoxybenzylchlorid der (3RS,4RS)-4-(4-Fluor-phenyl)-3-[4-(2-methoxybenzyloxy)-naphthalin-2-ylmethoxy)-piperidin-1-carbonsäure 2-trimethylsilanyl-äthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 3-Methoxybenzylchlorid der (3RS,4RS)-4-(4-Fluor-phenyl)-3-[4-(3-methoxybenzyloxy)-naphthalin-2-ylmethoxy)-piperidin-1-carbonsäure 2-trimethylsilanyl-äthylester als leicht gelbes Öl; MS : 615 $(M)^+$;

- aus (3RS,4RS)-4-(4-Fluorphenyl)-3-(4-hydroxy-naphthalin-2-ylmethoxy)-piperidin-1-carbonsäure β-trimethyl- silyläthylester und 4-Methoxybenzylchlorid der (3RS,4RS)-4-(4-Fluor-phenyl)-3-[4-(4-methoxybenzyloxy)-naphthalin-2-ylmethoxy)-piperidin-1-carbonsäure 2-trimethylsilanyl-äthylester als leicht gelbes Öl; MS : 616 $(M+H)^+$;

- aus (3RS,4RS)-4-(4-Fluorphenyl)-3-(4-hydroxy-naphthalin-2-ylmethoxy)-piperidin-1-carbonsäure β-trimethyl- silyläthylester und Phenäthylbromid der (3RS,4RS)-4-(4-Fluorphenyl)-3-(4-phenäthyloxynaphthalin-2-ylmethoxy)-piperidin-1-carbonsäure β-trimethylsilyläthylester, der roh in die nächste Stufe eingesetzt wurde;

- aus (3RS,4RS)-4-(4-Fluorphenyl)-3-(1-hydroxy-naphthalin-2-ylmethoxy)-piperidin-1-carbonsäure β-trimethylsilyläthylester und Phenäthylbromid der (3RS,4RS)-4-(4-Fluor-phenyl)-3-(1-phenäthyloxynaphthalin-2-ylmethoxy)-piperidin-1-carbonsäure 2-trimethylsilanyl-äthylester als leicht gelbes Öl; MS : 600 $(M+H)^+$;

- aus (3RS,4RS)-4-(4-Fluorphenyl)-3-(4-hydroxy-naphthalin-2-ylmethoxy)-piperidin-1-carbonsäure β-trimethyl- silyläthylester und 2-(2-Bromäthyl)-1,3-dioxolan der (3RS,4RS)-3-[4-(2-[1,3]Dioxolan-2-yl-äthoxy)-naphthalin-2-ylmethoxy]-4-(4-fluor-phenyl)-piperidin-1-carbonsäure 2-trimethylsilanyl-äthylester, der roh in die nächste Stufe eingesetzt wurde;

- aus (3RS,4RS)-4-(4-Fluorphenyl)-3-(1-hydroxy-naphthalin-2-ylmethoxy)-piperidin-1-carbonsäure β-trimethylsilyläthylester und 2-(2-Bromäthyl)-1,3-dioxolan der (3RS,4RS)-3-[1-(2-[1,3]Dioxolan-2-yl-äthoxy)-naphthalin-2-ylmethoxy]-4-(4-fluorphenyl)-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 3,4-Methylendioxybenzylchlorid der (3RS,4RS)-3-[4-(Benzo[1,3]dioxol-5-ylmethoxy)-naphthalin-2-ylmethoxy]-4-(4-fluorphenyl)-piperidin-1-carbonsäure β-trimethyl-silyläthylester, der roh in die nächste Stufe eingesetzt wurde;

- aus (3RS,4RS)-4-(4-Fluorphenyl)-3-(4-hydroxy-naphthalin-2-ylmethoxy)-piperidin-1-carbonsäure β-trimethyl- silyläthylester und 2-Cyclopropyl-äthylchlorid [Justus Liebigs Ann. Chem. 759, 132 (1972)] der (3RS,4RS)-3-[4-(2-Cyclopropyl-äthoxy)-naphthalin-2-ylmethoxy]-4-(4-fluorphenyl)-piperidin-1-carbonsäure β-trimethyl-silyläthylester als leicht rosafarbenes Öl; MS : 564 $(M+H)^+$;

- aus (3RS,4RS)-4-(4-Fluorphenyl)-3-(4-hydroxy-naphthalin-2-ylmethoxy)-piperidin-1-carbonsäure β-trimethyl- silyläthylester und 2-(2-Bromäthoxy)-tetrahydropyran das Gemisch von (3RS,4RS)-4-(4-Fluorphenyl)-3-{4-[2-[(RS)- und (SR)-tetrahydro-pyran-2-yloxy]-äthoxy]-naphthalin-2-ylmethoxy}-piperidin-1-carbonsäure β-trimethylsilanyläthylester als farbloses Öl; MS : 624 $(M+H)^+$.

Die folgende Abspaltung der THP-Schutzgruppe mit einer IM Lösung von Chlorwasserstoff in Methanol (10 Minuten, Raumtemperatur), lieferte den (3RS,4RS)-4-(4-Fluorphenyl)-3-[4-(2-hydroxy-äthoxy)-naphthalin-2-ylmethoxy]-piperidin-1-carbonsäure β-trimethyl-silyläthylester als farblosen Festkörper; MS : 540 $(M+H)^+$.

Beispiel 15

(a) Eine Lösung von 63 mg (0.116 mMol) (3RS,4RS)-4-(4-Fluorphenyl)-3-[4-(2-hydroxy-äthoxy)-naphthalin-2-ylmethoxy]-piperidin-1-carbonsäure β-trimethyl-silyläthylester in 2 ml Methylenchlorid wurde mit 38 mg (0.58 mMol) Natriumcyanat versetzt. Zu dieser Suspension wurden bei 0° C 44 µl (67 mg, 0.58 mMol) Trifluoressigsäure gegeben und das Reaktionsgemisch 2 Stunden bei Raumtemperatur gerührt. Anschließsend wurde das Gemisch zwischen Methylenchlorid und einer 5%igen Natriumhydrogencarbonat-Lösung verteilt, die organische Phase über Magnesiumsulfat getrocknet und schließlich das Lösungsmittel unter verminderten Druck abdestilliert. Der erhaltene rohe (3RS,4RS)-3-[4-(2-Carbamoyloxy-äthoxy)-naphthalin-2-ylmethoxy]-4-(4-fluorphenyl)-piperidin-1-carbonsäure β-trimethylsilyläthylester wurde ohne weitere Reinigung und Charakterisierung in der folgenden Stufe eingesetzt.

(b) Aus dem rohen (3RS,4RS)-3-[4-(2-Carbamoyloxy-äthoxy)-naphthalin-2-ylmethoxy]-4-(4-fluorphenyl)-piperidin-1-carbonsäure β-trimethylsilyläthylester wurde in Analogie zu dem im Beispiel 1 (e) beschriebenen Verfahren durch Abspaltung der Schutzgruppe mit Tetrabutylammoniumfluorid in Tetrahydrofuran das (3RS,4RS)-3-[4-(2-Carbamoyloxy-äthoxy)-naphthalin-2-ylmethoxy]-4-(4-fluorphenyl)-piperidin als leicht gelbes Öl erhalten; MS : 438 $(M)^+$.

Beispiel 16

In analoger Weise wie in Beispiel 1 (e) beschrieben wurden durch Abspaltung der Schutzgruppe mit Tetrabutylammoniumfluorid in Tetrahydrofuran die folgenden Verbindungen erhalten:

1) - Aus dem 4-(4-Fluorphenyl)-3-[4-[2-(pyridin-2-ylcarbamoyloxy)-äthoxy]-naphthalin-2-ylmethoxy]-piperidin-1-carbonsäure β-trimethylsilyläthylester das 4-(4-Fluorphenyl)-3-[4-[2-(pyridin-2-ylcarbamoyloxy)-äthoxy]-naphthalin-2-ylmethoxy]-piperidin als farbloser Festkörper; MS : 395 $[M-(PyNCO)]^+$;

2) - aus dem (3RS,4RS)-3-[4-(2-Benzoyloxy-äthoxy)-naphthalin-2-ylmethoxy]-4-(4-fluorphenyl)-piperidin-1-carbonsäure β-trimethylsilyläthylester das (3RS,4RS)-3-[4-(2-Benzoyloxy-äthoxy)-naphthalin-2-ylmethoxy]-4-(4-fluorphenyl)-piperidin als farbloses Harz; MS : 500 $(M+H)^+$.

Die als Ausgangsstoffe eingesetzten β-Trimethyl-silyläthylcarbamate wurden wie folgt erhalten:

(a) Eine Lösung von 54 mg (0.10 mMol) (3RS,4RS)-4-(4-Fluorphenyl)-3-[4-(2-hydroxy-äthoxy)-naphthalin-2-ylmethoxyl-piperidin-1-carbonsäure β-trimethyl-silyläthylester in 5 ml Toluol wurde mit 30 mg (0.20 mMol) 2-Pyridylcarbonylazid [Monatsh. Chem. 33, 397 (1912)] und 5 mg 4-Dimethylaminopyridin versetzt. Die Lösung wurde 2 Stunden lang unter Argon zum Rückfluß erhitzt. Das Gemisch wurde abgekühlt und das Lösungsmittel unter verminderten Druck entfernt. Der Rückstand wurde zwischen Methylenchlorid und gesättigter Kochsalzlösung verteilt,

die organische Phase über Magnesiumsulfat getrocknet und schließlich das Lösungsmittel unter verminderten Druck entfernt. Das Rohprodukt (103 mg) wurde durch Chromatographie an Kieselgel mit einem 1:2-Gemisch von Essigester und Hexan als Eluierungsmittel gereinigt. Es wurden 65 mg (99% d.Th.) (3RS,4RS)-4-(4-Fluorphenyl)-3-[4-[2-(pyridin-2-ylcarbamoyloxy)-äthoxy]-naphthalin-2-ylmethoxy]-piperidin-1-carbonsäure β-trimethyl-silyläthylester als farbloser Festkörper erhalten; MS : 660 $(M+H)^+$.

(b) Eine Lösung von 108 mg (0.20 mMol) (3RS,4RS)-4-(4-Fluorphenyl)-3-[4-(2-hydroxy-äthoxy)-naphthalin-2-ylmethoxy]-piperidin-1-carbonsäure β-trimethyl-silyläthylester in 2 ml Methylenchlorid wurde mit 56 µl (41 mg, 0.40 mMol) Triäthylamin versetzt. Dazu wurden 36 µl (42 mg, 0.30 mMol) Benzoylchlorid gegeben. Das Reaktionsgemisch wurde 6 Stunden bei Raumtemperatur und eine Stunde bei 50° C gerührt. Anschließend wurde das Gemisch zwischen Methylenchlorid und einer 5%igen Natriumhydrogencarbonat-Lösung verteilt, die organische Phase über Magnesiumsulfat getrocknet und schließlich das Lösungsmittel unter verminderten Druck entfernt. Der rohe (3RS, 4RS)-3-[4-(2-Benzoyloxy-äthoxy)-naphthalin-2-ylmethoxy]-4-(4-fluorphenyl)-piperidin-1-carbonsäure β-trimethylsilyläthylester wurde direkt in die nächste Stufe eingesetzt.

Beispiel 17

In analoger Weise wie in Beispiel 5 beschrieben, wurde die folgende Verbindung erhalten:

In Analogie zu Beispiel 5 (a)-(d) wurde wie folgt verfahren:

(a) Aus Salicylsäuremethylester wurde durch Einführung der SEM-Gruppe der 2-(2-Trimethylsilyläthoxymethoxy)-benzoesäuremethylester als farbloses Öl erhalten; MS 224 $[M-(C_2H_4 + CH_2O)]^+$.

(b) Die Reduktion des 2-(2-Trimethylsilyl-äthoxymethoxy)-benzoesäuremethylesters ergab das [2-(2-Trimethylsilyläthoxy-methoxy)-phenyl]-methanol als leicht gelbes Öl; MS : 226 $[M-(C_2H_4)]^+$.

(c) Die Chlorierung des [2-(2-Trimethylsilyläthoxy-methoxy)-phenyl]-methanols lieferte das 1-Chlormethyl-2-(2-trimethylsilyl-äthoxymethoxy)-benzol als farbloses Öl; MS : 214, 216 $[M-(C_2H_4 + CH_2O)]^+$.

(d) Die Alkylierung des (3RS,4RS)-4-(4-Fluorphenyl)-3-(4-hydroxynaphthalin-2-ylmethoxy)-piperidin-1-carbonsäure β-trimethylsilyläthylesters [Beispiel 5 (g)] mit 1-Chlormethyl-2-(2-trimethylsilyl-äthoxymethoxy)-benzol lieferte den (3RS, 4RS)-4-(4-Fluorphenyl)-3-[4-[2-(2-trimethylsilyl-äthoxymethoxy)-benzyloxy]-naphthalin-2-ylmethoxy]-piperidin-1-carbonsäure β-trimethylsilyläthylester als farbloses Öl; MS: 749 $(M+NH_4)^+$;

(e) Aus dem (3RS, 4RS)-4-(4-Fluorphenyl)-3-[4-[2-(2-trimethylsilyl-äthoxymethoxy)-benzyloxy]-naphthalin-2-ylmethoxy]-piperidin-1-carbonsäure β-trimethylsilyläthylester wurde, in Analogie zu dem im Beispiel 5 beschriebenen Verfahren, durch Spaltung des β-Trimethylsilyläthylcarbamats mit Tetrabutylammoniumfluorid in Tetrahydrofuran das (3RS, 4RS)-4-(4-Fluorphenyl)-3-[4-[2-(2-trimethylsilyl-äthoxymethoxy)-benzyloxy]-naphthalin-2-ylmethoxy]-piperidin als rosafarbenes Öl erhalten; MS : 588 $(M+H)^+$.

(f) Die Abspaltung der SEM-Gruppe des (3RS, 4RS)-4-(4-Fluorphenyl)-3-[4-[2-(2-trimethylsilyl-äthoxymethoxy)-benzyloxy)-naphthalin-2-ylmethoxy]-piperidins mittels einer 2 N Lösung von Chlorwasserstoff in Methanol, analog zu dem in Beispiel 5 (g) beschriebenen Verfahren, lieferte das (3RS,4RS)-4-(4-Fluorphenyl)-3-[4-[2-hydroxy-benzyloxy]-naphthalin-2-ylmethoxy]-piperidin als farbloses Harz; MS : 458 $(M+H)^+$.

Beispiel 18

In analoger Weise wie in Beispiel 12 (d) beschrieben, wurden durch Spaltung des 2,2,2-Trichloräthylcarbamats die folgenden Verbindungen erhalten:

1) - Aus (3RS,4RS)-4-(2-Fluorphenyl)-3-(naphthalin-2-ylmethoxy)-piperidin-1-carbonsäure 2,2,2-trichloräthylester das (3RS,4RS)-4-(2-Fluorphenyl)-3-(naphthalin-2-ylmethoxy)-piperidin als leicht gelbes Öl; MS : 336 $(M+H)^+$;

2) - aus (3RS,4RS)-4-(3-Fluorphenyl)-3-(naphthalin-2-ylmethoxy)-piperidin-1-carbonsäure 2,2,2-trichloräthylester das (3RS,4RS)-4-(3-Fluorphenyl)-3-(naphthalin-2-ylmethoxy)-piperidin als leicht gelbes Öl; MS : 336 $(M+H)^+$;

3) - aus (3RS,4RS)-3-(Naphthalin-2-ylmethoxy)-4-[3-(2,2,2-trichloräthoxycarbonyloxy)-phenyl)-piperidin-1-carbonsäure 2,2,2-trichloräthylester das (3RS,4RS)-4-(3-Hydroxyphenyl)-3-(naphthalin-2-ylmethoxy)-piperidin als beiger Festkörper; MS : 333 $(M)^+$.

Die als Ausgangsstoffen eingesetzten 2,2,2-Trichloräthylcarbamate wurden wie folgt hergestellt:

(a) In analoger Weise wie in Beispiel 1 (a)-(c) beschrieben, wurde wie folgt verfahren:
Aus 2-Bromfluorbenzol und 1-Benzyl-4-piperidon wurde das 1-Benzyl-4-(2-fluorphenyl)-piperidin-4-ol als gelbes Öl erhalten; MS : 285 $(M)^+$. Die anschließende Elimination lieferte das 1-Benzyl-4-(2-fluorphenyl)-1,2,3,6-tetrahydro-pyridin als leicht gelbes Öl; MS : 267 $(M)^+$. Die darauffolgende Hydroborierung ergab das (3RS,4RS)-1-Benzyl-4-(2-fluorphenyl)-piperidin-3-ol als farblosen Festkörper; MS : 285 $(M)^+$.

(b) In analoger Weise wie im Beispiel 1 (g) beschrieben, wurde durch Alkylierung des (3RS,4RS)-1-Benzyl-4-(2-fluorphenyl)-piperidin-3-ols mit 2-Brommethylnaphthalin das (3RS,4RS)-1-Benzyl-4-(2-fluorphenyl)-3-(naphthalin-2-ylmethoxy)-piperidin als gelbes Öl erhalten; MS: 284 $(M-C_{11}H_9)^+$. Durch Abspaltung der Benzyl-Schutzgruppe mit Chlorameisensäure 2,2,2-trichloräthylester, in analoger Weise wie im Beispiel 12 (c) beschrieben, wurde der (3RS,4RS)-4-(2-Fluorphenyl)-3-(naphthalin-2-ylmethoxy)-piperidin-1-carbonsäure 2,2,2-trichloräthylester als gelbes Öl erhalten; MS : 509 $(M)^+$.

(c) In analoger Weise wie in Beispiel 1 (a)-(c) beschrieben, wurde wie folgt verfahren:
Aus 3-Bromfluorbenzol und 1-Benzyl-4-piperidon wurde das 1-Benzyl-4-(3-fluorphenyl)-piperidin-4-ol als farbloser Festkörper erhalten; MS : 285 $(M)^+$. Die anschließende Elimination lieferte das 1-Benzyl-4-(3-fluorphenyl)-1,2,3,6-tetrahydro-pyridin als leicht gelbes Öl; MS : 267 $(M)^+$. Die darauffolgende Hydroborierung ergab das (3RS, 4RS)-1-Benzyl-4-(3-fluorphenyl)-piperidin-3-ol als farbloses Öl; MS : 285 $(M)^+$.

(d) In analoger Weise wie im Beispiel 1 (g) beschrieben, wurde durch Alkylierung des (3RS,4RS)-1-Benzyl-4-(3-fluorphenyl)-piperidin-3-ols mit 2-Brommethylnaphthalin das (3RS,4RS)-1-Benzyl-4-(3-fluorphenyl)-3-(naphthalin-2-ylmethoxy)-piperidin als farbloses Öl erhalten; MS : 426 $(M+H)^+$. Durch Abspaltung der Benzyl-Schutzgruppe mit Chlorameisensäure 2,2,2-trichloräthylester , in analoger Weise wie im Beispiel 12 (c) beschrieben, wurde der (3RS,4RS)-4-(3-Fluorphenyl)-3-(naphthalin-2-ylmethoxy)-piperidin-1-carbonsäure 2,2,2-trichloräthylester als gelbes Öl erhalten; MS : 510 $(M+H)^+$.

(e) In analoger Weise wie in Beispiel 1 (a)-(c) beschrieben, wurde wie folgt verfahren:
Aus 3-Benzyloxy-jodbenzol [J.Chem.Soc.2857(1932)] und 1-Benzyl-4-piperidon wurde das 1-Benzyl-4-(3-benzyloxy-phenyl)-piperidin-4-ol als leicht gelbes Öl erhalten; MS : 373 $(M)^+$. Die anschließende Elimination lieferte das 1-Benzyl-4-(3-benzyloxy-phenyl)-1,2,3,6-tetrahydro-pyridin als farblosen Festkörper; MS : 355 $(M)^+$. Die darauffolgende Hydroborierung ergab das (3RS,4RS)-1-Benzyl-4-(3-benzyloxy-phenyl)-piperidin-3-ol als farblosen Festkörper; MS: 373 $(M)^+$.

(f) In analoger Weise wie im Beispiel 1 (g) beschrieben, wurde durch Alkylierung des (3RS,4RS)-1-Benzyl-4-(3-benzyloxy-phenyl)-piperidin-3-ols mit 2-Brommethylnaphthalin das (3RS,4RS)-1-Benzyl-4-(3-benzyloxyphenyl)-3-(naphthalin-2-ylmethoxy)-piperidin als farbloses Harz erhalten; MS : 514 $(M+H)^+$.

(g) Eine Lösung von 250 mg (0.487 mMol) (3RS,4RS)-1-Benzyl-4-(3-benzyloxy-phenyl)-3-(naphthalin-2-ylmethoxy)-piperidin in 1.1 ml Methylenchlorid wurde bei Raumtemperatur mit 247 µl (236 mg, 1.946 mMol, 4 Äq.) N, N-Dimethylanilin und 195 mg (1.46 mMol, 3.0 Äq.) Aluminiumtrichlorid versetzt und 2.5 Stunden bei Raumtemperatur gerührt. Anschließend wurde das Gemisch zwischen Methylenchlorid und 5%iger Natriumhydrogencarbonat-Lösung verteilt, die organische Phase über Magnesiumsulfat getrocknet und schließlich das Lösungsmittel unter verminderten Druck entfernt. Das Rohprodukt wurde durch Chromatographie an Kieselgel mit einem 4:1-Gemisch von Methylenchlorid und Hexan als Eluierungsmittel gereinigt. Es wurden 65 mg (32% d.Th.) (3RS,4RS)-1-Benzyl-4-(3-hydroxyphenyl)-3-(naphthalin-2-ylmethoxy)-piperidin als beigefarbener Festkörper erhalten; MS : 423 $(M)^+$.

(h) In analoger Weise wie im Beispiel 12 (c) beschrieben, wurde durch Abspaltung der Benzyl-Gruppe mit Chlorameisensäure 2,2,2-trichloräthylester der (3RS,4RS)-3-(Naphthalin-2-ylmethoxy)-4-[3-(2,2,2-trichlor-äthoxycarbonyloxy)-phenyl]-piperidin-1-carbonsäure 2,2,2-trichloräthylester erhalten, der als Rohprodukt direkt in die nächste Stufe eingesetzt wurde.

Beispiel 19

In Analogie zu dem in Beispiel 2 (e) beschriebenen Verfahren, wurde durch katalytische Hydrierung des (3RS, 4RS)-1-Benzyl-4-(3-fluorphenyl)-piperidin-3-ols das (3RS,4RS)-4-(3-Fluorphenyl)-piperidin-3-ol als farbloser Festkörper erhalten; MS: 196 $(M+H)^+$. Die Einführung der BOC-Gruppe, in analoger Weise wie in Beispiel 1 (f) beschrieben,

lieferte den (3RS,4RS)-4-(3-Fluorphenyl)-3-hydroxy-piperidin-1-carbonsäure tert-butylester als farblosen Festkörper; MS : 296 $(M+H)^+$. Die anschließende Alkylierung mit 4-Benzyloxy-2-chlormethylnaphthalin, in Analogie zu dem im Beispiel 1 (g) beschriebenen Verfahren, ergab den (3RS,4RS)-3-(4-Benzyloxy-naphthalin-2-ylmethoxy)-4-(3-fluorphenyl)-piperidin- 1-carbonsäure tert-butylester als farblosen Festkörper; MS : 541 $(M)^+$. Die Abspaltung der BOC-Gruppe mittels einer Lösung von Chlorwasserstoff in Methanol, analog zu dem in Beispiel 1 (h) beschriebenen Verfahren, führte schließlich zum (3RS,4RS)-3-(4-Benzyloxy-naphthalin-2-ylmethoxy)-4-(3-fluorphenyl)-piperidin, das als farbloser Festkörper erhalten wurde; MS : 442 $(M+H)^+$.

Das als Ausgangsverbindung eingesetzte 4-Benzyloxy-2-chlormethyl-naphthalin wurde wie folgt erhalten:

(a) In analoger Weise wie in Beispiel 14 (a) beschrieben, wurde durch Alkylierung des 4-Hydroxy-naphthalin-2-carbonsäureäthylesters [J.Agric.Chem Soc.Japan 24, 313 (1950)] mit Benzylbromid der 4-Benzyloxy-naphthalin-2-carbonsäureäthylester als fast farbloser Festkörper erhalten; MS : 216 $(M)^+$.

(b) Die Reduktion von 4-Benzyloxy-naphthalin-2-carbonsäureäthylester analog zu Beispiel 5 (b) lieferte das (4-Benzyloxy-naphthalin-2-yl)-methanol als farblosen Festkörper; MS : 264 $(M)^+$.

(c) Die Chlorierung von (4-Benzyloxy-naphthalin-2-yl) mittels Tetrachlorkohlenstoff, analog zu Beispiel 7 (c), lieferte das 4-Benzyloxy-2-chlormethyl-naphthalin als farblosen Festkörper; MS : 282 $(M)^+$.

Beispiel 20

In analoger Weise wie in Beispiel 1 (h) beschrieben, wurden durch Abspaltung der BOC-Gruppe mittels einer Lösung von Chlorwasserstoff in Methanol die folgenden Verbindungen erhalten:

1) - Aus (3RS,4RS)-4-(4-Cyan-phenyl)-3-(naphthalin-2-ylmethoxy)-piperidin-1-carbonsäure tert-butylester das (3RS,4RS)-4-(4-Cyanphenyl)-3-(naphthalin-2-ylmethoxy)-piperidin als leicht gelber Festkörper; MS : 342 $(M+H)^+$;

2) - aus 4-[4-(Phenylsulfonylamino-methyl)-phenyl]-3-(naphthalin-2-ylmethoxy)-piperidin-1-carbonsäure tert-butylester das (3RS,4RS)-4-[4-(Phenylsulfonylamino-methyl)-phenyl]-3-(naphthalin-2-ylmethoxy)-piperidin als farbloser Festkörper; MS : 485 $(M-H)^-$;

3) - aus (3RS,4RS)-4-[4-(4-Methoxy-benzoylamino)-methyl]-phenyl-3-(naphthalin-2-ylmethoxy)-piperidin-1-carbonsäure tert-butylester das (3RS,4RS)-4-[4-(4-Methoxy-benzoylamino)-methyl]-phenyl-3-(naphthalin-2-ylmethoxy)-piperidin als farbloses Harz; MS : 481 $(M+H)^+$;

4) - aus (3RS,4RS)-3-(Naphthalin-2-ylmethoxy)-4-[4-(phenylacetylamino-methyl)-phenyl]-piperidin-1-carbonsäure tert-butylester das (3RS,4RS)-3-(Naphthalin-2-ylmethoxy)-4-[4-(phenylacetyl-aminomethyl)-phenyl]-piperidin als farbloses Harz; MS : 465 $(M+H)^+$;

5) - aus (3RS,4RS)-4-[4-(Benzoylamino-methyl)-phenyl]-3-[4-(2-trimethylsilyl-äthoxymethoxy)-naphthalin-2-ylmethoxy]-piperidin-1-carbonsäure tert-butylester durch gleichzeitige Abspaltung der BOCund SEM-Gruppe, analog wie in Beispiel 3 und 5 (g) beschrieben, das (3RS,4RS)-4-[4-(Benzoylamino-methyl)-phenyl]-3-(4-hydroxy-naphthalin-2-ylmethoxy)-piperidin als leicht orangefarbener Festkörper; MS: 467 $(M+H)^+$.

Die als Ausgangstoffe eingesetzten BOC-Verbindungen wurden wie folgt hergestellt:

(a) Eine Suspension von 20 mg (0.30 mMol) aktiviertem Zinkpulver, 76 mg (1.17 mMol) Kaliumcyanid, 52 mg (0.20 mMol) Triphenylphosphin und 74 mg (0.10 mMol) Bis(triphenylphosphin)-nickel(II)-dibromid in 2 ml Acetonitril wurde unter Argon 5 Minuten lang bei 60° C erhitzt. Dazu wurden 356 mg (1.00 mMol) (3RS,4RS)-4-(4-Bromphenyl)-3-hydroxy-piperidin-1-carbonsäure tert-butylester fester Form gegeben. Die grüne Suspension wurde unter Argon 20 Stunden lang bei 60° C gerührt. Die entstandene dunkelbraune Suspension wurde über Speedex filtriert und das ungelöste Material mit Methylenchlorid gewaschen. Das Filtrat wurde zwischen Methylenchlorid und 5%iger Natriumhydrogencarbonat-Lösung verteilt, die organische Phase über Magnesiumsulfat getrocknet und schließlich das Lösungsmittel unter vermindertem Druck entfernt. Das Rohprodukt (416 mg) wurde durch Chromatographie an Kieselgel mit einem 1:1-Gemisch von Essigester und Hexan als Eluierungsmittel gereinigt. Es wurden 168 mg (56% d.Th.) (3RS,4RS)-4-(4-Cyan-phenyl)-3-hydroxy-piperidin-1-carbonsäure tert-butylester als farbloser Festkörper erhalten; MS: 302 $(M)^+$.

(b) In Analogie zu dem in Beispiel 1 (g) beschriebenen Verfahren, wurde durch die Alkylierung des (3RS,4RS)-4-(4-Cyan-phenyl)-3-hydroxy-piperidin-1-carbonsäure tert-butylesters mit 2-Brommethyl-naphthalin der (3RS, 4RS)-4-(4-Cyan-phenyl)-3-(naphthalin-2-ylmethoxy)-piperidin-1-carbonsäure tert-butylester als leicht gelbes Harz erhalten; MS : 443 $(M+H)^+$.

(c) Eine Lösung von 133 mg (0.301 mMol) (3RS,4RS)-4-(4-Cyan-phenyl)-3-(naphthalin-2-ylmethoxy)-piperidin-1-carbonsäure tert-butylester in 0.5 ml Tetrahydrofuran wurde mit 1.5 ml (1.5 mMol) einer 1 M Boran-Tetrahydrofuran-Komplex-Lösung in Tetrahydrofuran versetzt und 6 Stunden lang unter Argon zum Rückfluß erhitzt. Das Reaktions-gemisch wurde zwischen Methylenchlorid und Wasser verteilt, die organische Phase über Magnesiumsulfat getrocknet und schließlich das Lösungsmittel unter vermindertem Druck entfernt. Das Rohprodukt (163 mg) wurde durch Chromatographie an Kieselgel mit einem 14:1:0.1-Gemisch von Methylenchlorid, Methanol und einer 25%igen Ammoniak-Lösung als Eluierungsmittel gereinigt. Es wurden 106 mg (79% d.Th.) (3RS,4RS)-4-(4-Aminomethyl-phenyl)-3-(naphthalin-2-ylmethoxy)-piperidin-1-carbonsäure tert-butylester als farbloses Harz erhalten; MS : 447 $(M+H)^+$.

(d) Eine Lösung von 47 mg (0.105 mMol) (3RS,4RS)-4-(4-Aminomethylphenyl)-3-(naphthalin-2-ylmethoxy)-piperidin-1-carbonsäure tert-butylester in 2 ml Methylenchlorid wurde mit 18 µl (12.7 mg, 0.126 mMol, 1.2 Äq.) Triäthylamin versetzt und auf 0° C abgekühlt. Es wurden tropfenweise 15 µl (20.4 mg, 0.116 mMol, 1.1 Äq.) Benzolsulfochlorid zugegeben, das Gemisch auf Raumtemperatur erwärmt und 1 Stunde lang nachgerührt. Das Reaktionsgemisch wurde zwischen Methylenchlorid und 5%iger Natriumhydrogen-carbonat-Lösung verteilt, die organische Phase über Magnesiumsulfat getrocknet und schließlich das Lösungsmittel unter vermindertem Druck entfernt. Es wurden 47 mg roher (3RS,4RS)-4-[4-(Phenylsulfonylamino-methyl)-phenyl]-3-(naphthalin-2-ylmethoxy)-piperidin-1-carbonsäure tert-butylester erhalten, der ohne weitere Reinigung und Charakterisierung in der folgenden Stufe eingesetzt wurde.

(e) In analoger Weise zu dem unter (d) beschriebenen Verfahren, wurde aus dem (3RS,4RS)-4-(4-Aminomethyl-phenyl)-3-(naphthalin-2-ylmethoxy)-piperidin-1-carbonsäure tert-butylester durch Acylierung mit p-Anisoylchlorid der rohe (3RS,4RS)-4-[4-(4-Methoxy-benzoylamino)-methyl]-phenyl-3-(naphthalin-2-ylmethoxy)-piperidin-1-carbonsäure tert-butylester erhalten, der ohne weitere Reinigung und Charakterisierung in der folgenden Stufe eingesetzt wurde.

(f) In analoger Weise zu dem unter (d) beschriebenen Verfahren, wurde aus dem (3RS,4RS)-4-(4-Aminomethyl-phenyl)-3-(naphthalin-2-ylmethoxy)-piperidin-1-carbonsäure tert-butylester durch Acylierung mit Phenylessigsäurechlorid der rohe (3RS,4RS)-3-(Naphthalin-2-ylmethoxy)-4-[4-(phenylacetylamino-methyl)-phenyl]-piperidin-1-carbonsäure tert-butylester erhalten, der ohne weitere Reinigung und Charakterisierung in der folgenden Stufe eingesetzt wurde.

(g) In analoger Weise wie im Beispiel 1 (g) beschrieben, wurde durch die Alkylierung des (3RS,4RS)-4-(4-Cyan-phenyl)-3-hydroxy-piperidin-1-carbonsäure tert-butylesters mit 2-Chlormethyl-4-(β-trimethylsilyl-äthoxymethoxy)-naphthalin der (3RS,4RS)-4-(4-Cyan-phenyl)-3- [4-(2-trimethylsilyl-äthoxymethoxy)-naphthalin-2-ylmethoxy]-piperidin-1-carbonsäure tert-butylester als leicht gelbes Öl erhalten; MS : 530 $[M-(C_2H_4+CH_2O)]^+$.

(h) In Analogie zu dem unter (c) beschriebenen Verfahren wurde durch Reduktion der Nitrilgruppe aus dem (3RS, 4RS)-4-(4-Cyanphenyl)-3-[4-(2-trimethylsilyl-äthoxymethoxy)-naphthalin-2-ylmethoxy]-piperidin-1-carbonsäure tert-butylester der (3RS,4RS)-4-(4-Aminomethyl-phenyl)-3-[4-(2-trimethylsilyl-äthoxymethoxy)-naphthalin-2-yhnethoxyl-piperidin-1-carbonsäure tert-butylester als leicht gelbes Öl erhalten; MS : 593 $(M+H)^+$.

(i) In Analogie zu dem unter (d) beschriebenen Verfahren wurde aus dem (3RS,4RS)-4-(4-Aminomethyl-phenyl)-3-[4-(2-trimethylsilyl-äthoxymethoxy)-naphthalin-2-ylmethoxy]-piperidin-1-carbonsäure tert-butylester durch Acylierung mit Benzoylchlorid der rohe (3RS,4RS)-4-[4-(Benzoylamino-methyl)-phenyl]-3-[4-(2-trimethylsilyl-äthoxymethoxy)-naphthalin-2-ylmethoxy]-piperidin-1-carbonsäure tert-butylester erhalten, der ohne weitere Reinigung und Charakterisierung in der folgenden Stufe eingesetzt wurde.

Beispiel 21

In analoger Weise wie in Beispiel 1 (h) beschrieben, wurden durch Abspaltung der BOC-Gruppe mittels einer Lösung von Chlorwasserstoff in Methanol die folgenden Verbindungen erhalten:

1) - Aus (3RS,4RS)-4-(4-Chlorphenyl)-4-hydroxy-3-(4-methoxybenzyloxy)-piperidin-1-carbonsäure tert-butylester das (3RS,4RS)-4-(4-Chlorphenyl)-4-hydroxy-3-(4-methoxy-benzyloxy)-piperidin als farbloser Festkörper; MS : 348 $(M+H)^+$;

2) - aus (3RS,4RS)-4-(4-Chlorphenyl)-4-hydroxy-3-(naphthalin-2-ylmethoxy)-piperidin-1-carbonsäure tert-butylester das (3RS,4RS)-4-(4-Chlorphenyl)-4-hydroxy-3-(naphthalin-2-ylmethoxy)-piperidin als farbloser Festkörper; MS : 226, 228 $[M-(C_{11}H_9)]^+$.

Die als Ausgangsstoffe eingesetzten Verbindungen wurden wie folgt hergestellt:

(a) Eine Lösung von 3.0 g (15.5 mMol) 4-(4-Chlorphenyl)-1,2,3,6-tetrahydro-pyridin in 20 ml absolutem Dimethylformamid wurde mit 2.37 ml (1.72 g, 17.0 mMol) Triäthylamin versetzt und auf 0° C abgekühlt. Eine Lösung von 3.7 g (17.0 mMol) Di-tert-butyldicarbonat in 8 ml absolutem Dimethylformamid wurde bei 0° C tropfenweise zugegeben. Das Gemisch wurde auf Raumtemperatur erwärmt und 20 Stunden gerührt. Das Lösungmittel wurde bei 0.1 mm Hg bei 50-55° C abdestilliert. Anschließend wurde der erhaltener Rückstand zwischen Methylenchlorid und Wasser verteilt, die organische Phase über Magnesiumsulfat getrocknet und schließlich das Lösungsmittel unter vermindertem Druck entfernt. Das Rohprodukt (5.0 g) wurde durch Chromatographie an Kieselgel mit einem 95:5-Gemisch von Methylenchlorid und Essigester als Eluierungsmittel gereinigt. Es wurden 4.5 g (99% d.Th.) 4-(4-Chlorphenyl)-1,2,3,6-tetrahydro-pyridin-1-carbonsäure tert-butylester als farbloses Öl erhalten; MS :236, 238 $[M-(C_4H_9)]^+$.

(b) Eine Lösung von 2.5 g (8.5 mMol) 4-(4-Chlorphenyl)-1,2,3,6-tetrahydro-pyridin-1-carbonsäure tert-butylester in 20 ml Aceton wurde mit 0.425 ml (0.0085 mMol, 0.01 Äq.) Osmiumtetroxid-Lösung (0.02 M in tert-Butanol) und 8.6 ml Wasserstoffperoxid-Lösung (30%ig in Wasser) versetzt und das Gemisch 18 Stunden bei Raumtemperatur gerührt. Das Gemisch wurde zwischen Essigester und Wasser verteilt, die organische Phase mit 10%iger Natriumbisulfit-Lösung und Wasser gewaschen, über Magnesiumsulfat getrocknet und schließlich das Lösungsmittel unter vermindertem Druck entfernt. Das Rohprodukt (2.2 g) wurde durch Chromatographie an Kieselgel mit einem 3:1-Gemisch von Methylenchlorid und Essigester als Eluierungsmittel gereinigt. Es wurden 544 mg (20% d.Th.) (3RS,4RS)-4-(4-Chlorphenyl)-3,4-dihydroxy-piperidin-1-carbonsäure tert-butylester als farbloses Harz erhalten; MS : 270, 272 $[M-(C_4H_9)]^+$.

(c) Zu einer Suspension von 16 mg (0.4 mMol) Natriumhydrid (60 %ige Dispersion in Weißöl) in 3 ml Dimethylsulfoxid wurde tropfenweise eine Lösung von 128 mg (0.392 mMol) (3RS,4RS)-4-(4-Chlorphenyl)-3,4-dihydroxy-piperidin-1-carbonsäure tert-butylester in 1.5 ml Dimethylsulfoxid gegeben. Nach 15 Minuten wurde bei Raumtemperatur eine Lösung von 61 mg (0.39 mMol) p-Methoxybenzylchlorid in 1 ml Dimethylsulfoxid innerhalb von 10 Minuten tropfenweise zugegeben und das Gemisch 18 Stunden gerührt. Anschließend wurde das Reaktionsgemisch zwischen Essigester und Wasser verteilt, die organische Phase über Magnesiumsulfat getrocknet und schließlich das Lösungsmittel unter vermindertem Druck entfernt. Das Rohprodukt (200 mg) wurde durch Chromatographie an Kieselgel mit einem 3:1-Gemisch von Hexan und Essigester als Eluierungsmittel gereinigt. Es wurden (neben Ausgangsmaterial und bisalkyliertem Produkt) 63 mg (38% d.Th.) (3RS,4RS)-4-(4-Chlorphenyl)-4-hydroxy-3-(4-methoxy-benzyloxy)-piperidin-1-carbonsäure tert-butylester als farbloses Öl erhalten; MS : 448, 450 $[M+H]^+$.

(d) In analoger Weise zu dem unter (c) beschriebenen Verfahren, wurde durch Alkylierung des (3RS,4RS)-4-(4-Chlorphenyl)-3,4-dihydroxy-piperidin-1-carbonsäure tert-butylesters mit 2-Brommethyl-naphthalin der (3RS, 4RS)-4-(4-Chlorphenyl)-4-hydroxy-3-(naphthalin-2-ylmethoxy)-piperidin-1-carbonsäure tert-butylester als leicht gelbes Öl erhalten; MS : 411, 413 $[M-(C_4H_8)]^+$.

Beispiel 22

(a) Zu einer auf -70° C gekühlten Lösung von 112.5 g (0.48 Mol) 1,4-Dibrombenzol in 1200 ml Diäthyläther wurden innerhalb von 45 Minuten 400 ml einer 1.2 M Lösung von n-BuLi in Hexan so getropft, daß die Temperatur nicht über -60° C stieg. Nach beendeter Zugabe wurde 2.5 Stunden lang bei -70° C nachgerührt. Danach wurde eine Lösung von 90.84 g (0.48 Mol) 1-Benzyl-4-piperidon in 300 ml Äther während einer Stunde bei -70 bis -65° C zugetropft. Nach dem Zutropfen wurde 2 Stunden lang bei -70° C gerührt. Zur Aufarbeitung wurde das kalte Reaktionsgemisch auf 1200 ml einer 15%igen Ammoniumchlorid-Lösung gegossen, das Gemisch in einen Scheidetrichter überführt und die organische Phase abgetrennt. Die wäßrige Phase wurde zweimal mit Äther extrahiert,

und anschließend wurden die vereinigten organischen Phasen zweimal mit Wasser und gesättigter Natriumchlorid-Lösung extrahiert. Dann wurde die organische Phase über Magnesiumsulfat getrocknet unter vermindertem Druck eingedampft, wobei das Rohprodukt als gelblicher Festkörper anfiel. Zur Reinigung wurde dieser in heißem Methylenchlorid gelöst, die Lösung mit Hexan bis zur beginnenden Trübung versetzt und unter Rühren auf Raumtemperatur abgekühlt. Der entstandene Niederschlag wurde abgesaugt und getrocknet. Es wurden 121.65 g (73% d.Th.) 1-Benzyl-4-(4-brom-phenyl)-piperidin-4-ol als gelblicher Festkörper erhalten; Smp.: 106° C, MS : 346, 348 $(M+H)^+$.

(b) Ein Gemisch von 121.6 g (0.35 Mol) 1-Benzyl-4-(4-brom-phenyl)-piperidin-4-ol und 121.6 g p-Toluolsulfonsäure Monohydrat (0.64 Mol) in 1200 ml Toluol wurde während 4.5 Stunden am Wasserabscheider zum Rückfluß erhitzt. Anschließend wurde das Reaktionsgemisch auf Raumtemperatur abgekühlt und mit 3N Natronlauge auf pH 10 gestellt. Danach wurde zunächst mit 2000 ml und dann mit 500 ml Methylenchlorid extrahiert. Die vereinigten organischen Phasen wurden zweimal mit je 1000 ml Wasser gewaschen, über Magnesiumsulfat getrocknet und dann unter vermindertem Druck eingedampft. Es wurden 109.1 g (99% d.Th.) 1-Benzyl-4-(4-brom-phenyl)-1,2,3,6-tetrahydro-pyridin als gelblicher Festkörper erhalten; MS : 328, 330 $(M+H)^+$.

(c) Zu einer Lösung von 51.1 g (0.156 Mol) 1-Benzyl-4-(4-brom-phenyl)-1,2,3,6-tetrahydro-pyridin in 350 ml Dimethoxyäthan wurden 16.9 g Natriumborhydrid gegeben und das Reaktionsgemisch 15 Minuten lang bei Raumtemperatur gerührt. Danach wurden während 30 Minuten 95.2 ml Bortrifluorid-Ätherat bei 25-30° C zugetropft, das Reaktionsgemisch anschließend 2 Stunden bei Raumtemperatur nachgerührt. In der Folge wurde zunächst eine Lösung von 98.4 g Kaliumhydroxid in 530 ml Wasser langsam zugetropft und danach innerhalb von 15 Minuten 80.7 ml einer 30%igen Wasserstoffperoxid-Lösung. Anschließend wurde 2 Stunden lang unter Rückfluß gekocht. Zur Aufarbeitung wurde das abgekühlte Reaktionsgemisch über Dicalit filtriert und dieses mit Methylenchlorid nachgewaschen. Die erhaltene Lösung wurde mit 700 ml Methylenchlorid versetzt, die organische Phase abgetrennt und dann die wäßrige Phase mit 300 ml Methylenchlorid nachextrahiert. Die vereinigten organischen Phasen wurden zweimal mit je 200 ml Wasser gewaschen, über Magnesiumsulfat getrocknet und unter vermindertem Druck eingedampft. Die Kristallisation des Rohprodukts aus Aceton lieferte 31 g (57% d.Th.) (3RS,4RS)-1-Benzyl-4-(4-brom-phenyl)-piperidin-3-ol als farblose Kristalle; Smp.: 125-129°C.

(d) Ein Schlenkrohr wurde unter Argon mit 74.9 mg (0.29 mMol) $PdCl_2(CH_3CN)_2$, 168.0 mg (0.303 mMol) 1,1'-Bis (diphenylphosphino)-ferrocen und 10 ml Methanol (unter Argon destilliert) beladen und das Reaktionsgemisch 30 Minuten bei Raumtemperatur gerührt. Die rotbraune Suspension wurde unter Argon in einen mit einem Glaseinsatz versehenen 185 ml Stahlautoklaven transferiert. Danach wurden 10.0 g (29 mMol) (3RS,4RS)-1-Benzyl-4-(4-brom-phenyl)-piperidin-3-ol (mit Aktivkohle vorbehandelt), 60 ml Methanol und 6 ml (43 mMol) Träthylamin zugegeben. Der Autoklav wurde verschlossen, Kohlenmonoxid mit 15 bar aufgedrückt und das Reaktionsgemisch bei 110° C 20 Stunden lang unter konstantem Druck gerührt. Nach dem Abkühlen des Autoklaven und Entspannen der Gase wurde das orangefarbene Reaktionsgemisch unter vermindertem Druck eingedampft. Der feste, orangefarbene Rückstand wurde in 20 ml Methylenchlorid gelöst, Die Lösung wurde zweimal mit je 100 ml 5%iger Natriumcarbonat-Lösung bzw. mit 100 ml Wasser gewaschen und dann unter vermindertem Druck eingedampft. Der gelbbraune feste Rückstand wurde zur Reinigung an Kieselgel unter Verwendung eines 1:1-Gemisches von Essigester und Hexan als Eluierungsmittel chromatographiert. Es wurden 7.74 g (82% d.Th.) (3RS,4RS)-4-(1-Benzyl-3-hydroxy-piperidin-4-yl)-benzoesäuremethylester als weißer Festkörper erhalten; Smp.: 103-104° C; MS : 326 $(M+H)^+$.

(e) Eine Lösung von 5.0 g (15.3 mMol) (3RS,4RS)-4-(1-Benzyl-3-hydroxypiperidin-4-yl)-benzoesäuremethylester in 50 ml Tetrahydrofuran wurde bei Raumtemperatur mit 720 mg (32.9 mMol) Lithiumborhydrid versetzt. Anschließend wurde das Reaktionsgemisch 15 Stunden lang auf 60° C erwärmt. Zur Aufarbeitung wurde das Reaktionsgemisch unter Eiskühlung mit 20 ml Wasser versetzt und danach zweimal mit je 50 ml Essigester ausgeschüttelt. Die organischen Phasen wurden vereinigt, über Natriumsulfat getrocknet und unter vermindertem Druck eingedampft. Das erhaltene rohe (3RS,4RS)-1-Benzyl-4-(4-hydroxymethyl-phenyl)-piperidin-3-ol (Rf: 0.23, $SiO_2$, Methylenchlorid:Methanol:Ammoniak=95:5:0.1) wurde ohne weitere Reinigung in der folgenden Stufe eingesetzt.

(f) Eine Lösung von 2.0 g (6.72 mMol) (3RS,4RS)-1-Benzyl-4-(4-hydroxymethyl-phenyl)-piperidin-3-ol in 100 ml Äthanol wurde in Gegenwart von 1.0 g Palladiumoxid/Kohle (20%ig) bei Raumtemperatur und 3 bar 4 Stunden lang hydriert. Zur Aufarbeitung wurde der Katalysator über Dicalit abgesaugt und der Rückstand zweimal mit je 50 ml Äthanol gewaschen. Die Äthanollösung wurde unter vermindertem Druck eingedampft und der Rückstand an Kieselgel unter Verwendung eines 65:10:1-Gemisches von Methylenchlorid, Methanol und Ammoniak als Eluierungsmittel chromatographiert. Es wurden 1.1 g (79% d.Th.) (3RS,4RS)-4-(4-Hydroxymethyl-phenyl)-piperidin-3-ol als farbloser Festkörper erhalten; MS : 207 $(M)^+$.

(g) Eine Lösung von 1.10 g (5.31 mMol) (3RS,4RS)-4-(4-Hydroxymethylphenyl)-piperidin-3-ol in 20 ml Dimethylformamid wurde bei 0° C mit 0.59 g (5.82 mMol) Triäthylamin und 1.22 g (5.57 mMol) Di-tert-butyldicarbonat versetzt und während 15 Stunden bei Raumtemperatur gerührt. Anschließend wurde das Dimethylformamid im Ölpumpenvakuum abdestilliert und zur Reinigung des Rückstandes an Kieselgel unter Verwendung eines 98: 2-Gemisches von Methylenchlorid und Methanol als Eluierungsmittel chromatographiert. Es wurden 1.51 g (92% d.Th.) (3RS,4RS)-3-Hydroxy-4-(4-hydroxymethylphenyl)-piperidin-1-carbonsäure tert-butylester als farbloser Schaum erhalten; MS : 233 $(M-C_4H_{10}O)^+$.

(h) Eine Lösung von 1.50 g (5.14 mMol) (3RS,4RS)-3-Hydroxy-4-(4-hydroxymethyl-phenyl)-piperidin-1-carbonsäure tert-butylester, 1.73 g (6.16 mMol) Triphenylchlormethan und 674 mg (6.68 mMol) Triäthylamin in 20 ml Methylenchlorid wurde 5 Stunden lang bei Raumtemperatur gerührt. Zur Aufarbeitung wurde das Reaktionsgemisch mit 10 ml Wasser und 10 ml gesättigter Natriumhydrogencarbonat-Lösung gewaschen, die organische Phase über Natriumsulfat getrocknet undunter vermindertem Druck eingedampft. Das Rohprodukt wurde an Kieselgel unter Verwendung eines 95:5-Gemisches von Toluol und Essigester als Eluierungsmittel chromatographiert. Es wurden 2.08 g (77.5% d.Th.) (3RS,4RS)-3-Hydroxy-4-(4-trityloxymethyl-phenyl)-piperidin-1-carbonsäure tert-butylester als farbloser Schaum erhalten; MS : 567 $(M+NH_4)^+$.

(i) Zu einer Lösung von 2.08 g (3.78 mMol) (3RS,4RS)-3-Hydroxy-4-(4-trityloxymethyl-phenyl)-piperidin-1-carbonsäure tert-butylester und 1.0 g (4.54 mMol) 2-Brommethylnaphthalin in 30 ml Dimethylformamid wurden 290 mg (6.05 mMol) Natriumhydrid (50%ige Dispersion in Weißöl) gegeben und die Reaktionsmischung 2 Stunden lang bei Raumtemperatur gerührt. Zur Aufarbeitung wurde das Reaktionsgemisch im Ölpumpenvakuum eingedampft, der Rückstand zwischen 100 ml gesättigter Ammoniumchlorid-Lösung und 100 ml Essigester verteilt und danach die abgetrennte Wasserphase zweimal mit je 50 ml Essigester ausgeschüttelt. Die vereinigten Essigesterextrakte wurden über Natriumsulfat getrocknet und unter vermindertem Druck eingedampft. Zur Reinigung des Rohproduktes wurde an Kieselgel unter Verwendung eines 4:1-Gemisches von Methylenchlorid und Hexan als Eluierungsmittel chromatographiert. Es wurden 2.27 g (87% d.Th.) (3RS,4RS)-3-Naphthalin-2-ylmethoxy-4-(4-trityloxymethyl-phenyl)-piperidin-1-carbonsäure tert-butylester als farbloser Festkörper erhalten; MS : 707 $(M+NH_4)^+$.

(j) Eine Lösung von 1.07 g (1.48 mMol) (3RS,4RS)-3-Naphthalin-2-ylmethoxy-4-(4-trityloxymethyl-phenyl)-piperidin-1-carbonsäure tert-butylester in 15 ml Methylenchlorid wurde bei Raumtemperatur mit 2 ml 2 N Chlorwasserstoff in Methanol versetzt und 15 Minuten lang bei Raumtemperatur gerührt. Anschließend wurde die Lösung in 30 ml gesättigte Natriumcarbonat-Lösung gegossen und diese zweimal mit je 50 ml Essigester ausgeschüttelt. Die vereinigten organischen Phasen wurden über Natriumsulfat getrocknet und unter vermindertem Druck eingedampft. Zur Reinigung des Rohproduktes wurde an Kieselgel unter Verwendung eines 99:1-Gemisches von Methylenchlorid und Methanol als Eluierungsmittel chromatographiert. Es wurden 580 mg (82% d.Th.) (3RS,4RS)-4-(4-Hydroxymethyl-phenyl)-3-(naphthalin-2-ylmethoxy)-piperidin-1-carbonsäure tert-butylester als farbloses Öl erhalten; MS : 447 $(M)^+$.

(k) Eine Lösung von 45 mg (0.1 mMol) (3RS,4RS)-4-(4-Hydroxymethylphenyl)-3-(naphthalin-2-ylmethoxy)-piperidin-1-carbonsäure tert-butylester, 12 mg (0.12 mMol) Triäthylamin und 12 mg (0.1 mMol) Pivalinsäurechlorid in 5 ml Methylenchlorid wurde bei Raumtemperatur 15 Stunden lang gerührt. Zur Aufarbeitung wurde die Reaktionslösung mit 10 ml Methylenchlorid verdünnt, dann mit 5 ml Wasser gewaschen, über Natriumsulfat getrocknet und unter vermindertem Druck eingedampft. Der Rückstand wurde an Kieselgel unter Verwendung eines 4:1-Gemisches von Hexan und Essigester als Eluierungsmittel chromatographiert. Es wurden 39 mg (73% d.Th.) (3RS, 4RS)-4-[4-(2,2-Dimethyl-propionyloxymethyl)-phenyl]-3-(naphthalin-2-ylmethoxy)-piperidin-1-carbonsäure tert-butylester als farbloses Öl erhalten; MS : 549 $(M+NH_4)^+$.

(1) Eine Lösung von 35 mg (0.07 mMol) (3RS,4RS)-4-[4-(2,2-Dimethylpropionyloxymethyl)-phenyl]-3-(naphthalin-2-ylmethoxy)-piperidin-1-carbonsäure tert-butylester in 2 ml 2 M Chlorwasserstoff in Methanol wurde 4.5 Stunden lang bei Raumtemperatur gerührt. Anschließend wurde die Reaktionslösung unter vermindertem Druck eingedampft. Der Rückstand wurde in Diäthyläther aufgenommen,wobei ein Teil (15 mg, 49% d.Th.) des 2,2-Dimethylpropionsäure (3RS,4RS)-4-(3-Naphthalin-2-ylmethoxy-piperidin-4-yl)-benzylester Hydrochlorid in Form weißer Kristalle erhalten wurde; MS : 432 $(M+H)^+$.

Beispiel 23

Eine Lösung von 1.10 g (1.59 mMol) (3RS,4RS)-3-Naphthalin-2-ylmethoxy-4-(4-trityloxymethyl-phenyl)-piperidin-1-carbonsäure tert-butylester in 50 ml Methanol wurde bei Raumtemperatur mit 30 ml einer 2 M Lösung von Chlorwasserstoff in Methanol versetzt und 4 Stunden lang bei Raumtemperatur gerührt. Anschließend wurde die Lösung unter vermindertem Druck eingedampft und der Rückstand zwischen 30 ml gesättigter Natriumcarbonat-Lösung und 50 ml Essigester verteilt. Die Wasserphase wurde nochmals mit 50 ml Essigester ausgeschüttelt, danach die organischen Phasen vereinigt, über Natriumsulfat getrocknet und unter vermindertem Druck eingedampft. Zur Reinigung des Rohproduktes wurde an Kieselgel unter Verwendung eines 90:10-Gemisches von Methylenchlorid und Methanol als Eluierungsmittel chromatographiert. Es wurden 462 mg (83% d.Th.) (3RS,4RS)-[4-[3-(Naphthalin-2-ylmethoxy)-piperidin-4-yl]-phenyl}-methanol als farbloser Festkörper erhalten; MS : 348 $(M+H)^+$.

Beispiel 24

In analoger Weise wie in Beispiel 22 (l) wurden durch Abspaltung der BOC-Gruppe mittels Säure die folgenden Verbindungen erhalten:

1) - Aus (3RS,4RS)-4-(4-Benzoyloxymethyl-phenyl)-3-(naphthalin-2-ylmethoxy)-piperidin-1-carbonsäure tert-butylester den Benzoesäure (3RS,4RS)-4-[3-(Naphthalin-2-ylmethoxy)-piperidin-4-yl]-benzylester als farbloser Schaum; MS : 452 $(M+H)^+$;

2) - aus (3RS,4RS)-4-[4-(3-Methoxy-benzoyloxymethyl)-phenyl]-3-naphthalin-2-ylmethoxy-piperidin-1-carbonsäure tert-butylester das 3-Methoxy-benzoesäure (3RS,4RS)-4-(3-Naphthalin-2-ylmethoxypiperidin-4-yl)-benzylester Hydrochlorid als farbloser Festkörper; MS : 482 $(M+H)^+$;

3) - aus (3RS,4RS)-4-[4-(3,5-Dimethoxy-benzoyloxymethyl)-phenyl]-3-(naphthalin-2-ylmethoxy)-piperidin-1-carbonsäure tert-butylester das 3,5-Dimethoxy-benzoesäure (3RS,4SR)-4-(3-Naphthalin-2-ylmethoxypiperidin-4-yl)-benzylester Hydrochlorid als farbloser Festkörper; MS : 512 $(M+H)^+$;

4) - aus (3RS,4RS)-4-(4-Cyclohexancarbonyloxymethyl-phenyl)-3-(naphthalin-2-ylmethoxy)-piperidin-1-carbonsäure tert-butylester unter Verwendung von Trifluoressigsäure in Methylenchlorid das Cyclohexancarbonsäure (3RS,4RS)-4-[3-(Naphthalin-2-ylmethoxy)-piperidin-4-yl]-benzylester Trifluoracetat als farbloser Festkörper; MS : 458 $(M+H)^+$;

5) - aus (3RS,4RS)-[4-(2-Chlor-benzoyloxymethyl)-phenyl]-3-naphthalin-2-ylmethoxy-piperidin-1-carbonsäure tert-butylester unter Verwendung von Trifluoressigsäure in Methylenchlorid das 2-Chlor-benzoesäure (4RS,3RS)-4-(3-Naphthalin-2-ylmethoxy-piperidin-4-yl)-benzylester Trifluoracetat als farbloser Festkörper; MS : 486 $(M+H)^+$;

6) - aus (3RS,4RS)-4-(4-Methoxycarbonyloxymethyl-phenyl)-3-(naphthalin-2-ylmethoxy)-piperidin-1-carbonsäure tert-butylester das Kohlensäuremethylester (3RS,4RS)-4-(Naphthalin-2-ylmethoxypiperidin-4-yl)-benzylester Hydrochlorid als farbloses Öl; MS : 406 $(M+H)^+$;

7) - aus Pyridin-4-carbonsäure (3RS,4RS)-4-(1-tert-Butoxycarbonyl-3-naphthalin-2-ylmethoxy-piperidin-4-yl)-benzylester das Pyridin-4-carbonsäure (3RS,4RS)-4-[3-(Naphthalin-2-ylmethoxy)-piperidin-4-yl]-benzyl ester Hydrochlorid als gelbliches Öl; MS : 453 $(M+H)^+$;

8) - aus Pyrazin-2-carbonsäure (3RS,4RS)-4-[1-tert-Butoxycarbonyl-3-(naphthalin-2-ylmethoxy)-piperidin-4-yl]-benzylester das Pyrazin-2-carbonsäure (3RS,4RS)-4-(3-Naphthalin-2-ylmethoxy-piperidin-4-yl)-benzylester Hydrochlorid als farbloser Festkörper; MS : 454 $(M+H)^+$;

9) - aus (3RS,4RS)-4-[4-(2-Chlor-benzoyloxymethyl)-phenyl]-3-(1-methoxy-naphthalin-2-ylmethoxy)-piperidin-1-carbonsäure tert-butylester unter Verwendung von Trifluoressigsäure in Methylenchlorid das 2-Chlor-benzoesäure (3RS,4RS)-4-[3-(1-Methoxynaphthalin-2-ylmethoxy)-piperidin-4-yl]-benzylester Trifluoracetat als farbloser Festkörper; MS : 516 $(M+H)^+$;

10) - aus (3RS,4RS)-4-[4-(4-Hydroxy-benzoyloxymethyl)-phenyl]-3-(naphthalin-2-ylmethoxy)-piperidin-1-carbonsäure tert-butylester unter Verwendung von Trifluoressigsäure in Methylenchlorid das 4-Hydroxy-benzoesäure (3RS,4RS)-4-(3-Naphthalin-2-ylmethoxy-piperidin-4-yl)-benzylester Hydrochlorid als gelblicher Festkörper; MS :

468 $(M+H)^+$;

11) - aus (3RS,4RS)-3-(Naphthalin-2-ylmethoxy)-4-[4-(pyridin-2-ylcarbamoyloxymethyl)-phenyl]-piperidin-1-carbonsäure tert-butylester das Pyridin-2-yl-carbaminsäure (3RS,4RS)-4-[3-(Naphthalin-2-ylmethoxy)-piperidin-4-yl]-benzylester Hydrochlorid als farbloser Schaum; $R_f$: 0.15 ($SiO_2$, Methylenchlorid : Methanol Ammoniak = 90 : 10 : 0.1);

12) - aus (3RS,4RS)-4-[4-(3-Hydroxy-propenyl)-phenyl]-3-(naphthalin-2-ylmethoxy)-piperidin-1-carbonsäure tert-butylester das (3RS,4RS)-4-[4-(3-Methoxy-propenyl)-phenyl]-3-(naphthalin-2-ylmethoxy)-piperidin als gelbes Öl; MS : 388 $(M+H)^+$;

13) - aus (3RS,4RS)-4-[4-(3-Benzoyloxy-propenyl)-phenyl]-3-(naphthalin-2-ylmethoxy)-piperidin-1-carbonsäure tert-butylester das (3RS,4RS)-4-[4-(3-Benzoyloxy-propenyl)-phenyl]-3-(naphthalin-2-ylmethoxy)-piperidin als gelbes Öl; MS : 478 $(M+H)^+$;

14) - aus (3RS,4RS)-4-[4-[3-(2-Chlor-benzoyloxy)-propyl]-phenyl]-3-(naphthalin-2-ylmethoxy)-piperidin-1-carbonsäure tert-butylester der (3RS,4RS)-2-Chlor-benzoesäure 3-[4-[3-(Naphthalin-2-ylmethoxy)-piperidin-4-yl]-phenyl]-propylester als gelbliches Öl; MS : 514, 516 $(M+H)^+$;

Die Ausgangssubstanzen wurden analog zu dem in Beispiel 22 (k) beschriebenen Verfahren wie folgt erhalten:

(a) Aus (3RS,4RS)-4-(4-Hydroxymethyl-phenyl)-3-(naphthalin-2-ylmethoxy)-piperidin-1-carbonsäure tert-butylester und Benzoylchlorid der (3RS,4RS)-4-(4-Benzoyloxymethyl-phenyl)-3-(naphthalin-2-ylmethoxy)-piperidin-1-carbonsäure tert-butylester als farbloses Öl; MS : 552 $(M+H)^+$.

(b) Aus (3RS,4RS)-4-(4-Hydroxymethyl-phenyl)-3-(naphthalin-2-ylmethoxy)-piperidin-1-carbonsäure tert-butylester und 3-Methoxybenzoylchlorid der (3RS,4RS)-4-[4-(3-Methoxy-benzoyloxymethyl)-phenyl]-3-naphthalin-2-ylmethoxy-piperidin-1-carbonsäure tert-butylester als farbloses Öl; MS: 582 $(M+H)^+$.

(c) Aus (3RS,4RS)-4-(4-Hydroxymethyl-phenyl)-3-(naphthalin-2-ylmethoxy)-piperidin-1-carbonsäure tert-butylester und 3,5-Dimethoxybenzoylchlorid der (3RS,4RS)-4-[4-(3,5-Dimethoxybenzoyloxymethyl)-phenyl]-3-(naphthalin-2-ylmethoxy)-piperidin-1-carbonsäure tert-butylester als farbloses Öl; MS: 612 $(M+H)^+$.

(d) Aus (3RS,4RS)-4-(4-Hydroxymethyl-phenyl)-3-(naphthalin-2-ylmethoxy)-piperidin-1-carbonsäure tert-butylester und Cyclohexancarbonsäurechlorid der (3RS,4RS)-4-(4-Cyclohexancarbonyloxymethyl-phenyl)-3-(naphthalin-2-ylmethoxy)-piperidin-1-carbonsäure tert-butylester als farbloses Öl; MS : 558 $(M+H)^+$.

(e) Aus (3RS,4RS)-4-(4-Hydroxymethyl-phenyl)-3-(naphthalin-2-ylmethoxy)-piperidin-1-carbonsäure tert-butylester und 2-Chlorbenzoylchlorid der (3RS,4RS)-[4-(2-Chlor-benzoyloxymethyl)-phenyl]-3-naphthalin-2-ylmethoxy-piperidin-1-carbonsäure tert-butylester als farbloses Öl; MS : 586 $(M+H)^+$.

(f) Aus (3RS,4RS)-4-(4-Hydroxymethyl-phenyl)-3-(naphthalin-2-ylmethoxy)-piperidin-1-carbonsäure tert-butylester und Chlorameisensäuremethylester der (3RS,4RS)-4-(4-Methoxycarbonyloxymethyl-phenyl)-3-(naphthalin-2-ylmethoxy)-piperidin-1-carbonsäure tert-butylester als farbloses Öl.

(g) Eine Lösung von 60 mg (0.13 mMol) (3RS,4RS)-4-(4-Hydroxymethylphenyl)-3-(naphthalin-2-ylmethoxy)-piperidin-1-carbonsäure tert-butylester, 16 mg (0.13 mMol) Isonicotinsäure, 34 mg (0.26 mMol) Äthyldiisopropylamin und 58 mg (0.13 mMol) Benzotriazol-1-yl-oxy-tris-(dimethylamino)-phosphonium Hexafluorphosphat (BOP) in 5 ml Acetonitril wurde 2 Stunden lang bei Raumtemperatur gerührt. Anschließend wurde das Reaktionsgemisch unter vermindertem Druck eingedampft und der Rückstand ohne weitere Aufarbeitung an Kieselgel unter Verwendung eines 3:1-Gemisches von Hexan und Essigester als Eluierungsmittel chromatographiert. Es wurden 15 mg (21% d.Th.) Pyridin-4-carbonsäure (3RS,4RS)-4-(1-tert-Butoxycarbonyl-3-naphthalin-2-ylmethoxy-piperidin-4-yl)-benzylester als farbloser Festkörper erhalten; MS : 552 $(M)^+$.

(h) In analoger Weise wie unter (g) beschrieben, wurde durch Kondensation von (3RS,4RS)-4-(4-Hydroxymethyl-phenyl)-3-(naphthalin-2-ylmethoxy)-piperidin-1-carbonsäure tert-butylester und Pyrazincarbonsäure unter Verwendung von 1,1-Carbonyldiimidazol als Kondensationsmittel der (3RS,4RS)-Pyrazin-2-carbonsäure 4-[1-tert-Butoxycarbonyl-3-(naphthalin-2-ylmethoxy)-piperidin-4-yl]-benzylester erhalten; MS : 554 $(M+H)^+$.

In analoger Weise wie in Beispiel 22 (i)-(k) beschrieben, wurde wie folgt verfahren:

(i) Die Alkylierung von (3RS,4RS)-4-(1-Benzyl-3-hydroxy-piperidin-4-yl)-benzoesäuremethylester mit 2-Brommethyl-1-methoxy-naphthalin ergab den (3RS,4RS)-3-(1-Methoxy-naphthalin-2-ylmethoxy)-4-(4-trityloxymethyl-phenyl)-piperidin-1-carbonsäure tert-butylester als farbloses Öl; MS : 720 $(M+H)^+$.

(j) Die Abspaltung der Tritylgruppe aus dem (3RS,4RS)-3-(1-Methoxynaphthalin-2-ylmethoxy)-4-(4-trityloxymethyl-phenyl)-piperidin-1-carbonsäure tert-butylester lieferte den (3RS,4RS)-4-(4-Hydroxymethylphenyl)-3-(1-methoxy-naphthalin-2-ylmethoxy)-piperidin-1-carbonsäure tert-butylester als farbloses Öl; MS : 478 $(M+H)^+$.

(k) Die Acylierung des (3RS,4RS)-4-(4-Hydroxymethyl-phenyl)-3-(1-methoxy-naphthalin-2-ylmethoxy)-piperidin-1-carbonsäure tert-butylesters mit 2-Chlorbenzoylchlorid ergab den (3RS,4RS)-4-[4-(2-Chlorbenzoyloxymethyl)-phenyl]-3-(1-methoxy-naphthalin-2-ylmethoxy)-piperidin-1-carbonsäure tert-butylester als farbloses Öl; MS : 615 $(M+H)^+$.

(l) In analoger Weise wie unter (g) beschrieben, wurde durch Kondensation von (3RS,4RS)-4-(4-Hydroxymethyl-phenyl)-3-(naphthalin-2-ylmethoxy)-piperidin-1-carbonsäure tert-butylester und 4-(2-Trimethylsilanyl-äthoxymethoxy)-benzoesäure unter Verwendung von N-Äthyl-N'-(3-dimethylaminopropyl)-carbodiimid Hydrochlorid (EDC) als Kondensationsmittel der (3RS,4RS)-3-(Naphthalin-2-ylmethoxy)-4-{4-[4-(2-trimethylsilanyl-äthoxymethoxy)-benzoyloxymethyl]-phenyl}piperidin-1-carbonsäure tert-butylester erhalten; MS : 715 $(M+NH_4)^+$.

Die 4-(2-Trimethylsilanyl-äthoxymethoxy)-benzoesäure wurde, in analoger Weise wie in Beispiel 5 (a) beschrieben, durch Umsetzung von 4-Hydroxybenzoesäuremethylester mit 2-(Trimethylsilyl)-äthoxymethylchlorid und anschließende basische Verseifung des Esters als farbloser Festkörper erhalten.

(m) Eine Lösung von 57 mg (0.13 mMol) (3RS,4RS)-4-(4-Hydroxymethylphenyl)-3-(naphthalin-2-ylmethoxy)-piperidin-1-carbonsäure tert-butylester, 20 mg (0.14 mMol) Pyridin-2-carbonylazid [H.Saikachi und T.Kitgawa, Chem. Pharm.Bull. 25 (7), 1651-1657 (1977)] und 3 mg 4-Dimethylaminopyridin in 3 ml Toluol wurde 3 Stunden lang auf 90° C erwärmt. Anschließend wurde das Toluol unter vermindertem Druck eingedampft, der Rückstand in 15 ml Methylenchlorid aufgenommen und die Lösung mit 5 ml Wasser gerwaschen. Die organische Phase wurde danach über Natriumsulfat getrocknet und unter vermindertem Druck eingedampft. Der ölige Rückstand wurde mit einem Gemisch von Äther und Hexan aufgenommen und zur Kristallisation gebracht. Es wurden 64 mg (88% d.Th.) (3RS, 4RS)-3-(Naphthalin-2-ylmethoxy)-4-[4-(pyridin-2-ylcarbamoyloxymethyl)-phenyl]-piperidin-1-carbonsäure tert-butylester als farbloser Festkörper erhalten; MS : 568 $(M+H)^+$.

(n) Ein Schlenkrohr wurde unter Argon mit 25.6 mg (0.114 mMol) $Pd(OAc)_2$, 69.6 mg (0.229 mMol) Tri(o-tolyl) phosphin und 20 ml DMF (unter Argon destilliert beladen und das Reaktionsgemisch 15 Minuten lang bei Raumtemperatur gerührt. Ein 200 ml-Sulfierkolben wurde unter Argon und unter Rühren mit 8.15 g (22.9 mMol) (3RS, 4RS)-4-(4-Brom-phenyl)-3-hydroxy-piperidin-1-carbonsäure tert-butylester [Beispiel 25 (c)], 100 ml DMF, 3.73 ml (34.3 mMol) Acrylsäureäthylester, 2.25 g (27.5 mMol) Natriumacetat und der gelben Katalysatorlösung beladen. Das Reaktionsgemisch wurde bei 120 °C während 6 Stunden gerührt. Zur Vervollständigung der Reaktion wurde nach 5 Stunden eine Lösung von 5.1 mg $Pd(OAc)_2$ und 14.3 mg Tri(o-tolyl)phosphin in 5 ml DMF zugegeben. Das dunkle Reaktionsgemisch wurde am Rotationsverdampfer eingedampft. Der graue feste Rückstand wurde in Äther aufgenommen und die trübe Lösung wurde dreimal mit Wasser gewaschen, über Natriumsulfat getrocknet und unter vermindertem Druck eingedampft. Der gelbe feste Rückstand wurde an 250 g Kieselgel unter Verwendung eines 2:1-Gemisches von Hexan und Essigester als Eluierungsmittel chromatographiert. Nach der Kristallisation aus Essigester wurden 6.66 g (77 % d.Th.) (3RS,4RS)-4-[4-(2-Äthoxycarbonyl-vinyl)-phenyl]-3-hydroxy-piperidin-1-carbonsäure tert-butylester als farblose Kristalle erhalten; MS : 376 $(M+H)^+$.

(o) Die Alkylierung des (3RS,4RS)-4-[4-(2-Äthoxycarbonyl-vinyl)-phenyl]-3-hydroxy-piperidin-1-carbonsäure tert-butylesters mit 2-Brommethyl-naphthalin, analog dem im Beispiel 22 (i) beschriebenen Verfahren, ergab den (3RS, 4RS)-4-[4-(2-Äthoxycarbonyl-vinyl)-phenyl]-3-(naphthalin-2-ylmethoxy)-piperidin-1-carbonsäure tert-butylester als leicht gelbes Harz; MS : 516 $(M+H)^+$.

(p) Zu einer Lösung von 698 mg (1.35 mMol) (3RS,4RS)-4-[4-(2-Äthoxycarbonyl-vinyl)-phenyl]-3-(naphthalin-2-ylmethoxy)-piperidin-1-carbonsäure tert-butylester in 15 ml Toluol wurden tropfenweise bei -50 °C 5 ml (5 mMol) einer Lösung von Diisobutylaluminiumhydrid (DIBAH) (1M in Hexan) gegeben und das Gemisch 30 Minuten bei -50 °C gerührt. Anschließend wurde das Reaktionsgemisch tropfenweise bei -50 °C mit 10 ml Äthanol versetzt

und auf Raumtemperatur erwärmt. Zur Aufarbeitung wurde das Reaktionsgemisch unter Eiskühlung mit 20 ml Wasser und 20 ml gesättigter Kaliumnatriumtartrat-Lösung versetzt und danach dreimal mit je 50 ml Essigester ausgeschüttelt. Die organischen Phasen wurden vereinigt, über Natriumsulfat getrocknet und unter vermindertem Druck eingedampft. Das Rohprodukt wurde an Kieselgel unter Verwendung eines 6:4-Gemisches von Hexan und Essigester als Eluierungsmittel chromatographiert. Es wurden 354 mg (55% d.Th.) (3RS,4RS)-4-[4-(3-Hydroxy-propenyl)-phenyl]-3-(naphthalin-2-ylmethoxy)-piperidin-1-carbonsäure tert-butylester als farbloser Festkörper erhalten; MS : 474 $(M+H)^+$.

(q) Die Acylierung des (3RS,4RS)-4-[4-(3-Hydroxy-propenyl)-phenyl]-3-(naphthalin-2-ylmethoxy)-piperidin-1-carbonsäure tert-butylesters mit Benzoylchlorid, analog dem im Beispiel 22 (k) beschriebenen Verfahren, lieferte den (3RS,4RS)-4-[4-(3-Benzoyloxy-propenyl)-phenyl]-3-(naphthalin-2-ylmethoxy)-piperidin-1-carbonsäure tert-butylester als farbloses Harz; MS : 578 $(M+H)^+$.

(r) Eine Lösung von 2.0 g (5.35 mMol) (3RS,4RS)-4-[4-(2-Äthoxycarbonylvinyl)-phenyl]-3-hydroxy-piperidin-1-carbonsäure tert-butylester in 100 ml Äthanol wurde mit 200 mg Palladium/Kohle (Typ E101R) versetzt und 1 Stunde lang bei Raumtemperatur hydriert. Anschließend wurde der Katalysator abfiltriert und mit Äthanol nachgewaschen. Das Filtrat wurde unter vermindertem Druck eingedampft und der leicht graue Rückstand (1.97 g) wurde mit denen aus drei analogen Hydrieransätzen vereinigt (total 3.08 g). Zur Reinigung wurde das Rohprodukt an Kieselgel unter Verwendung eines 2:1-Gemisches von Hexan und Essigester als Eluierungsmittel chromatographiert und danach aus Essigester/Hexan kristallisiert. Man erhielt 2.67 g (87 % d.Th.) (3RS,4RS)-4-[4-(2-Äthoxycarbonyl-äthyl)-phenyl]-3-hydroxypiperidin-1-carbonsäure tert-butylester als farblose Kristalle; MS : 378 $(M+H)^+$.

(s) Analog zu dem im Beispiel 22 (i) beschriebenen Verfahren wurde durch Alkylierung des (3RS,4RS)-4-[4-(2-Äthoxycarbonyl-äthyl)-phenyl]-3-hydroxy-piperidin-1-carbonsäure tert-butylesters mit 2-Brommethylnaphthalin der (3RS,4RS)-4-[4-(2-Äthoxycarbonyl-äthyl)-phenyl)-3-(naphthalin-2-ylmethoxy)-piperidin-1-carbonsäure tert-butylester erhalten; MS : 518 $(M+H)^+$.

(t) Analog zu dem im Beispiel 22 (e) beschriebenen Verfahren wurde durch Reduktion des (3RS,4RS)-4-[4-(2-Äthoxycarbonyl-äthyl)-phenyl]-3-(naphthalin-2-ylmethoxy)-piperidin-1-carbonsäure tert-butylesters mittels Lithiumborhydrid der (3RS,4RS)-4-[4-(3-Hydroxy-propyl)-phenyl]-3-(naphthalin-2-ylmethoxy)-piperidin-1-carbonsäure tert-butylester als farbloser Festkörper erhalten; MS : 476 $(M+H)^+$.

(u) Die Acylierung des (3RS,4RS)-4-[4-(3-Hydroxy-propyl)-phenyl]-3-(naphthalin-2-ylmethoxy)-piperidin-1-carbonsäure tert-butylesters mit 2-Chlor-benzoylchlorid, analog dem im Beispiel 22 (k) beschriebenen Verfahren, ergab den (3RS,4RS)-4-[4-[3-(2-Chlor-benzoyloxy)-propyl]-phenyl]-3-(naphthalin-2-ylmethoxy)-piperidin-1-carbonsäure tert-butylester als farblosen, amorphen Festkörper; MS : 614.6, 616 $(M+H)^+$.

Beispiel 25

(a) Das Gemisch von 1.0 g (2.9 mMol) (3RS,4RS)-1-Benzyl-4-(4-bromphenyl)-piperidin-3-ol [Beispiel 22 (c)], 1.36 ml (10.2 mMol) Chlorameisensäure-2,2,2-trichloräthylester und 0.90 g (12.6 mMol) Lithiumcarbonat in 20 ml Toluol wurde während 18 Stunden auf 105° C erhitzt. Zur Aufarbeitung wurde das abgekühlte Reaktionsgemisch auf 20 ml Eiswasser gegossen und anschließend dreimal mit je 25 ml Essigester extrahiert. Die vereinigten organischen Phasen wurden über Natriumsulfat getrocknet und unter vermindertem Druck eingedampft. Das erhaltene Rohmaterial wurde zur Reinigung an Kieselgel unter Verwendung eines 2:3-Gemisches von Methylenchlorid und Hexan als Eluierungsmittel chromatographiert. Es wurden 1.3 g (76% d.Th.) (3RS,4RS)-4-(4-Brom-phenyl)-3-(2,2,2-trichloräthoxycarbonyloxy)-piperidin-1-carbonsäure 2,2,2-trichloräthylester als farbloser Festkörper erhalten; $R_f$: 0.17 ($SiO_2$, Methylenchlorid : Hexan = 1 : 1), MS: 622, 624, 626 $(M+NH_4)^+$.

(b) Das Gemisch von 1.3 g (2.1 mMol) (3RS,4RS)-4-(4-Brom-phenyl)-3-(2,2,2-trichlor-äthoxycarbonyloxy)-piperidin-1-carbonsäure 2,2,2-trichloräthylester und 1.54 g aktiviertem Zink in 20 ml Eisessig wurde 5 Stunden lang bei Raumtemperatur gerührt. Zur Aufarbeitung wurde das Zink abfiltriert, der Rückstand mit Eisessig nachgewaschen und die Lösung anschließend unter vermindertem Druck zur Trockene eingedampft. Der Rückstand wurde zwischen 20 ml gesättigter Natriumcarbonat-Lösung und 30 ml Essigester verteilt, danach die abgetrennte wäßrige Phase zweimal mit je 30 ml Essigester ausgeschüttelt. Die vereinigten organischen Phasen wurden über Natriumsulfat getrocknet und unter vermindertem Druck eingedampft. Die Kristallisation des Rückstandes unter Verwendung eines 1:2-Gemisches von Essigester und Hexan lieferte 250 mg (46% d.Th.) (3RS,4RS)-4-(4-Brom-phenyl)-piperidin-3-ol als farblosen Festkörper; MS : 255, 257 $(M)^+$. Die Mutterlauge wurde zur Reinigung an Kieselgel unter Verwendung eines 4:1-Gemisches von Methylenchlorid und Methanol als Eluierungsmittel chromatographiert. Es wurden weitere 101 mg (18% d.Th.) erhalten.

(c) Eine Lösung von 351 mg (1.37 mMol) (3RS,4RS)-4-(4-Brom-phenyl)-piperidin-3-ol in 12 ml Dimethylformamid wurde mit 139 mg (1.37 mMol) Triäthylamin und 300 mg (1.37 mg) Di-tert-butyldicarbonat versetzt und während 15 Stunden bei Raumtemperatur gerührt. Anschließend wurde das Dimethylformamid im Ölpumpenvakuum abdestilliert und der Rückstand aus einem 1:1-Gemisch von Äther und Hexan kristallisiert. Es wurden 318 mg (65% d.Th.) (3RS,4RS)-4-(4-Brom-phenyl)-3-hydroxy-piperidin-1-carbonsäure tert-butylester als farbloser Festkörper erhalten; MS : 299, 301 $(M-C_4H_8)^+$. Die Chromatographie der Mutterlauge an Kieselgel unter Verwendung eines 98:2-Gemisches von Methylenchlorid und Methanol als Eluierungsmittel lieferte weitere 96 mg (19% d.Th.) des Produkts.

(d) In analoger Weise wie in Beispiel 22 (d) beschrieben, wurde durch Carbonylierung von (3RS,4RS)-4-(4-Brom-phenyl)-3-hydroxy-piperidin-1-carbonsäure tert-butylester unter Verwendung von $PdCl_2(CH_3CN)_2$ und 1,3-Bis (diphenylphosphino)-propan als Katalysator in Gegenwart von Triäthylamin unter 10 bar Kohlenmonoxid bei 100° C während 40 Stunden der (3RS,4RS)-3-Hydroxy-4-(4-methoxycarbonyl-phenyl)-piperidin-1-carbonsäure tert-butylester als weiße Kristalle erhalten; Smp.: 145.5-146° C, MS : 279 $(M-C_4H_8)^+$.

(e) In analoger Weise wie in Beispiel 22 (i) beschrieben, wurde durch Alkylierung von (3RS,4RS)-3-Hydroxy-4-(4-methoxycarbonyl-phenyl)-piperidin-1-carbonsäure tert-butylester mit 2-Brommethylnaphthalin das (3RS, 4RS)-4-(4-Methoxycarbonyl-phenyl)-3-naphthalin-2-ylmethoxypiperidin-1-carbonsäure tert-butylester als farbloses Öl erhalten; MS : 476 $(M+H)^+$.

(f) In analoger Weise wie in Beispiel 22 (l) beschrieben, wurde nach Abspaltung der BOC-Gruppe aus (3RS,4RS)-4-(4-Methoxycarbonylphenyl)-3-naphthalin-2-ylmethoxy-piperidin-1-carbonsäure tert-butylester das (3RS,4RS)-4-(3-Naphthalin-2-ylmethoxy-piperidin-4-yl)-benzoesäure methylester Hydrochlorid als farbloser Festkörper erhalten; MS : 344 $(M-OCH_3)^+$.

Beispiel 26

In analoger Weise wie in Beispiel 22 (l) beschrieben, wurden durch Abspaltung der BOC-Gruppe die folgenden Verbindungen erhalten:

1) - Aus (3RS,4RS)-3-(4-Benzyloxy-naphthalin-2-ylmethoxy)-4-(3-hydroxymethyl-phenyl)-piperidin-1-carbonsäure tert-butylester das (3RS,4RS)-[3-[3-(4-Benzyloxy-naphthalin-2-ylmethoxy)-piperidin-4-yl]-phenyl)-methanol als farbloses amorphes Pulver; MS : 454 $(M+H)^+$;

2) - aus (3RS,4RS)-4-(3-Benzoyloxymethyl-phenyl)-3-naphthalin-2-ylmethoxy-piperidin-1-carbonsäure tert-butylester das Benzoesäure (3RS,4RS)-3-(3-Naphthalin-2-ylmethoxy-piperidin-4-yl)-benzylester Hydrochlorid als farbloser Festkörper; MS : 452 $(M+H)^+$.

Die als Ausgangsstoffe eingesetzten BOC-Derivate wurden wie folgt erhalten:

(a) In analoger Weise wie in Beispiel 22 (a)-(d) beschrieben, wurde ausgehend von 3-Bromphenyllithium und 1-Benzyl-4-piperidon der (3RS,4RS)-3-( 1-Benzyl-3-hydroxy-piperidin-4-yl)-benzoesäure methylester als leicht gelbes Harz erhalten; MS : 325 $(M)^+$.

(b) In analoger Weise wie in Beispiel 2 (e) beschrieben, wurde durch katalytische Hydrierung des (3RS,4RS)-3-( 1-Benzyl-3-hydroxy-piperidin-4-yl)-benzoesäure methylesters die Benzyl-Gruppe abgespalten. Der erhaltene rohe (3RS,4RS)-3-(3-Hydroxy-piperidin-4-yl)-benzoesäure methylester wurde ohne weitere Reinigung und Charakterisierung, analog zu Beispiel 22 (g), mit Di-tert-butyldicarbonat zum (3RS,4RS)-3-Hydroxy-4-(3-methoxycarbonyl-phenyl)-piperidin-1-carbonsäure tert-butylester, MS : 304 $(M-OCH_3)^+$, umgesetzt.

(c) In analoger Weise wie in Beispiel 22 (i) beschrieben, wurde durch Alkylierung des (3RS,4RS)-3-Hydroxy-4-(3-methoxycarbonyl-phenyl)-piperidin-1-carbonsäure tert-butylesters mit 1-Benzyloxy-3-chlormethylnaphthalin der (3RS,4RS)-3-(4-Benzyloxy-naphthalin-2-ylmethoxy)-4-(3-methoxycarbonyl-phenyl)-piperidin-1-carbonsäure

tert-butylester als hellgelbes amorphes Pulver erhalten; MS : 582 $(M+H)^+$.

(d) In analoger Weise wie in Beispiel 22 (e) beschrieben, wurde durch Reduktion von (3RS,4RS)-3-(4-Benzyloxy-naphthalin-2-ylmethoxy)-4-(3-methoxycarbonyl-phenyl)-piperidin-1-carbonsäure tert-butylester mit Lithiumborhydrid der (3RS,4RS)-3-(4-Benzyloxy-naphthalin-2-ylmethoxy)-4-(3-hydroxymethyl-phenyl)-piperidin-1-carbonsäure tert-butylester als farbloser Festkörper erhalten; MS : 554 $(M+H)^+$.

(e) In analoger Weise wie in Beispiel 22 (i) beschrieben, wurde durch Alkylierung des (3RS,4RS)-3-Hydroxy-4-(3-methoxycarbonyl-phenyl)-piperidin-1-carbonsäure tert-butylesters mit 2-Brommethylnaphthalin der (3RS, 4RS)-4-(3-Methoxycarbonyl-phenyl)-3-naphthalin-2-ylmethoxypiperidin-1-carbonsäure tert-butylester als farbloses Öl erhalten; MS : 493 $(M+NH_4)^+$

(f) In analoger Weise wie in Beispiel 22 (e) beschrieben, wurde durch Reduktion von (3RS,4RS)-4-(3-Methoxycarbonyl-phenyl)-3-naphthalin-2-ylmethoxy-piperidin-1-carbonsäure tert-butylester mit Lithiumborhydrid der 4-(3-Hydroxymethyl-phenyl)-3-(naphthalin-2-ylmethoxy)-piperidin-1-carbonsäure tert-butylester als farbloses Öl erhalten; MS : 448 $(M+H)^+$.

(g) In analoger Weise wie in Beispiel 22 (k) beschrieben, wurde durch Acylierung des (3RS,4RS)-4-(3-Hydroxymethyl-phenyl)-3-(naphthalin-2-ylmethoxy)-piperidin-1-carbonsäure tert-butylester mit Benzoylchlorid der (3RS, 4RS)-4-(3-Benzoyloxymethyl-phenyl)-3-naphthalin-2-ylmethoxy-piperidin-1-carbonsäure tert-butylester als farbloses Öl erhalten; MS : 569 $(M+NH_4)^+$.

Beispiel 27

In analoger Weise wie in Beispiel 22 (l) beschrieben, wurden durch Abspaltung der BOC-Gruppe die folgenden Verbindungen erhalten:

1) - Aus (3RS,4RS)-3-(4-Benzyloxy-naphthalin-2-ylmethoxy)-4-(3-methoxycarbonyl-phenyl)-piperidin-1-carbonsäure tert-butylester der (3RS,4RS)-3-[3-(4-Benzyloxy-naphthalin-2-ylmethoxy)-piperidin-4-yl]-benzoesäure methylester als farbloses amorphes Pulver; MS : 482 $(M+H)^+$;
2) - aus (3RS,4RS)-4-(3-Methoxycarbonyl-phenyl)-3-naphthalin-2-ylmethoxy-piperidin-1-carbonsäure tert-butylester der (3RS,4RS)-3-[3-(Naphthalin-2-ylmethoxy)-piperidin-4-yl]-benzoesäure methylester als farbloses Öl; MS : 376 $(M+H)^+$.

Beispiel 28

Aus 57 mg rohem (3RS,4RS)-3-(4-Benzyloxy-naphthalin-2-ylmethoxy)-4-(3-fluormethyl-phenyl)-piperidin-1-carbonsäure tert-butylester wurde, in analoger Weise wie in den Beispiel 22 beschrieben, durch Abspaltung der BOC-Gruppe das (3RS,4RS)-3-(4-Benzyloxynaphthalin-2-ylmethoxy)-4-(3-fluormethyl-phenyl)-piperidin als leicht gelbes Harz erhalten; MS: 456 $(M+H)^+$.
Der als Ausgangsstoff eingesetzte (3RS,4RS)-3-(4-Benzyloxynaphthalin-2-ylmethoxy)-4-(3-fluormethyl-phenyl)-piperidin-1-carbonsäure tert-butylester wurde wie folgt hergestellt:
Eine Lösung von 18 mg (0.106 mMol) Diäthylaminoschwefeltrifluorid (DAST) in 1 ml Methylenchlorid wurde auf -65 °C abgekühlt und dazu eine Lösung von 56 mg (0.101 mMol) (3RS,4RS)-3-(4-Benzyloxy-naphthalin-2-ylmethoxy)-4-(3-hydroxymethyl-phenyl)-piperidin-1-carbonsäure tert-butylester in 1 ml Methylenchlorid bei - 60°C bis -65°C innerhalb von 3 Minuten getropft. Die entstandene gelbe Lösung wurde auf Raumtemperatur erwärmt und eine Stunde nachgerührt. Anschließend wurde das Gemisch zwischen Methylenchlorid und 5%iger Natriumhydrogencarbonat-Lösung verteilt, die organische Phase über Magnesiumsulfat getrocknet und schließlich das Lösungsmittel unter vermindertem Druck abdestilliert. Man erhielt 57 mg rohen (3RS,4RS)-3-(4-Benzyloxy-naphthalin-2-ylmethoxy)-4-(3-fluormethyl-phenyl)-piperidin-1-carbonsäure tert-butylester, der ohne weitere Reinigung und Charakterisierung in der nächsten Stufe eingesetzt wurde.

Beispiel 29

(a) In analoger Weise wie in Beispiel 22 (a) beschrieben, wurde, ausgehend von 1-Benzyl-4-piperidon und rac.-2-[2-(4-Brom-phenyl)-äthoxy]-tetrahydropyran, das (RS)-1-Benzyl-4-[4-[2-(tetrahydro-pyran-2-yloxy)-äthyl]-phenyl]-piperidin-4-ol als gelbes Öl erhalten; MS : 396 $(M+H)^+$.

(b) Eine Lösung von 78 g (197 mMol) (RS)-1-Benzyl-4-[4-[2-(tetrahydropyran-2-yloxy)-äthyl]-phenyl]-piperidin-4-ol in 400 ml Methanol wurde mit 470 ml 2 N Salzsäure versetzt und 5 Stunden bei Raumtemperatur gerührt. Zur Aufarbeitung wurde die Reaktionslösung in 1500 ml gesättigte Natriumhydrogencarbonat-Lösung gegossen und anschließend dreimal mit je 1000 ml Essigester extrahiert. Die vereinigten organischen Phasen wurden über Natriumsulfat getrocknet und unter vermindertem Druck eingedampft. Zur Reinigung der Rohsubstanz wurde eine Flash-Chromatographie an Kieselgel unter Verwendung eines 95:5-Gemisches von Methylenchlorid und Methanol als Eluierungsmittel durchgeführt. Es wurden 51.6 g (84% d.Th.) 1-Benzyl-4-[4-(2-hydroxy-äthyl)-phenyl]-piperidin-4-ol als gelblicher Festkörper erhalten; MS : 312 $(M+H)^+$.

(c) In analoger Weise wie in Beispiel 22 (b) beschrieben, wurde durch eine Eliminierungsreaktion aus 1-Benzyl-4-[4-(2-hydroxy-äthyl)-phenyl]-piperidin-4-ol mittels p-Toluolsulfonsäure das 2-[4-(1-Benzyl-1,2,3,6-tetrahydro-pyridin-4-yl)-phenyl]-äthanol als farbloses Öl erhalten; MS: 293 $(M)^+$.

(d) In analoger Weise wie in Beispiel 22 (c) beschrieben, wurde aus 2-[4-(1-Benzyl-1,2,3,6-tetrahydro-pyridin-4-yl)-phenyl]-äthanol mittels Hydroborierung das (3RS,4RS)-1-Benzyl-4-[4-(2-hydroxy-äthyl)-phenyl]- piperidin-3-ol als farbloses Öl erhalten; MS : 311 $(M)^+$.

(e) In analoger Weise wie in Beispiel 2 (e) und Beispiel 22 (g) beschrieben, wurde durch katalytische Hydrierung von (3RS,4RS)-1-Benzyl-4-[4-(2-hydroxy-äthyl)-phenyl]-piperidin-3-ol das (3RS,4RS)-4-[4-(2-Hydroxy-äthyl)-phenyl]-piperidin-3-ol erhalten, das ohne weitere Reinigung und Charakterisierung mit Di-tert-butyldicarbonat zum (3RS,4RS)-3-Hydroxy-4-[4-(2-hydroxy-äthyl)-phenyl]-piperidin-1-carbonsäure tert-butylester umgesetzt wurde; farbloses Öl, MS : 322 $(M+H)^+$.

(f) In analoger Weise wie in Beispiel 22 (h) beschrieben, wurde durch Einführung der Trityl-Gruppe aus (3RS,4RS)-3-Hydroxy-4-[4-(2-hydroxy-äthyl)-phenyl]-piperidin-1-carbonsäure tert-butylester der (3RS,4RS)-3-Hydroxy-4-[4-(2-trityloxy-äthyl)-phenyl]-piperidin-1-carbonsäure tert-butylester als farbloser Schaum erhalten; MS : 581 $(M+NH_4)^+$.

(g) In analoger Weise wie in Beispiel 22 (i) beschrieben, wurde durch Alkylierung mit 2-Brommethylnaphthalin aus dem (3RS,4RS)-3-Hydroxy-4-[4-(2-trityloxy-äthyl)-phenyl]-piperidin-1-carbonsäure tert-butylester der (3RS,4RS)-3-(Naphthalin-2-ylmethoxy)-4-[4-(2-trityloxy-äthyl)-phenyl]-piperidin-1-carbonsäure tert-butylester als farbloses Öl erhalten; MS : 721 $(M+H)^+$.

(h) In analoger Weise wie in Beispiel 22 (j) beschrieben, wurde aus dem (3RS,4RS)- 3-(Naphthalin-2-ylmethoxy)-4-[4-(2-trityloxy-äthyl)-phenyl]-piperidin-1-carbonsäure tert-butylester durch Abspaltung der Trityl-Gruppe der (3RS,4RS)-4-[4-(2-Hydroxy-äthyl)-phenyl]-3-naphthalin-2-ylmethoxy-piperidin-1-carbonsäure tert-butylester als farbloser Festkörper erhalten; MS : 462 $(M+H)^+$.

(i) In analoger Weise wie in Beispiel 22 (k) beschrieben, wurde durch Acylierung des (3RS,4RS)-4-[4-(2-Hydroxy-äthyl)-phenyl]-3-naphthalin-2-ylmethoxy-piperidin-1-carbonsäure tert-butylesters mit Cyclopropancarbonsäurechlorid der (3RS,4RS)-4-[4-(2-Cyclopropylcarbonyloxy-äthyl)-phenyl]-3-naphthalin-2-ylmethoxy-piperidin-1-carbonsäure tert-butylester als farbloses Öl erhalten; MS : 572 $(M+H)^+$.

(j) In analoger Weise wie in Beispiel 22 (l) beschrieben, wurde durch Abspaltung der BOC-Gruppe mittels Trifluoressigsäure in Methylenchlorid aus dem (3RS,4RS)-4-[4-(2-Cyclopropylcarbonyloxy-äthyl)-phenyl]-3-naphthalin-2-ylmethoxy-piperidin-1-carbonsäure tert-butylester das Cyclopropanecarbonsäure (3RS,4RS)-2-[4-(3-naphthalin-2-ylmethoxy-piperidin-4-yl)-phenyl]-äthylester Trifluoracetat als weißer Festkörper erhalten; MS : 430 $(M+H)^+$.

Beispiel 30

In analoger Weise wie in Beispiel 23 beschrieben, wurden durch gleichzeitige Abspaltung der BOC- und Tritylgruppe die folgenden Verbindung erhalten:

- aus (3RS,4RS)- 3-(Naphthalin-2-ylmethoxy)-4-[4-(2-trityloxy-äthyl)-phenyll-piperidin-1-carbonsäure tert-butylester das (3RS,4RS)-2-{4-[3-(Naphthalin-2-ylmethoxy)-piperidin-4-yl]-phenyl}-äthanol Hydrochlorid als weißes Pulver; MS : 362 $(M+H)^+$;

Beispiel 31

In analoger Weise wie in Beispiel 22 (l) wurden durch Abspaltung der BOC-Gruppe mittels Säure die folgenden Verbindungen erhalten:

1) - Aus (3RS,4RS)-4-[4-(2-Benzoyloxy-äthyl)-phenyl]-3-(naphthalin-2-ylmethoxy)-piperidin-1-carbonsäure tert-butylester das Benzoesäure (3RS,4RS)-2-[4-(3-Naphthalin-2-ylmethoxy-piperidin-4-yl)-phenyl]-äthyl ester Hydrochlorid als weißer Festkörper; MS : 466 $(M+H)^+$;

2) - aus (3RS,4RS)-4-{4-[2-(3-Methoxy-benzoyloxy)-äthyl]-phenyl}-3-(naphthalin-2-ylmethoxy)-piperidin-1-carbonsäure tert-butylester das 3-Methoxy-benzoesäure (3RS,4RS)-2-[4-(3-Naphthalin-2-ylmethoxypiperidin-4-yl)-phenyl]-äthylester Hydrochlorid als farbloses Öl; MS : 496 $(M+H)^+$;

3) - aus (3RS,4RS)-4-{4-[2-(2-Methoxy-benzoyloxy)-äthyl]-phenyl}-3-(naphthalin-2-ylmethoxy)-piperidin-1-carbonsäure tert-butylester das 2-Methoxy-benzoesäure (3RS,4RS)-2-[4-(3-Naphthalin-2-ylmethoxypiperidin-4-yl)-phenyl]-äthylester Hydrochlorid als farbloser Festkörper; MS : 496 $(M+H)^+$;

4) - aus (3RS,4RS)-4-[4-(2-Benzyloxyacetoxy-äthyl)-phenyl]-3-naphthalin-2-ylmethoxy-piperidin-1-carbonsäure tert-butylester das Benzyloxy-essigsäure (3RS,4RS)-2-[4-(3-Naphthalin-2-ylmethoxypiperidin-4-yl)-phenyl]-äthylester Hydrochlorid als farbloser Festkörper; MS : 510 $(M+H)^+$;

5) - aus (3RS,4RS)-4-[4-[2-[(4-Methoxy-phenyl)-acetoxy]-äthyl]-phenyl]-3-naphthalin-2-ylmethoxy-piperidin-1-carbonsäure tert-butylester unter Verwendung von Trifluoressigsäure in Methylenchlorid das (4-Methoxy-phenyl)-essigsäure (3RS,4RS)-2-[4-(3-Naphthalin-2-ylmethoxy)-piperidin-4-yl]-phenyl]-äthylester Trifluoracetat als farbloser Festkörper; MS : 510 $(M+H)^+$;

6) - aus (3RS,4RS)-4-[4-(2-Cyclohexylcarbonyloxy-äthyl)-phenyl]-3-naphthalin-2-ylmethoxy-piperidin-1-carbonsäure tert-butylester unter Verwendung von Trifluoressigsäure in Methylenchlorid das Cyclohexancarbonsäure (3RS,4RS)-2-[4-(3-Naphthalin-2-ylmethoxypiperidin-4-yl)-phenyl]-äthylester Trifluoracetat als farbloser Festkörper; MS : 472 $(M+H)^+$;

7) - aus (3RS,4RS)-4-{4-[2-(2,6-Dichlor-benzoyloxy)-äthyl]-phenyl}-3-(naphthalin-2-ylmethoxy)-piperidin-1-carbonsäure tert-butylester unter Verwendung von Trifluoressigsäure in Methylenchlorid das 2,6-Dichlor-benzoesäure (3RS,4RS)-2-[4-(3-naphthalin-2-ylmethoxypiperidin-4-yl)-phenyl]-äthylester Trifluoracetat als farbloser Feskörper; MS : 534 $(M+H)^+$;

8) - aus (3RS,4RS)-4-[4-[2-(2,6-Dimethoxy-benzoyloxy)-äthyl]-phenyl]-3-naphthalin-2-ylmethoxy-piperidin-1-carbonsäure tert-butylester unter Verwendung von Trifluoressigsäure in Methylenchlorid das 2,6-Dimethoxy-benzoesäure (3RS,4RS)-2-[4-(3-Naphthalin-2-ylmethoxypiperidin-4-yl)-phenyl]-äthylester Trifluoracetat als farbloser Festkörper; MS : 526 $(M+H)^+$;

9) - aus (3RS,4RS)-[4-[4-[2-(2-Acetoxy-benzoyloxy)-äthyl]-phenyl]-3-naphthalin-2-ylmethoxy-piperidin]-1-carbonsäure tert-butylester unter Verwendung von Trifluoressigsäure in Methylenchlorid das 2-Acetoxybenzoesäure (3RS,4RS)-2-[4-(3-Naphthalin-2-ylmethoxy-piperidin-4-yl)-phenyl]-äthylester Trifluoracetat als farbloser Festkörper; MS : 524 $(M+H)^+$;

10) - aus (3RS,4RS)-[4-[2-(2-Chlor-benzoyloxy)-äthyl]-phenyl]-3-naphthalin-2-ylmethoxy-piperidin-1-carbonsäure tert-butylester unter Verwendung von Trifluoressigsäure in Methylenchlorid das 2-Chlor-benzoesäure (3RS,4RS)-2-[4-(3-Naphthalin-2-ylmethoxy-piperidin-4-yl)-phenyl]-äthylester Trifluoracetat als farbloser Festkörper; MS : 500 $(M+H)^+$;

11) - aus (3RS,4RS)-3-Naphthalin-2-ylmethoxy-4-[4-(2-pyridin-2-ylcarbamoyloxy-äthyl)-phenyl]-piperidin-1-carbonsäure tert-butylester das Pyridin-2-yl-carbaminsäure (3RS,4RS)-2-[4-(3-naphthalin-2-ylmethoxy-piperidin-4-yl)-phenyl]-äthylester Hydrochlorid als farbloser Schaum; MS : 482 (M+H)+:

12) - aus (3RS,4RS)-3-Naphthalin-2-ylmethoxy-4-[4-(2-phenylcarbamoyloxy-äthyl)-phenyl]-piperidin-1-carbonsäure tert-butylester das Phenyl-carbaminsäure (3RS,4RS)-2-[4-(3-naphthalin-2-ylmethoxypiperidin-4-yl)-phenyl]-äthylester Hydrochlorid als farbloser Festkörper; MS : 598 $(M+H)^+$;

13) - aus (3RS,4RS)-3-Naphthalin-2-ylmethoxy-4-[4-(2-pyridin-3-ylcarbonyloxy-äthyl)-phenyl)-piperidin-1-carbonsäure tert-butylester das Pyridin-3-carbonsäure (3RS,4SR)-2-[4-(3-Naphthalin-2-ylmethoxypiperidin-4-yl)-phenyl]-äthylester Hydrochlorid als farbloser Festkörper; MS : 467 $(M+H)^+$;

14) - aus (3RS,4RS)-Pyridin-2-carbonsäure 2-{4-[1-tert-Butoxycarbonyl-3-(naphthalin-2-ylmethoxy)-piperidin-4-yl]-phenyl}-äthylester den Pyridin-2-carbonsäure (3RS,4RS)-2-[4-(3-Naphthalin-2-ylmethoxypiperidin-4-yl)-phenyl]-äthylester als farbloses Öl; MS : 467 $(M+H)^+$;

15) - aus (3RS,4RS)-3-Naphthalin-2-ylmethoxy-4-[4-(2-thiophen-3-ylcarbonyloxy-äthyl)-phenyl]-piperidin-1-carbonsäure tert-butylester unter Verwendung von Trifluoressigsäure in Methylenchlorid das Thiophen-3-carbonsäure (3RS,4RS)-2-[4-(3-Naphthalin-2-ylmethoxypiperidin-4-yl)-phenyl]-äthylester Trifluoracetat als farbloser Festkörper; MS : 472 $(M+H)^+$;

16) - aus (3RS,4RS)-3-Naphthalin-2-ylmethoxy-4-[4-(2-thiophen-3-ylacetoxy-äthyl)-phenyl]-piperidin-1-carbonsäure tert-butylester unter Verwendung von Trifluoressigsäure in Methylenchlorid das Thiophen-2-ylessigsäure (3RS,4RS)-2-[4-(3-Naphthalin-2-ylmethoxy-piperidin-4-yl)-phenyl]-äthylester Trifluoracetat als farbloser Festkörper; MS : 486 $(M+H)^+$;

17) - aus (3RS,4RS)-4-[4-(2-Imidazol-2-ylcarbonyloxy-äthyl)-phenyl]-3-naphthalin-2-ylmethoxy]-piperidin-1-carbonsäure tert-butylester unter Verwendung von Trifluoressigsäure in Methylenchlorid das Imidazol-2-carbonsäure (3RS,4RS)-2-[4-(3-Naphthalin-2-ylmethoxy-piperidin-4-yl)-phenyl]-äthylester Trifluoracetat als farbloser Festkörper; MS : 456 $(M+H)^+$;

18) - aus (3RS,4RS)-4-[4-(2-Benzoyloxy-äthyl)-phenyl-3-(4-benzyloxynaphthalin-2-ylmethoxy)-piperidin-1-carbonsäure tert-butylester das Benzoesäure (3RS,4RS)-2-[4-[3-(4-Benzyloxy-naphthalin-2-ylmethoxy)-piperidin-4-yl]-phenyl]-äthylester Hydrochlorid als gelblicher Festkörper; MS : 572 $(M+H)^+$.

Die folgenden Ausgangssubstanzen wurden analog zu dem in Beispiel 22 (k) beschriebenen Verfahren erhalten:

(a) Aus (3RS,4RS)-4-[4-(2-Hydroxy-äthyl)-phenyl]-3-naphthalin-2-ylmethoxy-piperidin-1-carbonsäure tert-butylester und Benzoylchlorid der (3RS,4RS)-4-[4-(2-Benzoyloxy-äthyl)-phenyl]-3-(naphthalin-2-ylmethoxy)-piperidin-1-carbonsäure tert-butylester als farbloses Öl; MS : 583 $(M+NH_4)^+$.

(b) Aus (3RS,4RS)-4-[4-(2-Hydroxy-äthyl)-phenyl]-3-naphthalin-2-ylmethoxy-piperidin-1-carbonsäure tert-butylester und 3-Methoxybenzoylchlorid der (3RS,4RS)-4-{4-[2-(3-Methoxy-benzoyloxy)-äthyl]-phenyl}-3-(naphthalin-2-ylmethoxy)-piperidin-1-carbonsäure tert-butylester als farbloses Öl; MS : 593 $(M+H)^+$.

(c) Aus (3RS,4RS)-4-[4-(2-Hydroxy-äthyl)-phenyl]-3-naphthalin-2-ylmethoxy-piperidin-1-carbonsäure tert-butylester und 2-Methoxybenzoylchlorid der (3RS,4RS)-4-{4-[2-(2-Methoxy-benzoyloxy)-äthyl]-phenyl}-3-(naphthalin-2-ylmethoxy)-piperidin-1-carbonsäure tert-butylester als farbloses Öl; $R_f$: 0.35 ($SiO_2$, Hexan:Essigester=2:1).

(d) Aus (3RS,4RS)-4-[4-(2-Hydroxy-äthyl)-phenyl]-3-naphthalin-2-ylmethoxy-piperidin-1-carbonsäure tert-butylester und Benzyloxyacetylchlorid der (3RS,4RS)-4-[4-(2-Benzyloxyacetoxy-äthyl)-phenyl]-3-naphthalin-2-ylmethoxy-piperidin-1-carbonsäure tert-butylester als farbloses Öl; MS : 627 $(M+NH_4)^+$.

(e) Aus (3RS,4RS)-4- [4-(2-Hydroxy-äthyl)-phenyl]-3-naphthalin-2-ylmethoxy-piperidin-1-carbonsäure tert-butylester und 4-Methoxyphenylacetylchlorid der (3RS,4RS)-4-[4-[2-[(4-Methoxy-phenyl)-acetoxy]-äthyl]-phenyl]-3-naphthalin-2-ylmethoxy-piperidin-1-carbonsäure tert-butylester als farbloses Öl; MS : 627 $(M+NH_4)^+$.

(f) Aus (3RS,4RS)-4-[4-(2-Hydroxy-äthyl)-phenyl]-3-naphthalin-2-ylmethoxy-piperidin-1-carbonsäure tert-butylester und Cyclohexancarbonsäurechlorid der (3RS,4RS)-4-[4-(2-Cyclohexylcarbonyloxy-äthyl)-phenyl]-3-naphthalin-2-ylmethoxypiperidin-1-carbonsäure tert-butylester als farbloses Öl; MS : 572 $(M+H)^+$.

(g) Aus (3RS,4RS)-4-[4-(2-Hydroxy-äthyl)-phenyl]-3-naphthalin-2-ylmethoxy-piperidin-1-carbonsäure tert-butylester und 2,6-Dichlorbenzoylchlorid der (3RS,4RS)-4-{4-[2-(2,6-Dichlor-benzoyloxy)-äthyl]-phenyl}-3-(naphthalin-2-ylmethoxy)-piperidin-1-carbonsäure tert-butylester als farbloses Öl; MS : 651 $(M+NH_4)^+$.

(h) Aus (3RS,4RS)-4-[4-(2-Hydroxy-äthyl)-phenyl]-3-naphthalin-2-ylmethoxy-piperidin-1-carbonsäure tert-butylester und 2,6-Dimethoxybenzoylchlorid der (3RS,4RS)-4-[4-[2-(2,6-Dimethoxybenzoyloxy)-äthyl]-phenyl]-3-naphthalin-2-ylmethoxy-piperidin-1-carbonsäure tert-butylester als farbloser amorpher Festkörper; MS : 643 $(M+NH_4)^+$.

(i) Aus (3RS,4RS)-4-[4-(2-Hydroxy-äthyl)-phenyl]-3-naphthalin-2-ylmethoxy-piperidin-1-carbonsäure tert-butylester und O-Acetylsalicylsäurechlorid der (3RS,4RS)-[4-[4-[2-(2-Acetoxy-benzoyloxy)-äthyl]-phenyl]-3-naphthalin-2-ylmethoxy-piperidin]-1-carbonsäure tert-butylester als farbloses Öl; MS : 641 $(M+NH_4)^+$.

(j) Aus (3RS,4RS)-4-[4-(2-Hydroxy-äthyl)-phenyl]-3-naphthalin-2-ylmethoxy-piperidin-1-carbonsäure tert-butylester und 2-Chlorbenzoylchlorid der (3RS,4RS)-4-[4-[2-(2-Chlor-benzoyloxy)-äthyl]-phenyl]-3-naphthalin-2-ylmethoxy-piperidin-1-carbonsäure tert-butylester als farbloses Öl; MS : 617 $(M+NH_4)^+$.

(k) In analoger Weise wie in Beispiel 24 (m) beschrieben, wurde durch Umsetzung von (3RS,4RS)-4-[4-(2-Hydroxy-äthyl)-phenyl]-3-naphthalin-2-ylmethoxy-piperidin-1-carbonsäure tert-butylester mit Pyridin-2-carbonylazid der (3RS,4RS)-3-Naphthalin-2-ylmethoxy-4-[4-(2-pyridin-2-ylcarbamoyloxy-äthyl)-phenyl]-piperidin-1-carbonsäure tert-butylester als farblose Kristalle erhalten; MS : 582 $(M+H)^+$.

(l) In analoger Weise wie in Beispiel 24 (m) beschrieben, wurde durch Umsetzung von (3RS,4RS)-4-[4-(2-Hydroxy-äthyl)-phenyl]-3-naphthalin-2-ylmethoxy-piperidin-1-carbonsäure tert-butylester mit Phenylisocyanat der (3RS, 4RS)-3-Naphthalin-2-ylmethoxy-4-[4-(2-phenylcarbamoyloxy-äthyl)-phenyl]-piperidin-1-carbonsäure tert-butylester als farbloses Öl erhalten; MS: 481 $(M+H)^+$.

(m) In analoger Weise wie in Beispiel 24 (g) beschrieben, wurde durch Kondensation von (3RS,4RS)-4-[4-(2-Hydroxy-äthyl)-phenyl]-3-naphthalin-2-ylmethoxy-piperidin-1-carbonsäure tert-butylester und Nicotinsäure der (3RS, 4RS)-3-Naphthalin-2-ylmethoxy-4-[4-(2-pyridin-3-ylcarbonyloxy-äthyl)-phenyl]-piperidin-1-carbonsäure tert-butylester als farbloses Öl erhalten; MS: 567 $(M+H)^+$.

(n) In analoger Weise wie in Beispiel 24 (g) beschrieben, wurde durch Kondensation von (3RS,4RS)-4-[4-(2-Hydroxy-äthyl)-phenyl]-3-naphthalin-2-ylmethoxy-piperidin-1-carbonsäure tert-butylester und Pyridin-2-carbonsäure unter Verwendung von 1,1-Carbonyldiimidazol als Kondensationsmittel der (3RS,4RS)-Pyridin-2-carbonsäure 2-{4-[1-tert-Butoxycarbonyl-3-(naphthalin-2-ylmethoxy)-piperidin-4-yl]-phenyl}-äthylester als farbloses Öl erhalten; MS : 467 $(M+H)^+$.

(o) In analoger Weise wie in Beispiel 24 (g) beschrieben, wurde durch Kondensation von (3RS,4RS)-4-[4-(2-Hydroxy-äthyl)-phenyl]-3-naphthalin-2-ylmethoxy-piperidin-1-carbonsäure tert-butylester und Thiophen-3-carbonsäure unter Verwendung von 1,1-Carbonyldiimidazol als Kondensationsmittel der (3RS,4RS)-3-Naphthalin-2-ylmethoxy-4-[4-(2-thiophen-3-ylcarbonyloxy-äthyl)-phenyl]-piperidin-1-carbonsäure tert-butylester als weiße Kristalle erhalten; MS : 572 $(M+H)^+$.

(p) In analoger Weise wie in Beispiel 24 (g) beschrieben, wurde durch Kondensation von (3RS,4RS)-4-[4-(2-Hydroxy-äthyl)-phenyl]-3-naphthalin-2-ylmethoxy-piperidin-1-carbonsäure tert-butylester und 3-Thiophenessigsäure unter Verwendung von 1,1-Carbonyldiimidazol als Kondensationsmittel der (3RS,4RS)-3-Naphthalin-2-ylmethoxy-4-[4-(2-thiophen-3-ylacetoxy-äthyl)-phenyl]-piperidin-1-carbonsäure tert-butylester als farbloser Festkörper erhalten; MS : 603 $(M+NH_4)^+$.

(q) In analoger Weise wie in Beispiel 24 (g) beschrieben, wurde durch Kondensation von (3RS,4RS)-4-[4-(2-Hydroxy-äthyl)-phenyl]-3-naphthalin-2-ylmethoxy-piperidin-1-carbonsäure tert-butylester und Imidazol-2-carbonsäure unter Verwendung von 1,1-Carbonyldiimidazol als Kondensationsmittel der (3RS,4RS)-4-[4-(2-Imidazol-2-ylcarbonyloxy-äthyl)-phenyl]-3-naphthalin-2-ylmethoxy]-piperidin-1-carbonsäure tert-butylester als farbloser Festkörper erhalten; MS : 556 $(M+H)^+$.

In analoger Weise wie in Beispiel 22 (i)-(k) beschrieben, wurde wie folgt verfahren:

(r) Die Alkylierung von (3RS,4RS)-3-Hydroxy-4-[4-(2-trityloxy-äthyl)-phenyl]-piperidin-1-carbonsäure tert-butylester mit 1-Benzyloxy-3-chlormethyl-naphthalin ergab den (3RS,4RS)-3-(4-Benzyloxynaphthalin-2-ylmethoxy)-4-[4-(2-trityloxy-äthyl)-phenyl]-piperidin-1-carbonsäure tert-butylester als farbloses Öl; MS : 827 $(M+NH_4)^+$.

(s) Die Abspaltung der Tritylgruppe aus dem (3RS,4RS)-3-(4-Benzyloxynaphthalin-2-ylmethoxy)-4-[4-(2-trityloxy-äthyl)-phenyl]-piperidin-1-carbonsäure tert-butylester lieferte den (3RS,4RS)-3-(4-Benzyloxynaphthalin-2-ylmethoxy)-4-[4-(2-hydroxy-äthyl)-phenyl]-piperidin-1-carbonsäure tert-butylester als farbloses Öl; MS : 568 $(M+H)^+$.

(t) Die Acylierung des (3RS,4RS)-3-(4-Benzyloxy-naphthalin-2-ylmethoxy)-4-[4-(2-hydroxy-äthyl)-phenyl]-piperidin-1-carbonsäure tert-butylester mit Benzoylchlorid ergab den (3RS,4RS)-4-[4-(2-Benzoyloxy-äthyl)-phenyl-3-(4-benzyloxy-naphthalin-2-ylmethoxy)-piperidin- 1-carbonsäure tert-butylester als farbloses Öl; MS : 689 $(M+NH_4)^+$.

Beispiel 32

In analoger Weise wie in Beispiel 22 (l) beschrieben, wurden durch Abspaltung der BOC-Gruppe mittels Säure die folgenden Verbindungen erhalten:

1) - Aus (3RS,4RS)-3-(Naphthalin-2-ylmethoxy)-4-[4-(naphthalin-2-ylmethoxymethyl)-phenyl]-piperidin-1-carbonsäure tert-butylester das (3RS,4RS)-3-Naphthalin-2-ylmethoxy-4-(4-naphthalin-2-ylmethoxymethyl-phenyl)-piperidin Hydrochlorid als farbloser Festkörper; MS : 488 $(M+H)^+$;

2) - aus (3RS,4RS)-4-[4-(4-Methoxy-phenoxymethyl)-phenyl]-3-naphthalin-2-ylmethoxy-piperidin-1-carbonsäure tert-butylester das (3RS,4RS)-4-[4-(4-Methoxy-phenoxymethyl)-phenyl]-3-naphthalin-2-ylmethaxy-piperidin als gelber Schaum; MS : 454 $(M+H)^+$;

3) - aus (3RS,4RS)-3-(1-Methoxy-naphthalin-2-ylmethoxy)-4-[4-(2-phenoxy-äthyl)-phenyl]-piperidin-1-carbonsäure tert-butylester unter Verwendung von Trifluoressigsäure in Methylenchlorid das (3RS,4RS)-3-(1-Methoxy-naphthalin-2-ylmethoxy)-4-[4-(2-phenoxy-äthyl)-phenyl)-piperidin Trifluoracetat als farbloses Öl; MS : 468 $(M+H)^+$;

4) - aus (3RS,4RS)-4-[4-(2-Methoxy-äthyl)-phenyl]-3-naphthalin-2-ylmethoxy]-piperidin-1-carbonsäure tert-butylester das (3RS,4RS)-4-[4-(2-Methoxy-äthyl)-phenyl]-3-naphthalin-2-ylmethoxy]-piperidin als farbloser Festkörper; MS : 376 $(M+H)^+$.

Die als Ausgangssubstanzen eingesetzten BOC-Derivate wurden wie folgt hergestellt:

(a) In analoger Weise wie in Beispiel 22 (i) beschrieben, wurde durch Alkylierung des (3RS,4RS)-3-Hydroxy-4-(4-hydroxymethyl-phenyl)-piperidin-1-carbonsäure tert-butylesters mit 2-Brommethylnaphthalin der (3RS,4RS)-3-(Naphthalin-2-ylmethoxy)-4-[4-(naphthalin-2-ylmethoxymethyl)-phenyl]-piperidin-1-carbonsäure tert-butylester als farbloses Öl erhalten; $R_f$: 0.31 ($SiO_2$, Hexan:Essigester=2:1).

(b) Eine Lösung von 200 mg (0.45 mMol) (3RS,4RS)-4-(4-Hydroxymethylphenyl)-3-(naphthalin-2-ylmethoxy)-piperidin-1-carbonsäure tert-butylester in 10 ml Tetrahydrofuran wurde in der Folge mit 143 mg (0.58 mMol) Triphenylphosphin, 94 mg (0.58 mMol) Azodicarbonsäurediethylester und 166 mg (1.35 mMol) Hydrochinonmonomethyläther versetzt und das Reaktionsgemisch bei Raumtemperatur 15 Stunden lang gerührt. Zur Aufarbeitung wurde das Reaktionsgemisch mit 20 ml Methylenchlorid verdünnt und mit 20 ml gesättigter Natriumcarbonat-Lösung ausgeschüttelt. Die organische Phase wurde über Natriumsulfat getrocknet und unter vermindertem Druck eingedampft. Zur Reinigung des Rohprodukts wurde an Kieselgel unter Verwendung von Methylenchlorid als Elutierungsmittel chromatographiert. Es wurden 245 mg eines farblosen Öls erhalten, das neben (3RS,4RS)-4- [4-(4-Methoxy-phenoxymethyl)-phenyl]-3-naphthalin-2-ylmethoxy-piperidin- 1-carbonsäure tert-butylester, $R_f$: 0.08 ($SiO_2$, Methylenchlorid) auch noch (3RS,4RS)-4-(4-Hydroxymethylphenyl)-3-(naphthalin-2-ylmethoxy)-piperidin-1-carbonsäure tert-butylester enthielt.

In analoger Weise wie in Beispiel 22 (i)-(j) beschrieben, wurde wie folgt verfahren:

(c) Die Alkylierung des (3RS,4RS)-3-Hydroxy-4-[4-(2-trityloxy-äthyl)-phenyl]-piperidin-1-carbonsäure tert-butylesters mit 2-Brommethyl-1-methoxy-naphthalin ergab den (3RS,4RS)-3-(1-Methoxy-naphthalin-2-ylmethoxy)-4-[4-(2-trityloxy-äthyl)-phenyl]-piperidin-1-carbonsäure tert-butylester, aus dem nach Abspaltung der Tritylgruppe der (3RS,4RS)-4-[4-(2-Hydroxy-äthyl)-phenyl]-3-(1-methoxy-naphthalin-2-ylmethoxy)-piperidin-1-carbonsäure tert-butylester und nach Reaktion mit Phenol, analog dem unter (b) beschriebenen Verfahren, der (3RS,4RS)-3-(1-Methoxy-naphthalin-2-ylmethoxy)-4-[4-(2-phenoxy-äthyl)-phenyl]-piperidin-1-carbonsäure tert-butylester als farbloses Öl erhalten wurde; $R_f$: 0.34 ($SiO_2$, Hexan:Essigester=2:1).

(d) In analoger Weise wie in Beispiel 22 (i) beschrieben, wurde durch Alkylierung des (3RS,4RS)-4-[4-(2-Hydroxy-äthyl)-phenyl]-3-naphthalin-2-ylmethoxy-piperidin-1-carbonsäure tert-butylesters mit Methyliodid der (3RS,4RS)-4-[4-(2-Methoxy-äthyl)-phenyl]-3-naphthalin-2-ylmethoxy]-piperidin-1-carbonsäure tert-butylester als farbloses Öl erhalten; MS : 493 $(M+NH_4)^+$.

Beispiel 33

In analoger Weise wie in Beispiel 22 (l) beschrieben, wurden durch Abspaltung der BOC-Gruppe mittels Säure die folgenden Verbindungen erhalten:

1) - Aus (3RS,4RS)-3-Naphthalin-2-ylmethoxy-4-[4-(2-phenylsulfanyl-äthyl)-phenyl]-piperidin-1-carbonsäure tert-butylester das (3RS,4RS)-3-Naphthalin-2-ylmethoxy-4-[4-(2-phenylsulfanyl-äthyl)-phenyl]-piperidin Hydrochlorid als weiße Kristalle; MS : 454 $(M+H)^+$;

2) - aus dem Gemisch von (3RS,4RS)-3-Naphthalin-2-ylmethoxy-4-[4-[(RS)- und -[(SR)-2-phenylsulfinyl-äthyl]-phenyl]-piperidin-1-carbonsäure tert-butyester das Gemisch von (3RS,4RS)-3-Naphthalin-2-ylmethoxy-4-[4-[(RS)- und -[(SR)-2-phenylsulfinyl-äthyl]-phenyl]-piperidin als farbloser, amorpher Festkörper; MS : 470 $(M+H)^+$;

3) - aus (3RS,4RS)-3-Naphthalin-2-ylmethoxy-4-[4-(2-phenylsulfonyl-äthyl)-phenyl]-piperidin-1-carbonsäure tert-butylester das (3RS,4RS)-3-Naphthalin-2-ylmethoxy-4- [4-(2-phenylsulfonyl-äthyl)-phenyl]-piperidin Hydrochlorid als weiße Kristalle; MS : 486 $(M+H)^+$;

4) - aus (3RS,4RS)-4-[4-(2-Benzothiazol-2-ylsulfanyl-äthyl)-phenyl]-3-naphthalin-2-ylmethoxy-piperidin-1-carbonsäure tert-butylester das (3RS,4RS)-4-[4-(2-Benzothiazol-2-ylsulfanyl-äthyl)-phenyl]-3-naphthalin-2-ylmethoxy-piperidin als farbloser Schaum; MS : 511 $(M+H)^+$;

5) - aus (3RS,4RS)-4-[4-(Benzothiazol-2-ylsulfanylmethyl)-phenyl]-3-naphthalin-2-ylmethoxy-piperidin-1-carbonsäure tert-butylester das (3RS,4RS)-2-[4-(3-Naphthalin-2-ylmethoxy-piperidin-4-yl)-benzylsulfanyl]-benzothiazol als gelber Schaum; MS : 497 $(M+H)^+$;

6) - aus (3RS,4RS)-3-Naphthalin-2-ylmethoxy-4-[4-(1-phenyl-1Htetrazol-5-ylsulfanylmethyl)-phenyl]-piperidin-1-carbonsäure tert-butylester das (3RS,4RS)-3-Naphthalin-2-ylmethoxy-4-[4-(1-phenyl-1Htetrazol-5-ylsulfanylmethyl)-phenyl]-piperidin Hydrochlorid als farbloses Öl; MS : 508 $(M+H)^+$;

7) - aus (3RS,4RS)-3-(Naphthalin-2-ylmethoxy)-4-[4-(2-phenoxy-äthyl)-phenyl]-piperidin-1-carbonsäure tert-butylester das (3RS,4RS)-3-(Naphthalin-2-ylmethoxy)-4-[4-(2-phenoxy-äthyl)-phenyl]-piperidin als gelblichen Schaum; MS : 438 $(M+H)^+$.

Die als Ausgangssubstanzen eingesetzten BOC-Derivate wurden wie folgt hergestellt:

(a) Ein Gemisch von 200 mg (0.43 m Mol) (3RS,4RS)-4-[4-(2-Hydroxy-äthyl)-phenyl]-3-naphthalin-2-ylmethoxy-piperidin-1-carbonsäure tert-butylester, 263 mg (1.29 mMol) Tributylphosphin und 284 mg (1.29 mMol) Diphenylsulfid in 1 ml Pyridin wurde bei Raumtemperatur 18 Stunden lang gerührt. Anschließend wurde das Reaktionsgemisch unter vermindertem Druck eingedampft und der Rückstand ohne weitere Aufarbeitung direkt an Kieselgel unter Verwendung von Methylenchlorid als Eluierungsmittel chromatographiert. Der (3RS,4RS)-3-Naphthalin-2-ylmethoxy-4-[4-(2-phenylsulfanyl-äthyl)-phenyl]-piperidin-1-carbonsäure tert-butylester wurde als farbloses Öl in quantitativer Ausbeute erhalten; MS : 554 $(M+H)^+$.

(b) Zu einer Lösung von 128 mg (0.23 mMol) (3RS,4RS)-3-Naphthalin-2-ylmethoxy-4-[4-(2-phenylsulfanyl-äthyl)-phenyl]-piperidin-1-carbonsäure tert-butylester in 10 ml Methylenchlorid wurde sehr langsam bei Raumtemperatur eine Lösung von 216 mg (0.23 mMol) Tetrabutylammoniumoxon getropft. Nach 6 Stunden wurde die Reaktionslösung unter vermindertem Druck eingedampft und das Rohprodukt zur Abtrennung des bereits entstandenen Sulfons an Kieselgel unter Verwendung eines 2:1-Gemisches von Hexan und Essigester als Eluierungsmittel chromatographiert. Es wurden 100 mg (76% d.Th.) des Gemisches von (3RS,4RS)-3-Naphthalin-2-ylmethoxy-4-[4-[(RS)- und -[(SR)-2-phenylsulfinyl-äthyl]-phenyl]-piperidin-1-carbonsäure tert-butylester als farbloses Öl, das allmählich durchkristallisierte, erhalten; MS : 570 $(M+H)^+$.

(c) In analoger Weise wie unter (b) beschrieben, wurde ausgehend von (3RS,4RS)-3-Naphthalin-2-ylmethoxy-4-[4-(2-phenylsulfanyl-äthyl)-phenyl]-piperidin-1-carbonsäure tert-butylester unter Verwendung eines Überschusses von Tetrabutylammoniumoxone der (3RS,4RS)-3-Naphthalin-2-ylmethoxy-4-[4-(2-phenylsulfonyl-äthyl)-phenyl]-piperidin-1-carbonsäure tert-butylester als farbloser Schaum erhalten; MS : 603 $(M+NH_4)^+$.

(d) In analoger Weise wie unter (a) beschrieben, wurde durch Reaktion von (3RS,4RS)-4-[4-(2-Hydroxy-äthyl)-phenyl]-3-naphthalin-2-ylmethoxypiperidin-1-carbonsäure tert-butylester mit Bis-(Benzothiazol-2-yl)-disulfid der (3RS,4RS)-4-[4-(2-Benzothiazol-2-ylsulfanyl-äthyl)-phenyl]-3-naphthalin-2-ylmethoxy-piperidin-1-carbonsäure tert-butylester als gelblicher Festkörper erhalten; MS : 611 $(M+H)^+$.

(e) In analoger Weise wie unter (a) beschrieben, wurde durch Reaktion von (3RS,4RS)-4-(4-Hydroxymethyl-phenyl)-3-(naphthalin-2-ylmethoxy)-piperidin-1-carbonsäure tert-butylester mit (Benzothiazol-2-yl)-disulfid der (3RS, 4RS)-4-[4-(Benzothiazol-2-ylsulfanylmethyl)-phenyl]-3-naphthalin-2-ylmethoxy-piperidin-1-carbonsäure tert-butylester als gelblicher Schaum erhalten; MS : 597 $(M+H)^+$.

(f) In analoger Weise wie unter (a) beschrieben, wurde durch Reaktion von (3RS,4RS)-4-(4-Hydroxymethyl-phenyl)-3-(naphthalin-2-ylmethoxy)-piperidin-1-carbonsäure tert-butylester mit Bis-(1-Phenyl-1H-tetrazol-5-yl)-disulfid der (3RS,4RS)-3-Naphthalin-2-ylmethoxy-4-[4-(1-phenyl-1Htetrazol-5-ylsulfanylmethyl)-phenyl]-piperidin-1-carbonsäure tert-butylester als farbloser Schaum erhalten; MS : 630 $(M+Na)^+$.

(g) In analoger Weise wie in Beispiel 32 (b) beschrieben, wurde aus (3RS,4RS)-4-[4-(2-Hydroxy-äthyl)-phenyl]-3-naphthalin-2-ylmethoxypiperidin-1-carbonsäure tert-butylester der (3RS,4RS)-3-(Naphthalin-2-ylmethoxy)-4-[4-(2-phenoxy-äthyl)-phenyl]-piperidin-1-carbonsäure tert-butylester als farbloses Öl erhalten; MS : 555 $(M+NH_4)^+$.

Beispiel 34

(a) Zu einer Lösung von 100 mg (0.22 mMol) (3RS,4RS)-4-(4-Hydroxymethyl-phenyl)-3-(naphthalin-2-ylmethoxy)-piperidin-1-carbonsäure tert-butylester in 10 ml Tetrahydrofuran wurden 68 mg (0.66 mMol) Triäthylamin gegeben und danach bei 0° C 25 mg (0.26 mMol) Methansulfonsäurechlorid getropft. Die Reaktionslösung wurde 90 Minuten bei Raumtemperatur gerührt, danach 38 mg (0.33 mMol) 2-Mercaptopyrimidin zugegeben und weitere 18 Stunden bei Raumtemperatur gerührt. Zur Aufarbeitung wurde das Reaktionsgemisch unter vermindertem Druck eingedampft, der Rückstand in 20 ml Methylenchlorid aufgenommen und dann mit 10 ml Wasser ausgeschüttelt. Die organische Phase wurde über Natriumsulfat getrocknet und anschließend unter vermindertem Druck eingedampft. Zur Reinigung des Rohprodukts wurde an Kieselgel unter Verwendung eines 5:1-Gemisches von Hexan und Essigester als Eluierungsmittel chromatographiert. Es wurden 100 mg (82% d.Th.) (3RS,4RS)-3-Naphthalin-2-ylmethoxy-4-[4-(pyrimidin-2-ylsulfanylmethyl)-phenyl]-piperidin-1-carbonsäure tert-butylester als gelbliches Öl erhalten; MS : 542 $(M+H)^+$.
(b) In analoger Weise wie in Beispiel 22 (l) beschrieben, wurde durch Abspaltung der BOC-Gruppe das (3RS, 4RS)-2-[4-(3-Naphthalin-2-ylmethoxy-piperidin-4-yl)-benzylsulfanyl]-pyrimidin als gelber Schaum erhalten; MS : 442 $(M+H)^+$.

Beispiel 35

In analoger Weise wie in Beispiel 22 (l) beschrieben, wurde durch Abspaltung der BOC-Gruppe mittels Säure die folgenden Verbindungen erhalten:

1) - Aus (3RS,4RS)-3-Naphthalin-2-ylmethoxy-4-[4-(6-nitrobenzothiazol-2-ylsulfanylmethyl)-phenyl]-piperidin-1-carbonsäure tert-butylester das (3RS,4RS)-2-[4-(3-Naphthalin-2-ylmethoxy-piperidin-4-yl)-benzylsulfanyl]-6-nitro-benzothiazol Hydrochlorid als gelblicher Schaum; MS : 542 $(M+H)^+$;

2) - aus (3RS,4RS)-4-[4-(2-Cyano-äthyl)-phenyl]-3-naphthalin-2-ylmethoxy-piperidin-1-carbonsäure tert-butylester unter Verwendung von Trifluoressigsäure in Methylenchlorid das (3RS,4RS)-3-{4-[3-(Naphthalin-2-ylmethoxy)-piperidin-4-yl]-phenyl}-propionitril Trifluoracetat als weißes Pulver; MS : 371 $(M+H)^+$.

Die als Ausgangsstoffe eingesetzten BOC-Derivate wurden wie folgt erhalten:

(a) In analoger Weise wie in Beispiel 34 (a) beschrieben, wurde durch Reaktion von (3RS,4RS)-4-(4-Hydroxymethyl-phenyl)-3-(naphthalin-2-ylmethoxy)-piperidin-1-carbonsäure tert-butylester mit 6-Nitro-2-mercaptobenzothiazol über das in situ hergestellte Mesylat der (3RS,4RS)-3-Naphthalin-2-ylmethoxy-4-[4-(6-nitro-benzothiazol-2-ylsulfanylmethyl)-phenyl]-piperidin-1-carbonsäure tert-butylester als gelber Festkörper erhalten; MS : 642 $(M+H)^+$.

(b) In analoger Weise wie in Beispiel 34 (a) beschrieben, wurde durch Reaktion von (3RS,4RS)-4-[4-(2-Hydroxyäthyl)-phenyl]-3-naphthalin-2-ylmethoxy-piperidin-1-carbonsäure tert-butylester mit Kaliumcyanid in Dimethylformamid über das entsprechende Mesylat das (3RS,4RS)-4-[4-(2-Cyano-äthyl)-phenyl]-3-naphthalin-2-ylmethoxy-piperidin-1-carbonsäure tert-butylester als weißes Pulver erhalten; MS : 471 $(M+H)^+$.

Beispiel 36

(a) Zu einer Lösung von 5.0 g (10.83 mMol) (3RS,4RS)-4-[4-(2-Hydroxy-äthyl)-phenyl]-3-naphthalin-2-ylmethoxy-piperidin-1-carbonsäure tert-butylester in 500 ml Benzol wurden 6.95 g (44 mMol) pulverisiertes Kaliumpermanganat, gelöst in einem Gemisch von 100 ml Wasser und 100 ml Eisessig, sowie 0.73 g (2 mMol) Tetrabutylammoniumjodid gegeben. Das Reaktionsgemisch wurde 48 Stunden lang intensiv gerührt. Zur Aufarbeitung wurden die Phasen getrennt. Die organische Phase wurde mit 100 ml gesättigter Natriumthiosulfat-Lösung gewaschen. Die wäßrige Phase wurde durch Zugabe von gesättigter Natriumthiosulfat-Lösung entfärbt und anschließend zweimal mit je 100 ml Essigester und 100 ml Methylenchlorid ausgeschüttelt. Die organischen Phasen wurden vereinigt, über Natriumsulfat getrocknet und unter vermindertem Druck eingedampft. Zur Reinigung des Rohmaterials wurde an Kieselgel unter Verwendung eines 9:1-Gemisches von Methylenchlorid und Methanol als Eluierungsmittel chromatographiert, nachdem zuvor die Säule mit einem 90:10:0.1-Gemisch von Methylenchlorid, Methanol und Ammoniak präpariert worden war. Es wurden 2.6 g (50% d.Th.) (3RS,4RS)-[4-(1-tert-Butoxycarbonyl-3-naphthalin-2-ylmethoxypiperidin-4-yl)-phenyl] -essigsäure als farbloser, amorpher Festkörper erhalten; MS : 476 $(M+H)^+$.

(b) Eine Lösung von 150 mg (0.32 mMol) (3RS,4RS)-[4-(1-tert-Butoxycarbonyl-3-naphthalin-2-ylmethoxy-piperidin-4-yl)-phenyl]-essigsäure und 55 mg (0.32 mMol) 2-Amino-1-phenyl-äthanon in 5 ml Dimethylformamid wurde in der Folge mit 44.6 µl (0.32 mMol) Triäthylamin und 96 mg (0.32 mMol) O-(1,2-Dihydro-2-oxo-1-pyridyl)-N,N,N',N'-tetramethyluronium Tetrafluorborat (TPTU) versetzt und 18 Stunden lang bei Raumtemperatur gerührt. Zur Aufarbeitung wurde das Reaktions im Ölpumpenvakuum eingedampft, der Rückstand in 20 ml Methylenchlorid aufgenommen und mit 5 ml Wasser gewaschen. Die organische Phase wurde über Natriumsulfat getrocknet und unter vermindertem Druck eingedampft. Zur Reinigung des Rohmaterials wurde an Kieselgel unter Verwendung eines 95:5-Gemisches von Methylenchlorid und Methanol als Eluierungsmittel chromatographiert. Es wurden 120 mg (64% d.Th.) (3RS,4RS)-3-(Naphthalin-2-ylmethoxy)-4-{4-[(2-oxo-2-phenyl-äthylcarbamoyl)-methyl]-phenyl}-piperidin-1-carbonsäure tert-butylester als farbloses Öl erhalten; MS : 615 $(M+Na)^+$.

(c) In analoger Weise wie in Beispiel 22 (l) beschrieben, wurde ausgehend von (3RS,4RS)-3-(Naphthalin-2-ylmethoxy)-4-{4-[(2-oxo-2-phenyl-äthylcarbamoyl)-methyl]-phenyl}-piperidin-1-carbonsäure tert-butylester durch Abspaltung der BOC-Gruppe mittels Trifluoressigsäure in Methylenchlorid das (3RS,4RS)-2-{4-[3-(Naphthalin-2-ylmethoxy)-piperidin-4-yl]-phenyl}-N-(2-oxo-2-phenyl-äthyl)-acetamid Trifluoracetat als weißes Pulver erhalten; MS : 493 $(M+H)^+$.

Beispiel 37

In analoger Weise wie in Beispiel 36 (b)-(c) beschrieben, wurden die folgenden Verbindungen erhalten:

1) - Aus (3RS,4RS)-[4-(1-tert-Butoxycarbonyl-3-naphthalin-2-ylmethoxy-piperidin-4-yl)-phenyl]-essigsäure und 3-Amino-1-phenylpropan-1-on [H.Zinner und G.Brossmann, J.Prakt.Chem. 5, 91 (1958)] wurde der (3RS,4RS)-3-(Naphthalin-2-ylmethoxy)-4-{4-[(3-oxo-3-phenylpropylcarbamoyl)-methyl]-phenyl}-piperidin-1-carbonsäure tert-butylester, MS : 607 $(M+H)^+$, erhalten, der nach Abspaltung der BOC-Gruppe das (3RS,4RS)-2-[4-[3-(Naphthalin-2-ylmethoxy)-piperidin-4-yl]-phenyl]-N-(3-oxo-3-phenyl-propyl)-acetamid Trifluoracetat als weißes Pulver ergab; MS : 507 $(M+H)^+$.

2) - Aus (3RS,4RS)-[4-(1-tert-Butoxycarbonyl-3-naphthalin-2-ylmethoxy-piperidin-4-yl)-phenyl]-essigsäure und 2-Hydroxy-1-phenyl-äthanon mit 1,1-Carbonyldiimidazol als Kondensationsmittel wurde der (3RS,4RS)-3-(Naphthalin-2-ylmethoxy)-4-[4-(2-oxo-2-phenyl-äthoxycarbonylmethyl)-phenyl]-piperidin-1-carbonsäure tert-butylester

erhalten, der nach Abspaltung der BOC-Gruppe das (3RS,4RS)-[4-[3-(Naphthalin-2-ylmethoxy)-piperidin-4-yl]-phenyl]-essigsäure 2-oxo-2-phenyl-äthylester Trifluoracetat als weißes Pulver ergab; MS : 494 $(M+H)^+$.

3) - Aus (3RS,4RS)-[4-(1-tert-Butoxycarbonyl-3-naphthalin-2-ylmethoxy-piperidin-4-yl)-phenyl]-essigsäure und Phenol mit 1,1-Carbonyldiimidazol als Kondensationsmittel wurde der (3RS,4RS)-3-(Naphthalin-2-ylmethoxy)-4-(4-phenoxycarbonylmethyl-phenyl)-piperidin-1-carbonsäure tert-butylester erhalten, der nach Abspaltung der BOC-Gruppe das (3RS,4RS)-[4-(3-Naphthalin-2-ylmethoxy-piperidin-4-yl)-phenyl]-essigsäure phenylester Trifluoracetat als weiße Kristalle ergab; MS : 452 $(M+H)^+$.

Beispiel 38

(a) Eine Lösung von 100 mg (0.21 mMol) (3RS,4RS)-[4-(1-tert-Butoxycarbonyl-3-naphthalin-2-ylmethoxy-piperidin-4-yl)-phenyl]-essigsäure in 5 ml Dimethylformamid wurde mit 50 mg (0.31 mMol) 1,1-Carbonyldümidazol versetzt ein Stunde bei 50° C gerührt. Anschließend wurde auf Raumtemperatur abgekühlt, eine Lösung von 45 mg (0.33 mMol) Benzamidoxim in 2 ml Dimethylformamid zugegeben und eine Stunde lang bei 50° C gerührt. Zur Aufarbeitung wurde auf Raumtemperatur abgekühlt und im Ölpumpenvakuum das Dimethylformamid abdestilliert. Der Rückstand wurde an Kieselgel unter Verwendung eines 98:2:0.1-Gemisches von Methylenchlorid, Methanol und Ammoniak als Eluierungsmittel chromatographiert. Es wurden 90 mg (72% d.Th.) des Benzamidoximesters als farbloser Schaum erhalten; MS : 594 $(M+H)^+$, $R_f$: 0.68 ($SiO_2$, Methylenchlorid:Methanol:Ammoniak=95:5:0.1)

(b) Eine Lösung von 90 mg (0.15 mMol) des Benzamidoximesters in 10 ml Dimethylformamidwurde 18 Stunden lang auf 130° C erhitzt. Zur Aufarbeitung wurde unter vermindertem Druck eingedampft und der Rückstand an Kieselgel unter Verwendung eines 4:1-Gemisches von Hexan und Essigester als Eluierungsmittel chromatographiert. Es wurden 63 mg (72% d.Th.) (3RS,4RS)-3-(Naphthalin-2-ylmethoxy)-4-[4-(3-phenyl-[1,2,4]oxadiazol-5-ylmethyl)-phenyl]-piperidin-1-carbonsäure tert-butylester als farbloses Öl erhalten; MS : 592 $(M+NH_4)^+$.

(c) In analoger Weise wie in Beispiel 22 (1) beschrieben, wurde ausgehend von (3RS,4RS)-3-(Naphthalin-2-ylmethoxy)-4-[4-(3-phenyl-[1,2,4]oxadiazol-5-ylmethyl)-phenyl]-piperidin- 1-carbonsäure tert-butylester durch Abspaltung der BOC-Gruppe mittels Trifluoressigsäure in Methylenchlorid das (3RS,4RS)-3-(3-Naphthalin-2-ylmethoxy)-4-[4-(3-phenyl-[1,2,4]oxadiazol-5-ylmethyl)-phenyl]-piperidin Trifluoracetat als weißes Pulver erhalten; MS : 476 (M+H)+.

Beispiel 39

(a) Eine Lösung von 100 mg (0.21 mMol) (3RS,4RS)-[4-(1-tert-Butoxycarbonyl-3-naphthalin-2-ylmethoxy-piperidin-4-yl)-phenyl]-essigsäure in 5 ml Methylenchlorid wurde mit 1 ml ätherischer Diazomethan-Lösung bei Raumtemperatur versetzt und 2 Stunden weitergerührt. Die Reaktionslösung wurde unter vermindertem Druck eingedampft und der in quantitativer Ausbeute erhaltene (3RS,4RS)-4-(4-Methoxycarbonylmethyl-phenyl)-3-(naphthalin-2-ylmethoxy)-piperidin-1-carbonsäure tert-butylester, $R_f$: 0.5 ($SiO_2$, Hexan:Essigester=2:1), wurde ohne weitere Reinigung und Charakterisierung in der folgenden Stufe eingesetzt.

(b) Eine Lösung von 102 mg (0.21 mM) (3RS,4RS)-4-(4-Methoxycarbonylmethyl-phenyl)-3-(naphthalin-2-ylmethoxy)-piperidin-1-carbonsäure tert-butylester in 0.5 ml Hydrazinhydrat wurde 18 Stunden lang auf 120° C erhitzt. Zur Aufarbeitung wurde das Reaktionsgemisch auf Raumtemperatur abgekühlt, mit 3 ml Eiswasser versetzt und zweimal mit je 5 ml Methylenchlorid extrahiert. Die vereinigten organischen Phasen wurden über Natriumsulfat getrocknet und unter vermindertem Druck eingedampft. Es wurden 63 mg (63% d.Th.) (3RS,4RS)-4-(4-Hydrazincarbonylmethyl-phenyl)-3-(naphthalin-2-ylmethoxy)-piperidin-1-carbonsäure tert-butylester als farbloser Schaum erhalten; $R_f$: 0.22 ($SiO_2$, Methylenchlorid: Methanol:Ammoniak=95:5:0.1).

(c) Ein Gemisch von 60 mg (0.12 mMol) (3RS,4RS)-4-(4-Hydrazincarbonylmethyl-phenyl)-3-(naphthalin-2-ylmethoxy)-piperidin-1-carbonsäure tert-butylester und 27.7 µl (0.12 mMol) Triäthylorthobenzoat in 5 ml Äthanol wurde 18 Stunden lang unter Rückfluß gekocht. Nach dem Abkühlen wurde unter vermindertem Druck eingedampft und der Rückstand an Kieselgel unter Verwendung eines 2:1-Gemisches von Hexan und Essigester als Eluierungsmittel chromatographiert. Es wurden 30 mg (44% d.Th.) (3RS,4RS)-3-(Naphthalin-2-ylmethoxy)-4-[4-(5-phenyl-[1,3,4]oxadiazol-2-ylmethyl)-phenyl]-piperidin-1-carbonsäure tert-butylester als farbloser Schaum erhalten; MS: 575 $(M+H)^+$.

(d) In analoger Weise wie in Beispiel 22 (l) beschrieben, wurde ausgehend von (3RS,4RS)-3-(Naphthalin-2-ylmethoxy)-4-[4-(5-phenyl-[1,3,4]oxadiazol-2-ylmethyl)-phenyl]-piperidin-1-carbonsäure tert-butylester durch Abspaltung der BOC-Gruppe mittels Trifluoressigsäure in Methylenchlorid das (3RS,4RS)-3-Naphthalin-2-ylmethoxy-4-[4-(5-phenyl-[1,3,4]oxadiazol-2-ylmethyl)-phenyl]-piperidin Trifluoracetat als weißes Pulver erhalten; MS : 476 $(M+H)^+$.

Beispiel 40

(a) Zu einer Lösung von 80 mg (0.17 mMol) (3RS,4RS)-4-[4-(2-Cyano-äthyl)-phenyl]-3-naphthalin-2-ylmethoxy-piperidin-1-carbonsäure tert-butylester (Beispiel 35) in 10 mL Toluol wurden mittels einer Spritze 70 mg (0.51 mMol) Phenylmagnesiumchlorid gegeben. Das Reaktionsgemisch wurde 3 Stunden lang unter Rückfluß gekocht, danach auf Raumtemperatur abgekühlt und mit 4 ml 1 N Salzsäure hydrolysiert. Das Gemisch wurde anschließend während 1 Stunde bei 80° C gerührt, dann auf Raumtemperatur abgekühlt und zweimal mit Essigester extrahiert. Die vereinigten organischen Phasen wurden über Natriumsulfat getrocknet und unter vermindertem Druck eingedampft. Der Rückstand wurde an Kieselgel unter Verwendung eines 90:10:0.1-Gemisches von Methylenchlorid, Methanol und Ammoniak als Eluierungsmittel chromatographiert. Das erhaltene Gemisch von (3RS,4RS)-3-[4-(3-Naphthalin-2-ylmethoxy-piperidin-4-yl)-phenyl]-1-phenyl-propan-1-on und (3RS,4RS)-3-{4-[3-(Naphthalin-2-ylmethoxy)-piperidin-4-yl]-phenyl}-propionsäure wurde, in analoger Weise wie in Beispiel 1 (f) beschrieben, in die entsprechenden BOC-Derivate überführt und anschließend an Kieselgel unter Verwendung eines 2:1-Gemisches von Hexan und Essigester als Eluierungsmittel chromatographiert. Es wurden 13 mg (14% d.Th.) (3RS, 4RS)-3-(Naphthalin-2-ylmethoxy)-4-[4-(3-oxo-3-phenyl-propyl)-phenyl]-piperidin-1-carbonsäure tert-butylester als farbloser Schaum erhalten; $R_f$: 0.38 (Hexan:Essigester=2:1).

(b) In analoger Weise wie in Beispiel 22 (l) beschrieben, wurde ausgehend von (3RS,4RS)-3-(Naphthalin-2-ylmethoxy)-4-[4-(3-oxo-3-phenyl-propyl)-phenyl]-piperidin-1-carbonsäure tert-butylester durch Abspaltung der BOC-Gruppe mittels Trifluoressigsäure in Methylenchlorid das (3RS,4RS)-3-[4-(3-Naphthalin-2-ylmethoxypiperidin-4-yl)-phenyl]-1-phenyl-propan-1-on Trifluoracetat als weißes Pulver erhalten; MS : 450 $(M+H)^+$.

Beispiel 41

(a) Eine Lösung von 60 mg (0.13 mMol) (3RS,4RS)-4-(3-Methoxycarbonylphenyl)-3-naphthalin-2-ylmethoxy-piperidin-1-carbonsäure tert-butylester [Beispiel 26 (e)] und 0.26 ml (0.26 mMol) 1 N Natronlauge in 2 ml Methanol wurde 18 Stunden lang bei 30 ° C gerührt. Zur Aufarbeitung wurde das Reaktionsgemisch mit 1 N Salzsäure neutralisiert und zweimal mit je 10 ml Methylenchlorid ausgeschüttelt. Die organischen Phasen wurden vereinigt, über Natriumsulfat getrocknet und unter vermindertem Druck eingedampft. Der Rückstand wurde aus einem Gemisch von Hexan und Diäthyläther kristallisiert. Es wurden 45 mg (75% d.Th.) (3RS,4RS)-3-(1-tert-Butoxycarbonyl-3-naphthalin-2-ylmethoxy-piperidin-4-yl)-benzoesäure als farblose Kristalle erhalten; MS : 462 $(M+H)^+$.

(b) In analoger Weise wie in Beispiel 36 (b) beschrieben, wurde durch Kondensation von (3RS,4RS)-3-(1-tert-Butoxycarbonyl-3-naphthalin-2-ylmethoxy-piperidin-4-yl)-benzoesäure mit Benzylamin unter Verwendung von 1,1-Carbonyldiimidazol als Kondensationsmittel das (3RS,4RS)-4-(3-Benzylcarbamoyl-phenyl)-3-(naphthalin-2-ylmethoxy)-piperidin-1-carbonsäure tert-butylester als farbloses Öl erhalten; MS : 551 $(M+H)^+$.

(c) In analoger Weise wie in Beispiel 22 (l) beschrieben, wurde durch Abspaltung der BOC-Gruppe aus (3RS, 4RS)-4-(3-Benzylcarbamoylphenyl)-3-(naphthalin-2-ylmethoxy)-piperidin-1-carbonsäure tert-butylester das (3RS,4RS)-N-Benzyl-3-(3-naphthalin-2-ylmethoxypiperidin-4-yl)-benzamid Hydrochlorid als weißes Pulver erhalten; MS : 451 $(M+H)^+$.

Beispiel 42

In analoger Weise wie in Beispiel 22 (l) beschrieben, wurden durch Abspaltung der BOC-Gruppe unter Verwendung von Trifluoressigsäure in Methylenchlorid die folgenden Verbindungen erhalten:

1) - Aus (3RS,4RS)-4-(4-Benzylcarbamoyl-phenyl)-3-(naphthalin-2-ylmethoxy)-piperidin-1-carbonsäure tert-butylester das (3RS,4RS)-N-Benzyl-4-[3-(naphthalin-2-ylmethoxy)-piperidin-4-yl]-benzamid als weißes Pulver; MS : 453 $(M+H)^+$;

2) - aus (3RS,4RS)-3-(Naphthalin-2-ylmethoxy)-4-[4-(3-oxo-3-phenylpropylcarbamoyl)-phenyl]-piperidin-1-carbonsäure tert-butylester das (3RS,4RS)-4-[3-(Naphthalin-2-ylmethoxy)-piperidin-4-yl]-N-(3-oxo-3-phenyl-propyl)-benzamid Trifluoracetat als weißes Pulver; MS : 493 $(M+H)^+$;

3) - aus (3RS,4RS)-3-(Naphthalin-2-ylmethoxy)-4-[4-(2-oxo-2-phenyl-äthylcarbamoyl)-phenyl]-piperidin-1-carbonsäure tert-butylester das (3RS,4RS)-4-[3-(Naphthalin-2-ylmethoxy)-piperidin-4-yl]-N-(2-oxo-2-phenyl-äthyl)-benzamid Trifluoracetat als weißes Pulver; MS : 479 $(M+H)^+$.

Die als Ausgangsstoffe eingesetzten BOC-Derivate wurden wie folgt erhalten:

(a) In analoger Weise wie in Beispiel 41 (b) beschrieben, wurde durch alkalische Verseifung von (3RS,4RS)-4-(4-Methoxycarbonyl-phenyl)-3-naphthalin-2-ylmethoxy-piperidin-1-carbonsäure tert-butylester die (3RS,4RS)-4-(1-tert-Butoxycarbonyl-3-naphthalin-2-ylmethoxy-piperidin-4-yl)-benzoesäure als farbloser Festkörper erhalten; MS : 461 $(M)^+$.

(b) In analoger Weise wie in Beispiel 36 (b) beschrieben, wurde durch Kondensation von (3RS,4RS)-4-(1-tert-Butoxycarbonyl-3-naphthalin-2-ylmethoxy-piperidin-4-yl)-benzoesäure mit Benzylamin der (3RS,4RS)-4-(4-Benzylcarbamoyl-phenyl)-3-(naphthalin-2-ylmethoxy)-piperidin-1-carbonsäure tert-butylester als farbloser Schaum erhalten; MS : 551 $(M+H)^+$.

(c) In analoger Weise wie in Beispiel 36 (b) beschrieben, wurde durch Kondensation von (3RS,4RS)-4-(1-tert-Butoxycarbonyl-3-naphthalin-2-ylmethoxy-piperidin-4-yl)-benzoesäure mit 3-Amino-1-phenyl-propan-1-on das (3RS,4RS)-3-(Naphthalin-2-ylmethoxy)-4-[4-(3-oxo-3-phenylpropylcarbamoyl)-phenyl]-piperidin-1-carbonsäure tert-butylester als farbloser Schaum erhalten; MS : 593 $(M+H)^+$.

(d) In analoger Weise wie in Beispiel 36 (b) beschrieben, wurde durch Kondensation von (3RS,4RS)-4-(1-tert-Butoxycarbonyl-3-naphthalin-2-ylmethoxy-piperidin-4-yl)-benzoesäure mit 2-Amino-1-phenyl-äthanon das (3RS,4RS)-3-(Naphthalin-2-ylmethoxy)-4-[4-(2-oxo-2-phenyl-äthylcarbamoyl)-phenyl]-piperidin-1-carbonsäure tert-butylester als farbloser Schaum erhalten; MS : 579 $(M+H)^+$.

Beispiel 43

(a) Eine Lösung von 1.0 g (3 mMol) (3RS,4RS)-3-Hydroxy-4-(4-methoxycarbonyl-phenyl)-piperidin-1-carbonsäure tert-butylester in 100 ml Methanol wurde mit 100 mg Rhodium(5%ig) auf Aluminiumoxid versetzt und während 5 Stunden bei 50° C unter 10 bar Wasserstoff hydriert. Danach wurde das Reaktionsgemisch über 30 g Dicalit filtriert, das Filtrierhilfsmittel mit 200 ml Methanol nachgewaschen und die erhaltene Lösung unter vermindertem Druck eingedampft. Es wurde in quantitativer Ausbeute ein 2:1- oder 1:2-Gemisch des (3RS,4RS)-3-Hydroxy-4-(trans- und -(cis-4-methoxycarbonyl-cyclohexyl)-piperidin-4-carbonsäure tert-butylesters als farbloser Festkörper erhalten; MS : 342 $(M+H)^+$.

In analoger Weise wie in Beispiel 22 (e) und (h)-(l) beschrieben, wurde wie folgt verfahren:

(b) Durch Reduktion mitttels Lithiumborhydrid wurde aus dem 2:1-oder 1:2-Gemisch des (3RS,4RS)-3-Hydroxy-4-(trans- und -(cis-4-methoxycarbonyl-cyclohexyl)-piperidin-4-carbonsäure tert-butylesters ein 2:1 oder 1:2 Gemisch des (3RS,4RS)-cis- und -trans-3-Hydroxy-4-(4-hydroxymethyl-cyclohexyl)-piperidin-1-carbonsäure tert-butylesters als farbloser Schaum erhalten; MS : 314 $(M+H)^+$.

(c) Durch Einführung der Tritylgruppe wurde aus dem 2:1- oder 1:2-Gemisch des (3RS,4RS)-cis- und -trans-3-Hydroxy-4-(4-hydroxymethylcyclohexyl)-piperidin-1-carbonsäure tert-butylesters das 2:1- oder 1:2-Gemisch des (3RS,4RS)-cis- und -trans-3-Hydroxy-4-(4-trityloxymethylcyclohexyl)-piperidin-1-carbonsäure tert-butylesters als

farbloser Schaum erhalten; MS : 573 $(M+H)^+$.

(d) Die Alkylierung mit 2-Brommethylnaphthalin ergab, ausgehend vom 2:1- oder 1:2- Gemisch des (3RS,4RS)-cis- und -trans-3-Hydroxy-4-(4-trityloxymethyl-cyclohexyl)-piperidin-1-carbonsäure tert-butylesters, das 2:1- oder 1:2- Gemisch des (3RS,4RS)-cis- und -trans-3-Naphthalin-2-ylmethoxy-4-(4-trityloxymethyl-cyclohexyl)-piperidin-1-carbonsäure tert-butylesters als farblosen Schaum: MS : 713 $(M+NH_4)^+$.

(e) Aus dem 2:1- oder 1:2- Gemisch des (3RS,4RS)-cis- und -trans-3-Naphthalin-2-ylmethoxy-4-(4-trityloxymethyl-cyclohexyl)-piperidin-1-carbonsäure tert-butylesters wurde durch Abspaltung der Trityl-Gruppe mittels Chlorwasserstoff in Methanol das 2:1- oder 1:2-Gemisch des (3RS,4RS)-cis- und -trans-3-Naphthalin-2-ylmethoxy-4-(4-hydroxymethyl-cyclohexyl)-piperidin-1-carbonsäure tert-butylesters als farbloser Schaum erhalten; MS : 453 $(M+H)^+$.

(f) Die Acylierung des 2:1- oder 1:2- Gemisches des (3RS,4RS)-cis- und -trans-3-Naphthalin-2-ylmethoxy-4-(4-hydroxymethyl-cyclohexyl)-piperidin-1-carbonsäure tert-butylesters mit Benzoylchlorid lieferte das 2:1- oder 1:2- Gemisch des (3RS,4RS)-cis- und -trans-4-(4-Benzoyloxymethyl-cyclohexyl)-3-naphthalin-2-ylmethoxy-piperidin-1-carbonsäure tert-butylesters als farbloses Öl; MS : 558 $(M+H)^+$.

(g) Durch Abspaltung der BOC-Gruppe mittels Chlorwasserstoff in Methanol wurde aus dem 2:1- oder 1:2- Gemisch des (3RS,4RS)-cis- und -trans-4-(4-Benzoyloxymethyl-cyclohexyl)-3-naphthalin-2-ylmethoxypiperidin-1-carbonsäure tert-butylesters das 2:1- oder 1:2- Gemisch des Benzoesäure (3RS,4RS)-cis-und-trans 4-(3-Naphthalin-2-ylmethoxypiperidin-4-yl)-cyclohexylmethylesters als farbloser Schaum erhalten; MS : 458 $(M+H)^+$.

Beispiel 44

In analoger Weise wie in Beispiel 22 (a)-(c) beschrieben, wurde wie folgt verfahren:

(a) Aus 1-Benzyl-4-piperidon und 4-Jodanisol wurde das 1-Benzyl-4-(4-methoxy-phenyl)-piperidin-4-ol als farbloser Festkörper erhalten; MS : 298 $(M+H)^+$.

(b) Aus 1-Benzyl-4-(4-methoxy-phenyl)-piperidin-4-ol wurde durch Elimination das 1-Benzyl-4-(4-methoxy-phenyl)-1,2,3,6-tetrahydropyridin als beiger Festkörper erhalten; MS : 280 $(M+H)^+$ .

(c) Die Hydroborierung von 1-Benzyl-4-(4-methoxy-phenyl)-1,2,3,6-tetrahydro-pyridin ergab das (3RS,4RS)-1-Benzyl-4-(4-methoxy-phenyl)-piperidin-3-ol als farblose Kristalle; MS : 297 $(M)^+$.

(d) Zu einer Lösung von 7.38 g (24.82 mMol) (3RS,4RS)-1-Benzyl-4-(4-methoxy-phenyl)-piperidin-3-ol in 248 ml Methylenchlorid wurden innerhalb von 10 Minuten bei 3-7° C 49.6 ml (49.6 mMol, 2 eq) einer ca. 1 M Bortribromid-Lösung in Methylenchlorid getropft. Diese Suspension wurde 3 Stunden lang bei Raumtemperatur gerührt. Anschließend wurde das Reaktionsgemisch auf 750 ml einer Eis/Wasser-Mischung gegossen, mit 2 N Natronlauge auf pH 8 gebracht und dreimal mit je 500 ml Methylenchlorid extrahiert. Die organischen Phasen wurden mit wenig Wasser gewaschen, über Magnesiumsulfat getrocknet, unter vermindertem Druck eingedampft und am Hochvakuum getrocknet. Dies lieferte 6.42 g (3RS,4RS)-1-Benzyl-4-(4-hydroxy-phenyl)-piperidin-3-ol in Form eines weißen Schaumes; MS : 283 $(M)^+$.

(e) Eine Lösung von 3.0 g, (10.6 mMol) (3RS,4RS)-1-Benzyl-4-(4-hydroxyphenyl)-piperidin-3-ol in 75 ml Äthylmethylketon wurde nacheinander mit 10.84 g (42.4 mMol, 4.8 eq) rac.-2-(2-Iodo-äthoxy)-tetrahydro-pyran und 5.25 g (53 mMol, 5 eq) Kaliumcarbonat versetzt. Dieses Gemisch wurde 25 Stunden lang bei 95° C gerührt. Anschließend wurde auf wenige Milliliter eingeengt, auf 200 ml einer Eis/Wasser-Mischung gegossen und dreimal mit je 300 ml Methylenchlorid extrahiert. Die vereinigten organischen Phasen wurden einmal mit wenig Wasser gewaschen, über Magnesiumsulfat getrocknet, unter vermindertem Druck eingedampft und am Hochvakuum getrocknet. Das so erhaltene Rohprodukt (11.81 g) wurde an Kieselgel mittels eines 99:1-Gemisches von Methylenchlorid und Methanol als Eluierungsmittel aufgetrennt und lieferte 3.2 g (73 % d.Th.) das Gemisch des (3RS,4RS)-1-Benzyl-4-[4-[2-[(RS)- und -[(SR)-tetrahydro-pyran-2-yloxy]-äthoxy]-phenyl]-piperidin-3-ols als farbloses Öl; $R_f$: 0.55 ($SiO_2$, Methylenchlorid: Methanol=9:1).

(f) In analoger Weise wie in Beispiel 22 (i) beschrieben, wurde durch Alkylierung des Gemisches des (3RS,4RS)-1-Benzyl-4-[4-[2-[(RS)- und - [(SR)-tetrahydro-pyran-2-yloxy]-äthoxy]-phenyl]-piperidin-3-ols mit 2-Brommethylnaphthalin das Gemisch des (3RS,4RS)-1-Benzyl-3-(naphthalin-2-ylmethoxy)-4-[4-[2-[(RS)- und -[(SR)-tetrahydro-pyran-2-yloxy]-äthoxy]-phenyl]-piperidins als farbloses Öl erhalten; $R_f$: 0.46 ($SiO_2$, Methylenchlorid:Essigester=1:1).

(g) In analoger Weise wie in Beispiel 25 (a) beschrieben, wurde durch Abspaltung der Benzylgruppe mittels Chlorameisensäure-2,2,2-trichloräthylester und Kaliumcarbonat das Gemisch des (3RS,4RS)-3-(Naphthalin-2-ylmethoxy)-4-[4-[2-[(RS)- und - [(SR)-tetrahydro-pyran-2-yloxy]-äthoxy]-phenyl]-piperidin-1-carbonsäure 2,2,2-trichloräthylesters als farbloses Öl erhalten; MS : 653.3, 655 $(M+H)^+$ .

(h) Eine Lösung von 500 mg (0.785 mMol) des Gemisches des (3RS,4RS)-3-(Naphthalin-2-ylmethoxy)-4-[4-[2-[(RS)- und - [(SR)-tetrahydro-pyran-2-yloxy]-äthoxy]-phenyl]-piperidin-1-carbonsäure 2,2,2-trichlor-äthylesters in 10 ml Methanol wurde mit 1 ml Wasser und 1.120 g (5.888 mMol) p-Toluolsulfonsäure Monohydrat versetzt. Diese Suspension wurde für 1.5 Stunden bei Raumtemperatur gerührt, dann unter vermindertem Druck auf die Hälfte des Volumens eingeengt und viermal mit Methylenchlorid gegen Wasser extrahiert. Die organischen Phasen wurden je einmal mit gesättigter Natriumhydrogencarbonat-Lösung und gesättigter Natriumchlorid-Lösung gewaschen, über Magnesiumsulfat getrocknet, unter vermindertem Druck eingedampft und am Hochvakuum getrocknet. Das Rohprodukt (465 mg) wurde an Kieselgel mittels eines 9:1-Gemisches von Methylenchlorid und Methanol als Eluierungsmittel aufgetrennt. Dies lieferte 344 mg (79 % d.Th.) (3RS,4RS)-4-[4-(2-Hydroxy-äthoxy)-phenyl]-3-(naphthalin-2-ylmethoxy)-piperidin-1-carbonsäure 2,2,2-trichloräthylester als farbloses, amorphes Pulver; MS : 569.3, 571 $(M+NH_4)^+$ .

(i) In analoger Weise wie in Beispiel 24 (m) beschrieben, wurde aus (3RS,4RS)-4-[4-(2-Hydroxy-äthoxy)-phenyl]-3-(naphthalin-2-ylmethoxy)-piperidin-1-carbonsäure 2,2,2-trichloräthylester und Pyridin-2-carbonylazid der (3RS,4RS)-3-(Naphthalin-2-ylmethoxy)-4-[4-(2-pyridin-2-ylcarbamoyloxy-äthoxy]-phenyl]-piperidin-1-carbonsäure 2,2,2-trichlor-äthylester als farbloses Öl erhalten; MS : 672.2, 674 $(M+H)^+$.

(j) In analoger Weise wie in Beispiel 25 (b) beschrieben, wurde durch Behandlung von (3RS,4RS)-3-(Naphthalin-2-ylmethoxy)-4-[4-(2-pyridin-2-ylcarbamoyloxy-äthoxy]-phenyl]-piperidin-1-carbonsäure 2,2,2-trichlor-äthylester mit Zink in Eisessig der Pyridin-2-yl-carbaminsäure (3RS,4RS)- 2-[4-[3-(naphthalin-2-ylmethoxy)-piperidin-4-yl]-phenoxy]-äthylester als farbloser Festkörper erhalten; MS : 498 (M+H)+.

Beispiel 45

In analoger Weise wie in Beispiel25 (b) beschrieben, wurden die folgenden Verbindungen erhalten:

1) - Aus (3RS,4RS)-4-[4-(2-Carbamoyloxy-äthoxy)-phenyl]-3-(naphthalin-2-ylmethoxy)-piperidin-1-carbonsäure 2,2,2-trichlor-äthylester der Carbaminsäure (3RS,4RS)- 2-[4-[3-(Naphthalin-2-ylmethoxy)-piperidin-4-yl]-phenoxy]-äthylester als farbloses Öl; MS : 421 $(M+H)^+$;

2) - aus (3RS,4RS)-4-[4-[2-(Morpholin-4-ylcarbonyloxy)-äthoxy]-phenyl]-3-(naphthalin-2-ylmethoxy)-piperidin-1-carbonsäure 2,2,2-trichlor-äthylester der Morpholin-4-carbonsäure (3RS,4RS)-2-[4-[3-(Naphthalin-2-ylmethoxy)-piperidin-4-yl]-phenoxy]-äthylester als farbloses Öl; MS : 491 $(M+H)^+$ ;

3) - aus (3RS,4RS)-4-(4-Methoxy-phenyl)-3-(naphthalin-2-ylmethoxy)-piperidin-1-carbonsäure 2,2,2-trichlor-äthylester das (3RS,4RS)-4-(4-Methoxy-phenyl)-3-(naphthalin-2-ylmethoxy)-piperidin als farbloses Öl; MS : 348 $(M+H)^+$ ;

4) - aus dem Gemisch des (3RS,4RS)-3-(Naphthalin-2-ylmethoxy)-4-[4-[2-[(RS)- und - [(SR)-tetrahydro-pyran-2-yloxy]-äthoxy]-phenyl]-piperidin-1-carbonsäure 2,2,2-trichlor-äthylesters unter gleichzeitiger Abspaltung der THP-Gruppe das (3RS,4RS)-2-[4-[3-(Naphthalin-2-ylmethoxy)-piperidin-4-yl]-phenoxy]-äthanol als farbloser, amorpher Festkörper; MS : 378 $(M+H)^+$.

Die als Ausgangsstoffe eingesetzten Derivate wurden wie folgt erhalten:

(a) In analoger Weise wie in Beispiel 24 (m) beschrieben, wurde aus (3RS,4RS)-4-[4-(2-Hydroxy-äthoxy)-phenyl]-3-(naphthalin-2-ylmethoxy)-piperidin-1-carbonsäure 2,2,2-trichloräthylester und Natriumisocyanat der (3RS,4RS)-4-[4-(2-Carbamoyloxy-äthoxy)-phenyl]-3-(naphthalin-2-ylmethoxy)-piperidin-1-carbonsäure 2,2,2-trichloräthylester als farbloses Öl erhalten; $R_f$: 0.28 ($SiO_2$, Methylenchlorid:Aceton=1:1).

(b) Eine Lösung von 250 mg (0.56 mMol) (3RS,4RS)-4-[4-(2-Hydroxy-äthoxy)-phenyl]-3-(naphthalin-2-ylmethoxy)-piperidin-1-carbonsäure 2,2,2-trichloräthylester in 20 ml Toluol wurde nacheinander mit 250 ml (2.8 mMol, 5.0 eq) Morpholin-4-carbonylchlorid, 375 mg (3.08 mMol, 5.5 eq) 4-Dimethylaminopyridin und 20µl (0.075 mMol, 0.13 eq) Dibutylzinndiacetat versetzt. Dieses Gemisch wurde für 64 Stunden unter Rückfluß gekocht. Im Verlaufe der Reaktion wurden nochmals 125 ml (1.40 mMol, 5.0 eq) Morpholin-4-carbonylchlorid und 188 mg (1.56 mMol, 2.3 eq) 4-Dimethylaminopyridin zugegeben und für weitere 24 Stunden unter Rückfluß gekocht. Das auf Raumtemperatur abgekühlte Reaktionsgemisch wurde auf 100 ml einer Eis/Wasser-Mischung gegossen, 5 Minuten lang gerührt und dreimal mit Methylenchlorid extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das Rohprodukt (546 mg) wurde an Kieselgel unter Verwendung eines 9:1-Gemisches von Hexan und Aceton als Eluierungsmittel aufgetrennt. Es wurden 42 mg (14 % d.Th.) (3RS,4RS)-4-[4-[2-(Morpholin-4-ylcarbonyloxy)-äthoxy]-phenyl]-3-(naphthalin-2-ylmethoxy)-piperidin-1-carbonsäure 2,2,2-trichlor-äthylester als farbloses Öl erhalten; MS : 665.3, 667 $(M+H)^+$ .

(c) In analoger Weise wie im Beispiel 12 (b) beschrieben, wurde durch die Alkylierung des (3RS,4RS)-1-Benzyl-4-(4-methoxy-phenyl)-piperidin-3-ol [Beispiel 44 (c)] mit 2-Brommethylnaphthalin das (3RS,4RS)-1-Benzyl-4-(4-methoxy-phenyl)-3-(naphthalin-2-ylmethoxy)-piperidin als beigefarbener Festkörper erhalten; MS : 437 $(M)^+$. Die anschließende Umsetzung mit Chlorameisensäure 2,2,2-trichloräthylester ergab den (3RS,4RS)-4-(4-Methoxy-phenyl)-3-(naphthalin-2-ylmethoxy)-piperidin-1-carbonsäure 2,2,2-trichlor-äthylester als gelbliches Öl; MS : 539, 541M+$NH_4)^+$.

<u>Beispiel 46</u>

(a) In analoger Weise wie in Beispiel 2 (e) beschrieben, wurde durch hydrogenolytische Abspaltung der Benzylgruppe aus (3RS,4RS)-1-Benzyl-4-(4-hydroxy-phenyl)-piperidin-3-ol [Beispiel 44 (d)] das (3RS,4RS)-4-(4-Hydroxy-phenyl)-piperidin-3-ol als farbloser Festkörper erhalten; MS : 194 $(M+H)^+$ .

(b) In analoger Weise wie in Beispiel 22 (g) beschrieben wurde aus 4-(4-Hydroxy-phenyl)-piperidin-3-ol durch Einführung der BOC-Gruppe der (3RS,4RS)-3-Hydroxy-4-(4-hydroxy-phenyl)-piperidin-1-carbonsäure tert-butylester als farbloser Schaum erhalten; MS : 237 $(M-C4H_8)^+$ .

(c) Ein Gemisch aus 4.5 g (15.3 mMol) (3RS,4RS)-3-Hydroxy-4-(4-hydroxy-phenyl)-piperidin-1-carbonsäure tert-butylester, 20.8 g (91.8 mMol, 6.0 eq) 2-(3-Chlorpropyl)-2-phenyl-[1,3]dioxolan [J. Med. Chem. 34, 12 (1991)], 14.6 g (105.5 mMol, 6.9 eq) Kaliumcarbonat und 2.0 g (0.012 mMol, 0.078 eq.) Kaliumjodid in 50 ml Äthyl-methylketon wurde in einem verschlossenen, drucksicheren Gefäß bei einer Badtemperatur von 100° C 60 Stunden lang gerührt. Das Reaktionsgemisch wurde auf eine Eis/Wasser-Mischung gegossen und dreimal mit Essigester extrahiert. Die organischen Phasen wurden je weils einmal mit Wasser und gesättigter Natriumchlorid-Lösung gewaschen, über Magnesiumsulfat getrocknet, unter vermindertem Druck eingeengt und am Hochvakuum getrocknet. Das gelbe Öl (25.72 g) wurde an Kieselgel mittels eines Laufmittelgradienten von 4:1 bis 1:1 eines Gemisches von Hexan und Essigester als Eluierungsmittel aufgetrennt. Dies lieferte 5.34 g (72 % d.Th.) weißen, kristallinen (3RS,4RS)-3-Hydroxy-4-[4-[3-(2-phenyl-[1,3] dioxolan-2-yl)-propoxy]-phenyl]-piperidin-1-carbonsäure tert-butylester; MS : 484 $(M+H)^+$.

(d) In analoger Weise wie in Beispiel 22 (i) beschrieben, wurde durch Alkylierung des (3RS,4RS)-3-Hydroxy-4-[4-[3-(2-phenyl-[1,3]dioxolan-2-yl)-propoxy]-phenyl]-piperidin-1-carbonsäure tert-butylesters mit 2-Brommethylnaphthalin der (3RS,4RS)-3-(Naphthalin-2-ylmethoxy)-4-[4-[3-(2-phenyl-[1,3]dioxolan-2-yl)-propoxy]-phenyl]-piperidin-1-carbonsäure tert-butylester als weiße Kristalle erhalten; MS : 624 $(M+H)^+$ .

(e) Eine Lösung aus 193 mg ( 0.31 mMol) (3RS,4RS)-3-(Naphthalin-2-ylmethoxy)-4-[4-[3-(2-phenyl-[1,3]dioxolan-2-yl)-propoxy)-phenyl)-piperidin-1-carbonsäure tert-butylester in 3 ml Tetrahydrofuran und 3 ml 2 N Salzsäure wurde 12 Stunden lang bei Raumtemperatur und 24 Stunden lang bei 50° C gerührt. Anschließend wurde das Reaktionsgemisch auf ein 1:1-Gemisch von Wasser und gesättigter Natriumhydrogencarbonat-Lösung gegossen und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingeengt und am Hochvakuum getrocknet. Das farblose Öl (165 mg) wurde an Kieselgel mittels eines 9:1-Gemisches von Methylenchlorid und Methanol (Extr. gegen 5 Vol-% conc. $NH_4OH$) aufgetrennt. Dies lieferte 127.4 mg (82 % d.Th.) (3RS,4RS)-4-[4-(3-Naphthalin-2-ylmethoxypiperidin-4-yl)-phenoxy]-1-phenyl-butan-1-an in Form eines farblosen Öles; MS : 502 (M+Na)+.

Beispiel 47

In analoger Weise wie in Beispiel 46 (d)-(e) bzw. 3 (c)-(e) beschrieben, wurden die folgenden Verbindungen erhalten:

1) - Aus (3RS,4RS)-3-Hydroxy-4-[4-[3-(2-phenyl-[1,3]dioxolan-2-yl)-propoxy]-phenyl]-piperidin-1-carbonsäure tert-butylester wurde durch Alkylierung mit 5-Brommethyl-benzo[b]thiophen der (3RS,4RS)-3-(Benzo[b]thiophen-5-ylmethoxy)-4-{4-[3-(2-phenyl-[1,3]dioxolan-2-yl)-propoxy]-phenyl}-piperidin-1-carbonsäure tert-butylester, MS : 630 $(M+H)^+$, als schwach gelbes Harz erhalten. Die folgende Abspaltung der BOC- und Acetalgruppe lieferte das (3RS,4RS)-4-[4-[3-(Benzo[b)thiophen-5-ylmethoxy)-piperidin-4-yl]-phenoxy]-1-phenylbutan-1-on als farbloses Harz; MS : 486 $(M+H)^+$.

2) - Aus (3RS,4RS)-3-Hydroxy-4-[4-[3-(2-phenyl-[1,3]dioxolan-2-yl)-propoxy]-phenyl)-piperidin-1-carbonsäure tert-butylester wurde durch Alkylierung mit 5-(Chlormethyl)indan der (3RS,4RS)-3-(Indan-5-ylmethoxy)-4-{4-[3-(2-phenyl-[1,3]dioxolan-2-yl)-propoxy]-phenyl}piperidin-1-carbonsäure tert-butylester, MS : 614 $(M+H)^+$, als schwach gelbes Harz erhalten. Die folgende Abspaltung der BOC- und Acetalgruppe lieferte das (3RS,4RS)-4-[4-[3-Indan-5-ylmethoxy)-piperidin-4-yl]-phenoxy]-1-phenyl-butan-1-an als farbloses Harz; MS : 470 $(M+H)^+$ .

3) - Aus (3RS,4RS)-3-Hydroxy-4-[4-[3-(2-phenyl-[1,3]dioxolan-2-yl)-propoxy]-phenyl]-piperidin-1-carbonsäure tert-butylester wurde durch Alkylierung mit 3-Chlormethyl-1-(2-trimethylsilanyl-äthöxymethoxy)-naphthalin der (3RS,4RS)-4-[4-[3-(2-Phenyl-[1,3]dioxolan-2-yl)-propoxy]phenyl]-3-[4-(2-trimethylsilanyl-äthoxymethoxy)-naphthalin-2-ylmethoxy]-piperidin-1-carbonsäure tert-butylester, MS : 770 $(M+H)^+$, als farbloses Harz erhalten. Die folgende Abspaltung der BOC- und der beiden Acetalgruppen lieferte das (3RS,4RS)-4-[4-[3-(4-Hydroxynaphthalin-2-ylmethoxy)-piperidin-4-yl]-phenoxy]-1-phenyl-butan-1-on als farbloses Harz; $R_f$: 0.17 ($SiO_2$, Methylenchlorid: Methanol=9:1, extrahiert gegen 5%igen Ammoniak).

4) - Aus dem (3RS,4RS)-3-Hydroxy-4-(4-hydroxy-phenyl)-piperidin-1-carbonsäure tert-butylester wurde durch Alkylierung mit 1-Brom-4-(2-brom-äthyl)-benzol, analog Beispiel 46 (c), der (3RS,4RS)-4-[4-[2-(4-Bromphenoxy)-äthoxy]-phenyl]-3-hydroxy-piperidin-1-carbonsäure tert-butylester als farbloses Harz erhalten. Die weitere Alkylierung mit 2-Brommethylnaphthalin ergab den (3RS,4RS)-4-[4-[2-(4-Brom-phenoxy)-äthoxy]-phenyl]-3-(naphthalin-2-ylmethoxy)-piperidin-1-carbonsäure tert-butylester, aus dem durch Abspaltung der BOC-Gruppe das (3RS, 4RS)-4-[4-[2-(4-Brom-phenoxy)-äthoxy]-phenyl]-3-(naphthalin-2-ylmethoxy)-piperidin als farbloses Harz erhalten wurde; MS : 532, 534M+H)$^+$.

5) - Aus dem (3RS,4RS)-3-Hydroxy-4-(4-hydroxy-phenyl)-piperidin-1-carbonsäure tert-butylester wurde durch Alkylierung mit 2-Brommethyl-5-phenyl-[1,3,4]oxadiazol, analog Beispiel 44 (e), der (3RS,4RS)-3-Hydroxy-4-[4-(5-phenyl-[1,3,4] oxadiazol-2-ylmethoxy)-phenyl]-piperidin-1-carbonsäure tert-butylester als gelblicher, amorpher Festkörper erhalten. Die weitere Alkylierung mit 2-Brommethylnaphthalin ergab den (3RS,4RS)-3-(Naphthalin-2-ylmethoxy)-4-[4-(5-phenyl-[1,3,4]oxadiazol-2-ylmethoxy)-phenyl]-piperidin-1-carbonsäure tert-butylester, aus dem durch Abspaltung der BOC-Gruppe das (3RS,4RS)-3-(Naphthalin-2-ylmethoxy)-4-[4-(5-phenyl-[1,3,4] oxadiazol-2-ylmethoxy)-phenyl]piperidin als farbloser Festkörper erhalten wurde; MS : 492 $(M+H)^+$.

6) - Aus dem (3RS,4RS)-3-Hydroxy-4-(4-hydroxy-phenyl)-piperidin-1-carbonsäure tert-butylester wurde durch Alkylierung mit β-Bromphenethol, analog Beispiel 46 (c), der (3RS,4RS)-3-Hydroxy-4-[4-(2-phenoxy-äthoxy)-phenyl]-piperidin-1-carbonsäure tert-butylester erhalten; MS : 414 $(M+H)^+$. Die weitere Alkylierung mit 2-Brommethylnaphthalin ergab den (3RS,4RS)-3-(Naphthalin-2-ylmethoxy)-4-[4-(2-phenoxy-äthoxy)-phenyl]-piperidin-1-carbonsäure tert-butyl ester , MS : 554 $(M+H)^+$, aus dem durch Abspaltung der BOC-Gruppe das (3RS,4RS)-3-(Naphthalin-2-ylmethoxy)-4-[4-(2-phenoxy-äthoxy)-phenyl]-piperidin erhalten wurde; MS : 454 $(M+H)^+$.

7) - Aus (3RS,4RS)-3-Hydroxy-4-[4-(2-phenoxy-äthoxy)-phenyl]-piperidin-1-carbonsäure tert-butylester wurde durch Alkylierung mit 3-Chlormethyl-1-(2-trimethylsilanyl-äthoxymethoxy)-naphthalin der (3RS,4RS)-4-[4-(2-Phenoxy-äthoxy)-phenyl]-3-[4-(2-trimethylsilanyl-äthoxymethoxy)-naphthalin-2-ylmethoxy]-piperidin-1-carbonsäure tert-butylester erhalten; MS : 700 $(M+H)^+$. Die folgende Abspaltung der BOCund Acetalgruppe lieferte das (3RS, 4RS)-3-{4-[4-(2-Phenoxy-äthoxy)-phenyl]-piperidin-3-yloxymethyl}-naphthalin-1-ol; MS : 470 $(M+H)^+$.

Beispiel 48

Zu einer Lösung von 44 mg (0.074 mMol) (3RS,4RS)-4-[4-[3-(4-Hydroxy-naphthalin-2-ylmethoxy)-piperidin-4-yl]-phenoxy]-1-phenylbutan-1-on in 1.5 ml Dioxan wurde mittels einer Spritze eine Lösung von 18.7 mg (0.494 mMol, 6.7 eq) Natriumborhydrid in 0.35 ml Wasser gegeben. Dieses Gemisch wurde 1.5 Stunden lang bei Raumtemperatur gerührt. Anschließend wurde die Reaktionslösung mit der gleichen Volumenmenge Eiswasser versetzt und das Gemisch mit 2 N Salzsäure auf pH 1 gebracht. Nach 5 - 10 Minuten Rühren bei Raumtemperatur wurde mit konzentriertem Ammoniak auf pH 9 eingestellt und die wäßrige Lösung dreimal mit je einem Volumenäquivalent Methylenchlorid extrahiert. Die vereinigten organischen Phasen wurden über Magnesiumsulfat getrocknet, unter vermindertem Druck bei 45 °C eingeengt und am Hochvakuum getrocknet.Der bräunliche Festkörper ( 40.1 mg) wurde an Kieselgel mittels eines 9:1-Gemisches von Methylenchlorid und Methanol (Extr. gegen 5 Vol-% conc. $NH_3$ aq) als Eluierungsmittel aufgetrennt. Dies lieferte 13 mg (35 % d.Th.) (3RS,4RS)-3-[4-[4-(4-Hydroxy-4-phenyl-butoxy)-phenyl]-piperidin-4-yloxymethyl]-naphthalin-1-ol (Konfiguration im Butanol-Teil unbekannt) als farbloses Öl; MS : 498 $(M+H)^+$.

Beispiel 49

In analoger Weise wie in Beispiel 48 beschrieben, wurden durch Reduktion der Ketone die folgenden Alkohole erhalten:

1) - Aus (3RS,4RS)-4-[4-[3-Indan-5-ylmethoxy)-piperidin-4-yl]-phenoxy]-1-phenyl-butan-1-on das 2:1 oder 1:2 Gemisch des (RS)- und (SR)-4-[4-[(3RS,4RS)-3-(Indan-5-ylmethoxy)-piperidin-4-yl]-phenoxy]-1-phenyl-butan-1-ols als farbloses Harz; MS : 472 $(M+H)^+$;

2) - aus (3RS,4RS)-4-[4-[3-(Benzo[b]thiophen-5-ylmethoxy)-piperidin-4-yl]-phenoxy]-1-phenyl-butan-1-on das Gemisch des (RS)- and (SR)-4-[4-[(3RS,4RS)-3-(Benzo[b]thiopen-5-ylmethoxy)-piperidin-4-yl]-phenoxy]-1-phenyl-butan-1-ols als farbloses Harz; MS : 488 $(M+H))^+$;

3) - aus (3RS,4RS)-4-[4-(3-Naphthalin-2-ylmethoxy-piperidin-4-yl)-phenoxy]-1-phenyl-butan-1-on das 4-[4-[(3RS,4RS)-3-Naphthalin-2-ylmethoxy-piperidin-4-yl]-phenoxy]-1-phenyl-butan-1-ol (Konfiguration im Butanol-Teil unbekannt) als farbloses Harz; MS : 482 $(M+H)^+$.

Beispiel 50

(a) In analoger Weise wie in Beispiel 22 (g) beschrieben, wurde durch Einführung der BOC-Gruppe aus (3RS,4RS)-4-[4-(3-Naphthalin-2-ylmethoxy-piperidin-4-yl)-phenoxy]-1-phenyl-butan-1-on der (3RS,4RS)-3-(Naphthalin-2-ylmethoxy)-4-[4-(4-oxo-4-phenyl-butoxy)-phenyl]-piperidin-1-carbonsäure tert-butylester als farbloser Festkörper erhalten; MS: 580 $(M+H)^+$.

(b) Die Reduktion des (3RS,4RS)-3-(Naphthalin-2-ylmethoxy)-4-[4-(4-oxo-4-phenyl-butoxy)-phenyl]-piperidin-1-carbonsäure tert-butylesters, analog Beispiel 48, lieferte den (3RS,4RS)-4-[4-(4-Hydroxy-4-phenylbutoxy)-phenyl]-3-naphthalin-2-ylmethoxy-piperidin-1-carbonsäure tert-butylester (Konfiguration im Butoxy-Teil unbekannt) als farblosen Festkörper; MS : 582 $(M+H)^+$.

(c) Die Alkylierung des (3RS,4RS)-4-[4-(4-Hydroxy-4-phenyl-butoxy)-phenyl]-3-naphthalin-2-ylmethoxy-piperidin-1-carbonsäure tert-butylesters mit Methyljodid, analog Beispiel 22 (i), ergab den (3RS,4RS)-4-[4-(4-Methoxy-4-phenyl-butoxy)-phenyl]-3-(naphthalin-2-ylmethoxy)-piperidin-1-carbonsäure tert-butylester (Konfiguration im Butoxy-Teil unbekannt) als farbloses Öl; $R_f$: 0.61 ($SiO_2$, Hexan:Essigester=2:1).

(d) In analoger Weise wie in Beispiel 22 (1) beschrieben, wurde durch Abspaltung der BOC-Gruppe aus (3RS,4RS)-4-[4-(4-Methoxy-4-phenylbutoxy)-phenyl]-3-(naphthalin-2-ylmethoxy)-piperidin-1-carbonsäure tert-butylester das (3RS,4RS)-4-[4-(4-Methoxy-4-phenyl-butoxy)-phenyl]-3-(naphthalin-2-ylmethoxy)-piperidin (Konfiguration im Butoxy-Teil unbekannt) als farbloses Harz erhalten; MS : 496 $(M+H)^+$.

Beispiel 51

Eine Lösung aus 30 mg (0.052 mMol) (3RS,4RS)-4-[4-(4-Hydroxy-4-phenyl-butoxy)-phenyl]-3-naphthalin-2-ylmethoxy-piperidin-1-carbonsäure tert-butylester (Konfiguration im Butoxy-Teil unbekannt), 7 25.5 µl (0.186 mMol, 3.6 eq) Triäthylamin und 1.7 ml Methylenchlorid wurde nacheinander mit 21.6 µl (0.186 mMol, 3.6 eq) Benzoylchlorid und 2 mg (0.016 mMol) 4-Dimethylaminopyridin versetzt. Diese Reaktionslösung wurde 10 Stunden lang bei Raumtemperatur gerührt, dann auf 5 ml Wasser gegossen und dreimal mit je 10 ml Essigester extrahiert. Die vereinigten organischen Phasen wurden über Magnesiumsulfat getrocknet, unter vermindertem Druck eingedampft und am Hochvakuum getrocknet. Das schwach gelbe Harz (62 mg) wurde in 1 ml Methanol gelöst, erneut eingeengt, am Hochvakuum getrocknet. Das erhaltene Rohprodukt wurde ohne weitere Reinigung und Charakterisierung, analog zu dem in Beispiel 22 (l) beschriebenen Verfahren, mit Chlorwasserstoff in Methanol umgesetzt. Das braungelbe Harz (56 mg) wurde an Kieselgel mittels eines 95:5-Gemisches von Methylenchlorid und Methanol (extrahiert gegen 5 Vol-% konz. Ammoniak) als Eluierungsmittel aufgetrennt. Es wurden 15 mg (50 % d.Th.) Benzoesäure 4-[4-[(3RS,4RS)-3-Naphthalin-2-ylmethoxypiperidin-4-yl]-phenoxy]-1-phenyl-butylester (Konfiguration im Butoxy-Teil unbekannt) als farbloses Harz erhalten; MS : 586 $(M+H)^+$.

Beispiel 52

Ein Gemisch aus 600 mg (0.962 mMol) (3RS,4RS)-3-(Naphthalin-2-ylmethoxy)-4-{4-[3-(2-phenyl-[1,3]dioxolan-2-yl)-propoxy]-phenyl}-piperidin-1-carbonsäure tert-butylester in 3.0 ml Methylenchlorid und 433 mg (1.92 mMol, 2.0 eq) Zinkbromid wurde 4 Stunden lang bei Raumtemperatur gerührt. Das Reaktionsgemisch wurde auf Wasser gegossen und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das amorphe, schwach gelbe Rohprodukt wurde an Kieselgel mittels eines 95:5:0.1-Gemisches von Methylenchlorid, Methanol und Ammoniak aufgetrennt. Es wurden 355 mg (71 % d.Th.) (3RS,4RS)-3-(Naphthalin-2-ylmethoxy)-4-[4-[3-(2-phenyl-[1,3]dioxolan-2-yl)-propoxy]-phenyl]-piperidin als farbloses Harz erhalten; MS : 524 $(M+H)^+$.

Beispiel 53

(a) In analoger Weise wie in Beispiel 44 (e)-(f) beschrieben, wurde durch Alkylierung des (3RS,4RS)-3-Hydroxy-4-(4-hydroxy-phenyl)-piperidin-1-carbonsäure tert-butylesters mit 2-(2-Iodo-äthoxy)-tetrahydro-pyran ein Gemisch des (3RS,4RS)-3-Hydroxy-4-[4-[2-[(RS)- und -[(SR)-tetrahydropyran-2-yloxy)-äthoxy]-phenyl]-piperidin-1-carbonsäure tert-butylesters als gelbes Öl erhalten; $R_f$: 0.45 ($SiO_2$, Hexan:Essigester=1:1).

(b) In analoger Weise wie in Beispiel 22 (i) beschrieben, wurde durch Alkylierung des Gemisches des (3RS,4RS)-3-Hydroxy-4-[4-[2-[(RS)- und -[(SR)-tetrahydro-pyran-2-yloxy]-äthoxy]-phenyl]-piperidin-1-carbonsäure tert-butylesters mit 2-Brommethylnaphthalin ein Gemisch des (3RS,4RS)-3-(Naphthalin-2-ylmethoxy)-4- [4-[2- [(RS)- und -[(SR)-tetrahydro-pyran-2-yloxy]-äthoxy]-phenyl]-piperidin-1-carbonsäure tert-butylesters als farbloser Festkörper erhalten; MS : 579 $(M+H)^+$

(c) Eine Lösung von 1.99 g (3.54 mMol) des Gemisches des (3RS,4RS)-3-(Naphthalin-2-ylmethoxy)-4-[4-[2-[(RS)- und -[(SR)-tetrahydro-pyran-2-yloxy]-äthoxy]-phenyl]-piperidin-1-carbonsäure tert-butylesters in 11.3 ml Methanol wurde mit 11.3 ml 2 N Chlorwasserstoff in Methanol versetzt und 5 Minuten lang bei Raumtemperatur gerührt. Das Reaktionsgemisch wurde auf 200 ml einer Mischung aus Eis und gesättigter Natriumhydrogencarbonat-Lösung gegossen und dreimal mit Essigester extrahiert. Die organischen Phasen wurden mit gesättigter Natriumchlorid-Lösung gewaschen, über Magnesiumsulfat getrocknet, unter vermindertem Druck eingedampft und am Hochvakuum getrocknet. Das gelbe Harz (1.80 g) wurde aus Hexan umkristallisiert. Es wurden 950 mg (56 %) (3RS,4RS)-4-[4-(2-Hydroxy-äthoxy)-phenyl]-3-(naphthalin-2-ylmethoxy)-piperidin-1-carbonsäure tert-butylester als farbloser Festkörper erhalten; MS : 478 $(M+H)^+$.

(d) In analoger Weise wie in Beispiel 22 (k) beschrieben, wurde durch Acylierung von (3RS,4RS)-4-[4-(2-Hydroxy-äthoxy)-phenyl]-3-(naphthalin-2-ylmethoxy)-piperidin-1-carbonsäure tert-butylester mit 4-Chlorbenzoylchlorid der (3RS,4RS)-4-[4-[2-(4-Chlor-benzoyloxy)-äthoxy]-phenyl]-3-(naphthalin-2-ylmethoxy)-piperidin-1-carbonsäure tert-butylester als farbloses Öl erhalten; MS : 616 $(M+H)^+$.

(e) In analoger Weise wie in Beispiel 22 (l) beschrieben, wurde durch Abspaltung der BOC-Gruppe aus (3RS, 4RS)-4-[4-[2-(4-Chlor-benzoyloxy)-äthoxy]-phenyl]-3-(naphthalin-2-ylmethoxy)-piperidin-1-carbonsäure tert-butylester das 4-Chlor-benzoesäure (3RS,4RS)-2-[4-(3-Napthalin-2-ylmethoxy-piperidin-4-yl)-phenoxy]-äthylester

Hydrochlorid als farbloser Festkörper erhalten; MS : 516 $(M+H)^+$

Beispiel 54

In analoger Weise wie in Beispiel 22 (l) beschrieben, wurden durch Abspaltung der BOC-Gruppe mittels Säure die folgenden Verbindungen erhalten:

1) - Aus (3RS,4RS)-4-[4-[2-(4-Methoxy-benzoyloxy)-äthoxy]-phenyl]-3-(naphthalin-2-ylmethoxy)-piperidin-1-carbonsäure tert-butylester das 4-Methoxy-benzoesäure (3RS,4RS)-2-[4-[3-(Naphthalin-2-ylmethoxy)-piperidin-4-yl]-phenoxy]-äthylester Hydrochlorid als farbloser Festkörper; MS : 512 $(M+H)^+$;

2) - aus (3RS,4RS)-4-[4-[2-(2-Chlor-benzoyloxy)-äthoxy]-phenyl]-3-(naphthalin-2-ylmethoxy)-piperidin-1-carbonsäure tert-butylester das 2-Chlor-benzoesäure (3RS,4RS)-2-[4-(3-Napthalin-2-ylmethoxy-piperidin-4-yl)-phenoxy]-äthylester Hydrochlorid als farbloser Festkörper; MS : 516 $(M+H)^+$;

3) - aus (3RS,4RS)-4-[4-(2-Benzoyloxy-äthoxy)-phenyl]-3-(naphthalin-2-ylmethoxy)-piperidin-1-carbonsäure tert-butylester das Benzoesäure (3RS,4RS)-2-[4-[3-(naphthalin-2-yloxy)-piperidin-4-yl]-phenoxy]-äthylester als farbloser, amorpher Festkörper; MS : 340 $(M\text{-}Naphthylmethyl)^+$;

4) - aus (3RS,4RS)-4-[4-[2-(2-Benzoyloxymethyl-benzoyloxy)-äthoxy]-phenyl]-3-(naphthalin-2-ylmethoxy)-piperidin-1-carbonsäure tert-butylester das 2-Benzoyloxymethyl-benzoesäure (3RS,4RS)-2-[4-[3-(Naphthalin-2-ylmethoxy)-piperidin-4-yl]-phenoxy]-äthylester Hydrochlorid als farbloser Festkörper; MS : 616 $(M+H)^+$;

5) - aus (3RS,4RS)-3-(Naphthalin-2-ylmethoxy)-4-[4-(2-phenylsulfanyl-äthoxy)-phenyl]-piperidin-1-carbonsäure tert-butylester das (3RS,4RS)-3-(Naphthalin-2-ylmethoxy)-4-[4-(2-phenylsulfanyl-äthoxy)-phenyl]-piperidin Hydrochlorid als farbloser Festkörper; MS : 470 (M+H)+;

6) - aus (3RS,4RS)-3-(Naphthalin-2-ylmethoxy)-4-[4-(2-phenylsulfonyl-äthoxy)-phenyl]-piperidin-1-carbonsäure tert-butylester das (3RS,4RS)-3-Naphthalin-2-ylmethoxy-4-[4-(2-phenylsulfonyl-äthoxy)-phenyl]-piperidin als gelblicher Schaum; MS : 502 $(M+H)^+$;

7) - aus (3RS,4RS)-4-[4-(2-Benzoylamino-äthoxy)-phenyl]-3-(naphthalin-2-ylmethoxy)-piperidin-1-carbonsäure tert-butylester das (3RS,4RS)-N-[2-[4-[3-(Naphthalin-2-yloxy)-piperidin-4-yl]-phenoxy]-äthyl-benzamid als farbloser, amorpher Festkörper; MS : 481 $(M+H)^+$;

8) - aus (3RS,4RS)-3-Naphthalin-2-ylmethoxy-4-[4-(2-[1,2,4]triazol-1-yl-äthoxy)-phenyl]-piperidin-1-carbonsäure tert-butylester das (3RS,4RS)-3-(Naphthalin-2-ylmethoxy)-4-[4-(2-(1,2,4]triazol-1-yl-äthoxy)-phenyl]-piperidin Hydrochlorid als weißes Pulver; MS : 428 $(M)^+$;

9) - aus (3RS,4RS)-4-[4-(2-Benzyloxy-äthoxy)-phenyl]-3-(naphthalin-2-ylmethoxy)-piperidin-1-carbonsäure tert-butylester das (3RS,4RS)-4-[4-(2-Benzyloxy-äthoxy)-phenyl]-3-(naphthalin-2-ylmethoxy)-piperidin; MS: 468 $(M+H)^+$.

Die als Ausgangsstoffe eingesetzten BOC-Derivate wurden wie folgt hergestellt:

(a) Eine Lösung von 150 mg (0.314 mMol) (3RS,4RS)-4-[4-(2-Hydroxy-äthoxy)-phenyl]-3-(naphthalin-2-ylmethoxy)-piperidin-1-carbonsäure tert-butylester, 3.7 mg (0.031 mMol, 0.1 eq) 4-Dimethylaminopyridin, 65.9 mg (0.375 mMol, 1.2 eq) 4-Methoxy-benzoylchlorid und 51.7 µl Triäthylamin in 10 ml Methylenchlorid wurde 15 Stunden lang bei Raumtemperatur gerührt. Diese Reaktionslösung wurde auf 50 ml einer Eis/Wasser-Mischung gegossen und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das Rohprodukt (208 mg) wurde an Kieselgel mittels eines 95:5-Gemisches von Hexan und Aceton als Eluierungsmittel aufgetrennt. Es wurden 104 mg (51 % d.Th.) (3RS,4RS)-4-[4-[2-(4-Methoxy-benzoyloxy)-äthoxy]-phenyl]-3-(naphthalin-2-ylmethoxy)-piperidin-1-carbonsäure tert-butylester als farbloses Öl erhalten; MS : 612 $(M+H)^+$.

(b) In analoger Weise wie in Beispiel 22 (k) beschrieben, wurde durch Acylierung von (3RS,4RS)-4-[4-(2-Hydroxy-äthoxy)-phenyl]-3-(naphthalin-2-ylmethoxy)-piperidin-1-carbonsäure tert-butylester mit 2-Chlorbenzoylchlorid der

(3RS,4RS)-4-[4-[2-(2-Chlor-benzoyloxy)-äthoxy]-phenyl]-3-(naphthalin-2-ylmethoxy)-piperidin-1-carbonsäure tert-butylester als gelbes Öl erhalten; MS : 616 $(M+H)^+$.

(c) In analoger Weise wie in Beispiel 22 (k) beschrieben, wurde durch Acylierung von (3RS,4RS)-4-[4-(2-Hydroxy-äthoxy)-phenyl]-3-(naphthalin-2-ylmethoxy)-piperidin-1-carbonsäure tert-butylester mit Benzoylchlorid der (3RS, 4RS)-4-[4-(2-Benzoyloxy-äthoxy)-phenyl]-3-(naphthalin-2-ylmethoxy)-piperidin-1-carbonsäure tert-butylester als farbloser, amorpher Festkörper erhalten; $R_f$: 0.61 ($SiO_2$, Hexan:Aceton=95:5).

(d) In analoger Weise wie in Beispiel 22 (k) beschrieben, wurde durch Acylierung von (3RS,4RS)-4-[4-(2-Hydroxy-äthoxy)-phenyl]-3-(naphthalin-2-ylmethoxy)-piperidin-1-carbonsäure tert-butylester mit Benzoesäure 2-chlorcarbonyl-benzylester das (3RS,4RS)-4-[4-[2-(2-Benzoyloxymethyl-benzoyloxy)-äthoxy]-phenyl]-3-(naphthalin-2-ylmethoxy)-piperidin-1-carbonsäure tert-butylester als farbloser Festkörper erhalten; MS : 716 $(M+H)^+$.

(e) In analoger Weise wie in Beispiel 33 (a) beschrieben, wurde aus (3RS,4RS)-4-[4-(2-Hydroxy-äthoxy)-phenyl]-3-(naphthalin-2-ylmethoxy)-piperidin-1-carbonsäure tert-butylester und Diphenylsulfid der (3RS,4RS)-3-(Naphthalin-2-ylmethoxy)-4-[4-(2-phenylsulfanyl-äthoxy)-phenyl] -piperidin-1-carbonsäure tert-butylester als farbloser Festkörper erhalten; MS : 570 $(M+H)^+$.

(f) In analoger Weise wie in Beispiel 33 (c) beschrieben, wurde durch Oxidation des (3RS,4RS)-3-(Naphthalin-2-ylmethoxy)-4-[4-(2-phenylsulfanyl-äthoxy)-phenyl]-piperidin-1-carbonsäure tert-butylesters das (3RS,4RS)-3-(Naphthalin-2-ylmethoxy)-4-[4-(2-phenylsulfonyl-äthoxy)-phenyl]-piperidin-1-carbonsäure tert-butylester als farbloser Schaum erhalten; MS : 619 $(M+NH_4)^+$.

(g) Eine Lösung aus 478 mg (1.00 mMol) (3RS,4RS)-4-[4-(2-Hydroxy-äthoxy)-phenyl]-3-(naphthalin-2-ylmethoxy)-piperidin-1-carbonsäure tert-butylester und 93 ml Mesylchlorid in 5 ml Pyridin wurde bei Raumtemperatur 2 Stunden lang gerührt. Anschließend wurde die Reaktionslösung auf 50 ml eines Eis/Wasser-Gemisches gegossen und dreimal mit Methylenchlorid extrahiert. Die organischen Phasen wurde zweimal mit Wasser gewaschen, über Magnesiumsulfat getrocknet, unter vermindertem Druck eingedampft und am Hochvakuum getrocknet. Dies lieferte 569 mg rohen (3RS,4RS)-4-[4-(2-Methylsulfonyloxy-äthoxy)-phenyl] -3-(naphthalin-2-ylmethoxy)-piperidin-1-carbonsäure tert-butylester, welcher ohne weitere Reinigung und Charakterisierung in der folgenden Stufe eingesetzt wurde.

(h) Ein Gemisch aus 569 mg rohem (3RS,4RS)-4-[4-(2-Methylsulfonyloxy-äthoxy)-phenyl]-3-(naphthalin-2-ylmethoxy)-piperidin-1-carbonsäure tert-butylester und 650 mg Natriumazid in 20 ml Dimethylsulfoxid wurde 3.5 Stunden lang bei 80° C gerührt. Anschließend wurde diese Reaktionslösung auf 50 ml einer Eis/Wasser-Mischung gegossen und dreimal mit Essigester extrahiert. Die organischen Phasen wurden mit Wasser gewaschen, über Magnesiumsulfat getrocknet, unter vermindertem Druck eingedampft und am Hochvakuum getrocknet. Das gelbe Öl (670 mg) wurde an Kieselgel mittels eines 9:1-Gemisches von Hexan und Essigester als Eluierungsmittel aufgetrennt. Es wurden 271 mg (54 % d.Th. über beide Stufen) (3RS,4RS)-4-[4-(2-Azido-äthoxy)-phenyl]-3-(naphthalin-2-ylmethoxy)-piperidin-1-carbonsäure tert-butylester als farbloser Festkörper erhalten; MS : 503 $(M+H)^+$.

(i) Ein Gemisch aus 115.9 mg (0.231 mMol) (3RS,4RS)-4-[4-(2-Azido-äthoxy)-phenyl]-3-(naphthalin-2-ylmethoxy)-piperidin-1-carbonsäure tert-butylester, 87.4 mg (0.330 mMol, 1.43 eq) Triphenylphosphin und 6.1 µl (6.1 mg, 0.339 mMol, 1.47 eq) entionisiertes Wasser wurde 4 Stunden lang bei Raumtemperatur gerührt. Anschließend wurden 3 ml Essigsäure zugegeben und 17 Stunden lang bei Raumtemperatur gerührt. Dieses Reaktionsgemisch wurde auf eine Eis/Wasser-Mischung gegossen, mit gesättigter Natriumhydrogencarbonat-Lösung basisch gestellt und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Dies lieferte 192.3 mg rohen (3RS,4RS)-4-[4-(2-Amino-äthoxy)-phenyl]-3-(naphthalin-2-ylmethoxy)-piperidin-1-carbonsäure tert-butylester, welcher ohne weitere Reinigung in der folgenden Reaktion eingesetzt wurde; $R_f$: 0.10 ($SiO_2$, Methylenchlorid:Aceton=95:5 + 0.1% Ammoniak)

(j) Eine Lösung aus 192.4 mg rohem (3RS,4RS)-4-[4-(2-Amino-äthoxy)-phenyl]-3-(naphthalin-2-ylmethoxy)-piperidin-1-carbonsäure tert-butylester in 3 ml Methylenchlorid wurde bei 0° C mit 51.7 ml Triäthylamin und 64.9 mg (0.462 mMol, 2.0 eq) Benzoylchlorid versetzt und 0.75 Stunden lang bei 0° C, respektive 3 Stunden lang bei Raumtemperatur gerührt. Das Reaktionsgemisch wurde auf eine Eis/Wasser-Mischung gegossen, mit gesättigter Natriumhydrogen-carbonat-Lösung basisch gestellt und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das farblose Öl wurde an Kieselgel mittels eines 95:5-Gemisches von Methylenchlorid und Aceton als Eluierungsmittel aufgetrennt. Es wurden 44.9 mg (33 % d.Th.) (3RS,4RS)-4-[4-(2-Benzoylamino-äthoxy)-phenyl]-3-(naphthalin-2-ylmethoxy)-piperidin-1-carbonsäure tert-butylester in Form eines schwach gelben Öles erhalten; MS : 581 $(M+H)^+$.

(k) Eine Lösung 110 mg (0.2 mMol) rohem (3RS,4RS)-4-[4-(2-Methylsulfonyloxy-äthoxy)-phenyl]-3-(naphthalin-2-ylmethoxy)-piperidin-1-carbonsäure tert-butylester in 5 ml Dimethylformamid wurde mit 70 mg (1.0 mMol) 1,2,4-Triazol-Natriumsalz versetzt und das Reaktionsgemisch 6 Stunden lang auf 100° C erhitzt. Anschließend wurde auf Raumtemperatur abgekühlt und das Dimethylformamid im Ölpumpenvakuum abdestilliert. Der Rückstand wurde in 10 ml Methylenchlorid aufgenommen, mit 2 ml Wasser gewaschen, die organische Phase über Natriumsulfat getrocknet und unter vermindertem Druck eingedampft. Der Rückstand wurde an Kieselgel unter Verwendung eines 95:5:0.1-Gemisches von Methylenchlorid, Methanol und Ammoniak als Eluierungsmittel chromatographiert. Es wurden 90 mg (85 % d.Th.) (3RS,4RS)-3-Naphthalin-2-ylmethoxy-4-[4-(2-[1,2,4]triazol-1-yl-äthoxy)-phenyl]-piperidin-1-carbonsäure tert-butylester als farbloses Öl erhalten; MS : 529 $(M+H)^+$.

(1) In Analogie zu dem im Beispiel 3 (c) beschriebenen Verfahren, wurde durch Alkylierung des (3RS,4RS)-4-[4-(2-Hydroxy-äthoxy)-phenyl]-3-(naphthalin-2-ylmethoxy)-piperidin-1-carbonsäure tert-butylester mit Benzylbromid der (3RS,4RS)-4-[4-(2-Benzyloxy-äthoxy)-phenyl]-3-(naphthalin-2-ylmethoxy)-piperidin-1-carbonsäure tert-butylester erhalten; MS : 568 $(M+H)^+$.

Beispiel 55

In analoger Weise wie in Beispiel 22 (l) beschrieben, wurden die folgenden Verbindungen erhalten:

1) - Aus (3RS,4RS)-4-[4-[2-(2-Chlormethyl-benzoyloxy)-äthoxy]-phenyl]-3-[4-(2-trimethylsilanyl-äthoxymethoxy)-naphthalin-2-ylmethoxy]-piperidin-1-carbonsäure tert-butylester durch gleichzeitige Abspaltung der BOC- und Acetalgruppe der 2-Chlormethyl-benzosäure (3RS,4RS)-2-{4-[3-(4-Hydroxy-naphthalin-2-ylmethoxy)-piperidin-4-yl]-phenoxy}-äthylester als farbloses Öl;

2) - aus (3RS,4RS)-4-[4-(2-Benzoyloxy-äthoxy)-phenyl]-3-[4-(2-methoxybenzyloxy)-naphthalin-2-ylmethoxy]-piperidin-1-carbonsäure tert-butylester das Benzoesäure (3RS,4RS)-2-[4-[3-[4-(2-Methoxy-benzyloxy)-naphthalin-2-ylmethoxy]-piperidin-4-yl]-phenoxy]-äthylester Hydrochlorid als farbloser Festkörper; MS : 618 $(M+H)^+$;

3) - aus (3RS,4RS)-4-[4-(2-Benzyloxy-äthoxy)-phenyl]-3-(4-benzyloxynaphthalin-2-ylmethoxy)-piperidin-1-carbonsäure tert-butylester das (3RS,4RS)-4-[4-(2-Benzyloxy-äthoxy)-phenyl]-3-(4-benzyloxy-naphthalin-2-ylmethoxy)-piperidin; MS : 574 $(M+H)^+$.

Die als Ausgangsstoffe eingesetzten BOC-Derivate wurden wie folgt hergestellt:

(a) In analoger Weise wie in Beispiel 44 (e) beschrieben, wurde durch Alkylierung des Gemisches der (3RS,4RS)-3-Hydroxy-4-[4-[2-[(RS)- und -[(SR)-tetrahydro-pyran-2-yloxy]-äthoxy]-phenyl]-piperidin-1-carbonsäure tert-butylesters mit 3-Chlormethyl-1-(2-trimethylsilanyl-äthoxymethoxy)-naphthalin das Gemisch des (3RS,4RS)-4-[4-[2-[(RS)-und -[(SR)-tetrahydro-pyran-2-yl]-äthoxy]-phenyl]-3-[4-(2-trimethylsilanyl-äthoxymethoxy)-naphthalin-2-ylmethoxy]-piperidin-1-carbonsäure tert-butylesters als farbloses Öl erhalten; MS : 725 $(M+NH_4)^+$.

(b) In analoger Weise wie in Beispiel 53 (c) beschrieben, wurde aus dem Gemisch der (3RS,4RS)-4-[4-[2-[(RS)- und -[(SR)-tetrahydro-pyran-2-yl]-äthoxy]-phenyl]-3-[4-(2-trimethylsilanyl-äthoxymethoxy)-naphthalin-2-ylmethoxy]-piperidin-1-carbonsäure tert-butylesters der (3RS,4RS)-4-[4-(2-Hydroxy-äthoxy)-phenyl]-3-[4-(2-trimethylsilanyl-äthoxymethoxy)-naphthalin-2-ylmethoxy]-piperidin-1-carbonsäure tert-butylester als farbloser Festkörper erhalten; MS : 624 $(M+H)^+$.

(c) In analoger Weise wie in Beispiel 22 (k) beschrieben, wurde durch die Acylierung des (3RS,4RS)-4-[4-(2-Hydroxy-äthoxy)-phenyl]-3-[4-(2-trimethylsilanyl-äthoxymethoxy)-naphthalin-2-ylmethoxy]-piperidin-1-carbonsäure tert-butylesters mit 2-Chlormethyl-benzoylchlorid der (3RS,4RS)-4-[4-[2-(2-Chlormethyl-benzoyloxy)-äthoxy]-phenyl]-3-[4-(2-trimethylsilanyl-äthoxymethoxy)-naphthalin-2-ylmethoxy]-piperidin-1-carbonsäure tert-butylester als farbloser Festkörper erhalten; MS : 776 $(M+H)^+$.

(d) 200 mg (0,28 mMol) des Gemisches des (3RS,4RS)-4-[4-[2-[(RS)- und -[(SR)-tetrahydro-pyran-2-yl]-äthoxy]-phenyl]-3-[4-(2-trimethylsilanyl-äthoxymethoxy)-naphthalin-2-ylmethoxy]-piperidin-1-carbonsäure tert-butylesters wurden in 7.6 ml 0.1 M Chlorwasserstoff in Methanol gelöst und 2 Stunden lang bei Raumtemperatur gerührt. Anschließend wurden erneut 0.36 ml 2 M Chlorwasserstoff in Methanol zugegeben und weitere 2 Stunden lang bei Raumtemperatur gerührt. Diese Reaktionslösung wurde auf halb gesättigte Natriumhydrogencarbonat-Lösung gegossen und dreimal mit Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das gelbliche Öl (204 mg) wurde an Kieselgel mittels eines 1:1-Gemisches von Hexan und Essigester (extrahiert gegen konzentrierten Ammoniak) aufgetrennt. Dies lieferte 111 mg (80 % d.Th.) (3RS,4RS)-4-[4-(2-Hydroxy-äthoxy)-phenyl]-3-(4-hydroxynaphthalin-2-ylmethoxy)-piperidin-1-carbonsäure tert-butylester in Form eines farblosen Öles; $R_f$: 0.26 ($SiO_2$, Hexan:Essigester=1:1).

(e) In analoger Weise wie in Beispiel 44 (e) beschrieben, wurde durch Alkylierung des (3RS,4RS)-4-[4-(2-Hydroxy-äthoxy)-phenyl]-3-(4-hydroxy-naphthalin-2-ylmethoxy)-piperidin-1-carbonsäure tert-butylesters mit 2-Methoxy-benzylchlorid das (3RS,4RS)-4-[4-(2-Hydroxy-äthoxy)-phenyl]-3-[4-(2-methoxy-benzyloxy)-naphthalin-2-ylmethoxy]-piperidin-1-carbonsäure tert-butylester als farbloses Öl erhalten; MS : 652 $(M+K)^+$.

(f) In analoger Weise wie in Beispiel 22 (k) beschrieben, wurde durch Acylierung des (3RS,4RS)-4-[4-(2-Hydroxy-äthoxy)-phenyl]-3-[4-(2-methoxy-benzyloxy)-naphthalin-2-ylmethoxy]-piperidin-1-carbonsäure tert-butylesters mit Benzoylchlorid das (3RS,4RS)-4-[4-(2-Benzoyloxy-äthoxy)-phenyl]-3-[4-(2-methoxy-benzyloxy)-naphthalin-2-ylmethoxy]-piperidin-1-carbonsäure tert-butylester als farbloses Öl erhalten; MS : 736 $(M+H)^+$.

(g) In analoger Weise wie im Beispiel 1 (g) beschrieben, wurde durch die zweifache Alkylierung des (3RS,4RS)-4-[4-(2-Hydroxy-äthoxy)-phenyl]-3-(4-hydroxy-naphthalin-2-ylmethoxy)-piperidin-1-carbonsäure tert-butylesters mit Benzylbromid das (3RS,4RS)-4-[4-(2-Benzyloxy-äthoxy)-phenyl]-3-(4-benzyloxy-naphthalin-2-ylmethoxy)-piperidin-1-carbonsäure tert-butylester erhalten.

Beispiel 56

In analoger Weise wie in Beispiel 22 (l) beschrieben, wurden durch gleichzeitige Abspaltung der BOC- und SEM-Gruppe mittels Säure die folgenden Verbindungen erhalten:

1) - Aus (3RS,4RS)-3-(Naphthalin-2-ylmethoxy)-4-[4-[2-[4-(2-trimethylsilanyl-äthoxymethoxy)-benzoyloxy]-äthoxy]-phenyl]-piperidin-1-carbonsäure tert-butyl ester das 4-Hydroxy-benzoesäure (3RS,4RS)-2-[4-(3-naphthalin-2-ylmethoxy-piperidin-4-yl)-phenoxy]-äthylester Hydrochlorid als farbloser Festkörper; MS : 498 $(M+H)^+$;

2) - aus (3RS,4RS)-4-[4-(2-Benzyloxy-äthoxy)-phenyl]-3-(4-hydroxynaphthalin-2-ylmethoxy)-piperidin-1-carbonsäure tert-butylester das (3RS,4RS)-3-[4-[4-(2-Benzyloxy-äthoxy)-phenyl]-piperidin-3-yloxymethyl)-naphthalin-1-ol; MS : 484 $(M+H)^+$.

Die als Ausgangsstoffe eingesetzten BOC-Derivate wurden wie folgt hergestellt:,

(a) In analoger Weise wie unter Beispiel 24 (l) beschrieben, wurde unter Verwendung von N-Äthyl-N'-(3-dimethylaminopropyl)-carbodiimid Hydrochlorid (EDC) als Kondensationsmittel aus (3RS,4RS)-4-[4-(2-Hydroxy-äthoxy)-phenyl]-3-(naphthalin-2-ylmethoxy)-piperidin-1-carbonsäure tert-butylester und 4-(2-Trimethylsilanyl-äthoxymethoxy)-benzoesäure der (3RS,4RS)-3-(Naphthalin-2-ylmethoxy)-4-[4-[2-[4-(2-trimethylsilanyl-äthoxymethoxy)-benzoyloxy]-äthoxy]-phenyl]-piperidin-1-carbonsäure tert-butyl ester als farbloser, amorpher Festkörper erhalten; MS : 728 $(M+H)^+$.

(b) In analoger Weise wie in Beispiel 1 (g) beschrieben, wurde durch Alkylierung des (3RS,4RS)-4-[4-(2-Hydroxy-äthoxy)-phenyl]-3-[4-(2-trimethylsilanyl-äthoxymethoxy)-naphthalin-2-ylmethoxy]-piperidin-1-carbonsäure tert-butylesters mit Benzylbromid das (3RS,4RS)-4-[4-(2-Benzyloxy-ethoxy)-phenyl]-3-(4-hydroxy-naphthalin-2-ylmethoxy)-piperidin-1-carbonsäure tert-butylester erhalten;

Beispiel 57

(a) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters mit rac.-2-(3-Brom-propoxy)-tetrahydropyran das Gemisch des (3RS,4RS)-3-Hydroxy-4-[4-[3-[(RS)- und -[(SR)-tetrahydro-pyran-2-yloxy]-propoxy]-phenyl]-piperidin-1-carbonsäure tert-butylesters als farbloser Festkörper erhalten; $R_f$: 0.23 ($SiO_2$, Hexan:Essigester=4:1).

(b) Die Alkylierung des Gemisches des (3RS,4RS)-3-Hydroxy-4-[4-[3-[(RS)- und -[(SR)-tetrahydro-pyran-2-yloxy]-propoxy]-phenyl]-piperidin-1-carbonsäure tert-butylesters mit 2-Brommethylnaphthalin, analog zu dem in Beispiel 22 (i) beschriebenen Verfahren, ergab ein Gemisch des (3RS,4RS)-3-(Naphthalin-2-ylmethoxy)-4-[4-[3-[(RS)- und -[(SR)-tetrahydro-pyran-2-yloxy]-propoxy]-phenyl]-piperidin-1-carbonsäure tert-butylesters als gelbes Öl; $R_f$: 0.35 ($SiO_2$, Hexan:Aceton=4:1).

(c) Eine Lösung von 5.22 g des rohen Gemisches des (3RS,4RS)-3-(Naphthalin-2-ylmethoxy)-4-[4-[3-[(RS)- und -[(SR)-tetrahydro-pyran-2-yloxy]-propoxy]-phenyl]-piperidin-1-carbonsäure tert-butylesters in 30 ml Tetrahydrofuran und 30 ml 2 N Salzsäure wurde 14 Stunden lang bei Raumtemperatur und 2 Stunden lang bei 40° C gerührt. Das Tetrahydrofuran wurde anschließend unter vermindertem Druck abdestilliert und die verbleibende wäßrige Phase dreimal mit Methylenchlorid extrahiert. Die organische Phase wurde über Magnesiumsulfat getrocknet und danach unter vermindertem Druck abdestilliert. Das resultierende gelbe Öl (2.9 g) wurde an Kieselgel mittels eines 9:1-Gemisches von Methylenchlorid und Essigester als Eluierungsmittel chromatographiert. Es wurden 1.08 g (34 % d.Th. über beide Stufen) (3RS,4RS)-4-[4-(3-Hydroxy-propoxy)-phenyl]-3-(naphthalin-2-ylmethoxy)-piperidin-1-carbonsäure tert-butylester in Form eines weißen amorphen Festkörpers erhalten; MS : 491 $(M)^+$.

(d) Die Umsetzung des (3RS,4RS)-4-[4-(3-Hydroxy-propoxy)-phenyl]-3-(naphthalin-2-ylmethoxy)-piperidin-1-carbonsäure tert-butylesters mit Mesylchlorid, analog zu dem in Beispiel 54 (h) beschriebenen Verfahren, ergab den (3RS,4RS)-4-[4-(3-Methylsulfonyloxy-propoxy)-phenyl]-3-(naphthalin-2-ylmethoxy)-piperidin-1-carbonsäure tert-butylester als gelbes Öl; $R_f$: 0.50 ($SiO_2$, Hexan:Essigester=1:1).

(e) Eine Lösung von 850 mg rohem (3RS,4RS)-4-[4-(3-Methylsulfonyloxypropoxy)-phenyl]-3-(naphthalin-2-ylmethoxy)-piperidin-1-carbonsäure tert-butylester in 6.1 ml (48.98 mMol, 32.4 eq) einer 33 %igen Methylamin-Lösung in Äthanol wurde 14 Stunden lang bei Raumtemperatur gerührt. Danach wurde Lösung unter vermindertem Druck eingedampft. Der gelbe Festkörper (932 mg) wurde an Kieselgel mittels eines 90:10:0.1-Gemisches von Methylenchlorid, Methanol und Ammoniak als Eluierungsmittel chromatographiert. Dies lieferte 610 mg (80 % d.Th. über beide Stufen) (3RS,4RS)-4-[4-(3-Methylaminopropoxy)-phenyl]-3-(naphthalin-2-ylmethoxy)-piperidin- 1-carbonsäure tert-butylester als farblosen Festkörper; MS : 505 $(M+H)^+$.

(f) Die Acylierung des (3RS,4RS)-4-[4-(3-Methylamino-propoxy)-phenyl]-3-(naphthalin-2-ylmethoxy)-piperidin-1-carbonsäure tert-butylesters mit Benzoylchlorid, analog zu dem in Beispiel 22 (k) beschriebenen Verfahren, ergab den (3RS,4RS)-4-[4-[3-(Benzoyl-methyl-amino)-propoxy]-phenyl]-3-(naphthalin-2-ylmethoxy)-piperidin-1-carbonsäure tert-butylester als farbloses Öl; MS : 609 (M+H)+.

(g) In analoger Weise zu dem in Beispiel 22 (l) beschriebenen Verfahren, wurde aus (3RS,4RS)-4-[4-[3-(Benzoyl-methyl-amino)-propoxy]-phenyl]-3-(naphthalin-2-ylmethoxy)-piperidin-1-carbonsäure tert-butylester durch Abspaltung der BOC-Gruppe das (3RS,4RS)-N-Methyl-N-[3-[4-[3-(naphthalin-2-yloxy)-piperidin-4-yl]-phenoxy]-propyl]-benzamid als farbloser Festkörper erhalten; MS : 509 $(M+H)^+$.

Beispiel 58

In Analogie zu dem in Beispiel 8 (g) beschriebenen Verfahren, wurden durch Abspaltung der BOC-Gruppe mittels Salzsäure in Methanol die folgenden Verbindungen erhalten:

1) -Aus (3RS,4RS)-Nicotinsäure 2-[4-[1-tert-Butoxycarbonyl-3-(naphthalin-2-ylmethoxy)-piperidin-4-yl]-phenoxy}-äthylester das Pyridin-3-carbonsäure (3RS,4RS)-2-[4-(3-Naphthalin-2-ylmethoxypiperidin-4-yl)-phenoxy]-äthylester Hydrochlorid als farbloser Festkörper; MS : 483 $(M+H)^+$;

2) - aus (3RS,4RS)-4-{4-[2-(Benzo[1,3]dioxol-5-carbonyloxy)-äthoxy]-phenyl}-3-(naphthalin-2-ylmethoxy)-piperidin-1-carbonsäure tert-butylester das 1,3-Benzodioxole-5-carbonsäure (3RS,4RS)-2-[4-(3-Naphthalin-2-ylmethoxy-piperidin-4-yl)-phenoxy]-äthylester Hydrochlorid als farbloser Festkörper; MS : 526 $(M+H)^+$;

3) - aus (3RS,4RS)-3-(Naphthalin-2-ylmethoxy)-4-{4-[2-(thiophen-3-carbonyloxy)-äthoxy]-phenyl}-piperidin-1-carbonsäure tert-butylester das Thiophen-3-carbonsäure (3RS,4RS)-2-[4-(3-Naphthalin-2-ylmethoxy-piperidin-4-yl)-

phenoxy]-äthylester Hydrochlorid als farbloser Festkörper; MS : 488 $(M+H)^+$;

4) - aus (3RS,4RS)-3-(Naphthalin-2-ylmethoxy)-4-[4-[2-(thiophen-2-carbonyloxy)-äthoxy]-phenyl]-piperidin-1-carbonsäure tert-butylester das Thiophen-2-carbonsäure (3RS,4RS)-2-[4-(3-Naphthalin-2-ylmethoxypiperidin-4-yl)-phenoxy]-äthylester Hydrochlorid als farbloser Festkörper; MS : 488 $(M+H)^+$;

5) - aus 4-[4-[2-(Furan-3-carbonyloxy)-äthoxy]-phenyl]-3-(naphthalin-2-ylmethoxy)-piperidin-1-carbonsäure tert-butylester das Furan-3-carbonsäure 2-[(3RS,4RS)-4-[3-(naphthalin-2-ylmethoxy)-piperidin-4-yl]-phenoxy]-äthylester Hydrochlorid als farbloser Festkörper; MS : 472 $(M+H)^+$;

6) - aus 4-[4-[2-(Furan-2-carbonyloxy)-äthoxy]-phenyl}-3-(naphthalin-2-ylmethoxy)-piperidin-1-carbonsäure tert-butylester das Furan-2-carbonsäure 2-[(3RS,4RS)-4-[3-(naphthalin-2-ylmethoxy)-piperidin-4-yl]-phenoxy]-äthylester Hydrochlorid als farbloser Festkörper; MS : 472 $(M+H)^+$;

7) - aus dem Gemisch des (3RS,4RS)-4-[4-[2-[(RS)- und (SR)-Methoxyphenyl-acetoxy]-äthoxy]-phenyl]-3-(naphthalin-2-ylmethoxy)-piperidin-1-carbonsäure tert-butylesters das Gemisch des (RS)- und (SR)-Methoxy-phenylessigsäure (3RS,4RS)-2-[4-(2-Naphthalin-2-ylmethoxypiperdin-4-yl)-phenoxy]-äthylester Hydrobromids als bräunlicher Festkörper; MS: 526 $(M+H)^+$;

8) - aus (3RS,4RS)-4-[4-[2-(2-Methylsulfanyl-benzoyloxy)-äthoxy]-phenyl]-3-(naphthalin-2-ylmethoxy)-piperidin-1-carbonsäure tert-butylester das 2-Methylsulfanyl-benzoesäure (3RS,4RS)-2-[4-(3-naphthalin-2-yl-methoxy-piperidin-4-yl)-phenoxy]-äthylester Hydrochlorid als beigefarbener Festkörper; MS : 528 $(M+H)^+$;

9) - aus (3RS,4RS))-4-{4-[2-[(RS)- und (SR)-2-Methylsulfinylbenzoyloxy]-äthoxy]-phenyl}-3-(naphthalin-2-ylmethoxy)-piperidin-1-carbonsäure tert-butylester das (RS)- und (SR)-2-Methylsulfinylbenzoesäure (3RS,4RS)-2-[4-(2-Naphthalin-2-ylmethoxy-piperidin-4-yl)-phenoxy]-äthylester Hydrochlorids als farbloser Festkörper; MS : 544 $(M+H)^+$.

Die als Ausgangssubstanzen eingesetzten BOC-Derivate wurden in analoger Weise wie unter Beispiel 24 (l) beschrieben, unter Verwendung von N-Äthyl-N'-(3-dimethylaminopropyl)-carbodiimid Hydrochlorid (EDC) als Kondensationsmittel wie folgt hergestellt:

(a) Aus (3RS,4RS)-4-[4-(2-Hydroxy-äthoxy)-phenyl]-3-(naphthalin-2-ylmethoxy)-piperidin-1-carbonsäure tert-butylester und Pyridin-3-carbonsäure der (3RS,4RS)-Nicotinsäure 2-[4-[1-tert-Butoxycarbonyl-3-(naphthalin-2-ylmethoxy)-piperidin-4-yl]-phenoxy}-äthylester als farbloser Festkörper; MS : 481 $(M-C_4H_9COO)^+$.

(b) Aus (3RS,4RS)-4-[4-(2-Hydroxy-äthoxy)-phenyl]-3-(naphthalin-2-ylmethoxy)-piperidin-1-carbonsäure tert-butylester und Piperonylsäure der (3RS,4RS)-4-{4-[2-(Benzo[1,3]dioxol-5-carbonyloxy)-äthoxy]-phenyl}-3-(naphthalin-2-ylmethoxy)-piperidin-1-carbonsäure tert-butylester als farbloser Festkörper; MS : 626 $(M+H)^+$.

(c) Aus (3RS,4RS)-4-[4-(2-Hydroxy-äthoxy)-phenyl]-3-(naphthalin-2-ylmethoxy)-piperidin- 1-carbonsäure tert-butylester und Thiophen-3-carbonsäure der (3RS,4RS)-3-(Naphthalin-2-ylmethoxy)-4-{4-[2-(thiophen-3-carbonyloxy)-äthoxy]-phenyl}-piperidin-1-carbonsäure tert-butylester als farbloser Festkörper; MS : 587 $(M)^+$.

(d) Aus (3RS,4RS)-4-[4-(2-Hydroxy-äthoxy)-phenyl)-3-(naphthalin-2-ylmethoxy)-piperidin-1-carbonsäure tert-butylester und Thiophen-2-carbonsäure der (3RS,4RS)-3-(Naphthalin-2-ylmethoxy)-4-[4-[2-(thiophen-2-carbonyloxy)-äthoxy]-phenyl]-piperidin-1-carbonsäure tert-butylester als farbloser Festkörper; MS: 588 $(M+H)^+$.

(e) Aus (3RS,4RS)-4-[4-(2-Hydroxy-äthoxy)-phenyl]-3-(naphthalin-2-ylmethoxy)-piperidin-1-carbonsäure tert-butylester und Furan-3-carbonsäure der 4-[4-[2-(Furan-3-carbonyloxy)-äthoxy]-phenyl]-3-(naphthalin-2-ylmethoxy)-piperidin-1-carbonsäure tert-butylester als farbloser Festkörper; MS: 572 $(M+H)^+$.

(f) Aus (3RS,4RS)-4-[4-(2-Hydroxy-äthoxy)-phenyl]-3-(naphthalin-2-ylmethoxy)-piperidin-1-carbonsäure tert-butylester und Furan-2-carbonsäure der 4-[4-[2-(Furan-2-carbonyloxy)-äthoxy]-phenyl}-3-(naphthalin-2-ylmethoxy)-piperidin-1-carbonsäure tert-butylester als farbloser Festkörper; MS : 572 $(M+H)^+$.

(g) Aus (3RS,4RS)-4-[4-(2-Hydroxy-äthoxy)-phenyl]-3-(naphthalin-2-ylmethoxy)-piperidin-1-carbonsäure tert-butylester und (RS)-$\alpha$-Methoxyphenylessigsäure das Gemisch des (3RS,4RS)-4-[4-[2-[(RS)- und (SR)-Methoxy-phenyl-acetoxy]-äthoxy]-phenyl]-3-(naphthalin-2-ylmethoxy)-piperidin-1-carbonsäure tert-butylesters als farbloses Öl; MS: 648 $(M+Na)^+$.

(h) Aus (3RS,4RS)-4-[4-(2-Hydroxy-äthoxy)-phenyl]-3-(naphthalin-2-ylmethoxy)-piperidin-1-carbonsäure tert-butylester und 2-Methylsulfanylbenzoesäure der (3RS,4RS)-4-[4-[2-(2-Methylsulfanylbenzoyloxy)-äthoxy]-phenyl]-3-(naphthalin-2-ylmethoxy)-piperidin-1-carbonsäure tert-butylester als farbloser Festkörper; MS : 628 $(M+H)^+$.

(i) Zu einer Lösung von 250 mg (0.4 mMol) (3RS,4RS)-4-[4-[2-(2-Methylsulfanyl-benzoyloxy)-äthoxy]-phenyl]-3-(naphthalin-2-ylmethoxy)-piperidin-1-carbonsäure tert-butylester in 25 ml Methanol wurde eine Lösung von 170 mg (0.8 mMol) Natriummetaperjodat in 2 ml Wasser gegeben. Das resultierende Reaktionsgemisch wurde 8 Stunden lang bei 50 °C gerührt. Danach wurde das Lösungsmittel unter vermindertem Druck abdestilliert, der Rückstand zwischen Essigester und Wasser verteilt, die organische Phase über Natriumsulfat getrocknet und unter vermindertem Druck eingedampft. Es wurden 230 mg (90 % d.Th.) roher (3RS,4RS)-4-{4-[2-[(RS)- und (SR)-2-Methylsulfinyl-benzoyloxy]-äthoxy]-phenyl)-3-(naphthalin-2-ylmethoxy)-piperidin-1-carbonsäure tert-butylester als farbloser Festkörper erhalten; MS : 644 (M+H)+.

Beispiel 59

In analoger Weise wie in Beispiel 22 (l) beschrieben, wurden durch Abspaltung der BOC-Gruppe die folgenden Verbindungen erhalten:

1) - Aus (3RS,4RS)-4-[4-(3-Morpholin-4-yl-propoxy)-phenyl]-3-(naphthalin-2-ylmethoxy)-piperidin-1-carbonsäure tert-butylester das (3RS,4RS)-4-[4-(3-Morpholin-4-yl-propoxy)-phenyl]-3-(naphthalin-2-ylmethoxy)-piperidin als farbloser Festkörper; MS : 461 $(M+H)^+$;

2) - aus (3RS,4RS)-4-[4-[3-(2,2-Dimethyl-propionyloxy)-propoxy]-phenyl]-3-(naphthalin-2-ylmethoxy)-piperidin-1-carbonsäure tert-butylester der 2,2-Dimethyl-propionsäure (3RS,4RS)-3-[4-(3-Naphthalin-2-ylmethoxy-piperidin-4-yl)-phenoxy]-propylester als farbloser, amorpher Festkörper; MS : 476 $(M+H)^+$;

3) - aus (3RS,4RS)-4-[4-(3-Benzoyloxy-propoxy)-phenyl]-3-(naphthalin-2-ylmethoxy)-piperidin-1-carbonsäure tert-butylester das Benzoesäure (3RS,4RS)-3-[4-(3-Napthalin-2-ylmethoxy-piperidin-4-yl)-phenoxy]-propylester Hydrochlorid als farbloser Festkörper; MS : 496 $(M+H)^+$;

4) - aus (3RS,4RS)-4-(4-Benzyloxy-phenyl)-3-(naphthalin-2-yloxy)-piperidin-1-carbonsäure tert-butylester das (3RS,4RS)-4-(4-Benzyloxyphenyl)-3-(naphthalin-2-yloxy)-piperidin als farbloser, amorpher Festkörper; MS : 424 $(M+H)^+$;

5) - aus (3RS,4RS)-3-(2-Methoxy-benzyloxy)-4-[4-(naphthalin-2-ylmethoxy)-phenyl]-piperidin-1-carbonsäure tert-butylester das (3RS,4RS)-3-(2-Methoxy-benzyloxy)-4-(4-naphthalin-2-ylmethoxyphenyl)-piperidin Hydrochlorid als farbloser Festkörper; MS : 454 $(M+H)^+$.

Die als Ausgangsstoffe eingesetzten BOC-Derivate wurden wie folgt hergestellt:

(a) Eine Lösung von 200 mg (0.35 mMol) (3RS,4RS)-4-[4-(3-Methylsulfonyloxy-propoxy)-phenyl]-3-(naphthalin-2-ylmethoxy)-piperidin-1-carbonsäure tert-butylester in 1 ml Essigester wurde mit 60 µl Morpholin versetzt und 3 Stunden lang unter Rückfluß gekocht. Anschließend wurde die Reaktionslösung mit 5 ml Essigester verdünnt und zweimal mit je 1 ml gesättigter Natriumhydrogencarbonat-Lösung extrahiert. Die organischen Phasen wurden über Magnesiumsulfat getrocknet, unter vermindertem Druck eingedampft und am Hochvakuum getrocknet. Das farblose Öl (169 mg) wurde an Kieselgel unter Verwendung von Essigester als Eluierungsmittel chromatographiert. Es wurden 142 mg (3RS,4RS)-4-[4-(3-Morpholin-4-yl-propoxy)-phenyl]-3-(naphthalin-2-ylmethoxy)-piperidin-1-carbonsäure tert-butylester in Form weisse Kristalle erhalten; MS : 561 $(M+H)^+$.

(b) In analoger Weise wie in Beispiel 22 (k) beschrieben, wurde durch Acylierung des (3RS,4RS)-4-[4-(3-Hydroxy-propoxy)-phenyl]-3-(naphthalin-2-ylmethoxy)-piperidin-1-carbonsäure tert-butylesters mit Pivalinsäurechlorid der (3RS,4RS)-4-[4-[3-(2,2-Dimethyl-propionyloxy)-propoxy]-phenyl]-3-(naphthalin-2-ylmethoxy)-piperidin-1-carbons ure tert-butylester als farbloses Öl erhalten; MS : 576 $(M+H)^+$.

(c) In analoger Weise wie in Beispiel 22 (k) beschrieben, wurde durch Acylierung des (3RS,4RS)-4-[4-(3-Hydroxy-propoxy)-phenyl]-3-(naphthalin-2-ylmethoxy)-piperidin-1-carbonsäure tert-butylesters mit Benzoylchlorid der (3RS,4RS)-4-[4-(3-Benzoyloxy-propoxy)-phenyl]-3-(naphthalin-2-ylmethoxy)-piperidin-1-carbonsäure tert-butylester als farbloses Öl erhalten; $R_f$: 0.84 ($SiO_2$, Hexan:Essigester=1:1).

(d) Ein Gemisch aus 1.0 g (3.41 mMol) (3RS,4RS)-3-Hydroxy-4-(4-hydroxy-phenyl)-piperidin-1-carbonsäure tert-butylester, 471 mg (34.1 mMol, 10 eq) Kaliumcarbonat und 607 ml (5.11 mMol, 1.5 eq) Benzylbromid in 30 ml Dimethylformamid wurde 2 Stunden lang bei Raumtemperatur und 15 Stunden lang bei 80° C gerührt. Anschließend wurden erneut 471 mg (34.1 mMol, 10 eq) Kaliumcarbonat und 607 ml (5.11 mMol, 1.5 eq) Benzylbromid zugegeben und für weitere 6 Stunden bei 80° C gerührt. Das auf Raumtemperatur abgekühlte Reaktionsgemisch wurde auf 300 ml einer Eis/Wasser-Mischung gegossen und dreimal mit 250 ml Essigester extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das gelbe Öl (1.82 g) wurde an Kieselgel mittels eines Laufmittelgradienten von 4:1 bis 3:2 eines Gemisches von Hexan und Essigester als Eluierungsmittel aufgetrennt. Es wurden 620 mg (72 % d.Th.) (3RS,4RS)-4-(4-Benzyloxy-phenyl)-3-hydroxy-piperidin-1-carbonsäure tert-butylester erhalten, das nach Umkristallisation aus einem Gemisch von Methylenchlorid und Hexan 361 mg (28 % d.Th.) als weisses, kristallines Produkt vorlag; MS : 383 $(M)^+$.

(e) In analoger Weise wie in Beispiel 22 (i) beschrieben, wurde durch Alkylierung des (3RS,4RS)-4-(4-Benzyloxy-phenyl)-3-hydroxy-piperidin-1-carbonsäure tert-butylesters mit 2-Brommethylnaphthalin der (3RS,4RS)-4-(4-Benzyloxy-phenyl)-3-(naphthalin-2-yloxy)-piperidin-1-carbonsäure tert-butylester als farbloser, amorpher Festkörper erhalten; MS : 523 $(M)^+$.

(f) In Analogie zu dem im Beispiel 14 beschriebenen Verfahren, wurde durch Alkylierung des (3RS,4RS)-3-Hydroxy-4-(4-hydroxy-phenyl)-piperidin-1-carbonsäure tert-butylesters mit 2-Brommethylnaphthalin in Gegenwart von Kaliumcarbonat der (3RS,4RS)-3-Hydroxy-4-[4-(naphthalin-2-ylmethoxy)-phenyl]-piperidin-1-carbonsäure tert-butylester als farbloser Festkörper erhalten; MS : 434 $(M+H)^+$.

(g) In Analogie zu dem im Beispiel 3 (c) beschriebenen Verfahren, wurde durch Alkylierung des (3RS,4RS)-3-Hydroxy-4-[4-(naphthalin-2-ylmethoxy)-phenyl]-piperidin-1-carbonsäure tert-butylesters mit 2-Methoxybenzylchlorid der (3RS,4RS)-3-(2-Methoxy-benzyloxy)-4-[4-(naphthalin-2-ylmethoxy)-phenyl]-piperidin-1-carbonsäure tert-butylester als farbloser Festkörper erhalten; MS : 554 $(M+H)^+$.

Beispiel 60

In analoger Weise wie in Beispiel 25 (b) beschrieben, wurden durch Abspaltung des 2,2,2-Trichloräthylcarbamats durch Behandlung mit Zink in Eisessig die folgenden Verbindungen erhalten:

1) - Aus (3RS, 4RS)-4-[4-(3-Carbamoyloxy-propoxy)-phenyl]-3-(naphthalin-2-ylmethoxy}-piperidin-1-carbonsäure 2,2,2-trichlor-äthylester der (3RS,4RS)-Carbaminsäure 3-{4-[3-(Naphthalin-2-ylmethoxy)-piperidin-4-yl]-phenoxy)-propylester als farbloser Festkörper; MS: 435 $(M+H)^+$;

2) - aus (3RS,4RS)-3-(Naphthalin-2-ylmethoxy)-4-{4-[3-(pyridin-2-ylcarbamoyloxy)-propoxy]-phenyl}-piperidin-1-carbonsäure 2,2,2-trichlor-äthylester der (3RS,4RS)-Pyridin-2-yl-carbaminsäure 3-{4-[3-(Naphthalin-2-ylmethoxy)-piperidin-4-yl]-phenoxy}-propylester als farbloses Öl; MS : 512 $(M+H)^+$;

3) - aus (3RS,4RS)-3-(Naphthalin-2-ylmethoxy)-4-[3-(2-pyridin-2-ylcarbamoyloxy-äthoxy]-phenyl]-piperidin-1-carbonsäure 2,2,2-trichlor-äthylester der Pyridin-2-yl-carbaminsäure (3RS,4RS)-2-[3-[3-(naphthalin-2-ylmethoxy)-piperidin-4-yl]-phenoxy]-äthylester als farbloses Öl; MS : 498 $(M+H)^+$;

4) - aus (3RS,4RS)-4-[3-(2-Carbamoyloxy-äthoxy)-phenyl]-3-(naphthalin-2-ylmethoxy)-piperidin-1-carbonsäure 2,2,2-trichlor-äthylester der Carbaminsäure (3RS,4RS)-2-[3-[3-(naphthalin-2-ylmethoxy)-piperidin-4-yl]-phenoxy]-äthylester als farbloser, amorpher Festkörper; MS : 421 $(M+H)^+$;

5) - aus dem Gemisch des (3RS,4RS)-3-(Naphthalin-2-ylmethoxy)-4-[3-[2-[(RS)- und - [(SR)-tetrahydro-pyran-2-yloxy]-äthoxy]-phenyl]-piperidin-1-carbonsäure 2,2,2-trichlor-äthylesters unter gleichzeitiger Abspaltung der THP-Gruppe das (3RS,4RS)-2-[3-[3-(Naphthalin-2-ylmethoxy)-piperidin-4-yl]-phenoxy]-äthanol als farbloser, amorpher Festkörper; MS : 378 $(M+H)^+$;

6) - aus (3RS,4RS)-4-[3-(2-Benzoyloxy-äthoxy)-phenyl]-3-(naphthalin-2-ylmethoxy)-piperidin-1-carbonsäure 2,2,2-trichloräthylester der Benzoesäure (3RS,4RS)-2-[3-(3-Naphthalin-2-ylmethoxy-piperidin-4-yl]-phenoxy]-äthylester als farbloser, amorpher Festkörper; MS : 482 $(M+H)^+$;

7) - aus (3RS,4RS)-3-(Naphthalin-2-ylmethoxy)-4-{3-[3-(pyridin-2-ylcarbamoyloxy)-propoxy]-phenyl}-piperidin-1-carbonsäure 2,2,2-trichloräthylester der (3RS,4RS)-Pyridin-2-yl-carbaminsäure 3-{3-[3-(Naphthalin-2-ylmethoxy)-piperidin-4-yl]-phenoxy}-propylester als farbloser, amorpher Festkörper; MS : 512 $(M+H)^+$;

8) - aus (3RS,4RS)-4-[3-(4-Benzoyloxy-butoxy)-phenyl]-3-(naphthalin-2-ylmethoxy)-piperidin-1-carbonsäure 2,2,2-trichlor-äthylester das Benzoesäure (3RS,4RS)-4-[3-[3-(naphthalin-2-yloxy)-piperidin-4-yl]-phenoxy]-butylester als farbloser, amorpher Festkörper; MS : 510 $(M+H)^+$.

Die als Ausgangsstoffe eingesetzten 2,2,2-Trichloräthylcarbamate wurden wie folgt hergestellt:

In analoger Weise wie in Beispiel 22 (a)-(c).Wos beschrieben, wurde wie folgt verfahren:

(a) Aus 1-Benzyl-4-piperidon und 3-Jodanisol wurde das 1-Benzyl-4-(3-methoxy-phenyl)-piperidin-4-ol als farbloser Festkörper erhalten; $R_f$: 0.18 ($SiO_2$, Methylenchlorid:Essigester=1:1). Die anschließende Elimination mittels p-Toluolsulfonsäure lieferte das 1-Benzyl-4-(3-methoxy-phenyl)-1,2,3,6-tetrahydro-pyridin als farblosen Festkörper; MS : 279 $(M)^+$. Die darauffolgende Hydroborierung ergab das 1-Benzyl-4-(3-methoxy-phenyl)-piperidin-3-ol als farbloses Pulver; MS : 297 $(M)^+$.

In analoger Weise wie in Beispiel 44 (d)-(i) beschrieben, wurde weiter wie folgt verfahren:

(b) Durch Spaltung des Methyläthers mittels Bortribromid in Methylenchlorid wurde aus dem 1-Benzyl-4-(3-methoxy-phenyl)-piperidin-3-ol das (3RS,4RS)-1-Benzyl-4-(3-hydroxy-phenyl)-piperidin-3-ol als hellgelber Festkörper erhalten; MS : 283 $(M)^+$. Die Alkylierung mit rac.-2-(2-Iod-äthoxy)-tetrahydro-pyran in Gegenwart von Kaliumcarbonat ergab ein Gemisch des (3RS,4RS)-1-Benzyl-4-[3-[2-[(RS)- und -[(SR)-tetrahydro-pyran-2-yloxy]-äthoxy]-phenyl]-piperidin-3-ols als farbloses Öl; MS : 410 $(M-H)^+$. In analoger Weise wie in Beispiel 22 (i) beschrieben, wurde durch anschließende Alkylierung mit 2-Brommethylnaphthalin ein Gemisch des (3RS,4RS)-1-Benzyl-3-(naphthalin-2-ylmethoxy)-4-[3-[2-[(RS)- und -[(SR)-tetrahydro-pyran-2-yloxy]-äthoxy]-phenyl]-piperidins als farbloses Öl erhalten; MS: 552 $(M)^+$. In analoger Weise wie in Beispiel 25 (a) beschrieben, wurde dann durch Abspaltung der Benzylgruppe mittels Chlorameisensäure-2,2,2-trichloräthylester und Kaliumcarbonat ein Gemisch des (3RS,4RS)-3-(Naphthalin-2-ylmethoxy)-4-[3-(2-[(RS)- und -[(SR)-tetrahydro-pyran-2-yloxy]-äthoxy]-phenyl]-piperidin-1-carbonsäure 2,2,2-trichlor-äthylesters erhalten; $R_f$: 0.60 ($SiO_2$, Methylenchlorid:Essigester=2:1). Durch die darauffolgende Abspaltung der THP-Gruppe mittels p-Toluolsulfonsäure wurde der (3RS,4RS)-4-[3-(2-Hydroxy-äthoxy)-phenyl]-3-(naphthalin-2-ylmethoxy)-piperidin-1-carbonsäure 2,2,2-trichlor-äthylester erhalten. In analoger Weise wie in Beispiel 24 (m) beschrieben, wurde schließlich durch Umsetzung mit Pyridin-2-carbonylazid der (3RS,4RS)-3-(Naphthalin-2-ylmethoxy)-4-[3-(2-pyridin-2-ylcarbamoyloxy-äthoxy]-phenyl]-piperidin-1-carbonsäure 2,2,2-trichlor-äthylester erhalten; $R_f$: 0.45 ($SiO_2$, Methylenchlorid:Essigester=9:1).

(c) In analoger Weise wie in Beispiel 44 (e) beschrieben, wurde durch Alkylierung des (3RS,4RS)-1-Benzyl-4-(4-hydroxy-phenyl)-piperidin-3-ols mit rac.-2-(3-Brom-propoxy)-tetrahydro-pyran das (3RS,4RS)-1-Benzyl-4-{4-[3-[(RS) und (SR)-tetrahydro-pyran-2-yloxy]-propoxy]-phenyl}piperidin-3-ol als farbloser Festkörper erhalten; MS : 426 $(M+H)^+$. Die anschließende Alkylierung mit 2-Brommethylnaphthalin, nach dem im Beispiel 12 (b) beschriebenen Verfahren ergab das (3RS,4RS)-1-Benzyl-3-(naphthalin-2-ylmethoxy)-4-{4-[3-[(RS)- und (SR)-tetrahydropyran-2-yloxy]-propoxy]-phenyl)-piperidin als farblosen Festkörper; MS : 566 $(M+H)^+$. Die weitere Umsetzung mit Chlorameisensäure 2,2,2-trichloräthylester, analog Beispiel 12 (c), lieferte den (3RS,4RS)-3-(Naphthalin-2-ylmethoxy)-4-{4-[3-[(RS)- und (SR)-tetrahydro-pyran-2-yloxy]-propoxy]-phenyl}-piperidin-1-carbonsäure 2,2,2-trichlor-äthylester als farblosen Festkörper; MS : 672 $(M+Na)^+$. Die Abspaltung der THP-Gruppe mittels p-Toluolsulfonsäure, analog Beispiel 44 (h), ergab den (3RS,4RS)-4-[4-(3-Hydroxy-propoxy)-phenyl]-3-(naphthalin-2-ylmethoxy)-piperidin-1-carbonsäure 2,2,2-trichlor-äthylester als farblosen Festkörper; MS : 583 $(M+NH_4)^+$. In analoger Weise wie in Beispiel 24 (m) beschrieben, wurde schließlich durch Umsetzung mit Natriumisocyanat der (3RS, 4RS)-4-[4-(3-Carbamoyloxy-propoxy)-phenyl]-3-(naphthalin-2-ylmethoxy)-piperidin-1-carbonsäure 2,2,2-trichlor-äthylester als farbloser Festkörper erhalten; MS : 609 $(M+H)^+$.

(d) In analoger Weise wie in Beispiel 24 (m) beschrieben, wurde durch Umsetzung des (3RS,4RS)-4-[4-(3-Hydroxy-propoxy)-phenyl]-3-(naphthalin-2-ylmethoxy)-piperidin-1-carbonsäure 2,2,2-trichlor-äthylesters mit Pyridin-2-carbonylazid das (3RS,4RS)-3-(Naphthalin-2-ylmethoxy)-4-{4-[3-(pyridin-2-ylcarbamoyloxy)-propoxy]-phenyl}piperidin-1-carbonsäure 2,2,2-trichlor-äthylester als farbloser Festkörper erhalten.

(e) In analoger Weise wie im Beispiel 24 (m) beschrieben, wurde durch Umsetzung des (3RS,4RS)-4-[3-(2-Hydroxy-äthoxy)-phenyl]-3-(naphthalin-2-ylmethoxy)-piperidin-1-carbonsäure 2,2,2-trichlor-äthylesters mit Natriumisocyanat der (3RS,4RS)-4-[3-(2-Carbamoyloxy-äthoxy)-phenyl]-3-(naphthalin-2-ylmethoxy)-piperidin-1-carbonsäure 2,2,2-trichlor-äthylester als farbloser Festkörper erhalten; $R_f$: 0.40 ($SiO_2$, Methylenchlorid: Methanol=9:1).

(f) In analoger Weise wie im Beispiel 22 (k) beschrieben, wurde durch Acylierung des (3RS,4RS)-4-[3-(2-Hydroxy-äthoxy)-phenyl]-3-(naphthalin-2-ylmethoxy)-piperidin-1-carbonsäure 2,2,2-trichlor-äthylesters mit Benzoylchlorid der (3RS,4RS)-4-[3-(2-Benzoyloxy-äthoxy)-phenyl]-3-(naphthalin-2-ylmethoxy)-piperidin-1-carbonsäure 2,2,2-trichloräthylester als farbloses Öl erhalten.

(g) In analoger Weise wie in Beispiel 14 beschrieben, wurde durch Alkylierung des (3RS,4RS)-1-Benzyl-4-(3-hydroxy-phenyl)-piperidin-3-ols mit rac.-2-(3-Brom-propoxy)-tetrahydro-pyran in Gegenwart von Kaliumcarbonat ein Gemisch der diastereomeren Racemate des (3RS,4RS)-1-Benzyl-4-{3-[3-(tetrahydro-pyran-2-yloxy)-propoxy]-phenyl}-piperidin-3-ols als farbloses Öl erhalten; $R_f$: 0.38 (Hexan:Aceton=1:1). Die anschließende Alkylierung mit 2-Brommethylnaphthalin, analog zu Beispiel 12 (b), ergab ein Gemisch der diastereomeren Racemate des (3RS, 4RS)-1-Benzyl-3-(naphthalin-2-ylmethoxy)-4-{3-[3-(tetrahydropyran-2-yloxy)-propoxy]-phenyl}-piperidins als farbloses Öl; MS : 566 $(M+H)^+$. Die darauffolgende Umsetzung mit Chlorameisensäure 2,2,2-trichloäthylester, analog zum Beispiel 12 (c), lieferte ein Gemisch der diastereomeren Racemate des (3RS,4RS)-3-(Naphthalin-2-ylmethoxy)-4-{3-[3-(tetrahydro-pyran-2-yloxy)-propoxy]-phenyl}-piperidin-1-carbonsäure 2,2,2-trichloräthylesters, das ohne weitere Reinigung und Charakterisierung mit p-Toluolsulfonsäure, analog zum Beispiel 53 (c), zum (3RS, 4RS)-4-[3-(3-Hydroxy-propoxy)-phenyl]-3-(naphthalin-2-ylmethoxy)-piperidin-1-carbonsäure 2,2,2-trichloräthylester umgesetzt wurde. Schließlich wurde durch Umsetzung mit Pyridin-2-carbonylazid, in analoger Weise wie im Beispiel 24 (m) beschrieben, der (3RS,4RS)-3-(Naphthalin-2-ylmethoxy)-4-{3-[3-(pyridin-2-ylcarbamoyloxy)-propoxy]-phenyl}-piperidin-1-carbonsäure 2,2,2-trichloräthylester als farbloses Öl erhalten; $R_f$: 0.55 (Methylenchlorid: Essigester=1:1).

(h) In analoger Weise wie im Beispiel 14 beschrieben, wurde durch Alkylierung des (3RS,4RS)-1-Benzyl-4-(3-hydroxy-phenyl)-piperidin-3-ols mit rac.-2-(4-Brom-butoxy)-tetrahydro-pyran [S.W.Baldwin et al., J.Org.Chem. 1985, 50, 4432-4439] und weiter wie unter (g) beschrieben, der (3RS,4RS)-4-[3-(4-Hydroxy-butoxy)-phenyl]-3-(naphthalin-2-ylmethoxy)-piperidin-1-carbonsäure 2,2,2-trichlor-äthylester als farbloser Festkörper erhalten; $R_f$: 0.50 (Methylenchlorid:Essigester=9:1). Die anschließende Acylierung mit Benzoylchlorid, analog zum Beispiel 22 (k), lieferte den (3RS,4RS)-4-[3-(4-Benzoyloxy-butoxy)-phenyl]-3-(naphthalin-2-ylmethoxy)-piperidin-1-carbonsäure 2,2,2-trichlor-äthylester als farblosen Festkörper; $R_f$: 0.85 ($SiO_2$, Methylenchlorid:Essigester=95:5)

<u>Beispiel 61</u>

In Analogie zu dem in Beispiel 10 (b) beschriebenen Verfahren, wurden durch Abspaltung der BOC-Gruppe mittels Zinkbromid in Methylenchlorid die folgenden Verbindungen erhalten:

1) - Aus (3RS,4RS)-3-(Naphthalin-2-ylmethoxy)-4-[4-(2-oxo-2-phenyl-äthoxy)-phenyl]-piperidin-1-carbonsäure tert-butylester das (3RS,4RS)-2-[4-[3-(Naphthalin-2-yloxy)-piperidin-4-yl]-phenoxy]-1-phenyl-äthanon Hydrobromid als farbloser Festkörper; MS : 452 $(M+H)^+$;

2) - aus (3RS,4RS)-4-[4-(2-Benzoyloxy-äthoxy)-phenyl]-3-(2-methoxybenzyloxy)-piperidin-1-carbonsäure tert-butylester das Benzoesäure (3RS,4RS)-2-[4-[3-(2-methoxy-benzyloxy)-piperidin-4-yl)-phenoxy]-äthylester Hydrobromid als beigefarbener Festkörper; MS : 462 $(M+H)^+$;

3) - aus (3RS,4RS)-4-[4-([1,3]Dioxolan-2-ylmethoxy)-phenyl]-3-(naphthalin-2-ylmethoxy)-piperidin-1-carbonsäure tert-butylester das (3RS,4RS)-4-(4-[1,3]Dioxolan-2-ylmethoxy-phenyl)-3-(naphthalin-2-ylmethoxy)-piperidin als gelblicher Festkörper; MS : 420 $(M+H)^+$.

Die als Ausgangsstoffe eingesetzten BOC-Derivate wurden wie folgt erhalten:

(a) In analoger Weise zu dem im Beispiel 14 beschriebenen Verfahren wurde durch Alkylierung des (3RS,4RS)-3-Hydroxy-4-(4-hydroxyphenyl)-piperidin-1-carbonsäure tert-butylesters mit Allylbromid in Gegenwart von Kaliumcarbonat der (3RS,4RS)-4-(4-Allyloxy-phenyl)-3-hydroxy-piperidin-1-carbonsäure tert-butylester als farbloser

Festkörper erhalten; Smp.: 113 °C (Hexan).

(b) In Analogie zu dem in Beispiel 3 (c) beschriebenen Verfahren wurde durch Alkylierung des (3RS,4RS)-4-(4-Allyloxy-phenyl)-3-hydroxypiperidin-1-carbonsäure tert-butylesters mit 2-Brommethylnaphthalin der (3RS,4RS)-4-(4-Allyloxy-phenyl)-3-(naphthalin-2-ylmethoxy)-piperidin-1-carbonsäure tert-butylester als farbloses Öl erhalten; MS: 474 $(M+H)^+$.

(c) Ein Gemisch von 400 mg (0.8 mMol) (3RS,4RS)-4-(4-Allyloxy-phenyl)-3-(naphthalin-2-ylmethoxy)-piperidin-1-carbonsäure tert-butylester, 0.2 ml Triäthylamin, 0.5 ml Wasser und 78 mg Tris-(triphenylphosphin)-rhodium(I) chlorid in 10 ml Äthanol wurde 1 Stunde lang unter Rückfluß gerührt. Der zum Teil gebildete (3RS,4RS)-4-(4-Hydroxyphenyl)-3-(naphthalin-2-ylmethoxy)-piperidin-1-carbonsäure tert-butylester (30% d.Th.), MS : 434 $(M+H)^+$, wurde chromatographisch abgetrennt und in der folgenden Stufe eingesetzt.

(d) In analoger Weise zu dem im Beispiel 14 beschriebenen Verfahren wurde durch Alkylierung des (3RS,4RS)-4-(4-Hydroxy-phenyl)-3-(naphthalin-2-ylmethoxy)-piperidin-1-carbonsäure tert-butylesters mit Phenacylbromid in Gegenwart von Kaliumcarbonat der (3RS,4RS)-3-(Naphthalin-2-ylmethoxy)-4- [4-(2-oxo-2-phenyl-äthoxy)-phenyl]-piperidin-1-carbonsäure tert-butylester als gelblicher Festkörper erhalten; MS : 551 $(M)^+$.

(e) In Analogie zu dem in Beispiel 3 (c) beschriebenen Verfahren wurde durch Alkylierung eines Gemisches des (3RS,4RS)-3-Hydroxy-4-[4-[2-[(RS)- und -[(SR)-tetrahydro-pyran-2-yloxy]-äthoxy]-phenyl]-piperidin-1- 1-carbonsäure tert-butylesters [Beispiel 53 (a)] mit 2-Methoxybenzylchlorid der (3RS,4RS)-3-(2-Methoxy-benzyloxy)-4-{4-[2-[(RS)- und [(SR)-tetrahydro-pyran-2-yloxy]-äthoxy]-phenyl}-piperidin-1-carbonsäure tert-butylester als farbloses Öl erhalten.

(f) In Analogie zu dem in Beispiel 53 (c) beschriebenen Verfahren wurde durch Spaltung des THP-Äthers aus dem (3RS,4RS)-3-(2-Methoxy-benzyloxy)-4-{4-[2-[(RS)- und [(SR)-tetrahydro-pyran-2-yloxy]-äthoxy]-phenyl}-piperidin-1-carbonsäure tert-butylester der (3RS,4RS)-4-[4-(2-Hydroxy-äthoxy)-phenyl]-3-(2-methoxy-benzyloxy)-piperidin-1-carbonsäure tert-butylester erhalten.

(g) In Analogie zu dem in Beispiel 22 (k) beschriebenen Verfahren wurde durch Acylierung des (3RS,4RS)-4-[4-(2-Hydroxy-äthoxy)-phenyl]-3-(2-methoxy-benzyloxy)-piperidin-1-carbonsäure tert-butylesters der (3RS, 4RS)-4-[4-(2-Benzoyloxy-äthoxy)-phenyl]-3-(2-methoxy-benzyloxy)-piperidin-1-carbonsäure tert-butylester als farblose, viskose Flüssigkeit erhalten; MS : 562 $(M+H)^+$.

(h) In analoger Weise zu dem im Beispiel 14 beschriebenen Verfahren wurde durch Alkylierung des (3RS,4RS)-3-Hydroxy-4-(4-hydroxyphenyl)-piperidin-1-carbonsäure tert-butylesters mit 2-Brommethyl-1,3-dioxolan in Gegenwart von Kaliumcarbonat der (3RS,4RS)-4-[4-([1,3]Dioxolan-2-ylmethoxy)-phenyl]-3-hydroxy-piperidin-1-carbonsäure tert-butylester als farbloser Festkörper erhalten; Smp.: 136-137 °C (Hexan).

(i) In Analogie zu dem in Beispiel 3 (c) beschriebenen Verfahren wurde durch Alkylierung des (3RS,4RS)-4-[4-([1,3] Dioxolan-2-ylmethoxy)-phenyl]-3-hydroxy-piperidin-1-carbonsäure tert-butylesters mit 2-Brommethylnaphthalin der (3RS,4RS)-4-[4-([1,3]Dioxolan-2-ylmethoxy)-phenyl]-3-(naphthalin-2-ylmethoxy)-piperidin-1-carbonsäure tert-butylester als farbloser Festkörper erhalten; MS : 520 $(M+H)^+$.

Beispiel 62

Eine Lösung von 210 mg (0.425 mMol) (3RS,4RS,5SR)-4-(4-Chlorphenyl)-3-naphthalin-2-ylmethoxy-5-propyl-piperidin-1-carbonsäure tert-butylester in 18 ml Methanol wurde mit 12 ml 1 N Salzsäure versetzt und über Nacht bei 50° C gerührt. Anschließend wurde die Lösung unter vermindertem Druck eingedampft, der Rückstand in warmem Toluol aufgenommen und wiederum eingedampft, wobei das Produkt auszufallen begann. Es wurden 153 mg (84% d. Th.) (3RS,4RS,5SR)-4-(4-Chlor-phenyl)-3-naphthalin-2-ylmethoxy-5-propylpiperidin Hydrochlorid als farbloser Festkörper erhalten. MS 252 $(M\text{-}Naphthylmethyl)^+$.

Der als Ausgangsverbindung eingesetzte (3RS,4RS,5SR)-4-(4-Chlor-phenyl)-3-naphthalin-2-ylmethoxy-5-propyl-piperidin-1-carbonsäure tert-butylester wurde wie folgt hergestellt:

(a) In analoger Weise zu dem von A.Ziering et al. in J.Org.Chem. 22, 1521-1528 (1957) beschriebenen Verfahren zur Herstellung von Piperidin-4-onen aus den entsprechenden Acrylsäureestern durch Umsetzung mit Methylamin oder Benzylamin, Reaktion mit Acrylsäureäthylester oder -methylester, Cyclisierung und schließlich Decarbalkoxylierung wurde ausgehend von 2-Propyl-acrylsäure-äthylester und Methylamin das (3RS)-1-Methyl-3-propyl-piperidin-4-on als farbloses Öl erhalten; $R_f$: 0.38 ($SiO_2$, Methylenchlorid:Methanol= 95:5).

(b) Zu einer auf -78° C gekühlten Lösung von 25.68 g (134 mMol) 1-Brom-4-chlor-benzol in 250 ml tert-Butylmethyläther wurden innerhalb von 30 Minuten 83.8 ml (134 mMol) n-BuLi (1.6 N in Hexan) getropft. Nach beendeter Zugabe wurde 1 Stunde lang bei -78° C nachgerührt. Danach wurde eine Lösung von 10.41 g (67.05 mMol) (3RS)-1-Methyl-3-propylpiperidin-4-on in 100 ml tert-Butylmethyläther bei -70 bis -65° C zugetropft. Nach dem Zutropfen wurde 2 Stunden lang bei -78° C gerührt. Zur Aufarbeitung wurde das Reaktionsgemisch auf Eis gegossen, das Gemisch in einen Scheidetrichter überführt und die organische Phase abgetrennt. Die wäßrige Phase wurde mit Essigester extrahiert, und anschließend wurden die vereinigten organischen Phasen über Natriumsulfat getrocknet und unter vermindertem Druck eingedampft. Zur Reinigung wurde das Rohprodukt an Kieselgel unter Verwendung eines 95:5:0.1-Gemisches von Methylenchlorid, Methanol und Ammoniak als Eluierungsmittel chromatographiert. Es wurden 15.9 g (88% d.Th.) (3RS,4RS)- und (3RS,4SR)-4-(4-Chlor-phenyl)-1-methyl-3-propyl-piperidin-4-ol als farbloser Festkörper erhalten; MS : 267 $(M)^+$.

(c) Eine Lösung von 13.68 g (51.06 mMol) (3RS,4RS)- und (3RS,4SR)-4-(4-Chlor-phenyl)-1-methyl-3-propyl-piperidin-4-ol in 67 ml Trifluoressigsäure wurde 18 Stunden lang unter Rückfluß gekocht. Danach wurde die Reaktionslösung unter vermindertem Druck eingedampft. Der Rückstand wurde zwischen einer gesättigten Natriumcarbonat-Lösung und Äther verteilt, die abgetrennte wäßrige Phase mit Äther nachextrahiert und schließlich die vereinigten organischen Phasen über Natriumsulfat getrocknet und unter vermindertem Druck eingedampft. Zur Reinigung des Rohprodukts und Auftrennung der isomeren Olefine wurde an Kieselgel unter Verwendung eines 96:4-Gemisches von Methylenchlorid und Methanol als Eluierungsmittel chromatographiert. Es wurden 9.71 g (76% d.Th.) (RS)-4-(4-Chlor-phenyl)-1-methyl-3-propyl-1,2,3,6-tetrahydro-pyridin, MS : 249 $(M)^+$, und 1.74 g 4-(4-Chlor-phenyl)-1-methyl-5-propyl-1,2,3,6-tetrahydro-pyridin, MS : 248 $(M-H)^+$, jeweils als gelbliches Öl erhalten.

(d) Zu einer Suspension von 9.7 g (39 mMol) (RS)-4-(4-Chlor-phenyl)-1-methyl-3-propyl-1,2,3,6-tetrahydro-pyridin in 80 ml 1,2-Dimethoxyäthan wurden spatelweise 2.23 g (60 mMol) Natriumborhydrid gegeben, so daß die Temperatur nicht über 35° C stieg. Anschließend wurden während 45 Minuten 13.2 ml Bortrifluorid-Ätherat, gelöst in 15 ml 1,2-Dimethoxyäthan zugetropft und danach das Reaktionsgemisch 2 Stunden lang bei Raumtemperatur gerührt. In der Folge wurde zunächst eine Lösung von 15.65 g (277 mMol) Kaliumhydroxid, gelöst in 60 ml Wasser, bei ca. 30° C langsam zugetropft und danach innerhalb von 15 Minuten 11.22 ml einer 30%igen Wasserstoffperoxid-Lösung, wobei die Temperatur auf 40° C anstieg. Anschließend wurde 2.5 Stunden lang unter Rückfluß gekocht. Zur Aufarbeitung wurde das abgekühlte Reaktionsgemisch über Dicalit filtriert und dieses mit Essigester nachgewaschen. Die erhaltene Lösung wurde mit je 100 ml Essigester und Wasser versetzt, die organische Phase abgetrennt und dann die wäßrige Phase mit 100 ml Essigester nachextrahiert. Die vereinigten organischen Phasen wurden über Natriumsulfat getrocknet und unter vermindertem Druck eingedampft. Zur Reinigung des Rohprodukts wurde an Kieselgel unter Verwendung eines 95:5-Gemisches von Methylenchlorid und Methanol als Eluierungsmittel chromatographiert. Es wurden 8.64 g (73% d.Th.) (3RS,4RS,5SR)-4-(4-Chlor-phenyl)-1-methyl-5-propyl-piperidin-3-ol als farbloser Festkörper erhalten; MS : 267 $(M)^+$.

(e) Das Gemisch von 7.19 g (26.85 mMol) (3RS,4RS,5SR)-4-(4-Chlorphenyl)-1-methyl-5-propyl-piperidin-3-ol, 5.96 g (80.7 mMol) Lithiumcarbonat und 14.22 g (67.1 mMol) Chlorameisensäure-2,2,2-trichloräthylester in 200 ml Toluol wurde während 8 Stunden bei 105° C gerührt. Zur Aufarbeitung wurde das abgekühlte Reaktionsgemisch mit wäßriger Natriumcarbonat-Lösung und Essigester versetzt. Die organische Phase wurde abgetrennt, über Natriumsulfat getrocknet und unter vermindertem Druck eingedampft. Zur Reinigung des Rohprodukts wurde an Kieselgel unter Verwendung eines 1:1-Gemisches von Methylenchlorid und Hexan als Eluierungsmittel chromatographiert. Es wurden 13.05 g (80% d.Th.) (3RS,4SR,5SR)-4-(4-Chlor-phenyl)-3-propyl-5-(2,2,2-trichlor-äthoxycarbonyloxy)-piperidin-1-carbonsäure-2,2,2-trichlor-äthylester als gelbliches Öl erhalten; MS : 621 $(M+NH_4)^+$.

(f) Das Gemisch von 13.05 g (21.6 mMol) (3RS,4SR,5SR)-4-(4-Chlorphenyl)-3-propyl-5-(2,2,2-trichlor-äthoxycarbonyloxy)-piperidin-1-carbonsäure 2,2,2-trichlor-äthylester und 14.5 g Zink in 200 ml Eiessig wurde während 15 Stunden im Ultraschallbad behandelt. Um die Reaktion zu vervollständigen wurden anschließend nochmals 5 g Zink hinzugefügt und weitere 5 Stunden im Ultraschallbad belassen. Zur Aufarbeitung wurde das Zink abgesaugt, der Rückstand mit Eisessig nachgewaschen und die Lösung unter vermindertem Druck zur Trockene eingedampft. Der Rückstand wurde zwischen 1 N NaOH und Essigester verteilt, dann die abgetrennte wäßrige Phase nochmals mit Essigester extrahiert und schließlich die vereinigten organischen Phasen unter vermindertem Druck eingedampft. Das erhaltene Rohprodukt wurde aus Diäthyläther kristallisiert und ergab 3.0 g (55% d.Th.) (3RS,4RS, 5SR)-4-(4-Chlor-phenyl)-5-propyl-piperidin-3-ol als farblose Kristalle; MS : 253 $(M)^+$. Zur Reinigung der Mutterlauge wurde an Kieselgel unter Verwendung eines 90:10:0.1-Gemisches von Methylenchlorid, Methanol und Ammoniak als Eluierungsmittel chromatographiert. Es wurden weitere 1.1 g (20% d.Th.) (3RS,4RS,5SR)-4-(4-Chlorphenyl)-5-propyl-piperidin-3-ol erhalten.

(g) Eine Lösung von 3.05 g (12.0 mMol) (3RS,4RS,5SR)-4-(4-Chlorphenyl)-5-propyl-piperidin-3-ol in 20 ml Dimethylformamid wurde bei 0° C mit 1.34 g (13.2 mMol) Triäthylamin und 3.02 g (13.8 mMol) Di-tert-butyldicarbonat versetzt und während 15 Stunden bei Raumtemperatur gerührt. Anschließend wurde das Dimethylformamid im Ölpumpenvakuum abdestilliert und zur Reinigung des Rückstandes an Kieselgel unter Verwendung eines 99: 1-Gemisches von Methylenchlorid und Methanol als Eluierungsmittel chromatographiert. Es wurden 3.92 g (92% d.Th.) (3RS,4RS,5SR)-4-(4-Chlor-phenyl)-3-hydroxy-5-propyl-piperidin-1-carbonsäure tert-butylester als farbloser Festkörper erhalten; MS : 297 $(M-C_4H_8)^+$.

(h) Zu einer Lösung von 200 mg (0.56 mMol) (3RS,4RS,5SR)-4-(4-Chlorphenyl)-3-hydroxy-5-propyl-piperidin-1-carbonsäure tert-butylester und 188 mg (0.85 mMol) 2-Brommethylnaphthalin in 10 ml Dimethylformamid wurden 37 mg (0.85 mMol) Natriumhydrid (55%ige Dispersion in Weißöl) gegeben und die Reaktionsmischung 5 Stunden lang bei Raumtemperatur gerührt. Zur Aufarbeitung wurde das Reaktionsgemisch im Ölpumpenvakuum eingedampft, der Rückstand zwischen Wasser und Äther verteilt und danach die abgetrennte Wasserphase fünfmal mit je 50 ml Äther ausgeschüttelt. Die vereinigten Ätherextrakte wurden mit gesättigter Natriumchlorid-Lösung gewaschen, über Natriumsulfat getrocknet und unter vermindertem Druck eingedampft. Zur Reinigung des Rohproduktes wurde an Kieselgel unter Verwendung eines 96:4-Gemisches von Toluol und Essigester als Eluierungsmittel chromatographiert. Es wurden 215 mg (77% d.Th.) (3RS,4RS,5SR)-4-(4-Chlor-phenyl)-3-naphthalin-2-ylmethoxy-5-propyl-piperidin-1-carbonsäure tert-butylester als farbloses Öl erhalten; MS : 494 $(M+H)^+$.

Beispiel 63

In analoger Weise wie in Beispiel 62 beschrieben, wurden die folgenden Verbindungen erhalten:

1) - Aus (3RS,4RS,5SR)-4-(4-Chlor-phenyl)-3-(4-methoxy-benzyloxy)-5-propyl-piperidin-1-carbonsäure tert-butylester das (3RS,4RS,5SR)-4-(4-Chlor-phenyl)-3-(4-methoxy-benzyloxy)-5-propyl-piperidin als gelbliches Öl; MS : 252 $(M\text{-Methoxybenzyl})^+$;
2) - aus (3RS,4RS,5SR)-4-(4-Chlor-phenyl)-3-(1-äthyl-1H-benzimidazol-2-ylmethoxy)-5-propyl-piperidin-1-carbonsäure tert-butylester das (3RS,4RS,5SR)-4-(4-Chlor-phenyl)-3-(1-äthyl-1H-benzimidazol-2-ylmethoxy)-5-propyl-piperidin Hydrochlorid als farbloser Festkörper, MS : 412 $(M+H)^+$.

Die als Ausgangssubstanzen eingesetzten BOC-Derivate wurden wie folgt erhalten:
In analoger Weise wie in Beispiel 62 (h) beschrieben, wurde durch Alkylierung des (3RS,4RS,5SR)-4-(4-Chlor-phenyl)-3-hydroxy-5-propylpiperidin-1-carbonsäure tert-butylesters mit 4-Methoxybenzylchlorid der (3RS,4RS,5SR)-4-(4-Chlor-phenyl)-3-(4-methoxy-benzyloxy)-5-propylpiperidin-1-carbonsäure tert-butylester als farbloser Festkörper erhalten; MS : 416 $(M-C_4H_9)^+$.
In analoger Weise wie in Beispiel 62 (h) beschrieben, wurde durch Alkylierung von (3RS,4RS,5SR)-4-(4-Chlor-phenyl)-3-hydroxy-5-propylpiperidin-1-carbonsäure tert-butylester mit 2-Chlormethyl-1-äthyl-1Hbenzoimidazol [Acta Pol. Pharm. 1977, 34(4), 359-369] der (3RS,4RS,5SR)-4-(4-Chlor-phenyl)-3-(1-äthyl-1H-benzimidazol-2-ylmethoxy)-5-propylpiperidin-1-carbonsäure tert-butylester als farbloses Öl erhalten; MS : 495 $(M+H)^+$.

Beispiel 64

In analoger Weise wie in Beispiel 5 beschrieben, wurde durch Behandlung von (3RS,4RS,5SR)-3-(4-Benzyloxy-naphthalin-2-ylmethoxy)-4-(4-fluor-phenyl)-5-propyl-piperidin-1-carbonsäure 2-trimethylsilanyläthylester mit Tetrabutylammoniumfluorid-Lösung das (3RS,4RS,5SR)-3-(4-Benzyloxy-naphthalin-2-ylmethoxy)-4-(4-fluorphenyl)-5-propyl-piperidin als fast farbloser Festkörper erhalten; MS : 484 $(M+H)^+$.
Der als Ausgangssubstanz eingesetzte (3RS,4RS,5SR)-3-(4-Benzyloxy-naphthalin-2-ylmethoxy)-4-(4-fluorphenyl)-5-propylpiperidin-1-carbonsäure 2-trimethylsilanyläthylester wurde wie folgt erhalten:

(a) In analoger Weise wie in Beispiel 62 (a)-(d) beschrieben, wurde ausgehend von (RS)-1-Benzyl-3-propyl-piperidin-4-on, MS : 231 $(M)^+$, und 1-Brom-4-fluorbenzol das (3RS,4RS,5SR)-1-Benzyl-4-(4-fluorphenyl)-5-propylpiperidin-3-ol als farbloser Festkörper erhalten; MS : 327 $(M)^+$.

(b) In analoger Weise wie in Beispiel 62 (h) beschrieben, wurde durch Alkylierung des (3RS,4RS,5SR)-1-Benzyl-4-(4-fluor-phenyl)-5-propylpiperidin-3-ols mit 1-Benzyloxy-3-chlormethyl-naphthalin das (3RS,4RS,5SR)-1-Benzyl-3-(4-benzyloxy-naphthalin-2-ylmethoxy)-4-(4-fluor-phenyl)-5-propyl-piperidin als gelbes Harz erhalten; MS (ISP): 574 $(M+H)^+$.

(c) In analoger Weise wie in Beispiel 1 (d) beschrieben, wurde durch Umsetzung von (3RS,4RS,5SR)-1-Benzyl-3-(4-benzyloxy-naphthalin-2-ylmethoxy)-4-(4-fluor-phenyl)-5-propyl-piperidin mit Chlorameisensäure-β-trimethylsilyläthylester [Synthesis 346 (1987)] der (3RS,4RS,5SR)-3-(4-Benzyloxy-naphthalin-2-ylmethoxy)-4-(4-fluorphenyl)-5-propyl-piperidin-1-carbonsäure 2-trimethylsilanyläthylester als hellgelber Sirup erhalten; MS: 628 $(M+H)^+$.

Das als Ausgangsverbindung eingesetzte 1-Benzyloxy-3-chlormethyl-naphthalin wurde wie folgt erhalten:

(a) In analoger Weise wie in Beispiel 14 (a) beschrieben, wurde durch Alkylierung des 4-Hydroxy-naphthalin-2-carbonsäureäthylesters [J.Agric.Chem Soc.Japan 24, 313 (1950)] mit Benzylbromid der 4-Benzyloxy-naphthalin-2-carbonsäure äthylester als fast farbloser Festkörper erhalten; $R_f$: 0.53 ($SiO_2$, Hexan:Essigester=4:1).

(b) Die Reduktion des 4-Benzyloxy-naphthalin-2-carbonsäure äthylesters, analog zu Beispiel 7 (b), lieferte das (4-Benzyloxynaphthalin-2-yl)-methanol als farblosen Festkörper; $R_f$: 0.42 ($SiO_2$, Hexan:Essigester=2:1).

(c) Die Chlorierung des (4-Benzyloxy-naphthalin-2-yl)-methanols, analog zu Beispiel 7 (c), lieferte das 1-Benzyloxy-3-chlormethylnaphthalin als farblosen Festkörper; MS : 282 $(M)^+$.

Beispiel 65

In analoger Weise wie in Beispiel 62 beschrieben, wurden die folgenden Verbindungen hergestellt:

1) - Aus (3RS,4RS,5SR)-4-(4-Chlor-phenyl)-5-isopropyl-3-naphthalin-2-ylmethoxy-piperidin-1-carbonsäure tert-butylester das (3RS,4RS,5SR)-4-(4-Chlor-phenyl)-5-isopropyl-3-(naphthalin-2-ylmethoxy)-piperidin Hydrochlorid als farbloser Festkörper; MS : 394 $(M+H)^+$;

2) - aus (3RS,4RS,5SR)-4-(4-Brom-phenyl)-5-isobutyl-3-naphthalin-2-ylmethoxy-piperidin-1-carbonsäure tert-butylester das (3RS,4RS,5SR)-4-(4-Brom-phenyl)-5-isobutyl-3-naphthalin-2-ylmethoxy-piperidin Hydrochlorid als farbloser Festkörper; MS : 310 $(M\text{-Naphthylmethyl})^+$;

3) - aus (3RS,4RS,5SR)-4-(4-Fluor-phenyl)-5-methyl-3-naphthalin-2-ylmethoxy-piperidin-1-carbonsäure tert-butylester das (3RS,4RS,5SR)-4-(4-Fluor-phenyl)-5-methyl-3-naphthalin-2-ylmethoxy-piperidin Hydrochlorid als farbloser Festkörper; $R_f$: 0.37 ($SiO_2$, Methylenchlorid:Methanol: Ammoniak=95:5:0.1);

4) - aus (3RS,4RS,5SR)-5-Benzyl-4-(4-fluor-phenyl)-3-(naphthalin-2-ylmethoxy)-piperidin-1-carbonsäure tert-butylester das (3RS,4RS,5SR)-5-Benzyl-4-(4-fluor-phenyl)-3-naphthalin-2-ylmethoxy-piperidin Hydrochlorid als farbloser Festkörper; MS : 426 $(M+H)^+$.

Die als Ausgangssubstanzen eingesetzten BOC-Derivate wurden wie folgt erhalten:
In analoger Weise wie in Beispiel 62 (a)-(h) beschrieben, wurde wie folgt verfahren:

(a) Ausgehend von 2-Isopropyl-acrylsäureäthylester und Methylamin wurde das (3RS)-1-Methyl-3-isopropyl-piperidin-4-on als farbloses Öl erhalten; MS : 155 $(M)^+$ . Durch Umsetzung mit 1-Brom-4-chlorbenzol wurde das (3RS,4RS)- und (3RS,4SR)-4-(4-Chlor-phenyl)-3-isopropyl-1-methyl-piperidin-4-ol als farbloser Festkörper erhalten; MS : 267 $(M)^+$. Die folgende Elimination mittels Trifluoressigsäure und anschließende chromatographische Trennung ergab die beiden isomeren Olefine, das (RS)-4-(4-Chlor-phenyl)-3-isopropyl-1-methyl-1,2,3,6-tetrahydro-pyridin, MS : 249 $(M)^+$, und das 4-(4-Chlor-phenyl)-5-isopropyl-1-methyl-1,2,3,6-tetrahydro-pyridin, jeweils als farbloses Öl. Die Hydroborierung des (RS)-4-(4-Chlor-phenyl)-3-isopropyl-1-methyl-1,2,3,6-tetrahydro-pyridins ergab das (3RS,4RS,5SR)-4-(4-Chlor-phenyl)-5-isopropyl-1-methylpiperidin-3-ol als farblosen Festkörper; MS : 267 $(M)^+$. Die weitere Umsetzung mit Chlorameisensäure-2,2,2-trichloräthylester lieferte den (3SR,4RS,5RS)-4-(4-Chlor-phenyl)-3-isopropyl-5-(2,2,2-trichlor-äthoxycarbonyloxy)-piperidin-1-carbonsäure 2,2,2-trichlor-äthylester als gelbliches Öl; MS : 619, 621, 623, 625 $(M+NH_4)^+$. Die Abspaltung der TROC-Gruppe mit Zink in Eisessig ergab das (3RS,4RS,5SR)-4-(4-Chlorphenyl)-5-isopropyl-piperidin-3-ol als farblosen Festkörper: MS : 253 $(M)^+$. Durch Einführung der BOC-Gruppe wurde daraus der (3RS,4RS,5SR)-4-(4-Chlor-phenyl)-3-hydroxy-5-isopropyl-piperidin-1-carbonsäure tert-butylester als farbloser Festkörper erhalten; MS : 297 $(M-C_4H_8)^+$. Die Alkylierung

schließlich mit 2-Brommethylnaphthalin lieferte den (3RS,4RS,5SR)-4-(4-Chlor-phenyl)-5-isopropyl-3-naphthalin-2-ylmethoxy-piperidin-1-carbonsäure tert-butylester als farblosen Festkörper; MS : 437 $(M-C_4H_8)^+$.

In analoger Weise wie in Beispiel 62 (a)-(h) beschrieben, wurde wie folgt verfahren:

(b) Ausgehend von 2-Isobutyl-acrylsäuremethylester und Benzylamin wurde das (3RS)-1-Benzyl-3-isobutyl-piperidin-4-on als gelbliches Öl erhalten; MS : 245 $(M)^+$. Durch Umsetzung mit 1,4-Dibrombenzol und anschließende Elimination mittels Trifluoressigsäure sowie chromatographische Trennung wurden die beiden isomeren Olefine, das (RS)-1-Benzyl-4-(4-brom-phenyl)-3-isobutyl-1,2,3,6-tetrahydropyridin, MS : 383 $(M)^+$, und das 1-Benzyl-4-(4-brom-phenyl)-5-isobutyl-1,2,3,6-tetrahydro-pyridin, jeweils als bräunliches Öl erhalten. Die darauffolgende Hydroborierung des (RS)-1-Benzyl-4-(4-brom-phenyl)-3-isobutyl-1,2,3,6-tetrahydro-pyridins ergab das (3RS, 4RS,5SR)-1-Benzyl-4-(4-brom-phenyl)-5-isobutyl-piperidin-3-ol als farblosen Festkörper; MS : 401 $(M)^+$. Die weitere Umsetzung mit Chlorameisensäure-2,2,2-trichloräthylester lieferte den (3RS,4RS,5SR)-4-(4-Brom-phenyl-5-isobutyl-3-(2,2,2-trichlor-äthoxycarbonyloxy)-piperidin-1-carbonsäure 2,2,2-trichlor-äthylester als farblosen Festkörper; $R_f$: 0.25 ($SiO_2$, Methylenchlorid:Hexan=1:1). Die Abspaltung der TROC-Gruppe mit Zink in Eisessig ergab das (3RS,4RS,5SR)-4-(4-Brom-phenyl)-5-isobutylpiperidin-3-ol als farblosen Festkörper: MS : 311 $(M)^+$. Durch Einführung der BOC-Gruppe wurde daraus der (3RS,4RS,5SR)-4-(4-Brom-phenyl)-3-hydroxy-5-isobutyl-piperidin-1-carbonsäure tert-butylester als farbloser Festkörper erhalten; MS : 412 $(M+H)^+$. Die Alkylierung schließlich mit 2-Brommethylnaphthalin lieferte den (3RS,4RS,5SR)-4-(4-Brom-phenyl)-5-isobutyl-3-naphthalin-2-ylmethoxypiperidin-1-carbonsäure tert-butylester als farblosen Schaum; MS : 552 $(M+H)^+$.

In analoger Weise wie in Beispiel 62 (b)-(d) beschrieben, wurde wie folgt verfahren:

(c) Aus (RS)-1-Benzyl-3-methyl-piperidin-4-on und und 1-Brom-4-fluorbenzol wurde das (3RS,4RS)- und (3RS, 4SR)-1-Benzyl-4-(4-fluorphenyl)-3-methyl-piperidin-4-ol als farbloser Festkörper erhalten; MS : 299 $(M)^+$. Durch Elimination mittels Trifluoressigsäure und anschließende chromatographische Trennung wurden die beiden isomeren Olefine, das (RS)-1-Benzyl-4-(4-fluor-phenyl)-3-methyl-1,2,3,6-tetrahydro-pyridin, MS : 281 $(M)^+$, und das 1-Benzyl-4-(4-fluor-phenyl)-5-methyl-1,2,3,6-tetrahydro-pyridin, MS : 281 $(M)^+$, jeweils als bräunliches Öl erhalten. Die darauffolgende Hydroborierung des (RS)-1-Benzyl-4-(4-fluor-phenyl)-3-methyl-1,2,3,6-tetrahydro-pyridins ergab das (3RS,4RS,5SR)-1-Benzyl-4-(4-fluor-phenyl)-5-methyl-piperidin-3-ol als farblosen Festkörper; MS : 299 $(M)^+$.

(d) Eine Lösung von 600 mg (2 mMol) (3RS,4RS,5SR)-1-Benzyl-4-(4-fluorphenyl)-5-methyl-piperidin-3-ol in 20 ml Methanol wurde mit 60 mg Palladium/Kohle (10%ig) bei Raumtemperatur unter Normaldruck hydriert. Zur Aufarbeitung wurde der Katalysator abfiltriert, dieser in warmem Methanol ausgerührt und wieder abfiltriert. Die vereinigten Methanol-Lösungen wurden unter vermindertem Druck eingedampft. Das erhaltene Rohprodukt (410 mg) wurde ohne weitere Reinigung in der folgenden Stufe eingesetzt. Zu analytischen Zwecken wurde eine Probe aus Äther/Hexan umkristallisiert. Das (3RS,4RS,5SR)-4-(4-Fluorphenyl)-5-methyl-piperidin-3-ol Hydrochlorid wurde in Form farbloser Kristalle erhalten; MS : 209 $(M)^+$.

(e) Durch Einführung der BOC-Schutzgruppe wurde aus dem (3RS,4RS,5SR)-4-(4-Fluor-phenyl)-5-methyl-piperidin-3-ol Hydrochlorid der (3RS,4RS,5SR)-4-(4-Fluor-phenyl)-3-hydroxy-5-methyl-piperidin-1-carbonsäure tert-butylester als farbloses Öl erhalten; MS : 253 $(M-C_4H_8)^+$. Die Alkylierung schließlich mit 2-Brommethylnaphthalin lieferte den (3RS,4RS,5SR)-4-(4-Fluor-phenyl)-5-methyl-3-naphthalin-2-ylmethoxy-piperidin-1-carbonsäure tert-butylester als farblosen Festkörper; MS : 393 $(M-C_4H_8)^+$.

In analoger Weise wie in Beispiel 62 (a)-(d) und (g)-(h) beschrieben, wurde wie folgt verfahren:

(a) Ausgehend von 2-Benzyl-acrylsäureäthylester und Benzylamin wurde das (3RS)-1,3-Dibenzyl-piperidin-4-on als gelbliches Öl erhalten; MS : 279 $(M)^+$ . Durch Umsetzung mit 1-Brom-4-fluorbenzol wurde das (3RS,4RS)- und (3RS,4SR)-1,3 Dibenzyl-4-(4-fluor-phenyl)-piperidin-4-ol als farbloser Festkörper erhalten; MS : 375 $(M)^+$. Durch Elimination mittels Trifluoressigsäure und anschließende chromatographische Trennung wurden die beiden isomeren Olefine, das (RS)-1,3-Dibenzyl-4-(4-fluor-phenyl)-1,2,3,6-tetrahydro-pyridin als gelber Festkörper, MS : 357 $(M)^+$, und das 1,5-Dibenzyl-4-(4-fluor-phenyl)-1,2,3,6-tetrahydropyridin, erhalten. Die darauffolgende Hydroborierung des (RS)-1,3-Dibenzyl-4-(4-fluor-phenyl)-1,2,3,6-tetrahydro-pyridins ergab das (3RS,4RS,5SR)-1,5-Dibenzyl-4-(4-fluor-phenyl)-piperidin-3-ol als farblosen Festkörper; MS : 375 $(M)^+$. Die Abspaltung der Benzylgruppe erfolgte mittels katalytischer Hydrierung, in analoger Weise wie in obigem Beispiel beschrieben, und lieferte das (3RS,4RS,5SR)-5-Benzyl-4-(4-fluor-phenyl)-piperidin-3-ol als farblosen Festkörper erhalten; MS : 285 $(M)^+$. Durch

Einführung der BOC-Gruppe wurde daraus der (3RS,4SR,5SR)-3-Benzyl-4-(4-fluor-phenyl)-5-hydroxy-piperidin-1-carbonsäure tert-butylester als farbloser Festkörper erhalten; MS : 329 $(M-C_4H_8)^+$. Die Alkylierung schließlich mit 2-Brommethylnaphthalin lieferte den (3RS,4RS,5SR)-5-Benzyl-4-(4-fluor-phenyl)-3-(naphthalin-2-ylmethoxy)-piperidin-1-carbonsäure tert-butylester als farblosen Schaum; $R_f$: 0.32 ($SiO_2$, Toluol:Essigester=95:5).

Beispiel 66

In analoger Weise zu dem von A.Mann et al. in Synth.Comm. 19(18), 3139-3142 (1989) beschriebenen Verfahren wurden 40 mg (0.08 mMol) (3SR,4RS,5RS)-4-(4-Chlor-phenyl)-3-(1-äthyl-propyl)-5-(naphthalin-2-ylmethoxy)-piperidin-carbonsäure tert-butylester in 5 ml trockenem Methylenchlorid gelöst und mit 35 mg (0.16 mMol) wasserfreiem Zinkbromid versetzt. Das Reaktionsgemisch wurde während 5 Stunden bei Raumtemperatur gerührt. Zur Aufarbeitung wurde das Reaktionsgemisch unter vermindertem Druck eingedampft und das erhaltene Rohprodukt zur Reinigung an Kieselgel unter Verwendung eines 90:10:0.1-Gemisches von Methylenchlorid, Methanol und Ammoniak als Elueirungsmittel chromatographiert. Es wurden 28 mg (86% d.Th.) (3SR,4RS,5RS)-4-(4-Chlor-phenyl)-3-(1-äthyl-propyl)-5-(naphthalin-2-ylmethoxy)-piperidin als farbloses Öl erhalten; MS : 422 $(M+H)^+$.

Der als Ausgangssubstanz eingesetzte (3SR,4RS,5RS)-4-(4-Chlorphenyl)-3-(1-äthyl-propyl)-5-(naphthalin-2-ylmethoxy)-piperidincarbonsäure tert-butylester wurde, in analoger Weise wie in Beispiel 62 (a)-(h) beschrieben, wie folgt erhalten:

Ausgehend von 2-(1-Äthyl-propyl)-acrylsäuremethylester und Methylamin wurde das (3RS)-3-(1-Äthyl-propyl)-1-methyl-piperidin-4-on als farbloses Öl erhalten; MS : 183 $(M)^+$ . Durch Umsetzung mit 1-Brom-4-chlorbenzol wurde daraus das (3RS,4RS)- und (3RS,4SR)-4-(4-Chlorphenyl)-3-(1-äthyl-propyl)-1-methyl-piperidin-4-ol als farbloser Festkörper erhalten; MS : 295 $(M)^+$. Die anschließende Elimination mittels Trifluoressigsäure und chromatographische Trennung ergab die beiden isomeren Olefine, das (RS)-4-(4-Chlor-phenyl)-3-(1-äthylpropyl)-1-methyl-1,2,3,6-tetrahydro-pyridin, MS : 277 $(M)^+$, und das 4-(4-Chlor-phenyl)-5-(1-äthyl-propyl)-1-methyl-1,2,3,6-tetrahydro-pyridin jeweils als farbloses Öl. Die darauffolgende Hydroborierung des (RS)-4-(4-Chlor-phenyl)-3-(1-äthyl-propyl)-1-methyl 1,2,3,6-tetrahydro-pyridins ergab das (3RS,4RS,5SR)-4-(4-Chlor-phenyl)-5-(1-äthyl-propyl)-1-methylpiperidin-3-ol als farblosen Festkörper; MS : 296 $(M+H)^+$. Die weitere Umsetzung mit Chlorameisensäure-2,2,2-trichloräthylester lieferte den (3SR,4RS,5RS)-4-(4-Chlor-phenyl)-3-(1-äthyl-propyl)-5-(2,2,2-trichlor-äthoxycarbonyloxy)-piperidin-1-carbonsäure 2,2,2-trichlor-äthylester als farbloses Öl; MS : 653 $(M+Na)^+$. Die Abspaltung der TROC-Gruppe mit Zink in Eisessig ergab das (3RS,4RS,5SR)-4-(4-Chlor-phenyl)-5-(1-äthylpropyl)-piperidin-3-ol als farblosen Festkörper: MS : 281 $(M)^+$. Durch Einführung der BOC-Schutzgruppe wurde daraus der (3SR,4RS,5RS)-4-(4-Chlor-phenyl)-3-(1-äthyl-propyl)-5-hydroxy-piperidin-1-carbonsäure tert-butylester als farbloses Öl erhalten; MS : 325 $(M-C_4H_8)^+$. Die Alkylierung mit 2-Brommethylnaphthalin lieferte schließlich den (3SR,4RS,5RS)-4-(4-Chlor-phenyl)-3-(1-äthyl-propyl)-5-(naphthalin-2-ylmethoxy)-piperidin-carbonsäure tert-butylester; $R_f$: 0.41 ($SiO_2$, Toluol:Essigester=95:5).

Beispiel 67

In analoger Weise wie in Beispiel 66 beschrieben, wurde aus dem (3SR,4RS,5RS)-4-(4-Chlor-phenyl)-3-isopropyl-5-(4-methoxy-benzyloxy)-piperidin-carbonsäure yert-butylester das (3SR,4RS,5RS)-4-(4-Chlorphenyl)-3-isopropyl-5-(4-methoxy-benzyloxy)-piperidin als farbloses Öl erhalten; $R_f$: 0.21 ($SiO_2$, Methylenchlorid:Methanol:Ammoniak=95:5:0.1.

Der als Ausgangssubstanz eingesetzte (3SR,4RS,5RS)-4-(4-Chlorphenyl)-3-isopropyl-5-(4-methoxy-benzyloxy)-piperidin-carbonsäure tert-butylester wurde, in analoger Weise wie in Beispiel 62 (h) beschrieben, durch Alkylierung des (3RS,4RS,5SR)-4-(4-Chlor-phenyl)-3-hydroxy-5-isopropyl-piperidin-1-carbonsäure tert-butylesters (Beispiel 65) mit 4-Methoxy-benzylchlorid erhalten; $R_f$: 0.39 ($SiO_2$, Toluol:Essigester=9:1).

Beispiel 68

Eine Lösung von 60 mg (0.11 mg) (3RS,4RS,5SR)-4-(4-Brom-phenyl)-5-methoxymethyl-3-(naphthalin-2-ylmethoxy)-piperidin-1-carbonsäure tert-butylester in 5 ml Methylenchlorid wurde mit 2 ml 2 N Chlorwasserstoff in Methanol versetzt und 2 Stunden lang bei Raumtemperatur gerührt. Zur Aufarbeitung wurde die Reaktionslösung unter vermindertem Druck eingedampf. Der Rückstand wurde aus Diäthyläther umkristallisiert und ergab 53 mg (98% d.Th.) (3RS,4RS,5SR)-4-(4-Brom-phenyl)-5-methoxymethyl-3-naphthalin-2-yl-methoxy-piperidin Hydrochlorid als farblosen Festkörper; MS : 442 $(M+H)^+$.

Der als Ausgangssubstanz eingesetzte (3RS,4RS,5SR)-4-(4-Bromphenyl)-5-methoxymethyl-3-naphthalin-2-ylmethoxy-piperidin-1-carbonsäure tert-butylester wurde wie folgt erhalten:

(a) Eine Lösung von 7.45 g (33.7 mMol) (3RS,4RS)-1-Benzyl-3-hydroxymethyl-piperidin-4-ol und (3SR,4RS)-1-Benzyl-3-hydroxymethylpiperidin-4-ol [E.Jaeger und J.H.Biel, J.Org.Chem. 30(3), 740-744 (1965)], 10.89 g (39.6 mMol) tert-Butyldiphenylchlorsilan, 3.44 g (50.5 mMol) Imidazol und 0.2 g (1.6 mMol) 4-Dimethylaminopyridin in 80 ml Dimethylformamid wurde in Gegenwart von Molekularsieb (4 Å) 5 Tage lang bei Raumtemperatur gerührt. Zur Aufarbeitung wurde das Molekularsieb abgesaugt und die Lösung im Ölpumpenvakuum eingedampft. Der Rückstand wurde viermal in einem Gemisch von Äther und Methylenchlorid digeriert, die erhaltenen Lösungen vereinigt, über Natriumsulfat getrocknet und unter vermindertem Druck eingedampft. Das Rohprodukt wurde zur Reinigung an Kieselgel unter Verwendung eines 98:2-Gemisches von Methylenchlorid und Methanol als Eluierungsmittel chromatographiert. Es wurden 8.23 g (53% d.Th.) eines Gemisches des (3RS,4RS)- und (3RS,4SR)-1-Benzyl-3-(tert-butyl-diphenyl-silanyloxymethyl)-piperidin-4-ols als farbloses Öl erhalten; MS : 459 $(M)^+$.

(b) Eine Lösung von 2.45 g (19.33 mMol) Oxalylchlorid in 60 ml Methylenchlorid wurde auf -70° C abgekühlt, dann tropfenweise mit 3.02 g (38.66 mMol) Dimethylsulfoxid versetzt und 5 Minuten lang bei -70° weitergerührt. Dazu wurde eine Lösung von 8.08 g (17.6 mMol) eines Gemisches des (3RS,4RS)- und (3RS,4SR)-1-Benzyl-3-(tert-butyldiphenyl-silanyloxymethyl)-piperidin-4-ols in 15 ml Methylenchlorid getropft, dann 15 Minuten nachgerührt. Anschließend wurden bei -70° C 8.86 g (87.6 mMol) Triäthylamin zugetropft. Nach dem Erwärmen des Reaktionsgemisches auf Raumtemperatur (ca. 15 Minuten) wurde in Eiswasser hydrolysiert und danach dreimal mit je 200 ml Methylenchlorid extrahiert. Die vereinigten organischen Phasen wurden über Natriumsulfat getrocknet und unter vermindertem Druck eingedampft. Das Rohprodukt wurde zur Reinigung an Kieselgel unter Verwendung von Methylenchlorid als Eluierungsmittel chromatographiert. Es wurden 6.5 g (81% d.Th.) (3RS)-1-Benzyl-3-(tert-butyl-diphenyl-silanyloxymethyl)-piperidin-4-on als farbloses Öl erhalten; MS : 458 $(M+H)^+$.

(c) In analoger Weise wie in Beispiel 62 (b) beschrieben, wurde aus (3RS)-1-Benzyl-3-(tert-butyl-diphenyl-silanyloxymethyl)-piperidin-4-on und 1,4-Dibrombenzol das (3RS,4RS)- und/oder (3RS,4SR)-1-Benzyl-4-(4-brom-phenyl)-3-(tert-butyl-diphenyl-silanyloxymethyl)-piperidin-4-ol als farbloser Schaum erhalten; MS : 616 $(M+H)^+$.

(d) In analoger Weise wie in Beispiel 62 (c) beschrieben, wurde durch Elimination des tert.-Alkohols und gleichzeitige Abspaltung der Silylgruppe mittels Trifluoressigsäure aus dem (3RS,4RS)- und/oder (3RS,4SR)-1-Benzyl-4-(4-brom-phenyl)-3-(tert-butyl-diphenylsilanyloxymethyl)-piperidin-4-ol das (3RS)-[1-Benzyl-4-(4-brom-phenyl)-1,2,3,6-tetrahydro-pyridin-3-yl]-methanol als farbloser Festkörper erhalten; MS: 360 $(M+H)^+$.

(e) In analoger Weise wie in Beispiel 62 (d) beschrieben, wurde durch Hydroborierung von (3RS)- [1-Benzyl-4-(4-brom-phenyl)-1,2,3,6-tetrahydro-pyridin-3-yl]-methanol ein Gemisch des (3RS,4RS,5SR)-1-Benzyl-4-(4-brom-phenyl)-5-hydroxymethyl-piperidin-3-ols und des (3RS,4RS)- und/oder (3RS,4SR)-1-Benzyl-4-(4-brom-phenyl)-3-hydroxymethyl-piperidin-4-ols als farbloser Schaum erhalten.

(f) In analoger Weise wie in Beispiel 62 (e) beschrieben, wurde durch Behandlung des obigen Gemisches mit Chlorameisensäure-2,2,2-trichloräthylester ein Gemisch des (3RS,4RS,5SR)-4-(4-Brom-phenyl)-3-(2,2,2-trichlor-äthoxycarbonyloxy)-5-(2,2,2-trichlor-äthoxycarbonyloxymethyl)-piperidin-1-carbonsäure 2,2,2-trichlor-äthylesters und des (3RS,4RS)- und/oder (3RS,4SR)-4-(4-Brom-phenyl)-4-(2,2,2-trichlor-äthoxycarbonyloxy)-3-(2,2,2-trichlor-äthoxycarbonyloxymethyl)-piperidin-1-carbonsäure 2,2,2-trichlor-äthylesters als farbloser Schaum erhalten.

(g) In analoger Weise wie in Beispiel 62 (f) beschrieben, wurde durch Reaktion des obigen Gemisches mit Zink in Eisessig das 4:1-Gemisch des (3RS,4RS,5RS)-4-(4-Brom-phenyl)-5-hydroxymethyl-piperidin-3-ols und des (3RS, 4RS)- und/oder (3RS,4SR)-4-(4-Brom-phenyl)-3-hydroxymethyl-piperidin-4-ols als farbloser Schaum erhalten.

(h) In analoger Weise wie in Beispiel 62 (g) beschrieben, wurden durch Einführung der BOC-Gruppe und anschließende chromatographische Trennung des Gemisches der (3RS,4RS,5SR)-4-(4-Brom-phenyl)-3-hydroxy-5-hydroxymethyl-piperidin-1-carbonsäure tert-butylester als farbloser Schaum, MS : 386 $(M+H)^+$, und der (3RS,4RS)- und/oder (3RS,4SR)-4-(4-Brom-phenyl)-4-hydroxy-3-hydroxymethyl-piperidin-1-carbonsäure tert-butylester als farbloser Festkörper, MS : 386 $(M+H)^+$, erhalten.

(i) Eine Lösung von 735 mg (1.91 mMol) (3RS,4RS,5SR)-4-(4-Bromphenyl)-3-hydroxy-5-hydroxymethyl-piperidin-1-carbonsäure tert-butylester, 766 mg (2.75 mMol) Triphenylchlormethan und 324 mg (3.20 mMol) Triäthylamin in 8 ml Methylenchlorid wurde 15 Stunden lang bei Raumtemperatur gerührt. Zur Aufarbeitung wurde das Reaktionsgemisch unter vermindertem Druck eingedampft und das Rohprodukt direkt an Kieselgel unter Verwendung von Methylenchlorid als Eluierungsmittel chromatographiert. Es wurden 1.01 g (84.5% d.Th.) (3RS,4RS,5SR)-4-(4-Brom-phenyl)-3-hydroxy-5-trityloxymethyl-piperidin-1-carbonsäure tert-butylester als farbloser Schaum erhalten; MS : 646 $(M+NH_4)^+$.

(j) In analoger Weise wie in Beispiel 62 (h) beschrieben, wurde durch Alkylierung des (3RS,4RS,5SR)-4-(4-Bromphenyl)-3-hydroxy-5-trityloxymethyl-piperidin-1-carbonsäure tert-butylesters mit 2-Brommethyl-naphthalin der (3RS,4RS,5SR)-4-(4-Brom-phenyl)-3-naphthalin-2-ylmethoxy-5-trityloxymethyl-piperidin-1-carbonsäure tert-butylester als farbloser Schaum erhalten; MS : 785 ($M+NH_4$)+.

(k) Eine Lösung von 990 mg (1.29 mMol) (3RS,4RS,5SR)-4-(4-Bromphenyl)-3-naphthalin-2-ylmethoxy-5-trityloxymethyl-piperidin-1-carbonsäure tert-butylester und 4 ml 2 N Chlorwasserstoff/Methanol in 5 ml Methylenchlorid wurde 45 Minuten lang bei Raumtemperatur gerührt. Zur Aufarbeitung wurde die Reaktionslösung in 40 ml gesättigte Natriumcarbonat-Lösung gegossen und diese zweimal mit je 40 ml Methylenchlorid extrahiert. Die vereinigten organischen Phasen wurden über Natriumsulfat getrocknet und unter vermindertem Druck eingedampft. Zur Reinigung und Auftrennung der erhaltenen Produkte wurde an Kieselgel chromatographiert: Zunächst mit einem 98:2-Gemisch von Methylenchlorid und Methanol als Eluierungsmittel. Dabei wurden 360 mg (54% d.Th.) (3RS,4RS,5SR)-4-(4-Brom-phenyl)-5-hydroxymethyl-3-naphthalin-2-ylmethoxy-piperidin-1-carbonsäure tert-butylester als farbloser Festkörper erhalten; MS : 528 $(M+H)^+$. Dann mit einem 90:10:1-Gemisch von Methylenchlorid, Methanol und Ammoniak, wobei das (3SR,4RS,5RS)-4-(4-Brom-phenyl)-5-naphthalin-2-ylmethoxy-piperidin-3-yl]-methanol als farbloser Festkörper erhalten wurde; MS : 426 $(M+H)^+$.

(1) In analoger Weise wie in Beispiel 62 (h) beschrieben, wurde durch Alkylierung des (3SR,4RS,5RS)-4-(4-Bromphenyl)-5-naphthalin-2-ylmethoxy-piperidin-3-yl]-methanols mit Methyliodid der (3RS,4RS,5SR)-4-(4-Brom-phenyl)-5-methoxymethyl-3-naphthalin-2-ylmethoxy-piperidin-1-carbonsäure tert-butylester als farbloser Festkörper erhalten; MS : 540 $(M+H)^+$.

Beispiel 69

a) Eine Lösung von 32.5 g (163 mMol) 4-Oxo-piperidin-1-carbonsäure tert-butylester in 200 ml Chloroform wurde mit 24.0 g (168 mMol) Dinatriumhydrogenphosphat versetzt und auf 5°C gekühlt. Eine Lösung von 27.9 g (175 mMol) Brom in 75 ml Chloroform wurde während 1 Stunde zugetropft, danach das Reaktionsgemisch auf Raumtemperatur aufgewärmt und während 18 Stunden gerührt. Das Reaktionsgemisch wurde extrahierend mit Eiswasser und Methylenchlorid aufgearbeitet, die organische Phase über Magnesiumsulfat getrocknet, filtriert und das Lösungsmittel im Wasserstrahlvakuum abdestilliert. Das Rohprodukt (38 g) wurde an Kieselgel mit Methylenchlorid und Essigester als Eluierungsmittel chromatographiert. Das so erhaltene Produkt wurde aus Essigester und n-Hexan umkristallisiert. Man erhielt 19.1 g (42% d.Th.) 3-Brom-4-oxo-piperidin-1-carbonsäure tert-butylester als hellgelben Festkörper; MS: 277, 279 $(M)^+$.

b) Eine Lösung von 2.78 g (10 mMol) 3-Brom-4-oxo-piperidin-1-carbonsäure tert-butylester und 2.09 g (12 mMol) 2-Mercaptomethylnaphthalin in 100 ml absolutem Acetonitril wurde mit 13.8 g (100 mMol) wasserfreiem Kaliumcarbonat versetzt und danach während 18 Stunden bei Raumtemperatur gerührt. Das Reaktionsgemisch wurde filtriert, das Filtrat auf Eiswasser gegossen und mit konzentrierter Salzsäure auf pH 2-3 gestellt; die wäßrige Phase wurde dreimal mit je 200 ml Essigester extrahiert, die organische Phase einmal mit 200 ml Wasser gewaschen, über Magnesiumsulfat getrocknet, filtriert und im Wasserstrahlvakuum eingeengt. Das Rohprodukt (5.5 g) wurde an Kieselgel mit Hexan und Essigester als Eluierungsmittel chromatographiert. Das Produkt wurde aus Essigester und Hexan umkristallisiert. Es wurden 2.27 g (61% d.Th.) (3RS)-3-(Naphthalin-2-ylmethylthio)-4-oxo-piperidin-1-carbonsäure tert-butylester als farbloser Festkörper erhalten; MS: 371 $(M)^+$.

c) 0.31 g (12.8 mgAtome) Magnesiumspäne wurden in 5 ml absolutem Tetrahydrofuran unter Argon suspendiert, dann bei Rückfluß mit einer Lösung von 1.75 g (10 mMol) 4-Brom-fluorbenzol in 10 ml Tetrahydrofuran zur Reaktion gebracht. Nach dem Abklingen der Reaktion wurde bei Raumtemperatur eine Lösung von 1.86 g (5 mMol) (3RS)-3-(Naphthalin-2-ylmethylthio)-4-oxo-piperidin-1-carbonsäure tert-butylester in 10 ml Tetrahydrofuran zugetropft und dann während 4 Stunden gerührt. Nach der Hydrolyse mit 10 ml Wasser wurde das Reaktionsgemisch extrahierend mit Essigester aufgearbeitet. Die organische Phase wurde über Magnesiumsulfat getrocknet, filtriert und das Lösungsmittel im Wasserstrahlvakuum abdestilliert. Das Rohprodukt (2.6 g) wurde an Kieselgel mit Hexan und Essigester als Eluierungsmittel chromatographiert. Man erhielt 1.45 g (62% d.Th. ) (3RS,4SR oder 3RS,4RS)-4-(4-Fluorphenyl)-4-hydroxy-3-(naphthalin-2-ylmethylthio)-piperidin-1-carbonsäure tert-butylester als farblosen Festkörper, MS: 468 $(M+H)^+$, und 0.37 g (16% d. Th.) (3RS,4RS oder 3RS,4SR)-4-(4-Fluorphenyl)-4-hydroxy-3-(naphthalin-2-ylmethylthio)-piperidin-1-carbonsäure tert-butylester als farblosen Festkörper; MS: 468 $(M+H)^+$.

d) Eine Lösung von 0.23 g ( 0.5 mMol) (3RS,4SR oder 3RS,4RS)-4-(4-Fluorphenyl)-4-hydroxy-3-(naphthalin-2-ylmethylthio)-piperidin-1-carbonsäure tert-butylester in 5 ml absolutem Methanol wurde mit 1 ml Salzsäure in Methanol (1.4 molar) versetzt und danach während 3 Stunden bei 50 °C gerührt. Nach dem Abdestillieren des Lösungsmittels im Wasserstrahlvakuum wurde das Produkt aus Methanol umkristallisiert. Dabei wurden 0.18 g (89% d.Th.) (3RS,4SR oder 3RS,4RS)-4-(4-Fluorphenyl)-4-hydroxy-3-(naphthalin-2-ylmethylthio)-piperidin Hydrochlorid als hellgelber Festkörper erhalten; MS: 368 $(M+H)^+$.

Beispiel 70

Eine Lösung von 0.23 g (0.5 mMol) (3RS,4RS oder 3RS,4SR)-4-(4-Fluorphenyl)-4-hydroxy-3-(naphthalin-2-ylmethylthio)-piperidin-1-carbonsäure tert-butylester [Beispiel 69 (c)] in 5 ml absolutem Methanol wurde mit 1 ml Salzsäure in Methanol (1.4 molar) versetzt und danach während 3 Stunden bei 50 °C gerührt. Nach dem Abdestillieren des Lösungsmittels im Wasserstrahlvakuum wurde der Rückstand zwischen Methylenchlorid und Wasser verteilt, mit gesättigter Natriumbicarbonat-Lösung neutralisiert und extrahiert; die organische Phase wurde über Magnesiumsulfat getrocknet, filtriert und eingeengt. Das Rohprodukt (0.15 g) wurde an Kieselgel mit Methylenchlorid und Methanol als Eluierungsmittel chromatographiert. Dabei wurden 0.041 g (22 % d.Th.) (3RS,4RS oder 3RS,4SR)-4-(4-Fluorphenyl)-4-methoxy-3-(naphthalin-2-ylmethylthio)-piperidin als gelbes Öl, MS: 381 $(M)^+$, und 0.067 g (37 % d.Th.) (3RS,4RS oder 3RS,4SR)-4-(4-Fluorphenyl)-4-hydroxy-3-(naphthalin-2-ylmethylthio)-piperidin als hellgelber Festkörper erhalten; MS: 367 $(M)^+$.

Beispiel 71

(a) 2.33 g (10.0 mMol) rac-3-Aza-7-oxa-bicyclo[4.1.0]heptan-3-carbonsäurebenzylester [S.V.D'Andrea et al., J. Org.Chem. (1991), 56(9), 3133-3137] und 1.88 g (20.0 mMol, 2 eq) Phenol wurden in 30 ml Acetonitril gelöst und bei Raumtemperatur mit 10.0 ml 2 N Natronlauge versetzt. Diese Lösung wurde 5 Stunden lang bei 95° C gerührt. Anschließend wurde die auf Raumtemperatur abgekühlte Lösung mit 60 ml Wasser versetzt und dreimal mit Methylenchlorid extrahiert. Die organischen Phasen wurden mit 100 ml 2 N Natronlauge und zweimal mit Wasser gewaschen, über Magnesiumsulfat getrocknet, unter vermindertem Druck eingedampft und am Hochvakuum getrocknet. Das Rohprodukt (3.05 g) wurde an Kieselgel mittels eines 7:3-Gemisches von Hexan und Essigester als Eluierungsmittel aufgetrennt. Dies lieferte 2.06 g (63 % d.Th.) (3RS,4RS)-3-Hydroxy-4-phenoxy-piperidin-1-carbonsäure benzyl ester als farbloses Öl; MS : 327 $(M)^+$.

(b) Eine Dispersion von 262 mg (6.0 mMol, 2 eq) Natriumhydrid (60%ige Dispersion in Weißöl) in 40 ml Dimethylsulfoxid wurde mit einer Lösung von 982 mg ( 3.0 mMol, 1 eq) (3RS,4RS)-3-Hydroxy-4-phenoxypiperidin-1-carbonsäure benzyl ester in 67 ml Dimethylsulfoxid versetzt. Dieses Gemisch wurde 2 Stunden lang bei 40° C gerührt, dann auf Raumtemperatur abgekühlt und tropfenweise mit einer Lösung von 1326 mg (6.0 mMol, 2 eq) 2-Brommethylnaphthalin in 40 ml Dimethylsulfoxid versetzt und 4 Stunden lang bei Raumtemperatur gerührt. Das Gemisch wurde auf 1 l einer Eis/Wasser-Mischung gegossen, 10 Minuten gerührt und dreimal mit Diäthyläther extrahiert. Die organischen Phasen wurden je einmal mit Wasser und gesättigter Natriumchlorid-Lösung gewaschen, über Magnesiumsulfat getrocknet, unter vermindertem Druck eingedampft und am Hochvakuum getrocknet. Das schwach gelbe Öl (1.75 g) wurde an Kieselgel mittels eines 4:1-Gemisches von Hexan und Essigester als Eluierungsmittel aufgetrennt. Dies lieferte 647 mg (46 % d.Th.) (3RS,4RS)-3-(Naphthalin-2-ylmethoxy)-4-phenoxy-piperidin-1-carbonsäure benzylester als amorpher Feststoff erhalten; MS : 476 $(M\text{-Benzyl})^+$.

(c) 30 mg, 0.065 mMol) (3RS,4RS)-3-(Naphthalin-2-ylmethoxy)-4-phenoxy-piperidin-1-carbonsäure benzylester wurden in 1.6 ml Tetrahydrofuran gelöst, auf 0° C gekühlt und nacheinander innerhalb von 25 Minuten mit einer Lösung von 95 ml (0.32 mMol, ca 5 eq) einer 70%igen Natrium-dihydrido-bis-(2-methoxyäthoxy)-aluminat-Lösung (SDMA) in Toluol und 1.6 ml Tetrahydrofuran versetzt. Diese Reaktionslösung wurde 2.5 Stunden lang bei 0° C gerührt. Anschließend wurde auf eine Mischung aus gesättigter Kaliumnatriumtartrat-Lösung und Eis gegossen und viermal mit je 50 ml Methylenchlorid extrahiert. Die organischen Phasen wurden zweimal mit Wasser gewaschen, unter vermindertem Druck eingedampft und am Hochvakuum getrocknet. Das orangefarbene Harz wurde an Kieselgel mittels eines 95:5-Gemisches von Methylenchlorid und Methanol als Eluierungsmittel aufgetrennt. Es wurden 10 mg (47 % d.Th.) (3RS,4RS)-3-(Naphthalin-2-ylmethoxy)-4-phenoxy-piperidin als farbloser, amorpher Feststoff erhalten; $R_f$: 0.38 ($SiO_2$, Methylenchlorid:Methanol=9:1).

Beispiel 72

In analoger Weise wie in Beispiel 71 beschrieben, wurden durch Abspaltung der Benzyloxycarbonyl-Gruppe die folgenden Verbindungen erhalten:

1) - Aus (3RS,4RS)-4-(4-Brom-phenoxy)-3-(naphthalin-2-ylmethoxy)-piperidin-1-carbonsäure benzylester das (3RS,4RS)-4-(4-Bromphenoxy)-3-(naphthalin-2-ylmethoxy)-piperidin als farbloser, amorpher Feststoff; MS : 412.4, 414 $(M+H)^+$;

2) - aus (3RS,4RS)-4-(4-Chlor-phenylsulfanyl)-3-(naphthalin-2-ylmethoxy)-piperidin-1-carbonsäure benzylester das (3RS,4RS)-4-(4-Chlorphenylsulfanyl)-3-(naphthalin-2-ylmethoxy)-piperidin als farbloser, amorpher Feststoff; MS : 384 $(M+H)^+$.

Die als Ausgangsstoffe eingesetzten Derivate wurden wie folgt erhalten:

(a) In analoger Weise wie in Beispiel 71 (a)-(b) beschrieben, wurde aus rac-3-Aza-7-oxa-bicyclo[4.1.0]heptan-3-carbonsäurebenzylester durch Umsetzung mit 4-Bromphenol der (3RS,4RS)-4-(4-Brom-phenoxy)-3-hydroxy-piperidin-1-carbonsäure benzylester als farbloser Feststoff erhalten [$R_f$: 0.40 ($SiO_2$, Methylenchlorid:Essigester=2:1)], dessen Alkylierung mit 2-Brommethylnaphthalin den (3RS,4RS)-4-(4-Bromphenoxy)-3-(naphthalin-2-ylmethoxy)-piperidin-1-carbonsäure benzylester als farblosen Feststoff ergab; MS : 454, 456 $(M\text{-}Benzyl)^+$.

(b) Ein Gemisch von 2.33 g (10.0 mMol), rac-3-Aza-7-oxabicyclo[4.1.0]heptan-3-carbonsäurebenzylester, 2.89 g (20.0 mMol, 2 eq) p-Chlorthiophenol und 10.0 ml 2 N Natronlauge in 20.5 ml Acetonitril wurde 4 Stunden lang unter Rückfluß gekocht. Anschließend wurde die auf Raumtemperatur abgekühlte Lösung mit 25 ml Wasser versetzt und dreimal mit Methylenchlorid extrahiert. Die organischen Phasen wurden einmal mit 1 N Natronlauge und zweimal mit Wasser gewaschen, über Magnesiumsulfat getrocknet, unter vermindertem Druck eingedampft und am Hochvakuum getrocknet. Das Rohprodukt (3.55 g) wurde an Kieselgel mittels 9:1-Gemisches von Methylenchlorid und Essigester als Eluierungsmittel aufgetrennt. Dies lieferte 1.89 g (46 % d.Th.) (3RS,4RS)-4-(4-Chlorphenylsulfanyl)-3-hydroxy-piperidin-1-carbonsäure benzylester als farblosen Feststoff, MS : 377 $(M)^+$, und 169 mg (4 %) (3RS,4SR)-4-(4-Chlor-phenylsulfanyl)-3-hydroxy-piperidin-1-carbonsäurebenzylester.

(c) In analoger Weise wie in Beispiel 71 (b) beschrieben, wurde aus (3RS,4RS)-4-(4-Chlor-phenylsulfanyl)-3-hydroxy-piperidin-1-carbonsäure benzylester durch Alkylierung mit 2-Brommethyl-naphthalin der (3RS,4RS)-4-(4-Chlor-phenylsulfanyl)-3-(naphthalin-2-ylmethoxy)-piperidin-1-carbonsäure benzylester als farbloser Feststoff erhalten; MS : 518 $(M+H)^+$.

Beispiel 73

a) Eine Lösung von 5.0 g (16.9 mMol) (3RS,4RS)-4-(4-Fluor-phenyl)-3-hydroxy-piperidin-1-carbonsäure tert-butylester in 50 ml Methylenchlorid wurde mit 240 mg (1.96 mMol) 4-Dimethylamino-pyridin und 4.2 ml (29 mMol) Triäthylamin versetzt und auf 0°C gekühlt. Anschließend wurden 4.65 g (24.4 mMol) 2-Naphthoylchlorid portionsweise zugegeben und das Reaktionsgemisch während 18 Stunden bei Raumtemperatur gerührt. Hierauf wurde das Reaktionsgemisch mit Eiswasser versetzt und mit Methylenchlorid extrahiert. Die vereinigten Methylenchloridphasen wurden über Magnesiumsulfat getrocknet, filtriert und das Lösungsmittel im Wasserstrahlvakuum abdestilliert. Das Rohprodukt wurde an Kieselgel mit Methylenchlorid als Eluierungsmittel chromatographiert. Die so erhaltene Produktfraktion wurde aus Äther und n-Hexan umkristallisiert. Man erhielt 7.4 g (97% d.Th.) (3RS,4RS)-4-(4-Fluorphenyl)-3-(naphthalin-2-ylcarbonyloxy)-piperidin-1-carbonsäure tert-butylester als farblosen Festkörper; MS: 450 $(M+H)^+$.

b) Unter Argon und Feuchtigkeitsausschluß wurde eine Lösung von 2.7 ml (24.6 mMol) Titantetrachlorid in 18 ml Methylenchlorid bei 0°C zu 30 ml Tetrahydrofuran zugetropft, wobei eine gelbe Suspension entstand. Nach Aufwärmen auf Raumtemperatur wurden 15 ml (95 mMol) Tetramethyläthylendiamin zugegeben und das Reaktionsgemisch 10 Minuten gerührt. Nach der Zugabe von 3.6 g (55 mMol) Zinkstaub wurde weitere 30 Minuten bei Raumtemperatur gerührt. Hierauf wurde eine Lösung von 2.1 ml (30 mMol) Dibrommethan und 2.7 g (6.0 mMol) (3RS,4RS)-4-(4-Fluor-phenyl)-3-(naphthalin-2-ylcarbonyloxy)-piperidin-1-carbonsäure tert-butylester gelöst in 30 ml Tetrahydrofuran so zugetropft, daß die Temperatur nicht über 36°C anstieg. Anschließend wurde das Reaktionsgemisch während 60 Stunden bei Raumtemperatur gerührt, dann auf gesättigte Ammoniumchloridlösung gegossen und mit Äther extrahiert. Die vereinigten Ätherphasen wurden über Magnesiumsulfat getrocknet, eingeengt und der Rückstand an Kieselgel unter Verwendung eines 99:1-Gemisches von Methylenchlorid und Triäthylamin als Eluierunsmittel chromatographiert. Daraus resultierten 1.23 g (46% d. Th.) (3RS,4RS)-4-(4-Fluor-phenyl)-3-(1-naphthalin-2-yl-vinyloxy)-piperidin-1-carbonsäure tert-butylester als amorpher farbloser Festkörper; MS: 448 $(M+H)^+$.

c) Eine Lösung von 70 mg (0.156 mMol) (3RS,4RS)-4-(4-Fluor-phenyl)-3-(1-naphthalin-2-yl-vinyloxy)-piperidin-1-carbonsäure tert-butylester in 10 ml Tetrahydrofuran wurde mit 0.2 ml Triäthylamin und 100 mg Palladium auf Kohle versetzt und in einer Wasserstoffatmosphäre bei Raumtemperatur und Normaldruck hydriert. Anschließend wurde das Reaktionsgemisch über einen 0.8 µ Cellulosefilter abgenutscht und das Lösungsmittel im Wasserstrahlvakuum eingedampft. Es wurden 68.4 mg (97% d. Th.) eines Gemisches von (3RS,4RS)-4-(4-Fluor-phenyl)-3-[(RS)- und -[(SR)-1-naphthalin-2-yl-äthoxy]-piperidin-1-carbonsäure tert-butylester als amorpher farbloser Festkörper erhalten; MS: 450 $(M+H)^+$.

d) 69 mg (0.153 mMol) eines Gemisches von (3RS,4RS)-4-(4-Fluorphenyl)-3-[(RS)- und -[(SR)-1-naphthalin-2-yl-äthoxy]-piperidin-1-carbonsäure tert-butylester wurden in 2 ml Methylenchlorid gelöst, mit 104 mg (0.46 mMol) wasserfreiem Zinkbromid versetzt und während 2.5 Stunden bei Raumtemperatur gerührt. Hierauf wurde das Reaktionsgemisch auf wässerige Natriumcarbonatlösung gegossen und diese mit Methylenchlorid extrahiert. Die vereinigten Methylenchloridphasen wurden über Magnesiumsulfat getrocknet, eingeengt und der dabei erhaltene Rückstand an Kieselgel mit einem 9:1- Gemisch von Methylenchlorid und Methanol als Eluierungsmittel chromatographiert. Daraus resultierten 22.1 mg (41% d. Th.) (3RS,4RS)-4-(4-Fluor-phenyl)-3-[(RS)- oder -[(SR)-1-naphthalin-2-yl-äthoxy]-piperidin als amorpher gelblicher Festkörper; MS: 350 $(M+H)^+$ und 13.6 mg (25% d. Th.) (3RS, 4RS)-4-(4-Fluor-phenyl)-3-[(SR)- oder -[(RS)-1-naphthalin-2-yl-äthoxy]-piperidin als amorpher farbloser Festkörper; MS: 350 $(M+H)^+$.

Beispiel 74

a) Zu einer Lösung von 10.0 g (46.8 mMol) 4-(4-Fluor-phenyl)-1,2,3,6-tetrahydro-pyridin Hydrochlorid in 400 ml Äthanol wurden 13.8 g (100 mMol) Kaliumcarbonat gegeben und das Reaktionsgemisch anschließend auf Rückflußtemperatur erwärmt. Innerhalb einer Stunde wurde eine Lösung von 5.8 ml (49 mMol) Benzylbromid in 100 ml Äthanol zugetropft und danach noch 1 Stunde bei dieser Temperatur weitergerührt. Das Reaktionsgemisch wurde auf Raumtemperatur abgekühlt und filtriert, das Filtrat mit Wasser und Essigester extrahiert und die organische Phase schließlich über Magnesiumsulfat getrocknet. Nach dem Eindampfen im Wasserstrahlvakuum wurde das erhaltene Rohprodukt an Kieselgel mit Hexan und Essigester als Eluierungsmittel chromatographiert. Man erhielt 8.90 g (71% d. Th.) 1-Benzyl-4-(4-fluor-phenyl)-1,2,3,6-tetrahydro-pyridin; MS: 267 $(M)^+$.

b) 4.5 g (16.8 mMol) 1-Benzyl-4-(4-fluor-phenyl)-1,2,3,6-tetrahydropyridin wurden in 55 ml Wasser suspendiert, dann durch Zugabe von 70 ml konzentrierter Salzsäure teilweise gelöst, 1.36 g (45.3 mMol) Paraformaldehyd zugegeben und während 5 Stunden bei 100 °C gerührt. Nach dem Abkühlen auf Raumtemperatur wurde mit Natronlauge auf pH 5-6 gestellt und das Produkt zweimal mit 100 ml Essigester extrahiert. Die organischen Phasen wurden einmal mit 100 ml Wasser gewaschen, über Magnesiumsulfat getrocknet, filtriert und im Wasserstrahlvakuum eingeengt. Das so erhaltene Rohprodukt wurde an Kieselgel mit Hexan und Essigester als Eluierunsmittel chromatographiert. Man erhielt 3.91 g (78% d. Th.) (RS)-[1-Benzyl-4-(4-fluor-phenyl)-1,2,3,6-tetrahydro-pyridin-3-yl]-methanol; MS: 297 $(M)^+$.

c) Zu einer Lösung von 17.4 g (58.5 mMol) (RS)-[1-Benzyl-4-(4-fluorphenyl)-1,2,3,6-tetrahydro-pyridin-3-yl]-methanol in 580 ml absolutem Toluol wurden unter Argon bei Raumtemperatur unter Rühren 58 ml (203 mMol) Natrium dihydrido-bis-(2-methoxyäthoxy)aluminat (70% in Toluol) getropft. Anschließend wurde 4 Stunden lang bei 80°C gerührt. Zum Reaktionsgemisch wurden bei Raumtemperatur 100 ml Wasser tropfenweise gegeben, danach extrahierend mit Wasser und Essigester aufgearbeitet. Das Rohprodukt wurde an Kieselgel mit Hexan und Essigester als Eluierungsmittel chromatographiert. Man erhielt 3.90 g (44% d. Th.) (3RS,4SR)-[1-Benzyl-4-(4-fluor-phenyl)-piperidin-3-yl]-methanol; MS: 300 $(M+H)^+$.

d) 6.86 g (22.9 mMol) (3RS,4SR)-[1-Benzyl-4-(4-fluor-phenyl)-piperidin-3-yl]-methanol wurden in 70 ml Methanol unter Zusatz von 1.5 g Pd-Kohle (10%) bei Raumtemperatur hydriert. Nach dem Abfiltrieren des Katalysators

wurde das Lösungsmittel im Wasserstrahlvakuum abdestillert. Dabei erhielt man 4.79 g ( 100% d. Th.) (3RS,4SR) -[4-(4-Fluor-phenyl)-piperidin-3-yl]-methanol; MS: 210 $(M+H)^+$.

e) Zu einer Lösung von 4.89 g (23.4 mMol) (3RS,4SR)-[4-(4-Fluor-phenyl)-piperidin-3-yl]-methanol in 60 ml Dioxan wurden 4.20 g (50 mMol) Natriumhydrogencarbonat und 20 ml Wasser gegeben, dann portionsweise 6.10 g (28 mMol) Di-tert-butyldicarbonat eingetragen und 4 Stunden bei Raumtemperatur gerührt. Das Reaktionsgemisch wurde auf Eiswasser gegossen und das Produkt zweimal mit 200 ml Essigester extrahiert; die organischen Phasen wurden einmal mit 300 ml Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol als Eluierungsmittel chromatographiert. Man erhielt 7.03 g (97% d. Th.) (3RS,4SR)-4-(4-Fluor-phenyl)-3-hydroxymethyl-piperidin-1-carbonsäure tert-butylester; MS: 309 $(M)^+$.

f) Zu einer Lösung von 2.34 ml (27.3 mMol) Oxalylchlorid in 250 ml Methylenchlorid wurden unter Argon bei -70 °C 3.28 ml (46.2 mMol) Dimethylsulfoxid getropft. Nach 30 Minuten wurden 6.50 g (21 mMol) (3RS,4SR)-4-(4-Fluor-phenyl)-3-hydroxymethyl-piperidin- 1-carbonsäure tert-butylester, gelöst in 75 ml Methylenchlorid, zugetropft und 2 Stunden bei -70 °C gerührt. Anschließend wurden 7.25 ml (52.5 mMol) Triäthylamin zugetropft. Das Reaktionsgemisch wurde während 3 Stunden auf Raumtemperatur aufgewärmt und anschließend mit Wasser und Methylenchlorid extrahiert. Nach dem Trocknen über Magnesiumsulfat und dem Eindampfen im Wasserstrahlvakuum wurde das Rohprodukt durch Umkristallisation aus n-Hexan gereinigt. Dabei erhielt man 5.51 g (85% d. Th.) (3RS, 4SR)-4-(4-Fluor-phenyl)-3-formyl-piperidin-1-carbonsäure tert-butylester; MS: 279 $(M-CO)^+$.

g) 28.0 ml (54 mMol) Hexabutyldistannan wurden in 150 ml Tetrahydrofuran bei 0 °C unter Argon vorgelegt. Dazu wurden 31.3 ml (50 mMol) n-Butyllithium-Lösung (1.6 M in n-Hexan) getropft. Nach 30 Minuten wurde eine Lösung von 11.1 g (50 mMol) 2-Brommethyl-naphthalin in 50 ml Tetrahydrofuran zugetropft und danach bei Raumtemperatur gerührt. Nach 2 Stunden wurde das Lösungsmittel im Wasserstrahlvakuum abdestilliert und der Rückstand an Kieselgel unter Verwendung von Hexan und Essigester als Eluierungsmittel chromatographiert. Man erhielt 16.8 g (78% d. Th.) Tributylnaphthalin-2-ylmethyl-stannan; MS: 432 $(M+H)^+$.

h) 16.8 g (38.9 mMol) Tributyl-naphthalin-2-ylmethyl-stannan wurden in 150 ml Tetrahydrofuran unter Argon gelöst. Danach wurden bei -78 °C 12.5 ml (20 mMol) n-Butyllithium-Lösung (1.6 M in n-Hexan) zugetropft. Nach 30 Minuten wurde eine Lösung von 4.80 g (15.6 mMol) (3RS,4SR)-4-(4-Fluor-phenyl)-3-formyl-piperidin-1-carbonsäure tert-butylester in 70 ml Tetrahydrofuran bei -78 °C zugetropft und das Reaktionsgemisch 2 Stunden weitergerührt. Anschließend wurde während 3 Stunden auf Raumtemperatur erwärmt und noch weitere 18 Stunden gerührt. Nach dem Abdestillieren des Lösungsmittels im Wasserstrahlvakuum wurde das Reaktionsgemisch zwischen Wasser und Methylenchlorid verteilt, die organische Phase über Magnesiumsulfat getrocknet und eingeengt. Das Rohprodukt wurde an Kieselgel mit Hexan und Essigester als Eluierungsmittel chromatographiert. Man erhielt 5.50 g (78% d. Th.) eines Gemisches des (3RS,4SR)-4-(4-Fluor-phenyl-3-[(RS)- und -[(SR)-1-hydroxy-2-naphthalin-2-yl-äthyl]-piperidin-1-carbonsäure tert-butylesters; MS: 450 $(M+H)^+$.

i) 0.45 g (1 mMol) des Gemisches des (3RS,4SR)-4-(4-Fluor-phenyl-3-[(RS)- und -[(SR)-1-hydroxy-2-naphthalin-2-yl-äthyl]-piperidin-1-carbonsäure tert-butylesters wurden in 10 ml Methanol gelöst. Zu der Lösung wurden 2.0 ml (2.8 mMol) HCl in Methanol gegeben und das Reaktionsgemisch während 1 Stunde bei 50 °C gerührt. Nach dem Abdestillieren des Lösungsmittels im Wasserstrahlvakuum wurde der Rückstand aus Methanol und Äther umkristallisiert. Man erhielt 0.16 g (42% d. Th.) eines Gemisches des (RS)- und (SR)-1-[(3RS,4SR)-4-(4-Fluor-phenyl)-piperidin-3-yl]-2-naphthalin-2-yl-äthanol Hydrochlorids (1:1) in Form farbloser Kristalle; MS: 350 $(M+H)^+$.

Beispiel 75

a) 0.45 g (1 mMol) eines Gemisch des (3RS,4SR)-4-(4-Fluor-phenyl-3-[(RS)- und -[(SR)-1-hydroxy-2-naphthalin-2-yl-äthyl]-piperidin-1-carbonsäure tert-butylesters wurden in 5 ml Dimethylformamid unter Argon gelöst, dann nacheinander mit 0.16 g (1.2 mMol) 4-Dimethylamino-pyridin und 0.14 ml (1.2 mMol) Benzoylchlorid versetzt und während 16 Stunden bei Raumtemperatur gerührt. Das Reaktionsgemisch wurde anschließend extrahierend mit Eiswasser und Methylenchlorid aufgearbeitet. Das so erhaltene Rohprodukt wurde an Kieselgel mit Hexan und Essigester als Eluierungsmittel chromatographiert. Es wurden 0.27 g (49% d. Th.) (3RS,4SR)-3-[(RS)-oder -[(SR)-1-Benzoyloxy-2-naphthalin-2-yl-äthyl]-4-(4-fluor-phenyl)-piperidin-1-carbonsäure tert-butylester, MS: 554 $(M+H)^+$, und 0.19 g (34% d. Th.) (3RS,4SR)-3-[(SR)- oder -[(RS)-1-Benzoyloxy-2-naphthalin-2-yl-äthyl]-4-(4-fluor-phenyl)-piperidin-1-carbonsäure tert-butylester, MS: 554 $(M+H)^+$, erhalten.

b) 0.15 g (0.27 mMol) (3RS,4SR)-3-[(SR)- oder -[(RS)-1-Benzoyloxy-2-naphthalin-2-yl-äthyl]-4-(4-fluor-phenyl)-piperidin-1-carbonsäure tert-butylester wurden in 10 ml Methanol unter Zusatz von 1.0 ml (1.4 mMol) HCl in Methanol während 1 Stunde bei 50 °C gerührt. Nach dem Abdestillieren des Lösungsmittels im Wasserstrahlvakuum und anschließender Trocknung im Hochvakuum wurden 0.12 g (91% d. Th.) Benzoesäure (SR)- oder (RS)-1-[(3RS, 4SR)-4-(4-fluor-phenyl)-piperidin-3-yl]-2-naphthalin-2-yl-äthylester Hydrochlorid (1:1) als farbloser Schaum erhalten; MS: 454 $(M+H)^+$.

Beispiel 76

a) 0.25 g (0.45 mMol) (3RS,4SR)-3-[(RS)- oder -[(SR)-1-Benzoyloxy-2-naphthalin-2-yl-äthyl]-4-(4-fluor-phenyl)-piperidin-1-carbonsäure tert-butylester wurden in Analogie zu Beispiel 75 (b) mit 1.2 ml (1.68 mMol) HCl in Methanol umgesetzt. Dabei wurden 0.21 g (95% d. Th.) Benzoesäure (RS)- oder (SR)-1-[(3RS,4SR)-4-(4-fluor-phenyl)-piperidin-3-yl]-2-naphthalin-2-yl-äthylester Hydrochlorid (1:1) als farbloser Schaum erhalten; MS: 454 $(M+H)^+$.

Beispiel 77

a) 0.45 g (1 mMol) eines Gemisches des (3RS,4SR)-4-(4-Fluor-phenyl-3-[(RS)- und -[(SR)-1-hydroxy-2-naphthalin-2-yl-äthyl]-piperidin-1-carbonsäure tert-butylesters wurden in Analogie zu Beispiel 75 (a) mit 0.09 ml (1.26 mMol) Acetylchlorid und 0.16 g (1.3 mMol) 4-Dimethylamino-pyridin umgesetzt. Dabei wurden 0.38 g (77% d. Th.) eines Gemisch des (3RS,4SR)-3[(RS)- und -[(SR)-1-Acetyloxy-2-naphthalin-2-yl-äthyl]-4-(4-fluor-phenyl)-piperidin-1-carbonsäure tert-butylesters erhalten, welches direkt in die nächste Stufe eingesetzt wurde.

b) 0.15 g (0.31 mMol) des Gemisches des (3RS,4SR)-3[(RS)- und -[(SR)-1-Acetyloxy-2-naphthalin-2-yl-äthyl]-4-(4-fluor-phenyl)-piperidin-1-carbonsäure tert-butylesters wurden in 10 ml Methylenchlorid mit 0.5 ml (6.5 mMol) Trifluoressigsäure versetzt und während 16 Stunden bei Raumtemperatur gerührt. Nach dem Abdestillieren des Lösungsmittels im Wasserstrahlvakuum und anschließender Trocknung im Hochvakuum wurden 0.15 g (96% d. Th.) eines Gemisches des Essigsäure (RS)- und (SR)-1-[(3RS,4SR)-4-(4-Fluorphenyl)-piperidin-3-yl]-2-naphthalin-2-yl-äthylester Trifluoracetats (1:1) als farbloser Schaum erhalten; MS: 392 $(M+H)^+$.

Beispiel 78

a) 0.45 g (1 mMol) eines Gemisches des (3RS,4SR)-4-(4-Fluor-phenyl-3-[(RS)- und -[(SR)-1-hydroxy-2-naphthalin-2-yl-äthyl]-piperidin-1-carbonsäure tert-butylesters wurden in 10 ml Dimethylformamid unter Argon gelöst und dazu 0.07 g (1.6 mMol) Natriumhydrid-Dispersion (55% in Mineralöl) bei Raumtemperatur unter Rühren gegeben. Nach 1 Stunde wurden 0.14 ml (1.2 mMol) Benzylbromid zugetropft und das Reaktionsgemisch während 18 Stunden bei Raumtemperatur gerührt. Anschließend wurde extrahierend mit Eiswasser und Methylenchlorid aufgearbeitet. Das so erhaltene Rohprodukt wurde an Kieselgel mit Hexan und Essigester als Eluierungsmittel chromatographiert. Man erhielt 0.21 g (39% d. Th.) (3RS,4SR)-3-[(RS)- oder -[(SR)-1-Benzyloxy-2-naphthalin-2-yl-äthyl]-4-(4-fluor-phenyl)-piperidin-1-carbonsäure tert-butylester, MS: 540 $(M+H)^+$, und 0.17 g (31% d. Th.) (3RS,4SR)-3-[(SR)-oder -[(RS)-1-Benzyloxy-2-naphthalin-2-yl-äthyl]-4-(4-fluor-phenyl)-piperidin-1-carbonsäure tert-butylester, MS: 540 $(M+H)^+$.

b) 0.12 g (0.22 mMol) (3RS,4SR)-3-[(RS)- oder -[(SR)-1-Benzyloxy-2-naphthalin-2-yl-äthyl]-4-(4-fluor-phenyl)-piperidin-1-carbonsäure tert-butylester wurden in Analogie zu Beispiel 75 (b) mit 1.0 ml (1.40 mMol) HCl in Methanol umgesetzt. Dabei wurden 0.10 g (95% d. Th.) (3RS,4SR)-3-[(RS)- oder -[(SR)-1-Benzyloxy-2-naphthalin-2-yl-äthyl)-4-(4-fluor-phenyl)-piperidin Hydrochlorid (1:1) als farbloser Schaum erhalten; MS: 440 $(M+H)^+$.

Beispiel 79

a) 0.16 g (0.3 mMol) (3RS,4SR)-3-[(SR)- oder -[(RS)-1-Benzyloxy-2-naphthalin-2-yl-äthyl]-4-(4-fluor-phenyl)-piperidin- 1-carbonsäure tert-butylester wurden in Analogie zu Beispiel 75 (b) mit 1.0 ml (1.40 mMol) HCl in Methanol umgesetzt. Nach dem Abdestillieren des Lösungsmittels im Wasserstrahlvakuum wurde der Rückstand aus Methanol und Diäthyläther umkristallisiert. Dabei wurden 0.085 g (60% d. Th.) (3RS,4SR)-3-[(SR)- oder -[(RS)-1-Benzyloxy-2-naphthalin-2-yl-äthyl]-4-(4-fluor-phenyl)-piperidin Hydrochlorid (1:1) als weisser Festkörper erhalten; MS: 440 $(M+H)^+$.

Beispiel 80

a) 0.45 g (1 mMol) eines Gemisches des (3RS,4SR)-4-(4-Fluor-phenyl-3-[(RS)- und -[(SR)-1-hydroxy-2-naphthalin-2-yl-äthyl]-piperidin-1-1-carbonsäure tert-butylesters wurden in 10 ml Dimethylformamid unter Argon gelöst, dann 0.07 g (1.6 mMol) Natriumhydrid-Dispersion (55% in Mineralöl) unter Rühren bei Raumtemperatur zugesetzt. Nach 1 Stunde wurden 0.27 g ( 1.2 mMol) 2-(Brommethyl)-naphthalin zugegeben und und das Reaktionsgemisch während 72 Stunden bei Raumtemperatur gerührt. Anschließend wurde mit Eiswasser und Essigester extrahiert, die organische Phase über Magnesiumsulfat getrocknet und anschließend im Wasserstrahlvakuum eingedampft. Das so erhaltene Rohprodukt wurde an Kieselgel mit Hexan und Essigester als Eluierungsmittel chromatographiert. Man erhielt 0.19 g (32% d. Th.) (3RS,4SR)-4-(4-Fluor-phenyl)-3-[(SR)- oder (RS)-2-naphthalin-2-yl-1-(naphthalin-2-ylmethoxy)-äthyl)-piperidin-1-carbonsäure tert-butylester, welcher direkt in die nächste Stufe eingesetzt wurde, und 0.09 g (15% d. Th.) (3RS,4SR)-4-(4-Fluor-phenyl)-3-[(RS)- oder (SR)-2-naphthalin-2-yl-1-(naphthalin-2-ylmethoxy)-äthyl]-piperidin-1-carbonsäure tert-butylester, welcher ebenfalls direkt in die nächste Stufe eingesetzt wurde.

b) 0.19 g (0.32 mMol) (3RS,4SR)-4-(4-Fluor-phenyl)-3-[(SR)- oder (RS)-2-naphthalin-2-yl-1-(naphthalin-2-ylmethoxy)-äthyl]-piperidin-1-carbonsäure tert-butylester wurden in Analogie zu Beispiel 75 (b) mit 1.0 ml (1.40 mMol) HCl in Methanol umgesetzt. Die Reaktionslösung wurde auf Eiswasser gegossen, mit gesättigter Natriumhydrogencarbonat-Lösung neutralisiert, dann das Produkt zweimal mit 50 ml Methylenchlorid extrahiert. Die organischen Phasen wurden über Magnesiumsulfat getrocknet, filtriert und das Lösungsmittel im Wasserstrahlvakuum abdestilliert. Das so erhaltene Rohprodukt wurde an Kieselgel mit Methylenchlorid und Methanol als Eluierungsmittel chromatographiert. Dabei wurden 0.028 g (18% d. Th.) (3RS,4SR)-4-(4-Fluor-phenyl)-3-[(SR)- oder (RS)-2-naphthalin-2-yl-1-(naphthalin-2-ylmethoxy)-äthyl]-piperidin als amorpher, farbloser Festkörper erhalten; MS: 490 $(M+H)^+$.

Beispiel 81

a) 0.09 g (0.15 mMol) (3RS,4SR)-4-(4-Fluor-phenyl)-3-[(RS)- oder (SR)-2-naphthalin-2-yl-1-(naphthalin-2-ylmethoxy)-äthyl)-piperidin-1-carbonsäure tert-butylester wurden in Analogie zu Beispiel 75 (b) mit 1.0 ml (1.40 mMol) HCl in Methanol umgesetzt. Die Reaktionslösung wurde auf Eiwasser gegossen, mit gesättigter Natriumhydrogencarbonat-Lösung neutralisiert, dann das Produkt zweimal mit 50 ml Methylenchlorid extrahiert. Die organischen Phasen wurden über Magnesiumsulfat getrocknet, filtriert und das Lösungsmittel im Wasserstrahlvakuum abdestilliert. Das so erhaltene Rohprodukt wurde an Kieselgel mit Methylenchlorid und Methanol als Eluierungsmittel chromatographiert. Dabei wurden 0.036 g (49% d. Th.) (3RS,4SR)-4-(4-Fluor-phenyl)-3-[(RS)- oder (SR)-2-naphthalin-2-yl-1-(naphthalin-2-ylmethoxy)-äthyl]-piperidin als amorpher, farbloser Festkörper erhalten; MS: 490 $(M+H)^+$.

Beispiel 82

(a) Zu einer Suspension von 0.206 g (4.3 mMol) Natriumhydrid (50%ige Dispersion in Weißöl) in 6 ml Tetrahydrofuran tropfte man eine Lösung von 1.08 g (4.3 mMol) (1RS, 2RS, 3RS, 5SR)-3-(4-Chlor-phenyl)-8-methyl-8-aza-bicyclo[3.2.1]octan-2-ol [J. Org. Chem. 35, 802, 1970)] in 5 ml Tetrahydrofuran und rührte 60 Minuten bei 50 °C. Anschließend wurde auf Raumtemperatur abgekühlt und mit 0.95 g (4.3 mMol) 2-Brommethylnaphthalin in 5 ml Tetrahydrofuran versetzt. Nach 2 Stunden bei 50 °C wurde die Reaktionslösung auf 60 ml Eiswasser gegossen und dreimal mit 25 ml Essigsäureäthylester extrahiert. Die organischen Phasen wurden mit gesättigter Kochsalzlösung gewaschen, über Magnesiumsulfat getrocknet, filtriert und eingedampft. Der Rückstand wurde an Kieselgel unter Verwendung eines 95:5-Gemisches von Methylenchlorid und Methanol als Eluierungsmittel chromatographiert und lieferte 1.04 g (62 % d.Th.) (1RS, 2RS, 3RS, 5SR)-3-(4-Chlor-phenyl)-8-methyl-2-(naphthalin-2-ylmethoxy)-8-aza-bicyclo[3.2.1]octan als hellgelbes Öl, $R_f$: 0.43 (Kieselgel, Methylenchlorid;Methanol=95:5), MS: 392 $(M)^+$.

(b) Eine Lösung von 1.02 g (2.6 mMol) (1RS, 2RS, 3RS, 5SR)-3-(4-Chlorphenyl)-8-methyl-2-(naphthalin-2-ylmethoxy)-8-aza-bicyclo[3.2.1]octan in 40 ml Toluol wurde mit 150 mg Kaliumcarbonat versetzt und auf 100 °C erhitzt. Anschließend wurden 0.635 g (0.400 ml) (3 mMol) Chlorameisensäure 2,2,2-trichloräthylester dazugegeben und 12 Stunden bei 100 °C gerührt. Die Reaktionslösung wurde unter vermindertem Druck eingedampft, der Rückstand in 70 ml Essigester aufgenommen und mit 30 ml Wasser und 30 ml gesättigter Natriumhydrogencarbonat-Lösung gewaschen. Trocknen über Magnesiumsulfat, filtieren und eindampfen lieferte ein farbloses Öl, welches an Kieselgel unter Verwendung eines 3:2-Gemisches von Hexan und Essigester chromatographiert wurde. Man erhielt 1.14 g (79 % d.Th.) (1RS, 2RS, 3RS, 5SR)-3-(4-Chlor-phenyl)-2-(naphthalin-2-ylmethoxy)-8-aza-bicyclo [3.2.1] octan-8-carbonsäure-2,2,2-trichlorethyl ester als farbloses Öl, $R_f$: 0.38 (Kieselgel, Hexan:Essigester=3:2).

(c) Eine Suspension von 1.14 g (2.06 mMol) (1RS, 2RS, 3RS, 5SR)-3-(4-Chlor-phenyl)-2-(naphthalin-2-ylmethoxy)-8-aza-bicyclo[3.2.1]octan-8-carbonsäure-2,2,2-trichloräthyl ester und 400 mg Zink in 10 ml Essigsäure wurde 12 Stunden bei Raumtemperatur gerührt. Die Reaktionslösung wurde mit 50 ml Wasser verdünnt und viermal mit 40 ml Methylenchlorid extrahiert. Die organische Phase wurde zweimal mit 50 ml 1N Natronlauge gewaschen, über Natriumsulfat getrocknet, filtriert und eingedampft. Der Rückstand wurde an Kieselgel unter Verwendung eines 9:1-Gemisches von Methylenchlorid und Methanol als Eluierungsmittel chromatographiert. Es wurden 0.480 g (61 % d.Th.) (1RS, 2RS, 3RS, 5SR)-3-(4-Chlor-phenyl)-2-(naphthalin-2-ylmethoxy)-8-aza-bicyclo[3.2.1]octan vom Schmp. 184-185° erhalten; MS: 379 $(M+H)^+$.

Beispiel 83

In analoger Weise wie in Beispiel 82 (a) -(c) beschrieben, wurden folgende Verbindungen hergestellt:

1) -Aus (1RS, 2RS, 3RS, 5SR)-3-(4-Chlor-phenyl)-8-methyl-8-azabicyclo[3.2.1]octan-2-ol und 4-Chlormethyl-biphenyl das (1RS, 2RS, 3RS, 5SR)-2-(Biphenyl-4-ylmethoxy)-3-(4-chlor-phenyl)-8-azabicyclo[3.2.1]octan, MS: 236 $(M-C_{13}H_{11})^+$, welches mit Chlorwasserstoff in Äthanol in das Hydrochlorid vom Schmp.175-177° (Zers.) überführt wurde;

2) - aus (1RS, 2RS, 3RS, 5SR)-3-(4-Chlor-phenyl)-8-methyl-8-azabicyclo[3.2.1]octan-2-ol und 3,4-Dichlor-1-chlormethylbenzol das (1RS, 2RS, 3RS, 5SR)-3-(4-Chlor-phenyl)-2-(3,4-dichlor-benzyloxy)-8-azabicyclo[3.2.1]octan, MS: 236 $(M-C_7H_5Cl_2)^+$, welches mit Chlorwasserstoff in Äthanol in das Hydrochlorid vom Schmp. 211-213° überführt wurde;

3) - aus (1RS, 2RS, 3RS, 5SR)-3-(4-Chlor-phenyl)-8-methyl-8-azabicyclo[3.2.1]octan-2-ol und 1-Chlor-methyl-4-methoxy-benzol das (1RS, 2RS, 3RS, 5SR)-3-(4-Chlor-phenyl)-2-(4-methoxy-benzyloxy)-8-azabicyclo[3.2.1]octan, MS: 358 $(M+H)^+$, welches mit Methansulfonsäure in Dioxan/Wasser und anschließender Lyophilisation in das entsprechende Methansulfonat überführt wurde, $R_f$: 0.26 (Kieselgel, Methylenchlorid:Methanol: Ammoniak=200: 10:1);

4) - aus (1RS, 2RS, 3RS, 5SR)-3-(4-Chlor-phenyl)-8-methyl-8-azabicyclo[3.2.1]octan-2-ol und 3-Chlormethyl-benzo[b]thiophen (J. Am.Chem. Soc. 71, 2856 (1949) das (1RS, 2RS, 3RS, 5SR)-2-(Benzo[b]thiophen-2-ylmethoxy)-3-(4-chlor-phenyl)-8-azabicyclo[3.2.1]octan , MS: 236 $(M-C_8H_7S)^+$, welches mit Chlorwasserstoff in Äthanol in das Hydrochlorid vom Schmp. 196-198°C (Zers.) überführt wurde;

5) - aus (1RS, 2RS, 3RS, 5SR)-3-(4-Chlor-phenyl)-8-methyl-8-azabicyclo[3.2.1]octan-2-ol und 4'-Brommethyl-biphenyl-2-carbonsäuremethylester [J. Med.Chem. 34, 2525 (1991) der (1RS, 2RS, 3RS, 5SR)-4'-[3-(4-Chlor-phenyl)-8-aza-bicyclo [3.2.1] oct-2-yloxymethyl]-biphenyl-2-carbonsäuremethylester, MS: 236 $(M-C_{15}H_{13}O_2)^+$, welcher mit Chlorwasserstoff in Äthanol in das Hydrochlorid vom Schmp. 101-103°C (Zers.) überführt wurde;

6) - aus (1RS, 2RS, 3RS, 5SR)-3-(4-Chlor-phenyl)-8-methyl-8-azabicyclo[3.2.1]octan-2-ol und 6-Chlormethyl-1,1,4,4,-tetramethyl-1,2,3,4-tetrahydronaphthalin das (1RS, 2RS, 3RS, 5SR)-3-(4-Chlor-phenyl)-2-(5,5,8,8-tetramethyl-5,6,7,8-tetrahydro-naphthalin-2-ylmethoxy)-8-azabicyclo[3.2.1]octan, MS: 437 $(M)^+$, welches mit Chlorwasserstoff in Äthanol in das Hydrochlorid vom Schmp. 87-90°C (Zers.) überführt wurde.

Beispiel 84

(a) In analoger Weise zu dem im Beispiel 12 (c) beschriebenen Verfahren, erfolgte die Abspaltung der N-Methyl-Gruppe durch Umsetzung von Chlorameisensäure 2,2,2-trichloräthylester mit (1RS, 2RS, 3RS, 5SR)-3-(4-Fluor-phenyl)-8-methyl-8-aza-bicyclo[3.2.1]octan-2-ol , das analog zu dem 4-Chlor-phenyl-Derivat [J. Org. Chem. 35, 802, 1970)] erhalten worden war. Dabei wurde der (1RS,2RS,3RS,5RS)-3-(4-Fluor-phenyl)-2-(2,2,2-trichloräthoxycarbonyloxy)-8-azabicyclo[3.2.1]octan-8-carbonsäure 2,2,2-trichlor-äthylester als gelblicher Festkörper erhalten; MS: 587, 589, 591, 593 $(M+NH_4)^+$.

(b) Durch die Abspaltung des 2,2,2-Trichloräthylcarbamats und 2,2,2-Trichloräthylcarbonats wurde, in analoger Weise wie im Beispiel 12 (d) beschrieben, aus dem (1RS,2RS,3RS,5RS)-3-(4-Fluor-phenyl)-2-(2,2,2-trichloräthoxycarbonyloxy)-8-aza-bicyclo[3.2.1]octan-8-carbonsäure 2,2,2-trichlor-äthylester das (1RS,2RS,3RS,5RS)-3-(4-Fluor-phenyl)-8-aza-bicyclo[3.2.1]octan-2-ol als farbloser Festkörper erhalten; MS: 221 $(M)^+$.

(c) In Analogie zu dem im Beispiel 1 (f) beschriebenen Verfahren wurde durch Einführung der BOC-Gruppe aus dem (1RS,2RS,3RS,5RS)-3-(4-Fluor-phenyl)-8-aza-bicyclo[3.2.1]octan-2-ol der (1RS,2RS,3RS,5RS)-3-(4-Fluor-phenyl)-2-hydroxy-8-aza-bicyclo[3.2.1]octan-8-carbonsäure tert-butylester als farbloser Schaum erhalten; MS: 265 $(M-C_4H_8)^+$.

(d) Die Alkylierung des (1RS,2RS,3RS,5RS)-3-(4-Fluor-phenyl)-2-hydroxy-8-aza-bicyclo[3.2.1]octan-8-carbonsäure tert-butylesters mit 1-Benzyloxy-3-chlormethyl-naphthalin (Beispiel 19), analog zu dem im Beispiel 1 (g) beschriebenen Verfahren, lieferte den (1RS,2RS,3RS,5SR)-2-(4-Benzyloxy-naphthalin-2-ylmethoxy)-3-(4-fluor-phenyl)-8-azabicyclo[3.2.1]octan-8-carbonsäure tert-butylester als farblosen Festkörper; MS: 568 $(M+H)^+$.

(e) Die Abspaltung der BOC-Gruppe mittels Chlorwasserstoff in Äthanol, analog dem im Beispiel 22 (1) beschriebenen Verfahren, ergab das (1RS,2RS,3RS,5SR)-2-(4-Benzyloxy-naphthalin-2-ylmethoxy)-3-(4-fluor-phenyl)-8-aza-bicyclo[3.2.1]octan als beigefarbenen Festkörper; MS: 468 $(M+H)^+$.

Beispiel 85

Eine Lösung von 0.330 g (0.71 mMol) (1RS, 2RS, 3RS, 5SR)-4'-[3-(4-Chlor-phenyl)-8-aza-bicyclo[3.2.1]oct-2-yloxymethyl]-biphenyl-2-carbonsäuremethylester in 10 ml Äther wurde langsam zu einer Suspension von 33 mg Lithiumaluminiumhydrid in 5 ml Äther getropft und 4 Stunden bei Raumtemperatur gerührt. Nach Zugabe von wässerigem Äther und anschließend Wasser wurden die Phasen getrennt, die organische Phase mit gesättigter Kochsalzlösung gewaschen, über Natriumsulfat getrocknet, filtriert und eingedampft. Der Rückstand wurde an Kieselgel unter Verwendung eines 140:10:1-Gemisches von Methylenchlorid, Methanol und Ammoniak als Eluierungsmittel chromatographiert. Es wurden 0.210 g (68% d.Th.) (1RS, 2RS, 3RS, 5SR)-[4'-[3-(4-Chlor-phenyl)-8-aza-bicyclo[3.2.1]oct-2-yloxymethyl]-biphenyl-2-yl]-methanol als farbloser Schaum erhalten, $R_f$: 0.18 ($SiO_2$, Methylenchlorid:Methanol:Ammoniak=140:10:1), MS: 434 $(M+H)^+$.

Beispiel 86

Durch Abspaltung der BOC-Gruppe wurden die folgenden Verbindungen hergestellt:

1) Aus dem (3RS,4RS)-3-(Naphthalin-2-ylmethoxy)-4-(4-prop-2-ynyloxy-phenyl)-piperidin-1-carbonsäure tert-butylester mit Trifluoressigsäure das (3RS,4RS)-3-(Naphthalin-2-ylmethoxy)-4-[4-(prop-2-ynyloxy)-phenyl]-piperidin Trifluoracetat als farbloser Festkörper; Smp.: 186° C (Zers.);

2) aus dem (3RS,4RS)-4-(4-Allyloxy-phenyl)-3-(naphthalin-2-ylmethoxy)-piperidin-1-carbonsäure tert-butylester mit Salzsäure in Methanol das (3RS,4RS)-4-(4-Allyloxy-phenyl)-3-naphthalin-2-ylmethoxy-piperidin als leicht gelbes Öl; MS : 374 $(M+H)^+$;

3) aus dem Gemisch des (3RS,4RS)- und (3SR,4SR)-4-[4-[(RS)-2,2-dimethyl-[1,3]dioxolan-4-ylmethoxy]-phenyl]-3-(naphthalin-2-ylmethoxy)-piperidin-1-carbonsäure tert-butylesters mit Salzsäure in Methanol unter gleichzeitiger Abspaltung der Isopropylidengruppe das Gemisch des (RS)- und (SR)-3-[(3RS,4RS)-4-[3-(Naphthalin-2-ylmethoxy)-piperidin-4-yl]-phenoxy]-propan-1,2-diols als gelblicher Festkörper; MS : 408 $(M+H)^+$;

4) aus dem Gemisch des (3RS,4RS)- und (3SR,4SR)-4-[4-[(RS)-2-hydroxy-3-phenoxy-propoxy]-phenyl]-3-(naphthalin-2-ylmethoxy)-piperidin-1-carbonsäure tert-butylesters mit Trifluoressigsäure das Gemisch des (RS)- und (SR)-1-[4-[(3RS,4RS)-3-(Naphthalin-2-ylmethoxy)-piperidin-4-yl]-phenoxy]-3-phenoxy-propan-2-ol Trifluoracetats als weißer Festkörper; MS : 484 $(M+H)^+$;

5) aus dem Gemisch des (3RS,4RS)- und (3SR,4SR)-4-[4-[(RS)-2-benzyloxy-3-methoxy-propoxy]-phenyl]-3-(naphthalin-2-ylmethoxy)-piperidin-1-carbonsäure tert-butylesters mit Trifluoressigsäure das Gemisch des (RS)- und (SR)-4-[(3RS,4RS)-4-(2-Benzyloxy-3-methoxypropoxy)-phenyl]-3-(naphthalin-2-ylmethoxy)-piperidin Trifluoracetats als weißer Festkörper; Smp.: 138-139 °C.

6) aus dem Gemisch des (3RS,4RS)- und (3SR,4SR)-4-[4-[(RS)-2-hydroxy-3-phenylsulfanyl-propoxy]-phenyl]-3-(naphthalin-2-ylmethoxy)-piperidin-1-carbonsäure tert-butylesters mit Trifluoressigsäure das Gemisch des (RS)- und (SR)-1-[(3RS,4RS)-4-[3-(Naphthalin-2-yloxymethyl)-piperidin-4-yl]-phenoxy]-3-phenylsulfanyl-propan-2-ol Trifluoracetats als weißer Festkörper; MS : 500 $(M+H)^+$;

7) aus dem Gemisch des (3RS,4RS)- und (3SR,4SR)-4-[4-[(RS)-2-Methoxy-3-phenoxy-propoxy)-phenyl]-3-(naphthalin-2-ylmethoxy)-piperidin-1-carbonsäure tert-butylesters mit Trifluoressigsäure das Gemisch des (3RS,4RS)-4-[4-[(RS) und [(SR)-2-Methoxy-3-phenoxypropoxy]-phenyl]-3-(naphthalin-2-ylmethoxy)-piperidin Trifluoracetats als weißer Festkörper; MS : 498 $(M+H)^+$;

8) aus dem Gemisch des (3RS,4RS)- und (3SR,4SR)-4-[4-[(RS)-2-Benzoyloxy-3-methoxy-propoxy] -phenyl] -3-(naphthalin-2-ylmethoxy)-piperidin-1-carbonsäure tert-butylesters mit Trifluoressigsäure das Gemisch des (RS)- und (SR)- Benzoesäure 1-Methoxymethyl-2-[4-[(3RS,4RS)-3-(naphthalin-2-ylmethoxy)-piperidin-4-yl]-phenoxy]-äthylester Trifluoracetats als weißer Festkörper; MS : 526 $(M+H)^+$;

9) aus dem Gemisch des (3RS,4RS)-4-[4-[(RS)- und [(SR)-(3-Benzyloxy-2-methoxy-propoxy)-phenyl]-3-(naphthalin-2-ylmethoxy)-piperidin-1-carbonsäure tert-butylesters das Gemisch des (3RS,4RS)-4-[4-[(RS)- und - [(SR)-3-Benzyloxy-2-methoxypropoxy]-phenyl]-3-(naphthalin-2-ylmethoxy)-piperidin Trifluoracetats als weißer Festkörper; MS : 512 $(M+H)^+$;

10) aus dem (3RS,4RS)-3-(Naphthalin-2-ylmethoxy)-4-{4-[2-(pyridin-3-ylmethoxy)-äthoxy]-phenyl}-piperidin-1-carbonsäure tert-butylester mit Chlorwasserstoff in Methanol das (3RS,4RS)-3-(2-[4-[3-(Naphthalin-2-ylmethoxy)-piperidin-4-yl]-phenoxy]-äthoxymethyl)-pyridin als farbloses Harz; MS : 469 $(M+H)^+$;

11) aus dem (3RS,4RS)-4-{4-[2-(Pyridin-3-ylmethoxy)-äthoxy]-phenyl}-3-[4-(2-trimethylsilanyl-äthoxymethoxy)-naphthalin-2-ylmethoxy]-piperidin-1-carbonsäure tert-butylester mit Chlorwasserstoff in Methanol unter gleichzeitiger Abspaltung der SEM-Gruppe das (3RS,4RS)-3-(4-[4-[2-(Pyridin-3-ylmethoxy)-äthoxy]-phenyl]-piperidin-3-yloxymethyl)-naphthalin-1-ol als farbloses Harz; MS : 485$(M+H)^+$;

12) aus dem (3RS,4RS)-3-(Naphthalin-2-ylmethoxy)-4-{4-[2-(pyridin-4-ylmethoxy)-äthoxy]-phenyl}-piperidin-1-carbonsäure tert-butylester mit Chlorwasserstoff in Methanol das (3RS,4RS)-4-(2-{4-[3-(Naphthalin-2-ylmethoxy)-piperidin-4-yl]-phenoxy}-äthoxymethyl)-pyridin als farbloses Harz; MS : 469 $(M+H)^+$;

13) aus dem (3RS,4RS)-3-(Naphthalin-2-ylmethoxy)-4-{4-[2-(pyridin-2-ylmethoxy)-äthoxy]-phenyl}-piperidin-1-carbonsäure tert-butylester mit Chlorwasserstoff in Methanol das (3RS,4RS)-2-(2-{4-[3-(Naphthalin-2-ylmethoxy)-piperidin-4-yl]-phenoxy}-äthoxymethyl)-pyridin als farbloses Harz; MS : 469 $(M+H)^+$;

14) aus dem (3RS,4RS)-4-[4-(3-Benzyloxy-propoxy)-phenyl]-3-(naphthalin-2-ylmethoxy)-piperidin-1-carbonsäure tert-butylester mit Chlorwasserstoff in Methanol das (3RS,4RS)-4-[4-(3-Benzyloxy-propoxy)-phenyl]-3-(naphthalin-2-ylmethoxy)-piperidin als farbloses Harz; MS : 482 $(M+H)^+$;

15) aus dem (3RS,4RS)-3-(Naphthalin-2-ylmethoxy)-4-[4-[3-(pyridin-2-ylmethoxy)-propoxy]-phenyl]-piperidin-1-carbonsäure tert-butylester mit Chlorwasserstoff in Methanol das (3RS,4RS)-2-(3-{4-[3-(Naphthalin-2-ylmethoxy)-piperidin-4-yl]-phenoxy}-propoxymethyl)-pyridin als farbloser Festkörper; MS : 483 $(M+H)^+$;

16) aus dem (3RS,4RS)-3-(Naphthalin-2-ylmethoxy)-4-[4-[3-(pyridin-2-ylmethoxy)-propyl]-phenyl]-piperidin-1-carbonsäure tert-butylester mit Chlorwasserstoff in Methanol das (3RS,4RS)-2-[3-[4-[3-(Naphthalin-2-ylmethoxy)-piperidin-4-yl]-phenyl]-propoxymethyl]-pyridin als farbloser, amorpher Festkörper; MS : 467 $(M+H)^+$;

17) aus dem (3RS,4RS)-4-[4-[3-(Benzyl-methyl-amino)-propoxy]-phenyl]-3-(naphthalin-2-ylmethoxy)-piperidin-1-carbonsäure tert-butylester mit Chlorwasserstoff in Methanol das (3RS,4RS)-Benzyl-methyl-(3-{4-[3-(naphthalin-2-ylmethoxy)-piperidin-4-yl]-phenoxy}-propyl)-amin als farbloser Festkörper; MS : 495 $(M+H)^+$;

18) aus dem (3RS,4RS)-4-[4-[3-(Benzothiazol-2-ylsulfonyl)-propoxy]-phenyl]-3-(1-methoxy-naphthalin-2-ylmethoxy)-piperidin-1-carbonsäure tert-butylester mit Chlorwasserstoff in Methanol das (3RS,4RS)-2-[3-[4-[3-(1-Methoxy-naphthalin-2-ylmethoxy)-piperidin-4-yl]-phenoxy]-propylsulfonyl]-benzothiazol als farbloser Schaum; MS : 571 $(M+H)^+$;

19) aus dem (3RS,4RS)-3-(Naphthalin-2-ylmethoxy)-4-[4-(3-phenylsulfanyl-propyl)-phenyl]-piperidin-1-carbonsäure tert-butylester mit Chlorwasserstoff in Methanol das (3RS,4RS)-3-(Naphthalin-2-ylmethoxy)-4- [4-(3-phenylsulfanyl-propyl)-phenyl]-piperidin als weißer Festkörper; MS : 468 $(M+H)^+$;

20) aus dem (3RS,4RS)-4-{4-[3-(Benzothiazol-2-ylsulfanyl)-propyl]-phenyl)-3-(naphthalin-2-ylmethoxy)-piperidin-1-carbonsäure tert-butylester mit Chlorwasserstoff in Methanol das (3RS,4RS)-2-(3-{4-[3-(Naphthalin-2-ylmethoxy)-piperidin-4-yl]-phenyl}-propylsulfanyl)-benzothiazol als weißer Festkörper; MS : 525 $(M+H)^+$;

21) aus dem (3RS,4RS)-4-{4-[2-(Pyrimidin-2-ylsulfanyl)-äthyl]-phenyl}-3-[4-(2-trimethylsilanyl-äthoxymethoxy)-naphthalin-2-ylmethoxy]-piperidin-1-carbonsäure tert-butylester mit Chlorwasserstoff in Methanol unter gleichzeitiger Abspaltung der SEM-Gruppe das (3RS,4RS)-3-[4-[4-[2-(Pyrimidin-2-ylsulfanyl)-äthyl]-phenyl]-piperidin-3-yloxymethyl]-naphthalin-1-ol als farbloser Schaum;MS : 472 $(M+H)^+$;

22) aus dem (3RS,4RS)-4-{4-[2-(Pyridin-2-ylsulfanyl)-äthyl]-phenyl}-3-[4-(2-trimethylsilanyl-äthoxymethoxy)-naphthalin-2-ylmethoxy]-piperidin-1-carbonsäure tert-butylester mit Chlorwasserstoff in Methanol unter gleichzeitiger Abspaltung der SEM-Gruppe das (3RS,4RS)-3-(4-{4-[2-(Pyridin-2-ylsulfanyl)-äthyl]-phenyl}-piperidin-3-yloxymethyl)-naphthalin-1-ol; MS : 471 $(M+H)^+$;

23) aus dem (3RS,4RS)-4-[4-[2-(Benzothiazol-2-ylsulfanyl)-äthyl]-phenyl)-3-[4-(2-trimethylsilanyl-äthoxymethoxy)-naphthalin-2-ylmethoxyl-piperidin-1-carbonsäure tert-butylester mit Chlorwasserstoff in Methanol unter gleichzeitiger Abspaltung der SEM-Gruppe das (3RS,4RS)-3-[4-[4-[2-(Benzothiazol-2-ylsulfanyl)-äthyl]-phenyl]-piperidin-3-yloxymethyl]-naphthalin-1-ol als farbloser Festkörper; MS : 527 $(M+H)^+$;

24) aus dem (3RS,4RS)-3-(Naphthalin-2-ylmethoxy)-4-[4-(pyridin-3-ylmethoxymethyl)-phenyl]-piperidin-1-carbonsäure tert-butylester mit Chlorwasserstoff in Methanol das (3RS,4RS)-3-[4-[3-(Naphthalin-2-ylmethoxy)-piperidin-4-yl]-benzyloxymethyl]-pyridin als farbloser Schaum; MS : 438 $(M)^+$;

25) aus dem Gemisch des (3RS,4RS)-4-(4-{2-[(RS)-2- und (SR)-2-(4-Fluor-phenyl)-3-methyl-butyryloxy]-äthoxy}-phenyl)-3-(naphthalin-2-ylmethoxy)-piperidin-1-carbonsäure tert-butylesters mit Zinkbromid in Methylenchlorid das Gemisch des (RS)- und (SR)-2-(4-Fluor-phenyl)-3-methyl-buttersäure (3RS,4RS)-2-[4-(3-Naphthalin-2-ylmethoxypiperidin-4-yl)-phenoxy]-äthylester Hydrobromids als weißer Festkörper; MS : 556 $(M+H)^+$;

26) aus dem (3RS,4RS)-4-[4-[2-(1-Methyl-1H-pyrrol-2-carbonyloxy)-äthoxy]-phenyl]-3-(naphthalin-2-ylmethoxy)-piperidin-1-carbonsäure tert-butylester mit Zinkbromid in Methylenchlorid das 1-Methyl-1Hpyrrol-2-carbonsäure (3RS,4RS)-[4-[3-(Naphthalin-2-ylmethoxy)-piperidin-4-yl]-phenoxy]-äthylester Hydrobromid als beiger Festkörper; MS : 485 $(M+H)^+$;

27) aus dem (3RS,4RS)-4-[4-(3-Benzoyloxy-propyl)-phenyl]-3-(naphthalin-2-ylmethoxy)-piperidin-1-carbonsäure tert-butylester mit Chlorwasserstoff in Methanol der (3RS,4RS)-Benzoesäure 3-{4-[3-(Naphthalin-2-ylmethoxy)-piperidin-4-yl]-phenyl}-propylester als gelber Sirup; MS: 480 $(M+H)^+$;

28) aus dem (3RS,4RS)-4-{4-[3-(3-Methoxy-benzoyloxy)-propyl]-phenyl}-3-(naphthalin-2-ylmethoxy)-piperidin-1-carbonsäure tert-butylester mit Chlorwasserstoff in Methanol der (3RS,4RS)-3-Methoxy-benzoesäure 3-{4-[3-(Naphthalin-2-ylmethoxy)-piperidin-4-yl]-phenyl}-propylester als gelber Festkörper; MS : 510 $(M+H)^+$;

29) aus dem (3RS,4RS)-4-[4-(3-Methoxy-benzoyloxymethyl)-phenyl]-3-[1-(2-trimethylsilanyl-äthoxymethoxy)-naphthalin-2-ylmethoxy]-piperidin-1-carbonsäure tert-butylester mit Chlorwasserstoff in Methanol unter gleichzeitiger Abspaltung der SEM-Gruppe der 3-Methoxy-benzoesäure (3RS,4RS)-4-[3-(1-Hydroxy-naphthalin-2-ylmethoxy)-piperidin-4-yl]-benzylester als farbloser Schaum; MS : 498 $(M+H)^+$;

30) aus dem (3RS,4RS)-4-(4-Äthoxycarbonylmethoxy-phenyl)-3-(naphthalin-2-ylmethoxy)-piperidin-1-carbonsäure tert-butylester mit Trifluoressigsäure das (3RS,4RS)-[4-[3-(Naphthalin-2-ylmethoxy)-piperidin-4-yl]-phenoxy]-essigsäureäthylester Trifluoracetat als weißer Festkörper; MS : 420 $(M+H)^+$;

31) aus dem (3RS,4RS)-4-[4-(Benzylcarbamoyl-methoxy)-phenyl]-3-(naphthalin-2-ylmethoxy)-piperidin-1-carbonsäure tert-butylester mit Trifluoressigsäure das (3RS,4RS)-N-Benzyl-2-[4-[3-(naphthalin-2-ylmethoxy)-piperidin-4-yl]-phenoxy]-acetamid Trifluoracetat als weißer Festkörper; Smp.: 185 °C;

32) aus dem (3RS,4RS)-3-(Naphthalin-2-ylmethoxy)-4-[4-(pyridin-2-ylcarbamoyloxy)-phenyl]-piperidin-1-carbonsäure tert-butylester mit Trifluoressigsäure das Pyridin-2-yl-carbaminsäure (3RS,4RS)-4-[3-(Naphthalin-2-ylmethoxy)-piperidin-4-yl]-3-phenylester Trifluoracetat als weißer Festkörper; Smp.: 158 °C

33) aus dem (3RS,4RS)-4-(4-Carboxymethoxy-phenyl)-3-(naphthalin-2-ylmethoxy)-piperidin-1-carbonsäure tert-butylester [Beispiel 86 (ee)] mit Trifluoressigsäure das (3RS,4RS)-[4-[3-(Naphthalin-2-ylmethoxy)-piperidin-4-yl]-phenoxy]-essigsäure Trifluoracetat als weißer Festkörper; Smp.: 183-184 °C;

34) aus dem (3RS,4RS)-3-(Naphthalin-2-ylmethoxy)-4-[4-(2-oxo-3-phenoxypropoxy)-phenyl]-piperidin-1-carbonsäure tert-butylester mit Trifluoressigsäure das (3RS,4RS)-1-[4-[3-(Naphthalin-2-ylmethoxy)-piperidin-4-yl]-phenoxy]-3-phenoxy-propan-2-on Trifluoracetat als weißer Festkörper; Smp.: 145-146 °C;

35) aus dem (3RS,4RS)-4-[4-(2-Äthoxycarbonyl-äthyl)-phenyl]-3-(naphthalin-2-ylmethoxy)-piperidin-1-carbonsäure tert-butylester [Beispiel 24 (s)] mit Zinkbromid in Methylenchlorid der (3RS,4RS)-3-{4-[3-(Naphthalin-2-ylmethoxy)-piperidin-4-yl]-phenyl}-propionsäureäthylester als gelblicher Sirup; MS : 418 $(M+H)^+$;

36) aus dem Gemisch des (3RS,4RS)-4-[4-[(RS)-2- und [(SR)-2-Hydroxy-2-phenyl-äthyl]-phenyl]-3-(naphthalin-2-ylmethoxy)-piperidin-1-carbonsäure tert-butylesters mit Chlorwasserstoff in Methanol das Gemisch des (RS)- und (SR)-2-[4-[(3RS,4RS)-3-Naphthalin-2-ylmethoxypiperidin-4-yl]-phenyl]-1-phenyl-äthanol Hydrochlorids; MS : 438 $(M+H)^+$;

37) aus dem (3RS,4RS)-4-(4-Fluor-phenyl)-3-(2-{[(pyridin-2-carbonyl)-amino]-methyl}-benzyloxy)-piperidin-1-carbonsäure tert-butylester mit Chlorwasserstoff in Methanol das Pyridin-2-carbonsäure (3RS,4RS)-2-[4-(4-fluor-phenyl)-piperidin-3-yloxymethyl]-benzylamid Dihydrochlorid als weißer Festkörper; MS : 420 $(M+H)^+$;

38) aus dem (3RS,4RS)-3-(3-Benzoyl-benzyloxy)-4-(4-fluor-phenyl)-piperidin-1-carbonsäure tert-butylester mit Chlorwasserstoff in Methanol das (3RS,4RS)-[3-[4-(4-Fluor-phenyl)-piperidin-3-yloxymethyl]-phenyl]-phenyl-methanon Hydrochlorid als weißer, amorpher Festkörper; MS: 390 $(M+H)^+$;

39) aus dem (3RS,4RS)-3-(Naphthalin-2-ylmethoxy)-4-[4-[2-(5-phenyl-[1,2,4]oxadiazol-3-yl)-äthyl]-phenyl]-piperidin-1-cärbonsäure tert-butylester mit Chlorwasserstoff in Methanol das (3RS,4RS)-3-(Naphthalin-2-ylmethoxy)-4-[4-[2-(5-phenyl-[1.2.4]oxadiazol-3-yl)-äthyl]-phenyl]-piperidin als weißer Festkörper; MS : 490 $(M+H)^+$;

40) aus dem (3RS,4RS)-3-(Naphthalin-2-ylmethoxy)-4-[4-[2-(3-phenyl-[1,2,4)oxadiazol-5-yl)-äthyl)-phenyl)-piperidin-1-carbonsäure tert-butylester mit Chlorwasserstoff in Methanol das (3RS,4RS)-3-(Naphthalin-2-ylmethoxy)-4-[4-[2-(3-phenyl-[1.2.4]oxadiazol-5-yl)-äthyl]-phenyl]-piperidin als weißer Festkörper; MS : 490 $(M+H)^+$;

41) aus dem (3RS,4RS)-3-(Naphthalin-2-ylmethoxy)-4-[4-(3-phenyl-[1,2,4]oxadiazol-5-ylmethoxy)-phenyl]-piperidin-1-carbonsäure tert-butylester mit Trifluoressigsäure das (3RS,4RS)-3-(Naphthalin-2-ylmethoxy)-4-[4-(3-phenyl-[1,2,4] oxadiazol-5-ylmethoxy)-phenyl]-piperidin Trifluoracetat als weißer Festkörper; Smp.: 195-196 °C;

42) aus dem (3RS,4RS)-3-(Naphthalin-2-ylmethoxy)-4-[4-(3-pyridin-3-yl-[1,2,4] oxadiazol-5-ylmethoxy)-phenyl] -piperidin-1-carbonsäure tert-butylester mit Trifluoressigsäure das (3RS,4RS)-3-(5-[4-[3-(Naphthalin-2-ylmethoxy)-piperidin-4-yl]-phenoxymethyl]-[1,2,4]oxadiazol-3-yl)-pyridin Trifluoracetat als weißer Festkörper; MS : 493 $(M+H)^+$;

43) aus dem (3RS,4RS)-3-(Naphthalin-2-ylmethoxy)-4-[4-(3-phenylisoxazol-5-ylmethoxy)-phenyl]-piperidin-1-carbonsäure tert-butylester mit Trifluoressigsäure das (3RS,4RS)-3-(Naphthalin-2-ylmethoxy)-4-[4-(3-phenylisoxazol-5-ylmethoxy)-phenyl]-piperidin Trifluoracetat als weißer Festkörper; MS : 491 $(M+H)^+$;

44) aus dem (3RS,4RS)-4-[4-(3-Benzyloxy-propoxy)-phenyl]-3-(3-methoxy-benzyloxy)-piperidin-1-carbonsäure tert-butylester mittels Chlorwasserstoff in Methanol das (3RS,4RS)-4-[4-(3-Benzyloxy-propoxy)-phenyl]-3-(3-methoxy-benzyloxy)-piperidin Hydrochlorid als farbloses Öl; MS : 462 $(M+H)^+$;

45) aus dem (3RS,4RS)-4-[4-(3-Benzyloxy-propoxy)-phenyl]-3-[3-(2-trimethylsilanyl-äthoxymethoxy)-benzyloxy]-piperidin-1-carbonsäure tert-butylester mit Chlorwasserstoff in Methanol unter gleichzeitiger Abspaltung der SEM-Gruppe das (3RS,4RS)-3-{4-[4-(3-Benzyloxypropoxy)-phenyl]-piperidin-3-yloxymethyl}-phenol als farbloses Öl; MS: 448 $(M+H)^+$;

46) aus dem (3RS,4RS)-4-[4-(3-Benzyloxy-propoxy)-phenyl]-3-(3-chlor-4-methoxy-benzyloxy)-piperidin-1-carbonsäure tert-butylester mit Zinkbromid in Methylenchlorid das (3RS,4RS)-4-[4-(3-Benzyloxypropoxy)-phenyl]-3-(3-chlor-4-methoxy-benzyloxy)-piperidin Hydrobromid als farbloses Öl; MS : 497 $(M+H)^+$;

47) aus dem (3RS,4RS)-4-[4-(3-Benzyloxy-propoxy)-phenyl]-3-(3,4-dichlor-benzyloxy)-piperidin-1-carbonsäure tert-butylester mittels Chlorwasserstoff in Methanol das (3RS,4RS)-4-[4-(3-Benzyloxy-propoxy)-phenyl]-3-(3,4-dichlor-benzyloxy)-piperidin Hydrochlorid als farbloses Öl; MS : 500 $(M)^+$;

48) aus dem (3RS,4RS)-4-[4-(3-Benzyloxy-propoxy)-phenyl]-3-(2,5-dichlor-benzyloxy)-piperidin-1-carbonsäure tert-butylester mittels Chlorwasserstoff in Methanol das (3RS,4RS)-4-[4-(3-Benzyloxy-propoxy)-phenyl]-3-(2,5-dichlor-benzyloxy)-piperidin Hydrochlorid als farbloses Öl; MS : 500 $(M)^+$;

49) aus dem (3RS,4RS)-3-Benzyloxy-4-[4-(3-benzyloxy-propoxy)-phenyl]-piperidin-1-carbonsäure tert-butylester mit Zinkbromid in Methylenchlorid das (3RS,4RS)-3-Benzyloxy-4-[4-(3-benzyloxy-propoxy)-phenyl)-piperidin Hydrobromid als farbloser Festkörper; MS : 432 $(M+H)^+$;

50) aus dem (3RS,4RS)-4-[4-(3-Benzyloxy-propoxy)-phenyl]-3-(2,5-dimethyl-benzyloxy)-piperidin-1-carbonsäure tert-butylester mit Zinkbromid in Methylenchlorid das (3RS,4RS)-4-[4-(3-Benzyloxypropoxy)-phenyl]-3-(2,5-dimethyl-benzyloxy)-piperidin Hydrobromid als farbloses Öl; MS : 460 $(M+H)^+$;

51) aus dem (3RS,4RS)-4-[4-(3-Benzyloxy-propoxy)-phenyl]-3-(4-äthylbenzyloxy)-piperidin-1-carbonsäure tert-butylester mit Zinkbromid in Methylenchlorid das (3RS,4RS)-4-[4-(3-Benzyloxy-propoxy)-phenyl]-3-(4-äthyl-benzyloxy)-piperidin als farbloses Öl; MS: 460 $(M+H)^+$;

52) aus dem Gemisch des (3RS,4RS)-4-[4-(3-Benzyloxy-propoxy)-phenyl]-3-(3- und 4-vinyl-benzyloxy)-piperidin-1-carbonsäure tert-butylesters mit Zinkbromid in Methylenchlorid das Gemisch des (3RS,4RS)-4-[4-(3-Benzyloxy-propoxy)-phenyl]-3-(3 und 4-vinylbenzyloxy)-piperidin Hydrobromids als farbloser Festkörper; MS : 458 $(M+H)^+$;

53) aus dem (3RS,4RS)-4-[4-(3-Benzyloxy-propoxy)-phenyl]-3-(2,3-dihydro-benzo[1,4]dioxin-6-ylmethoxy)-piperidin-1-carbonsäure tert-butylester mit Zinkbromid in Methylenchlorid das (3RS,4RS)-4-[4-(3-Benzyloxy-propoxy)-phenyl]-3-(2,3-dihydro-benzo[1,4]dioxin-6-ylmethoxy)-piperidin als farbloses Harz; MS : 490 $(M+H)^+$;

54) aus dem (3RS,4RS)-4-[4-(3-Benzyloxy-propoxy)-phenyl]-3-(5,6,7,8-tetrahydro-naphthalin-2-ylmethoxy)-piperidin-1-carbonsäure tert-butylester mit Zinkbromid in Methylenchlorid das (3RS,4RS)-4-[4-(3-Benzyloxy-propoxy)-phenyl]-3-(5,6,7,8-tetrahydro-naphthalin-2-ylmethoxy)-piperidin als farbloses Harz; MS : 486 $(M+H)^+$;

55) aus dem (3RS,4RS)-4-[4-(3-Benzyloxy-propoxy)-phenyl]-3-[4-(2-trimethylsilanyl-äthoxymethoxy)-naphthalin-2-ylmethoxy]-piperidin-1-carbonsäure tert-butylester mit Chlorwasserstoff in Methanol unter gleichzeitiger Abspaltung der SEM-Gruppe das (3RS,4RS)-3-{4-[4-(3-Benzyloxy-propoxy)-phenyl]-piperidin-3-yloxymethyl}-naphthalin-1-ol als farbloses Harz; MS : 498 (M+H)+;

56) aus dem (3RS,4RS)-4-[4-(4-Benzyloxy-butoxy)-phenyl]-3-(naphthalin-2-ylmethoxy)-piperidin-1-carbonsäure

tert-butylester mit Chlorwasserstoff in Methanol das (3RS,4RS)-4-[4-(4-Benzyloxy-butoxy)-phenyl]-3-(naphthalin-2-ylmethoxy)-piperidin als farbloses Öl; MS : 496 $(M+H)^+$;

57) aus dem (3SR,4RS,5RS)-4-[4-(2-Chloro-benzoyloxymethyl)-phenyl]-3-methoxymethyl-5-(naphthalin-2-ylmethoxy)-piperazin-1-carbonsäure tert-butylester mit Chlorwasserstoff in Methanol der (3SR,4RS,5RS)-2-Chlor-benzoesäure 4-[3-Methoxymethyl-5-(naphthalin-2-ylmethoxy)-piperidin-4-yl]-benzylester als farbloser Schaum; MS : 530 $(M+H)^+$;

58) aus dem (3SR,4RS,5RS)-4-(4-Methoxycarbonyl-phenyl)-3-methoxymethyl-5-(naphthalin-2-ylmethoxy)-piperidin-1-carbonsäure tert-butylester mit Chlorwasserstoff in Methanol der (3SR,4RS,5RS)-4-[3-Methoxymethyl-5-naphthalin-2-ylmethoxy-piperidin-4-yl]-benzoesäuremethylester als farbloser Festkörper; MS : 420 $(M+H)^+$;

59) aus dem (3SR,4RS,5RS)-4-(4-Benzyloxymethyl-phenyl)-3-methoxymethyl-5-(naphthalin-2-ylmethoxy)-piperidin-1-carbonsäure tert-butylester mit Chlorwasserstoff in Methanol das (3SR,4RS,5RS)-4-(4-Benzyloxymethyl-phenyl)-3-methoxymethyl-5-(naphthalin-2-ylmethoxy)-piperidin als farbloser Schaum; MS : 482 $(M+H)^+$;

60) aus dem (1RS,2RS,3RS ,5SR)-3-[4-(2-Benzyloxy-propoxymethyl)-phenyl] -2-(naphthalin-2-ylmethoxy)-8-aza-bicyclo [3.2.1] octan-8-carbonsäure tert-butylester mit Chlorwasserstoff in Methanol das (1RS,2RS,3RS,5SR)-2-(Naphthalin-2-ylmethoxy)-3-[4-(3-phenoxypropoxymethyl)phenyl]-8-azabicyclo[3.2.1]octan als farbloses Öl; MS: 508 $(M+H)^+$.

Die als Ausgangsstoffe eingesetzten BOC-Derivate wurden wie folgt hergestellt:

(a) In analoger Weise wie im Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Propargylbromid in Gegenwart von Kaliumcarbonat in Aceton der (3RS,4RS)-3-Hydroxy-4-(4-prop-2-ynyloxy-phenyl)-piperidin-1-carbonsäure tert-butylester erhalten, dessen Alkylierung mit 2-Brommethylnaphthalin, in Analogie zu Beispiel 1 (g), den (3RS, 4RS)-3-(Naphthalin-2-ylmethoxy)-4-(4-prop-2-ynyloxy-phenyl)-piperidin-1-carbonsäure tert-butylester als hellgelben Festkörper ergab; MS : 472 $(M+H)^+$.

(b) In analoger Weise wie im Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Allylbromid in Gegenwart von Kaliumcarbonat in Aceton der (3RS,4RS)-4-(4-Allyloxyphenyl)-3-hydroxy-piperidin-1-carbonsäure tert-butylester erhalten, dessen Alkylierung mit 2-Brommethylnaphthalin, in Analogie zu Beispiel 1 (g), den (3RS,4RS)-4-(4-Allyloxy-phenyl)-3-(naphthalin-2-ylmethoxy)-piperidin-1-carbonsäure tert-butylester als farbloses Harz ergab; MS : 474 $(M+H)^+$.

(c) In analoger Weise wie im Beispiel 1 (g) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Methanesulfonsäure (RS)-2,2-Dimethyl-[1,3]dioxolan-4-ylmethylester in Gegenwart von Natriumhydrid das Gemisch des (3RS,4RS)- und (3SR,4SR)-4-[4-[(RS)-2,2-Dimethyl- [1,3) dioxolan-4-ylmethoxy]-phenyl]-3-hydroxy-piperidin-1-carbonsäure tert-butylesters erhalten, dessen Alkylierung mit 2-Brommethylnaphthalin, in Analogie zu Beispiel 1 (g), das Gemisch des (3RS, 4RS)- und (3SR,4SR)-4-[4-[(RS)-2,2-Dimethyl-[1,3]dioxolan-4-ylmethoxy]-phenyl]-3-(naphthalin-2-ylmethoxy)-piperidin-1-carbonsäure tert-butylesters als weißen Festkörper ergab; MS : 547 $(M)^+$.

(d) In analoger Weise wie im Beispiel 1 (g) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit (RS)-2,3-Epoxypropyl p-toluolsulfonat in Gegenwart von Natriumhydrid das Gemisch des (3RS,4RS)- und (3SR,4SR)-3-Hydroxy-4-[(RS)-4-oxiranylmethoxy-phenyl]-piperidin-1-carbonsäure tert-butylesters erhalten, dessen Alkylierung mit 2-Brommethylnaphthalin, in Analogie zu Beispiel 1 (g), das Gemisch des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters ergab. Die anschließende Epoxidöffnung mit Kaliumphenolat, in Analogie zu Beispiel 71 (a), lieferte das Gemisch des (3RS,4RS)- und (3SR, 4SR)-4-[4-[(RS)-2-Hydroxy-3-phenoxy-propoxy]-phenyl]-3-(naphthalin-2-ylmethoxy)-piperidin-1-carbonsäure tert-butylesters als weißen Festkörper; MS : 584 $(M+H)^+$.

(e) Die Epoxidöffnung des Gemisches des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters [Beispiel 86 (d)] mit Natriummethylat in N,N-Dimethylformamid lieferte das Gemisch des (3RS,4RS)- und (3SR,4SR)-4-[4-[(RS)-2-Hydroxy-3-methoxy-propoxy]-phenyl]-3-(naphthalin-2-ylmethoxy)-piperidin-1-carbonsäure tert-butylesters, dessen Alkylierung mit Benzylbromid, analog zu Beispiel 1 (g), das Gemisch des (3RS,4RS)- und (3SR,4SR)-4-[4-[(RS)-2-Benzyloxy-3-methoxy-propoxy]-phenyl]-3-(naphthalin-2-ylmethoxy)-piperidin-1-carbonsäure tert-butylesters als farbloses Harz ergab; MS : 612 $(M+H)^+$.

(f) Die Epoxidöffnung des Gemisches des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters [Beispiel 86 (d)] mit Natriumthiophenolat, in Analogie zu Beispiel 71 (a), ergab das Gemisch des (3RS,4RS)- und (3SR,4SR)-4-[4-[(RS)-2-Hydroxy-3-phenylsulfanylpropoxy]-phenyl]-3-(naphthalin-2-ylmethoxy)-piperidin-1-carbonsäure tert-butylesters als weißen Festkörper; MS : 600 $(M+H)^+$.

(g) Die Alkylierung des Gemisches des (3RS,4RS)- und (3SR,4SR)-4-[4-[(RS)-2-Hydroxy-3-phenoxy-propoxy]-phenyl]-3-(naphthalin-2-ylmethoxy)-piperidin-1-carbonsäure tert-butylesters [Beispiel 86 (d)] mit Methyliodid, in Analogie zum Beispiel 1 (g), lieferte das Gemisch des (3RS,4RS)- und (3SR,4SR)-4-[4-[(RS)-2-Methoxy-3-phenoxy-propoxy)-phenyl]-3-(naphthalin-2-ylmethoxy)-piperidin- 1-carbonsäure tert-butylesters als farbloses Harz; MS : 598 $(M+H)^+$.

(h) In Analogie zu Beispiel 22 (k) wurde durch Benzoylierung des Gemisches des (3RS,4RS)- und (3SR,4SR)-4-[4-[(RS)-2-Hydroxy-3-methoxy-propoxy]-phenyl]-3-(naphthalin-2-ylmethoxy)-piperidin-1-carbonsäure tert-butylesters [Beispiel 86 (e)] das Gemisch des (3RS,4RS)- und (3SR,4SR)-4-[4-[(RS)-2-Benzoyloxy-3-methoxy-propoxy]-phenyl]-3-(naphthalin-2-ylmethoxy)-piperidin-1-carbonsäure tert-butylesters als farbloses Harz erhalten; MS : 626 $(M+H)^+$.

(i) Die Epoxidöffnung des Gemisches des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters [Beispiel 86 (d)] mit Natriumbenzylat in N,N-Dimethylformamid ergab das Gemisch des (3RS,4RS)- und (3SR,4SR)-4-[4-[(RS)-3-Benzyloxy-2-hydroxy-propoxy]-phenyl]-3-(naphthalin-2-ylmethoxy)-piperidin-1-carbonsäure tert-butylesters, dessen Alkylierung mit Methyliodid, analog zu Beispiel 1 (g), das Gemisch des (3RS,4RS)-und (3SR,4SR)-4-[4-[(RS)-3-Benzyloxy-2-methoxypropoxy]-phenyl]-3-(naphthalin-2-ylmethoxy)-piperidin-1-carbonsäure tert-butylesters als farbloses Harz lieferte; MS : 612 $(M+H)^+$.

(j) In analoger Weise wie in Beispiel 1 (g) beschrieben, wurde durch Alkylierung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit 3-Chlormethyl-pyridin der (3RS,4RS)-3-(Naphthalin-2-ylmethoxy)-4-{4-[2-(pyridin-3-ylmethoxy)-äthoxy]-phenyl}-piperidin-1-carbonsäure tert-butylester erhalten; MS : 569 $(M+H)^+$.

(k) In analoger Weise wie in Beispiel 1 (g) beschrieben, wurde durch Alkylierung des (3RS,4RS)-4-[4-(2-Hydroxy-äthoxy)-phenyl]-3-[4-(2-trimethylsilanyl-äthoxymethoxy)-naphthalin-2-ylmethoxy]-piperidin-1-carbonsäure tert-butylesters [Beispiel 55 (b)] mit 3-Chlormethyl-pyridin der (3RS,4RS)-4-{4-[2-(Pyridin-3-ylmethoxy)-äthoxy]-phenyl}-3-(4-(2-trimethylsilanyl-äthoxymethoxy)-naphthalin-2-ylmethoxy]-piperidin-1-carbonsäure tert-butylester erhalten.

(1) In analoger Weise wie in Beispiel 1 (g) beschrieben, wurde durch Alkylierung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit 4-Chlormethyl-pyridin der (3RS,4RS)-3-(Naphthalin-2-ylmethoxy)-4-{4-[2-(pyridin-4-ylmethoxy)-äthoxy]-phenyl}-piperidin-1-carbonsäure tert-butylester als farbloses Öl erhalten.

(m) In analoger Weise wie in Beispiel 1 (g) beschrieben, wurde durch Alkylierung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit 2-Chlormethyl-pyridin der (3RS,4RS)-3-(Naphthalin-2-ylmethoxy)-4-{4-[2-(pyridin-2-ylmethoxy)-äthoxy]-phenyl}-piperidin-1-carbonsäure tert-butylester als farbloses Öl erhalten.

(n) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Benzyl 3-brompropyläther in Gegenwart von Kaliumcarbonat in Butan-2-on der (3RS,4RS)-4-[4-(3-Benzyloxy-propoxy)-phenyl]-3-hydroxy-piperidin-1-carbonsäure tert-butylester erhalten, dessen Alkylierung mit 2-Brommethyl-naphthalin, analog Beispiel 1 (g), den (3RS,4RS)-4-[4-(3-Benzyloxypropoxy)-phenyl]-3-(naphthalin-2-ylmethoxy)-piperidin-1-carbonsäure tert-butylester als farbloses Öl ergab; MS : 582 $(M+H)^+$.

o) In analoger Weise wie in Beispiel 1 (g) beschrieben, wurde durch Alkylierung des (3RS,4RS)-4-[4-(3-Hydroxy-propoxy)-phenyl]-3-(naphthalin-2-ylmethoxy)-piperidin-1-carbonsäure tert-butylesters [Beispiel 57 (c)] mit 2-Chlormethyl-pyridin der (3RS,4RS)-3-(Naphthalin-2-ylmethoxy)-4-[4-[3-(pyridin-2-ylmethoxy)-propoxy]-phenyl]-piperidin-1-carbonsäure tert-butylester als farbloses Harz erhalten; MS : 583 $(M+H)^+$.

(p) In analoger Weise wie in Beispiel 1 (g) beschrieben, wurde durch Alkylierung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit 2-Chlormethyl-pyridin der (3RS,4RS)-3-(Naphthalin-2-ylmethoxy)-4-[4-[3-(pyridin-2-ylmethoxy)-propyl]-phenyl]-piperidin-1-carbonsäure tert-butylester als farbloser, amorpher Festkörper erhalten, der ohne weitere Reinigung und Charakterisierung in der folgenden Stufe eingesetzt wurde.

(q) In analoger Weise wie in Beispiel 44 (e) beschrieben, wurde durch Alkylierung des (3RS,4RS)-4-[4-(3-Methylamino-propoxy)-phenyl]-3-(naphthalin-2-ylmethoxy)-piperidin-1-carbonsäure tert-butylesters [Beispiel 57 (e)] mit Benzylbromid der (3RS,4RS)-4-[4-[3-(Benzyl-methylamino)-propoxy]-phenyl]-3-(naphthalin-2-ylmethoxy)-piperidin-1-carbonsäure tert-butylester als farbloser Festkörper erhalten; MS : 595 $(M+H)^+$.

(r) In analoger Weise wie in Beispiel 1 (g) beschrieben, wurde durch Alkylierung des Gemisches des (3RS,4RS)- und (3SR,4SR)-3-Hydroxy-4-[4-[3-[(RS)-tetrahydro-pyran-2-yloxy]-propoxy]-phenyl]-piperidin-1-carbonsäure tert-butylesters [Beispiel 57 (a)] mit 1-Methoxy-2-brommethyl-naphthalin [Beispiel 7 (f)] das Gemisch des (3RS,4RS)- und (3SR,4SR)-3-(1-Methoxy-naphthalin-2-ylmethoxy)-4-[4-[3-[(RS)-tetrahydro-pyran-2-yloxy]-propoxy]-phenyl]-piperidin-1-carbonsäure tert-butylesters erhalten. Die Abspaltung der THP-Gruppe mittels Pyridinium-(toluol-4-sulfonat) in Äthanol, analog Beispiel 53 (c) ergab den (3RS,4RS)-4-(4-(3-Hydroxy-propoxy)-phenyl]-3-(1-methoxynaphthalin-2-ylmethoxy)-piperidin-1-carbonsäure tert-butylester, dessen Umsetzung mit Bis-(benzothiazol-2-yl)-disulfid, analog Beispiel 33 (a), den (3RS,4RS)-4-[4-[3-(Benzothiazol-2-ylsulfonyl)-propoxy]-phenyl]-3-(1-methoxy-naphthalin-2-ylmethoxy)-piperidin-1-carbonsäure tert-butylester als farblose Flüssigkeit ergab; MS : 671 $(M+H)^+$.

(s) In analoger Weise wie in Beispiel 33 (a) beschrieben, wurde durch Umsetzung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit Diphenyldisulfid der (3RS,4RS)-3-(Naphthalin-2-ylmethoxy)-4-[4-(3-phenylsulfanyl-propyl)-phenyl]-piperidin-1-carbonsäure tert-butylester als farbloser, amorpher Festkörper erhalten; MS : 568 $(M+H)^+$.

(t) In analoger Weise wie in Beispiel 33 (a) beschrieben, wurde durch Umsetzung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit Bis-(benzothiazol-2-yl)-disulfid der (3RS,4RS)-4-{4-[3-(Benzothiazol-2-ylsulfanyl)-propyl]-phenyl)-3-(naphthalin-2-ylmethoxy)-piperidin-1-carbonsäure tert-butylester erhalten, der ohne weitere Reinigung und Charakterisierung in der folgenden Stufe eingesetzt wurde.

(u) (α) In analoger Weise wie in Beispiel 1 (g) beschrieben, wurde durch Alkylierung des (3RS,4RS)-3-Hydroxy-4-[4-(2-trityloxy-äthyl)-phenyl]-piperidin-1-carbonsäure tert-butylesters [Beispiel 29 (t)] mit 3-Chlormethyl-1-(2-trimethylsilanyl-äthoxymethoxy)-naphthalin [Beispiel 5 (c)] der (3RS,4RS)-3-[4-(2-Trimethylsilanyl-äthoxymethoxy)-naphthalin-2-ylmethoxy]-4-[4-(2-trityloxy-äthyl)-phenyl]-piperidin-1-carbonsäure tert-butylester erhalten.

(β) Die selektive Abspaltung der Trityl-Gruppe erfolgte analog zu dem von E.Krainer et al. in Tetrahedron Letters 1993, 1713-1716 publizierten Verfahren, indem eine Lösung von 780 mg (0.92 mM) (3RS,4RS)-3-[4-(2-Trimethylsilanyl-äthoxymethoxy)-naphthalin-2-ylmethoxy]-4-[4-(2-trityloxy-äthyl)-phenyl]-piperidin-1-carbonsäure tert-butylester in 15 ml Methylenchlorid mit einer Lösung von 436 mg (3.68 mMol) Trifluoressigsäure und 803 mg (3.68 mMol) Trifluoressigsäureanhydrid in 2 ml Methylenchlorid versetzt wurde. Nach 30 Sekunden wurde das Reaktionsgemisch auf 0 °C abgekühlt und mit 4 ml Triäthylamin versetzt. Nach 5 Minuten wurden 10 ml Methanol zugegeben und das Gemisch 10 Minuten lang gerührt. Anschließend wurde mit gesättigter Natriumhydrogencarbonat-Lösung gewaschen und die wäßrige Phase danach mit 10 ml Methylenchlorid nachextrahiert. Die vereinigten organischen Phasen w urden über Natriumsulfat getrocknet und dann unter vermindertem Druck eingedampft. Das Rohprodukt wurde an Kieselgel mittels eines 4:1-Gemisches von Hexan und Essigester als Eluierungsmittel chromatographiert. Es wurden 553 mg (3RS,4RS)-4-[4-(2-Hydroxy-äthyl)-phenyl]-3-[4-(2-trimethylsilanyl-äthoxymethoxy)-naphthalin-2-ylmethoxy]-piperidin-1-carbonsäure tert-butylester als farbloses Öl erhalten.

(γ) In analoger Weise wie in Beispiel 34 beschrieben, wurde aus dem (3RS,4RS)-4-[4-(2-Hydroxy-äthyl)-phenyl]-3-[4-(2-trimethylsilanyl-äthoxymethoxy)-naphthalin-2-ylmethoxy]-piperidin-1-carbonsäure tert-butylester über das

entsprechende Mesylat durch Umsetzung mit 2-Mercaptopyrimidin der (3RS,4RS)-4-{4-[2-(Pyrimidin-2-ylsulfanyl)-äthyl]-phenyl}-3-[4-(2-trimethylsilanyl-äthoxymethoxy)-naphthalin-2-ylmethoxy]-piperidin-1-carbonsäure tert-butylester als farbloses Öl erhalten, das ohne weitere Reinigung und Charakterisierung in der folgenden Stufe eingesetzt wurde.

(v) In analoger Weise wie in Beispiel 33 (a) beschrieben, wurde aus dem (3RS,4RS)-4-[4-(2-Hydroxy-äthyl)-phenyl]-3-[4-(2-trimethylsilanyl-äthoxymethoxy)-naphthalin-2-ylmethoxy]-piperidin-1-carbonsäure tert-butylester durch Umsetzung mit 2,2'-Dithiopyridin der (3RS,4RS)-4-{4-[2-(Pyridin-2-ylsulfanyl)-äthyl]-phenyl}-3-[4-(2-trimethylsilanyl-äthoxymethoxy)-naphthalin-2-ylmethoxy]-piperidin-1-carbonsäure tert-butylester als farbloses Öl erhalten, das ohne weitere Reinigung und Charakterisierung in der folgenden Stufe eingesetzt wurde.

(w) In analoger Weise wie in Beispiel 33 (a) beschrieben, wurde aus dem (3RS,4RS)-4-[4-(2-Hydroxy-äthyl)-phenyl]-3-[4-(2-trimethylsilanyl-äthoxymethoxy)-naphthalin-2-ylmethoxy]-piperidin-1-carbonsäure tert-butylester durch Umsetzung mit Bis-(benzothiazol-2-yl)-disulfid der (3RS,4RS)-4-[4-[2-(Benzothiazol-2-ylsulfanyl)-äthyl]-phenyl]-3-[4-(2-trimethylsilanyl-äthoxymethoxy)-naphthalin-2-ylmethoxy]-piperidin-1-carbonsäure tert-butylester als farbloser Schaum erhalten; MS : 757 $(M+H)^+$.

(x) In analoger Weise wie in Beispiel 1 (g) beschrieben, wurde durch Alkylierung des (3RS,4RS)-4-(4-Hydroxymethyl-phenyl)-3-(naphthalin-2-ylmethoxy)-piperidin-1-carbonsäure tert-butylesters [Beispiel 22 (j)] mit 3-Chlormethyl-pyridin der (3RS,4RS)-3-(Naphthalin-2-ylmethoxy)-4-[4-(pyridin-3-ylmethoxymethyl)-phenyl]-piperidin-1-carbonsäure tert-butylester als farbloses Öl erhalten; MS : 539 $(M+H)^+$.

(y) In analoger Weise wie in Beispiel 9 (c) beschrieben, wurde durch Veresterung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit (RS)-2-(4-Fluor-phenyl)-3-methyl-buttersäure (DE 2365555) in Gegenwart von EDC das Gemisch des (3RS,4RS)- und (3SR,4SR)-4-(4-2-[(RS)-2-(4-Fluor-phenyl)-3-methyl-butyryloxy]-äthoxy]-phenyl)-3-(naphthalin-2-ylmethoxy)-piperidin-1-carbonsäure tert-butylester als farbloser Festkörper erhalten; MS : 656 $(M+H)^+$.

(z) In analoger Weise wie in Beispiel 9 (c) beschrieben, wurde durch Veresterung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit 1-Methyl-pyrrol-2-carbonsäure in Gegenwart von EDC der (3RS,4RS)-4-[4-[2-(1-Methyl-1H-pyrrol-2-carbonyloxy)-äthoxy]-phenyl]-3-(naphthalin-2-ylmethoxy)-piperidin-1-carbonsäure tert-butylester als farbloses Öl erhalten; MS : 585 $(M+H)^+$.

(aa) In analoger Weise wie in Beispiel 22 (k) beschrieben, wurde durch Acylierung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit Benzoylchlorid der (3RS,4RS)-4-[4-(3-Benzoyloxypropyl)-phenyl]-3-(naphthalin-2-ylmethoxy)-piperidin-1-carbonsäure tert-butylester als fast farbloser Festkörper erhalten; MS : 580 $(M+H)^+$.

(bb) In analoger Weise wie in Beispiel 22 (k) beschrieben, wurde durch Acylierung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit 3-Methoxy-benzoylchlorid der (3RS,4RS)-4-{4-[3-(3-Methoxy-benzoyloxy)-propyl]-phenyl}-3-(naphthalin-2-ylmethoxy)-piperidin-1-carbonsäure tert-butylester als farbloser, amorpher Festkörper erhalten; MS : 610 $(M+H)^+$.

(cc) In analoger Weise wie in Beispiel 1 (g) beschrieben, wurde durch Alkylierung des (3RS,4RS)-3-Hydroxy-4-(4-trityloxymethyl-phenyl)-piperidin-1-carbonsäure tert-butylesters [Beispiel 22 (h)] mit 2-Chlormethyl-1-(2-trimethylsilanyl-äthoxymethoxy)-naphthalin [Beispiel 6 (c)] der (3RS,4RS)-3-[1-(2-Trimethylsilanyl-äthoxymethoxy)-naphthalin-2-ylmethoxy]4-(4-trityloxymethyl-phenyl)-piperidin-1-carbonsäure tert-butylester erhalten. Die selektive Abspaltung der Trityl-Gruppe, analog Beispiel 86 (u) (β) ergab den (3RS,4RS)-4-(4-Hydroxymethyl-phenyl)-3-[1-(2-trimethylsilanyl-äthoxymethoxy)-naphthalin-2-ylmethoxy]-piperidin-1-carbonsäure tert-butylester, dessen Acylierung mit 3-Methoxy-benzoylchlorid, analog Beispiel 22 (k) den (3RS,4RS)-4-[4-(3-Methoxy-benzoyloxymethyl)-phenyl]-3-[1-(2-trimethylsilanyl-äthoxymethoxy)-naphthalin-2-ylmethoxy]-piperidin-1-carbonsäure tert-butylester als farbloses Öl ergab, MS : 745 $(M+NH_4)^+$.

(dd) In analoger Weise wie in Beispiel 44 (e) beschrieben, wurde durch Alkylierung des (3RS,4RS)-4-(4-Hydroxy-phenyl)-3-(naphthalin-2-ylmethoxy)-piperidin-1-carbonsäure tert-butylester [Beispiel 61 (c)] mit Bromessigsäureäthylester der (3RS,4RS)-4-(4-Äthoxycarbonylmethoxyphenyl)-3-(naphthalin-2-ylmethoxy)-piperidin-1-carbonsäure tert-butylester als farbloser Festkörper erhalten; MS : 520 $(M+H)^+$.

(ee) Die Verseifung des (3RS,4RS)-4-(4-Äthoxycarbonylmethoxy-phenyl)-3-(naphthalin-2-ylmethoxy)-piperidin-1-carbonsäure tert-butylesters [Beispiel 86 (dd)] mittels 1 N Natronlauge in Methanol lieferte den (3RS,4RS)-4-(4-Carboxymethoxy-phenyl)-3-(naphthalin-2-ylmethoxy)-piperidin-1-carbonsäure tert-butylester, dessen Kondensation mit Benzylamin in Gegenwart von HBTU, analog Beispiel 36 (b), den (3RS,4RS)-4-[4-(Benzylcarbamoylmethoxy)-phenyl]-3-(naphthalin-2-ylmethoxy)-piperidin-1-carbonsäure tert-butylester als gelblichen Festkörper ergab; MS : 598 $(M+NH_4)^+$.

(ff) In analoger Weise wie in Beispiel 24 (m) beschrieben, wurde durch Umsetzung des (3RS,4RS)-4-(4-Hydroxy-phenyl)-3-(naphthalin-2-ylmethoxy)-piperidin-1-carbonsäure tert-butylesters (3RS,4RS)-4-(4-Hydroxy-phenyl)-3-(naphthalin-2-ylmethoxy)-piperidin-1-carbonsäure tert-butylester mit 2-Pyridylisocyanat der (3RS,4RS)-3-(Naphthalin-2-ylmethoxy)-4-[4-(pyridin-2-ylcarbamoyloxy)-phenyl]-piperidin-1-carbonsäure tert-butylester als weißer Festkörper erhalten; MS : 554 $(M+H)^+$.

(gg) In analoger Weise wie in Beispiel 68 (b) beschrieben, wurde durch Swern-Oxidation des Gemisches des (3RS, 4RS)- und (3SR,4SR)-4-[4-[(RS)-2-Hydroxy-3-phenoxy-propoxy]-phenyl]-3-(naphthalin-2-ylmethoxy)-piperidin-1-carbonsäure tert-butylesters der (3RS,4RS)-3-(Naphthalin-2-ylmethoxy)-4-[4-(2-oxo-3-phenoxypropoxy)-phenyl]-piperidin-1-carbonsäure tert-butylester als weißer Festkörper erhalten; MS : 582 $(M+H)^+$.

(hh) (α) Zu einer Lösung von 0.270 g (0.58 mMol) (3RS,4RS)-4-[4-(2-Hydroxy-äthyl)-phenyl]-3-naphthalin-2-ylmethoxy-piperidin-1-carbonsäure tert-butylester [Beispiel 29 (h)] in 10 ml Methylenchlorid wurde unter Argon und unter Rühren bei Raumtemperatur eine Lösung von 5mg (0.04 mMol) Kaliumbromid und 20 mg (0.24mMol) Natriumhydrogencarbonat in 10 ml Wasser gegeben. Das Reaktionsgemisch wurde auf 0 °C abgekühlt und mit 2 mg (0.01mMol) 2,2,6,6-Tetramethyl-1-piperidinyloxyl (TEMPO) versetzt. Dann wurde unter kontinuierlichem Rühren 1 ml (0.658mMol) Javellauge in das Reaktiongemisch eingespritzt. Nach der Zugabe wird das Reaktionsgemisch bei 0 °C ca. 30 Minuten lang nachgerührt . Zur Aufarbeitung wurden 20 ml eines 1:1- Gemisches von Methylenchlorid und Wasser dazugegeben und das Reaktionsgemisch mit 10 ml gesättigter Natriumchlorid-Lösung gewaschen und die wäßrige Phase wurde mit 10 ml Methylenchlorid nachextrahiert. Die vereinigten organischen Phasen wurden über Natriumsulfat getrocknet und unter vermindertem Druck eingedampft. Das Rohprodukt wurde Flash-Chromatographie an Kieselgel mit einem 2:1-Gemisch von Hexan und Essigester als Eluierungsmittel gereinigt. Es wurden 200 mg (75 % d.Th.) (3RS,4RS)-3-(Naphthalin-2-ylmethoxy)-4-[4-(2-oxo-äthyl)-phenyl]-piperidin-1-carbonsäure tert-butylester als Schaum erhalten erhalten; MS : 460 $(M+H)^+$.

(β) In analoger Weise wie in Beispiel 40 (a) beschrieben, wurde durch eine Grignard-Reaktion des (3RS,4RS)-3-(Naphthalin-2-ylmethoxy)-4-[4-(2-oxo-äthyl)-phenyl]-piperidin-1-carbonsäure tert-butylesters mit Phenylmagnesiumchlorid das Gemisch des (3RS,4RS)-4-[4-[(RS)-2- und [(SR)-2-Hydroxy-2-phenyl-äthyl]-phenyl]-3-(naphthalin-2-ylmethoxy)-piperidin-1-carbonsäure tert-butylesters als farbloses Öl erhalten.

(ii) In analoger Weise wie in Beispiel 9 (a) - (c) beschrieben, wurde durch Alkylierung des (3RS,4RS)-4-(4-Fluorphenyl)-3-hydroxypiperidin-1-carbonsäure tert-butylesters [Beispiel 3 (b)] mit 2-Brommethylbenzonitril, der (3RS, 4RS)-3-(2-Cyano-benzyloxy)-4-(4-fluorphenyl)-piperidin-1-carbonsäure tert-butylester erhalten, dessen Reduktion mittels Boran-Dimethylsulfid-Komplex den (3RS,4RS)-3-(2-Aminomethyl-benzyloxy)-4-(4-fluor-phenyl)-piperidin-1-carbonsäure tert-butylester ergab. Die darauffolgende Acylierung mit Pyridin-2-carbonsäure in Gegenwart von EDC lieferte den (3RS,4RS)-4-(4-Fluorphenyl)-3-(2-{[(pyridin-2-carbonyl)-amino]-methyl}-benzyloxy)-piperidin-1-carbonsäure tert-butylester als weißen Festkörper.

(jj) In analoger Weise wie in Beispiel 1 (g) beschrieben, wurde durch Alkylierung des (3RS,4RS)-4-(4-Fluor-phenyl)-3-hydroxy-piperidin-1-carbonsäure tert-butylester [Beispiel 3 (b)] mit (3-Brommethyl-phenyl)-phenyl-methanon [J. Med.Chem. 1984, 27 (12), 1682-1690] der (3RS,4RS)-3-(3-Benzoyl-benzyloxy)-4-(4-fluor-phenyl)-piperidin-1-carbonsäure tert-butylester als gelbliche Flüssigkeit erhalten; MS : 490 $(M+H)^+$.

(kk) (α) Eine Lösung von 470 mg (1.0 mMol) (3RS,4RS)-4-[4-(2-Cyano-äthyl)-phenyl]-3-naphthalin-2-ylmethoxy-piperidin-1-carbonsäure tert-butylester [Beispiel 35 (b)] und 348 mg (5.0 mMol) Hydroxylaminhydrochlorid in 6 ml einer IM Natriummethylatlösung in Methanol wurde 5 Stunden lang bei 65 °C gerührt. Das Gemisch wurde zur Aufarbeitung zwischen 40 ml Essigester und 40 ml Wasser verteilt, danach die organische Phase abgetrennt. Die wäßrige Phase wurde zweimal mit je 40 ml Essigester extrahiert. Die vereinigten organischen Phasen wurden über Magnesiumsulfat getrocknet und schließlich das Lösungsmittel unter verminderten Druck abdestilliert. Das Rohprodukt (550 mg) wurde durch Chromatographie an Kieselgel mit einem 20:1:0.1-Gemisch von Methylenchlorid, Methanol und 28%iger Ammoniaklösung gereinigt. Es wurden 501 mg (99% d.Th.) (3RS,4RS)-4-{4-[2-(N-Hydroxycarbamimidoyl)-äthyl]-phenyl}-3-(naphthalin-2-ylmethoxy)-piperidin-1-carbonsäure tert-butylester als gelbes Öl erhalten, der direkt in die nächste Stufe eingesetzt wurde.

(β) In analoger Weise wie in Beispiel 38 beschrieben, wurde durch Kondensation des (3RS,4RS)-4-{4-[2-(N-Hydroxycarbamimidoyl)-äthyl]-phenyl}-3-(naphthalin-2-ylmethoxy)-piperidin-1-carbonsäure tert-butylesters mit Benzoesäure in Gegenwart von EDC und anschließende Cyclisierung der (3RS,4RS)-3-(Naphthalin-2-ylmethoxy)-4-[4-[2-(5-phenyl-[1,2,4]oxadiazol-3-yl)-äthyl]-phenyl]-piperidin-1-carbonsäure tert-butylester erhalten; MS : 590 $(M+H)^+$.

(ll) Durch alkalische Verseifung des (3RS,4RS)-4-[4-(2-Äthoxycarbonyl-äthyl)-phenyl]-3-(naphthalin-2-ylmethoxy)-piperidin-1-carbonsäure tert-butylesters [Beispiel 24 (s)] mittels wäßriger Natronlauge in Tetrahydrofuran wurde der (3RS,4RS)-4-[4-(2-Carboxy-äthyl)-phenyl]-3-(naphthalin-2-ylmethoxy)piperidin-1-carbonsäure tert-butylester erhalten, dessen Kondensation mit N-Hydroxy-benzamidin in Gegenwart von EDC, in analoger Weise wie in Beispiel 38 beschrieben, den entsprechenden N-Hydroxy-benzamidinester ergab und dessen Cyclisierung den (3RS,4RS)-3-(Naphthalin-2-ylmethoxy)-4-[4-[2-(3-phenyl-[1,2,4]oxadiazol-5-yl)-äthyl]-phenyl]-piperidin-1-carbonsäure tert-butylester als gelblichen, amorphen Festkörper lieferte; MS : 590 $(M+H)^+$.

(mm) Die Verseifung des (3RS,4RS)-4-(4-Äthoxycarbonylmethoxyphenyl)-3-(naphthalin-2-ylmethoxy)-piperidin-1-carbonsäure tert-butylesters [Beispiel 86 (dd)] mittels 1 N Natronlauge in Methanol lieferte den (3RS,4RS)-4-(4-Carboxymethoxy-phenyl)-3-(naphthalin-2-ylmethoxy)-piperidin-1-carbonsäure tert-butylester, dessen Kondensation mit N-Hydroxy-benzamidin in Gegenwart von HBTU, in analoger Weise wie in Beispiel 38 beschrieben, den entsprechenden N-Hydroxy-benzamidinester ergab und dessen Cyclisierung den (3RS,4RS)-3-(Naphthalin-2-ylmethoxy)-4-[4-(3-phenyl-[1,2,4]oxadiazol-5-ylmethoxy)-phenyl]-piperidin-1-carbonsäure tert-butylester als farbloses Öl ergab; MS : 592 $(M+H)^+$.

(nn) In analoger Weise wie in Beispiel 38 beschrieben, wurde durch Kondensation des (3RS,4RS)-4-(4-Carboxymethoxy-phenyl)-3-(naphthalin-2-ylmethoxy)-piperidin-1-carbonsäure tert-butylester [Beispiel 86 (mm)] mit 3-Pyridinamidoxim in Gegenwart von HBTU und anschließende Cyclisierung der (3RS,4RS)-3-(Naphthalin-2-ylmethoxy)-4-[4-(3-pyridin-3-yl-[1,2,4]oxadiazol-5-ylmethoxy)-phenyl]-piperidin-1-carbonsäure tert-butylester als gelblicher Festkörper erhalten; MS : 593 $(M+H)^+$.

(oo) In analoger Weise wie in Beispiel 44 (e) beschrieben, wurde durch Alkylierung des (3RS,4RS)-4-(4-Hydroxyphenyl)-3-(naphthalin-2-ylmethoxy)-piperidin-1-carbonsäure tert-butylester [Beispiel 61 (c)] mit dem Mesylat des 5-Hydroxymethyl-3-phenyl-4,5-dihydro-isoxazols, das nach allgemein bekanntem Verfahren hergestellt worden war, der (3RS,4RS)-3-(Naphthalin-2-ylmethoxy)-4-[4-(3-phenyl-isoxazol-5-ylmethoxy)-phenyl)-piperidin-1-carbonsäure tert-butylester als farbloser Festkörper erhalten; MS : 591 $(M+H)^+$.

Die folgenden BOC - Derivate wurden erhalten, indem, in analoger Weise wie in Beispiel 1 (g) beschrieben, der (3RS,4RS)-4-[4-(3-Benzyloxypropoxy)-phenyl]-3-hydroxy-piperidin-1-carbonsäure tert-butylester [Beispiel 86 (n)] wie folgt alkyliert wurde:

(pp) mit 3-Methoxybenzylchlorid zum (3RS,4RS)-4-[4-(3-Benzyloxypropoxy)-phenyl]-3-(3-methoxy-benzyloxy)-piperidin-1-carbonsäure tert-butylester, der als farbloses Öl erhalten wurde; MS : 579 $(M+NH_4)^+$.

(qq) mit 1-Chlormethyl-3-(2-trimethylsilanyl-äthoxymethoxy)-benzol zum (3RS,4RS)-4-[4-(3-Benzyloxy-propoxy)-phenyl]-3-[3-(2-trimethylsilanyl-äthoxymethoxy)-benzyloxy]-piperidin-1-carbonsäure tert-butylester, der als farbloses Öl erhalten wurde; MS : 695 $(M+NH_4)^+$.

Das als Alkylierungsreagenz verwendete 1-Chlormethyl-3-(2-trimethylsilanyl-äthoxymethoxy)-benzol wurde hergestellt, indem, in Analogie zu Beispiel 5 (a) - (d), 3-Hydroxybenzoesäuremethylester durch Einführung der SEM-Gruppe zu 3-(2-Trimethylsilanyl-äthoxymethoxy)-benzoesäuremethylester umgesetzt wurde. Die anschließende Reduktion mit Lithiumaluminiumhydrid ergab das [3-(2-Trimethylsilanyläthoxy-methoxy)-phenyl]-methanol und dessen Chlorierung 1-Chlormethyl-3-(2-trimethylsilanyl-äthoxymethoxy)-benzol als farbloses Öl; MS : 272 $(M)^+$.

(rr) mit 3-Chlor-4-methoxy-benzylchlorid zum (3RS,4RS)-4-[4-(3-Benzyloxy-propoxy)-phenyl]-3-(3-chlor-4-methoxy-benzyloxy)-piperidin-1-carbonsäure tert-butylester, der ohne weitere Reinigung und Charakterisierung in der folgenden Stufe eingesetzt wurde;

(ss) mit 3,4-Dichlor-benzylchlorid zum (3RS,4RS)-4-[4-(3-Benzyloxypropoxy)-phenyl]-3-(3,4-dichlor-benzyloxy)-piperidin-1-carbonsäure tert-butylester, der ohne weitere Reinigung und Charakterisierung in der folgenden Stufe eingesetzt wurde;

(tt) mit 2,5-Dichlor-benzylchlorid zum (3RS,4RS)-4-[4-(3-Benzyloxypropoxy)-phenyl]-3-(2,5-dichlor-benzyloxy)-piperidin-1-carbonsäure tert-butylester, der ohne weitere Reinigung und Charakterisierung in der folgenden Stufe eingesetzt wurde;

(uu) mit Benzylchlorid zum (3RS,4RS)-3-Benzyloxy-4-[4-(3-benzyloxypropoxy)-phenyl]-piperidin-1-carbonsäure tert-butylester, der ohne weitere Reinigung und Charakterisierung in der folgenden Stufe eingesetzt wurde;

(vv) mit 2,5-Dimethyl-benzylchlorid zum (3RS,4RS)-4-[4-(3-Benzyloxypropoxy)-phenyl]-3-(2,5-dimethyl-benzyloxy)-piperidin-1-carbonsäure tert-butylester, der ohne weitere Reinigung und Charakterisierung in der folgenden Stufe eingesetzt wurde;

(ww) mit 4-Äthyl-benzylchlorid zum (3RS,4RS)-4-[4-(3-Benzyloxypropoxy)-phenyl]-3-(4-äthyl-benzyloxy)-piperidin-1-carbonsäure tert-butylester, der ohne weitere Reinigung und Charakterisierung in der folgenden Stufe eingesetzt wurde;

(xx) mit dem Gemisch des 3- und 4-Vinyl-benzylchlorids zum Gemisch des (3RS,4RS)-4-[4-(3-Benzyloxy-propoxy)-phenyl]-3-(3 und 4-vinylbenzyloxy)-piperidin-1-carbonsäure tert-butylesters, der ohne weitere Reinigung und Charakterisierung in der folgenden Stufe eingesetzt wurde;

(yy) mit 6-Chlormethyl-2,3-dihydro-benzo[1,4]dioxin zum (3RS,4RS)-4-[4-(3-Benzyloxy-propoxy)-phenyl]-3-(2,3-dihydro-benzo[1,4] dioxin-6-ylmethoxy)-piperidin-1-carbonsäure tert-butylester, der als farbloses Harz erhalten wurde; MS : 607 $(M+NH_4)^+$;

(zz) mit 6-Chlormethyl-1,2,3,4-tetrahydro-naphthalin zum (3RS,4RS)-4-[4-(3-Benzyloxy-propoxy)-phenyl]-3-(5,6,7,8-tetrahydro-naphthalin-2-ylmethoxy)-piperidin-1-carbonsäure tert-butylester, der als farbloses Harz erhalten wurde; MS : 603 $(M+NH_4)^+$;

(aaa) mit 3-Chlormethyl-1-(2-trimethylsilanyl-äthoxymethoxy)-naphthalin [Beispiel 5 (c)] zum (3RS,4RS)-4-[4-(3-Benzyloxy-propoxy)-phenyl]-3-[4-(2-trimethylsilanyl-äthoxymethoxy)-naphthalin-2-ylmethoxy]-piperidin-1-carbonsäure tert-butylester, der als farbloses Harz erhalten wurde; MS : 745 $(M+NH_4)^+$.

(bbb) In analoger Weise wie im Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit 2-(4-Chlor-butoxy)-tetrahydro-2H-pyran das Gemisch des (3RS,4RS)- und (3SR,4SR)-3-Hydroxy-4-[4-[4-[(RS)-tetrahydropyran-2-yloxy]-butoxy]-phenyl]-piperidin-1-carbonsäure tert-butylesters erhalten, dessen Alkylierung mit 2-Brommethyl-naphthalin, analog Beispiel 1 (g), das Gemisch des (3RS,4RS)- und (3SR,4SR)-3-(Naphthalin-2-ylmethoxy)-4-[4-[4-[(RS)-tetrahydro-pyran-2-yloxy]-butoxy]-phenyl]-piperidin-1-carbonsäure tert-butylesters ergab. Die Abspaltung der THP-Gruppe mittels Chlorwasserstoff in Methanol, analog Beispiel 53 (c) lieferte den (3RS,4RS)-4-[4-(4-Hydroxy-butoxy)-phenyl]-3-(naphthalin-2-ylmethoxy)-piperidinl-carbonsäure tert-butylester, dessen Alkylierung mit Benzylbromid, analog zu Beispiel 1 (g), den (3RS,4RS)-4-[4-(4-Benzyloxybutoxy)-phenyl]-3-(naphthalin-2-ylmethoxy)-piperidin-1-carbonsäure tert-butylester als farbloses Öl ergab; MS : 613 $(M+NH_4)^+$.

(ccc) In analoger Weise wie in Beispiel 22 (d) beschrieben, wurde aus dem (3RS,4RS,5SR)-4-(4-Brom-phenyl)-5-methoxymethyl-3-naphthalin-2-yl-methoxy-piperidin-1-carbonsäure tert-butylester [Beispiel 68 (l)] durch eine Palladium-katalysierte Carbonylierung mit Kohlenmonoxid in Methanol der (3SR,4RS,5RS)-4-(4-Methoxycarbonyl-phenyl)-3-methoxymethyl-5-(naphthalin-2-ylmethoxy)-piperidin-1-carbonsäure tert-butylester erhalten, dessen Reduktion mit Lithiumborhydrid, analog zu Beispiel 22 (e) den (3SR,4RS,5RS)-4-(4-Hydroxymethylphenyl)-3-methoxymethyl-5-(naphthalin-2-yl-methoxy)-piperidin-1-carbonsäure tert-butylester lieferte. Die darauffolgende Acylierung mit 2-Chlorbenzoylchlorid, analog Beispiel 22 (k), ergab den (3SR,4RS,5RS)-4-[4-(2-Chlor-benzoyloxymethyl)-phenyl]-3-methoxymethyl-5-(naphthalin-2-ylmethoxy)-piperazin-1-carbonsäure tert-butylester als farblosen Schaum; MS : 630 $(M+H)^+$.

(ddd) In analoger Weise wie in Beispiel 1 (g) beschrieben, wurde durch Alkylierung des (3SR,4RS,5RS)-4-(4-Hydroxymethyl-phenyl)-3-methoxymethyl-5-(naphthalin-2-yl-methoxy)-piperidin-1-carbonsäure tert-butylesters mit

Benzylbromid der (3SR,4RS,5RS)-4-(4-Benzyloxymethyl-phenyl)-3-methoxymethyl-5-(naphthalin-2-ylmethoxy)-piperidin-1-carbonsäure tert-butylester als farbloses Öl erhalten; MS : 582 $(M+H)^+$.

(eee) In analoger Weise wie in Beispiel 12 (c) - (d) beschrieben, erfolgte die Abspaltung der N-Methyl-Gruppe des (1RS,2RS,3RS,5SR)-3-(4-Bromphenyl)-8-methyl-8-aza-bicyclo[3.2.1]octan-2-ols, das analog zum 4-Chlorphenyl-Derivat [J.Org.Chem. 35, 802 (1970)] erhalten worden war, indem zunächst durch Umsetzung mit Chlorameisensäure 2,2,2-trichloräthylester der (1RS,2RS,3RS,5SR)-3-(4-Brom-phenyl)-2-(2,2,2-trichlor-äthoxycarbonyloxy)-8-aza-bicyclo[3.2.1]octan-8-carbonsäure 2,2,2-trichlor-äthylester synthetisiert wurde und anschließend durch Reaktion mit Zink in Eisessig der (1RS,2RS,3RS,5SR)-3-(4-Bromphenyl)-8-aza-bicyclo[3.2.1]octan-2-ol erhalten wurde. Die darauffolgende Einführung der BOC-Gruppe, analog Beispiel 1 (f), ergab den (1RS,2RS,3RS,5SR)-3-(4-Brom-phenyl)-2-hydroxy-8-azabicyclo[3.2.1]octan-8-carbonsäure tert-butylester, dessen Palladium-katalysierte Carbonylierung mittels Kohlenmonoxid in Methanol, analog Beispiel 22 (d), den (1RS,2RS,3RS,5SR)-2-Hydroxy-3-(4-methoxycarbonyl-phenyl)-8-aza-bicyclo[3.2.1]octan-8-carbonsäure tert-butylester lieferte. Die anschließende Reduktion mit Lithiumborhydrid, analog Beipiel 22 (e), führte zu dem (1RS,2RS,3RS,5SR)-2-Hydroxymethyl-phenyl)-8-aza-bicyclo[3.2.1]octan-8-carbonsäure tert-butylester, dessen Umsetzung mit Tritylchlorid, analog Beispiel 22 (h), den (1RS,2RS,3RS,5SR)-2-Hydroxy-3-(4-trityloxymethyl)-phenyl)-8-azabicyclo[3.2.1]octan-8-carbonsäure tert-butylester ergab. Die weitere Reaktion mit 2-Brommethyl-naphthalin, analog Beispiel 1 (g), führte zum (1RS,2RS,3RS,5SR)-2-(Naphthalin-2-ylmethoxy)-3-(4-trityloxymethyl-phenyl)-8-aza-bicyclo[3.2.1]octan-8-carbonsäure tert-butylester. In analoger Weise wie in Beispiel 86 (u) (β) beschrieben, wurde durch Abspaltung der Trityl-Gruppe mittels eines Gemisches von Trifluoressigsäure und Trifluoressigsäureanhydrid der (1RS, 2RS,3RS,5SR)-3-(4-Hydroxymethyl-phenyl)-2-(naphthalin-2-ylmethoxy)-8-aza-bicyclo [3.2.1] octan-8-carbonsäure tert-butylester erhalten, dessen Alkylierung mit Benzyl 3-brompropyläther, analog Beispiel 44 (e), den (1RS, 2RS,3RS,5SR)-3-[4-(2-Benzyloxypropoxymethyl)-phenyl]-2-(naphthalin-2-ylmethoxy)-8-azabicyclo[3.2.1]octan-8-carbonsäure tert-butylester als farbloses Öl ergab.

Beispiel 87

In analoger Weise wie in Beispiel 73 (d) beschrieben, wurden durch Abspaltung der BOC-Gruppe mittels wasserfreiem Zinkbromid die folgenden Verbindungen erhalten:

1) - Aus (3RS,4RS)-3-(Naphthalin-2-carbonyloxy)-4-[4-(2-phenoxy-äthoxy)-phenyl]-piperidin-1-carbonsäure tert-butylester Naphthalin-2-carbonsäure (3RS,4RS)-4-[4-(2-phenoxy-äthoxy)-phenyl]-piperidin-3-ylester als farbloser Festkörper; MS: 468 $(M+H)^+$;

2) - aus dem Gemisch des (3RS,4RS)-4-(4-Fluor-phenyl)-3-[(RS)- und (SR)-2-hydroxy-1-naphthalin-2-yl-äthoxy]-piperidin-1-carbonsäure tert-butylesters ein Gemisch des (RS)- und (SR)-2-[(3RS,4RS)-4-(4-Fluorphenyl)-piperidin-3-yloxy]-2-naphthalin-2-yl-äthanols als farbloser amorpher Festkörper; MS: 366 $(M+H)^+$.

Die als Ausgangssubstanzen eingesetzten BOC-Derivate wurden wie folgt hergestellt:

(a) Zu einer Lösung von 5.50 g (13.3 mMol) (3RS,4RS)-3-Hydroxy-4-[4-(2-phenoxy-äthoxy)-phenyl]-piperidin-1-carbonsäure tert-butylester (Beispiel 47.6) in 50 ml Methylenchlorid wurden 250 mg 4-Dimethylamino-pyridin und 2.5 ml Triäthylamin gegeben und anschliessend unter Eiskühlung 2.77 g (14.5 mMol) 2-Naphthoylchlorid fest hinzugefügt. Hierauf wurde das Reaktionsgemisch während 20 Stunden bei Raumtemperatur gerührt, die Reaktionslösung zwischen Wasser und Methylenchlorid verteilt, die vereinigten Methylenchloridphasen über Magnesiumsulfat getrocknet, eingeengt und der so erhaltene Rückstand an Kieselgel mit Methylenchlorid/Aether (95:5) chromatographiert. Dabei resultierten 7.3 g (97% d. Th.) (3RS,4RS)-3-(Naphthalin-2-carbonyloxy)-4-[4-(2-phenoxy-äthoxy)-phenyl]-piperidin-1-carbonsäure tert-butylester in Form eines amorphen, farblosen Festkörpers; MS: 568 $(M+H)^+$.

(b) Unter Argon und Feuchtigkeitsauschluss wurden 255 mg (0.57 mMol) (3RS,4RS)-4-(4-Fluor-phenyl)-3-(1-naphthalin-2-yl-vinyloxy)-piperidin-1-carbonsäure tert-butylester [Beispiel 73 (b)] in 3 ml Tetrahydrofuran gelöst, bei 0° C mit 50 mg Triäthylamin gefolgt von 0.11 ml (ca. 1.1 mMol) Borandimethylsuflidkomplex versetzt und 30 Minuten bei Raumtemperatur gerührt. Hierauf wurden erneut unter Eiskühlung 1.5 ml 50 %ige KOH-Lösung in Wasser gefolgt von 1.5 ml 30 %iger Wasserstoffperoxid-Lösung in Wasser zugesetzt und das Reaktionsgemisch während 1.5 Stunden unter Rückfluss erwärmt. Nun wurde die Reaktionslösung zwischen Wasser und Methylenchlorid verteilt, die vereinigten Methylenchloridphasen über Magnesiumsulfat getrocknet, eingeengt und der so erhaltene Rückstand an Kieselgel mit Hexan/Essigester (1:1) chromatographiert. Dabei resultierten 30 mg (11% d. Th.) eines

Gemisches des (3RS,4RS)-4-(4-Fluor-phenyl)-3-[(RS)- und (SR)-2-hydroxy-1-naphthalin-2-yl-äthoxy]-piperidin-1-carbonsäure tert-butylesters in Form eines amorphen, farblosen Festkörpers; MS: 466 $(M+H)^+$.

Beispiel 88

(a) 25.23 g (91 mMol) 4-(4-Fluor-phenyl)-1,2,3,6-tetrahydro-pyridin-1-carbonsäure tert-butylester [hergestellt aus 4-(4-Fluor-phenyl)-1,2,3,6-tetrahydro-pyridin und Di-tert-butyl-dicarbonat in Analogie zu Beispiel 1 (f)] wurden in 200 ml 1,2-Dimethoxy-äthan suspendiert, dazu wurden bei 20° C 5.1 g (135 mMol) Natriumborhydrid zugegeben und anschliessend eine Lösung von 22.85 ml (182 mMol) Bortrifluorid-äthylätherat in 35 ml 1,2-Dimethoxyäthan während 45 Minuten unter zeitweiliger Kühlung bei 20° C zugetropft. Nach 2 1/2 Stunden Nachrühren bei Raumtemperatur wurde unter intensivem Rühren eine Lösung von 82 g (1.26 Mol) Kaliumhydroxid (86%) in 430 ml destilliertem Wasser während 1 Stunde bei Raumtemperatur zugetropft. Hierauf wurden innert 30 Minuten 69.3 ml (0.68 Mol) Wasserstoffperoxid (30%) bei Raumtemperatur zugetropft, dann während 3 Stunden bei Rückfluss gerührt. Nach dem Abkühlen auf Raumtemperatur wurde der Ansatz auf Eiswasser gegossen, das Produkt 3 mal mit je 200 ml Essigester extrahiert, die organischen Phasen wurden 2 mal mit je 300 ml destilliertem Wasser gewaschen, dann über Magnesiumsulfat getrocknet, filtriert und im Wasserstrahlvakuum eingeengt. Nach 90 Minuten Nachtrocknen im Hochvakuum bei Raumtemperatur wurden so 24.4 g (91% d. Th.) (3RS,4RS)-4-(4-Fluor-phenyl)-3-hydroxy-piperidin-1-carbonsäure tert-butylester in Form farbloser Kristalle erhalten; MS: 296 $(M+H)^+$.

(b) 39.2 g (0.2 Mol) 3-Methylbenzophenon und 38.4 g (0.24 Mol) Brom wurden in 1 1 Tetrachlorkohlenstoff während 8 Stunden bei Rückfluss gerührt. Nach dem Abdestillieren des Lösungsmittels im Wasserstrahlvakuum wurde das so erhaltene Rohprodukt an 500 g Kieselgel mit Hexan und Methylenchlorid chromatographiert. Das so gereinigte Produkt wurde aus n-Hexan umkristallisiert. Dabei erhielt man 21.76 g (40% d. Th.) (3-Brommethyl-phenyl)-phenyl-methanon in Form farbloser Kristalle; MS: 274, 276 $(M)^+$.

(c) 0.29 g (1 mMol) (3RS,4RS)-4-(4-Fluor-phenyl)-3-hydroxy-piperidin- 1-carbonsäure tert-butylester und 0.30 g (1.1 mMol) (3-Brommethylphenyl)-phenyl-methanon wurden in 10 ml Dimethylformamid unter Argon bei Raumtemperatur gelöst, dann unter Zusatz von 0.25 g (1.5 mMol) Kaliumiodid mit 0.056 g (1.3 mMol) Natriumhydrid-Dispersion (55% in Mineralöl) versetzt und während 18 Stunden bei Raumtemperatur gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. So erhielt man 0.42 g (86% d. Th.) (3RS,4RS)-3-(3-Benzoyl-benzyloxy)-4-(4-fluor-phenyl)-piperidin-1-carbonsäure tert-butylester als farbloses Öl; MS: 490 $(M+H)^+$.

(d) In analoger Weise wie in Beispiel 22 (1) beschrieben, wurde aus (3RS,4RS)-3-(3-Benzoyl-benzyloxy)-4-(4-fluor-phenyl)-piperidin-1-carbonsäure tert-butylester durch Abspaltung der BOC-Gruppe mit Chlorwasserstoff in Methanol1[3-[(3RS,4RS)-4-(4-Fluor-phenyl)-piperidin-3-yloxymethyl]-phenyl]-phenyl-methanon Hydrochlorid (1: 1) in Form farbloser Kristalle erhalten; MS: 390 $(M+H)^+$.

Beispiel 89

In analoger Weise wie in Beispiel 73 (d) beschrieben, wurden durch Abspaltung der BOC-Gruppe mittels wasserfreiem Zinkbromid die folgenden Verbindungen erhalten:

1) - Aus (E)-(3RS,4RS)-4-(4-Fluor-phenyl)-3-(3-phenyl-allyloxy)-piperidin-1-carbonsäure tert-butylester (E)-(3RS, 4RS)-4-(4-Fluorphenyl)-3-(3-phenyl-allyloxy)-piperidin als hellgelbes Öl; MS: 312 $(M+H)^+$;

2) - aus (3R,4s,5S)-4-[4-(3-Benzyloxy-propoxy)-phenyl]-3,5-bis-(4,5-dimethoxy-pyrimidin-2-yloxymethyl)-piperidin-1-carbonsäure tert-butylester 2-[(3R,4S,5S)-4-[4-(3-Benzyloxy-propoxy)-phenyl]-5-(4,5-dimethoxy-pyrimidin-2-yloxymethyl)-piperidin-3-ylmethoxy}-4,5-dimethoxy-pyrimidin als hellgelbes Harz; MS: 662 $(M+H)^+$;

3) - aus (3R,4s,5S)-4-[4-(3-Benzyloxy-propoxy)-phenyl]-3,5-bis-(4-methoxy-phenoxymethyl)-piperidin-1-carbonsäure tert-butylester (3R,4s,5S)-4-[4-(3-Benzyloxy-propoxy)-phenyl]-3,5-bis-(4-methoxyphenoxymethyl)-piperidin als hellgelbes Öl; MS: 598 $(M+H)^+$;

4) - aus (3R,4s,5S)-4-[4-(3-Benzyloxy-propoxy)-phenyl]-3,5-bis-ptolylsulfanylmethyl-piperidin-1-carbonsäure tert-butylester (3R,4S,5S)-4-[4-(3-Benzyloxy-propoxy)-phenyl]-3,5-bis-p-tolylsulfanylmethyl-piperidin als hellgelbes Öl; MS: 598 $(M+H)^+$.

5) - aus (3RS,4RS)-4-[4-[3-(2-Methoxy-benzyloxy)-propoxy]-phenyl]-3-[ [7-[2-(4-methyl-piperazin-1-yl)-äthoxy]-naphthalin-2-ylmethoxy]-piperidin-1-carbonsäure tert-butylester 1-[2-[7-[(3RS,4RS)-4-[4-[3-(2-Methoxybenzyloxy)-propoxy]-piperidin-3-yloxymethyl]-naphthalin-2-yloxy]-äthyl]-4-methyl-piperazin in Form eines amorphen, farblosen Festkörpers; MS: 654 $(M+H)^+$.

Die als Ausgangssubstanzen eingesetzten BOC-Derivate wurden wie folgt hergestellt:

(a) 1.48 g (5 mMol) (3RS,4RS)-4-(4-Fluor-phenyl)-3-hydroxy-piperidin-1-carbonsäure tert-butylester [Beispiel 88 (a)] und 1.08 g (5.5 mMol) 3-Brom-1-phenyl-propen wurden in 10 ml Dimethylformamid unter Argon bei Raumtemperatur gelöst, dann unter Zusatz von 1.25 g (7.5 mMol) Kaliumiodid mit 0.284 g (6.5 mMol) Natriumhydrid-Dispersion (55% in Mineralöl) versetzt und während 18 Stunden bei Raumtemperatur gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 1.01 g (49% d. Th.) (E)-(3RS,4RS)-4-(4-Fluor-phenyl)-3-(3-phenyl-allyloxy)-piperidin-1-carbonsäure tert-butylester als farbloses Öl; MS : 412 $(M+H)^+$.

(b) 0.49 g (1 mMol) (3R,4s,5S)-4-[4-(3-Benzyloxy-propoxy)-phenyl]-3,5-bishydroxymethyl-piperidin-1-carbonsäure tert-butylester [Beispiel 101 (f)] und 0.45 g (2 mMol) 4,5-Dimethoxy-2-methylsulfonyl-pyrimidin [hergestellt aus 4,5-Dimethoxy-2-methylsulfanyl-pyrimidin durch Oxidation mit m-Chlorperbenzoesäure in analoger Weise wie in Beispiel 129 (c) beschrieben] wurden in 5 ml Dimethylformamid unter Argon bei 5 °C vorgelegt, unter Rühren mit 0.10 g (2.2 mMol) Natriumhydrid-Dispersion (55% in Mineralöl) versetzt und während 2 Stunden bei Raumtemperatur gerührt. Das Reaktionsgemisch wurde hierauf auf Eiswasser gegossen, das Produkt 3 mal mit Methylenchlorid extrahiert, die organischen Phasen wurden 2 mal mit destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.47 g (61% d. Th.) (3R,4s,5S)-4-[4-(3-Benzyloxy-propoxy)-phenyl]-3,5-bis-(4,5-dimethoxy-pyrimidin-2-yloxymethyl)-1-carbonsäure tert-butylester als farbloses Öl; MS : 762 $(M+H)^+$.

(c) ($\alpha$) 2.91 g (6 mMol) (3R,4s,5S)-4-[4-(3-Benzyloxy-propoxy)-phenyl]-3,5-bis-hydroxymethyl-piperidin-1-carbonsäure tert-butylester [Beispiel 101 (f)] und 1.93 ml (24 mMol) Pyridin wurden in 30 ml Acetonitril unter Argon bei 5 °C vorgelegt, unter Rühren 8.00 g (18 mMol) Triphenylphosphin-dibromid portionenweise eingetragen und danach bei Raumtemperatur weitergerührt. Nach 90 Minuten wurde das Reaktionsgemisch wurde auf Eiswasser gegossen, dann das Produkt 3 mal mit Essigester extrahiert, die organischen Phasen wurden 2 mal mit destilliertem Wasser gewaschen, über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 2.81 g (77% d. Th.) (3R,4s,5S)-4-[4-(3-Benzyloxy-propoxy)-phenyl]-3,5-bis-bromomethyl-piperidin-1-carbonsäure tert-butylester als farbloses Öl; MS : 610 $(M+H)^+$.

(c) ($\beta$) 0.30 g (0.5 mMol) (3R,4s,5S)-4-[4-(3-Benzyloxy-propoxy)-phenyl]-3,5-bis-bromomethyl-piperidin-1-carbonsäure tert-butylester und 0.19 g (1.5 mMol) Hydrochinon-monomethyläther wurden unter Zusatz von 0.69 g (5 mMol) wasserfreiem Kaliumcarbonat in 15 ml Acetonitril während 18 Stunden unter Argon bei Rückfluss gerührt. Nach dem Abkühlen auf Raumtemperatur wurde das Reaktionsgemisch auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.15 g (43% d. Th.) (3R,4s,5S)-4-[4-(3-Benzyloxypropoxy)-phenyl]-3,5-bis-(4-methoxy-phenoxymethyl)-piperidin-1-carbonsäure tert-butylester als hellgelbe Kristalle; MS : 698 $(M+H)^+$.

(d) In analoger Weise wie in Beispiel 89 (c) ($\beta$) beschrieben, wurde aus (3R,4s,5S)-4-[4-(3-Benzyloxy-propoxy)-phenyl]-3,5-bis-bromomethylpiperidin-1-carbonsäure tert-butylester und 4-Methyl-thiophenol (3R,4s,5S)-4-[4-(3-Benzyloxy-propoxy)-phenyl]-3,5-bis-p-tolylsulfanylmethyl-piperidin-1-carbonsäure tert-butylester in Form eines farblosen Öles erhalten; MS : 698 $(M+H)^+$.

(e) In analoger Weise wie in Beispiel 95 (a) beschrieben, wurde aus (3RS,4RS)-3-Hydroxy-4-[4-[3-(2-methoxy-benzyloxy)-propoxy]-phenyl]-piperidin-1-carbonsäure tert-butylester [Beispiel 120 (g) (α)] und 2-Chlormethyl-7-(2-trimethylsilanyl-äthoxymethoxy)-naphthalin [Beispiel 6 (u)] (3RS,4RS)-4-[4-[3-(2-Methoxy-benzyloxy)-propoxy]-phenyl]-3-[7-(2-trimethylsilanyl-äthoxymethoxy)-naphthalin-2-ylmethoxy]-piperidin-1-carbonsäure tert-butylester als hellgelbes Öl erhalten; MS: 758 $(M+H)^+$. Hierauf wurde der (3RS,4RS)-4-[4-[3-(2-Methoxy-benzyloxy)-propoxy]-phenyl]-3-[7-(2-trimethylsilanyl-äthoxymethoxy)-naphthalin-2-ylmethoxy]-piperidin-1-carbonsäure tert-butylester in analoger Weise wie in Beispiel 95 (b) beschrieben durch Abspaltung der SEM Schutzgruppe zu dem (3RS,4RS)-4-[4-[3-(2-Methoxy-benzyloxy)-propoxy]-phenyl]-3-(7-hydroxy-naphthalin-2-ylmethoxy)-piperidin-1-carbonsäure tert-butylester [gelbes Öl; MS: 628 $(M+H)^+$] umgesetzt, dessen Alkylierung mit 1-(2-Chlor-äthyl)-4-methyl-piperazin Hydrochlorid (1:2) [Chim. Ther. 4, 283 (1969)] in analoger Weise wie in Beispiel 90 (n) beschrieben den (3RS,4RS)-4-[4-[3-(2-Methoxy-benzyloxy)-propoxy]-phenyl]-3-[7-[2-(4-methyl-piperazin-1-yl)-äthoxy]-naphthalin-2-ylmethoxy]-piperidin-1-carbonsäure tert-butylester als hellbraunes Öl ergab; MS: 754 $(M+H)^+$.

Beispiel 90

In analoger Weise wie in Beispiel 22 (l) beschrieben, wurden durch Abspaltung der BOC-Gruppe mittels Chlorwasserstoff in Methanol die folgenden Verbindungen erhalten:

1) - Aus dem Gemisch des (3RS,4SR)-4-(4-Fluor-phenyl)-3-[(RS)- und -[(SR)-1-[4-(2-morpholin-4-yl-äthoxy)-benzoyloxy]-2-naphthalin-2-yl-äthyl)-piperidin-1-carbonsäure tert-butylesters ein Gemisch des 4-(2-Morpholin-4-yläthoxy)-benzoesäure (RS)- und (SR)-1-[(3RS,4SR)-4-(4-fluor-phenyl)-piperidin-3-yl]-2-naphthalin-2-yl-äthylesters als farbloses Öl; MS: 583 $(M+H)^+$;

2) - aus dem (3RS,4SR)-4-(4-Fluor-phenyl)-3-(naphthalin-2-yl-acetyl)-piperidin-1-carbonsäure tert-butylester das 1-[(3RS,4SR)-4-(4-Fluorphenyl)-piperidin-3-yl]-2-naphthalin-2-yl-äthanon Hydrochlorid (1:1) in Form farbloser Kristalle; MS: 348 $(M+H)^+$;

3) - aus dem Gemisch des (E)- und (Z)-(3RS,4SR)-3-(1-Carboxymethoxyimino-2-naphthalin-2-yl-äthyl)-4-(4-fluor-phenyl)-piperidin-1-carbonsäure tert-butylesters ein 3:1 Gemisch des (E)- und (Z)-[1-[(3RS,4SR)-4-(4-Fluor-phenyl)-piperidin-3-yl]-2-naphthalin-2-yl-äthyliden-aminooxy]-essigsäure methylesters als gelbes Öl; MS: 435 $(M+H)^+$;

4) - aus dem (3RS,4RS)-3-[2-(3H-Benzoimidazol-5-yloxy)-äthoxy]-4-[4-(3-benzyloxy-propoxy)-phenyl]-piperidin-1-carbonsäure tert-butylester das 6-[2-[(3RS,4RS)-4-[4-(3-Benzyloxy-propoxy)-phenyl]-piperidin-3-yloxy]-äthoxy]-1H-benzoimidazol als gelbes Öl; MS: 502 $(M+H)^+$;

5) - aus dem (3RS,4RS)-4-[4-(3-Benzyloxy-propoxy)-phenyl]-3-[2-(2-oxo-2,3-dihydro-1H-benzo-imidazol-5-yloxy)-äthoxy]-piperidin-1-carbonsäure tert-butylester das 5-[2-[(3RS,4RS)-4-[4-(3-Benzyloxypropoxy)-phenyl]-piperidin-3-yloxy]-äthoxy]-1,3-dihydro-benzoimidazol-2-on in Form gelber Kristalle; MS: 518 $(M+H)^+$;

6) - aus dem (3RS,4RS)-4-[4-(3-Benzyloxy-propoxy)-phenyl]-3-[2-(3,4-dinitro-phenoxy)-äthoxy]-piperidin-1-carbonsäure tert-butylester [Beispiel 94 (d)] das (3RS,4RS)-4-[4-(3-Benzyloxy-propoxy)-phenyl]-3-[2-(3,4-dinitro-phenoxy)-äthoxy]-piperidin als amorpher, gelber Schaum; MS: 552 $(M+H)^+$;

7) - aus dem (3RS,4RS)-4-[4-(3-Benzyloxy-propoxy)-phenyl]-3-[7-(2-morpholin-4-yl-äthoxy)-naphthalin-2-ylmethoxy]-piperidin-1-carbonsäure tert-butylester das 4-[2-[7-[(3RS,4RS)-4-[4-(3-Benzyloxypropoxy)-phenyl]-piperidin-3-yloxymethyl]-naphthalin-2-yloxy]-äthyl]-morpholin Hydrochlorid (1:2) in Form beiger Kristalle; MS: 611 $(M+H)^+$;

8) - aus dem Gemisches des (3RS,4RS)- und (3SR,4SR)-4-[4-(3-Benzyloxy-propoxy)-phenyl]-3-[7-[(RS)-2,2-dimethyl-[1,3] dioxolan-4-ylmethoxy]-naphthalin-2-ylmethoxy]-piperidin-1-carbonsäure tert-butylesters ein Gemisch des (RS)- und (SR)-3-[7-[(3RS,4RS)-4-[4-(3-Benzyloxy-propoxy)-phenyl]-piperidin-3-yloxymethyl]-naphthalin-2-yloxy]-propan-1,2-diols als farbloses Öl; MS: 572 $(M+H)^+$;

9) - aus dem (3RS,4RS)-4-[4-(3-Benzyloxy-propoxy)-phenyl]-3-[6-(2-trimethylsilanyl-äthoxymethoxy)-naphthalin-2-ylmethoxy]-piperidin-1-carbonsäure tert-butylester [Beispiel 97 (a)] das 6-[(3RS,4RS)-4-[4-(3-Benzyloxy-propoxy)-phenyl]-piperidin-3-yloxymethyl]-naphthalin-2-ol Hydrochlorid (1:1) in Form farbloser Kristalle; MS: 498 (M+H)+;

10) - aus dem Gemisch des [3RS,4RS]- und [3SR,4SR]-4-[4-(3-Benzyloxy-propoxy)-phenyl]-3-[6-[(RS)-2,2-dimethyl-[1,3]dioxolan-4-ylmethoxy)-naphthalin-2-ylmethoxy]-piperidin-1-carbonsäure tert-butylesters unter gleichzeitiger Abspaltung der Dioxolan Schutzgruppe ein Gemisch des (RS)- und (SR)-3-[6-[(3RS,4RS)-4-[4-(3-Benzyloxy-propoxy)-phenyl]-piperidin-3-yloxymethyl]-naphthalin-2-yloxy]-propan-1,2-diol Hydrochlorides (1:1) in Form hellbrauner Kristalle; MS: 572 $(M+H)^+$;

11) - aus dem Gemisch des (3RS,4RS)- und (3SR,4SR)-4-[4-(3-Benzyloxy-propoxy)-phenyl]-3-[6-[2-[(RS)-2,2-dimethyl-[1,3]dioxolan-4-ylmethoxy]-äthoxy]-naphthalin-2-ylmethoxy]-piperidin-1-carbonsäure tert-butylesters unter gleichzeitiger Abspaltung der Dioxolan Schutzgruppe ein Gemisch des [RS]- und [SR]-3-[2-[6-[(3RS,4RS)-4-[4-(3-Benzyloxy-propoxy)-phenyl]-piperidin-3-yloxymethyl]-naphthalin-2-yloxy]-äthoxy]-propan-1,2-diol Hydrochlorides (1:1) in Form farbloser Kristalle; MS: 616 $(M+H)^+$;

12) - aus dem Gemisch des (3RS,4SR)-4-[4-(3-Benzyloxy-propoxy)-phenyl]-3-[(RS)- und -[(SR)1-hydroxy-2-naphthalin-2-yl-äthyl]-piperidin-1-carbonsäure tert-butylesters [Beispiel 100 (b)] ein Gemisch des (RS)-und (SR)-1-[(3RS,4SR)-4- [4-(3-Benzyloxy-propoxy)-phenyl]-piperidin-3-yl]-2-naphthalin-2-yl-äthanol Hydrochlorides (1:1) in Form beiger Kristalle; MS: 496 $(M+H)^+$;

13) aus dem (3RS,4RS)-4-[4-(3-Benzyloxy-propoxy)-phenyl]-3-[7-[2-(4-methyl-piperazin-1-yl)-äthoxy]-naphthalin-2-ylmethoxy]-piperidin- 1-carbonsäure tert-butylester das 1-[2-[7-[(3RS,4RS)-4-[4-(3-Benzyloxypropoxy)-phenyl]-piperidin-3-yloxymethyl]-naphthalin-2-yloxy]-äthyl]-4-methyl-piperazin Hydrochlorid (1:3) in Form farbloser Kristalle; MS: 624 $(M+H)^+$;

14) aus dem Gemisch des (E)- und (Z)-(3RS,4SR)-4-[4-(3-Benzyloxypropoxy)-phenyl]-3-(1-methoxycarbonyl-methoxyimino-2-naphthalin-2-yl-äthyl)-piperidin-1-carbonsäure tert-butylesters ein Gemisch des (E)-und (Z) -(1-[(3RS,4SR)-4-[4-(3-Benzyloxy-propoxy)-phenyl]-piperidin-3-yl]-2-naphthalin-2-yl-äthyliden-aminooxy)-essigsäure methyl esters als hellgelbes Öl; MS: 581 $(M+H)^+$;

15) aus dem (3R,4S,5S)-4-[4-(3-Benzyloxy-propoxy)-phenyl]-3,5-bis-(2-morpholin-4-yl-äthoxymethyl)-piperidin-1-carbonsäure tert-butylester [Beispiel 101 (g)] das (3R,4s,5S)-4-[4-(3-Benzyloxy-propoxy)-phenyl]-3,5-bis-(2-morpholin-4-yl-äthoxymethyl)-piperidin als gelbes Öl; MS: 612 $(M+H)^+$.

Die als Ausgangssubstanzen eingesetzten BOC-Derivate wurden wie folgt hergestellt:

(a) 0.45 g (1 mMol) des Gemisches des (3RS,4SR)-4-(4-Fluor-phenyl)-3-[(RS)- und -[(SR)-1-hydroxy-2-naphthalin-2-yl-äthyl]-piperidin-1-carbonsäure tert-butylesters [Beispiel 74 (h)] und 0.28 g (1.1 mMol) 4-(2-Morpholin-4-yl-äthoxy)-benzoesäure (hergestellt durch Alkylierung von 4-Hydroxybenzoesäuremethylester mit 4-(2-Chloräthyl)-morpholin in Dimethylformamid in Gegenwart von Kaliumcarbonat bei 100° C und anschliessende basische Verseifung) wurden in 15 ml Methylenchlorid unter Argon gelöst, dann wurden 0.23 g (1.2 mMol) N-Aethyl-N'-(3-dimethylaminopropyl)-carbodiimid Hydrochlorid und 0.04 g (0.33 mMol) 4-Dimethylamino-pyridin zugegeben und während 70 Stunden bei Raumtemperatur gerührt. Nach dem Abdestillieren des Lösungsmittels im Wasserstrahlvakuum wurde das Rohprodukt an Kieselgel mit n-Hexan, Methylenchlorid und Methanol chromatographiert. Dabei erhielt man 0.54 g (79% d. Th.) eines Gemisch des (3RS,4SR)-4-(4-Fluorphenyl)-3-[(RS)- und -[(SR)-1-[4-(2-morpholin-4-yl-äthoxy)-benzoyloxy]-2-naphthalin-2-yl-äthyl]-piperidin-1-carbonsäure tert-butylesters als hellgelbes Öl; MS: 683 $(M+H)^+$.

(b) In analoger Weise wie in Beispiel 74 (f) beschrieben, wurde aus dem Gemisch des (3RS,4SR)-4-(4-Fluor-phenyl)-3-[(RS)- und -[(SR)-1-hydroxy-2-naphthalin-2-yl-äthyl]-piperidin-1-carbonsäure tert-butylesters [Beispiel 74 (h)] durch Oxidation mit Dimethylsulfoxid/Oxalylchlorid in Methylenchlorid der (3RS,4SR)-4-(4-Fluor-phenyl)-3-(naphthalin-2-ylacetyl)-piperidin-1-carbonsäure tert-butylester als gelbes Öl erhalten; MS: 447 $(M)^+$.

(c) 0.22 g ( 0.5 mMol) (3RS,4SR)-4-(4-Fluor-phenyl)-3-(naphthalin-2-ylacetyl)-piperidin-1-carbonsäure tert-butylester und 0.11 g (1 mMol) Aminooxy-essigsäure Hydrochlorid (1:0.5) [Organic Synthesis Collect. Vol. III, 172 (1955)] wurden in 2 ml Pyridin während 18 Stunden unter Argon bei 60° C gerührt. Das Reaktionsgemisch wurde hierauf auf Eiswasser gegossen, das Produkt 3 mal mit Essigester extrahiert, die organischen Phasen wurden 2 mal mit destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.24 g (92% d. Th.) eines Gemisches des (E)- und (Z)-(3RS,4SR)-3-(1-Carboxymethoxyimino-2-naphthalin-2-yl-äthyl)-4-(4-fluor-phenyl)-piperidin-1-carbonsäure tert-butylesters als hellgelbes Öl; MS: 521

$(M+H)^+$.

(d) 0.55 g (0.93 mMol) (3RS,4RS)-4-[4-(3-Benzyloxy-propoxy)-phenyl]-3-[2-(3,4-diamino-phenoxy)-äthoxy]-piperidin-1-carbonsäure tert-butylester [Beispiel 94 (e)] wurden in 5 ml Orthoameisensäure-triäthylester während 1 Stunde unter Argon bei 50° C gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Kaliumcarbonat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.38 g (68% d. Th.) 3-[2-(3H-Benzoimidazol-5-yloxy)-äthoxy]-4-[(3RS,4RS)-4-(3-benzyloxypropoxy)-phenyl]-piperidin-1-carbonsäure tert-butylester als hellbraunes Öl; MS: 602 $(M+H)^+$.

(e) 0.60 g (1.0 mMol) (3RS,4RS)-4-[4-(3-Benzyloxy-propoxy)-phenyl]-3-[2-(3,4-diamino-phenoxy)-äthoxy]-piperidin-1-carbonsäure tert-butylester [Beispiel 94 (e)] wurden in 5 ml Dimethylformamid unter Argon bei Raumtemperatur gelöst, dann 0.18 g (1.1 mMol) 1,1'-Carbonyldiimidazol zugegeben. Nach 1 Stunde wurde das Reaktionsgemisch auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.46 g (74% d. Th.) (3RS,4RS)-4-[4-(3-Benzyloxypropoxy)-phenyl]-3-[2-(2-oxo-2,3-dihydro-1H-benzo-imidazol-5-yloxy)-äthoxy]-piperidin-1-carbonsäure tert-butylester als gelbes Öl; MS: 618 $(M+H)^+$.

(g) 0.30 g ( 0.5 mMol) (3RS,4RS)-4-[4-(3-Benzyloxy-propoxy)-phenyl]-3-(7-hydroxy-naphthalin-2-ylmethoxy)-piperidin-1-carbonsäure tert-butylester [Beispiel 95 (b)] und 0.13 g (0.7 mMol) 4-(2-Chloräthyl)-morpholin-hydrochlorid wurden in 15 ml Dimethylformamid unter Zusatz von 0.69 g (5 mMol) Kaliumcarbonat (wasserfrei) während 18 Stunden unter Argon bei 60° C gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.32 g (90% d. Th.) (3RS,4RS)-4-[4-(3-Benzyloxy-propoxy)-phenyl]-3-[7-(2-morpholin-4-yl-äthoxy)-naphthalin-2-ylmethoxy]-piperidin-1-carbonsäure tert-butylester als farbloses Öl; MS: 711 $(M+H)^+$.

(h) 0.33 g ( 0.54 mMol) (3RS,4RS)-4-[4-(3-Benzyloxy-propoxy)-phenyl]-3-(7-hydroxy-naphthalin-2-ylmethoxy)-piperidin-1-carbonsäure tert-butylester [Beispiel 95 (b)] und 0.20 g (0.70 mMol) D,L-$\alpha$,$\beta$-Isopropylidenglycerin-$\gamma$-tosylat wurden in 15 ml Dimethylformamid unter Zusatz von 0.69 g (5 mMol) Kaliumcarbonat (wasserfrei) während 3 Stunden unter Argon bei Rückfluss gerührt. Das Reaktionsgemisch wurde hierauf auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.32 g (83% d. Th.) eines Gemisch des (3RS,4RS)- und (3SR,4SR)-4-[4-(3-Benzyloxy-propoxy)-phenyl]-3-[7-[(RS)-2,2-dimethyl-[1,3] dioxolan-4-ylmethoxy]-naphthalin-2-ylmethoxy]-piperidin-1-carbonsäure tert-butylesters als farbloses Öl; MS: 712 $(M+H)^+$.

(k) In analoger Weise wie in Beispiel 90 (h) beschrieben, wurde aus dem (3RS,4RS)-4-[4-(3-Benzyloxy-propoxy)-phenyl]-3-(6-hydroxy-naphthalin-2-ylmethoxy)-piperidin-1-carbonsäure tert-butylester [Beispiel 97 (b)] ein Gemisch des [3RS,4RS]- und [3SR,4SR]-4-[4-(3-Benzyloxy-propoxy)-phenyl]-3-[6-[(RS)-2,2-dimethyl-[1,3]dioxolan-4-ylmethoxy)-naphthalin-2-ylmethoxy]-piperidin-1-carbonsäure tert-butylesters in Form farbloser Kristalle erhalten; MS: 712 $(M+H)^+$.

(l) In analoger Weise wie in Beispiel 90 (g) beschrieben, wurde aus dem (3RS,4RS)-4-[4-(3-Benzyloxy-propoxy)-phenyl]-3-(6-hydroxy-naphthalin-2-ylmethoxy)-piperidin-1-carbonsäure tert-butylester [Beispiel 97 (b)] und dem Toluol-4-sulfonsäure- (RS)-2-(2,2-dimethyl-[1,3]dioxolan-4-ylmethoxy)-äthylester [Beispiel 98 (a)] ein Gemisch des (3RS,4RS)- und (3SR,4SR)-4-[4-(3-Benzyloxy-propoxy)-phenyl]-3-[6-[2-[(RS)-2,2-dimethyl-[1,3] dioxolan-4-ylmethoxy]-äthoxy]-naphthalin-2-ylmethoxy]-piperidin-1-carbonsäure tert-butylesters in Form farbloser Kristalle erhalten; MS: 773 $(M+NH4)^+$.

(n) 0.30 g (0.5 mMol) (3RS,4RS)-4-[4-(3-Benzyloxy-propoxy)-phenyl]-3-(7-hydroxy-naphthalin-2-yl-methoxy)-piperidin-1-carbonsäure tert-butylester [Beispiel 95 (b)] und 0.47 g (2 mMol) 1-(2-Chlor-äthyl)-4-methyl-piperazin Hydrochlorid (1:2) [Chim. Ther. 4, 283 (1969)] wurden in 5 ml Dimethylformamid unter Argon bei Raumtemperatur

gelöst, dann unter Zusatz von 0.05 g (0.3 mMol) Kaliumiodid und 0.22 g (5 mMol) Natriumhydrid-Dispersion (55% in Mineralöl) während 3 Stunden bei 100° C gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. So erhielt man 0.20 g (55% d. Th.) (3RS,4RS)-4-[4-(3-Benzyloxy-propoxy)-phenyl]-3-[7-[2-(4-methyl-piperazin-1-yl)-äthoxy]-naphthalin-2-yl-methoxy]-piperidin-1-carbonsäure tert-butylester als hellbraunes Öl; MS: 724 $(M+H)^+$.

(o) In analoger Weise wie in Beispiel 102 (a) beschrieben, wurde aus (3RS,4SR)-4-[4-(3-Benzyloxy-propoxy)-phenyl]-3-(naphthalin-2-yl-acetyl)-piperidin-1-carbonsäure tert-butylester [Beispiel 100 (c)] und Aminooxyessigsaure methylester- hydrochlorid [J. Med. Chem. 28, 1447 (1985)] ein Gemisch des (E)- und (Z)-(3RS,4SR)-4-[4-(3-Benzyloxy-propoxy)-phenyl]-3-(1-methoxycarbonylmethoxyimino-2-naphthalin-2-yl-äthyl)-piperidin-1-carbonsäure tert-butylesters als farbloses Öl erhalten; MS: 698 $(M+NH4)^+$.

Beispiel 91

(a) 0.90 g (2 mMol) des Gemisches des (3RS,4SR)-4-(4-Fluor-phenyl)-3-[(RS)- und -[(SR)-1-hydroxy-2-naphthalin-2-yl-äthyl]-piperidin-1-carbonsäure tert-butylesters [Beispiel 74 (h)] und 0.55 g (2.2 mMol) 4-(2-Morpholin-4-yl-äthoxy)-benzoesäure (hergestellt durch Alkylierung von 4-Hydroxybenzoesäuremethylester mit 4-(2-Chloräthyl)-morpholin in Dimethylformamid in Gegenwart von Kaliumcarbonat bei 100° C und anschliessende basische Verseifung) wurden in 30 ml Tetrahydrofuran unter Argon gelöst, dann nach Zusatz von 0.66 g (2.5 mMol) Triphenylphosphin bei 5° C 0.44 ml (2.8 mMol) Diäthyl-azo-dicarboxylat zugetropft und anschliessend während 18 Stunden bei Raumtemperatur gerührt. Nach dem Abdestillieren des Lösungsmittels im Wasserstrahlvakuum wurde das Rohprodukt an Kieselgel mit n-Hexan und Essigester gereinigt. Dabei erhielt man 0.31 g (23% d. Th.) (E)-(3RS, 4SR)-4-(4-Fluor-phenyl)-3-(2-naphthalin-2-yl-vinyl)-piperidin-1-carbonsäure tert-butylester als hellgelbes Harz; MS: 432 $(M+H)^+$.

(b) In analoger Weise wie in Beispiel 22 (1) beschrieben, wurde durch Abspaltung der BOC-Gruppe mittels Chlorwasserstoff in Methanol aus dem (E)-(3RS,4SR)-4-(4-Fluor-phenyl)-3-(2-naphthalin-2-yl-vinyl)-piperidin-1-carbonsäure tert-butylester das (E)-(3RS,4SR)-4-(4-Fluorphenyl)-3-(2-naphthalin-2-yl-vinyl)-piperidin als hellgelbes Öl erhalten; MS: 331 $(M)^+$.

(c) 0.060 g (0.18 mMol) (E)-(3RS,4SR)-4-(4-Fluor-phenyl)-3-(2-naphthalin-2-yl-vinyl)-piperidin wurden in 3 ml Methanol unter Zusatz von 30 mg Pd-C (10%) unter Normalbedingungen hydriert. Nach dem Abfiltrieren des Katalysators wurde das Lösungsmittel im Wasserstrahlvakuum abdestilliert. So wurden 0.055 g (92% d. Th.) (3RS, 4SR)-4-(4-Fluorphenyl)-3-(2-naphthalin-2-yl-äthyl)-piperidin als hellgelbes Öl erhalten; MS: 334 $(M+H)^+$.

Beispiel 92

(a) 2.95 g (10 mMol) (3RS,4RS)-4-(4-Fluor-phenyl)-3-hydroxy-piperidin-1-carbonsäure tert-butylester [Beispiel 88 (a)] und 3.82 g (11 mMol) 4'-Brommethyl-biphenyl-2-carbonsäure tert-butylester [J. Med. Chem. 34, 2525 (1991)] wurden in 100 ml Dimethylformamid unter Argon bei Raumtemperatur gelöst, dann zuerst 2.49 g (15 mMol) Kaliumiodid und danach 0.57 g (13 mMol) Natriumhydrid-Dispersion (55% in Mineralöl) zugegeben. Nach 4 Stunden Rühren bei Raumtemperatur wurde das Reaktionsgemisch auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 5.08 g (90% d. Th.) (3RS,4RS)-3-(2'-tert-Butoxycarbonyl-biphenyl-4-ylmethoxy)-4-(4-fluor-phenyl)-piperidin-1-carbonsäure tert-butylester als amorphen, farblosen Schaum.

(b) 2.25 g (4 mMol) (3RS,4RS)-3-(2'-tert-Butoxycarbonyl-biphenyl-4-ylmethoxy)-4-(4-fluor-phenyl)-piperidin-1-carbonsäure tert-butylester wurden in 50 ml Aethylenglykol-monomethyläther unter Zusatz von 8 ml (36 mMol) Natronlauge (14%) während 18 Stunden bei Rückfluss gerührt. Nach dem Abdestillieren des Lösungsmittels im Wasserstrahlvakuum wurde der Rückstand in Eiswasser gelöst, dann mit 2N Salzsäure auf pH 3 gestellt und das

Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 1.66 g (82% d. Th.) (3RS,4RS)-3-(2'-Carboxy-biphenyl-4-ylmethoxy)-4-(4-fluor-phenyl)-piperidin-1-carbonsäure tert-butylester als amorphen, farblosen Schaum; MS: 504 $(M-H)^-$.

(c) 0.51 g (1 mMol) (3RS,4RS)-3-(2'-Carboxy-biphenyl-4-ylmethoxy)-4-(4-fluor-phenyl)-piperidin-1-carbonsäure tert-butylester und 0.18 g (1 mMol) 2-Chlor-4,6-dimethoxy-1,3,5-triazin wurden in einem Gemisch von 3.5 ml Dimethylformamid und 5 ml Acetonitril unter Argon gelöst, dazu bei 0° C 0.22 ml (2 mMol) N-Methylmorpholin zugetropft und während 2 Stunden bei 0° C gerührt. Nun wurde eine Lösung von 0.08 ml (1 mMol) (RS)-3-Amino-1,2-propandiol in 6 ml Acetonitril zugetropft und danach während 18 Stunden bei Raumtemperatur gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.29 g (50% d. Th.) eines Gemisch des (3RS,4RS)- und (3SR,4SR)-3-[2'[(RS)-(2,3-Dihydroxypropylcarbamoyl]-biphenyl-4-ylmethoxy]-4-(4-fluor-phenyl)-piperidin-1-carbonsäure tert-butylesters als amorphen, farblosen Schaum; MS: 579 $(M+H)^+$.

(d) In analoger Weise wie in Beispiel 22 (1) beschrieben, wurde aus dem Gemisch des (3RS,4RS)- und (3SR,4SR)-3-[2'[(RS)-(2,3-dihydroxypropylcarbamoyl]-biphenyl-4-ylmethoxy]-4-(4-fluor-phenyl)-piperidin-1-carbonsäure tert-butylesters durch Abspaltung der BOC Schutzgruppe mit Chlorwasserstoff in Methanol ein Gemisch der 4'-[(3RS,4RS)-4-(4-Fluor-phenyl)-piperidin-3-yloxymethyl]-biphenyl-2-carbonsäure (RS)-und (SR)-(2,3-dihydroxy-propyl)-amid Hydrochloride (1:1) als amorphen, farblosen Schaum erhalten; MS : 479 $(M+H)^+$.

Beispiel 93

(a) 0.050 g ( 0.12 mMol) des 3:1 Gemisches der (E)- und (Z)-[1-[(3RS,4SR)-4-(4-Fluor-phenyl)-piperidin-3-yl]-2-naphthalin-2-yl-äthylidenaminooxy]-essigsäuremethylester [Beispiel 90.03] wurden in 3 ml Methanol unter Zusatz von 1.0 ml 1N Natronlauge während 18 Stunden bei 50° C gerührt. Nach dem Abkühlen auf Raumtemperatur wurden 1.0 ml 1N Salzsäure zugetropft und danach im Wasserstrahlvakuum eingeengt. Der Rückstand wurde in Aethanol suspendiert, dann filtriert, zum Filtrat 0.025 ml (0.3 mMol) Salzsäure (37%) zugegeben und wiederum im Wasserstrahlvakuum eingeengt. Dieser Rückstand wurde nun während 2 Stunden im Hochvakuum bei Raumtemperatur getrocknet. So erhielt man 0.040 g (73% d. Th.) eines Gemisches der (E)-und (Z)-(3RS,4SR)-(1-[4-(4-Fluor-phenyl)-piperidin-3-yl]-2-naphthalin-2-yl-äthylidenaminooxy)-essigsäure Hydrochloride (1:1) als amorphen, hellgelben Schaum; MS : 421 $(M+H)^+$.

Beispiel 94

(a) 4.30 g ( 9.7 mMol) (3RS,4RS)-4-[4-(3-Benzyloxy-propoxy)-phenyl]-3-hydroxy-piperidin-1-carbonsäure tert-butylester [Beispiel 86 (n)] wurden in 215 ml Tetrahydrofuran unter Argon bei 0° C vorgelegt und unter Rühren 1.23 g ( 28.1 mMol) Natriumhydrid-Dispersion (55% in Mineralöl) zugegeben. Nach 30 Minuten wurde eine Lösung von 1.86 ml (12.7 mMol) Bromessigsäure-tert-butylester in 10 ml Tetrahydrofuran zugetropft und auf Raumtemperatur erwärmt. Das Reaktionsgemisch wurde auf Eiswasser gegossen, das Produkt 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 4.77 g (88% d. Th.) (3RS,4RS)-4-[4-(3-Benzyloxy-propoxy)-phenyl]-3-tert-butoxycarbonylmethoxy-piperidin-1-carbonsäure tert-butylester als gelbes Öl; MS: 556 $(M+H)^+$.

(b) 0.36 g (16.5 mMol) Lithiumborhydrid wurden in 55 ml Tetrahydrofuran unter Argon bei Raumtemperatur vorgelegt und unter Rühren eine Lösung von 4.58 g (8.24 mMol) (3RS,4RS)-4-[4-(3-Benzyloxypropoxy)-phenyl]-3-tert-butoxycarbonylmethoxy-piperidin-1-carbonsäure tert-butylester in 55 ml Tetrahydrofuran zugetropft und anschliessend unter Rückfluss erwärmt. Nach 4 Stunden wurde das Reaktionsgemisch auf Eiswasser gegossen, mit Salzsäure (2N) auf pH 3 gestellt, dann das Produkt 3 mal mit Essigester extrahiert. Die organischen Phasen wurden

hierauf 2 mal mit destilliertem Wasser gewaschen, über Magnesiumsulfat getrocknet, filtriert und im Wasserstrahlvakuum eingeengt. So erhielt man 3.95 g (99% d. Th.) (3RS,4RS)-4-[4-(3-Benzyloxy-propoxy)-phenyl]-3-(2-hydroxy-äthoxy)-piperidin-1-carbonsäure tert-butylester als gelbes Öl; MS: 486 $(M+H)^+$.

(c) 5.56 g (13.2 mMol) Triphenylphosphin-dibromid wurden in 20 ml Acetonitril unter Argon gelöst, dann bei 0° C 1.06 ml (13.2 mMol) Pyridin zugetropft; diese Lösung wurde zu einer Lösung von 3.95 g (8.1 mMol) (3RS,4RS)-4-[4-(3-Benzyloxy-propoxy)-phenyl]-3-(2-hydroxy-äthoxy)-piperidin-1-carbonsäure tert-butylester in 20 ml Acetonitril bei 0° C zugetropft und anschliessend während 18 Stunden bei Raumtemperatur gerührt. Das Reaktionsgemisch wurde auf Eiswasser gegossen, dann das Produkt 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 3.14 g (71% d. Th.) (3RS,4RS)-4-[4-(3-Benzyloxy-propoxy)-phenyl]-3-(2-brom-äthoxy)-piperidin-1-carbonsäure tert-butylester als gelbes Öl; MS: 548, 550 $(M+H)^+$.

(d) 3.14 g (5.72 mMol) (3RS,4RS)-4-[4-(3-Benzyloxy-propoxy)-phenyl]-3-(2-brom-äthoxy)-piperidin-1-carbonsäure tert-butylester und 2.53 g (13.76 mMol) 3,4-Dinitrophenol wurden in 230 ml Acetonitril unter Zusatz von 7.9 g (57.2 mMol) Kaliumcarbonat (wasserfrei) während 22 Stunden unter Argon bei Rückfluss gerührt. Nach dem Abdestillieren des Lösungsmittels im Wasserstrahlvakuum wurde der Rückstand auf Eiswasser gegossen und das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 2.57 g (69% d. Th.) (3RS,4RS)-4-[4-(3-Benzyloxypropoxy)-phenyl]-3-[2-(3,4-dinitro-phenoxy)-äthoxy]-piperidin-1-carbonsäure tert-butylester als braunes Öl; MS: 652 $(M+H)^+$.

(e) 1.63 g (2.5 mMol) (3RS,4RS)-4-[4-(3-Benzyloxy-propoxy)-phenyl]-3-[2-(3,4-dinitro-phenoxy)-äthoxy]-piperidin-1-carbonsäure tert-butylester wurden in 80 ml Essigester unter Zusatz von 0.50 g Platinoxid unter Normal-bedingungen während 2 Stunden hydriert. Der Katalysator wurde durch ein Dicalit-Polster abfiltriert und das Lösungsmittel im Wasserstrahlvakuum abdestilliert. Man erhielt so 1.44 g (97% d. Th.) (3RS,4RS)-4-[4-(3-Benzyloxy-propoxy)-phenyl]-3-[2-(3,4-diaminophenoxy)-äthoxy]-piperidin-1-carbonsäure tert-butylester als violettes Öl; MS: 592 $(M+H)^+$.

(f) In analoger Weise wie in Beispiel 22 (l) beschrieben, wurde aus dem (3RS,4RS)-4-[4-(3-Benzyloxy-propoxy)-phenyl]-3-[2-(3,4-diaminophenoxy)-äthoxy]-piperidin-1-carbonsäure tert-butylester durch Abspaltung der BOC Schutzgruppe mit Chlorwasserstoff in Methanol das 4-[2-[(3RS,4RS)-4-[4-(3-Benzyloxy-propoxy)-phenyl]-piperidin-3-yloxy]-äthoxy]-benzol-1,2-diamin Hydrochlorid (1:3) als hellviolette Kristalle erhalten; MS: 492 $(M+H)^+$.

Beispiel 95

(a) 4.37 g (9.9 mMol) (3RS,4RS)-4-[4-(3-Benzyloxy-propoxy)-phenyl]-3-hydroxy-piperidin-1-carbonsäure tert-butylester [Beispiel 86 (n)] und 3.20 g (9.9 mMol) 2-Chlormethyl-7-(2-trimethylsilanyl-äthoxymethoxy)-naphthalin [Beispiel 6 (u)] wurden in 35 ml Dimethylformamid unter Argon gelöst, dann 0.46 g (10.5 mMol) Natriumhydrid-Dispersion (55% in Mineralöl) hinzugefügt. Anschliessend wurde während 18 Stunden bei Raumtemperatur gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahl-vakuum eingeengt. Das so erhaltene Rohprodukt wurde an Kieselgel mit Methylenchlorid und Methanol chromatographiert. Dabei erhielt man 7.15 g (99% d. Th.) (3RS,4RS)-4-[4-(3-Benzyloxy-propoxy)-phenyl]-3-[7-(2-trimethylsilanyl-äthoxymethoxy)-naphthalin-2-ylmethoxy]-piperidin-1-carbonsäure tert-butylester als hellgelbes Öl; MS: 728 $(M+H)^+$.

(b) 6.72 g (9.23 g) (3RS,4RS)-4-[4-(3-Benzyloxy-propoxy)-phenyl]-3-[7-(2-trimethylsilanyl-äthoxymethoxy)-naphthalin-2-ylmethoxy]-piperidin-1-carbonsäure tert-butylester wurden in 140 ml Methanol abs. bei 0° C vorgelegt, dann bei max. 5° C 2.8 ml (19.4 mMol) Salzsäure in Methanol (7.0 molar) zugetropft und danach auf Raumtemperatur aufgewärmt. Nach 90 Minuten wurde das Reaktionsgemisch auf eiskalte Natriumhydrogen-carbonat-Lösung gegossen und das Produkt 3 mal mit Methylenchlorid extrahiert, die organischen Phasen wurden 1 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert.

Dabei erhielt man 4.92 g (89% d. Th.) (3RS,4RS)-4-[4-(3-Benzyloxy-propoxy)-phenyl]-3-(7-hydroxy-naphthalin-2-ylmethoxy)-piperidin-1-carbonsäure tert-butylester als gelbes Öl; MS: 598 $(M+H)^+$.

(c) In analoger Weise wie in Beispiel 22 (1) beschrieben, wurde aus dem (3RS,4RS)-4-[4-(3-Benzyloxy-propoxy)-phenyl]-3-(7-hydroxy-naphthalin-2-ylmethoxy)-piperidin-1-carbonsäure tert-butylester durch Abspaltung der BOC Schutzgruppe mit Chlorwasserstoff in Methanol das 7-[(3RS,4RS)-4-[4-(3-Benzyloxy-propoxy)-phenyl]-piperidin-3-yloxymethyl]-naphthalin-2-ol Hydrochlorid (1:1) als amorpher, beiger Schaum erhalten; MS: 498 $(M+H)^+$.

Beispiel 96

(a) 0.33 g (0.54 mMol) (3RS,4RS)-4-[4-(3-Benzyloxy-propoxy)-phenyl]-3-(7-hydroxy-naphthalin-2-ylmethoxy)-piperidin-1-carbonsäure tert-butylester [Beispiel 95 (b)] und 0.27 g (0.7 mMol) 2,3;4,5-Di-Oisopropyliden-1-O-(4-methyl-phenylsulfonyl)-D-arabinitol [Liebigs Ann. Chem. 1992, 1131] wurden in 15 ml Dimethylformamid unter Zusatz von 0.69 g (5 mMol) Kaliumcarbonat (wasserfrei) während 2 Stunden bei Rückfluss unter Argon gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.34 g (78% d. Th.) eines 1:1 Gemisch des (3R, 4R)-und (35,45)-4-[4-(3-Benzyloxy-propoxy)-phenyl]-3-[7-[(4S,4'R,SR)-2,2,2',2'tetramethyl-[4,4']bi[[1,3]dioxolan-5-ylmethoxy]-naphthalin-2-ylmethoxy]-piperidin-1-carbonsäure tert-butylesters als farbloses Öl; MS: 829 $(M+NH4)^+$.

(b) 0.10 g (0.12 mMol) des 1:1 Gemisches des (3R,4R)- und (3S,4S)-4-[4-(3-Benzyloxy-propoxy)-phenyl]-3-[7-[(4S,4'R,5R)-2,2,2',2'-tetramethyl-[4,4']bi[[1,3] dioxolan-5-ylmethoxy]-naphthalin-2-ylmethoxy]-piperidin-1-carbonsäure tert-butylesters wurden in 5 ml Aethanol abs. gelöst, dazu 1 ml Salzsäure in Aethanol (5.6 molar) zugegeben und während 90 Stunden bei 50° C unter Argon gerührt. Nach dem Abdestillieren des Lösungsmittels im Wasserstrahlvakuum wurde der Rückstand während 3 Stunden im Hochvakuum bei 50° C über Phosphorpentoxid getrocknet. Dabei erhielt man 0.07 g (87% d. Th.) eines 1:1 Gemisches des (2R,3R,4R)-5-[7-[(3R,4R)- und (3S,4S)-4-[4-(3-Benzyloxy-propoxy)-phenyl]-piperidin-3-yloxymethyl]-naphthalin-2-yloxy]-pentan-1,2,3,4-tetraol Hydrochlorides (1:1) in Form hellgelber Kristalle; MS: 632 $(M+H)^+$.

Beispiel 97

(a) 4.77 g (10.8 mMol) (3RS,4RS)-4-[4-(3-Benzyloxy-propoxy)-phenyl]-3-hydroxy-piperidin-1-carbonsäure tert-butylester [Beispiel 86 (n)] und 3.49 g (10.8 mMol) 2-Chlormethyl-6-(2-trimethylsilanyl-äthoxymethoxy)-naphthalin [Beispiel 6 (o)] wurden in 35 ml Dimethylformamid unter Argon bei Raumtemperatur gelöst, dann 0.50 g (11.5 mMol) Natriumhydrid-Dispersion (55% in Mineralöl) zugegeben und während 18 Stunden bei Raumtemperatur gerührt. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Methylenchlorid chromatographiert. Dabei erhielt man 6.74 g (83% d. Th.) (3RS,4RS)-4-[4-(3-Benzyloxy-propoxy)-phenyl]-3-[6-(2-trimethylsilanyl-äthoxymethoxy)-naphthalin-2-ylmethoxy]-piperidin-1-carbonsäure tert-butylester als hellgelbes Öl; MS: 728 $(M+H)^+$.

(b) In analoger Weise wie in Beispiel 95 (b) beschrieben, wurde aus dem (3RS,4RS)-4-[4-(3-Benzyloxy-propoxy)-phenyl]-3-[6-(2-trimethylsilanyl-äthoxymethoxy)-naphthalin-2-ylmethoxy] -piperidin-1-carbonsäure tert-butylester der (3RS,4RS)-4-[4-(3-Benzyloxy-propoxy)-phenyl]-3-(6-hydroxy-naphthalin-2-ylmethoxy)-piperidin-1-carbonsäure tert-butylester als amorpher, farbloser Schaum erhalten; MS: 598 $(M+H)^+$.

(c) In analoger Weise wie in Beispiel 90 (g) beschrieben, wurde aus dem (3RS,4RS)-4-[4-(3-Benzyloxy-propoxy)-phenyl]-3-(6-hydroxy-naphthalin-2-ylmethoxy)-piperidin-1-carbonsäure tert-butylester der (3RS,4RS)-4-[4-(3-Benzyloxy-propoxy)-phenyl]-3-[6-(2-morpholin-4-yl-äthoxy)-naphthalin-2-ylmethoxy]-piperidin-1-carbonsäure tert-butylester als hellgelbes Öl erhalten; MS: 711 $(M+H)^+$.

(d) In analoger Weise wie in Beispiel 22 (l) beschrieben, wurde aus dem (3RS,4RS)-4-[4-(3-Benzyloxy-propoxy)-phenyl]-3-[6-(2-morpholin-4-yl-äthoxy)-naphthalin-2-ylmethoxy]-piperidin-1-carbonsäure tert-butylester durch Abspaltung der BOC Schutzgruppe mit Chlorwasserstoff in Methanol das 4-[2-[6-[(3RS,4RS)-4-[4-(3-Benzyloxypropoxy)-phenyl]-piperidin-3-yloxymethyl]-naphthalin-2-yloxy]-äthyl]-morpholin Hydrochlorid (1:2) in Form farbloser Kristalle erhalten; M: 611 $(M+H)^+$.

Beispiel 98

(a) 4.10 g (21.5 mMol) p-Toluolsulfochlorid wurden in 20 ml Pyridin abs. unter Argon bei 5° C vorgelegt, 0.06 g (0.5 mMol) 4-Dimethylaminopyridin zugegeben und eine Lösung von 3.58 g (20.3 mMol) (RS)-2-[(2,2-Dimethyl-1,3-dioxolan-4-yl)methoxy]-äthanol [J. Chem. Soc. 1965, 2968] in 20 ml Pyridin abs. unter Rühren zugetropft. Nach 6 Stunden Rühren bei Raumtemperatur wurde das Reaktionsgemisch auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 1.72 g (26% d. Th.) Toluol-4-sulfonsäure (RS)-2-(2,2-dimethyl-[1,3]dioxolan-4-ylmethoxy)-äthylester als farbloses Öl; MS: 315 (M-CH3).

(b) In analoger Weise wie in Beispiel 90 (g) beschrieben, wurde aus dem Toluol-4-sulfonsäure (RS)-2-(2,2-dimethyl- [1,3] dioxolan-4-ylmethoxy)-äthylester und dem (3RS,4RS)-4-[4-(3-Benzyloxy-propoxy)-phenyl]-3-(7-hydroxy-naphthalin-2-ylmethoxy)-piperidin-1-carbonsäure tert-butylester [Beispiel 95 (b)] ein Gemisch des2-dimethyl-[1,3]dioxolan-4-ylmethoxy]-äthoxy]-naphthalin-2-ylmethoxy]-piperidin-1-carbonsäure tert-butylesters in Form farbloser Kristalle erhalten; MS: 773 $(M+NH4)^+$.

(c) In analoger Weise wie in Beispiel 22 (l) beschrieben, wurde aus dem Gemisch des (3RS,4RS)- und (3SR,4SR)-4-[4-(3-Benzyloxy-propoxy)-phenyl]-3-[7-[2-[(RS)-2,2-dimethyl-[1,3]dioxolan-4-ylmethoxy]-äthoxy]-naphthalin-2-ylmethoxy]-piperidin-1-carbonsäure tert-butylesters durch Abspaltung der BOC Schutzgruppe mit Chlorwasserstoff in Methanol ein Gemisch des (RS)- und (SR)-3-[2-[7-[(3RS,4RS)-4-[4-(3-Benzyloxy-propoxy)-phenyl]-piperidin-3-yloxymethyl]-naphthalin-2-yloxy]-äthoxy]-propan-1,2-diol Hydrochlorides (1:1) als amorpher, beiger Schaum erhalten; MS: 616 $(M+H)^+$.

Beispiel 99

(a) 9.92 g (227.4 mMol) Natriumhydrid-Dispersion (55% in Mineralöl) wurden in 220 ml Tetrahydrofuran abs. unter Argon bei 5° C vorgelegt, dazu eine Lösung von 68.3 ml (341.1 mMol) Phosphonoessigsäuretriäthylester in 220 ml Tetrahydrofuran abs. während 1 Stunde bei 5° C zugetropft und anschliessend 1 Stunde bei Raumtemperatur gerührt. Nun wurde wiederum bei 5° C eine Lösung von 24.1 g (113.7 mMol) 4-Benzyloxy-benzaldehyd in 220 ml Tetrahydrofuran während 30 Minuten zugetropft, danach 2 Stunden bei 5° C gerührt. Das Reaktionsgemisch wurde mit 300 ml Eiswasser versetzt und das Lösungsmittel im Wasserstrahlvakuum abdestilliert; die wässrige Suspension vom Produkt wurde 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 30.8 g (96% d. Th.) (E)-3-(4-Benzyloxy-phenyl)-acrylsäure äthylester als farbloser Festkörper; MS: 282 $(M)^+$.

(b) 17.85 g (136.1 mMol) Malonsäure-monoethylester-monoamid wurden in 350 ml Aethanol abs. unter Argon mit 15.3 g (136.1 mMol) Kalium-tert-butylat versetzt, dann unter Rühren bei Raumtemperatur 19.2 g (68.1 mMol) (E)-3-(4-Benzyloxy-phenyl)-acrylsäure äthylester zugegeben und während 1 Stunde bei Rückfluss gerührt. Nach dem Abkühlen auf 10° C wurden 15.4 ml (269.7 mMol) Eisessig zugetropft. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 17.2 g (69% d. Th.) (3RS,4SR)-4-(4-Benzyloxy-phenyl)-2,6-dioxo-piperidin-3-carbonsäure äthylester als farblosen Festkörper; MS: 367 $(M)^+$.

(c) 4.33 g (114.2 mMol) Lithiumaluminiumhydrid wurden in 200 ml Tetrahydrofuran unter Argon suspendiert, dann bei Raumtemperatur eine Lösung von 18.31 g (49.8 mMol) (3RS,4SR)-4-(4-Benzyloxy-phenyl)-2,6-dioxo-piperidin-3-carbonsäure äthylester in 200 ml Tetrahydrofuran zugetropft und anschliessend während 2 Stunden bei Rückfluss gerührt. Zum Reaktionsgemisch wurden vorsichtig bei 5 - 10° C 100 ml destilliertes Wasser zugetropft und der dabei gebildete Niederschlag abfiltriert. Das Filtrat wurde hierauf 3 mal mit Essigester extrahiert, die organischen Phasen wurden 2 mal mit destilliertem Wasser gewaschen, über Magnesiumsulfat getrocknet, filtriert und im Wasserstrahlvakuum eingeengt. Das so erhaltene Rohprodukt wurde aus Methylenchlorid und n-Hexan umkristallisiert. Dabei erhielt man 11.14 g (75% d. Th.) (3RS,4SR)-[4-(4-Benzyloxy-phenyl)-piperidin-3-yl]-methanol in Form farbloser Kristalle; MS : 297 $(M)^+$.

(d) 11.14 g (37.5 mMol) (3RS,4SR)-[4-(4-Benzyloxy-phenyl)-piperidin-3-yl]-methanol wurden in 140 ml Dioxan unter Argon gelöst, dann bei Raumtemperatur eine Lösung von 6.72 g (80 mMol) Natriumhydrogencarbonat in 45 ml Wasser zugegeben und portionenweise 9.78 g (44.8 mMol) Di-tert-butyl-dicarbonat eingetragen. Nach 18 Stunden Rühren bei Raumtemperatur wurde das Reaktionsgemisch 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 13.38 g (90% d. Th.) (3RS,4SR)-4-(4-Benzyloxy-phenyl)-3-hydroxymethyl-piperidin-1-carbonsäure tert-butylester als amorphen, farblosen Schaum; MS: 398 $(M+H)^+$.

(e) 3.92 ml (45.6 mMol) Oxalylchlorid wurden in 400 ml Methylenchlorid unter Argon bei -70° C vorgelegt, dazu 5.48 ml (77.2 mMol) Dimethylsulfoxid zugetropft und während 30 Minuten gerührt. Nun wurde eine Lösung von 13.95 g (35.1 mMol) (3RS,4SR)-4-(4-Benzyloxyphenyl)-3-hydroxymethyl-piperidin-1-carbonsäure tert-butylester in 200 ml Methylenchlorid bei -70° C zugetropft und danach während 2 Stunden bei dieser Temperatur gerührt. Zum Reaktionsgemisch wurden anschliessend 12.2 ml (87.7 mMol) Triäthylamin zugetropft und danach auf Raumtemperatur erwärmt. Nach 18 Stunden Rühren bei dieser Temperatur wurde der Ansatz auf Eiswasser gegossen und das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde aus n-Hexan umkristallisiert. Dabei erhielt man 11.31 g (81% d. Th.) (3RS,4SR)-4-(4-Benzyloxy-phenyl)-3-formyl-piperidin-1-carbonsäure tert-butylester in Form farbloser Kristalle; MS: 395 $(M)^+$.

(f) 11.04 g (25.6 mMol) Tributyl-naphthalin-2-yl-stannan [Beispiel 74 (g)] wurden in 100 ml Tetrahydrofuran unter Argon bei -70° C vorgelegt, dazu 12.0 ml (19.2 mMol) n-Butyllithium-Lösung (1.6 molar in n-Hexan) zugetropft. Nach 30 Minuten Rühren bei dieser Temperatur wurde eine Lösung von 5.94 g (15 mMol) (3RS,4SR)-4-(4-Benzyloxy-phenyl)-3-formylpiperidin-1-carbonsäure tert-butylester in 45 ml Tetrahydrofuran zugetropft und nochmals 1 Stunde bei -70° C gerührt. Nun wurde auf Raumtemperatur erwärmt und nach 18 Stunden auf Eiswasser gegossen und das Produkt 3 mal mit Essigester extrahiert; die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. Dabei erhielt man 6.88 g (85% d. Th.) eines Gemisches des (3RS,4SR)-4-(4-Benzyloxy-phenyl)-3-[(RS)- und -[(SR)-1-hydroxy-2-naphthalin-2-yl-äthyl]-piperidin-1-carbonsäure tert-butylesters in Form gelber Kristalle; MS: 538 $(M+H)^+$.

(g) In analoger Weise wie in Beispiel 22 (1) beschrieben, wurde aus dem Gemisch des (3RS,4SR)-4-(4-Benzyloxy-phenyl)-3-[(RS)- und -[(SR)-1-1-hydroxy-2-naphthalin-2-yl-äthyl]-piperidin-1-carbonsäure tert-butylesters durch Abspaltung der BOC Schutzgruppe mit Chlorwasserstoff in Methanol ein Gemisch des (RS)- und (SR)-1-[(3RS,4SR)-4-(4-Benzyloxy-phenyl)-piperidin-3-yl]-2-naphthalin-2-yl-äthanol Hydrochlorides (1:1) in Form beiger Kristalle erhalten; MS: 438 $(M+H)^+$.

Beispiel 100

(a) 6.36 g (11.8 mMol) des Gemisches des (3RS,4SR)-4-(4-Benzyloxyphenyl)-3-[(RS)- und [(SR)-1-hydroxy-2-naphthalin-2-yl-äthyl]-piperidin-1-carbonsäure tert-butylesters [Beispiel 99 (f)] wurden in 50 ml Methanol unter Zusatz von 2.0 g Palladiumkohle (10%) während 4 Stunden unter Normalbedingungen hydriert. Nach Filtration des Katalysators durch ein Dicalitpolster und dem Abdestillieren des Lösungsmittels im Wasserstrahlvakuum wurden 4.97 g (94% d. Th.) eines Gemisch des (3RS,4SR)-4-(4-Hydroxy-phenyl)-3-[(RS)-und-(SR)-1-hydroxy-2-naphthalin-2-yl-äthyl] -piperidin-1-carbonsäure tert-butylesters als amorpher, hellgrauer Schaum erhalten; MS: 448 $(M+H)^+$.

(b) 3.97 g (8.9 mMol) des Gemisch des (3RS,4SR)-4-(4-Hydroxy-phenyl)-3-[(RS)-und-(SR)-1-hydroxy-2-naphthalin-2-yl-äthyl]-piperidin-1-carbonsäure tert-butylesters wurden in 60 ml Methyläthylketon unter Argon bei Raumtemperatur vorgelegt, dann wurden 4.90 g (35.5 mMol) Kaliumcarbonat (wasserfrei) und 4.54 ml (27.7 mMol) Benzyl-3-brompropyläther zugegeben und danach während 8 Stunden bei Rückfluss gerührt. Nach dem Abkühlen auf Raumtemperatur wurde das Reaktionsgemisch auf Eiswasser gegossen und das Produkt 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 5.13 g (97% d. Th.) eines Gemisch des (3RS, 4SR)-4-[4-(3-Benzyloxy-propoxy)-phenyl]-3-[(RS)-und -[(SR)1-hydroxy-2-naphthalin-2-yl-äthyl]-piperidin-1-carbonsäure tert-butylesters als amorphen, gelben Schaum; MS: 596 $(M+H)^+$.

(c) In analoger Weise wie in Beispiel 99 (e) beschrieben, wurde aus dem Gemisch des (3RS,4SR)-4-[4-(3-Benzyloxy-propoxy)-phenyl]-3-[(RS)- und - [(SR)1-hydroxy-2-naphthalin-2-yl-äthyl]-piperidin-1-carbonsäure tert-butylesters mit Oxalylchlorid und Dimethylsulfoxid (3RS,4SR)-4-[4-(3-Benzyloxy-propoxy)-phenyl]-3-(naphthalin-2-yl-acetyl)-piperidin-1-carbonsäure tert-butylester als farbloses Öl erhalten; MS: 594 $(M+H)^+$.

(d) In analoger Weise wie in Beispiel 22 (l) beschrieben, wurde aus dem (3RS,4SR)-4-[4-(3-Benzyloxy-propoxy)-phenyl]-3-(naphthalin-2-yl-acetyl)-piperidin-1-carbonsäure tert-butylester durch Abspaltung der BOC Schutzgruppe mit Chlorwasserstoff in Methanol das 1-[(3RS,4SR)-4-[4-(3-Benzyloxy-propoxy)-phenyl]-piperidin-3-yl]-2-naphthalin-2-yl-äthanon Hydrochlorid (1:1) in Form farbloser Kristalle erhalten; MS: 494 $(M+H)^+$.

Beispiel 101

(a) 10.66 g (50.2 mMol) 4-Benzyloxy-benzaldehyd und 8.54 ml (56.2 mMol) Malonsäure-diäthylester wurden in 100 ml Toluol unter Zusatz von 10.15 g Molekularsieb (4Å), 1.0 ml (10.0 mMol) Piperidin und 1.0 ml (17.6 mMol) Eisessig während 18 Stunden unter Argon bei Rückfluss gerührt. Nach Filtration des Reaktionsgemisches wurde das Lösungsmittel im Wasserstrahlvakuum abdestilliert und der Rückstand an Kieselgel mit n-Hexan und Essigester chromatographiert. So erhielt man 14.05 g (83% d. Th.) 2-(4-Benzyloxy-benzyliden)-malonsäure diäthylester in Form gelber Kristalle; MS: 354 $(M)^+$.

(b) 6.42 g (48.9 mMol) Malonsäure-monoäthylester-monoamid wurden in 115 ml Aethanol abs. unter Argon mit 5.49 g (48.9 mMol) Kalium-tert-butylat versetzt, dann unter Rühren bei Raumtemperatur 17.35 g (48.9 mMol) 2-(4-Benzyloxy-benzyliden)-malonsäure diäthylester zugegeben und während 2 Stunden bei Rückfluss gerührt. Nach dem Abkühlen auf 10° C wurden 13.0 ml (227 mMol) Eisessig zugetropft. Das Reaktionsgemisch wurde auf Eiswasser gegossen, das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahl-vakuum eingeengt. Das so erhaltene Rohprodukt wurde aus Methylenchlorid und n-Hexan umkristallisiert. Dabei erhielt man 16.76 g (78% d. Th.) (3R,4s,5S)-4-(4-Benzyloxy-phenyl)-2,6-dioxo-piperidin-3,5-dicarbonsäure diäthylester als farbloser Festkörper; MS: 439 $(M)^+$.

(c) 3.83 g (100.9 mMol) Lithiumaluminiumhydrid wurden in 200 ml Tetrahydrofuran unter Argon suspendiert, dann bei Raumtemperatur eine Lösung von 18.37 g (41.8 mMol) (3R,4s,5S)-4-(4-Benzyloxy-phenyl)-2,6-dioxo-piperidin-3,5-dicarbonsäure diäthylester in 200 ml Tetrahydrofuran zugetropft und anschliessend während 1 Stunde bei Rückfluss gerührt. Zum Reaktionsgemisch wurden anschliessend vorsichtig bei 5 - 10° C 25 ml destilliertes Wasser zugetropft. Nach Filtration des Reaktionsgemisches wurde das Lösungsmittel im Wasserstrahlvakuum abdestilliert. Dabei erhielt man 11.04 g (81% d. Th.) (3R,4s,5S)-[4-(4-Benzyloxy-phenyl)-5-hydroxymethyl-piperidin-3-yl]-methanol in Form farbloser Kristalle; MS: 328 $(M+H)^+$.

(d) 8.10 g (24.7 mMol) (3R,4s,5S)-[4-(4-Benzyloxy-phenyl)-5-hydroxymethyl-piperidin-3-yl]-methanol wurden in 100 ml Dioxan unter Argon gelöst, dann bei Raumtemperatur eine Lösung von 4.44 g (52.8 mMol) Natriumhydrogencarbonat in 34 ml Wasser zugetropft und anschliessend portionenweise 6.46 g (29.6 mMol) Di-tert-butyldicarbonat eingetragen. Nach 66 Stunden Rühren bei Raumtemperatur wurde das Reaktionsgemisch 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 8.10 g (77% d. Th.) (3R,4s,5S)-4-(4-Benzyloxy-phenyl)-3,5-bis-hydroxymethyl-piperidin-1-carbonsäure tert-butylester als amorphen, farblosen

Schaum; MS: 428 (M+H)+.

(e) 7.46 g (17.5 mMol) (3R,4s,5S)-4-(4-Benzyloxy-phenyl)-3,5-bishydroxymethyl-piperidin-1-carbonsäure tert-butylester wurden in 250 ml Methanol unter Zusatz von 1.5 g Palladiumkohle (10%) während 2 Stunden bei Normalbedingungen hydriert. Der Katalysator wurde anschliesssend über ein Dicalit-Polster abfiltriert und das Lösungsmittel im Wasserstrahlvakuum abdestilliert. Das so erhaltene Rohprodukt wurde an Kieselgel mit Methylenchlorid und Methanol chromatographiert. Dabei erhielt man 6.07 g (99% d. Th.) (3R,4s,5S)-3,5-Bis-hydroxymethyl-4-(4-hydroxy-phenyl)-piperidin-1-carbonsäure tert-butylester als amorphen, farblosen Schaum; MS: 338 (M+H)+.

(f) 6.77 g (20 mMol) (3R,4s,5S)-3,5-Bis-hydroxymethyl-4-(4-hydroxyphenyl)-piperidin-1-carbonsäure tert-butylester wurden in 90 ml Methyläthylketon unter Argon bei Raumtemperatur vorgelegt. Hierauf wurden 11.05 g (80 mMol) Kaliumcarbonat (wasserfrei) und 10.25 ml (58 mMol) Benzyl-3-brom-propyläther hinzugefügt und danach während 18 Stunden bei Rückfluss gerührt. Nach dem Abkühlen auf Raumtemperatur wurde das Reaktionsgemisch auf Eiswasser gegossen und das Produkt 3 mal mit Essigester extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 7.95 g (82% d. Th.) (3R,4s,5S)-4-[4-(3-Benzyloxypropoxy)-phenyl]-3,5-bis-hydroxymethyl-piperidin-1-carbonsäure tert-butylester als amorphen, farblosen Schaum ; MS: 486 (M+H)+.

(g) 1.51 g (33.2 mMol) Natriumhydrid-Dispersion (55% in Mineralöl) und 2.94 g (15.9 mMol) 4-(2-Chloräthyl)-morpholin-hydrochlorid wurden unter Argon in 25 ml Dimethylformamid bei Raumtemperatur gelöst, dazu unter Rühren eine Lösung von 7.33 g (15.1 mMol) (3R,4s,5S)-4-[4-(3-Benzyloxy-propoxy)-phenyl]-3,5-bis-hydroxymethylpiperidin-1-carbonsäure tert-butylester in 50 ml Dimethylformamid zugetropft und 0.1 g (0.6 mMol) Kaliumiodid zugegeben. Das Reaktionsgemisch wurde während 9 Stunden bei 100° C gerührt. Nach dem Abkühlen auf Raumtemperatur wurde das Reaktionsgemisch auf Eiswasser gegossen und das Produkt 3 mal mit Methylenchlorid extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.29 g (3 % d. Th.) (3R,4s,5S)-4-[4-(3-Benzyloxy-propoxy)-phenyl]-3,5-bis-(2-morpholin-4-yl-äthoxymethyl)-piperidin-1-carbonsäure tert-butylester als hellbraunes Öl [MS: 712 (M+H)+] und 2.37 g (26% d. Th.) (3RS,4RS,5SR)-4-[4-(3-Benzyloxypropoxy)-phenyl]-3-hydroxymethyl-5-(2-morpholin4-yl-äthoxymethyl)-piperidin-1-carbonsäure tert-butylester als hellbraunes Öl; MS: 599 (M+H)+.

(h) In analoger Weise wie in Beispiel 74 (f) beschrieben, wurde aus dem (3RS,4RS,5SR)-4-[4-(3-Benzyloxy-propoxy)-phenyl]-3-hydroxymethyl-5-(2-morpholin4-yl-äthoxymethyl)-piperidin-1-carbonsäure tert-butylester durch Oxidation mit Dimethylsulfoxid/Oxalylchlorid in Methylenchlorid (3RS,4SR,5SR)-4-[4-(3-Benzyloxy-propoxy)-phenyl]-3-formyl-5-(2-morpholin-4-yl-äthoxymethyl)-piperidin-1-carbonsäure tert-butylester als gelbes Öl erhalten; MS: 597 (M+H)+.

(i) 1.20 g (0.05 g Atome) Magnesiumspäne wurden in 15 ml Äther abs. unter Argon bei Raumtemperatur vorgelegt, 1 Jodkristall und 5 Tropfen 1,2-Dibrommethan zugegeben und auf Rückfluss erwärmt. Nach dem Anspringen der Reaktion (Entfärbung) wurde während 30 Minuten eine Lösung von 1.77 g (10 mMol) 2-(Chlormethyl)-naphthalin in 10 ml Äther abs. zugetropft. Nach Ende der Zugabe liess man auf Raumtemperatur abkühlen und nach einer Stunde wurde eine Lösung von 0.70 g (1.17 mMol) (3RS,4SR,5SR)-4-[4-(3-Benzyloxy-propoxy)-phenyl]-3-formyl-5-(2-morpholin-4-yl-äthoxymethyl)-piperidin-1-carbonsäure tert-butylester in 15 ml Äther abs. zugetropft. Dann rührte man während 18 Stunden bei Raumtemperatur. Nach dem Zutropfen von 3 ml Wasser unter Eiskühlung wurde das Reaktionsgemisch auf Eiswasser gegossen, das Produkt 3 mal mit Äther extrahiert, die organischen Phasen wurden 2 mal mit destilliertem Wasser gewaschen, dann über Magnesiumsulfat getrocknet, filtriert und im Wasserstrahlvakuum eingeengt. Das so erhaltene Rohprodukt wurde an Kieselgel mit Methylenchlorid und Methanol chromatographiert. So erhielt man 0.77 g (89% d. Th.) eines Gemisches des (3RS,4SR,5SR)-4-[4-(3-Benzyloxy-propoxy)-phenyl]-3-[(RS)- und -[(SR)-1-hydroxy-2-naphthalin-2-yl-äthyl]-5-(2-morpholin-4-yl-äthoxymethyl)-piperidin-1-carbonsäure tert-butylesters; MS: 739 (M+H)+.

(k) In analoger Weise wie in Beispiel 74 (f) beschrieben, wurden aus 0.74 g (1 mMol) des Gemisches des (3RS,4SR,5SR)-4-[4-(3-Benzyloxypropoxy)-phenyl]-3-[(RS)- und -[(SR)-1-hydroxy-2-naphthalin-2-yl-äthyl]-5-(2-morpholin-4-yl-äthoxymethyl)-piperidin-1-carbonsäure tert-butylesters durch Oxidation mit Dimethylsulfoxid/Oxalylchlorid in Methylenchlorid 0.15 g (20% d. Th.) (3RS,4SR,5SR)-4-[4-(3-Benzyloxypropoxy)-phenyl]-5-(2-morpholin-4-yl-äthoxymethyl)-3-(naphthalin-2-ylacetyl)-piperidin-1-carbonsäure tert-butylester als hellgelbes Öl erhalten;

MS: 737 $(M+H)^+$.

(l) In analoger Weise wie in Beispiel 73 (d) beschrieben, wurde aus dem (3RS,4SR,5SR)-4-[4-(3-Benzyloxy-propoxy)-phenyl]-5-(2-morpholin-4-yl-äthoxymethyl)-3-(naphthalin-2-yl-acetyl)-piperidin-1-carbonsäure tert-butyl ester durch Abspaltung der BOC-Schutzgruppe mittels wasserfreiem Zinkbromid in Methylenchlorid das 1-[(3RS,4SR,5SR)-4-[4-(3-Benzyloxy-propoxy)-phenyl]-5-(2-morpholin-4-yl-äthoxymethyl)-piperidin-3-yl]-2-naphthalin-2-yl-äthanon als gelbes Öl erhalten; MS: 637 $(M+H)^+$.

Beispiel 102

(a) 0.30 g (0.5 mMol) (3RS,4SR)-4-[4-(3-Benzyloxy-propoxy)-phenyl]-3-(naphthalin-2-yl-acetyl)-piperidin-1-carbonsäure tert-butylester [Beispiel 100 (c)] und 0.071 g (0.5 mMol) 3-(Aminooxy)propionsäure hydrochlorid [J. Am. Chem. Soc. 77, 2345 (1955)] wurden in 3 ml Pyridin während 18 Stunden unter Argon bei 60° C gerührt. Das Reaktionsgemisch wurde hierauf auf Eiswasser gegossen, mit verdünnter Salzsäure auf pH 3 gestellt, dann das Produkt 3 mal mit Essigester extrahiert, die organischen Phasen wurden 2 mal mit . destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. Dabei erhielt man 0.018 g (5 % d. Th.) eines Gemisches des (E)- und (Z)-(3RS,4SR)-4-[4-(3-Benzyloxy-propoxy)-phenyl]-3-[1-(2-carboxy-äthoxyimino)-2-naphthalin-2-yl-äthyl]-piperidin-1-carbonsäure tert-butylesters als hellgelbes Öl [MS: 681 $(M+H)^+$] und 0.21 g (69 % d. Th.) eines Gemisch des (E)- und (Z)-(3RS,4SR)-4-[4-(3-Benzyloxy-propoxy)-phenyl]-3-(1-hydroxyimino-2-naphthalin-2-yl-äthyl)-piperidin-1-carbonsäure tert-butylesters als farbloses Öl; MS: 609 $(M+H)^+$.

(b) In analoger Weise wie in Beispiel 22 (1) beschrieben, wurde aus dem Gemisch des (E)- und (Z)-(3RS,4SR)-4-[4-(3-Benzyloxy-propoxy)-phenyl]-3-[1-(2-carboxy-äthoxyimino)-2-naphthalin-2-yl-äthyl]-piperidin-1-carbonsäure tert-butylesters durch Abspaltung der BOC Schutzgruppe mit Chlorwasserstoff in Dioxan ein Gemisch der (E)- und (Z)-3-(1-[(3RS,4SR)-4-[4-(3-Benzyloxy-propoxy)-phenyl]-piperidin-3-yl]-2-naphthalin-2-yl-äthylidenaminooxy)-propionsäure als amorpher, beiger Schaum erhalten [MS: 581 $(M+H)^+$]

und aus dem Gemisch des (E)- und (Z)-(3RS,4SR)-4-[4-(3-Benzyloxypropoxy)-phenyl]-3-(1-hydroxyimino-2-naphthalin-2-yl-äthyl)-piperidin-1-carbonsäure tert-butylesters durch Abspaltung der BOC Schutzgruppe mit Chlorwasserstoff in Methanol ein Gemisch des (E)-und (Z)-1-[(3RS,4SR)-4-[4-(3-Benzyloxy-propoxy)-phenyl]-piperidin-3-yl]-2-naphthalin-2-yl-äthanon oxims als farbloser Festkörper; MS: 509 $(M+H)^+$.

Beispiel 103

(a) 0.15 g (0.22 mMol) des Gemisches des (E)- und (Z)-(3RS,4SR)-4-[4-(3-Benzyloxy-propoxy)-phenyl]-3-(1-methoxycarbonyl-methoxyimino-2-naphthalin-2-yl-äthyl)-piperidin-1-carbonsäure tert-butylesters [Beispiel 90 (p)] wurden in 10 ml Methanol gelöst, mit 1.3 ml 3.1 molarer Salzsäure in Methanol (4 mMol) versetzt und das Reaktionsgemisch während 7 Stunden bei Raumtemperatur gerührt. Nach Zugabe von 1 ml (9 mMol) Natronlauge (28%) bei 5° C wurde das Reaktionsgemisch während 18 Stunden bei Raumtemperatur gerührt. Durch Zutropfen von 0.7 ml (8.75 mMol) Salzsäure (37%) wurde der pH-Wert der Reaktionslösung auf 1 gestellt, filtriert und das Lösungsmittel im Hochvakuum abdestilliert. Der Rückstand wurde in 5 ml Aethanol abs. suspendiert, filtriert und das Filtrat eingeengt. Das so erhaltene Rohprodukt wurde an Kieselgel mit Methylenchlorid, Methanol und Ammoniak-Lösung (25%) chromatographiert. Dabei erhielt man 0.059 g (47% d. Th.) eines Gemisches der (E)- und (Z)-(1-[(3RS,4SR)-4-[4-(3-Benzyloxy-propoxy)-phenyl]-piperidin-3-yl]-2-naphthalin-2-yl-äthylidenaminooxy)-essigsäure als amorphen, beigen Schaum; MS: 567 $(M+H)^+$.

Beispiel 104

Die folgenden Verbindungen wurden erhalten, indem, analog Beispiel 1 (g), durch Alkylierung des (3RS,4RS)-4-[4-(3-Benzyloxy-propoxy)-phenyl]-3-hydroxy-piperidin-1-carbonsäure tert-butylesters [Beispiel 86 (n)] die entsprechenden BOC-Derivate synthetisiert wurden, die ohne weitere Reinigung und Charakterisierung in die Abspaltungsreaktion der BOC-Gruppe mittels Chlorwasserstoff in Methanol, analog Beispiel 22 (l), oder mittels Zinkbromid in Methylenchlorid, analog Beispiel 10 (b), eingesetzt wurden:

1) - Durch Alkylierung mit 4-Trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(4-trifluormethylbenzyloxy)-piperidin Hydrochlorid als farbloses Öl; MS : 500 $(M+H)^+$;

2) - durch Alkylierung mit 4-Fluor-benzylbromid und Abspaltung der BOC-Gruppe mittels Chlorwasserstoff in Methanol das (3RS,4RS)-4-[4-(3-Benzyloxy-propoxy)-phenyl]-3-(4-fluor-benzyloxy)-piperidin Hydrochlorid als farbloses Öl; MS : 450 $(M+H)^+$;

3) - durch Alkylierung mit 2-Chlor-benzylbromid und Abspaltung der BOC-Gruppe mittels Chlorwasserstoff in Methanol das (3RS,4RS)-4-[4-(3-Benzyloxy-propoxy)-phenyl]-3-(2-chlor-benzyloxy)-piperidin Hydrochlorid als farbloses Öl; MS : 466 $(M)^+$;

4) - durch Alkylierung mit 4-Brom-benzylbromid und Abspaltung der BOC-Gruppe mittels Chlorwasserstoff in Methanol das (3RS,4RS)-4-[4-(3-Benzyloxy-propoxy)-phenyl]-3-(4-brom-benzyloxy)-piperidin Hydrochlorid als farbloses Öl; MS : 510 $(M)^+$;

5) - durch Alkylierung mit 3-Brom-benzylbromid und Abspaltung der BOC-Gruppe mittels Chlorwasserstoff in Methanol das (3RS,4RS)-4-[4-(3-Benzyloxy-propoxy)-phenyl]-3-(3-brom-benzyloxy)-piperidin Hydrochlorid als farbloses Öl; MS: 511 $(M+H)^+$;

6) - durch Alkylierung mit 4-Iod-benzylbromid und Abspaltung der BOC-Gruppe mittels Chlorwasserstoff in Methanol das (3RS,4RS)-4-[4-(3-Benzyloxy-propoxy)-phenyl]-3-(4-iod-benzyloxy)-piperidin Hydrochlorid als farbloses Öl; MS : 558 $(M+H)^+$;

7) - durch Alkylierung mit 2-Trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(2-trifluormethylbenzyloxy)-piperidin Hydrochlorid als farbloses Öl; MS : 500 $(M+H)^+$;

8) - durch Alkylierung mit 3,5-Dimethyl-benzylbromid und Abspaltung der BOC-Gruppe mittels Chlorwasserstoff in Methanol das (3RS,4RS)-4-[4-(3-Benzyloxy-propoxy)-phenyl]-3-(3,5-dimethyl-benzyloxy)-piperidin Hydrochlorid als farbloses Öl; MS : 460 $(M+H)^+$;

9) - durch Alkylierung mit 2,4-Dimethyl-benzylchlorid und Abspaltung der BOC-Gruppe mittels Chlorwasserstoff in Methanol das (3RS,4RS)-4-[4-(3-Benzyloxy-propoxy)-phenyl]-3-(2,4-dimethyl-benzyloxy)-piperidin Hydrochlorid als farbloses Öl; MS : 460 $(M+H)^+$;

10) - durch Alkylierung mit 4-Methyl-benzylbromid und Abspaltung der BOC-Gruppe mittels Chlorwasserstoff in Methanol das (3RS,4RS)-4-[4-(3-Benzyloxy-propoxy)-phenyl]-3-(4-methyl-benzyloxy)-piperidin Hydrochlorid als farbloses Öl; MS : 446 $(M+H)^+$;

11) - durch Alkylierung mit 4-Isopropyl-benzylchlorid und Abspaltung der BOC-Gruppe mittels Chlorwasserstoff in Methanol das (3RS,4RS)-4-[4-(3-Benzyloxy-propoxy)-phenyl]-3-(4-isopropyl-benzyloxy)-piperidin Hydrochlorid als farbloses Öl; MS : 474 $(M+H)^+$;

12) - durch Alkylierung mit 4-tert-Butyl-benzylbromid und Abspaltung der BOC-Gruppe mittels Chlorwasserstoff in Methanol das (3RS,4RS)-4-[4-(3-Benzyloxy-propoxy)-phenyl]-3-(4-tert-butyl-benzyloxy)-piperidin Hydrochlorid als farbloses Öl; MS : 488 $(M+H)^+$;

13) - durch Alkylierung mit 2-Methoxy-benzylchlorid und Abspaltung der BOC-Gruppe mittels Chlorwasserstoff in Methanol das (3RS,4RS)-4-[4-(3-Benzyloxy-propoxy)-phenyl]-3-(2-methoxy-benzyloxy)-piperidin Hydrochlorid als farbloses Öl; MS : 462 $(M+H)^+$;

14) - durch Alkylierung mit 2-Fluor-benzylbromid und Abspaltung der BOC-Gruppe mittels Chlorwasserstoff in Methanol das (3RS,4RS)-4-[4-(3-Benzyloxy-propoxy)-phenyl]-3-(2-fluor-benzyloxy)-piperidin Hydrochlorid als farbloses Öl; MS : 450 $(M+H)^+$;

15) - durch Alkylierung mit 2-Fluor-6-trifluormethyl-benzylbromid und Abspaltung der BOC-Gruppe mittels Chlorwasserstoff in Methanol das (3RS,4RS)-4-[4-(3-Benzyloxy-propoxy)-phenyl] -3-(2-fluor-6-trifluormethyl-benzyloxy)-piperidin hydrochlorid als farbloses Öl; MS : 518 $(M+H)^+$;

16) - durch Alkylierung mit 2-Brom-5-fluor-benzylbromid und Abspaltung der BOC-Gruppe mittels Chlorwasserstoff in Methanol das (3RS,4RS)-4-[4-(3-Benzyloxy-propoxy)-phenyl]-3-(2-brom-5-fluorbenzyloxy)-piperidin Hydrochlorid als farbloses Öl; MS : 528 $(M)^+$;

17) - durch Alkylierung mit 4-Fluor-3-trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(4-fluor-3-trifluormethyl-benzyloxy)-piperidin Hydrochlorid als farbloses Öl; MS : 518 $(M+H)^+$;

18) - durch Alkylierung mit 3,5-Di-trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(3,5-bis-trifluormethylbenzyloxy)-piperidin Hydrochlorid als farbloses Öl; MS : 568 $(M+H)^+$;

19) - durch Alkylierung mit 2-Fluor-3-methyl-benzylbromid und Abspaltung der BOC-Gruppe mittels Chlorwasserstoff in Methanol das (3RS,4RS)-4-[4-(3-Benzyloxy-propoxy)-phenyl]-3-(2-fluor-3-methylbenzyloxy)-piperidin Hydrochlorid als farbloses Öl; MS : 464 $(M+H)^+$;

20) - durch Alkylierung mit 2-Fluor-4-trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(2-fluor-4-trifluormethyl-benzyloxy)-piperidin Hydrochlorid als gelbliches Öl; MS : 518 $(M+H)^+$;

21) - durch Alkylierung mit 2-Fluor-5-trifluormethyl-benzylbromid und Abspaltung der BOC-Gruppe mittels Chlorwasserstoff in Methanol das (3RS,4RS)-4-[4-(3-Benzyloxy-propoxy)-phenyl)-3-(2-fluor-5-trifluormethyl-benzyloxy)-piperidin Hydrochlorid als gelbliches Öl; MS : 518 $(M+H)^+$;

22) - durch Alkylierung mit 4-Fluor-2-trifluormethyl-benzylbromid und Abspaltung der BOC-Gruppe mittels Chlorwasserstoff in Methanol das (3RS,4RS)-4-[4-(3-Benzyloxy-propoxy)-phenyl]-3-(4-fluor-2-trifluormethyl-benzyloxy)-piperidin Hydrochlorid als farbloses Öl; MS : 518 $(M+H)^+$;

23) - durch Alkylierung mit 3,5-Dichlor-benzylchlorid und Abspaltung der BOC-Gruppe mittels Chlorwasserstoff in Methanol das (3RS,4RS)-4-[4-(3-Benzyloxy-propoxy)-phenyl]-3-(3,5-dichlor-benzyloxy)-piperidin Hydrochlorid als farbloses Öl; MS: 500 $(M)^+$;

24) - durch Alkylierung mit 2,4-Dichlor-benzylchlorid und Abspaltung der BOC-Gruppe mittels Chlorwasserstoff in Methanol das (3RS,4RS)-4-[4-(3-Benzyloxy-propoxy)-phenyl]-3-(2,4-dichlor-benzyloxy)-piperidin Hydrochlorid als farbloses Öl; MS : 500 $(M)^+$;

25) - durch Alkylierung mit 2-Brom-benzylchlorid und Abspaltung der BOC-Gruppe mittels Chlorwasserstoff in Methanol das (3RS,4RS)-4-[4-(3-Benzyloxy-propoxy)-phenyl]-3-(2-brom-benzyloxy)-piperidin Hydrochlorid als farbloses Öl; MS : 510 $(M)^+$;

26) - durch Alkylierung mit 2,6-Dichlor-benzylchlorid und Abspaltung der BOC-Gruppe mittels Chlorwasserstoff in Methanol das (3RS,4RS)-4-[4-(3-Benzyloxy-propoxy)-phenyl]-3-(2,6-dichlor-benzyloxy)-piperidin Hydrochlorid als farbloses Öl; MS : 500 $(M+H)^+$;

27) - durch Alkylierung mit 3-Fluor-benzylchlorid und Abspaltung der BOC-Gruppe mittels Chlorwasserstoff in Methanol das (3RS,4RS)-4-[4-(3-Benzyloxy-propoxy)-phenyl]-3-(3-fluor-benzyloxy)-piperidin Hydrochlorid als farbloses Öl; MS : 450 $(M+H)^+$;

28) - durch Alkylierung mit 6-Chlor-2-fluor-benzylchlorid und Abspaltung der BOC-Gruppe mittels Chlorwasserstoff in Methanol das (3RS,4RS)-4-[4-(3-Benzyloxy-propoxy)-phenyl]-3-(2-chlor-6-fluorbenzyloxy)-piperidin Hydrochlorid als farbloses Öl; MS : 484 $(M)^+$;

29) - durch Alkylierung mit 2-Iod-benzylchlorid und Abspaltung der BOC-Gruppe mittels Chlorwasserstoff in Methanol das (3RS,4RS)-4-[4-(3-Benzyloxy-propoxy)-phenyl]-3-(2-iod-benzyloxy)-piperidin Hydrochlorid als farbloses Öl; MS : 558 $(M+H)^+$;

30) - durch Alkylierung mit 3,4-Difluor-benzylbromid und Abspaltung der BOC-Gruppe mittels Chlorwasserstoff in Methanol das (3RS,4RS)-4-[4-(3-Benzyloxy-propoxy)-phenyl]-3-(3,4-difluor-benzyloxy)-piperidin Hydrochlorid als farbloses Öl; MS : 468 $(M+H)^+$;

31) - durch Alkylierung mit 2,3-Difluor-benzylbromid und Abspaltung der BOC-Gruppe mittels Chlorwasserstoff in Methanol das (3RS,4RS)-4-[4-(3-Benzyloxy-propoxy)-phenyl]-3-(2,3-difluor-benzyloxy)-piperidin Hydrochlorid als farbloses Öl; MS : 468 $(M+H)^+$;

32) - durch Alkylierung mit 2,5-Difluor-benzylbromid und Abspaltung der BOC-Gruppe mittels Chlorwasserstoff in Methanol das (3RS,4RS)-4-[4-(3-Benzyloxy-propoxy)-phenyl)]-3-(2,5-difluor-benzyloxy)-piperidin Hydrochlorid als farbloses Öl; MS : 468 $(M+H)^+$;

33) - durch Alkylierung mit 2,6-Difluor-benzylchlorid und Abspaltung der BOC-Gruppe mittels Chlorwasserstoff in Methanol das (3RS,4RS)-4-[4-(3-Benzyloxy-propoxy)-phenyl]-3-(2,6-difluor-benzyloxy)-piperidin Hydrochlorid als farbloses Öl; MS : 468 $(M+H)^+$;

34) - durch Alkylierung mit 2,4-Difluor-benzylbromid und Abspaltung der BOC-Gruppe mittels Chlorwasserstoff in Methanol das (3RS,4RS)-4-[4-(3-Benzyloxy-propoxy)-phenyl]-3-(2,4-difluor-benzyloxy)-piperidin Hydrochlorid als farbloses Öl; MS : 468 $(M+H)^+$;

35) - durch Alkylierung mit 3,5-Difluor-benzylbromid und Abspaltung der BOC-Gruppe mittels Chlorwasserstoff in Methanol das (3RS,4RS)-4-[4-(3-Benzyloxy-propoxy)-phenyl]-3-(3,5-difluor-benzyloxy)-piperidin Hydrochlorid als farbloses Öl; MS : 468 $(M+H)^+$;

36) - durch Alkylierung mit 4-Brommethyl-benzoesäuremethylester, Verseifung des Methylesters während der wässerigen Aufarbeitung und anschliessende Abspaltung der BOC-Gruppe mittels Chlorwasserstoff in Methanol das 4-{(3RS,4RS)-4-[4-(3-Benzyloxypropoxy)-phenyl]-piperidin-3-yloxymethyl}-benzoesäure Hydrochlorid als farbloses Öl; MS : 476 $(M+H)^+$;

37) - durch Alkylierung mit 1-Brommethyl-4-trifluormethoxy-benzol und Abspaltung der BOC-Gruppe mittels Chlorwasserstoff in Methanol das (3RS,4RS)-4-[4-(3-Benzyloxy-propoxy)-phenyl]-3-(4-trifluormethoxybenzyloxy)-piperidin Hydrochlorid als farbloses Öl; MS : 516 $(M+H)^+$;

38) - durch Alkylierung mit 3-Brommethyl-benzonitril und Abspaltung der BOC-Gruppe mittels Chlorwasserstoff in Methanol das 3-[(3RS,4RS)-4-[4-(3-Benzyloxy-propoxy)-phenyl]-piperidin-3-yloxymethyl)-benzonitril Hydrochlorid als farbloses Öl; MS : 457 $(M+H)^+$;

39) - durch Alkylierung mit 4-Brom-2-fluor-benzylbromid und Abspaltung der BOC-Gruppe mittels Chlorwasserstoff in Methanol das (3RS,4RS)-4-[4-(3-Benzyloxy-propoxy)-phenyl]-3-(4-brom-2-fluorbenzyloxy)-piperidin Hydrochlorid als farbloses Öl; MS : 529 $(M+H)^+$;

40) - durch Alkylierung mit 3-Chlor-benzylbromid und Abspaltung der BOC-Gruppe mittels Chlorwasserstoff in Methanol das (3RS,4RS)-4-[4-(3-Benzyloxy-propoxy)-phenyl]-3-(3-chlor-benzyloxy)-piperidin Hydrochlorid als farbloses Öl; MS : 466 $(M+H)^+$;

41) - durch Alkylierung mit 3-Chlor-2-fluor-benzylbromid und Abspaltung der BOC-Gruppe mittels Chlorwasserstoff in Methanol das (3RS,4RS)-4-[4-(3-Benzyloxy-propoxy)-phenyl]-3-(3-chlor-2-fluorbenzyloxy)-piperidin Hydrochlorid als farbloses Öl; MS: 484 $(M+H)^+$;

42) - durch Alkylierung mit 3,5-Dibrom-benzylbromid und Abspaltung der BOC-Gruppe mittels Chlorwasserstoff in Methanol das (3RS,4RS)-4-[4-(3-Benzyloxy-propoxy)-phenyl]-3-(3,5-dibrom-benzyloxy)-piperidin Hydrochlorid als farbloses Öl; MS : 590 $(M+H)^+$;

43) - durch Alkylierung mit 2,5-Dimethoxy-benzylchlorid und Abspaltung der BOC-Gruppe mittels Chlorwasserstoff in Methanol das (3RS,4RS)-4-[4-(3-Benzyloxy-propoxy)-phenyl]-3-(2,5-dimethoxybenzyloxy)-piperidin Hydrochlorid als farbloses Öl; MS : 492 $(M+H)^+$;

44) - durch Alkylierung mit 2-Methy-benzylchlorid und Abspaltung der BOC-Gruppe mittels Chlorwasserstoff in

Methanol das (3RS,4RS)-4-[4-(3-Benzyloxy-propoxy)-phenyl]-3-(2-methyl-benzyloxy)-piperidin als farbloses Öl; MS : 446 $(M+H)^+$;

45) - durch Alkylierung mit 3-Brommethyl-pyridin und Abspaltung der BOC-Gruppe mittels Zinkbromid in Methylenchlorid das 3-[(3RS,4RS)-4-[4-(3-Benzyloxy-propoxy)-phenyl]-piperidin-3-yloxymethyl]-pyridin als farbloses Öl; $R_f$: 0.08 ($SiO_2$, Methylenchlorid:Methanol=98:2, extrahiert gegen 5 Vol-% gesättigten Ammoniak).

46) - durch Alkylierung mit 4-Methylthio-benzylchlorid [J.Org.Chem. (1988), 53(3), 561-569] und Abspaltung der BOC-Gruppe mittels Zinkbromid in Methylenchlorid das (3RS,4RS)-4-[4-(3-Benzyloxypropoxy)-phenyl]-3-(4-methylsulfanyl-benzyloxy)-piperidin Hydrochlorid als farbloses Öl; MS : 478 $(M+H)^+$;

47) - durch Alkylierung mit 5-Chlormethyl-benzo[1.3]dioxol und Abspaltung der BOC-Gruppe mittels Zinkbromid in Methylenchlorid das (3RS,4RS)-3-(Benzo[1,3]dioxol-5-ylmethoxy)-4- [4-(3-benzyloxypropoxy)-phenyl]-piperidin Hydrobromid als farbloses Öl; MS : 476 $(M+H)^+$;

48) - durch Alkylierung mit 4-Methoxy-benzylchlorid und Abspaltung der BOC-Gruppe mittels Zinkbromid in Methylenchlorid das (3RS,4RS)-4-[4-(3-Benzyloxy-propoxy)-phenyl]-3-(4-methoxy-benzyloxy)-piperidin als farbloses Öl; MS : 462 $(M+H)^+$;

49) - durch Alkylierung mit 3,4,5-Trimethoxy-benzylchlorid und Abspaltung der BOC-Gruppe mittels Zinkbromid in Methylenchlorid das (3RS,4RS)-4-(4-(3-Benzyloxy-propoxy)-phenyl]-3-(3,4,5-trimethoxybenzyloxy)-piperidin als farbloses Öl; MS : 522 $(M+H)^+$;

50) - durch Alkylierung mit 4-Methoxy-3-methyl-benzylchlorid und Abspaltung der BOC-Gruppe mittels Zinkbromid in Methylenchlorid das (3RS,4RS)-4-[4-(3-Benzyloxy-propoxy)-phenyl]-3-(4-methoxy-3-methyl-benzyloxy)-piperidin als farbloses Öl; MS : 476 $(M+H)^+$;

51) - durch Alkylierung mit 3,5-Dimethoxy-benzylchlorid und Abspaltung der BOC-Gruppe mittels Zinkbromid in Methylenchlorid das (3RS,4RS)-4-[4-(3-Benzyloxy-propoxy)-phenyl]-3-(3,5-dimethoxybenzyloxy)-piperidin als farbloses Öl; MS : 492 $(M+H)^+$;

52) - durch Alkylierung mit 2,3,5,6-Tetramethyl-benzylchlorid und Abspaltung der BOC-Gruppe mittels Chlorwasserstoff in Methanol das (3RS,4RS)-4-[4-(3-Benzyloxy-propoxy)-phenyl]-3-(2,3,5,6-tetramethylbenzyloxy)-piperidin Hydrochlorid als farbloses Öl; MS : 488 $(M+H)^+$;

53) - durch Alkylierung mit 3-Methyl-benzylbromid und Abspaltung der BOC-Gruppe mittels Chlorwasserstoff in Methanol das (3RS,4RS)-4-[4-(3-Benzyloxy-propoxy)-phenyl]-3-(3-methyl-benzyloxy)-piperidin Hydrochlorid als farbloses Öl; MS : 446 $(M+H)^+$;

54) - durch Alkylierung mit 4-Chlor-benzylchlorid und Abspaltung der BOC-Gruppe mittels Chlorwasserstoff in Methanol das (3RS,4RS)-4-[4-(3-Benzyloxy-propoxy)-phenyl]-3-(4-chlor-benzyloxy)-piperidin Hydrochlorid als farbloses Öl; MS : 466 $(M+H)^+$.

Beispiel 105

Die folgenden Verbindungen wurden erhalten, indem, analog Beispiel 12 (b), durch Umsetzung der entsprechenden Benzylbromide mit 3 Aequivaltenten der jeweiligen Alkoholate die entsprechenden BOC-Derivate synthetisiert wurden, die ohne weitere Reinigung und Charakterisierung in die Abspaltungsreaktion der BOC-Gruppe mittels Chlorwasserstoff in Methanol, analog Beispiel 22 (l), oder mittels Zinkbromid in Methylenchlorid, analog Beispiel 10 (b), eingesetzt wurden:

1) - Durch Umsetzung des (3RS,4RS)-4-[4-(3-Benzyloxy-propoxy)-phenyl]-3-(3-brommethyl-benzyloxy)-piperidin-1-carbonsäure tert-butylesters mit Äthanol und Abspaltung der BOC-Gruppe mittels Zinkbromid in Methylenchlorid das (3RS,4RS)-4-[4-(3-Benzyloxypropoxy)-phenyl]-3-(3-äthoxymethyl-benzyloxy)-piperidin, MS : 490 $(M+H)^+$, und das [3-[(3RS,4RS)-4-[4-(3-Benzyloxy-propoxy)-phenyl]-piperidin-3-yloxymethyl)-phenyl]-methanol, MS : 479 $(M+NH_4)^+$, jeweils als farbloses Öl;

2) - durch Umsetzung des (3RS,4RS)-4-[4-(3-Benzyloxy-propoxy)-phenyl]-3-(3-brommethyl-benzyloxy)-piperidin-1-carbonsäure tert-butylesters mit Cyclobutyl-methanol und Abspaltung der BOC-Gruppe mittels Chlorwasserstoff in Methanol das (3RS,4RS)-4-[4-(3-Benzyloxypropoxy)-phenyl]-3-(3-cyclobutylmethoxymethyl-benzyloxy)-piperidin als farbloses Öl; MS : 530 $(M+H)^+$;

3) - durch Umsetzung des (3RS,4RS)-4-[4-(3-Benzyloxy-propoxy)-phenyl)-3-(3-brommethyl-benzyloxy)-piperidin-1-carbonsäure tert-butylesters mit 3-Phenyl-propan-1-ol und Abspaltung der BOC-Gruppe mittels Chlorwasserstoff in Methanol das (3RS,4RS)-4-[4-(3-Benzyloxypropoxy)-phenyl]-3-[3-(3-phenyl-propoxymethyl)-benzyloxy]-piperidin als farbloses Öl; MS : 580 $(M+H)^+$;

4) - durch Umsetzung des (3RS,4RS)-4-[4-(3-Benzyloxy-propoxy)-phenyl]-3-(3-brommethyl-benzyloxy)-piperidin-1-carbonsäure tert-butylesters mit 3,3-Dimethyl-butan-1-ol und Abspaltung der BOC-Gruppe mittels Chlorwasserstoff in Methanol das (3RS,4RS)-4-[4-(3-Benzyloxy-propoxy)-phenyl]-3-[3-(3,3-dimethyl-butoxymethyl)-benzyloxy]-piperidin als farbloses Öl; MS : 546 $(M+H)^+$;

5) - durch Umsetzung des (3RS,4RS)-4-[4-(3-Benzyloxy-propoxy)-phenyl]-3-(3-brommethyl-benzyloxy)-piperidin-1-carbonsäure tert-butylesters mit Pyridin-3-yl-methanol und Abspaltung der BOC-Gruppe mittels Chlorwasserstoff in Methanol das 3-[3-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

6) - durch Umsetzung des (3RS,4RS)-4-[4-(3-Benzyloxy-propoxy)-phenyl]-3-(3-brommethyl-benzyloxy)-piperidin-1-carbonsäure tert-butylesters mit Pyridin-4-yl-methanol und Abspaltung der BOC-Gruppe mittels Chlorwasserstoff in Methanol das 4-[3-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

7) - durch Umsetzung des (3RS,4RS)-4-[4-(3-Benzyloxy-propoxy)-phenyl]-3-(3-brommethyl-benzyloxy)-piperidin-1-carbonsäure tert-butylesters mit 2-Pyridin-2-yl-äthanol und Abspaltung der BOC-Gruppe mittels Chlorwasserstoff in Methanol das 2-[2-[3-[(3RS,4RS)-4-[4-(3-Benzyloxy-propoxy)-phenyl]-piperidin-3-yloxymethyl]-benzyloxy]-äthyl]-pyridin als farbloses Öl; MS : 567 $(M+H)^+$;

8) - durch Umsetzung des (3RS,4RS)-4-[4-(3-Benzyloxy-propoxy)-phenyl]-3-(4-brommethyl-benzyloxy)-piperidin-1-carbonsäure tert-butylesters mit Cyclobutyl-methanol und Abspaltung der BOC-Gruppe mittels Chlorwasserstoff in Methanol das (3RS,4RS)-4-[4-(3-Benzyloxypropoxy)-phenyl]-3-(4-cyclobutylmethoxymethyl-benzyloxy)-piperidin als farbloses Öl; MS : 530 $(M+H)^+$;

9) - durch Umsetzung des (3RS,4RS)-4-[4-(3-Benzyloxy-propoxy)-phenyl]-3-(4-brommethyl-benzyloxy)-piperidin-1-carbonsäure tert-butylesters mit 3-Phenyl-propan-1-ol und Abspaltung der BOC-Gruppe mittels Chlorwasserstoff in Methanol das (3RS,4RS)-4-[4-(3-Benzyloxypropoxy)-phenyl]-3-[4-(3-phenyl-propoxymethyl)-benzyloxy]-piperidin als farbloses Öl; MS : 580 $(M+H)^+$;

10) - durch Umsetzung des (3RS,4RS)-4-[4-(3-Benzyloxy-propoxy)-phenyl]-3-(4-brommethyl-benzyloxy)-piperidin-1-carbonsäure tert-butylesters mit 3,3-Dimethyl-butan-1-ol und Abspaltung der BOC-Gruppe mittels Chlorwasserstoff in Methanol das (3RS,4RS)-4-[4-(3-Benzyloxy-propoxy)-phenyl]-3-[4-(3,3-dimethyl-butoxymethyl)-benzyloxy]-piperidin als farbloses Öl; MS : 546 $(M+H)^+$;

11) - durch Umsetzung des (3RS,4RS)-4-[4-(3-Benzyloxy-propoxy)-phenyl]-3-(4-brommethyl-benzyloxy)-piperidin-1-carbonsäure tert-butylesters mit Pyridin-3-yl-methanol und Abspaltung der BOC-Gruppe mittels Chlorwasserstoff in Methanol das 3-[4-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

12)-durchUmsetzungdes(3RS,4RS)-4-[4-(3-Benzyloxy-propoxy)-phenyl]-3-(4-brommethyl-benzyloxy)-piperidin-1-carbonsäure tert-butylesters mit Pyridin-4-yl-methanol und Abspaltung der BOC-Gruppe mittels Chlorwasserstoff in Methanol das 4-[4-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

13) - durch Umsetzung des (3RS,4RS)-4-[4-(3-Benzyloxy-propoxy)-phenyl]-3-(4-brommethyl-benzyloxy)-piperidin-1-carbonsäure tert-butylesters mit 2-Pyridin-2-yl-äthanol und Abspaltung der BOC-Gruppe mittels Chlorwasserstoff in Methanol das 2-[2-[4-[(3RS,4RS)-4-[4-(3-Benzyloxy-propoxy)-phenyl]-piperidin-3-yloxymethyl]-benzyloxy]-äthyl]-pyridin als farbloses Öl; MS : 567 $(M+H)^+$;

14) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit 3-Phenyl-propan-1-ol und Abspaltung der BOC-Gruppe mittels Chlorwasserstoff in Methanol das (3RS,4RS)-4-[4-(3-Benzyloxypropoxy)-phenyl]-3-[2-(3-phenyl-propoxymethyl)-benzyloxy]-piperidin als farbloses Öl; MS : 580 $(M+H)^+$;

15) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit 3,3-Dimethyl-butan-1-ol und Abspaltung der BOC-Gruppe mittels Chlorwasserstoff in Methanol das (3RS,4RS)-4-[4-(3-Benzyloxy-propoxy)-phenyl]-3-[2-(3,3-dimethyl-butoxymethyl)-benzyloxy]-piperidin als farbloses Öl; MS : 546 $(M+H)^+$;

16) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit Pyridin-3-yl-methanol und Abspaltung der BOC-Gruppe mittels Chlorwasserstoff in Methanol das 3-[2-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

17) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit Pyridin-4-yl-methanol und Abspaltung der BOC-Gruppe mittels Chlorwasserstoff in Methanol das 4-[2-[(3RS,4RS)-4-[4-(3-Benzyloxy-propoxy)-phenyl]-piperidin-3-yloxymethyl]-benzyloxymethyl]-pyridin als farbloses Öl; MS : 553 $(M+H)^+$;

18) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit 2-Pyridin-2-yl-äthanol und Abspaltung der BOC-Gruppe mittels Chlorwasserstoff in Methanol das 2-[2-[2-[(3RS,4RS)-4-[4-(3-Benzyloxy-propoxy)-phenyl]-piperidin-3-yloxymethyl]-benzyloxy]-äthyl]-pyridin als farbloses Öl; MS : 567 $(M+H)^+$.

19) - durch Umsetzung des (3RS,4RS)-4-[4-(3-Benzyloxy-propoxy)-phenyl]-3-(2-brommethyl-benzyloxy)-piperidin-1-carbonsäure tert-butylesters mit Cyclobutyl-methanol und Abspaltung der BOC-Gruppe mittels Chlorwasserstoff in Methanol das (3RS,4RS)-4-[4-(3-Benzyloxypropoxy)-phenyl)-3-(2-cyclobutylmethoxymethyl-benzyloxy)-piperidin als farbloses Öl; MS : 530 $(M+H)^+$.

Die als Ausgangsstoffe eingesetzten Benzylbromide wurden durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit dem entsprechenden Bis-brommethyl-benzol in analoger Weise wie in Beispiel 1 (g) beschrieben, jedoch unter Verwendung von 4 Aequivalenten Dibromid und durch vorsichtiges Hydrolysieren der Reaktionslösung mit eiskalter Natriumbicarbonat-Lösung, hergestellt:

(a) - mit 1,3-Bis-brommethyl-benzol der (3RS,4RS)-4-[4-(3-Benzyloxypropoxy)-phenyl]-3-(3-brommethyl-benzyloxy)-piperidin-1-carbonsäure tert-butylester als farbloses Harz; MS : 624, 626 $(M+H)^+$;

(b) - mit 1,4-Bis-brommethyl-benzol der (3RS,4RS)-4-[4-(3-Benzyloxypropoxy)-phenyl]-3-(4-brommethyl-benzyloxy)-piperidin-1-carbonsäure tert-butylester als gelbliches Öl; MS : 643, 645 $(M+NH_4)^+$;

(c) - mit 1,2-Bis-brommethyl-benzol der (3RS,4RS)-4-[4-(3-Benzyloxypropoxy)-phenyl]-3-(2-brommethyl-benzyloxy)-piperidin-1-carbonsäure tert-butylester als farbloses Öl; MS : 624, 626 $(M+H)^+$.

Beispiel 106

Die folgenden Verbindungen wurden erhalten, indem, analog Beispiel 1 (g), durch Umsetzung der entsprechenden Benzylbromide mit 3 Aequivaltenten der jeweiligen Alkoholate die entsprechenden BOC-Derivate synthetisiert wurden, die, soweit nicht anders bemerkt, ohne weitere Reinigung und Charakterisierung in die Abspaltungsreaktion der BOC-Gruppe mittels Chlorwasserstoff in Methanol, analog Beispiel 22 (1), eingesetzt wurden:

1) - Durch Umsetzung des (3RS,4RS)-4-[4-(3-Benzyloxy-propoxy)-phenyl]-3-(6-brommethyl-naphthalin-2-ylmethoxy)-piperidin-1-carbonsäure tert-butylesters mit (RS)-(2,2-Dimethyl-[1,3]dioxolan-4-yl)-methanol und anschliessend gleichzeitige Abspaltung der BOC- und der Dioxolan-Gruppe das Gemisch des (3RS,4RS)- und (3SR, 4SR)-4-[4-(3-Benzyloxy-propoxy)-phenyl]-3-[6-[(RS)-2,3-dihydroxy-propoxymethyl]-naphthalin-2-ylmethoxy]-piperidins als farbloses Öl; MS : 586 $(M+H)^+$.

2) - Durch Umsetzung des (3RS,4RS)-4-[4-(3-Benzyloxy-propoxy)-phenyl]-3-(7-brommethyl-naphthalin-2-ylmethoxy)-piperidin- 1-carbonsäure tert-butylesters mit (RS)-(2,2-Dimethyl-[1,3]dioxolan-4-yl)-methanol und anschliessend gleichzeitige Abspaltung der BOC- und der Dioxolan-Gruppe das Gemisch des (3RS,4RS)- und (3SR, 4SR)-4-[4-(3-Benzyloxy-propoxy)-phenyl]-3-[7-[(RS)-2,3-dihydroxy-propoxymethyl]-naphthalin-2-ylmethoxy]-piperidins als farbloses Öl; MS : 586 $(M+H)^+$.

3) - Durch Umsetzung des (3RS,4RS)-4-[4-(3-Benzyloxy-propoxy)-phenyl]-3-(7-brommethyl-naphthalin-2-ylmethoxy)-piperidin-1-carbonsäure tert-butylesters mit rac-2-[(Tetrahydro-2H-pyran-2-yl)oxy]-1-äthanol das Gemisch des (3RS,4RS)- und (3SR,4SR)-4-[4-(3-Benzyloxypropoxy)-phenyl]-3-{7-[2-[(RS)-tetrahydro-pyran-2-yloxy]-äthoxymethyl]-naphthalin-2-ylmethoxy}-piperidin-1-carbonsäure tert-butyl esters [farbloses Öl, MS:740 $(M+H)^+$] und der (3RS,4RS)-4-[4-(3-Benzyloxypropoxy)-phenyl]-3-(7-dimethylaminomethyl-naphthalin-2-ylmethoxy)-piperidin-1-carbonsäure tert-butyl ester [Nebenprodukt, farbloses Öl, , MS: 639 $(M)^+$]. Gleichzeitige Abspaltung der BOC- und der Tetrahydropyranyl-Gruppe des Hauptproduktes ergab das 2-(7-{(3RS,4RS)-4-[4-(3-Benzyloxy-propoxy)-phenyl]-piperidin-3-yloxymethyl}naphthalin-2-ylmethoxy)-äthanol als farbloses Öl; MS: 556 $(M+H)^+$. Abspaltung der BOC-Gruppe des Nebenproduktes das (7-{(3RS,4RS)-4-[4-(3-Benzyloxy-propoxy)-phenyl]-piperidin-3-yloxymethyl}-naphthalin-2-ylmethyl)-dimethyl-amin als farbloses Öl; MS: 539 $(M+H)^+$.

4) - Durch Umsetzung des Gemisches des (3RS,4RS)-4-[4-(3-Benzyloxy-propoxy)-phenyl]-3-(8-brommethyl-naphthalin-2-ylmethoxy)-piperidin-1-carbonsäure tert-butylesters und des (3RS,4RS)-4-[4-(3-Benzyloxy-propoxy)-phenyl]-3-(7-brommethyl-naphthalin-1-ylmethoxy)-piperidin-1-carbonsäure tert-butylesters mit (RS)-(2,2-Dimethyl-[1,3]dioxolan-4-yl)-methanol und anschliessend gleichzeitige Abspaltung der BOC- und der Dioxolan-Gruppe das Gemisch des (3RS,4RS)- und (3SR,4SR)-4-[4-(3-Benzyloxy-propoxy)-phenyl)-3-[8-[(RS)-2,3-dihydroxy-propoxymethyl]-naphthalin-2-ylmethoxy]-piperidins und des (3RS,4RS)- und (3SR,4SR)-4-[4-(3-Benzyloxy-propoxy)-phenyl]-3-[7-[(RS)-2,3-dihydroxy-propoxymethyl]-naphthalin-1-ylmethoxy]-piperidins als farbloses Öl; MS: 586 $(M+H)^+$.

Die als Ausgangsstoffe eingesetzten Naphthylmethylbromide wurden durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit dem entsprechenden Bis-brommethyl-naphthalin in analoger Weise wie in Beispiel 1 (g) beschrieben, jedoch unter Verwendung von 4 Äquivalenten Dibromid und durch vorsichtiges Hydrolysieren der Reaktionslösung mit eiskalter Natriumbicarbonat-Lösung, hergestellt:

(a) - mit 2,6-Bis-brommethyl-naphthalin [J.Chem.Soc. (1961), 3741-3748] der (3RS,4RS)-4-[4-(3-Benzyloxy-propoxy)-phenyl]-3-(6-brommethylnaphthalin-2-ylmethoxy)-piperidin-1-carbonsäure tert-butylester als farbloses Öl; MS : 675 $(M+H)^+$;

(b) - mit 2,7-Bis-brommethyl-naphthalin [J.Am.Chem.Soc. (1979), 101 (15), 4259-4267] der (3RS,4RS)-4-[4-(3-Benzyloxy-propoxy)-phenyl]-3-(7-brommethyl-naphthalin-2-ylmethoxy)-piperidin-1-carbonsäure tert-butylester als farbloses Öl; MS : 675 $(M+H)^+$;

(c) - mit 1,7-Bis-brommethyl-naphthalin [Chem. Ber. 91, 1981 (1958)] das Gemisch des (3RS,4RS)-4-[4-(3-Benzyloxy-propoxy)-phenyl]-3-(8-brommethyl-naphthalin-2-ylmethoxy)-piperidin-1-carbonsäure tert-butylesters und des (3RS,4RS)-4-[4-(3-Benzyloxy-propoxy)-phenyl]-3-(7-brommethyl-naphthalin-1-ylmethoxy)-piperidin-1-carbonsäure tert-butylesters als farbloses Öl; MS : 675 $(M+H)^+$.

Beispiel 107

Die folgenden Verbindungen wurden erhalten, indem, analog Beispiel 1 (g), durch Alkylierung des (3RS,4RS)-4-[4-(3-Hydroxy-propoxy)-phenyl]-3-(naphthalin-2-ylmethoxy)-piperidin-1-carbonsäure tert-butylesters [Beispiel 57 (c)] die entsprechenden BOC-Derivate synthetisiert wurden, die ohne weitere Reinigung und Charakterisierung in die Abspaltungsreaktion der BOC-Gruppe mittels Chlorwasserstoff in Methanol, analog Beispiel 22 (l), eingesetzt wurden:

1) - Durch Alkylierung mit 4-Fluor-benzylbromid und Abspaltung der BOC-Gruppe das (3RS,4RS)-4-{4-[3-(4-Fluor-benzyloxy)-propoxy]-phenyl}-3-(naphthalin-2-ylmethoxy)-piperidin Hydrochlorid als farbloses Öl; MS : 500 $(M+H)^+$;

2) - durch Alkylierung mit 2-Chlor-benzylbromid und Abspaltung der BOC-Gruppe das (3RS,4RS)-4-{4-[3-(2-Chlor-benzyloxy)-propoxy]-phenyl}-3-(naphthalin-2-ylmethoxy)-piperidin als farbloses Öl; MS : 516 $(M)^+$;

3) - durch Alkylierung mit 2,3,4,5,6-Pentafluor-benzylbromid und Abspaltung der BOC-Gruppe das (3RS,4RS)-3-(Naphthalin-2-ylmethoxy)-4-{4-[3-(2,3,4,5,6-pentafluor-benzyloxy)-propoxy]-phenyl}-piperidin als farbloses Öl; MS : 572 $(M+H)^+$;

4) - durch Alkylierung mit 4-Brom-benzylbromid und Abspaltung der BOC-Gruppe das (3RS,4RS)-4-{4-[3-(4-Brom-benzyloxy)-propoxy]-phenyl}-3-(naphthalin-2-ylmethoxy)-piperidin Hydrochlorid

5) - durch Alkylierung mit 3-Brom-benzylbromid und Abspaltung der BOC-Gruppe das (3RS,4RS)-4-{4-[3-(3-Brom-benzyloxy)-propoxy]-phenyl}-3-(naphthalin-2-ylmethoxy)-piperidin Hydrochlorid als farbloses Öl; MS : 560 $(M)^+$;

6) - durch Alkylierung mit 4-Iod-benzylbromid und Abspaltung der BOC-Gruppe das (3RS,4RS)-4-{4-[3-(4-Iod-benzyloxy)-propoxy]-phenyl}-3-(naphthalin-2-ylmethoxy)-piperidin Hydrochlorid als farbloses Öl; MS : 608 $(M+H)^+$;

7) - durch Alkylierung mit 2-Trifluormethyl-benzylbromid und Abspaltung der BOC-Gruppe das (3RS,4RS)-3-(Naphthalin-2-ylmethoxy)-4-{4-[3-(2-trifluormethyl-benzyloxy)-propoxy]-phenyl}piperidin Hydrochlorid als farbloses Öl; MS: 550 $(M+H)^+$;

8) - durch Alkylierung mit 3-Trifluormethyl-benzylbromid und Abspaltung der BOC-Gruppe das (3RS,4RS)-3-(Naphthalin-2-ylmethoxy)-4-{4-[3-(3-trifluormethyl-benzyloxy)-propoxy]-phenyl}piperidin Hydrochlorid als farbloses Öl; MS : 550 $(M+H)^+$;

9) - durch Alkylierung mit 4-Trifluormethyl-benzylbromid und Abspaltung der BOC-Gruppe das (3RS,4RS)-3-(Naphthalin-2-ylmethoxy)-4-{4-[3-(4-trifluormethyl-benzyloxy)-propoxy]-phenyl}piperidin Hydrochlorid als farbloses Öl; MS : 550 $(M+H)^+$;

10) - durch Alkylierung mit 2-Fluor-benzylbromid-benzylbromid und Abspaltung der BOC-Gruppe das (3RS,4RS)-4-{4-[3-(2-Fluor-benzyloxy)-propoxy]-phenyl}-3-(naphthalin-2-ylmethoxy)-piperidin als farbloses Öl; MS : 500 $(M+H)^+$;

11) - durch Alkylierung mit 2-Methyl-benzylchlorid und Abspaltung der BOC-Gruppe das (3RS,4RS)-4-{4-[3-(2-Methyl-benzyloxy)-propoxy]-phenyl}-3-(naphthalin-2-ylmethoxy)-piperidin Hydrochlorid als farbloses Öl; MS : 496 $(M+H)^+$;

12) - durch Alkylierung mit 3,5-Dimethyl-benzylbromid und Abspaltung der BOC-Gruppe das (3RS,4RS)-4-{4-[3-(3,5-Dimethylbenzyloxy)-propoxy]-phenyl}-3-(naphthalin-2-ylmethoxy)-piperidin Hydrochlorid als farbloses Öl; MS : 510 $(M+H)^+$;

13) - durch Alkylierung mit 3-Methoxy-benzylchlorid und Abspaltung der BOC-Gruppe das (3RS,4RS)-4-{4-[3-(3-Methoxy-benzyloxy)-propoxy]-phenyl}-3-(naphthalin-2-ylmethoxy)-piperidin Hydrochlorid als farbloses Öl; MS : 512 $(M+H)^+$;

14) - durch Alkylierung mit 4-Isopropyl-benzylchlorid und Abspaltung der BOC-Gruppe das (3RS,4RS)-4-{4-[3-(4-Isopropyl-benzyloxy)-propoxy]-phenyl}-3-(naphthalin-2-ylmethoxy)-piperidin Hydrochloridals farbloses Öl; MS : 524 $(M+H)^+$;

15) - durch Alkylierung mit 2,4-Dimethyl-benzylchlorid und Abspaltung der BOC-Gruppe das (3RS,4RS)-4-{4-[3-(2,4-Dimethylbenzyloxy)-propoxy]-phenyl}-3-(naphthalin-2-ylmethoxy)-piperidin Hydrochlorid als farbloses Öl; MS : 510 $(M+H)^+$;

16) - durch Alkylierung mit 4-Methyl-benzylbromid und Abspaltung der BOC-Gruppe das (3RS,4RS)-4-{4-[3-(4-Methyl-benzyloxy)-propoxy]-phenyl}-3-(naphthalin-2-ylmethoxy)-piperidin Hydrochlorid als farbloses Öl; MS : 496 $(M+H)^+$;

17) - durch Alkylierung mit 4-tert-Butyl-benzylbromid und Abspaltung der BOC-Gruppe das (3RS,4RS)-4-{4-[3-(4-tert-Butyl-benzyloxy)-propoxy]-phenyl}-3-(naphthalin-2-ylmethoxy)-piperidin Hydrochlorid als farbloses Öl; MS : 538 $(M+H)^+$;

18) - durch Alkylierung mit 2,3,5,6-Tetramethyl-benzylchlorid und Abspaltung der BOC-Gruppe das (3RS,4RS)-3-(Naphthalin-2-ylmethoxy)-4-{4-[3-(2,3,5,6-tetramethyl-benzyloxy)-propoxy]-phenyl}-piperidin Hydrochlorid als farbloses Öl; MS : 538 $(M+H)^+$;

19) - durch Alkylierung mit 3,5-Dichlor-benzylchlorid und Abspaltung der BOC-Gruppe das (3RS,4RS)-4-{4-[3-(3,5-Dichlor-benzyloxy)-propoxy]-phenyl}-3-(naphthalin-2-ylmethoxy)-piperidin Hydrochlorid als farbloses Öl; MS : 550 $(M+H)^+$;

20) - durch Alkylierung mit 2,4-Dichlor-benzylchlorid und Abspaltung der BOC-Gruppe das (3RS,4RS)-4-{4-[3-(2,4-Dichlor-benzyloxy)-propoxy]-phenyl}-3-(naphthalin-2-ylmethoxy)-piperidin Hydrochlorid als farbloses Öl; MS : 550 $(M+H)^+$;

21) - durch Alkylierung mit 2,6-Dichlor-benzylchlorid und Abspaltung der BOC-Gruppe das (3RS,4RS)-4-{4-[3-(2,6-Dichlor-benzyloxy)-propoxy]-phenyl}-3-(naphthalin-2-ylmethoxy)-piperidin Hydrochlorid als farbloses Öl; MS : 550 $(M+H)^+$;

22) - durch Alkylierung mit 2,5-Dichlor-benzylchlorid und Abspaltung der BOC-Gruppe das (3RS,4RS)-4-{4-[3-(2,5-Dichlor-benzyloxy)-propoxy]-phenyl}-3-(naphthalin-2-ylmethoxy)-piperidin Hydrochlorid als farbloses Öl; MS : 550 $(M+H)^+$;

23) - durch Alkylierung mit 2-Chlor-6-fluor-benzylchlorid und Abspaltung der BOC-Gruppe das (3RS,4RS)-4-{4-[3-(2-Chlor-6-fluorbenzyloxy)-propoxy]-phenyl}-3-(naphthalin-2-ylmethoxy)-piperidin Hydrochlorid als farbloses Öl; MS : 534 $(M+H)^+$;

24) - durch Alkylierung mit 2-Iod-benzylchlorid und Abspaltung der BOC-Gruppe das (3RS,4RS)-4-{4-[3-(2-Iodo-benzyloxy)-propoxy]-phenyl}-3-(naphthalin-2-ylmethoxy)-piperidin Hydrochlorid als farbloses Öl; MS : 608 $(M+H)^+$;

25) - durch Alkylierung mit 2-Brom-benzylchlorid und Abspaltung der BOC-Gruppe das (3RS,4RS)-4-{4-[3-(2-Brom-benzyloxy)-propoxy]-phenyl}-3-(naphthalin-2-ylmethoxy)-piperidin Hydrochlorid als farbloses Öl; MS : 560 $(M+H)^+$;

26) - durch Alkylierung mit 4-Chlor-benzylchlorid und Abspaltung der BOC-Gruppe das (3RS,4RS)-4-{4-[3-(4-Chlor-benzyloxy)-propoxy]-phenyl}-3-(naphthalin-2-ylmethoxy)-piperidin Hydrochlorid als farbloses Öl; MS : 517 $(M+H)^+$;

27) - durch Alkylierung mit 4-Methylthio-benzylchlorid [J.Org.Chem. (1988), 53(3), 561-569] und Abspaltung der BOC-Gruppe das (3RS,4RS)-4-{4-[3-(4-Methylsulfanyl-benzyloxy)-propoxy]-phenyl}-3-(naphthalin-2-ylmethoxy)-piperidin Hydrochlorid als farbloses Öl; MS : 528 $(M+H)^+$;

28) - durch Alkylierung mit einem Gemisch von 3- und 4-Vinyl-benzylchlorid und Abspaltung der BOC-Gruppe das Gemisch des (3RS,4RS)-3-(Naphthalin-2-ylmethoxy)-4-{4-[3-(3- und 4-vinyl-benzyloxy)-propoxy]-phenyl}-piperidin Hydrochlorids als farbloses Öl; MS : 508 $(M+H)^+$;

29) - durch Alkylierung mit 4-Methoxy-benzylchlorid und Abspaltung der BOC-Gruppe das (3RS,4RS)-4-{4-[3-(4-Methoxy-benzyloxy)-propoxy]-phenyl}-3-(naphthalin-2-ylmethoxy)-piperidin Hydrochlorid als farbloses Öl; MS : 512 $(M+H)^+$;

30) - durch Alkylierung mit 2,4-Dimethoxy-benzylchlorid und Abspaltung der BOC-Gruppe das (3RS,4RS)-4-{4-[3-(2,4-Dimethoxybenzyloxy)-propoxy]-phenyl}-3-(naphthalin-2-ylmethoxy)-piperidin Hydrochlorid als farbloses Öl; MS : 542 $(M+H)^+$;

31) - durch Alkylierung mit 3,4,5-Trimethoxy-benzylchlorid und Abspaltung der BOC-Gruppe das (3RS,4RS)-3-(Naphthalin-2-ylmethoxy)-4-{4-[3-(3,4,5-trimethoxy-benzyloxy)-propoxy]-phenyl}piperidin Hydrochlorid als farbloses Öl; MS : 572 $(M+H)^+$;

32) - durch Alkylierung mit 5-Chlormethyl-benzo[1.3]dioxol und Abspaltung der BOC-Gruppe das (3RS,4RS)-4-{4-[3-(Benzo[1,3]dioxol-5-ylmethoxy)-propoxy]-phenyl}-3-(naphthalin-2-ylmethoxy)-piperidin Hydrochlorid als

farbloses Öl; MS : 526 $(M+H)^+$;

33) - durch Alkylierung mit 3-Chlor-4-methoxy-benzylchlorid und Abspaltung der BOC-Gruppe das (3RS,4RS)-4-{4-[3-(3-Chlor-4-methoxybenzyloxy)-propoxy]-phenyl}-3-(naphthalin-2-ylmethoxy)-piperidin Hydrochlorid als farbloses Öl; MS : 546 $(M+H)^+$;

34) - durch Alkylierung mit 3-Methyl-benzylbromid und Abspaltung der BOC-Gruppe das (3RS,4RS)-4-{4-[3-(3-Methyl-benzyloxy)-propoxy]-phenyl}-3-(naphthalin-2-ylmethoxy)-piperidin Hydrochlorid als farbloses Öl; MS : 496 $(M+H)^+$;

35) - durch Alkylierung mit 3-Fluor-benzylchlorid und Abspaltung der BOC-Gruppe das (3RS,4RS)-4-{4-[3-(3-Fluor-benzyloxy)-propoxy]-phenyl}-3-(naphthalin-2-ylmethoxy)-piperidin Hydrochlorid als farbloses Öl; MS: 500 $(M+H)^+$;

36) - durch Alkylierung mit 2-Methoxy-benzylchlorid und Abspaltung der BOC-Gruppe das (3RS,4RS)-4-{4-[3-(2-Methoxy-benzyloxy)-propoxy]-phenyl}-3-(naphthalin-2-ylmethoxy)-piperidin als farbloses Öl; MS : 512 $(M+H)^+$;

37) - durch Alkylierung mit 2,5-Dimethyl-benzylchlorid und Abspaltung der BOC-Gruppe das (3RS,4RS)- 4-{4-[3-(2,5-Dimethylbenzyloxy)-propoxy]-phenyl}-3-(naphthalin-2-ylmethoxy)-piperidin Hydrochlorid als farbloses Öl; MS : 510 $(M+H)^+$;

38) - durch Alkylierung mit 4-Äthyl-benzylchlorid und Abspaltung der BOC-Gruppe das (3RS,4RS)-4-{4-[3-(4-Äthyl-benzyloxy)-propoxy]-phenyl}-3-(naphthalin-2-ylmethoxy)-piperidin Hydrochlorid als farbloses Öl; MS: 510 $(M+H)^+$;

Beispiel 108

Die folgenden Verbindungen wurden erhalten, indem, analog Beispiel 44 (e), durch Alkylierung des (3RS, 4RS)-4-{4-[3-(2-Hydroxy-benzyloxy)-propoxy]-phenyl}-3-(naphthalin-2-ylmethoxy)-piperidin-1-carbonsäure tert-butylesters die entsprechenden BOC-Derivate synthetisiert wurden, die ohne weitere Reinigung und Charakterisierung in die Abspaltungsreaktion der BOC-Gruppe mittels Zinkbromid in Methylenchlorid, analog Beispiel 10 (b), eingesetzt wurden:

1) - Durch Alkylierung mit 1-Brom-propan und Abspaltung der BOC-Gruppe das (3RS,4RS)-3-(Naphthalin-2-ylmethoxy)-4-{4-[3-(2-propoxybenzyloxy)-propoxy]-phenyl}-piperidin Hydrobromid als farbloses Öl; MS: 540 $(M+H)^+$;

2) - durch Alkylierung mit 1-Brom-butan und Abspaltung der BOC-Gruppe das (3RS,4RS)-4-{4-[3-(2-Butoxy-benzyloxy)-propoxy]-phenyl}-3-(naphthalin-2-ylmethoxy)-piperidin Hydrobromid als farbloses Öl; MS : 554 $(M+H)^+$;

3) - durch Alkylierung mit Brommethyl-cyclopropan und Abspaltung der BOC-Gruppe das (3RS,4RS)-4-{4-[3-(2-Cyclopropylmethoxybenzyloxy)-propoxy]-phenyl}-3-(naphthalin-2-ylmethoxy)-piperidin Hydrobromid als farbloses Öl; MS : 552 $(M+H)^+$;

4) - durch Alkylierung mit Äthyliodid und Abspaltung der BOC-Gruppe das (3RS,4RS)-4-{4-[3-(2-Äthoxy-benzyloxy)-propoxy]-phenyl}-3-(naphthalin-2-ylmethoxy)-piperidin Hydrobromid als farbloses Öl; MS : 526 $(M+H)^+$;

5) - durch Alkylierung mit Brommethyl-cyclobutan und Abspaltung der BOC-Gruppe das (3RS,4RS)-4-{4-[3-(2-Cyclobutylmethoxy-benzyloxy)-propoxy]-phenyl}-3-(naphthalin-2-ylmethoxy)-piperidin Hydrobromid als farbloses Öl; MS : 566 $(M+H)^+$;

6) - durch Alkylierung mit Isobutylbromid und Abspaltung der BOC-Gruppe das (3RS,4RS)-4-{4-[3-(2-Isobutoxy-benzyloxy)-propoxy]-phenyl}-3-(naphthalin-2-ylmethoxy)-piperidin Hydrobromid als farbloses Öl; MS: 554 $(M+H)^+$;

7) - durch Alkylierung mit Benzylbromid und Abspaltung der BOC-Gruppe das (3RS,4RS)-4-{4-[3-(2-Benzyloxy-benzyloxy)-propoxy]-phenyl}-3-(naphthalin-2-ylmethoxy)-piperidin Hydrobromid als farbloses Öl; MS: 588 $(M+H)^+$;

8) - durch Alkylierung mit 4-Brom-1-buten und Abspaltung der BOC-Gruppe das (3RS,4RS)-4-{4-[3-(2-But-3-enyloxy-benzyloxy)-propoxy]-phenyl}-3-(naphthalin-2-ylmethoxy)-piperidin Hydrobromid als farbloses Öl; MS: 552 $(M+H)^+$;

9) - durch Alkylierung mit Allylbromid und Abspaltung der BOC-Gruppe das (3RS,4RS)-4-{4-[3-(2-Allyloxy-benzyloxy)-propoxy]-phenyl}-3-(naphthalin-2-ylmethoxy)-piperidin Hydrobromid als farbloses Öl; MS: 538 $(M+H)^+$;

10) - durch Alkylierung mit Brom-cyclopropan und Abspaltung der BOC-Gruppe das (3RS,4RS)-4-{4-[3-(2-Cyclopropyloxy-benzyloxy)-propoxy]-phenyl}-3-(naphthalin-2-ylmethoxy)-piperidin Hydrobromid als farbloses Öl; MS : 538 $(M+H)^+$.

Der als Ausgangssubstanz eingesetzte (3RS,4RS)-4-{4-[3-(2-Hydroxybenzyloxy)-propoxy]-phenyl}-3-(naphthalin-2-ylmethoxy)-piperidin-1-carbonsäure tert-butylester wurde wie folgt erhalten:

(a) In analoger Weise wie in Beispiel 1 (g) beschrieben, wurde durch Alkylierung des (3RS,4RS)-4-[4-(3-Hydroxy-propoxy)-phenyl]-3-(naphthalin-2-ylmethoxy)-piperidin-1-carbonsäure tert-butylesters [Beispiel 57 (c)] mit 1-Chlormethyl-(2-trimethylsilyl-äthoxymethoxy)-benzol [Beispiel 17 (c)] der (3RS,4RS)-3-(Naphthalin-2-ylmethoxy)-4-(4-{3-[2-(2-trimethylsilanyl-äthoxymethoxy)-benzyloxy]-propoxy}-phenyl)-piperidin-1-carbonsäure tert-butylester als farbloses Öl erhalten; MS : 746 $(M+NH_4)^+$.

(b) Eine Lösung von 50 mg (0.069 mMol) 3-(Naphthalin-2-ylmethoxy)-4-(4-(3-[2-(2-trimethylsilanyl-äthoxymethoxy)-benzyloxy]-propoxy}-phenyl)-piperidin-1-carbonsäure tert-butylester in 0.5 ml Methanol wurde unter Argon auf 0 °C abgekühlt und mit 69 µl (0.138 mMol) einer 2 N Lösung von Chlorwasserstoff in Methanol versetzt. Anschließend ließ man auf Raumtemperatur erwärmen und eine Stunde weiterrühren. Zur Aufarbeitung wurde die Reaktionslösung mit einem 95:5-Gemisch von Methylenchlorid und Methanol (extrahiert gegen 5 Vol-% gesättigten Ammoniak) versetzt und unter vermindertem Druck zur Trockene eingedampft. Der Rückstand wurde zur Reinigung an Kieselgel unter Verwendung eines 3:1-Gemisches von Hexan und Essigester als Eluierungsmittel chromatographiert. Es wurden 33.1 mg (81 % d.Th.) (3RS,4RS)-4-{4-[3-(2-Hydroxy-benzyloxy)-propoxy]-phenyl}-3-(naphthalin-2-ylmethoxy)-piperidin-1-carbonsäure tert-butylester als farbloses Öl erhalten; MS : 598 $(M+H)^+$.

Beispiel 109

Durch Abspaltung der BOC-Gruppe mittels Chlorwasserstoff in Methanol, analog Beispiel 22 (l), oder mittels Zinkbromid in Methylenchlorid, analog Beispiel 10 (b), wurden die folgenden Verbindungen erhalten:

1) - Aus dem (3R,4s,5S)-4-[4-(3-Benzyloxy-propoxy)-phenyl]-3,5-bis-(naphthalin-2-ylmethoxy)-piperidin-1-carbonsäure tert-butylester mittels Chlorwasserstoff in Methanol das (3R,4s,5S)-4-[4-(3-Benzyloxypropoxy)-phenyl]-3,5-bis-(naphthalin-2-ylmethoxy)-piperidin Hydrochlorid als farbloser Festkörper; MS : 638 $(M+H)^+$;

2) - aus dem (3RS,4SR,5SR)-4-[4-(3-Benzyloxy-propoxy)-phenyl]-5-hydroxy-3-(naphthalin-2-ylmethoxy)-piperidin-1-carbonsäure tert-butylester mittels Chlorwasserstoff in Methanol das (3RS,4SR,5SR)-4-[4-(3-Benzyloxy-propoxy)-phenyl]-3-(naphthalin-2-ylmethoxy)-piperidin-5-ol als farbloses Öl; MS : 498 $(M+H)^+$;

3) - aus dem (3R,4s,5S)-4-[4-(3-Benzyloxy-propoxy)-phenyl]-3,5-bis-(1,4-dimethoxy-naphthalin-2-ylmethoxy)-piperidin-1-carbonsäure tert-butylester mittels Zinkbromid in Methylenchlorid das (3R,4s,5S)-4-[4-(3-Benzyloxy-propoxy)-phenyl]-3,5-bis-(1,4-dimethoxy-naphthalin-2-ylmethoxy)-piperidin als farbloses Wachs; MS: 758 $(M+H)^+$;

4) - aus dem (3RS,4SR,5SR)-4-[4-(3-Benzyloxy-propoxy)-phenyl]-3-(1,4-dimethoxy-naphthalin-2-ylmethoxy)-5-hydroxy-piperidin-1-carbonsäure tert-butylester mittels Zinkbromid in Methylenchlorid das (3RS,4SR,5SR)-4-[4-(3-Benzyloxy-propoxy)-phenyl]-3-(1,4-dimethoxynaphthalin-2-ylmethoxy)-piperidin-5-ol als farbloses Öl; MS : 558 $(M+H)^+$;

5) - aus dem (3R,4s,5S)-4-[4-(3-Benzyloxy-propoxy)-phenyl]-3,5-bis-(2,4-dichlor-benzyloxy)-piperidin-1-carbonsäure tert-butylester mittels Chlorwasserstoff in Methanol das (3R,4s,55)-4-[4-(3-Benzyloxy-propoxy)-phenyl]-3,5-bis-(2,4-dichlor-benzyloxy)-piperidin Hydrochlorid als farbloser Festkörper; MS : 676 $(M+H)^+$;

6) - aus dem (3RS,4SR,5SR)-4-[4-(3-Benzyloxy-propoxy)-phenyl]-3-(2,4-dichlor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(2,4-dichlor-benzyloxy)-piperidin-5-ol Hydrochlorid als farbloses Öl; MS : 516 $(M+H)^+$;

7) - aus dem (3R,4s,5S)-4-[4-(3-Benzyloxy-propoxy)-phenyl]-3,5-bis-(2,5-difluor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(2,5-difluor-benzyloxy)-piperidin Hydrochlorid als farbloses Öl; MS : 610 (M+H)+;

8) - aus dem (3RS,4SR,5SR)-4-[4-(3-Benzyloxy-propoxy)-phenyl]-3-(2,5-difluor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(2,5-difluor-benzyloxy)-piperidin-5-ol Hydrochlorid als farbloses Öl; MS : 484 $(M+H)^+$;

9) - aus dem (3R,4s,5S)-4-[4-(3-Benzyloxy-propoxy)-phenyl]-3,5-bis-(4-carboxy-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(4-carboxy-benzyloxy)-piperidin Hydrochlorid als farbloses Öl; , $R_f$: 0.63 ($SiO_2$, Methylenchlorid:Methanol=95:5, extrahiert gegen 5 vol% gesättigte wässerige Ammoniaklösung);

10) - aus dem (3RS,4SR,5SR)-4-[4-(3-Benzyloxy-propoxy)-phenyl]-3-(4-carboxy-benzyloxy)-5-hydroxy-piperidin-1-carbonsäure tert-butylester mittels Chlorwasserstoff in Methanol das 4-{4-(3RS,4SR,5SR)-[4-(3-Benzyloxy-propoxy)-phenyl]-5-hydroxy-piperidin-3-yloxymethyl)-benzoesäure Hydrochlorid als farbloses Öl; $R_f$: 0.30 ($SiO_2$, Methylenchlorid:Methanol=9:1, extrahiert gegen 5 vol% gesättigte wässerige Ammoniaklösung);

11) - aus dem (3R,4s,5S)-4-[4-(3-Benzyloxy-propoxy)-phenyl]-3,5-bis-(2,4-difluor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(2,4-difluor-benzyloxy)-piperidin Hydrochlorid als farbloses Öl; MS : 610 $(M+H)^+$;

12) - aus dem (3RS,4SR,5SR)-4-[4-(3-Benzyloxy-propoxy)-phenyl]-3-(2,4-difluor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(2,4-difluor-benzyloxy)-piperidin-5-ol Hydrochlorid als farbloses Öl; $R_f$: 0.28 ($SiO_2$, Methylenchlorid: Methanol= 9:1, extrahiert gegen 5 vol% gesättigte wässerige Ammoniaklösung);

13) - aus dem (3R,4s,5S)-4-[4-(3-Benzyloxy-propoxy)-phenyl]-3,5-bis-(4-chlor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(4-chlor-benzyloxy)-piperidin Hydrochlorid als farbloses Öl; MS : 606 $(M+H)^+$;

14) - aus dem (3RS,4SR,5SR)-4-[4-(3-Benzyloxy-propoxy)-phenyl]-3-(4-chlor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(4-chlor-benzyloxy)-piperidin-5-ol Hydrochlorid als farbloser Festkörper; MS : 482 $(M+H)^+$;

15) - aus dem (3R,4s,5S)-4-[4-(3-Benzyloxy-propoxy)-phenyl]-3,5-bis-(3,4-dichlor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(3,4-dichlor-benzyloxy)-piperidin Hydrochlorid als farbloser Festkörper; MS : 676 $(M+H)^+$;

16) - aus dem (3RS,4SR,5SR)-4-[4-(3-Benzyloxy-propoxy)-phenyl]-3-(3,4-dichlor-benzyloxy)-5-hydroxy-piperidin-1-carbonsäure tert-butylester mittels Chlorwasserstoff in Methanol das (3RS,4SR,5SR)-[4-(3-Benzyloxy-propoxy)-phenyl]-3-(3,4-dichlor-benzyloxy)-piperidin-5-ol Hydrochlorid als farbloser Festkörper; MS : 516 $(M+H)^+$;

17) - aus dem (3R,4s,5S)-4-[4-(3-Benzyloxy-propoxy)-phenyl]-3,5-bis-(3,5-dichlor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(3,5-dichlor-benzyloxy)-piperidin Hydrochlorid als farbloses Öl; MS : 676 $(M+H)^+$;

18) - aus dem (3RS,4SR,5SR)-4-[4-(3-Benzyloxy-propoxy)-phenyl]-3-(3,5-dichlor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(3,5-dichlor-benzyloxy)-piperidin-5-ol Hydrochlorid als farbloses Öl; MS : 516 $(M+H)^+$;

19) - aus dem (3R,4s,5S)-4-[4-(3-Benzyloxy-propoxy)-phenyl]-3,5-bis-(3-chlor-2-fluor-benzyloxy)-piperidin-1-carbonsäure tert-butylester mittels Chlorwasserstoff in Methanol das (3R,4s,5S)-4-[4-(3-Benzyloxy-propoxy)-phenyl]-3,5-bis-(3-chlor-2-fluor-benzyloxy)-piperidin als farbloses Öl; MS: 642 $(M+H)^+$;

20) - aus dem (3RS,4SR,5SR)-4-[4-(3-Benzyloxy-propoxy)-phenyl]-3-(3-chlor-2-fluor-benzyloxy)-5-hydroxy-piperidin-1-carbonsäure tert-butylester mittels Chlorwasserstoff in Methanol das (3RS,4SR,5SR)-4-[4-(3-Benzyloxy-propoxy)-phenyl]-3-(3-chlor-2-fluor-benzyloxy)-piperidin-5-ol Hydrochlorid als farbloses Öl; MS : 500 $(M+H)^+$;

21) - aus dem (3R,4s,5S)-4-[4-(3-Benzyloxy-propoxy)-phenyl]-3,5-bis-(quinolin-7-ylmethoxy)-piperidin-1-carbonsäure tert-butylester mittels Chlorwasserstoff in Methanol das (3R,4s,5S)-4-[4-(3-Benzyloxy-propoxy)-phenyl]-3,5-bis-(quinolin-7-ylmethoxy)-piperidin Hydrochlorid als farbloses Öl; MS : 640 $(M+H)^+$;

22) - aus dem (3RS,4SR,5SR)-4-[4-(3-Benzyloxy-propoxy)-phenyl]-5-hydroxy-3-(quinolin-7-ylmethoxy)-piperidin-1-carbonsäure tert-butylester mittels Chlorwasserstoff in Methanol das (3RS,4SR,5SR)-4-[4-(3-Benzyloxy-propoxy)-phenyl]-3-(quinolin-7-ylmethoxy)-piperidin-5-ol Hydrochlorid als farbloses Öl; MS : 499 $(M+H)^+$;

23) - aus dem (3R,4s,5S)-4-[4-(3-Benzyloxy-propoxy)-phenyl]-3,5-bis-(4-äthyl-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-äthyl-benzyloxy)-piperidin als farbloses Öl; MS : 594 $(M+H)^+$;

24) - aus dem (3RS,4SR,5SR)-4-[4-(3-Benzyloxy-propoxy)-phenyl]-3-(4-äthyl-benzyloxy)-5-hydroxy-piperidin-1-carbonsäure tert-butylester mittels Zinkbromid in Methylenchlorid das (3RS,4SR,5SR)-4-[4-(3-Benzyloxy-propoxy)-phenyl]-3-(4-äthyl-benzyloxy)-piperidin-5-ol als farbloses Öl; MS : 476 $(M+H)^+$;

25) - aus dem (3R,4s,5S)-4-[4-(3-Benzyloxy-propoxy)-phenyl]-3,5-bis-(4-vinyl-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-vinyl-benzyloxy)-piperidin als farbloses Öl; MS : 590 $(M+H)^+$;

26) - aus dem (3RS,4SR,5SR)-4-[4-(3-Benzyloxy-propoxy)-phenyl]-5-hydroxy-3-(4-vinyl-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-vinyl-benzyloxy)-piperidin-5-ol als farbloses Öl; MS : 474 $(M+H)^+$;

27) - aus dem (3R,4s,5S)-4-[4-(3-Benzyloxy-propoxy)-phenyl]-3,5-bis-(4-methoxy-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-methoxy-benzyloxy)-piperidin als farbloses Öl; MS : 598 $(M+H)^+$;

28) - aus dem (3RS,4SR,5SR)-4-[4-(3-Benzyloxy-propoxy)-phenyl]-5-hydroxy-3-(4-methoxy-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-methoxy-benzyloxy)-piperidin-5-ol als farbloses Öl; MS : 478 $(M+H)^+$;

29) - aus dem (3R,4s,5S)-4-[4-(3-Benzyloxy-propoxy)-phenyl]-3,5-bis-(3,4,5-trimethoxy-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(3,4,5-trimethoxy-benzyloxy)-piperidin als farbloses Öl; MS : 718 $(M+H)^+$;

30) - aus dem (3RS,4SR,5SR)-4-[4-(3-Benzyloxy-propoxy)-phenyl]-5-hydroxy-3-(3,4,5-trimethoxy-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(3,4,5-trimethoxy-benzyloxy)-piperidin-5-ol als farbloses Öl; MS : 538 $(M+H)^+$;

31) - aus dem (3R,4s,5S)-4-[4-(3-Benzyloxy-propoxy)-phenyl]-3,5-bis-(3,5-dimethoxy-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(3,5-dimethoxy-benzyloxy)-piperidin als farbloses Öl; MS : 658 $(M+H)^+$;

32) - aus dem (3RS,4SR,5SR)-4-[4-(3-Benzyloxy-propoxy)-phenyl]-3-(3,5-dimethoxy-benzyloxy)-5-hydroxy-piperidin-1-carbonsäure tert-butylester mittels Zinkbromid in Methylenchlorid das (3RS,4SR,5SR)-4-[4-(3-Benzyloxy-propoxy)-phenyl]-3-(3,5-dimethoxy-benzyloxy)-piperidin-5-ol als farbloses Öl; MS : 508 $(M+H)^+$;

33) - aus dem (3R,4s,5S)-4-[4-(3-Benzyloxy-propoxy)-phenyl]-3,5-bis-(4-trifluormethoxy-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-trifluormethoxy-benzyloxy)-piperidin als farbloses Öl; MS : 706 $(M+H)^+$;

34) - aus dem (3RS,4SR,5SR)-4-[4-(3-Benzyloxy-propoxy)-phenyl]-5-hydroxy-3-(4-trifluormethoxy-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-trifluormethoxy-benzyloxy)-piperidin-5-ol als farbloses Öl; MS : 532 $(M+H)^+$;

35) - aus dem (3R,4s,5S)-4-[4-(3-Benzyloxy-propoxy)-phenyl]-3,5-bis-(4-methylsulfanyl-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-methylsulfanyl-benzyloxy)-piperidin als farbloses Öl; MS : 630 $(M+H)^+$;

36) - aus dem (3RS,4SR,5SR)-4-[4-(3-Benzyloxy-propoxy)-phenyl]-5-hydroxy-3-(4-methylsulfanyl-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-methylsulfanyl-benzyloxy)-piperidin-5-ol als farbloses Öl; MS : 494 $(M+H)^+$;

37) - aus dem (3R,4s,5S)-4-[4-(3-Benzyloxy-propoxy)-phenyl]-3,5-bis-(4-isopropyl-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-isopropyl-benzyloxy)-piperidin als farbloses Öl; MS : 622 $(M+H)^+$;

38) - aus dem (3RS,4SR,5SR)-4-[4-(3-Benzyloxy-propoxy)-phenyl]-5-hydroxy-3-(4-isopropyl-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-isopropyl-benzyloxy)-piperidin-5-ol als farbloses Öl; MS : 490 $(M+H)^+$;

39) - aus dem (3R,4s,5S)-4-[4-(3-Benzyloxy-propoxy)-phenyl]-3,5-bis-(3-chlor-4-methoxy-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(3-chlor-4-methoxy-benzyloxy)-piperidin als farbloses Öl; MS : 666 $(M+H)^+$;

40) - aus dem (3RS,4SR,5SR)-4-[4-(3-Benzyloxy-propoxy)-phenyl]-3-(3-chlor-4-methoxy-benzyloxy)-5-hydroxy-piperidin-1-carbonsäure tert-butylester mittels Zinkbromid in Methylenchlorid das (3RS,4SR,5SR)-4-[4-(3-Benzyloxy-propoxy)-phenyl]-3-(3-chlor-4-methoxy-benzyloxy)-piperidin-5-ol als farbloses Öl; MS : 512 $(M+H)^+$;

41) - aus dem (3R,4s,5S)-4-[4-(3-Benzyloxy-propoxy)-phenyl]-3,5-bis-(4-methoxy-3-methyl-benzyloxy)-piperidin-1-carbonsäure tert-butylester mittels Zinkbromid in Methylenchlorid das (3R,4s,5S)-4-[4-(3-Benzyloxypropoxy)-phenyl]-3,5-bis-(4-methoxy-3-methyl-benzyloxy)-piperidin als farbloses Öl; MS : 626 $(M+H)^+$;

42) - aus dem (3RS,4SR,5SR)-4-[4-(3-Benzyloxy-propoxy)-phenyl]-5-hydroxy-3-(4-methoxy-3-methyl-benzyloxy)-piperidin-1-carbonsäure tert-butylester mittels Zinkbromid in Methylenchlorid das (3RS,4SR,5SR)-4-[4-(3-Benzyloxy-propoxy)-phenyl]-3-(4-methoxy-3-methyl-benzyloxy)-piperidin-5-ol als farbloses Öl; MS : 492 $(M+H)^+$.

Die als Ausgangsverbindungen eingesetzten BOC-Derivate wurden wie folgt erhalten:

In analoger Weise wie in Beispiel 1 (g) beschrieben, wurden durch Alkylierung des (3R,4s,5S)-4-[4-(3-Benzyloxy-propoxy)-phenyl]-3,5-dihydroxy-piperidin-1-carbonsäure tert-butylesters unter Verwendung von einem Äquivalent eines benzylischen Halogenides in etwa gleichen Mengenanteilen unverändertes Ausgangsmaterial sowie die entsprechenden mono- und dialkylierten BOC-Derivate erhalten. Diese Gemische wurden anschließend mittels Chromatographie aufgetrennt:

(a) - Durch Alkylierung mit 2-Brommethyl-naphthalin der (3R,4s,5S)-4-[4-(3-Benzyloxy-propoxy)-phenyl]-3,5-bis-(naphthalin-2-ylmethoxy)-piperidin-1-carbonsäure tert-butylester, MS : 739 $(M+H)^+$, und der (3RS,4SR,5SR)-4-[4-(3-Benzyloxy-propoxy)-phenyl]-5-hydroxy-3-(naphthalin-2-ylmethoxy)-piperidin-1-carbonsäure tert-butylester, MS : 598 $(M+H)^+$, jeweils als farbloser Festkörper;

(b) - durch Alkylierung mit 2-Chlormethyl-1,4-dimethoxy-naphthalin [J.Org.Chem. (1983), 48(19),3265-3268] der (3R,4s,5S)-4-[4-(3-Benzyloxypropoxy)-phenyl]-3,5-bis-(1,4-dimethoxy-naphthalin-2-ylmethoxy)-piperidin-1-carbonsäure tert-butylester, MS : 876 $(M+H)^+$, als farbloser Schaum und der (3RS,4SR,5SR)-4-[4-(3-Benzyloxy-propoxy)-phenyl]-3-(1,4-dimethoxy-naphthalin-2-ylmethoxy)-5-hydroxy-piperidin-1-carbonsäure tert-butylester, MS : 659 $(M+H)^+$, als farbloses Öl;

(c) - durch Alkylierung mit 2,4-Dichlor-benzylchlorid der (3R,4s,5S)-4-[4-(3-Benzyloxy-propoxy)-phenyl]-3,5-bis-(2,4-dichlor-benzyloxy)-piperidin-1-carbonsäure tert-butylester, $R_f$: 0.83 ($SiO_2$, Methylenchlorid:Essigsäure-äthylester=8:2), und der (3RS,4SR,5SR)-4-[4-(3-Benzyloxy-propoxy)-phenyl]-3-(2,4-dichlor-benzyloxy)-5-hydroxypiperidin-1-carbonsäure tert-butylester, $R_f$: 0.30 ($SiO_2$, Methylenchlorid:Essigsäureäthylester=8:2), jeweils als farbloses Öl;

(d) - durch Alkylierung mit 2,5-Difluor-benzylbromid der (3R,4s,5S)-4-[4-(3-Benzyloxy-propoxy)-phenyl]-3,5-bis-(2,5-difluor-benzyloxy)-piperidin-1-carbonsäure tert-butylester, MS : 727 $(M+NH_4)^+$, und der (3RS,4SR,5SR)-4-[4-(3-Benzyloxy-propoxy)-phenyl]-3-(2,5-difluorbenzyloxy)-5-hydroxy-piperidin-1-carbonsäure tert-butylester, $R_f$: 0.26 ($SiO_2$, Methylenchlorid:Essigsäure-äthylester=8:2), jeweils als farbloses Öl;

(e) - durch Alkylierung mit 4-Brommethyl-benzoesäuremethylester und Verseifung der Methylester während der wässerigen Aufarbeitung der (3R,4s,5S)-4-[4-(3-Benzyloxy-propoxy)-phenyl]-3,5-bis-(4-carboxybenzyloxy)-piperidin-1-carbonsäure tert-butylester, $R_f$: 0.18 ($SiO_2$, Methylenchlorid:Methanol=9:1), und der (3RS,4SR,5SR)-4-[4-(3-Benzyloxy-propoxy)-phenyl]-3-(4-carboxy-benzyloxy)-5-hydroxypiperidin-1-carbonsäure tert-butylester, $R_f$: 0.42 ($SiO_2$, Methylenchlorid:Methanol=9:1), jeweils als farbloses Öl;

(f) - durch Alkylierung mit 2,4-Difluor-benzylbromid der (3R,4s,5S)-4-[4-(3-Benzyloxy-propoxy)-phenyl]-3,5-bis-(2,4-difluor-benzyloxy)-piperidin-1-carbonsäure tert-butylester, MS : 727 $(M+NH_4)^+$, und der (3RS,4SR,5SR)-4-[4-(3-Benzyloxy-propoxy)-phenyl]-3-(2,4-difluorbenzyloxy)-5-hydroxy-piperidin-1-carbonsäure tert-butylester, $R_f$: 0.24 ($SiO_2$, Methylenchlorid:Essigsäureäthylester=8:2),jeweils als farbloses Öl;

(g) - durch Alkylierung mit 4-Chlor-benzylchlorid der (3R,4s,5S)-4-[4-(3-Benzyloxy-propoxy)-phenyl]-3,5-bis-(4-chlor-benzyloxy)-piperidin-1-carbonsäure tert-butylester, MS : 724 $(M+NH_4)^+$, und der (3RS,4SR,5SR)-4-[4-(3-Benzyloxy-propoxy)-phenyl]-3-(4-chlor-benzyloxy)-5-hydroxy-piperidin-1-carbonsäure tert-butylester, MS: 582 $(M+H)^+$, jeweils als farbloses Öl;

(h) - durch Alkylierung mit 3,4-Dichlor-benzylchorid der (3R,4s,5S)-4-[4-(3-Benzyloxy-propoxy)-phenyl]-3,5-bis-(3,4-dichlor-benzyloxy)-piperidin-1-carbonsäure tert-butylester, MS : 793 $(M+NH_4)^+$, und der (3RS,4SR,5SR)-4-[4-(3-Benzyloxy-propoxy)-phenyl]-3-(3,4-dichlorbenzyloxy)-5-hydroxy-piperidin-1-carbonsäure tert-butylester, $R_f$: 0.55 ($SiO_2$, Methylenchlorid:Essigsäureäthylester=8:2), jeweils als farbloses Öl;

(i) - durch Alkylierung mit 3,5-Dichlor-benzylchorid der (3R,4s,5S)-4-[4-(3-Benzyloxy-propoxy)-phenyl]-3,5-bis-(3,5-dichlor-benzyloxy)-piperidin-1-carbonsäure tert-butylester, MS : 793 $(M+NH_4)^+$, und der (3RS,4SR,5SR)-4-[4-(3-Benzyloxy-propoxy)-phenyl]-3-(3,5-dichlorbenzyloxy)-5-hydroxy-piperidin-1-carbonsäure tert-butylester, MS : 634 $(M+NH_4)^+$, jeweils als farbloses Öl;

(j) - durch Alkylierung mit 3-Chlor-2-fluor-benzylbromid der (3R,4s,5S)-4-[4-(3-Benzyloxy-propoxy)-phenyl]-3,5-bis-(3-chlor-2-fluorbenzyloxy)-piperidin-1-carbonsäure tert-butylester, MS : 760 $(M+NH_4)^+$, und der (3RS,4SR,5SR)-4-[4-(3-Benzyloxy-propoxy)-phenyl]-3-(3-Chlor-2-fluor-benzyloxy)-5-hydroxy-piperidin-1-carbonsäure tert-butylester, $R_f$: 0.54 ($SiO_2$, Methylenchlorid:Essigsäureäthylester=8:2), jeweils als farbloses Öl;

(k) - durch Alkylierung mit 7-Brommethyl-chinolin Hydrobromid [J.Am.Chem.Soc. 77, 1054(1955)], unter Verwendung von entsprechend mehr Natriumhydrid, der (3R,4s,5S)-4-[4-(3-Benzyloxy-propoxy)-phenyl]-3,5-bis-(quinolin-7-ylmethoxy)-piperidin-1-carbonsäure tert-butylester, MS : 740 $(M+H)^+$, und der (3RS,4SR,5SR)-4-[4-(3-Benzyloxypropoxy)-phenyl]-5-hydroxy-3-(quinolin-7-ylmethoxy)-piperidin-1-carbonsäure tert-butylester , MS : 599 $(M+H)^+$, $R_f$: 0.35 ($SiO_2$, Methylenchlorid:Essigsäureäthylester=2:3), jeweils als farbloses Öl;

(l) - durch Alkylierung mit 4-Äthyl-benzylchlorid der (3R,4s,5S)-4-[4-(3-Benzyloxy-propoxy)-phenyl]-3,5-bis-(4-äthyl-benzyloxy)-piperidin-1-carbonsäure tert-butylester, MS : 711 $(M+NH_4)^+$, und der (3RS,4SR,5SR)-4-[4-(3-Benzyloxy-propoxy)-phenyl]-3-(4-äthyl-benzyloxy)-5-hydroxy-piperidin-1-carbonsäure tert-butylester, $R_f$: 0.30 ($SiO_2$, Methylenchlorid:Essigsäur-eäthylester=8:2), jeweils als farbloses Öl;

(m) - durch Alkylierung mit 4-Vinyl-benzylchlorid der (3R,4s,5S)-4-[4-(3-Benzyloxy-propoxy)-phenyl]-3,5-bis-(4-vinyl-benzyloxy)-piperidin-1-carbonsäure tert-butylester, MS : 707 $(M+NH_4)^+$, und der (3RS,4SR,5SR)-4-[4-(3-Benzyloxy-propoxy)-phenyl]-5-hydroxy-3-(4-vinylbenzyloxy)-piperidin-1-carbonsäure tert-butylester, $R_f$: 0.30 ($SiO_2$, Methylenchlorid:Essig-säureäthylester=8:2), jeweils als farbloses Öl;

(n) - durch Alkylierung mit 4-Methoxy-benzylchlorid der (3R,4s,5S)-4-[4-(3-Benzyloxy-propoxy)-phenyl]-3,5-bis-(4-methoxy-benzyloxy)-piperidin-1-carbonsäure tert-butylester, MS: 715 $(M+NH_4)^+$, und der (3RS,4SR,5SR)-4-[4-(3-Benzyloxy-propoxy)-phenyl]-5-hydroxy-3-(4-methoxy-benzyloxy)-piperidin-1-carbonsäure tert-butylester, MS: 595 $(M+NH_4)^+$, jeweils als farbloses Öl;

(o) - durch Alkylierung mit 3,4,5-Trimethoxy-benzylchlorid der (3R,4s,5S)-4-[4-(3-Benzyloxy-propoxy)-phenyl]-3,5-bis-(3,4,5-trimethoxybenzyloxy)-piperidin-1-carbonsäure tert-butylester, MS : 835 $(M+NH_4)^+$, und der (3RS,4SR,5SR)-4-[4-(3-Benzyloxy-propoxy)-phenyl]-5-hydroxy-3-(3,4,5-trimethoxy-benzyloxy)-piperidin-1-carbonsäure tert-butylester, MS: 655 $(M+NH_4)^+$, jeweils als farbloses Öl;

(p) - durch Alkylierung mit 3,5-Dimethoxy-benzylchlorid der (3R,4s,5S)-4-[4-(3-Benzyloxy-propoxy)-phenyl]-3,5-bis-(3,5-dimethoxybenzyloxy)-piperidin-1-carbonsäure tert-butylester, MS : 775 $(M+NH_4)^+$, und der (3RS,4SR, 5SR)-4-[4-(3-Benzyloxy-propoxy)-phenyl]-3-(3,5-dimethoxy-benzyloxy)-5-hydroxy-piperidin-1-carbonsäure tert-butylester, MS : 625 $(M+NH_4)^+$, jeweils als farbloses Öl;

(q) - durch Alkylierung mit 4-Trifluormethoxy-benzylbromid der (3R,4s,5S)-4-[4-(3-Benzyloxy-propoxy)-phenyl]-3,5-bis-(4-trifluormethoxybenzyloxy)-piperidin-1-carbonsäure tert-butylester, MS : 823 $(M+NH_4)^+$, und der (3RS, 4SR,5SR)-4-[4-(3-Benzyloxy-propoxy)-phenyl]-5-hydroxy-3-(4-trifluormethoxy-benzyloxy)-piperidin-1-carbonsäure tert-butylester, $R_f$: 0.32 ($SiO_2$, Methylenchlorid:Essigsäureäthylester=8:2), jeweils als farbloses Öl;

(r) - durch Alkylierung mit 4-Methylthio-benzylchlorid [J.Org.Chem. (1988), 53(3), 561-569] der (3R,4s,5S)-4-[4-(3-Benzyloxy-propoxy)-phenyl]-3,5-bis-(4-methylsulfanyl-benzyloxy)-piperidin-1-carbonsäure tert-butylester, MS: 747 $(M+NH_4)^+$, und der (3RS,4SR,5SR)-4-[4-(3-Benzyloxy-propoxy)-phenyl]-5-hydroxy-3-(4-methylsulfanyl-benzyloxy)-piperidin-1-carbonsäure tert-butylester, MS : 611 $(M+NH_4)^+$, jeweils als farbloses Öl;

(s) - durch Alkylierung mit 4-Isopropyl-benzylchlorid der (3R,4s,5S)-4-[4-(3-Benzyloxy-propoxy)-phenyl]-3,5-bis-(4-isopropyl-benzyloxy)-piperidin-1-carbonsäure tert-butylester, MS : 739 $(M+NH_4)^+$, und der (3RS,4SR, 5SR)-4-[4-(3-Benzyloxy-propoxy)-phenyl]-5-hydroxy-3-(4-isopropyl-benzyloxy)-piperidin-1-carbonsäure tert-butylester, MS : 607 $(M+NH_4)^+$, jeweils als farbloses Öl;

(t) - durch Alkylierung mit 3-Chlor-4-methoxy-benzylchlorid der (3R,4s,5S)-4-[4-(3-Benzyloxy-propoxy)-phenyl]-3,5-bis-(3-chlor-4-methoxybenzyloxy)-piperidin-1-carbonsäure tert-butylester, MS : 784 $(M+NH_4)^+$, und der (3RS, 4SR,5SR)-4-[4-(3-Benzyloxy-propoxy)-phenyl]-3-(3-chlor-4-methoxy-benzyloxy)-5-hydroxy-piperidin-1-carbonsäure tert-butylester, MS : 630 $(M+NH_4)^+$, jeweils als farbloses Öl;

(u) - durch Alkylierung mit 4-Methoxy-3-methyl-benzylchlorid der (3R,4s,5S)-4-[4-(3-Benzyloxy-propoxy)-phenyl]-3,5-bis-(4-methoxy-3-methyl-benzyloxy)-piperidin-1-carbonsäure tert-butylester, MS : 743 $(M+NH_4)+$, und der (3RS,4SR,5SR)-4-[4-(3-Benzyloxy-propoxy)-phenyl]-5-hydroxy-3-(4-methoxy-3-methyl-benzyloxy)-piperidin-1-carbonsäure tert-butylester, MS : 609 $(M+NH_4)^+$, jeweils als farbloses Öl.

Der als Ausgangsverbindung eingesetzte (3R,4s,5S)-4-[4-(3-Benzyloxypropoxy)-phenyl]-3,5-dihydroxy-piperidin-1-carbonsäure tert-butylester wurde wie folgt erhalten:

($\alpha$) 50.0 g (179 mMol) 1-Benzyl-4-(4-methoxy-phenyl)-1,2,3,6-tetrahydropyridin [Beispiel 44 (b)] wurden in 500 ml Tetrahydrofuran gelöst, bei Raumtemperatur mit 36.3 ml (322 mMol) 48 prozentiger wässeriger Bromwasserstoffsäure versetzt und anschliessend das Reaktionsgemisch am Rotationsverdampfer eingeengt. Der so gebildete Rückstand wurde zweimal mit 500 ml Toluol aufgeschlämmt und erneut eingeengt, dann in 1500 ml Dioxan und 1200 ml Wasser gelöst, mit 51.6 g (501 mMol) Natriumbromid und 9.3 ml (181 mMol) Brom versetzt und 2 Stunden bei Raumtemperatur gerührt. Die so erhaltene orangefarbene Lösung wurde anschliessend auf 0° C gekühlt und bei 5° bis 10° C mit 1240 ml 2 N Natronlauge versetzt und für weitere 2 Stunden bei Raumtemperatur gerührt. Hierauf wurde das Reaktionsgemisch dreimal mit 2 Liter Essigsäureäthylester extrahiert, die vereinigten organischen Phasen mit Wasser gewaschen, über Magnesiumsulfat getrocknet und am Rotationsverdampfer bei maximal 40° C eingedampft. Dabei wurden 53.64 g (ca. 100% d. Th.) (1RS,6R5)-3-Benzyl-6-(4-methoxy-phenyl)-7-oxa-3-aza-bicyclo[4.1.0]-heptan in Form eines braunen Festkörpers erhalten; MS: 295 $(M)^+$.

($\beta$) 53.44 g (179 mMol) (1RS,6RS)-3-Benzyl-6-(4-methoxy-phenyl)-7-oxa-3-aza-bicyclo[4.1.0]heptan wurden in 980 ml Äther suspendiert und diese Suspension unter Argon und Feuchtigkeitsausschluss zu 226 ml 1.6 M Methyllithium-Lösung in Diäthyläther (362 mMol) bei Raumtemperatur zugetropft. Anschliessend wurde das Reaktionsgemisch während einer Stunden unter Rückfluss erwärmt. Nach dem Abkühlen auf Raumtemperatur wurde es auf 1.5 Liter gesättigte Natriumhydrogen-carbonat-Lösung gegossen und zweimal mit 1.5 Liter Essigsäureäthylester extrahiert, die vereinigten Essigsäureäthylesterphasen mit Wasser gewaschen, über Magnesiumsulfat getrocknet und am Rotationsverdampfer bei maximal 40° C eingedampft. Dabei wurden 52.8 g (ca. 100% d. Th.) (RS)-1-Benzyl-4-(4-methoxy-phenyl)-1,2,3,6-tetrahydro-pyridin-3-ol in Form eines braunen Öles erhalten; MS: 296 $(M+H)^+$.

($\gamma$) 52.6 g (178 mMol) (RS)-1-Benzyl-4-(4-methoxy-phenyl)-1,2,3,6-tetrahydro-pyridin-3-ol wurden in 300 ml N, N-Dimethylformamid gelöst, portionenweise mit 25 g (ca. 600 mMol) Natriumhydriddispersion in Weissöl (55 - 65%) versetzt und das Reaktionsgemisch unter Argon während 1 Stunde auf 50° C erwärmt. Nach dem Abkühlen auf 5° C wurde langsam mit 23 ml (285 mMol) Äthyliodid versetzt und eine Stunde ohne Kühlung gerührt. Hierauf

wurde das Reaktionsgemisch auf 2 Liter Eiswasser gegossen und dreimal mit 1 Liter Essigsäureäthylester extrahiert. Die vereinigten Essigsäureäthylesterphasen wurden anschliessend mit Wasser gewaschen, über Magnesiumsulfat getrocknet und am Rotationsverdampfer bei maximal 40° C eingedampft. Der dabei erhaltene Rückstand wurde an Kieselgel mit Hexan/Essigsäureäthylester (5:1) chromatographiert. Dabei wurden 42.51 g (74% d. Th.) (RS)-1-Benzyl-3-äthoxy-4-(4-methoxyphenyl)-1,2,3,6-tetrahydro-pyridin in Form eines orangefarbenen Öles erhalten; MS: 324 $(M)^+$.

(δ) 42.3 g (131 mMol) (RS)-1-Benzyl-3-äthoxy-4-(4-methoxy-phenyl)-1,2,3,6-tetrahydro-pyridin wurden in 500 ml 1,2-Dimethoxyäthan gelöst, mit 7.45 g (196 mMol) Natriumborhydrid versetzt und unter Kühlung bei maximal 28° C mit einer Lösung von 44.3 ml (353 mMol) Bortrifluorid-ätherat in 44.3 ml 1,2-Dimethoxyäthan versetzt und das Reaktionsgemisch während 2 Stunden bei Raumtemperatur gerührt. Anschliessend wurde unter Kühlung bei maximal 35° C 169 ml 4.1 N Kaliumhydroxid-Lösung gefolgt von 33.9 ml 30 prozentiger Wasserstoffperoxid-Lösung zugetropft und das Reaktionsgemisch während 3 Stunden unter Rückfluss erwärmt. Nach Abkühlen auf Raumtemperatur wurde die Reaktionslösung auf 2 Liter Wasser gegossen und zweimal mit je 1 Liter Methylenchlorid extrahiert. Die vereinigten Methylenchloridphasen wurden mit Wasser gewaschen, über Magnesiumsulfat getrocknet und am Rotationsverdampfer bei maximal 40° C eingedampft. Der dabei erhaltene Rückstand wurde an Kieselgel mit Hexan/Essigsäureäthylester (zunächst 4:1, dann Essigsäureäthylesteranteil stufenweise bis 1:1 erhöht) chromatographiert. So wurden 22.1 g (49% d. Th.) (3RS,4RS,5SR)-5-Äthoxy-1-benzyl-3-hydroxy-4-(4-methoxy-phenyl)-piperidin in Form eines gelblichen Öles erhalten; MS: 342 $(M+H)^+$.

(ε) 52.39 g (153.4 mMol) (3RS,4RS,5SR)-5-Äthoxy-1-benzyl-3-hydroxy-4-(4-methoxy-phenyl)-piperidin wurden in 525 ml Methylenchlorid gelöst und bei maximal 40° C mit 306 ml IM Bortribromid-Lösung in Methylenchlorid versetzt und das Reaktionsgemisch während 4 Stunden bei Raumtemperatur gerührt. Hierauf wurde das Reaktionsgemisch auf 5° C gekühlt und die gebildeten Kristalle abfiltriert. Diese wurden anschliessend in Methylenchlorid/Methanol (8:2, extrahiert gegen 5 vol% konz. wässerigen Ammoniak) gelöst mit ebendiesem Eluens an Kieselgel chromatographiert. Dabei resultierten 34.18 g (74% d. Th.) (3R,4s,5S)-1-Benzyl-4-(4-hydroxy-phenyl)-piperidin-3,5-diol in Form eines amorphen, farblosen Festkörpers; MS: 300 $(M+H)^+$.

(ζ) 33.98 g (113.5 mMol) (3R,4s,5S)-1-Benzyl-4-(4-hydroxy-phenyl)-piperidin-3,5-diol wurden in 1.7 Liter Methanol gelöst, mit 5.1 g Palladium auf Kohle (10%) versetzt und bei Raumtemperatur unter Normaldruck erschöpfend hydriert. Anschliessend wurde das Reaktionsgemisch über Kieselgel filtriert und im Wasserstrahlvakuum eingeengt. Dabei resultierten 22.56 g (95% d. Th.) (3R,4s,5S)-4-(4-Hydroxy-phenyl)-piperidin-3,5-diol in Form eines amorphen, farblosen Festkörpers; MS: 209 $(M)^+$.

(η) 22.36 g (106 mMol) (3R,4s,5S)-4-(4-Hydroxy-phenyl)-piperidin-3,5-diol wurden in 559 ml Dioxan und 186 ml Wasser gelöst, mit 18.85 g (224 mMol) Natriumhydrogencarbonat und 25.65 g (117.5 mMol) Di-tert-butyldicarbonat versetzt und während 2 Stunden bei Raumtemperatur gerührt. Hierauf wurde das Reaktionsgemisch auf 1.5 Liter Eiswasser gegossen und zweimal mit 1.5 Liter Essigsäureäthylester extrahiert. Die vereinigten Essigsäureäthylesterphasen wurden über Magnesiumsulfat getrocknet und am Rotationsverdampfer bei maximal 50° C eingedampft. Der so erhaltene Rückstand wurde an Kieselgel mit Methylenchlorid/Methanol (95:5) chromatographiert. Dabei resultierten 15.79 g (48% d. Th.) (3R,4s,5S)-3,5-Dihydroxy-4-(4-hydroxy-phenyl)-piperidin-1-carbonsäure tert-butylester in Form farbloser Kristalle; MS: 310 $(M+H)^+$.

(θ) 15.59 g (50.4 mMol) (3R,4s,5S)-3,5-Dihydroxy-4-(4-hydroxy-phenyl)-piperidin-1-carbonsäure tert-butylester wurden in 510 ml Methyläthylketon gelöst, mit 28 g (201 mMol) Kaliumcarbonat gefolgt von 34.7 g (151 mMol) Benzyl-3-bromopropyläther versetzt und das Reaktionsgemisch während 24 Stunden unter Rückfluss erwärmt. Nach dem Abkühlen auf Raumtemperatur wurde es auf 800 ml Eiswasser gegossen und zweimal mit 500 ml Essigsäureäthylester extrahiert, die vereinigten Essigsäureäthylesterphasen mit Wasser gewaschen, über Magnesiumsulfat getrocknet und am Rotationsverdampfer bei maximal 40° C eingedampft. Der so erhaltene Rückstand wurde an Kieselgel mit Methylenchlorid/Essig-säureäthylester (7:3) chromatographiert. Dabei wurden 20.5 g (89% d. Th.) (3R,4s,5S)-4-[4-(3-Benzyloxy-propoxy)-phenyl]-3,5-dihydroxy-piperidin-1-carbonsäure tert-butylester in Form eines amorphen, farblosen Festkörpers erhalten; MS: 458 $(M+H)^+$.

Beispiel 110

Durch Abspaltung der BOC-Gruppe mittels Chlorwasserstoff in Methanol, analog Beispiel 22 (l), oder mittels Zinkbromid in Methylenchlorid, analog Beispiel 10 (b), wurden die folgenden Verbindungen erhalten:

1) - Aus dem (3RS,4SR,5SR)-4-[4-(3-Benzyloxy-propoxy)-phenyl]-5-äthoxy-3-(naphthalin-2-ylmethoxy)-piperidin-1-carbonsäure tert-butyl ester mittels Zinkbromid in Methylenchlorid das (3RS,4SR,5SR)-4-[4-(3-Benzyloxy-propoxy)-phenyl]-5-äthoxy-3-(naphthalin-2-ylmethoxy)-piperidin als farbloses Öl; MS : 526 $(M+H)^+$;

2) - aus dem (3RS,4RS,5SR)-4-[4-(3-Benzyloxy-propoxy)-phenyl]-5-[2-(4-methyl-piperazin-1-yl)-äthoxy]-3-(naphthalin-2-ylmethoxy)-piperidin-1-carbonsäure tert-butyl ester mittels Chlorwasserstoff in Methanol das 1-{2-[(3RS,4RS,5SR)-4-[4-(3-Benzyloxy-propoxy)-phenyl]-3-(naphthalin-2-ylmethoxy)-piperidin-5-yloxy]-äthyl}-4-methyl-piperazin als farbloses Öl; MS : 624 $(M+H)^+$;

3) - aus dem (3RS,4SR,5SR)-4-[4-(3-Benzyloxy-propoxy)-phenyl]-3-(naphthalin-2-ylmethoxy)-5-propoxy-piperidin-1-carbonsäure tert-butyl ester mittels Chlorwasserstoff in Methanol das (3RS,4SR,5SR)-4-[4-(3-Benzyloxy-propoxy)-phenyl]-3-(naphthalin-2-ylmethoxy)-5-propoxypiperidin als farbloses Öl; MS : 540 $(M+H)^+$;

4) - aus dem (3RS,4SR,5SR)-4-[4-(3-Benzyloxy-propoxy)-phenyl]-5-butoxy-3-(naphthalin-2-ylmethoxy)-piperidin-1-carbonsäure tert-butyl ester mittels Chlorwasserstoff in Methanol das (3RS,4SR,5SR)-4-[4-(3-Benzyloxy-propoxy)-phenyl]-5-butoxy-3-(naphthalin-2-ylmethoxy)-piperidin als farbloses Öl; MS : 554 $(M+H)^+$;

5) - aus dem (3RS,4SR,5SR)-4-[4-(3-Benzyloxy-propoxy)-phenyl]-5-methoxy-3-(naphthalin-2-ylmethoxy)-piperidin-1-carbonsäure tert-butyl ester mittels Chlorwasserstoff in Methanol das (3RS,4SR,5SR)-4-[4-(3-Benzyloxy-propoxy)-phenyl]-5-methoxy-3-(naphthalin-2-ylmethoxy)-piperidin als farbloses Öl; MS : 512 $(M+H)^+$;

6) - aus dem Gemisch des (3RS,4RS,5SR)- und (3SR,4SR,5RS)-4-[4-(3-Benzyloxy-propoxy)-phenyl]-3-(naphthalin-2-ylmethoxy)-5-[2-[(RS)-tetrahydro-pyran-2-yloxy]-äthoxy]-piperidin-1-carbonsäure tert-butylesters **R**mittels Chlorwasserstoff in Methanol unter gleichzeitiger Abspaltung des Tetrahydropyranyl-Restes der 2-[(3RS,4RS,5SR)-4-[4-(3-Benzyloxy-propoxy)-phenyl]-3-(naphthalin-2-ylmethoxy)-piperidin-5-yloxy]-äthanol als farbloses Öl; MS : 542 $(M+H)^+$;

7) - aus dem Gemisch des (3RS,4SR,5SR)- und (3SR,4RS,5RS)-4-[4-(3-Benzyloxy-propoxy)-phenyl]-3-(naphthalin-2-ylmethoxy)-5-[3-[(RS)-tetrahydro-pyran-2-yloxy]-propoxy]-piperidin-1-carbonsäure tert-butylesters mittels Chlorwasserstoff in Methanol unter gleichzeitiger Abspaltung des Tetrahydropyranyl-Restes das 3-[(3RS,4SR,5SR)-4-[4-(3-Benzyloxy-propoxy)-phenyl]-3-(naphthalin-2-ylmethoxy)-piperidin-5-yloxy]-propan-1-ol als farbloses Öl; MS : 556 $(M+H)^+$;

8) - aus dem Gemisch des (3RS,4SR,5SR)- und (3SR,4RS,5RS)-4-[4-(3-Benzyloxy-propoxy)-phenyl]-3-(naphthalin-2-ylmethoxy)-5-[4-[(RS)-tetrahydro-pyran-2-yloxy] -butoxy] -piperidin- 1-carbonsäure tert-butylesters mittels Chlorwasserstoff in Methanol unter gleichzeitiger Abspaltung des Tetrahydropyranyl-Restes das 4-[(3RS,4SR,5SR)-4-[4-(3-Benzyloxy-propoxy)-phenyl]-3-(naphthalin-2-ylmethoxy)-piperidin-5-yloxy]-butan-1-ol als farbloses Öl; MS : 570 $(M+H)^+$.

9) - aus dem (3RS,4RS,5SR)-4-[4-(3-Benzyloxy-propoxy)-phenyl]-5-(2-morpholin-4-yl-äthoxy)-3-(naphthalin-2-ylmethoxy)-piperidin-1-carbonsäure tert-butyl ester mittels Chlorwasserstoff in Methanol das 4-{2-[(3RS,4RS,5SR)-4-[4-(3-Benzyloxy-propoxy)-phenyl]-3-(naphthalin-2-ylmethoxy)-piperidin-5-yloxy]-äthyl}-morpholin als farbloses Öl; MS : 611 $(M+H)^+$;

10) - aus dem (3RS,4RS)-3-(Naphthalin-2-ylmethoxy)-4-(4-{3-[2-(2-trimethylsilanyl-äthoxymethoxy)-benzyloxy]-propoxy}-phenyl)-piperidin-1-carbonsäure tert-butylester [Beispiel 108 (b)] mittels Chlorwasserstoff in Methanol unter gleichzeitiger Abspaltung des 2-Trimethylsilanyl-äthoxymethoxy-Restes das 2-(3-{4-[(3RS,4RS)-3-(Naphthalin-2-ylmethoxy)-piperidin-4-yl]-phenoxy}-propoxymethyl)-phenol als farbloses Öl; MS : 579 (M+H)+.

In analoger Weise wie in Beispiel 1 (g) beschrieben, jedoch bei einer Reaktionstemperatur von 50 °C und unter Verwendung eines großen Überschusses an Natriumhydrid und Alkylierungsreagenz, wurden die als Ausgangsverbindungen eingesetzten BOC-Derivate aus dem (3RS,4SR,5SR)-4-[4-(3-Benzyloxy-propoxy)-phenyl]-5-hydroxy-3-(naphthalin-2-ylmethoxy)-piperidin-1-carbonsäure tert-butylester [Beispiel 109 (a)] wie folgt erhalten:

(a) - Durch Alkylierung mit Äthylbromid der (3RS,4SR,5SR)-4-[4-(3-Benzyloxy-propoxy)-phenyl]-5-äthoxy-3-(naphthalin-2-ylmethoxy)-piperidin-1-carbonsäure tert-butyl ester als farbloses Öl; MS : 626 $(M+H)^+$;

(b) - durch Alkylierung mit 1-(2-Chloräthyl)-4-methyl-piperazin [Austr. J. Chem. 9 (1956), 89] der (3RS,4RS,5SR)-4-[4-(3-Benzyloxypropoxy)-phenyl)-5-[2-(4-methyl-piperazin-1-yl)-äthoxy]-3-(naphthalin-2-ylmethoxy)-piperidin-1-carbonsäure tert-butyl ester als farbloses Öl; MS: 724 $(M+H)^+$;

(c) - durch Alkylierung mit n-Propylbromid der (3RS,4SR,5SR)-4-[4-(3-Benzyloxy-propoxy)-phenyl]-3-(naphthalin-2-ylmethoxy)-5-propoxypiperidin-1-carbonsäure tert-butyl ester als gelbliches Öl; MS: 640 $(M+H)^+$;

(d) - durch Alkylierung mit n-Butylbromid der (3RS,4SR,5SR)-4-[4-(3-Benzyloxy-propoxy)-phenyl]-5-butoxy-3-(naphthalin-2-ylmethoxy)-piperidin-1-carbonsäure tert-butyl ester als gelbliches Öl; MS : 654 $(M+H)^+$;

(e) - durch Alkylierung mit Methyliodid der (3RS,4SR,5SR)-4-[4-(3-Benzyloxy-propoxy)-phenyl]-5-methoxy-3-(naphthalin-2-ylmethoxy)-piperidin-1-carbonsäure tert-butyl ester als gelbliches Öl; MS : 612 $(M+H)^+$;

(f) - durch Alkylierung mit rac-2-(2-Bromäthoxy)-tetrahydropyran [J. Amer. Chem. Soc. 70, 4187 (1948)] ein Gemisch des (3RS,4RS,5SR)- und (3SR,4SR,5RS)-4-[4-(3-Benzyloxy-propoxy)-phenyl]-3-(naphthalin-2-ylmethoxy)-5-[2-[(RS)-tetrahydro-pyran-2-yloxy]-äthoxy]-piperidin-1-carbonsäure tert-butylesters als farbloses Öl; MS: 743 $(M+NH_4)^+$.

(g) - durch Alkylierung mit rac-2-(3-Brom-propoxy)-tetrahydropyran [J. Chem. Soc. 1955, 1770] ein Gemisch des (3RS,4SR,5SR)- und (3SR,4RS,5RS)-4-[4-(3-Benzyloxy-propoxy)-phenyl]-3-(naphthalin-2-ylmethoxy)-5-[3-[(RS)-tetrahydro-pyran-2-yloxy]-propoxy]-piperidin-1-carbonsäure tert-butylesters als farbloses Öl; MS: 740 $(M+H)^+$.

(h) - durch Alkylierung mit rac-2-(4-Brom-butoxy)-tetrahydropyran [S.W.Baldwin et al., J.Org.Chem. 1985, 50, 4432-4439] ein Gemisch des (3RS,4SR,5SR)- und (3SR,4RS,5RS)-4-[4-(3-Benzyloxy-propoxy)-phenyl]-3-(naphthalin-2-ylmethoxy)-5- [4-[(RS)-tetrahydro-pyran-2-yloxy]-butoxy]-piperidin-1-carbonsäure tert-butylesters als farbloses Öl; MS: 771 $(M+NH_4)^+$.

(i) - durch Alkylierung mit 4-(2-Chloräthyl)-morpholin der (3RS,4RS,5SR)-4-[4-(3-Benzyloxy-propoxy)-phenyl]-5-(2-morpholin-4-yl-äthoxy)-3-(naphthalin-2-ylmethoxy)-piperidin-1-carbonsäure tert-butyl ester als farbloses Öl; MS : 711 $(M+H)^+$.

Beispiel 111

Durch Abspaltung der BOC-Gruppe und falls vorhanden gleichzeitig der Tetrahydropyranyl Schutzgruppe mittels Chlorwasserstoff in Methanol, analog Beispiel 22 (l), oder der BOC-Gruppe mittels Zinkbromid in Methylenchlorid, analog Beispiel 10 (b), wurden die folgenden Verbindungen erhalten:

1) - Aus dem (3RS,4SR,5RS)-4-[4-(3-Benzyloxy-propoxy)-phenyl]-5-hydroxy-3-(naphthalin-2-ylmethoxy)-piperidin-1-carbonsäure tert-butylester mittels Zinkbromid in Methylenchlorid das (3RS,4SR,5RS)-4-[4-(3-Benzyloxy-propoxy)-phenyl]-3-(naphthalin-2-ylmethoxy)-piperidin-5-ol als farbloser Festkörper; MS: 499 $(M+H)^+$;

2) - aus dem (RS)-4-[4-(3-Benzyloxy-propoxy)-phenyl]-3-(naphthalin-2-ylmethoxy)-3,6-dihydro-2H-pyridin-1-carbonsäure tert-butylester mittels Chlorwasserstoff in Methanol das (RS)-4-[4-(3-Benzyloxy-propoxy)-phenyl]-3-(naphthalin-2-ylmethoxy)-1,2,3,6-tetrahydro-pyridin in Form eines farblosen Öles; MS: 480 $(M+H)^+$;

3) - aus dem (3RS,4SR,5SR)-5-Amino-4-[4-(3-benzyloxy-propoxy)-phenyl]-3-(naphthalin-2-ylmethoxy)-piperidin-1-carbonsäure tert-butylester mittels Zinkbromid in Methylenchlorid das (3RS,4SR,5SR)-4-[4-(3-Benzyloxy-propoxy)-phenyl]-3-(naphthalin-2-ylmethoxy)-piperidin-5-ylamin in Form eines farblosen Festkörpers; MS: 497 $(M+H)^+$.

4) - aus dem Gemisch des (3RS,4RS,5SR)- und (3SR,4SR,5RS)-4-[4-(3-Benzyloxy-propoxy)-phenyl]-3-(quinolin-7-ylmethoxy)-5-[2-[(RS)-tetrahydro-pyran-2-yloxy]-äthoxy]-piperidin-1-carbonsäure tert-butylesters der 2-[4-(3RS,4RS,5SR)-[4-(3-Benzyloxy-propoxy)-phenyl]-3-(quinolin-7-ylmethoxy)-piperidin-5-yloxy]-äthanol in Form eines farblosen Öles; MS: 543 $(M+H)^+$;

5) - aus dem Gemisch des (3RS,4SR,5SR)- und (3SR,4RS,5RS)-4-[4-(3-Benzyloxy-propoxy)-phenyl]-3-(quinolin-7-ylmethoxy)-5-[3-[(RS)-tetrahydro-pyran-2-yloxy]-propoxy]-piperidin-1-carbonsäure tert-butylesters das 3-[4-(3R5,4SR,5SR)- [4-(3-Benzyloxy-propoxy)-phenyl]-3-(quinolin-7-ylmethoxy)-piperidin-5-yloxy]-propan-1-ol in

Form eines farblosen Öles; MS: 557 $(M+H)^+$;

6) - aus dem Gemisch des (3RS,4SR,5SR)- und (3SR,4RS,5RS)-4-[4-(3-Benzyloxy-propoxy)-phenyl]-3-(quinolin-7-ylmethoxy)-5-[4-[(RS)-tetrahydro-pyran-2-yloxy]-butoxy]-piperidin-1-carbonsäure tert-butylesters das 4-(4-(3RS,4SR,5SR)-[4-(3-Benzyloxy-propoxy)-phenyl]-3-(quinolin-7-ylmethoxy)-piperidin-5-yloxy]-butan-1-ol in Form eines farblosen Öles; MS: 571 $(M+H)^+$.

Die als Ausgangsverbindungen eingesetzten BOC-Derivate wurden wiefolgt erhalten:

(a) Eine Lösung von 850 mg (1.422 mMol) (3RS,4SR,5SR)-4-[4-(3-Benzyloxy-propoxy)-phenyl]-5-hydroxy-3-(naphthalin-2-ylmethoxy)-piperidin-1-carbonsäure tert-butylester [Beispiel 109 (a)] und 1.884 g (7.11 mMol) Triphenylphosphin in 170 ml trockenem Tetrahydrofuran wurde unter Rühren mit 274 µl (7.11 mMol) trockener Ameisensäure und einer Lösung von 1.238 g (7.11 mMol) Diäthylazodicarboxylat in 42.5 ml Tetrahydrofuran versetzt. Das Reaktionsgemisch wurde anschliessend 90 Stunden bei Raumtemperatur gerührt. Hierauf wurde es unter vermindertem Druck bei 40 °C eingedampft und der Rückstand mit einer Lösung aus 42.5 ml Methanol und 464 mg (7.11 mMol) Kaliumhydroxyd versetzt und 3 Stunden bei Raumtemperatur gerührt. Anschliessend wurde die Lösung mit 500 ml entionisiertem Wasser versetzt und das Gemisch vier mal mit je 200 ml Methylenchlorid extrahiert. Die vereinigten organischen Phasen wurden über Natriumsulfat getrocknet, unter vermindertem Druck bei 40 °C eingedampft und der Rückstand am Hochvakuum getrocknet. Der weisse kristalline Rückstand (4.2 g) wurde an Kieselgel unter Verwendung eines 4:1-Gemisches von Methylenchlorid und Essigsäureäthylester als Elutierungsmittel chromatographiert. Es wurden 560 mg (66 % d.Th) (3RS,4SR,5RS)-4-[4-(3-Benzyloxy-propoxy)-phenyl]-5-hydroxy-3-(naphthalin-2-ylmethoxy)-piperidin-1-carbonsäure tert-butylester in Form eines farblosen Festkörpers erhalten; MS: 598 $(M+H)^+$.

(b) Eine Lösung von 560 mg (0.937 mMol) (3RS,4SR,5RS)-4-[4-(3-Benzyloxy-propoxy)-phenyl]-5-hydroxy-3-(naphthalin-2-ylmethoxy)-piperidin-1-carbonsäure tert-butylester und 273 mg (1.031 mMol) Triphenylphosphin in 20 ml trockenem Tetrahydrofuran wurde unter Rühren mit 160.2 µl (1.031 mMol) Diäthylazodicarboxylat und zehn Minuten später mit einer Lösung von 319.8 µl (1.405 mMol) Diphenylphosphorylazid in 2 ml Tetrahydrofuran versetzt. Dieses Gemisch wurde 72 Stunden bei Raumtemperatur gerührt, dann unter vermindertem Druck bei 40 °C eingedampft und der Rückstand am Hochvakuum getrocknet. Der gelbe ölige Rückstand wurde an Kieselgel unter Verwendung eines 4:1-Gemisches von n-Hexan und Essigsäureäthylester als Eluierungsmittel chromatographiert. Es wurden 210 mg (36 % d. Th) (3RS,4SR,5SR)-5-Azido-4-[4-(3-benzyloxypropoxy)-phenyl]-3-(naphthalin-2-ylmethoxy)-piperidin-1-carbonsäure tert-butylester in Form eines farblosen Öles erhalten [MS: 623 $(M+H^+)$]. Als weiteres Produkt wurden 180 mg (33.1 % d.Th) (RS)-4-[4-(3-Benzyloxy-propoxy)-phenyl]-3-(naphthalin-2-ylmethoxy)-3,6-dihydro-2Hpyridin-1-carbonsäure tert-butylester ebenfalls als farbloses Öl erhalten; MS: 580 $(M+H)^+$.

(c) Eine Lösung von 50 mg (0.0803 mMol dem (3RS,4SR,5SR)-5-Azido-4- [4-(3-benzyloxy-propoxy)-phenyl] -3-(naphthalin-2-ylmethoxy)-piperidin-1-carbonsäure tert-butylester in 0.36 ml trockenem Tetrahydrofuran wurde unter Rühren mit 21 mg (0.0793 mMol) Triphenylphosphin gelöst in 0.36 ml trockenem Tetrahydrofuran versetzt. Hierauf wurde vier Stunden bei Raumtemperatur gerührt (ca 50 % Umsatz) und anschliessend nochmals mit 10.6 mg (0.040 mM) Triphenylphosphin versetzt und schliesslich weitere 24 h bei Raumtemperatur gerührt. Dann wurde mit 2 µl (0.111 mM) entionisiertem Wasser versetzt und eine Stunde bei Raumtemperatur gerührt. Anschliessend wurde unter vermindertem Druck bei 40 °C eingedampft, der Rückstand in Äther aufgenommen und gegen Wasser extrahiert. Die organische Phase wurde über Natriumsulfat getrocknet und das Filtrat eingedampft. Der farblose ölige Rückstand (110 mg) wurde an Kieselgel unter Verwendung eines 4:1-Gemisches von n-Hexan und Essigsäureäthylester als Eluierungsmittel chromatographiert. Es wurden 30 mg (63 % d. Th) (3RS,4SR,5SR)-5-Amino-4-[4-(3-benzyloxy-propoxy)-phenyl]-3-(naphthalin-2-ylmethoxy)-piperidin-1-carbonsäure tert-butyl ester in Form eines farblosen Festkörpers erhalten; MS: 597 $(M+H)^+$.

In analoger Weise wie in Beispiel 1 (g) beschrieben, jedoch bei einer Reaktionstemperatur von 50 °C, wurden aus (3RS,4SR,5SR)-4-[4-(3-Benzyloxy-propoxy)-phenyl]-5-hydroxy-3-(quinolin-7-ylmethoxy)-piperidin-1-carbonsäure tert-butylester [Beispiel 109 (k)] die folgenden BOC-Derivate wie folgt erhalten:

(d) - Durch Alkylierung mit rac-2-(2-Bromäthoxy)-tetrahydropyran [J. Amer. Chem. Soc. 70, 4187 (1948)] das Gemisch des (3RS,4RS,5SR)- und (3SR,4SR,5RS)-4-[4-(3-Benzyloxy-propoxy)-phenyl]-3-(quinolin-7-ylmethoxy)-5-[2-[(RS)-tetrahydro-pyran-2-yloxy]-äthoxy]-piperidin-1-carbonsäure tert-butyl esters als farbloses Öl; MS: 727 $(M+H)^+$;

(e) - durch Alkylierung mit rac-2-(3-Brom-propoxy)-tetrahydropyran [J. Chem. Soc. 1955, 1770] das Gemisch des (3RS,4SR,5SR)- und (3SR,4RS,5RS)-4-[4-(3-Benzyloxy-propoxy)-phenyl)-3-(quinolin-7-ylmethoxy)-5-[3-[(RS)-tetrahydro-pyran-2-yloxy]-propoxy]-piperidin-1-carbonsäure tert-butyl esters als farbloses Öl; MS: 741 $(M+H)^+$;

(f) - durch Alkylierung mit rac-2-(4-Brom-butoxy)-tetrahydropyran [S.W.Baldwin et al., J.Org.Chem. 1985, 50, 4432-4439] das Gemisch des (3RS,4SR,5SR)- und (3SR,4RS,5RS)-4-[4-(3-Benzyloxy-propoxy)-phenyl]-3-(quinolin-7-ylmethoxy)-5-[4-[(RS)-tetrahydro-pyran-2-yloxy]-butoxy]-piperidin-1-carbonsäure tert-butyl esters als farbloses Öl; MS: 755 $(M+H)^+$.

Beispiel 112

Durch Abspaltung der BOC-Gruppe mittels Zinkbromid in Methylenchlorid, analog Beispiel 10 (b), wurden die folgenden Verbindungen erhalten:

1) - Aus dem (3R,4s,5S)-3,5-Bis-(4-methoxy-benzyloxy)-4-{4-[3-(2-methoxy-benzyloxy)-propoxy]-phenyl}-piperidin-1-carbonsäure tert-butylester das (3R,4s,5S)-4-[4-[3-(2-Methoxy-benzyloxy)-propoxy]-phenyl]-3,5-bis-(naphthalin-2-ylmethoxy)-piperidin als farbloses Öl; MS : 628 $(M+H)^+$;

2) - aus dem (3RS,4SR,5SR)-5-Hydroxy-3-(4-methoxy-benzyloxy)-4-{4-[3-(2-methoxy-benzyloxy)-propoxy]-phenyl}-piperidin-1-carbonsäure tert-butylester das (3RS,4SR,5SR)-3-(4-Methoxy-benzyloxy)-4-{4-[3-(2-methoxy-benzyloxy)-propoxy]-phenyl}-piperidin-5-ol als farbloses Öl; MS: 508 $(M+H)^+$;

3) - aus dem (3R,4s,5S)-4-[4-[3-(2-Methoxy-benzyloxy)-propoxy]-phenyl]-3,5-bis-(pyridin-2-ylmethoxy)-piperidin-1-carbonsäure tert-butylester das (3R,4s,5S)-2-[5-(Pyridin-2-ylmethoxy-4-[4-[3-(2-methoxybenzyloxy)-propoxy]-phenyl]-piperidin-3-yloxymethyl]-pyridin als farbloses Öl; MS : 570 $(M+H)^+$;

4) - aus dem (3RS,4SR,5SR)-5-Hydroxy-4-{4-[3-(2-methoxy-benzyloxy)-propoxy]-phenyl}-3-(pyridin-2-ylmethoxy)-piperidin-1-carbonsäure tert-butylester das (3RS,4SR,5SR)-4-[4-[3-(2-Methoxy-benzyloxy)-propoxy]-phenyl]-3-(pyridin-2-ylmethoxy)-piperidin-5-ol als farbloses Öl; MS : 479 $(M+H)^+$;

5) - aus dem (3R,4s,5S)-4-[4-[3-(2-Methoxy-benzyloxy)-propoxy]-phenyl]-3,5-bis-(pyridin-3-ylmethoxy)-piperidin-1-carbonsäure tert-butylester das (3R,4s,5S)-3-[5-(Pyridin-3-ylmethoxy-4-[4-[3-(2-methoxybenzyloxy)-propoxy]-phenyl]-piperidin-3-yloxymethyl]-pyridin als farbloses Öl; MS : 570 $(M+H)^+$;

6) - aus dem (3RS,4SR,5SR)-5-Hydroxy-4-{4-[3-(2-methoxy-benzyloxy)-propoxy]-phenyl}-3-(pyridin-3-ylmethoxy)-piperidin-1-carbonsäure tert-butylester das (3RS,4SR,5SR)-4-[4-[3-(2-Methoxy-benzyloxy)-propoxy]-phenyl]-3-(pyridin-3-ylmethoxy)-piperidin-5-ol als farbloses Öl; MS : 479 $(M+H)^+$;

7) - aus dem (3R,4s,5S)-4-[4-[3-(2-Methoxy-benzyloxy)-propoxy]-phenyl]-3,5-bis-(pyridin-4-ylmethoxy)-piperidin-1-carbonsäure tert-butylester das (3R,4s,5S)-4-[5-(Pyridin-4-ylmethoxy-4-[4-[3-(2-methoxybenzyloxy)-propoxy]-phenyl)-piperidin-3-yloxymethyl]-pyridin als farbloses Öl; MS : 570 $(M+H)^+$;

8) - aus dem (3RS,4SR,5SR)-5-Hydroxy-4-{4-[3-(2-methoxy-benzyloxy)-propoxy]-phenyl}-3-(pyridin-4-ylmethoxy)-piperidin-1-carbonsäure tert-butylester das (3RS,4SR,5SR)-4-[4-[3-(2-Methoxy-benzyloxy)-propoxy]-phenyl]-3-(pyridin-4-ylmethoxy)-piperidin-5-ol als farbloses Öl; MS: 479 $(M+H)^+$;

9) - aus dem (3RS,4SR,5SR)-5-Hydroxy-4-[4-[3-(2-methoxy-benzyloxy)-propoxy]-phenyl]-3-[7-[2-(4-methyl-piperazin-1-yl)-äthoxy] -naphthalin-2-ylmethoxy]-piperidin-1-carbonsäure tert-butylester das 1-[2-[7-[(3RS,4SR,5SR)-5-Hydroxy-4-[4-[3-(2-methoxy-benzyloxy)-propoxy]-piperidin-3-yloxymethyl]-naphthalin-2-yloxy]-äthyl]-4-methyl-piperazin in Form eines amorphen, farblosen Festkörpers; MS: 670 $(M+H)^+$;

10) - aus dem (3R,4s,5S)-3,5-Bis-(1,4-dimethoxy-naphthalin-2-ylmethoxy)-4-[4-[3-(2-methoxy-benzyloxy)-propoxy]-phenyl]-piperidin-1-carbonsäure tert-butylester mittels Zinkbromid in Methylenchlorid das (3R,4s,5S)-3,5-Bis-(1,4-dimethoxy-naphthalin-2-ylmethoxy)-4-[4-[3-(2-methoxy-benzyloxy)-propoxy]-phenyl]-piperidin als farbloses Wachs; MS : 788 $(M+H)^+$;

11) - aus dem (3RS,4SR,5SR)-3-(1,4-Dimethoxy-naphthalin-2-ylmethoxy)-5-hydroxy-4-[4-[3-(2-methoxy-benzyloxy)-propoxy]-phenyl]-piperidin-1-carbonsäure tert-butylester mittels Zinkbromid in Methylenchlorid das (3RS,4SR,5SR)-3-(1,4-Dimethoxy-naphthalin-2-ylmethoxy)-4-[4-[3-(2-methoxy-benzyloxy)-propoxy]-phenyl]-piperidin-5-ol als farbloses Öl; MS : 588 $(M+H)^+$.

Die als Ausgangsverbindungen eingesetzten BOC-Derivate wurden wie folgt erhalten:

(α) In analoger Weise wie in Beispiel 44 (e) beschrieben, wurde durch Alkylierung des (3R,4s,5S)-3,5-Dihydroxy-4-(4-hydroxy-phenyl)-piperidin-1-carbonsäure tert-butylesters [Beispiel 109 (η)] mit 2-Methoxybenzyl-3-chlorpropyläther [Beispiel 120 (g)] der (3R,4s,5S)-3,5-Dihydroxy-4-{4-[3-(2-methoxy-benzyloxy)-propoxy]-phenyl}-piperidin-1-carbonsäure tert-butylester in Form eines amorphen, farblosen Festkörpers erhalten; MS: 505 $(M+NH_4)^+$.
(β) In analoger Weise wie in Beispiel 1 (g) beschrieben, wurden durch Alkylierung des (3R,4s,5S)-3,5-Dihydroxy-4-{4-[3-(2-methoxy-benzyloxy)-propoxy]-phenyl}-piperidin-1-carbonsäure tert-butylesters unter Verwendung von einem Äquivalent eines benzylischen Halogenides in etwa gleichen Mengenanteilen unverändertes Ausgangsmaterial sowie die entsprechenden mono- und dialkylierten BOC-Derivate erhalten. Diese Gemische wurden anschließend mittels Chromatographie aufgetrennt:

(a) - Durch Alkylierung mit 4-Methoxy-benzylchlorid der (3R,4s,5S)-3,5-Bis-(4-methoxy-benzyloxy)-4-{4-[3-(2-methoxy-benzyloxy)-propoxy]-phenyl}-piperidin-1-carbonsäure tert-butylester, MS : 746 $(M+NH_4)^+$, und der (3RS,4SR,5SR)-5-Hydroxy-3-(4-methoxy-benzyloxy)-4-{4-[3-(2-methoxy-benzyloxy)-propoxy]-phenyl}-piperidin-1-carbonsäure tert-butylester, MS : 626 $(M+NH_4)^+$, jeweils als amorpher, farbloser Festkörper;

(b) - durch Alkylierung mit 2-Chlormethyl-pyridin Hydrochlorid und entsprechendem Basenüberschuss der (3R,4s,5S)-4-[4-[3-(2-Methoxybenzyloxy)-propoxy]-phenyl]-3,5-bis-(pyridin-2-ylmethoxy)-piperidin-1-carbonsäure tert-butylester, MS : 670 $(M+H)^+$, und der (3RS,4SR,5SR)-5-Hydroxy-4-{4-[3-(2-methoxy-benzyloxy)-propoxy]-phenyl}-3-(pyridin-2-ylmethoxy)-piperidin-1-carbonsäure tert-butylester, 579 $(M+H)^+$, jeweils als amorpher, farbloser Festkörper;

(c) - durch Alkylierung mit 3-Chlormethyl-pyridin Hydrochlorid und entsprechendem Basenüberschuss der (3R,4s,5S)-4-[4-[3-(2-Methoxybenzyloxy)-propoxy]-phenyl]-3,5-bis-(pyridin-3-ylmethoxy)-piperidin-1-carbonsäure tert-butylester, MS : 670 $(M+H)^+$, und der (3RS,4SR,5SR)-5-Hydroxy-4-{4-[3-(2-methoxy-benzyloxy)-propoxy]-phenyl}-3-(pyridin-3-ylmethoxy)-piperidin-1-carbonsäure tert-butylester, 579 $(M+H)^+$, jeweils als amorpher, farbloser Festkörper;

(d) - durch Alkylierung mit 4-Chlormethyl-pyridin Hydrochlorid und entsprechendem Basenüberschuss der (3R,4s,5S)-4-[4-[3-(2-Methoxybenzyloxy)-propoxy]-phenyl]-3,5-bis-(pyridin-4-ylmethoxy)-piperidin-1-carbonsäure tert-butylester, MS : 670 $(M+H)^+$, und der (3RS,4SR,5SR)-5-Hydroxy-4-{4-[3-(2-methoxy-benzyloxy)-propoxy]-phenyl}-3-(pyridin-4-ylmethoxy)-piperidin-1-carbonsäure tert-butylester, 579 $(M+H)^+$, jeweils als amorpher, farbloser Festkörper,

(e) - durch Alkylierung von (3R,4s,5S)-3,5-Dihydroxy-4-{4-[3-(2-methoxy-benzyloxy)-propoxy]-phenyl}-piperidin-1-carbonsäure tert-butylester [Beispiel 112 (α)] mit 2-Chlormethyl-7-(2-trimethylsilanyl-äthoxymethoxy)-naphthalin [Beispiel 6 (u)] der (3RS,4SR,5SR)-5-Hydroxy-4-[4-[3-(2-methoxy-benzyloxy)-propoxy]-phenyl]-3-[7-(2-trimethylsilanyl-äthoxymethoxy)-naphthalin-2-ylmethoxy]-piperidin-1-carbonsäure tert-butylester als hellgelbes Öl; MS: 774 $(M+H)^+$. Dieser wurde hierauf in analoger Weise wie in Beispiel 95 (b) beschrieben durch Abspaltung der SEM-Schutzgruppe zu dem (3RS,4SR,5SR)-5-Hydroxy-4-[4-[3-(2-methoxy-benzyloxy)-propoxy]-phenyl]-3-(7-hydroxynaphthalin-2-ylmethoxy)-piperidin-1-carbonsäure tert-butylester [gelbes Öl; MS: 644 $(M+H)^+$] umgesetzt, dessen Alkylierung mit 1-(2-Chlor-äthyl)-4-methyl-piperazin Hydrochlorid (1:2) [Chim. Ther. 4, 283 (1969)] in analoger Weise wie in Beispiel 90 (n) beschrieben den (3RS,4SR,5SR)-5-Hydroxy-4-[4-[3-(2-methoxy-benzyloxy)-propoxy]-phenyl]-3-[7- [2-(4-methyl-piperazin-1-yl)-äthoxy]-naphthalin-2-ylmethoxy]-piperidin-1-carbonsäure tert-butylester als hellbraunes Öl ergab; MS: 770 $(M+H)^+$;

(f) - durch Alkylierung von (3R,4s,5S)-3,5-Dihydroxy-4-{4-[3-(2-methoxy-benzyloxy)-propoxy]-phenyl}-piperidin-1-carbonsäure tert-butylester [Beispiel 112 (α)] mit 2-Chlormethyl-1,4-dimethoxynaphthalin [J.Org.Chem. (1983), 48(19),3265-3268] der (3R,4s,5S)-3,5-Bis-(1,4-dimethoxy-naphthalin-2-ylmethoxy)-4-[4-[3-(2-methoxy-benzyloxy)-propoxy]-phenyl]-piperidin-1-carbonsäure tert-butylester, MS : 906 $(M+H)^+$, als farbloser Schaumundder(3RS,4SR,5SR)-3-(1,4-Dimethoxy-naphthalin-2-ylmethoxy)-5-hydroxy-4-[4-[3-(2-methoxybenzyloxy)-propoxy]-phenyl]-piperidin-1-carbonsäure tert-butylester , MS :705 $(M+NH_4)^+$, als farbloses Öl.

Beispiel 113

Durch Abspaltung der BOC-Gruppe mittels Zinkbromid in Methylenchlorid, analog Beispiel 10 (b), wurden die folgenden Verbindungen erhalten:

1) - Aus dem (3RS,4SR,5SR)-4-[4-(3-Benzyloxy-propoxy)-phenyl]-5-hydroxy-3-(4-hydroxy-benzyloxy)-piperidin-1-carbonsäure tert-butylester das (3RS,4SR,5SR)-4-[4-(3-Benzyloxy-propoxy)-phenyl]-3-(4-hydroxybenzyloxy)-piperidin-5-ol als farbloses Öl; MS : 464 $(M+H)^+$;

2) - aus dem (3R,4s,5S)-4-[4-(3-Benzyloxy-propoxy)-phenyl]-3,5-bis-(4-hydroxy-benzyloxy)-piperidin-1-carbonsäure tert-butylester das 4-[(3R,4s,5S)-5-(4-Hydroxybenzyloxy)-4-[4-(3-benzyloxy-propoxy)-phenyl]-piperidin-3-yloxymethyl}-phenol als farbloses Öl; MS : 570 $(M+H)^+$.

Die als Ausgangsverbindungen eingesetzten BOC-Derivate wurden wie folgt erhalten:

(a) (α) In analoger Weise wie in Beispiel 1 (g) beschrieben, wurden durch Alkylierung des (3R,4s,5S)-4-[4-(3-Benzyloxy-propoxy)-phenyl]-3,5-dihydroxy-piperidin-1-carbonsäure tert-butylesters [Beispiel 109 (θ)] mit 1-Chlormethyl-4-(2-trimethylsilanyl-äthoxymethoxy)-benzol der (3RS,4SR,5SR)-4-[4-(3-Benzyloxy-propoxy)-phenyl]-5-hydroxy-3-[4-(2-trimethylsilanyl-äthoxymethoxy)-benzyloxy]-piperidin-1-carbonsäure tert-butylester, $R_f$: 0.33 ($SiO_2$, n-Hexan:Essigsäureäthylester = 2:1),.und der (3R,4s,55)-4-[4-(3-Benzyloxy-propoxy)-phenyl]-3,5-bis-[4-(2-trimethylsilanyl-äthoxymethoxy)-benzyloxy]-piperidin-1-carbonsäure tert-butylester, $R_f$: 0.64 ($SiO_2$, n-Hexan:Essigsäureäthylester = 2:1), jeweils als amorpher, farbloser Festkörper erhalten.

(a) (β) Eine Lösung von 2.16 g (3.113 mMol) (3RS,4SR,5SR)-4-[4-(3-Benzyloxy-propoxy)-phenyl]-5-hydroxy-3-[4-(2-trimethylsilanyl-äthoxymethoxy)-benzyloxy]-piperidin-1-carbonsäure tert-butylester in 50 ml Methanol wurde mit 2.02 ml (4.046 mMol) einer wasserfreien 2 M Chlorwasserstofflösung in Methanol versetzt und zwei Stunden bei Raumtemperatur gerührt. Anschliessend wurde mit 100 ml eines 95:5-Gemisches aus Methylenchlorid und Methanol (Extrahiert gegen 5-Volumen-% einer gesättigten wässerigen Ammoniaklösung) versetzt und die Lösung am Rotationsverdampfer bei 30 °C eingedampft. Der weisse Festkörper (2.17 g) wurde hierauf an Kieselgel unter Verwendung eines 4:1-Gemisches von Methylenchlorid und Essigsäureäthylester als Eluierungsmittel chromatographiert. Es wurden 780 mg (45 % d.Th) (3RS,4SR,5SR)-4-[4-(3-Benzyloxy-propoxy)-phenyl]-5-hydroxy-3-(4-hydroxy-benzyloxy)-piperidin-1-carbonsäure tert-butylester in Form eines farblosen Öles erhalten; MS: 581 $(M+NH4)^+$.

b) Eine Lösung von 1.1 g (1.18 mMol) (3R,4s,5S)-4-[4-(3-Benzyloxypropoxy)-phenyl)-3,5-bis-[4-(2-trimethylsilanyl-äthoxymethoxy)-benzyloxy]-piperidin-1-carbonsäure tert-butylester in 20 ml Methanol wurde mit 1.30 ml (2.60 mMol) einer wasserfreien 2 M Chlorwasserstofflösung in Methanol versetzt und 70 Minuten bei Raumtemperatur gerührt. Anschliessend wurde mit 50 ml eines 95:5-Gemisches aus Methylenchlorid und Methanol (Extrahiert gegen 5-Volumen-% einer gesättigten wässerigen Ammoniaklösung) versetzt und die Lösung am Rotationsverdampfer bei 30 °C eingedampft. Der weisse Festkörper (920 mg) wurde hierauf an Kieselgel unter Verwendung eines 4:1-Gemisches von Methylenchlorid und Essigsäureäthylester als Eluierungsmittel chromatographiert. Es wurden 300 mg (38 % d.Th) (3R,4s,5S)-4-[4-(3-Benzyloxy-propoxy)-phenyl]-3,5-bis-(4-hydroxy-benzyloxy)-piperidin-1-carbonsäure tert-butylester in Form eines farblosen Öles erhalten; $R_f$: 0.26 ($SiO_2$, n-Hexan:Essigsäureäthylester=1:1).

Das als Alkylierungsreagenz verwendete 1-Chlormethyl-4-(2-trimethylsilanyl-äthoxymethoxy)-benzol wurde hergestellt, indem in Analogie zu Beispiel 5 (a) -(c) 4-Hydroxybenzoesäuremethylester durch Einführung der SEM-Gruppe zu 4-(2-Trimethylsilanyläthoxymethoxy)-benzoesäuremethylester umgesetzt wurde. Die anschliessende Reduktion mit Lithiumaluminiumhydrid ergab das [4-(2-Trimethylsilanyläthoxy-methoxy)-phenyl]-methanol und dessen Chlorierung 1-Chlormethyl-4-(2-trimethylsilanyl-äthoxymethoxy)-benzol als farbloses Öl; MS : 272 $(M)^+$.

Beispiel 114

In Analogie zu dem im Beispiel 1 (e) beschriebenen Verfahren wurden durch Abspaltung der SEM-Gruppe mittels Tetrabutylammoniumfluorid in Tetrahydrofuran die folgenden Verbindungen erhalten:

1) - Aus dem (3RS,4RS)-4-(4-Fluor-phenyl)-3-[4-(2-morpholin-4-yl-äthoxy)-naphthalin-2-ylmethoxy]-piperidin-1-carbonsäure 2-trimethylsilyläthylester das (3RS,4RS)-4-(4-Fluor-phenyl)-3-[4-(2-morpholin-4-yl-äthoxy)-naphthalin-2-ylmethoxy]-piperidin als farbloser Festkörper; MS : 465 $(M)^+$;

2) - aus dem (3RS,4RS)-4-(4-Fluor-phenyl)-3-[8-(3-methoxy-propoxy)-naphthalin-2-ylmethoxy]-piperidin-1-carbonsäure 2-trimethylsilyläthylester das (3RS,4RS)-4-(4-Fluor-phenyl)-3-[8-(3-methoxypropoxy)-naphthalin-2-ylmethoxy]-piperidin als farbloser Festkörper; MS : 424 $(M)^+$;

3) - aus dem (3RS,4RS)-4-(4-Fluor-phenyl)-3-[5-(3-methoxy-propoxy)-naphthalin-2-ylmethoxy]-piperidin-1-carbonsäure 2-trimethylsilyläthylester das (3RS,4RS)-4-(4-Fluor-phenyl)-3-[5-(3-methoxy-propoxy)-naphthalin-2-ylmethoxy]-piperidin als farbloser Festkörper; MS : 424 $(M)^+$;

4) - aus dem (3RS,4RS)-4-(4-Fluor-phenyl)-3-[7-(3-methoxy-propoxy)-naphthalin-2-ylmethoxy]-piperidin-1-carbonsäure 2-trimethylsilyläthylester das (3RS,4RS)-4-(4-Fluor-phenyl)-3- [7-(3-methoxy-propoxy)-naphthalin-2-ylmethoxy]-piperidin als farbloser Festkörper; MS : 424 $(M)^+$.

Die als Ausgangssubstanzen eingesetzten SEM-Derivate wurden wie folgt hergestellt:

(a) - Aus dem (3RS,4RS)-4-(4-Fluorphenyl)-3-(4-hydroxy-naphthalin-2-ylmethoxy)-piperidin-1-carbonsäure 2-trimethyl- silyläthylester [Beispiel 5 (g)] und 4-(2-Chloräthyl)-morpholin Hydrochlorid der (3RS,4RS)-4-(4-Fluor-phenyl)-3-[4-(2-morpholin-4-yl-äthoxy)-naphthalin-2-ylmethoxy]-piperidin-1-carbonsäure 2-trimethylsilyläthylester als gelbes Öl; MS : 609 $(M)^+$;

(b) - aus dem (3RS,4RS)-4-(4-Fluorphenyl)-3-(8-hydroxy-naphthalin-2-ylmethoxy)-piperidin-1-carbonsäure 2-trimethylsilyläthylester [Beispiel 6 (dd)] und 1-Chlor-3-methoxypropane der (3RS,4RS)-4-(4-Fluor-phenyl)-3-[8-(3-methoxy-propoxy)-naphthalin-2-ylmethoxy]-piperidin-1-carbonsäure 2-trimethylsilyläthylester als leicht gelbes Öl; MS : 568 $(M)^+$;

(c) - aus dem (3RS,4RS)-4-(4-Fluorphenyl)-3-(5-hydroxy-naphthalin-2-ylmethoxy)-piperidin-1-carbonsäure 2-trimethylsilyläthylester [Beispiel 6 (l)] und 1-Chlor-3-methoxypropane der (3RS,4RS)-4-(4-Fluor-phenyl)-3-[5-(3-methoxy-propoxy)-naphthalin-2-ylmethoxy]-piperidin-1-carbonsäure 2-trimethylsilyläthylester als farbloses Harz, der ohne weitere Reinigung und Charakterisierung in der nächsten Stufe eingesetzt wurde;

(d) - aus dem (3RS,4RS)-4-(4-Fluorphenyl)-3-(7-hydroxy-naphthalin-2-ylmethoxy)-piperidin-1-carbonsäure 2-trimethylsilyläthylester [Beispiel 6 (x)] und 1-Chlor-3-methoxypropan der (3RS,4RS)-4-(4-Fluor-phenyl)-3-[7-(3-methoxy-propoxy)-naphthalin-2-ylmethoxy]-piperidin-1-carbonsäure 2-trimethylsilyläthylester als farbloses Harz, der ohne weitere Reinigung und Charakterisierung in der nächsten Stufe eingesetzt wurde.

Beispiel 115

In analoger Weise wie in Beispiel 5 beschrieben, wurde das (3RS, 4RS)-4-(4-Fluorphenyl)-3-[4-[3-hydroxy-benzyloxy]-naphthalin-2-ylmethoxy]-piperidin wie folgt erhalten:

(a) In analoger Weise wie in Beispiel 5 (a)-(d) beschrieben, wurde zunächst aus dem 3-Hydroxybenzoesäureäthylester durch Einführung der SEM-Gruppe der 3-(2-Trimethylsilyläthoxymethoxy)-benzoesäureäthylester als farbloses Öl erhalten; MS 238 $[M-(C_2H_4 + CH_2O)]^+$. Die sich anschließende Reduktion ergab das [3-(2-Trimethylsilyläthoxy-methoxy)-phenyl]-methanol, MS : 196 $[M-(C_2H_4 + CH_2O)]^+$, als farbloses Öl, dessen Chlorierung das 1-Chlormethyl-3-(2-trimethylsilyl-äthoxymethoxy)-benzol als farbloses Öl lieferte; MS : 214, 216 $[M-(C_2H_4 + CH_2O)]^+$. Die darauffolgende Alkylierung des (3RS,4RS)-4-(4-Fluorphenyl)-3-(4-hydroxy-naphthalin-2-ylmethoxy)-piperidin-1-carbonsäure 2-trimethyl-silyläthylesters [Beispiel 5 (g)] mit 1-Chlormethyl-3-(2-trimethylsilyläthoxymethoxy)-benzol lieferte den (3RS, 4RS)-4-(4-Fluorphenyl)-3-[4-[3-(2-trimethylsilyl-äthoxymethoxy)-benzyloxy]-naphthalin-2-ylmethoxy]-piperidin-1-carbonsäure 2-trimethylsilyläthylester als leicht gelbes Öl; MS : 749 $(M+NH_4)^+$.

(b) Aus dem (3RS, 4RS)-4-(4-Fluorphenyl)-3-[4-[3-(2-trimethylsilyl-äthoxymethoxy)-benzyloxy]-naphthalin-2-ylmethoxy]-piperidin-1-carbonsäure 2-trimethylsilyläthylester wurde, in Analogie zu dem im Beispiel 1 (e) beschriebenen Verfahren, durch Spaltung des 2-Trimethylsilyläthylcarbamats mit Tetrabutylammoniumfluorid in Tetrahydrofuran das (3RS, 4RS)-4-(4-Fluorphenyl)-3-[4-[3-(2-trimethylsilyl-äthoxymethoxy)-benzyloxy]-naphthalin-2-ylmethoxy]-piperidin, MS : 588 $(M+H)^+$, als leicht gelbes Öl erhalten, aus dem durch Abspaltung der SEM-Gruppe mittels einer 2 N Lösung von Chlorwasserstoff in Methanol, analog zu dem in Beispiel 5 (g) beschriebenen Verfahren, das (3RS, 4RS)-4-(4-Fluorphenyl)-3-[4-[3-hydroxy-benzyloxy]-naphthalin-2-ylmethoxy]-piperidin als farbloser Festkörper erhalten wurde; MS : 458 $(M+H)^+$.

Beispiel 116

In analoger Weise wie in Beispiel 10 (b) beschrieben, wurde durch Abspaltung der BOC-Gruppe mittels Zinkbromid in Methylenchlorid die folgendeVerbindung erhalten:

- Aus dem (3RS,4RS)-3-(Naphthalin-2-ylmethoxy)-4-[4-[2-(5-phenyl-[1,3,4]oxadiazol-2-yl)-äthyl]-phenyl]-piperidin-1-carbonsäure tert-butylester mittels Zinkbromid in Methylenchlorid das (3RS,4RS)-3-(Naphthalin-2-ylmethoxy)-4-[4-[2-(5-phenyl-[1,3,4]oxadiazol-2-yl)-äthyl]-phenyl]-piperidin als farbloser Festkörper; MS : 490 (M+H).

Das als Ausgangssubstanz eingesetzte BOC-Derivat wurde wie folgt hergestellt:

(a) Aus dem (3RS,4RS)-4-[4-(2-Carboxy-äthyl)-phenyl]-3-(naphthalin-2-ylmethoxy)piperidin-1-carbonsäure tert-butylester [Beispiel 86 (ll)] wurde durch Kondensation mit Benzoesäure-hydrazid in Gegenwart von EDC, in analoger Weise wie in Beispiel 38 beschrieben, der (3RS,4RS)-4-[4-[3-(N'-Benzoyl-hydrazino)-3-oxo-propyl]-phenyl]-3-(naphthalin-2-ylmethoxy)-piperidin-1-carbonsäure tert-butylester als farbloses Harz erhalten; MS : 608 $(M+H)^+$.

(b) Eine Lösung von 106 mg (0.174 mMol) (3RS,4RS)-4-[4-[3-(N'-Benzoylhydrazino)-3-oxo-propyl]-phenyl]-3-(naphthalin-2-ylmethoxy)-piperidin-1-carbonsäure tert-butylester in 1.5 ml Hexamethyldisilazan wurde mit 39 µl (0.039 mMol) einer 1 M Lösung von Tetrabutylammoniumfluorid in Tetrahydrofuran versetzt und 20 Stunden lang zum Rückfluß erhitzt. Zur Aufarbeitung wurde das Reaktionsgemisch mit 50 ml eines 1:1-Gemisches von Methylenchlorid und Wasser versetzt, anschließend die organische Phase abgetrennt und die wäßrige Phase zweimal mit je 25 ml Methylenchlorid nachextrahiert. Die vereinigten organischen Phasen wurden über Natriumsulfat getrocknet und unter vermindertem Druck eingedampft. Das Rohprodukt wurde mittels Flash-Chromatographie an Kieselgel mit einem 7:3-Gemisch von Hexan und Essigester als Eluierungsmittel gereinigt. Es wurden 70 mg (68 % d.Th) (3RS,4RS)-3-(Naphthalin-2-ylmethoxy)-4-[4-[2-(5-phenyl-[1,3,4)oxadiazol-2-yl)-äthyl]-phenyl]-piperidin-1-carbonsäure tert-butylester als farbloser Festkörper erhalten; MS : 590 $(M+H)^+$.

Beispiel 117

In analoger Weise wie in Beispiel 22 (l) beschrieben, wurde durch Abspaltung der BOC-Gruppe mittels Chlorwasserstoff in Methanol die folgende Verbindung erhalten:

- Aus dem (3RS,4RS)-4-[4-(3-Benzyloxy-propyl)-phenyl]-3-(naphthalin-2-ylmethoxy)-piperidin-1-carbonsäure tert-butylester das (3RS,4RS)-4-[4-(3-Benzyloxy-propyl)-phenyl]-3-(naphthalin-2-ylmethoxy)-piperidin als farbloser, amorpher Festkörper; MS : 467 $(M+H)^+$.

Das als Ausgangssubstanz eingesetzte BOC-Derivat wurde wie folgt hergestellt:

In analoger Weise wie in Beispiel 1 (g) beschrieben, wurde durch Alkylierung des (3RS,4RS)-4-[4-(3-Hydroxy-propyl)-phenyl]-3-(naphthalin-2-ylmethoxy)-piperidin-1-carbonsäure tert-butylesters [Beispiel 24 (t)] mit Benzylbromid der (3RS,4RS)-4-[4-(3-Benzyloxypropyl)-phenyl]-3-(naphthalin-2-ylmethoxy)-piperidin-1-carbonsäure tert-butylester als farbloser, amorpher Festkörper erhalten, der ohne Charakterisierung in der nächsten Stufe eingesetzt wurde.

Beispiel 118

Durch Abspaltung der BOC-Gruppe mittels Chlorwasserstoff in Methanol, analog Beispiel 22 (1), wurden die folgenden Verbindungen hergestellt:

1) - Aus dem (3RS,4RS)-3-(Naphthalin-2-ylmethoxy)-4-[4-(2-phenoxy-äthoxymethyl)-phenyl]-piperidin-1-carbonsäure tert-butylester das (3RS,4RS)-3-(Naphthalin-2-ylmethoxy)-4-[4-(2-phenoxy-äthoxymethyl)-phenyl]-piperidin als leicht gelbes Öl; MS : 468 $(M+H)^+$;

2) - aus dem (3RS,4RS)-3-(Naphthalin-2-ylmethoxy)-4-[4-(3-phenoxypropoxymethyl)-phenyl]-piperidin -1-carbonsäure tert-butylester das (3R5,4RS)-3-(Naphthalin-2-ylmethoxy)-4-[4-(3-phenoxy-propoxymethyl)-phenyl]-piperidin als farbloser Schaum; MS : 482 $(M+H)^+$;

3) - aus dem (3RS,4RS)-4-[4-(3-Benzyloxy-propoxymethyl)-phenyl]-3-(naphthalin-2-ylmethoxy)-piperidin-1-carbonsäure tert-butylester das (3RS,4RS)-4-[4-(3-Benzyloxy-propoxymethyl)-phenyl]-3-(naphthalin-2-ylmethoxy)-piperidin als leicht gelbes Öl; MS : 496 $(M+H)^+$;

4) - aus dem (3RS,4RS)-4-[4-[3-(4-Fluor-phenoxy)-propoxymethyl]-phenyl]-3-(naphthalin-2-ylmethoxy)-piperidin-1-carbonsäure tert-butylester (3RS,4RS)-4-[4-[3-(4-Fluor-phenoxy)-propoxymethyl]-phenyl]-3-(naphthalin-2-ylmethoxy)-piperidin als leicht gelbes Öl; MS : 500 $(M+H)^+$.

Die als Ausgangsstoffe eingesetzten BOC-Derivate wurden durch Alkylierung des (3RS,4RS)-4-(4-Hydroxymethyl-phenyl)-3-(naphthalin-2-ylmethoxy)-piperidin-1-carbonsäure tert-butylesters [Beispiel 22 (j)], in analoger Weise wie in Beispiel 1 (g) beschrieben, wie folgt hergestellt:

(a) - Durch Alkylierung mit β-Bromphenetol der (3RS,4RS)-3-(Naphthalin-2-ylmethoxy)-4-[4-(2-phenoxy-äthoxymethyl)-phenyl]-piperidin-1-carbonsäure tert-butylester als farbloses Harz , das ohne weitere Charakterisierung in der nächsten Stufe eingesetzt wurde;

(b)- durch Alkylierung mit 3-Phenoxypropylbromid der(3RS,4RS)-3-(Naphthalin-2-ylmethoxy)-4-[4-(3-phenoxypropoxymethyl)-phenyl]-piperidin-1-carbonsäure tert-butylester als farbloses Öl; MS : 582 $(M+H)^+$;

(c)- durch Alkylierung mit Benzyl-3-brompropyläther der (3RS,4RS)-4-[4-(3-Benzyloxy-propoxymethyl)-phenyl]-3-(naphthalin-2-ylmethoxy)-piperidin -1-carbonsäure tert-butylester als farbloses Harz; MS : 596 $(M+H)^+$;

(d) - durch Alkylierung mit 1-(3-Chlorpropoxy)-4-fluorbenzol der (3RS,4RS)- 4-[4-[3-(4-Fluor-phenoxy)-propoxymethyl]-phenyl]-3-(naphthalin-2-ylmethoxy)-piperidin-1-carbonsäure tert-butylester als farbloses Harz; MS : 600 $(M+H)^+$.

Beispiel 119

Durch Abspaltung der BOC-Gruppe mittels Chlorwasserstoff in Methanol, analog Beispiel 22 (l), oder mittels Zinkbromid in Methylenchlorid, analog Beispiel 10 (b), wurden die folgenden Verbindungen erhalten:

1) - Aus dem (3RS,4RS)-4-[4-(3-Benzyloxy-propoxy)-phenyl]-3-(8-hydroxy-naphthalin-2-ylmethoxy)-piperidin-1-carbonsäure tert-butylester mittels Chlorwasserstoff in Methanol das (3RS,4RS)-4-[4-(3-Benzyloxy-propoxy)-phenyl]-3-(8-hydroxy-naphthalin-2-ylmethoxy)-piperidin als farbloser Festkörper; MS : 498 $(M+H)^+$;

2) - aus dem (3RS,4RS)-4-[4-(3-Benzyloxy-propoxy)-phenyl]-3-[8-(3-methoxy-äthoxy)-naphthalin-2-ylmethoxy]-piperidin-1-carbonsäure tert-butylester mittels Chlorwasserstoff in Methanol das (3RS,4RS)-4-[4-(3-Benzyloxy-propoxy)-phenyl]-3-[8-(3-methoxy-äthoxy)-naphthalin-2-ylmethoxy]-piperidin als leicht gelbes Öl; MS : 556 $(M+H)^+$;

3) - aus dem (3RS,4RS)-4-[4-(3-Benzyloxy-propoxy)-phenyl]-3-[8-(3-methoxy-propoxy)-naphthalin-2-ylmethoxy]-piperidin-1-carbonsäure tert-butylester mittels Chlorwasserstoff in Methanol das (3RS,4RS)-4-[4-(3-Benzyloxy-propoxy)-phenyl]-3-[8-(3-methoxy-propoxy)-naphthalin-2-ylmethoxy]-piperidin als leicht gelbes Öl; MS : 570 $(M+H)^+$;

4) - aus dem (3RS,4RS)-4-[4-(3-Benzyloxy-propoxy)-phenyl]-3-(8-methoxycarbonylmethoxy-naphthalin-2-ylmethoxy)-piperidin-1-carbonsäure tert-butylester mittels Chlorwasserstoff in Methanol das (3RS,4RS)-4-[4-(3-Benzyloxy-propoxy)-phenyl]-3-(8-methoxycarbonylmethoxy-naphthalin-2-ylmethoxy)-piperidin als farbloser Festkörper; MS : 570 $(M+H)^+$;

5) - aus dem (3RS,4RS)-4-[4-(3-Benzyloxy-propoxy)-phenyl]-3-(8-carbamoylmethoxy-naphthalin-2-ylmethoxy)-piperidin-1-carbonsäure tert-butylester mittels Zinkbromid in Methylenchlorid das (3RS,4RS)-4-[4-(3-Benzyloxy-propoxy)-phenyl]-3-(8-carbamoylmethoxy-naphthalin-2-ylmethoxy)-piperidin als farbloser Schaum; MS : 555 $(M+H)^+$;

Die als Ausgangsstoffe eingesetzten BOC-Derivate wurden, in analoger Weise wie in den Beispielen 1 und 5 beschrieben, durch Alkylierung des (3RS,4RS)-4-[4-(3-Benzyloxy-propoxy)-phenyl]-3-(8-hydroxy-naphthalin-2-ylmethoxy)-piperidin-1-carbonsäure tert-butylesters wie folgt hergestellt:

(a) - Durch Alkylierung mit 2-Methoxyäthylbromid wurde der (3RS,4RS)-4-[4-(3-Benzyloxy-propoxy)-phenyl]-3-[8-(3-methoxy-äthoxy)-naphthalin-2-ylmethoxy]-piperidin-1-carbonsäure tert-butylester als farbloses Harz, der ohne Charakterisierung in die nächste Stufe eingesetzt wurde;

(b) - durch Alkylierung mit 1-Chlor-3-methoxypropan wurde der (3RS,4RS)-4-[4-(3-Benzyloxy-propoxy)-phenyl]-3-[8-(3-methoxy-propoxy)-naphthalin-2-ylmethoxy]-piperidin-1-carbonsäure tert-butylester als leicht gelbes Harz, der ohne Charakterisierung in die nächste Stufe eingesetzt wurde;

(c) - durch Alkylierung mit Bromessigsäuremethylester der (3RS,4RS)-4- [4-(3-Benzyloxy-propoxy)-phenyl]-3-(8-methoxycarbonylmethoxy-naphthalin-2-ylmethoxy)-piperidin-1-carbonsäure tert-butylester als farbloser Festkörper; MS : 670 $(M+H)^+$;

(d) - durch Alkylierung mit Iodacetamid der (3RS,4RS)-4-[4-(3-Benzyloxy-propoxy)-phenyl]-3-(8-carbamoylmethoxy-naphthalin-2-ylmethoxy)-piperidin-1-carbonsäure tert-butylester als leicht gelbes Harz, der ohne Charakterisierung in die nächste Stufe eingesetzt wurde;

Der als Ausgangsstoff eingesetzte (3RS,4RS)-4-[4-(3-Benzyloxy-propoxy)-phenyl]-3-(8-hydroxy-naphthalin-2-ylmethoxy)-piperidin-1-carbonsäure tert-butylester wurde, in analoger Weise wie in den Beispielen 1, 5 und 6 beschrieben, wie folgt hergestellt:

(a) Durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit 2-Chlormethyl-8-(2-trimethylsilyläthoxy-methoxy)-naphthalin [Beispiel 6 (aa)] wurde der (3RS,4RS)-4-[4-(3-Benzyloxy-propoxy)-phenyl]-3-[8-(2-trimethylsilanyl-äthoxymethoxy)-naphthalin-2-ylmethoxypiperidin-1-carbonsäure tert-butylester als leicht gelbes Öl erhalten; MS: 728 $(M+H)^+$.

(b) Eine Lösung von 552 mg (3RS,4RS)-4-[4-(3-Benzyloxy-propoxy)-phenyl]-3-[8-(2-trimethylsilanyl-äthoxymethoxy)-naphthalin-2-ylmethoxypiperidin-1-carbonsäure tert-butylester in 4 ml Methanol wurde mit 4 ml einer 2 N Lösung von Chlorwasserstoff in Methanol versetzt und 45 Minuten bei Raumtemperatur gerührt. Das Gemisch wurde zur Aufarbeitung zwischen 50 ml Essigester und 50 ml wäßriger 5%iger Natriumhydrogen-carbonat-Lösung verteilt und dann die organische Phase abgetrennt. Die wäßrige Phase wurde dreimal mit je 25 ml Essigester extrahiert. Die vereinigten Essigesterphasen wurden über Magnesiumsulfat getrocknet und schließlich das Lösungsmittel unter verminderten Druck abdestilliert. Das Rohprodukt (538 mg) wurde durch Chromatographie an Kieselgel mit einem 1:2-Gemisch von Essigester und Hexan als Eluierungsmittel gereinigt. Es wurden 348 mg (77% d.Th.) (3RS,4RS)-4-[4-(3-Benzyloxy-propoxy)-phenyl]-3-(8-hydroxy-naphthalin-2-ylmethoxy)-piperidin-1-carbonsäure tert-butylester als farbloser Schaum erhalten; MS: 598 $(M+H)^+$.

Beispiel 120

Durch Abspaltung der BOC-Gruppe mittels Chlorwasserstoff in Methanol, analog Beispiel 22 (1), wurden die folgenden Verbindungen hergestellt:

1) - Aus dem (3RS,4RS)-4-[4-(3-Benzyloxy-propoxy)-phenyl]-3-(quinolin-7-ylmethoxy)-piperidin-1-carbonsäure tert-butylester das (3RS,4RS)-4-[4-(3-Benzyloxy-propoxy)-phenyl]-3-(quinolin-7-ylmethoxy)-piperidin als farbloses Öl; MS : 483 $(M+H)^+$;

2) - aus dem (3RS,4RS)-4-[4-(3-Benzyloxy-propoxy)-phenyl]-3-(1,2,3,4-tetrahydro-quinolin-7-ylmethoxy)-piperidin-1-carbonsäure tert-butylester das (3RS,4RS)-4-[4-(3-Benzyloxy-propoxy)-phenyl]-3-(1,2,3,4-tetrahydro-quinolin-7-ylmethoxy)-piperidin als leicht gelbes Harz; MS : 487 $(M+H)^+$;

3) - aus dem (3RS,4RS)- 7-[4-[4-(3-Benzyloxy-propoxy)-phenyl]-1-tertbutoxycarbonyl-piperidin-3-yloxymethyl]-1-methyl-quinolinium Iodid das (3RS,4RS)-7-[4-[4-(3-Benzyloxy-propoxy)-phenyl]-piperidin-3-yloxymethyl]-1-methyl-quinolinium Chlorid als farbloser Festkörper; MS : 497 $(M)^+$;

4) - aus dem (3RS,4RS)-4-[4-(3-Benzyloxy-propoxy)-phenyl]-3-(1-methyl-1,2,3,4-tetrahydro-quinolin-7-ylmethoxy)-piperidin-1-carbonsäure tert-butylester das (3RS,4RS)-4-[4-(3-Benzyloxy-propoxy)-phenyl]-3-(1-methyl-1,2,3,4-tetrahydro-quinolin-7-ylmethoxy)-piperidin als leicht gelbes Öl; MS : 501 $(M+H)^+$;

5) - aus dem (3RS,4RS)-4-[4-(3-Benzyloxy-propoxy)-phenyl]-3-(isoquinolin-6-ylmethoxy)-piperidin-1-carbonsäure tert-butylester das (3RS,4RS)-4-[4-(3-Benzyloxy-propoxy)-phenyl]-3-(isoquinolin-6-ylmethoxy)-piperidin als leicht gelbes Harz; MS : 483 $(M+H)^+$;

6) - aus dem (3RS,4RS)-4-[4-(3-Benzyloxy-propoxy)-phenyl]-3-(2-methyl-1,2,3,4-tetrahydro-isoquinolin-6-ylmethoxy)-piperidin-1-carbonsäure tert-butylester das (3RS,4RS)- 4-[4-(3-Benzyloxy-propoxy)-phenyl]-3-(2-methyl-1,2,3,4-tetrahydro-isoquinolin-6-ylmethoxy)-piperidin als leicht gelbes Öl; MS : 501 $(M+H)^+$;

7) - aus dem (3RS,4RS)-4-[4-[3-(2-Methoxy-benzyloxy)-propoxy]-phenyl]-3-(quinolin-7-ylmethoxy)-piperidin-1-carbonsäure tert-butylester das (3RS,4RS)-4-[4-[3-(2-Methoxy-benzyloxy)-propoxy]-phenyl]-3-(quinolin-7-ylmethoxy)-piperidin als leicht gelbes Harz; MS : 513 $(M+H)^+$;

8) - aus dem (3RS,4RS)-3-(lH-Benzimidazol-5-ylmethoxy)-4-[4-(3-benzyloxy-propoxy)-phenyl]-piperidin-1-carbonsäure tert-butylester mit Chlorwasserstoff in Methanol das (3RS,4RS)-3-(1H-Benzimidazol-5-ylmethoxy)-4-[4-(3-benzyloxy-propoxy)-phenyl]-piperidin als farbloses Harz; MS : 472 $(M+H)^+$;

9) aus dem (3RS,4RS)-4-[4-[3-(2-Methoxy-benzyloxy)-propoxy]-phenyl]-3-(1-oxy-chinolin-7-ylmethoxy)-piperidin-1-carbonsäure tert-butylester mit Chlorwasserstoff in Methanol das (3RS,4RS)-4-[4-[3-(2-Methoxybenzyloxy)-propoxy]-phenyl]-3-(1-oxy-chinolin-7-ylmethoxy)-piperidin als farbloser Festkörper; MS : 529 $(M+H)^+$;

10) aus dem (3RS,4RS)-4-[4-[3-(2-Methoxy-benzyloxy)-propoxy]-phenyl]-3-(2-oxo-1,2-dihydro-chinolin-7-ylmethoxy)-piperidin-1-carbonsäure tert-butylester mit Zinkbromid in Methylenchlorid das (3RS,4RS)-4-[4-[3-(2-Methoxy-benzyloxy)-propoxy]-phenyl]-3-(2-oxo-1,2-dihydro-chinolin-7-ylmethoxy)-piperidin als farbloser Festkörper; MS : 529 $(M+H)^+$;

11) aus dem(3RS,4RS)-3-(Isochinolin-7-ylmethoxy)-4-[4-[3-(2-methoxybenzyloxy)-propoxy]-phenyl]-piperidin-1-carbonsäure tert-butylester mit Chlorwasserstoff in Methanol das (3RS,4RS)-3-(Isochinolin-7-ylmethoxy)-4-[4-[3-(2-methoxy-benzyloxy)-propoxy]-phenyl]-piperidin als leicht gelbes Öl; MS : 513 $(M+H)^+$;

12) - aus dem (3RS,4RS)-4-[4-[3-(2-Methoxy-benzyloxy)-propoxy]-phenyl)-3-(1,2,3,4-tetrahydro-chinolin-7-ylmethoxy)-piperidin-1-carbonsäure tert-butylester mit Chlorwasserstoff in Methanol das (3RS,4RS)-4-[4-[3-(2-Methoxy-benzyloxy)-propoxy]-phenyl]-3-(1,2,3,4-tetrahydro-chinolin-7-ylmethoxy)-piperidin als hellgelber Sirup; MS: 517 $(M+H)^+$;

13) - aus dem (3RS,4RS)-4-[4-[3-(2-Methoxy-benzyloxy)-propoxy]-phenyl]-3-(chinoxalin-6-ylmethoxy)-piperidin-1-carbonsäure tert-butyl ester mit Zinkbromid in Methylenchlorid das (3RS,4RS)-4-[4-[3-(2-Methoxy-benzyloxy)-propoxy]-phenyl]-3-(chinoxalin-6-ylmethoxy)-piperidin als gelbes Öl; MS : 514 $(M+H)^+$.

Die als Ausgangsstoffe eingesetzten BOC-Derivate wurden, in analoger Weise wie in den Beispielen 1 und 5 beschrieben, wie folgt hergestellt:

(a) Durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit 7-Brommethyl-chinolin Hydrobromid [J.Am.Chem.Soc. 77, 1054(1955)], analog Beispiel 1 (g), wurde der (3RS,4RS)-4-[4-(3-Benzyloxypropoxy)-phenyl]-3-(quinolin-7-ylmethoxy)-piperidin-1-carbonsäure tert-butylester als farbloses Öl erhalten; MS : 583 $(M+H)^+$.

(b) Eine Lösung von 116 mg (0.20 mMol) (3RS,4RS)-4-[4-(3-Benzyloxypropoxy)-phenyl]-3-(quinolin-7-ylmethoxy)-piperidin-1-carbonsäure tert-butylester in 1.5 ml Methanol wurde mit 24 mg (0.1 mMol) Nickel(II)-chlorid Hexahydrat und 30 mg (0.8 mMol) Natriumborhydrid versetzt. Die dunkle Suspension wurde eine Stunde bei 0 °C und eine weitere Stunde lang bei Raumtemperatur gerührt. Das Gemisch wurde zur Aufarbeitung zwischen 20 ml Äther und 5 ml wäßriger gesättigter Ammoniumchlorid-Lösung verteilt und dann die organische Phase wurde abgetrennt. Die wäßrige Phase wurde dreimal mit je 20 ml Äther extrahiert. Die vereinigten Ätherphasen wurden über Magnesiumsulfat getrocknet und schließlich das Lösungsmittel unter verminderten Druck abdestilliert. Das Rohprodukt (150 mg) wurde durch Chromatographie an Kieselgel mit einem 1:1-Gemisch von Essigester und Hexan als Eluierungsmittel gereinigt. Es wurden 70 mg (60% d.Th.) (3RS,4RS)-4-[4-(3-Benzyloxy-propoxy)-phenyl]-3-(1,2,3,4-tetrahydro-quinolin-7-ylmethoxy)-piperidin-1-carbonsäure tert-butylester als leicht gelbes Harz erhalten; MS: 587 $(M+H)^+$.

(c) Eine Lösung von 146 mg (0.25 mMol) (3RS,4RS)-4-[4-(3-Benzyloxypropoxy)-phenyl]-3-(quinolin-7-ylmethoxy)-piperidin-1-carbonsäure tert-butylester in 0.5 ml absolutem Chloroform wurde mit 40 µl (0.6 mMol) Methyljodid versetzt und 3 Stunden lang zum Rückfluß erhitzt. Anschließend wurden nochmals 40 µl Methyliodid zugegeben und das Gemisch über Nacht bei Rückflußtemperatur erhitzt. Das Lösungsmittel wurde unter verminderten Druck abdestilliert und man erhielt das rohe (3RS,4RS)-7-[4-[4-(3-Benzyloxy-propoxy)-phenyl] -1-tertbutoxycarbonyl-piperidin-3-yloxymethyl]-1-methyl-quinolinium Iodid, das ohne Charakterisierung in der nächsten Stufe eingesetzt wurde.

(d) Eine Lösung von 91 mg (0.125 mMol) rohem (3RS,4RS)-7-[4-[4-(3-Benzyloxy-propoxy)-phenyl]-1-tert-butoxycarbonyl-piperidin-3-yloxymethyl]-1-methyl-quinolinium Iodid in 1 ml Methanol wurde bei 0 °C mit 47 mg (0.125 mMol) Natriumborhydrid versetzt, dann auf Raumtemperatur erwärmt und 2 Stunden lang bei Raumtemperatur gerührt. Das Gemisch wurde zur Aufarbeitung zwischen 50 ml Essigester und 50 ml wäßriger 5%iger Natriumhydrogencarbonat-Lösung verteilt und danach die organische Phase abgetrennt. Die wäßrige Phase wurde dreimal mit je 25 ml Essigester extrahiert. Die vereinigten Essigesterphasen wurden über Magnesiumsulfat getrocknet und schließlich das Lösungsmittel unter verminderten Druck abdestilliert. Der rohe (3RS,4RS)-4-[4-(3-Benzyloxy-propoxy)-phenyl)-3-(1-methyl-1,2,3,4-tetrahydro-quinolin-7-ylmethoxy)-piperidin-1-carbonsäure tert-butylester wurde ohne Charakterisierung in der nächsten Stufe eingesetzt.

(e) Durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit 6-Brommethyl-isochinolin Hydrobromid (Beispiel 4), analog Beispiel 1 (g), wurde der (3RS,4RS)-4-[4-(3-Benzyloxy-propoxy)-phenyl]-3-(isochinolin-6-ylmethoxy)-piperidin-1-carbonsäure tert-butylester als farbloses Öl erhalten; MS : 583 $(M+H)^+$.

(f) In analoger Weise wie vorstehend beschrieben, wurde durch Alkylierung des (3RS,4RS)-4-[4-(3-Benzyloxy-propoxy)-phenyl]-3-(isochinolin-6-ylmethoxy)-piperidin-1-carbonsäure tert-butylesters mit Methyljodid in Chloroform und anschließende Reduktion mit Natriumborhydrid in Methanol der (3RS,4RS)-4-[4-(3-Benzyloxypropoxy)-phenyl]-3-(2-methyl-1,2,3,4-tetrahydro-isoquinolin-6-ylmethoxy)-piperidin-1-carbonsäure tert-butylester erhalten, der als Rohprodukt ohne weitere Charakterisierung in der nächsten Stufe eingesetzt wurde.

(g) (α) Eine Lösung von 5.2 g (17.7 mMol) (3RS,4RS)-3-Hydroxy-4-(4-hydroxy-phenyl)-piperidin-1-carbonsäure tert-butylester [Beispiel 46 (b)] und 3.37 ml (3.8 g, 17.7 mMol) 2-Methoxybenzyl-3-chlorpropyläther in 18 ml absolutem DMF wurde mit 3.7 g (26.9 mMol) wasserfreiem Kaliumcarbonat versetzt und 60 h bei 120 °C gerührt. Zur Aufarbeitung wurde das Reaktions-gemisch zwischen 250 ml Wasser und 250 ml Essigester verteilt. Die organische Phase wurde abgetrennt, die wäßrige Phase wurde dreimal mit je 100 ml Essigester extrahiert. Die vereinigten organischen Phasen wurden zweimal mit je 100 ml Wasser gewaschen und schließlich das Lösungsmittel unter verminderten Druck abdestilliert. Das Rohprodukt wurde durch Behandlung mit Äther zur Kristallisation gebracht. Es wurden 7.3 g (88% d.Th.) (3RS,4RS)-3-Hydroxy-4-[4-[3-(2-methoxy-benzyloxy)-propoxy]-phenyl]-piperidin-1-carbonsäure tert-butylester als farbloser Festkörper erhalten; MS: 472 $(M+H)^+$.

(β) Die sich anschließende Alkylierung des (3RS,4RS)-3-Hydroxy-4-[4-[3-(2-methoxy-benzyloxy)-propoxy]-phenyl]-piperidin-1-carbonsäure tert-butylesters mit 7-Brommethyl-chinolin Hydrobromid [J.Am.Chem.Soc. 77, 1054 (1955)], analog Beispiel 1 (g), ergab den (3RS,4RS)-4-[4-[3-(2-Methoxy-benzyloxy)-propoxy]-phenyl]-3-(chinolin-7-ylmethoxy)-piperidin-1-carbonsäure tert-butylester als leicht gelbes Harz ergab; MS: 613 $(M+H)^+$.

Der als Alkylierungsmittel eingesetzte 2-Methoxybenzyl-3-chlorpropyläther wurde wie folgt hergestellt:

Eine Lösung von 24.6 g (0.157 Mol) 2-Methoxybenzylchlorid und 26 ml (29.4 g, 0.311 Mol) 3-Chlor-1-propanol in 150 ml absolutem DMF wurde bei 10 °C portionsweise innerhalb von 2.5 Stunden mit 8.4 g (0.196 Mol) Natriumhydriddispersion (55 %ig in Weißöl) versetzt und 1Stunde lang bei Raumtemperatur gerührt. Anschließend wurde nochmals 1.0 g (0.023 Mol) Natriumhydriddispersion bei Raumtemperatur zugegeben und das Gemisch 3 Stunden lang weitergerührt. Zur Aufarbeitung wurde das Reaktionsgemisch zwischen 500 ml Wasser und 500 ml Essigester verteilt. Die organische Phase wurde abgetrennt. Die wäßrige Phase wurde viermal mit je 250 ml Essigester extrahiert. Die vereinigten organischen Phasen wurden zweimal mit je 250 ml Wasser gewaschen und schließlich das Lösungsmittel unter vermindertem Druck abdestilliert. Das Rohprodukt (44 g) wurde durch Chromatographie an Kieselgel mit einem 1:2-Gemisch von Methylenchlorid und Hexan als Eluierungsmittel gereinigt. Es wurden 25.0 g (74% d.Th.) 2-Methoxybenzyl-3-chlorpropyläther als farbloses Öl erhalten; MS: 214, 216 $(M)^+$.

(h) (α) Eine Lösung von 2.15 g (14.50 mMol) (1H-Benzimidazol-5-yl)-methanol [DE 2'813'523] in 55 ml absolutem DMF wurde mit 4.01 g wasserfreiem Kaliumcarbonat und tropfenweise mit 3.15 ml (2.96 g, 16.02 mMol) SEM-Chlorid versetzt. Nach 3 Stunden bei Raumtemperatur wurde das Reaktionsgemisch filtriert und die Hauptmenge DMF unter Hochvakuum abdestillert. Der Rückstand wurde zur Aufarbeitung zwischen 60 ml Essigester und 60 ml Wasser verteilt, danach die organische Phase abgetrennt. Die wäßrige Phase wurde zweimal mit je 60 ml Essigester extrahiert. Die vereinigten organischen Phasen wurden über Magnesiumsulfat getrocknet und schließlich das Lösungsmittel unter verminderten Druck abdestilliert. Das Rohprodukt (4.57 g) wurde durch Chromatographie

an Kieselgel mit einem 14:1:0.1-Gemisch von Methylenchlorid, Methanol und 28%iger Ammoniaklösung als Elueirungsmittel gereinigt. Es wurden 1.26 g (31% d.Th.) eines 1:2 oder 2:1 Gemisches des [3-(2-Trimethylsilyl-äthoxymethyl)-3H-benzimidazol-5-yl]-methanols und [1-(2-trimethylsilyl-äthoxymethyl)-1H-benzimidazol-5-yl]-methanols als orangefarbenes Öl erhalten; MS: 278 $(M)^+$;

(β) Die Chlorierung des 1:2 oder 2:1 Gemisches des [3-(2-Trimethylsilyläthoxymethyl)-3H-benzimidazol-5-yl]-methanols und [1-(2-trimethylsilyl-äthoxymethyl)-1H-benzimidazol-5-yl]-methanols erfolgte in analoger Weise wie in Beispiel 5 (c) beschrieben und lieferte das 1:2 oder 2:1 Gemisch des 6-Chlormethyl-1-(2-trimethylsilyl-äthoxymethyl)-1H-benzimidazols und 5-Chlormethyl-1-(2-trimethylsilyl-äthoxymethyl)-1H-benzimidazols als leicht gelbes Öl; MS : 296, 298 $(M)^+$.

(γ) Die Alkylierung des (3RS,4RS)-4-[4-(3-Benzyloxy-propoxy)-phenyl]-3-hydroxy-piperidin-1-carbonsäure tert-butylesters [Beispiel 86 (n)] mit dem 1:2 oder 2:1 Gemisch des 6-Chlormethyl-1-(2-trimethylsilyl-äthoxymethyl)-1H-benzimidazols und 5-Chlormethyl-1-(2-trimethylsilyl-äthoxymethyl)-1H-benzimidazols lieferte das 2:1 oder 1:2 Gemisch des (3RS,4RS)-4-[4-(3-Benzyloxy-propoxy)-phenyl]-3-[1-(2-trimethylsilyl-äthoxymethyl)-1H-benzimidazol-5-ylmethoxy]-piperidin-1-carbonsäure tert-butylesters und (3RS,4RS)-4-[4-(3-Benzyloxy-propoxy)-phenyl]-3-[3-(2-trimethylsilanyl-äthoxymethyl)-3H-benzimidazol-5-ylmethoxy]-piperidin-1-carbonsäure tert-butylesters als gelbes Öl; MS : 702 $(M+H)^+$.

(δ) Eine Lösung von 328 mg (0.467 mMol) des 2:1 oder 1:2 Gemisches des (3RS,4RS)-4-[4-(3-Benzyloxy-propoxy)-phenyl]-3-[1-(2-trimethylsilyl-äthoxymethyl)-lH-benzimidazol-5-ylmethoxy]-piperidin-1-carbonsäure tert-butylesters and (3RS,4RS)-4-[4-(3-Benzyloxy-propoxy)-phenyl]-3-[3-(2-trimethylsilanyl-äthoxymethyl)-3H-benzimidazol-5-ylmethoxy]-piperidin-1-carbonsäure tert-butylesters in 14 ml absolutem Tetrahydrofuran wurde mit 3.5 ml einer 1.1 M Tetrabutylammoniumfluorid-Lösung in Tetrahydrofuran versetzt und 2 Stunden lang bei Rückflußtemperatur erhitzt. Das Reaktionsgemisch wurde zur Aufarbeitung zwischen 50 ml Essigester und 50 ml Natriumhydrogencarbonat-Lösung (5 %ig) verteilt, anschließend die organische Phase abgetrennt. Die wäßrige Phase wurde zweimal mit je 50 ml Essigester extrahiert. Die vereinigten organischen Phasen wurden mit je 25 ml Wasser gewaschen, über Magnesiumsulfat getrocknet und schließlich das Lösungsmittel unter vermindertem Druck abdestilliert. Das Rohprodukt (280 mg) wurde durch Chromatographie an Kieselgel mit einem 14:1:0.1-Gemisch von Methylenchlorid, Methanol und 28%iger Ammoniaklösung als Eluierungsmittel gereinigt. Es wurden 176 mg (66% d.Th.) (3RS,4RS)-3-(1H-Benzimidazol-5-ylmethoxy)-4-[4-(3-benzyloxy-propoxy)-phenyl]-piperidin-1-carbonsäure tert-butylester als gelbes Öl erhalten; MS: 572 $(M+H)^+$.

(i) Zu einer Lösung von 459 mg (0.75 mMol) (3RS,4RS)-4-[4-[3-(2-Methoxy-benzyloxy)-propoxy]-phenyl]-3-(chinolin-7-ylmethoxy)-piperidin-1-carbonsäure tert-butylester in 30 ml Chloroform wurde bei 0 °C eine Lösung von 240 mg (ca. 0.82 mMol) 60-70%ige 3-Chlorperbenzoesäure in 15 ml Chloroform tropfenweise gegeben. Das Reaktionsgemisch wurde 2.5 Stunden lang bei Raumtemperatur gerührt, dann zur Aufarbeitung zwischen 50 ml Chloroform und 50 ml 10%iger Kaliumcarbonatlösung verteilt. Die organische Phase wurde abgetrennt, die wäßrige Phase wurde dreimal mit je 25 ml Chloroform extrahiert. Die vereinigten organischen Phasen wurden zweimal mit je 25 ml Wasser gewaschen, über Magnesiumsulfat getrocknet und schließlich das Lösungsmittel unter verminderten Druck abdestilliert. Das Rohprodukt wurde durch Chromatographie an Kieselgel mit einem 19:1-Gemisch von Methylenchlorid und Methanol als Eluierungsmittel gereinigt. Es wurden 450 mg (96% d.Th.) (3RS,4RS)-4-[4-[3-(2-Methoxy-benzyloxy)-propoxy]-phenyl]-3-(1-oxy-chinolin-7-ylmethoxy)-piperidin-1-carbonsäure tert-butylester als leicht gelbes Harz erhalten; MS: 629 $(M+H)^+$.

(j) Eine Lösung von 50 mg (0.080 mMol) (3RS,4RS)-4-[4-[3-(2-Methoxybenzyloxy)-propoxy]-phenyl]-3-(1-oxy-chinolin-7-ylmethoxy)-piperidin-1-carbonsäure tert-butylester in 0.5 ml Chloroform wurde mit 17 mg (0.088 mMol) Tosylchlorid und 0.5 ml 10%iger Kaliumcarbonat-Lösung versetzt und 3 Stunden lang bei Raumtemperatur intensiv gerührt. Das Reaktionsgemisch wurde zur Aufarbeitung zwischen 20 ml Essigester und 20 ml Wasser verteilt, dann die organische Phase abgetrennt und die wäßrige Phase dreimal mit je 20 ml Essigester extrahiert. Die vereinigten organischen Phasen wurden zweimal mit je 25 ml Wasser gewaschen, über Magnesiumsulfat getrocknet und schließlich das Lösungsmittel unter vermindertem Druck abdestilliert. Das Rohprodukt (60 mg) wurde durch Chromatographie an Kieselgel mit einem 2:1-Gemisch von Essigester und Hexan als Eluierungsmittel gereinigt. Es wurden 37 mg (74% d.Th.) (3RS,4RS)-4-[4-[3-(2-Methoxybenzyloxy)-propoxy]-phenyl]-3-(2-oxo-1,2-dihydro-chinolin-7-ylmethoxy)-piperidin-1-carbonsäure tert-butylester als farbloser Festkörper erhalten, der ohne Charakterisierung direkt in die nächste Stufe eingesetzt wurde.

(k) In analoger Weise wie in Beispiel 1 (g) beschrieben, wurde durch Alkylierung des (3RS,4RS)-3-Hydroxy-4-[4-[3-(2-methoxy-benzyloxy)-propoxy]-phenyl]-piperidin-1-carbonsäure tert-butylesters [Beispiel 120 (g) (a)] mit 7-Brommethyl-isochinolin (WO-9'319'059) der (3RS,4RS)-3-(Isochinolin-7-ylmethoxy)-4-[4-[3-(2-methoxy-benzyloxy)-propoxy]-phenyl]-piperidin-1-carbonsäure tert-butylester als leicht gelbes Harz erhalten; MS : 613 $(M+H)^+$.

(l) In analoger Weise wie in Beispiel 120 (b) beschrieben, wurde durch Reduktion des [Beispiel 120 (g) (β) mittels Nickel(II)-chlorid Hexahydrat und Natriumborhydrid der (3RS,4RS)-4-[4-[3-(2-Methoxy-benzyloxy)-propoxy]-phenyl]-3-(1,2,3,4-tetrahydro-chinolin-7-ylmethoxy)-piperidin-1-carbonsäure tert-butylester als hellgelber Sirup erhalten, der ohne weitere Reinigung und Charakterisierung in der folgenden Stufe eingesetzt wurde.

(m) In analoger Weise wie in Beispiel 1 (g) beschrieben, wurde durch Alkylierung des (3RS,4RS)-3-Hydroxy-4-[4-[3-(2-methoxy-benzyloxy)-propoxy]-phenyl]-piperidin-1-carbonsäure tert-butyl esters [Beispiel 120 (g) (α)] mit 6-Brommethyl-chinoxalin [J. Heterocycl. Chem. 11, 595 (1974)] der (3RS,4RS)-4-[4-[3-(2-Methoxy-benzyloxy)-propoxy]-phenyl]-3-(chinoxalin-6-ylmethoxy)-piperidin-1-carbonsäure tert-butyl ester als leicht gelbes Öl erhalten; MS : 614 $(M+H)^+$.

Beispiel 121

Durch Abspaltung der BOC-Gruppe wurden die folgenden Verbindungen erhalten:

1) - Aus dem Gemisch des (3RS,4RS)- und (3SR,4SR)-4-[4-[(RS)-3-Benzyloxy-2-methoxy-propoxy]-phenyl]-3-(1-methoxy-naphthalin-2-ylmethoxy)-piperidin-1-carbonsäure tert-butylesters mittels Zinkbromid in Methylenchlorid das Gemisch des (3RS,4RS)-4-[4-[(RS)- und [(SR)-3-Benzyloxy-2-methoxy-propoxy]-phenyl]-3-(1-methoxy-naphthalin-2-ylmethoxy)-piperidins als gelbes Öl; MS : 542 $(M+H)^+$;

2) - aus dem Gemisch des (3RS,4RS)- und (3SR,4SR)-4-[4-[(RS)-2-Benzyloxy-3-phenoxy-propoxy]-phenyl]-3-(naphthalin-2-ylmethoxy)-piperidin-1-carbonsäure tert-butylesters mittels Trifluoressigsäure in Methylenchlorid das Gemisch des (3RS,4RS)-4-[4- [(RS)- und -[(SR)-2-Benzyloxy-3-phenoxy-propoxy]-phenyl]-3-(naphthalin-2-ylmethoxy)-piperidin trifluoracetats als weißer Festkörper; MS : 574 $(M+H)^+$;

3) - aus dem Gemisch des (3RS,4RS)- und (3SR,4SR)-4-[4-[(RS)-2-Hydroxy-3-(4-methyl-phenylsulfonylamino)-propoxy]-phenyl]-3-(naphthalin-2-ylmethoxy)-piperidin-1-carbonsäure tert-butylesters mittels Trifluoressigsäure in Methylenchlorid das Gemisch des N-[(RS)- und -(SR)-2-Hydroxy-3-[(3RS,4RS)-4-[3-(naphthalin-2-ylmethoxypiperidin-4-yl]-phenoxy]-propyl]-4-methyl-benzolsulfonamid Trifluoracetats als weißer Festkörper; MS : 561 $(M+H)^+$;

4) - aus dem Gemisch des (3RS,4RS)- und (3SR,4SR)-4-[4-[(RS)-2-Allyloxy-4-phenyl-butoxy]-phenyl]-3-(naphthalin-2-ylmethoxy)-piperidin-1-carbonsäure tert-butylesters mittels Trifluoressigsäure in Methylenchlorid das Gemisch des (3RS,4RS)-4-[4-[(RS)- und -[(SR)-2-Allyloxy-4-phenyl-butoxy]-phenyl]-3-(naphthalin-2-ylmethoxy)-piperidin Trifluoracetats als weißer Festkörper; MS : 522 $(M+H)^+$;

Die als Ausgangsverbindungen eingesetzten BOC-Derivate wurden wie folgt hergestellt:

(a) In analoger Weise wie in Beispiel 1 (g) beschrieben, wurde durch Alkylierung des Gemisches des (3RS,4RS)- und (3SR,4SR)-3-Hydroxy-4-[(RS)-4-oxiranylmethoxy-phenyl]-piperidin-1-carbonsäure tert-butylesters [Beispiel 86 (d)] mit 1-Methoxy-2-brommethyl-naphthalin [Beispiel 7 (f)] das Gemisch des (3RS,4RS)- und (3SR,4SR)-3-(1-Methoxynaphthalin-2-ylmethoxy)-4-[(RS)-4-oxiranylmethoxy-phenyl]-piperidin-1-carbonsäure tert-butylesters erhalten. Die anschließende Epoxidöffnung mit Natriumbenzylat in N,N-Dimethylformamid lieferte das Gemisch des (3RS,4RS)- und (3SR,4SR)-4-[4-[(RS)-3-Benzyloxy-2-hydroxy-propoxy]-phenyl]-3-(1-methoxy-naphthalin-2-ylmethoxy)-piperidin-1-carbonsäure tert-butylesters, dessen Alkylierung mit Methyliodid, analog Beispiel 1 (g), das Gemisch des (3RS,4RS)- und (3SR,4SR)-4-[4-[(RS)-3-Benzyloxy-2-methoxy-propoxy]-phenyl]-3-(1-methoxy-naphthalin-2-ylmethoxy)-piperidin-1-carbonsäure tert-butylesters als farbloses Öl ergab; MS : 628 $(M+H)^+$;

(b) In analoger Weise wie in Beispiel 1 (g) beschrieben, wurde durch Alkylierung des Gemisches des (3RS,4RS)- und (3SR,4SR)-4-[4-[(RS)-2-Hydroxy-3-phenoxy-propoxy]-phenyl]-3-(naphthalin-2-ylmethoxy)-piperidin-1-carbonsäure tert-butylesters [Beispiel 86 (d)] mit Benzylbromid das Gemisch des (3RS,4RS)- und (3SR,4SR)-4-[4-[(RS)-2-Benzyloxy-3-phenoxy-propoxy]-phenyl]-3-(naphthalin-2-ylmethoxy)-piperidin-1-carbonsäure tert-butylesters als Festkörper erhalten; MS : 674 $(M+H)^+$;

(c) Die Epoxidöffnung des Gemisches des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters [Beispiel 86 (d)] mit dem Kaliumsalz des Toluol-4-sulfonamids, in Analogie zu Beispiel 71 (a), lieferte das Gemisch des (3RS,4RS)- und (3SR,4SR)-4-[4-[(RS)-2-Hydroxy-3-(4-methyl-phenylsulfonylamino)-propoxy]-phenyl]-3-(naphthalin-2-ylmethoxy)-piperidin-1-carbonsäure tert-butylesters als weißen Festkörper; MS : 661 $(M+H)^+$;

(d) Die Epoxidöffnung des Gemisches des (3RS,4RS)- und (3SR,4SR)-3-(Naphthalin-2-ylmethoxy)-4-[(RS)-4-oxiranylmethoxy-phenyl]-piperidin-1-carbonsäure tert-butylesters [Beispiel 86 (d)] mit Benzylmagnesiumchlorid in Tetrahydrofuran lieferte das Gemisch des (3RS,4RS)- und (3SR,4SR)-4-[4-[(RS)-2-Hydroxy-4-phenyl-butoxy]-phenyl]-3-(naphthalin-2-ylmethoxy)-piperidin-1-carbonsäure tert-butylesters, dessen Alkylierung mit Allylbromid, analog Beispiel 1 (g), das Gemisch des (3RS,4RS)- und (3SR,4SR)-4-[4-[(RS)-2-Allyloxy-4-phenyl-butoxy]-phenyl]-3-(naphthalin-2-ylmethoxy)-piperidin-1-carbonsäure tert-butylesters als farbloses Öl ergab; MS : 621 $(M)^+$.

Beispiel 122

Durch Abspaltung der BOC-Gruppe wurden die folgenden Verbindungen erhalten:

1) - Aus dem (3RS,4RS)-3-(Naphthalin-2-ylmethoxy)-4-[4-(3-phenylpropoxy)-phenyl]-piperidin-1-carbonsäure tert-butylester mittels Trifluoressigsäure in Methylenchlorid das (3RS,4RS)-3-(Naphthalin-2-ylmethoxy)-4-[4-(3-phenyl-propoxy)-phenyl]-piperidin Trifluoracetat als farbloser Festkörper; MS : 452 $(M+H)^+$;

2) - aus dem (3RS,4RS)-3-(Naphthalin-2-ylmethoxy)-4-[4-(4-phenylbutoxy)-phenyl]-piperidin-1-carbonsäure tert-butylester mittels Trifluoressigsäure in Methylenchlorid das (3RS,4RS)-3-(Naphthalin-2-ylmethoxy)-4-[4-(4-phenyl-butoxy)-phenyl]-piperidin Trifluoracetat als farbloser Festkörper; MS : 466 $(M+H)^+$;

3) - aus dem (3RS,4RS)-3-(Naphthalin-2-ylmethoxy)-4-[4-(5-phenylpentyloxy)-phenyl]-piperidin-1-carbonsäure tert-butylester mittels Trifluoressigsäure in Methylenchlorid das (3RS,4RS)-3-(Naphthalin-2-ylmethoxy)-4-[4-(5-phenyl-pentyloxy)-phenyl]-piperidin Trifluoracetat als farbloser Festkörper; MS : 480 $(M+H)^+$;

4) - aus dem (E)-(3RS,4RS)-3-(Naphthalin-2-ylmethoxy)-4-[4-(3-phenylallyloxy)-phenyl]-piperidin-1-carbonsäure tert-butylester mittels Zinkbromid in Methylenchlorid das (E)-(3RS,4RS)-3-(Naphthalin-2-ylmethoxy)-4-[4-(3-phenyl-allyloxy)-phenyl]-piperidin als farbloser Festkörper; MS : 450 $(M+H)^+$;

5) - aus dem (E)-(3RS,4RS)-3-(1,4-Dimethoxy-naphthalin-2-ylmethoxy)-4-[4-(3-phenyl-allyloxy)-phenyl]-piperidin-1-carbonsäure tert-butylester mittels Zinkbromid in Methylenchlorid das (E)-(3RS,4RS)-3-(1,4-Dimethoxy-naphthalin-2-ylmethoxy)-4-[4-(3-phenylallyloxy)-phenyl]-piperidin als beiger Festkörper; MS : 510 $(M+H)^+$;

6) - aus dem (E)-(3RS,4RS)-3-(4-Methylsulfanyl-benzyloxy)-4-[4-(3-phenyl-allyloxy)-phenyl]-piperidin-1-carbonsäure tert-butylester mittels Zinkbromid in Methylenchlorid das (E)-(3RS,4RS)-3-(4-Methylsulfanylbenzyloxy)-4-[4-(3-phenyl-allyloxy)-phenyl]-piperidin als gelbliches Öl; MS : 446 $(M+H)^+$;

7) - aus dem (E)-(3RS,4RS)-3-(1,4-Dimethoxy-naphthalin-2-ylmethoxy)-4-[4-(4-phenyl-but-3-enyloxy)-phenyl]-piperidin-1-carbonsäure tert-butylester mittels Zinkbromid in Methylenchlorid das (E)-(3RS,4RS)-3-(1,4-Dimethoxy-naphthalin-2-ylmethoxy)-4-[4-(4-phenylbut-3-enyloxy)-phenyl]-piperidin als beiger Festkörper; MS : 524 $(M+H)^+$;

8) - aus dem (3RS,4RS)-4-[4-(3-Cyano-benzyloxy)-phenyl]-3-[1-(2-methoxy-äthoxy)-naphthalin-2-ylmethoxy]-piperidin-1-carbonsäure tert-butylester mittels Zinkbromid in Methylenchlorid das (3RS,4RS)-3-[4-[3-[1-(2-Methoxy-äthoxy)-naphthalin-2-ylmethoxy]-piperidin-4-yl]-phenoxymethyl]-benzonitril als viskoses, hellgelbes Öl; MS : 523 $(M+H)^+$;

9) - aus dem (3R5,4R5)-3-(1,4-Dimethoxy-naphthalin-2-ylmethoxy)-4-[4-(4-phenyl-butoxy)-phenyl]-piperidin-1-carbonsäure tert-butylester mittels Zinkbromid das (3RS,4RS)-3-(1,4-Dimethoxy-naphthalin-2-ylmethoxy)-4-[4-(4-phenyl-butoxy)-phenyl]-piperidin als beiger Festkörper; MS : 526 $(M+H)^+$.

Die als Ausgangsverbindungen eingesetzten BOC-Derivate wurden wie folgt hergestellt:

(a) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit 1-Brom-3-phenylpropan in Gegenwart von Kaliumcarbonat der (3RS,4RS)-3-Hydroxy-4-[4-(3-phenyl-propoxy)-phenyl]-piperidin-1-carbonsäure tert-butylester erhalten, dessen Alkylierung, analog Beispiel 1 (g), mit 2-Brommethyl-naphthalin den (3RS,4RS)-3-(Naphthalin-2-ylmethoxy)-4-[4-(3-phenylpropoxy)-phenyl]-piperidin-1-carbonsäure tert-butylester als farblosen Festkörper lieferte; MS : 551 $(M)^+$.

(b) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des 4-Phenyl-butanols, das nach allgemein bekanntem Verfahren hergestellt worden war, in Gegenwart von Kaliumcarbonat der (3RS,4RS)-3-Hydroxy-4-[4-(4-phenyl-butoxy)-phenyl]-piperidin-1-carbonsäure tert-butylester erhalten, dessen Alkylierung, analog Beispiel 1 (g), mit 2-Brommethyl-naphthalin den (3RS,4RS)-3-(Naphthalin-2-ylmethoxy)-4-[4-(4-phenyl-butoxy)-phenyl]-piperidin-1-carbonsäure tert-butylester als farbloses Öl lieferte; MS : 566 $(M+H)^+$.

(c) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des 5-Phenyl-pentanols, das nach allgemein bekanntem Verfahren hergestellt worden war, in Gegenwart von Kaliumcarbonat in DMF der (3RS,4RS)-3-Hydroxy-4-[4-(3-phenyl-propoxy)-phenyl]-piperidin-1-carbonsäure tert-butylester erhalten, dessen Alkylierung, analog Beispiel 1 (g), mit 2-Brommethyl-naphthalin den (3RS,4RS)-3-(Naphthalin-2-ylmethoxy)-4-[4-(5-phenyl-pentyloxy)-phenyl]-piperidin-1-carbonsäure tert-butylester als farblosen Festkörpen lieferte; MS : 580 $(M+H)^+$.

(d) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Cinnamylbromid in Gegenwart von Kaliumcarbonat in Aceton der (E)-(3RS,4RS)-3-Hydroxy-4-[4-(3-phenyl-allyloxy)-phenyl]-piperidin- 1-carbonsäure tert-butylester erhalten, dessen Alkylierung, analog Beispiel 1 (g), mit 2-Brommethyl-naphthalin den (E) -(3RS,4RS)-3-(Naphthalin-2-ylmethoxy)-4-[4-(3-phenyl-allyloxy)-phenyl]-piperidin-1-carbonsäure tert-butylester als farblosen Festkörper lieferte; MS : 550 $(M+H)^+$.

(e) Die Alkylierung des (E)-(3RS,4RS)-3-Hydroxy-4-[4-(3-phenyl-allyloxy)-phenyl]-piperidin-1-carbonsäure tert-butylesters, analog Beispiel 1 (g), mit 2-Chlormethyl-1,4-dimethoxy-naphthalin [J.Org.Chem. (1983), 48(19), 3265-3268] ergab den (E)-(3RS,4RS)-3-(1,4-Dimethoxy-naphthalin-2-ylmethoxy)-4-[4-(3-phenyl-allyloxy)-phenyl]-piperidin-1-carbonsäure tert-butylester als gelblichen Festkörper; MS : 610 $(M+H)^+$.

(f) Die Alkylierung des (E)-(3RS,4RS)-3-Hydroxy-4-[4-(3-phenyl-allyloxy)-phenyl]-piperidin-1-carbonsäure tert-butylesters, analog Beispiel 1 (g), mit 4-Methylthio-benzylchlorid [J.Org.Chem. (1988), 53(3), 561-569] ergab den (E)-(3RS,4RS)-3-(4-Methylsulfanyl-benzyloxy)-4-[4-(3-phenylallyloxy)-phenyl]-piperidin-1-carbonsäure tert-butylester als gelblichen Festkörper; MS : 546 $(M+H)^+$.

(g) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des (E)-4-Phenyl-3-buten-1-ols der (E)-(3RS,4RS)-3-Hydroxy-4-[4-(4-phenyl-but-3-enyloxy)-phenyl]-piperidin-1-carbonsäure tert-butylester erhalten, dessen Alkylierung, analog Beispiel 1 (g), mit 2-Chlormethyl-1,4-dimethoxy-naphthalin den (E)-(3RS,4RS)-3-(1,4-Dimethoxy-naphthalin-2-ylmethoxy)-4-[4-(4-phenyl-but-3-enyloxy)-phenyl]-piperidin-1-carbonsäure tert-butylester als viskoses, hellgelbes Öl lieferte; MS : 624 $(M+H)^+$.

Der als Alkylierungsmittel eingesetzte Methansulfonsäure (E)-4-phenyl-but-3-enylester wurde wie folgt hergestellt:

($\alpha$) Eine Lösung von 3.24 (20 mMol) (E)-Styrylessigsäure in 20 ml Methanol, 2 ml Trimethylorthoformiat und 192 mg (2 mMol) Methansulfonsäure wurde währen einer Stunde bei 50 °C unter Argon gerührt. Zur Aufarbeitung wurde mit 2 mMol Natriummethylat neutralisiert und anschließend das Lösungsmittelgemisch unter vermindertem Druck abdestilliert. Man erhielt den (E)-4-Phenyl-but-3-ensäure methylester als farblose Flüsigkeit in quantitativer Ausbeute; MS : 176 $(M)^+$.

($\beta$) In analoger Weise wie in Beispiel 5 (b) beschrieben, wurde durch Reduktion des (E)-4-Phenyl-but-3-ensäure methylesters mittels Lithiumaluminiumhydrid in Tetrahydrofuran das (E)-4-Phenyl-but-3-en-l-ol erhalten, das in Analogie zu dem zur Herstellung des (Z)-Isomers in J.Chem.Soc. Perk.Trans. 1 (1988), (6), 1517-1519 beschriebenen Verfahren zum Methansulfonsäure (E)-4-phenyl-but-3-enylester umgesetzt wurde.

(h) In analoger Weise wie in Beispiel 1 (g) beschrieben, wurde durch Alkylierung des (3RS,4RS)-4-(4-Allyloxy-phenyl)-3-hydroxy-piperidin-1-carbonsäure tert-butylesters [Beispiel 86 (b)] mit 2-Chlormethyl-1-(2-methoxy-äthoxy)-naphthalin der (3RS,4RS)-4-(4-Allyloxy-phenyl)-3- [1-(2-methoxy-äthoxy)-naphthalin-2-ylmethoxy]-piperidin-1-carbonsäure tert-butylester erhalten, aus dem nach Abspaltung der Allyl-Gruppe mittels Bis-(triphenylphosphin)-palladium(II)-diacetat, analog Beispiel 152 (e), der (3RS,4RS)-4-(4-Hydroxy-phenyl)-3-[1-(2-methoxy-äthoxy)-naphthalin-2-ylmethoxy]-piperidin-1-carbonsäure tert-butylester resultierte, dessen Alkylierung, analog Beispiel 44 (e), mit 3-Brommethyl-benzonitril den (3RS,4RS)-4-[4-(3-Cyano-benzyloxy)-phenyl]-3-[1-(2-methoxy-äthoxy)-naphthalin-2-ylmethoxy]-piperidin-1-carbonsäure tert-butylester als farbloses Öl ergab; MS : 623 $(M+H)^+$.

Das als Alkylierungsmittel eingesetzte 2-Chlormethyl-1-(2-methoxy-äthoxy)-naphthalin wurde wie folgt erhalten:

Durch Alkylierung des 1-Hydroxy-naphthalin-2-carbonsäure methylesters, analog Beispiel 1 (g), mit 2-Bromäthyl-methyläther wurde der 1-(2-Methoxy-äthoxy)-naphthalin-2-carbonsäure methylester erhalten, der in der Folge, analog Beispiel 5 (b)-(c), zunächst zum [1-(2-Methoxy-äthoxy)-naphthalin-2-yl]-methanol und dann zum 2-Chlormethyl-1-(2-methoxy-äthoxy)-naphthalin umgesetzt wurde, das schließlich als beiger Festkörper erhalten wurde; MS : 250 $(M)^+$.

(i) In analoger Weise wie in Beispiel 1 (g) beschrieben, wurde durch Alkylierung des (3RS,4RS)-3-Hydroxy-4-[4-(4-phenyl-butoxy)-phenyl]-piperidin-1-carbonsäure tert-butylesters [Beispiel 122 (b)] mit 2-Chlormethyl-1,4-dimethoxy-naphthalin [J.Org.Chem. (1983), 48(19),3265-3268] der (3RS,4RS)-3-(1,4-Dimethoxy-naphthalin-2-ylmethoxy)-4-[4-(4-phenyl-butoxy)-phenyl]-piperidin-1-carbonsäure tert-butylester als farbloses viskoses Öl erhalten; MS : 626 $(M+H)^+$.

Beispiel 123

Durch Abspaltung der BOC-Gruppe wurden die folgenden Verbindungen erhalten:

1) - Aus dem (3RS,4RS)-4-[4-(3-Benzyloxy-propoxy)-phenyl]-3-(1,4-dimethoxy-naphthalin-2-ylmethoxy)-piperidin-1-carbonsäure tert-butylester mittels Zinkbromid in Methylenchlorid das (3RS,4RS)-4-[4-(3-Benzyloxy-propoxy)-phenyl]-3-(1,4-dimethoxy-naphthalin-2-ylmethoxy)-piperidin Hydrobromid als beiger Festkörper; MS : 542 $(M+H)^+$;

2) - aus dem (3RS,4RS)-3-(1,4-Dimethoxy-naphthalin-2-ylmethoxy)-4-{4-[3-(thiophen-2-ylmethoxy)-propoxy]-phenyl}-piperidin-1-carbonsäure tert-butylester mittels Zinkbromid in Methylenchlorid das {3RS,4RS}-3-{1,4-Dimethoxy-naphthalin-2-ylmethoxy}-4-[4-[3-(thiophen-2-ylmethoxy)-propoxy]-phenyl]-piperidin als beiger Festkörper; MS : 548 $(M+H)^+$;

3) - aus dem (3RS,4RS)-3-(4-Methylsulfanyl-benzyloxy)-4-[4-[3-(thiophen-2-ylmethoxy)-propoxy]-phenyl]-piperidin-1-carbonsäure tert-butylester mittels Zinkbromid in Methylenchlorid das (3RS,4RS)-3-(4-Methylsulfanyl-benzyloxy)-4-[4-[3-(thiophen-2-ylmethoxy)-propoxy]-phenyl]-piperidin als gelbliches Harz; MS : 484 $(M+H)^+$;

4) - aus dem (3RS,4RS)-4-[4-(3-Benzylsulfanyl-propoxy)-phenyl]-3-(1,4-dimethoxy-naphthalin-2-ylmethoxy)-piperidin-1-carbonsäure tert-butylester mittels Zinkbromid in Methylenchlorid das (3RS,4RS)-4-[4-(3-Benzylsulfanyl-propoxy)-phenyl]-3-(1,4-dimethoxy-naphthalin-2-ylmethoxy)-piperidin als gelber Festkörper; MS : 558 $(M+H)^+$;

5) - aus dem (3RS,4RS)-4-[4-(3-Benzylsulfanyl-propoxy)-phenyl]-3-(naphthalin-2-ylmethoxy)-piperidin-1-carbonsäure tert-butylester mittels Trifluoressigsäure in Methylenchlorid das (3RS,4RS)-4-[4-(3-Benzylsulfanyl-propoxy)-phenyl]-3-(naphthalin-2-ylmethoxy)-piperidin Trifluoracetat als farbloser Festkörper; MS : 498 $(M+H)^+$;

6) - aus dem (3RS,4RS)-3-(Naphthalin-2-ylmethoxy)-4-[4-(2-phenäthyloxy-äthoxy)-phenyl]-piperidin-1-carbonsäure tert-butylester mittels Trifluoressigsäure in Methylenchlorid das (3RS,4RS)-3-(Naphthalin-2-ylmethoxy)-4-[4-(2-phenäthyloxy-äthoxy)-phenyl]-piperidin Trifluoracetat als farbloser Festkörper; MS : 482 $(M+H)^+$;

7) - aus dem (3RS,4RS)-3-(4-Methylsulfanyl-benzyloxy)-4-[4-(2-phenäthyloxy-äthoxy)-phenyl)-piperidin-1-carbonsäure tert-butylester mittels Zinkbromid in Methylenchlorid das (3RS,4RS)-3-(4-Methylsulfanyl-benzyloxy)-4-[4-(2-phenäthyloxy-äthoxy)-phenyl]-piperidin Hydrobromid als hellgelber Festkörper; MS : 478 $(M+H)^+$;

8) - aus dem (3RS,4RS)-3-(1,4-Dimethoxy-naphthalin-2-ylmethoxy)-4-[4-(2-phenäthyloxy-äthoxy)-phenyl]-piperidin-1-carbonsäure tert-butylester mittels Zinkbromid in Methylenchlorid das (3RS,4RS)-3-(1,4-Dimethoxy-naphthalin-2-ylmethoxy)-4-[4-(2-phenäthyloxy-äthoxy)-phenyl]-piperidin als farbloser Festkörper; MS : 542 $(M+H)^+$;

9) - aus dem (3RS,4RS)-3-(Naphthalin-2-ylmethoxy)-4-[4-[2-(pyrimidin-2-yloxy)-äthoxy]-phenyl]-piperidin-1-carbonsäure tert-butylester mittels Trifluoressigsäure in Methylenchlorid das (3RS,4RS)-2-(2-[4-[3-(Naphthalin-2-ylmethoxy)-piperidin-4-yl]-phenoxy]-äthoxy)-piperidin Trifluoracetat als farbloser Festkörper; MS : 456 $(M+H)^+$;

10) - aus dem (3RS,4RS)-3-(Naphthalin-2-ylmethoxy)-4-[4-(3-phenoxypropoxy)-phenyl)-piperidin-1-carbonsäure tert-butylester mittels Trifluoressigsäure in Methylenchlorid das (3RS,4RS)-3-(Naphthalin-2-ylmethoxy)-4-[4-(3-phenoxy-propoxy)-phenyl]-piperidin Trifluoracetat als farbloser Festkörper; MS : 468 $(M+H)^+$;

11) - aus dem (3RS,4RS)-3-(Naphthalin-2-ylmethoxy)-4-[4-(4-phenoxybutoxy)-phenyl]-piperidin-1-carbonsäure tert-butylester mittels Trifluoressigsäure in Methylenchlorid das (3RS,4RS)-3-(Naphthalin-2-ylmethoxy)-4-[4-(4-phenoxy-butoxy)-phenyl]-piperidin Trifluoracetat als farbloser Festkörper; MS : 482 $(M+H)^+$;

12) - aus dem (3RS,4RS)-3-(1,4-Dimethoxy-naphthalin-2-ylmethoxy)-4-[4-(4-phenoxy-butoxy)-phenyl]-piperidin-1-carbonsäure tert-butylester mittels Zinkbromid in Methylenchlorid das (3RS,4RS)-3-(1,4-Dimethoxynaphthalin-2-ylmethoxy)-4-[4-(4-phenoxy-butoxy)-phenyl]-piperidin als farbloser Festkörper; MS : 542 $(M+H)^+$;

13) - aus dem (3RS,4RS)-3-(1,4-Dimethoxy-naphthalin-2-ylmethoxy)-4-[4-(3-phenylsulfanyl-propoxy)-phenyl]-piperidin-1-carbonsäure tert-butylester mittels Zinkbromid in Methylenchlorid das (3RS,4RS)-3-(1,4-Dimethoxy-naphthalin-2-ylmethoxy)-4-[4-(3-phenylsulfanyl-propoxy)-phenyl]-piperidin als brauner Festkörper; MS : 544 $(M+H)^+$;

14) - aus dem (3RS,4RS)-3-(Naphthalin-2-ylmethoxy)-4-[4-(4-phenoxybut-2-ynyloxy)-phenyl]-piperidin-1-carbonsäure tert-butylester mittels Zinkbromid in Methylenchlorid das (3RS,4RS)-3-(Naphthalin-2-ylmethoxy)-4-[4-(4-phenoxy-but-2-ynyloxy)-phenyl]-piperidin als gelbes, viskoses Öl; MS : 478 $(M+H)^+$;

15) - aus dem (3RS,4RS)-3-(1,4-Dimethoxy-naphthalin-2-ylmethoxy)-4-[4-(4-phenoxy-but-2-ynyloxy)-phenyl] - piperidin-1-carbonsäure tert-butylester mittels Zinkbromid in Methylenchlorid das (3RS,4RS)-3-(1,4-Dimethoxy-naphthalin-2-ylmethoxy)-4-[4-(4-phenoxy-but-2-ynyloxy)-phenyl]-piperidin als brauner Festkörper; MS : 538 $(M+H)^+$;

16) - aus dem (E)-(3RS,4RS)-3-(Naphthalin-2-ylmethoxy)-4-[4-(4-phenoxy-but-2-enyloxy)-phenyl]-piperidin-1-carbonsäure tert-butylester mittels Zinkbromid in Methylenchlorid das (E)-(3RS,4RS)-3-(Naphthalin-2-ylmethoxy)-4-[4-(4-phenoxy-but-2-enyloxy)-phenyl]-piperidin als farbloser Festkörper; MS : 480 $(M+H)^+$;

17) - aus dem (Z)-(3RS,4RS)-3-(Naphthalin-2-ylmethoxy)-4-[4-(4-phenoxy-but-2-enyloxy)-phenyl]-piperidin-1-carbonsäure tert-butylester mittels Zinkbromid in Methylenchlorid das (Z)-(3RS,4RS)-3-(Naphthalin-2-ylmethoxy)-4-[4-(4-phenoxy-but-2-enyloxy)-phenyl]-piperidin als hellgelber Festkörper; MS : 480 $(M+H)^+$;

18) - aus dem (3RS,4RS)-3-(4,8-Dimethoxy-naphthalin-2-ylmethoxy)-4-{4-[3-(thiophen-2-ylmethoxy)-propoxy]-phenyl}-piperidin-1-carbonsäure tert-butylester mittels Zimkbromid in Methylenchlorid das (3RS,4RS)-3-(4,8-Dimethoxy-naphthalin-2-ylmethoxy)-4-[4-[3-(thiophen-2-ylmethoxy)-propoxy]-phenyl]-piperidin als beiger Festkörper; MS : 548 $(M+H)^+$;

19) - aus dem (3RS,4RS)-3-(7-Hydroxy-naphthalin-2-ylmethoxy)-4-{4-[3-(thiophen-2-ylmethoxy)-propoxy]-phenyl)-piperidin-1-carbonsäure tert-butylester mittels Chlorwasserstoff in Methanol das (3RS,4RS)-7-[4-[4-[3-(Thiophen-2-ylmethoxy)-propoxy]-phenyl]-piperidin-3-yloxymethyl]-naphthalin-2-ol als farbloser Festkörper; MS : 504 $(M+H)^+$;

20) - aus dem (3RS,4RS)-3-[8-Methoxy-4-(3-methoxy-propoxy)-naphthalin-2-ylmethoxy]-4-{4-[3-(thiophen-2-ylmethoxy)-propoxy]-phenyl}-piperidin-1-carbonsäure tert-butylester mittels Zinkbromid in Methylenchlorid das (3RS,4RS)-3-[8-Methoxy-4-(3-methoxy-propoxy)-naphthalin-2-ylmethoxy]-4-[4-[3-(thiophen-2-ylmethoxy)-propoxy]-phenyl]-piperidin als beiger Festkörper; MS : 606 $(M+H)^+$;

21) - aus dem Gemisch des (3RS,4RS)- und (3SR,4SR)-3-[7-[(RS)-2,3-Dimethoxy-propoxy]-naphthalin-2-ylmethoxy]-4-{4-[3-(thiophen-2-ylmethoxy)-propoxy]-phenyl}-piperidin-1-carbonsäure tert-butylesters mittels Zinkbromid in Methylenchlorid das Gemisch des (3RS,4RS)- and (3SR,4SR)-3-[7-[(RS)-2,3-Dimethoxy-propoxy]-naphthalin-2-ylmethoxy]-4-[4-[3-(thiophen-2-ylmethoxy)-propoxy]-phenyl]-piperidins als gelbes viskoses Öl; MS : 606 $(M+H)^+$;

22) - aus dem Gemisch des (3RS,4RS)- und (3SR,4SR)-3-[1-[(RS)-2,3-Dimethoxy-propoxy]-naphthalin-2-ylmethoxy]-4-{4-[3-(thiophen-2-ylmethoxy)-propoxy]-phenyl}-piperidin-1-carbonsäure tert-butylesters mittels Zinkbromid in Methylenchlorid das Gemisch des (3RS,4RS)- and (3SR,4SR)-3-[1-[(RS)-2,3-Dimethoxy-propoxy]-naphthalin-2-ylmethoxy]-4-[4-[3-(thiophen-2-ylmethoxy)-propoxy]-phenyl]-piperidins als gelbes viskoses Öl; MS : 606 $(M+H)^+$;

23) - aus dem (3RS,4RS)-3-(1,4-Dimethoxy-naphthalin-2-ylmethoxy)-4-{4-[3-(thiophen-3-ylmethoxy)-propoxy]-phenyl}-piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(thiophen-3-ylmethoxy)-propoxy]-phenyl]-piperidin als brauner Festkörper; MS : 548 $(M+H)^+$;

24) - aus dem (3RS,4RS)-3-(7-Hydroxy-naphthalin-2-ylmethoxy)-4-{4-[3-(thiophen-3-ylmethoxy)-propoxy]-phenyl}-piperidin-1-carbonsäure tert-butylester mittels Zinkbromid in Methylenchlorid das (3RS,4RS)-7-[4-[4-[3-(Thiophen-3-ylmethoxy)-propoxy]-phenyl]-piperidin-3-yloxymethyl]-naphthalin-2-ol als brauner Festkörper; MS : 504 $(M+H)^+$;

25) - aus dem (3RS,4RS)-3-[8-Methoxy-4-(3-methoxy-propoxy)-naphthalin-2-ylmethoxy]-4-{4-[3-(thiophen-3-ylmethoxy)-propoxy]-phenyl}-piperidin-1-carbonsäure tert-butylester mittels Zinkbromid in Methylenchlorid das (3RS,4RS)-3-[8-Methoxy-4-(3-methoxy-propoxy)-naphthalin-2-ylmethoxy]-4-[4-[3-(thiophen-3-ymethoxy)-propoxyl]-phenyl]-piperidin als gelbliches viskoses Öl; MS : 606 $(M+H)^+$;

26) - aus dem (3RS,4RS)-3-(1,4-Dimethoxy-naphthalin-2-ylmethoxy)-4-[4-(3-phenoxy-propoxy)-phenyl]-piperidin-1-carbonsäure tert-butylester mittels Zinkbromid in Methylenchlorid das (3RS,4RS)-3-(1,4-Dimethoxynaphthalin-2-ylmethoxy)-4-[4-(3-phenoxy-propoxy)-phenyl]-piperidin als gelbes viskoses Öl; MS : 528 $(M+H)^+$;

27) - aus dem (3RS,4RS)-3-(1,4-Dimethoxy-naphthalin-2-ylmethoxy)-4-{4-[3-(2-methoxy-phenoxy)-propoxy]-phenyl}-piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(2-methoxy-phenoxy)-propoxy]-phenyl]-piperidin als hellbrauner Festkörper; MS : 558 $(M+H)^+$;

28) - aus dem (3RS,4RS)-3-(1,4-Dimethoxy-naphthalin-2-ylmethoxy)-4-{4-[3-(3-methoxy-phenoxy)-propoxy]-phenyl}-piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(3-methoxy-phenoxy)-propoxy]-phenyl]-piperidin als gelbes viskoses Öl; MS : 558 $(M+H)^+$;

29) - aus dem (3RS,4RS)-4-{4-[3-(2-Chlor-phenoxy)-propoxy]-phenyl}-3-(1,4-dimethoxy-naphthalin-2-ylmethoxy)-piperidin-1-carbonsäure tert-butylester mittels Zinkbromid in Methylenchlorid das (3RS,4RS)-4-[4-[3-(2-Chlor-phenoxy)-propoxy]-phenyl]-3-(1,4-dimethoxy-naphthalin-2-ylmethoxy)-piperidin als gelbes halbfestes Produkt; MS : 562 $(M+H)^+$;

30) - aus dem (3RS,4RS)-3-(1,4-Dimethoxy-naphthalin-2-ylmethoxy)-4-{4-[3-(2-methoxy-phenylsulfanyl)-propoxy]-phenyl}-piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(2-methoxyphenylsulfanyl)-propoxy]-phenyl]-piperidin als gelbes viskoses Öl; MS : 574 $(M+H)^+$;

31) - aus dem (3RS,4RS)-4-[4-(3-Benzylsulfanyl-propoxy)-phenyl]-3-(4,8-dimethoxy-naphthalin-2-ylmethoxy)-piperidin-1-carbonsäure tert-butylester mittels Zinkbromid in Methylenchlorid das (3RS,4RS)-4-[4-(3-Benzylsulfanyl-propoxy)-phenyl]-3-(4,8-dimethoxy-naphthalin-2-ylmethoxy)-piperidin als farbloser Festkörper; MS : 558 $(M+H)^+$;

32) - aus dem (3RS,4RS)-3-(1,4-Dimethoxy-naphthalin-2-ylmethoxy)-4-[4-[3-(2-methoxy-benzyloxy)-propoxy]-phenyl) -piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(2-methoxy-benzyloxy)-propoxy]-phenyl]-piperidin als gelber Sirup; MS : 572 $(M+H)^+$;

33) - aus dem 1:1-Gemisch des (3R,4R)- und (3S,4S)-3-[7-[(R)-2-Hydroxy-3-morpholin-4-yl-propoxy]-naphthalin-2-ylmethoxy]-4-[4-[3-(thiophen-2-ylmethoxy)-propoxy]-phenyl)-piperidin-1-carbonsäure tert-butylesters mittels Chlorwasserstoff in Methanol das 1:1 Gemisch des (R)-1-Morpholin-4-yl-3-[(3R,4R)- und -[(3S,4S)-7-[4-[4-[3-(thiophen-2-ylmethoxy)-propoxy]-phenyl]-piperidin-3-yloxymethyl]-naphthalin-2-yloxy]-propan-2-ol Dihydrochlorid als beiger Festkörper; MS : 647 $(M+H)^+$;

34) - aus dem (3RS,4RS)-3-(1,4-Dimethoxy-naphthalin-2-ylmethoxy)-4-[4-(2-phenäthyloxy-äthoxy)-phenyl)-piperidin-1-carbonsäure tert-butylester mittels Zinkbromid in Methylenchlorid das (3RS,4RS)-3-(1,4-Dimethoxy-naphthalin-2-ylmethoxy)-4-[4-(2-phenäthyloxy-äthoxy)-phenyl)-piperidin, das wie folgt weiter oxidiert wurde:

Zu einer Lösung von 118 mg (0.22 mMol) (3RS,4RS)-3-(1,4-Dimethoxynaphthalin-2-ylmethoxy)-4-[4-(2-phenäthyloxy-äthoxy)-phenyl]-piperidin in 10 ml Acetonitril wurde bei Raumtemperatur eine Lösung von 240 mg Cer(IV)-ammoniumnitrat in 1 ml Wasser getropft. Die Reaktionslösung wurde 15 Minuten lang bei Raumtemperatur gerührt und anschließend unter vermindertem Druck eingedampft. Der Rückstand wurde zwischen Methylenchlorid und Wasser verteilt, die organische Phase getrocknet und unter vermindertem Druck eingedampft. Das Rohprodukt wurde zur Reinigung an Kieselgel unter Verwendung eines 9:1-Gemisches von Methylenchlorid und Methanol als Eluierungsmittel chromatographiert. Es wurden 95 mg (85 % d.Th.) (3RS,4RS)-2-[4-[4-(2-Phenäthyloxy-äthoxy)-phenyl]-piperidin-3-yloxymethyl]-[1,4]naphthochinon als roter Festkörper erhalten; MS : 512 $(M+H)^+$

Die als Ausgangssubstanzen eingesetzten BOC-Derivate wurden wie folgt hergestellt:

(a) In analoger Weise wie in Beispiel 1 (g) beschrieben, wurde durch Alkylierung des (3RS,4RS)-4-[4-(3-Benzyloxy-propoxy)-phenyl]-3-hydroxy-piperidin-1-carbonsäure tert-butylesters [Beispiel 86 (n)] mit 2-Chlormethyl-1,4-dimethoxy-naphthalin [J.Org.Chem. (1983), 48(19),3265-3268) der (3RS,4RS)-4-[4-(3-Benzyloxy-propoxy)-phenyl]-3-(1,4-dimethoxy-naphthalin-2-ylmethoxy)-piperidin-1-carbonsäure tert-butylester als farbloser Festkörper erhalten; MS : 642 $(M+H)^+$.

(b) In analoger Weise wie in Beispiel 1 (g) beschrieben, wurde durch Alkylierung des (RS)-2-(3-Brom-propoxy)-tetrahydro-pyrans mit 2-Hydroxymethyl-thiophen in DMF das (RS)-2-[3-(Thiophen-2-ylmethoxy)-propoxy]-tetrahydro-pyran erhalten, das nach Abspaltung der THP-Gruppe, analog Beispiel 53 (c), das 3-(Thiophen-2-ylmethoxy)-propan-1-ol lieferte. Die darauffolgende Umwandlung zum Mesylat nach literaturbekanntem Verfahren und die damit erfolgende Alkylierung, analog Beispiel 44 (e), des (3RS,4RS)-3-Hydroxy-4-(4-hydroxy-phenyl)-piperidin-1-carbonsäure tert-butylesters [Beispiel 46 (b)] ergab den (3RS,4RS)-3-Hydroxy-4-[4-[3-(thiophen-2-ylmethoxy)-propoxy]-phenyl]-piperidin-1-carbonsäure tert-butylester . Durch Alkylierung mit 2-Chlormethyl-1,4-dimethoxy-naphthalin, analog Beispiel 1 (g), wurde der (3RS,4RS)-3-(1,4-Dimethoxy-naphthalin-2-ylmethoxy)-4-{4-[3-(thiophen-2-ylmethoxy)-propoxy]-phenyl)-piperidin-1-carbonsäure tert-butylester als hellgelbes Harz erhalten; MS : 648 $(M+H)^+$.

(c) In analoger Weise wie in Beispiel 1 (g) beschrieben, wurde durch Alkylierung des (3RS,4RS)-3-Hydroxy-4-[4-[3-(thiophen-2-ylmethoxy)-propoxy]-phenyl]-piperidin-1-carbonsäure tert-butylesters mit 4-Methylthio-benzylchlorid [J.Org.Chem. (1988), 53(3), 561-569] der (3RS,4RS)-3-(4-Methylsulfanyl-benzyloxy)-4-[4-[3-(thiophen-2-ylmethoxy)-propoxy]-phenyl]-piperidin-1-carbonsäure tert-butylester als gelbliches Öl erhalten; MS : 584 $(M+H)^+$.

(d) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit (3-Brompropylsulfanylmethyl)-benzol [J.Org.Chem. (1986), 51, 846-850] in Gegenwart von Kaliumcarbonat der (3RS,4RS)-4-[4-(3-Benzylsulfanylpropoxy)-phenyl]-3-hydroxy-piperidin-1-carbonsäure tert-butylester erhalten, dessen Alkylierung mit 2-Chlormethyl-1,4-dimethoxynaphthalin, analog Beispiel 1 (g), den (3RS,4RS)-4-[4-(3-Benzylsulfanylpropoxy)-phenyl]-3-(1,4-dimethoxy-naphthalin-2-ylmethoxy)-piperidin-1-carbonsäure tert-butylester als hellgelbes Öl ergab; MS : 584 $(M+H)^+$.

(e) In analoger Weise wie in Beispiel 1 (g) beschrieben, wurde durch Alkylierung des (3RS,4RS)-4-[4-(3-Benzylsulfanyl-propoxy)-phenyl]-3-hydroxy-piperidin-1-carbonsäure tert-butylesters mit 2-Brommethyl-naphthalin der (3RS,4RS)-4-[4-(3-Benzylsulfanyl-propoxy)-phenyl]-3-(naphthalin-2-ylmethoxy)-piperidin-1-carbonsäure tert-butylester als farbloses Harz erhalten; MS : 598 $(M+H)^+$.

(f) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des 2-Phenäthyloxy-äthanols [J.Med.Chem. (1983), 26 (11), 1570-1576], hergestellt nach literaturbekanntem Verfahren, in Gegenwart von Kaliumcarbonat der (3RS,4RS)-3-Hydroxy-4-[4-(2-phenäthyloxy-äthoxy)-phenyl]-piperidin-1-carbonsäure tert-butylester erhalten, dessen Alkylierung, analog Beispiel 1 (g), den (3RS,4RS)-3-(Naphthalin-2-ylmethoxy)-4-[4-(2-phenäthyloxy-äthoxy)-phenyl]-piperidin-1-carbonsäure tert-butylester als farbloses Öl lieferte; MS: 582 $(M+H)^+$.

(g) In analoger Weise wie in Beispiel 1 (g) beschrieben, wurde durch Alkylierung des (3RS,4RS)-3-Hydroxy-4-[4-(2-phenäthyloxy-äthoxy)-phenyl]-piperidin-1-carbonsäure tert-butylesters mit 4-Methylthio-benzylchlorid [J. Org.Chem. (1988), 53(3), 561-569] der (3RS,4RS)-3-(4-Methylsulfanyl-benzyloxy)-4-[4-(2-phenäthyloxy-äthoxy)-phenyl]-piperidin-1-carbonsäure tert-butylester als farbloses Öl erhalten; MS : 578 $(M+H)^+$.

(h) In analoger Weise wie in Beispiel 1 (g) beschrieben, wurde durch Alkylierung des (3RS,4RS)-3-Hydroxy-4-[4-(2-phenäthyloxy-äthoxy)-phenyl]-piperidin-1-carbonsäure tert-butylesters mit 2-Chlormethyl-1,4-dimethoxy-naphthalin der (3RS,4RS)-3-(1,4-Dimethoxy-naphthalin-2-ylmethoxy)-4-[4-(2-phenäthyloxy-äthoxy)-phenyl]-piperidin-1-carbonsäure tert-butylester als gelber Festkörper erhalten; MS : 642 $(M+H)^+$.

(i) In analoger Weise wie in Beispiel 1 (g) beschrieben, wurde durch Alkylierung des (3RS,4RS)-4-[4-(2-Hydroxy-äthoxy)-phenyl]-3-(naphthalin-2-ylmethoxy)-piperidin-1-carbonsäure tert-butylesters [Beispiel 53 (c)] mit 2-Chlor-pyrimidin der (3RS,4RS)-3-(Naphthalin-2-ylmethoxy)-4-[4-[2-(pyrimidin-2-yloxy)-äthoxy]-phenyl]-piperidin-1-carbonsäure tert-butylester als farbloser Festkörper erhalten; MS : 556 $(M+H)^+$.

(j) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des 3-Phenoxy-propanols, das nach literaturbekanntem Verfahren hergestellt worden war, der (3RS,4RS)-3-Hydroxy-4-[4-(3-phenoxy-propoxy)-phenyl]-piperidin-1-carbonsäure tert-butylester erhalten, dessen Alkylierung mit 2-Brommethyl-naphthalin, analog Beispiel 1 (g), den (3RS,4RS)-3-(Naphthalin-2-ylmethoxy)-4-[4-(3-phenoxy-propoxy)-phenyl]-piperidin-1-carbonsäure tert-butylester als farblosen Festkörper lieferte; MS : 568 $(M+H)^+$.

(k) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit (E)-(4-Brom-but-2-enyloxy)-benzol der (E)-(3RS,4RS)-3-Hydroxy-4-[4-(4-phenoxy-but-2-enyloxy)-phenyl]-piperidin-1-carbonsäure tert-butylester erhalten. Die anschließende Hydrierung mit Palladium/Kohle, analog Beispiel 73 (c) lieferte den (3RS,4RS)-3-Hydroxy-4-[4-(4-phenoxy-butoxy)-phenyl]-piperidin-1-carbonsäure tert-butylester, dessen Alkylierung mit 2-Brommethyl-naphthalin, analog Beispiel 1 (g), den (3RS,4RS)-3-(Naphthalin-2-ylmethoxy)-4-[4-(4-phenoxy-butoxy)-phenyl]-piperidin-1-carbonsäure tert-butylester als farblosen Festkörper ergab; MS : 582 $(M+H)^+$.

Das als Alkylierungsmittel eingesetzte (E)-(4-Brom-but-2-enyloxy)-benzol wurde, in analoger Weise wie in Beispiel 44 (e) beschrieben, durch Alkylierung von Phenol mit 1,4-Dibrom-2-buten erhalten.

(1) In analoger Weise wie in Beispiel 1 (g) beschrieben, wurde durch Alkylierung des (3RS,4RS)-3-Hydroxy-4-[4-(4-phenoxy-butoxy)-phenyl]-piperidin-1-carbonsäure tert-butylesters mit 2-Chlormethyl-1,4-dimethoxy-naphthalin der (3RS,4RS)-3-(1,4-Dimethoxy-naphthalin-2-ylmethoxy)-4-[4-(4-phenoxy-butoxy)-phenyl]-piperidin-1-carbonsäure tert-butylester als hellgelber Festkörper erhalten; MS : 642 $(M+H)^+$.

(m) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit dem Mesylat des 3-Phenylthio-propanols, das nach literaturbekanntem Verfahren hergestellt worden war, der (3RS,4RS)-3-Hydroxy-4-[4-(3-phenylsulfanyl-propoxy)-phenyl]-piperidin-1-carbonsäure tert-butylester erhalten, dessen Alkylierung, analog Beispiel 1 (g), mit 2-Chlormethyl-1,4-dimethoxy-naphthalin den (3RS,4RS)-3-(1,4-Dimethoxy-naphthalin-2-ylmethoxy)-4-[4-(3-phenylsulfanyl-propoxy)-phenyl]-piperidin-1-carbonsäure tert-butylester als gelbes, viskoses Öl lieferte; MS : 644 $(M+H)^+$.

(n) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Methansulfonsäure 4-phenoxy-but-2-ynylester der (3RS,4RS)-3-Hydroxy-4-[4-(4-phenoxy-but-2-ynyloxy)-phenyl]-piperidin-1-carbonsäure tert-butylester erhalten, dessen Alkylierung mit 2-Brommethyl-naphthalin den (3RS,4RS)-3-(Naphthalin-2-ylmethoxy)-4-[4-(4-phenoxy-but-2-ynyloxy)-phenyl]-piperidin-1-carbonsäure tert-butylester als hellgelbes, viskoses Öl ergab; MS : 578 $(M+H)^+$.

Der als Alkylierungsmittel eingesetzte Methansulfonsäure 4-phenoxybut-2-ynylester wurde, in analoger Weise wie in Beispiel 44 (e) beschrieben, durch Alkylierung von Phenol mit dem Dimesylat des 2-Butyn-1,4-diols, das nach literaturbekanntem Verfahren hergestellt worden war, erhalten.

(o) In analoger Weise wie in Beispiel 1 (g) beschrieben, wurde durch Alkylierung des (3RS,4RS)-3-Hydroxy-4-[4-(4-phenoxy-but-2-ynyloxy)-phenyl]-piperidin-1-carbonsäure tert-butylesters mit 2-Chlormethyl-l,4-dimethoxy-naphthalin der (3RS,4RS)-3-(1,4-Dimethoxy-naphthalin-2-ylmethoxy)-4-[4-(4-phenoxy-but-2-ynyloxy)-phenyl]-piperidin-1-carbonsäure tert-butylester als hellgelber Festkörper erhalten; MS : 638 $(M+H)^+$.

(p) In analoger Weise wie in Beispiel 1 (g) beschrieben, wurde durch Alkylierung des (E)-(3RS,4RS)-3-Hydroxy-4- [4-(4-phenoxy-but-2-enyloxy)-phenyl] -piperidin-1-carbonsäure tert-butylesters [Beispiel 123 (k)] mit 2-Brommethyl-naphthalin der (E)-(3RS,4RS)-3-(Naphthalin-2-ylmethoxy)-4-[4-(4-phenoxy-but-2-enyloxy)-phenyl]-piperidin-1-carbonsäure tert-butylester als farbloser Festkörper erhalten; MS : 580 (M+H)+.

(q) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Methansulfonsäure (Z)-4-phenoxy-but-2-enylester der (Z)-(3RS,4RS)-3-Hydroxy-4-[4-(4-phenoxybut-2-enyloxy)-phenyl]-piperidin-1-carbonsäure tert-butylester erhalten, dessen Alkylierung mit 2-Brommethyl-naphthalin den (Z)-(3RS,4RS)-3-(Naphthalin-2-ylmethoxy)-4-[4-(4-phenoxy-but-2-enyloxy)-phenyl]-piperidin-1-carbonsäure tert-butylester als farblosen Festkörper ergab; MS : 580 $(M+H)^+$.

Der als Alkylierungsmittel eingesetzte Methansulfonsäure (Z)-4-phenoxy-but-2-enylester wurde, in analoger Weise wie in Beispiel 44 (e) beschrieben, durch Alkylierung von Phenol mit dem Methansulfonsäure (Z)-4-methylsulfonyloxy-but-2-enylester, der nach literaturbekanntem Verfahren aus (Z)-2-Buten-1,4-diol hergestellt worden war, erhalten.

(r) In analoger Weise wie in Beispiel 1 g) beschrieben, wurde durch Alkylierung des (3RS,4RS)-3-Hydroxy-4-[4-[3-(thiophen-2-ylmethoxy)-propoxy]-phenyl]-piperidin-1-carbonsäure tert-butylesters [Beispiel 123 b)] mit 2-Chlormethy-4,8-dimethoxy-naphthalin der (3RS,4RS)-3-(4,8-Dimethoxy-naphthalin-2-ylmethoxy)-4-(4-[3-(thiophen-2-ylmethoxy)-propoxy]-phenyl)-piperidin-1-carbonsäure tert-butylester als beiger Festkörper erhalten; MS : 648 $(M+H)^+$.

Das als Alkylierungsmittel eingesetzte 2-Chlormethy-4,8-dimethoxynaphthalin wurde wie folgt erhalten:

(a) In analoger Weise wie in Beispiel 5 b) beschrieben, wurde durch Reduktion des 4,8-Dimethoxy-naphthalin-2-carbonsäure methylesters (J.Chem.Soc. 1959, 1024) mittels Lithiumaluminiumhydrid das (4,8-Dimethoxy-naphthalin-2-yl)-methanol, MS : 218 $(M)^+$, als farbloser Festkörper erhalten.

(β) Zu einer auf-10 °C abgekühlten Lösung von 7.7 g (35.3 mMol) (4,8-Dimethoxy-naphthalin-2-yl)-methanol und 4.4 g (38.8 mMol) Triäthylamin in 50 ml Methylenchlorid wurde eine Lösung von 3.92 g Methansulfonsäurechlorid in 20 ml Methylenchlorid getropft. Das Reaktionsgemisch wurde 18 Stunden lang bei Raumtemperatur gerührt. Zur Aufarbeitung wurde mit 50 ml eiskalter Natriumhydrogencarbonat-Lösung ausgeschüttelt, die wäßrige Phase abgetrennt und mit 25 ml Methylenchlorid nachextrahiert. Die vereinigten organischen Phasen wurden über Natriumsulfat getrocknet und anschließend unter verminderten Druck eingedampft. Der Rückstand wurde zur Reinigung über eine Schicht Kieselgel filtriert unter Verwendung von Methylenchlorid als Eluierungsmittel.

Es wurden 7.2 g 2-Chlormethy-4,8-dimethoxy-naphthalin als beiger Festkörper erhalten; MS : 296 $(M)^+$

(s) In analoger Weise wie in Beispiel 1 g) beschrieben, wurde durch Alkylierung des (3RS,4RS)-3-Hydroxy-4-[4-[3-(thiophen-2-ylmethoxy)-propoxy]-phenyl]-piperidin-1-carbonsäure tert-butylesters [Beispiel 123 b)] mit 2-Chlormethyl-7-(b-trimethyl-silyläthoxymethoxy)-naphthalin [Beispiel 6 u)] der (3RS,4RS)-4-{4-[3-(Thiophen-2-ylmethoxy)-propoxy]-phenyl}-3-[7-(2-trimethylsilanyl-äthoxymethoxy)-naphthalin-2-ylmethoxy]-piperidin-1-carbonsäure tert-butylester als farbloses viskoses Öl erhalten, MS : 734 $(M+H)^+$, aus dem durch Abspaltung der SEM-Gruppe mittels Chlorwasserstoff in Methanol der (3RS,4RS)-3-(7-Hydroxy-naphthalin-2-ylmethoxy)-4-{4-[3-(thiophen-2-ylmethoxy)-propoxy]-phenyl}-piperidin-1-carbonsäure tert-butylester als farbloser Festkörper erhalten wurde; MS : 604 $(M+H)^+$.

(t) In analoger Weise wie in Beispiel 1 g) beschrieben, wurde durch Alkylierung des (3RS,4RS)-3-Hydroxy-4-[4-[3-(thiophen-2-ylmethoxy)-propoxy] -phenyl]-piperidin-1-carbonsäure tert-butylesters [Beispiel 123 b)] mit 3-Chlormethyl-5-methoxy-1-(3-methoxy-propoxy)-naphthalin der (3RS,4RS)-3-[8-Methoxy-4-(3-methoxy-propoxy)-naphthalin-2-ylmethoxy]-4-{4-[3-(thiophen-2-ylmethoxy)-propoxy]-phenyl}-piperidin-1-carbonsäure tert-butylester als hellgelbes viskoses Öl erhalten; MS : 706 $(M+H)^+$.

Das als Alkylierungsmittel eingesetzte 3-Chlormethyl-5-methoxy-1-(3-methoxy-propoxy)-naphthalin wurde wie folgt hergestellt:

In analoger Weise wie in Beispiel 44 e) beschrieben, wurde durch Alkylierung des 4-Hydroxy-8-methoxy-naphthalin-2-carbonsäure methylesters [Justus Liebigs Ann.Chem. (1967), 702, 94-100] mit dem Mesylat des 3-Methoxy-butan-1-ols, hergestellt nach literaturbekanntem Verfahren, der 8-Methoxy-4-(3-methoxy-propoxy)-naphthalin-2-carbonsäure äthylester erhalten, dessen Reduktion mit Lithiumaluminiumhydrid, analog Beispiel 5 (b), das [8-Methoxy-4-(3-methoxy-propoxy)-naphthalin-2-yl]-methanol ergab. Die sich anschließende Chlorierung, analog Beispiel 123 (r) (β), lieferte das 3-Chlormethyl-5-methoxy-1-(3-methoxy-propoxy)-naphthalin als hellgelbe Flüssigkeit; MS : 276 $(M)^+$.

(u) In analoger Weise wie in Beispiel 1 (g) beschrieben, wurde durch Alkylierung des (3RS,4RS)-3-Hydroxy-4-[4-[3-(thiophen-2-ylmethoxy)-propoxy]-phenyl]-piperidin-1-carbonsäure tert-butylesters [Beispiel 123 b)] mit (RS)-2-Chlormethyl-7-(2,3-dimethoxy-propoxy)-naphthalin das Gemisch des (3RS,4RS)- und (3SR,4SR)-3-[7-[(RS)-2,3-Dimethoxypropoxy]-naphthalin-2-ylmethoxy]-4-{4-[3-(thiophen-2-ylmethoxy)-propoxy]-phenyl}-piperidin-1-carbonsäure tert-butylesters als farbloses viskoses Öl erhalten; MS : 706 $(M+H)^+$.

Das als Alkylierungsmittel eingesetzte (RS)-2-Chlormethyl-7-(2,3-dimethoxy-propoxy)-naphthalin wurde wie folgt hergestellt:

In analoger Weise wie in Beispiel 44 (e) beschrieben, wurde durch Alkylierung des 7-Hydroxy-naphthalin-2-carbonsäure äthylesters (EPA 61800) mit dem Mesylat des (RS)-2,3-Dimethoxy-propan-1-ols (J.Chem.Soc. C, 1966, 415-419), hergestellt nach literaturbekannter Weise, der (RS)-7-(2,3-Dimethoxy-propoxy)-naphthalin-2-carbonsäure methylester erhalten, dessen Reduktion mit Lithiumaluminiumhydrid, analog Beispiel 5 (b), das (RS)-[7-(2,3-Dimethoxy-propoxy)-naphthalin-2-yl]-methanol ergab. Die sich anschließende Chlorierung, analog Beispiel 123 (r) (β), lieferte das (RS)-2-Chlormethyl-7-(2,3-dimethoxypropoxy)-naphthalin als farblosen Festkörper; MS : 294 $(M)^+$.

(v) In analoger Weise wie in Beispiel 1 (g) beschrieben, wurde durch Alkylierung des (3RS,4RS)-3-Hydroxy-4-[4-[3-(thiophen-2-ylmethoxy)-propoxy]-phenyl] -piperidin-1-carbonsäure tert-butylesters [Beispiel 123 (b)] mit (RS)-2-Chlormethyl-1-(2,3-dimethoxy-propoxy)-naphthalin das Gemisch des (3RS,4RS)- und (3SR,4SR)-3-[1-[(RS)-2,3-Dimethoxypropoxy]-naphthalin-2-ylmethoxy]-4-{4-[3-(thiophen-2-ylmethoxy)-propoxy] -phenyl}-piperidin-1-carbonsäure tert-butylesters als farbloses Öl erhalten; MS : 706 $(M+H)^+$.

Das als Alkylierungsmittel eingesetzte (RS)-2-Chlormethyl-1-(2,3-dimethoxy-propoxy)-naphthalin wurde wie folgt hergestellt:

In analoger Weise wie in Beispiel 44 (e) beschrieben, wurde durch Alkylierung des 1-Hydroxy-naphthalin-2-carbonsäure methylesters mit dem Mesylat des (RS)-2,3-Dimethoxy-propan-1-ols (J.Chem.Soc. C, 1966, 415-419), hergestellt nach literaturbekannter Weise, der (RS)-1-(2,3-Dimethoxy-propoxy)-naphthalin-2-carbonsäure methylester erhalten, dessen Reduktion mit Lithiumaluminiumhydrid, analog Beispiel 5 (b), das (RS)-[1-(2,3-Dimethoxy-propoxy)-naphthalin-2-yl]-methanol ergab. Die sich anschließende Chlorierung, analog Beispiel 123 (r) (β), lieferte das (RS)-2-Chlormethyl-1-(2,3-dimethoxy-propoxy)-naphthalin als farbloses viskoses Öl; MS : 294 $(M)^+$.

(w) In analoger Weise wie in Beispiel 1 (g) beschrieben, wurde durch Alkylierung des (RS)-2-(3-Brom-propoxy)-tetrahydro-pyrans mit 3-Hydroxymethyl-thiophen in DMF das (RS)-2-[3-(Thiophen-3-ylmethoxy)-propoxy]-tetrahydro-pyran erhalten, das nach Abspaltung der THP-Gruppe, analog Beispiel 53 (c), das 3-(Thiophen-3-ylmethoxy)-propan-1-ol lieferte. Die darauffolgende Umwandlung zum Mesylat nach literaturbekanntem Verfahren und die damit erfolgende Alkylierung, analog Beispiel 44 (e), des (3RS,4RS)-3-Hydroxy-4-(4-hydroxy-phenyl)-piperidin-1-carbonsäure tert-butylesters [Beispiel 46 (b)] ergab den (3R5,4RS)-3-Hydroxy-4-[4-[3-(thiophen-3-ylinethoxy)-propoxy)-phenyl]-piperidin-1-carbonsäure tert-butylester. Durch Alkylierung mit 2-Chlormethyl-1,4-dimethoxy-naphthalin, analog Beispiel 1 (g), wurde der (3RS,4RS)-3-(1,4-Dimethoxy-naphthalin-2-ylmethoxy)-4-{4-[3-(thiophen-3-ylmethoxy)-propoxy]-phenyl}-piperidin-1-carbonsäure tert-butylester als hellgelber Festkörper erhalten; MS : 648 $(M+H)^+$.

(x) In analoger Weise wie in Beispiel 1 (g) beschrieben, wurde durch Alkylierung des (3RS,4RS)-3-Hydroxy-4-[4-[3-(thiophen-3-ylmethoxy)-propoxy]-phenyl]-piperidin-1-carbonsäure tert-butylesters mit 2-Allyloxy-7-chlormethyl-naphthalin der (3RS,4RS)-3-(7-Allyloxynaphthalin-2-ylmethoxy)-4-{4-[3-(thiophen-3-ylmethoxy)-propoxy]-phenyl}-piperidin-1-carbonsäure tert-butylester erhalten, aus dem nach Abspaltung der Allyl-Gruppe mittels Bis-(triphenylphosphin)-palladium(II)-diacetat, analog Beispiel 152 (e), der (3RS,4RS)-3-(7-Hydroxy-naphthalin-2-ylmethoxy)-4-{4-[3-(thiophen-3-ylmethoxy)-propoxy]-phenyl}-piperidin-1-carbonsäure tert-butylester als gelbliches viskoses Öl erhalten wurde; MS : 604 $(M+H)^+$.

Das als Alkylierungsmittel eingesetzte 2-Allyloxy-7-chlormethylnaphthalin wurde wie folgt hergestellt:

In analoger Weise wie in Beispiel 44 (e) beschrieben, wurde durch Alkylierung des 7-Hydroxy-naphthalin-2-carbonsäure äthylesters (EPA 61800) mit Allylbromid der 7-Allyloxy-naphthalin-2-carbonsäure methylester erhalten, dessen Reduktion mit Lithiumaluminiumhydrid, analog Beispiel 5 (b), das (7-Allyloxy-naphthalin-2-yl)-methanol ergab. Die sich anschließende Chlorierung, analog Beispiel 123 (r) (β), lieferte das 2-Allyloxy-7-chlormethyl-naphthalinals farblosen Festkörper; MS : 232 $(M)^+$.

(y) In analoger Weise wie in Beispiel 1 (g) beschrieben, wurde durch Alkylierung des (3RS,4RS)-3-Hydroxy-4-[4-[3-(thiophen-3-ylmethoxy)-propoxy]-phenyl]-piperidin-1-carbonsäure tert-butylesters mit 3-Chlormethyl-5-methoxy-1-(3-methoxy-propoxy)-naphthalin [Beispiel 123 (t)] der (3RS,4RS)-3-[8-Methoxy-4-(3-methoxy-propoxy)-naphthalin-2-ylmethoxy]-4-{4-[3-(thiophen-3-ylmethoxy)-propoxy]-phenyl}-piperidin-1-carbonsäure tert-butylester als hellgelbes viskoses Öl erhalten; MS : 723 $(M+NH_4)^+$.

(z) In analoger Weise wie in Beispiel 1 (g) beschrieben, wurde durch Alkylierung des (3RS,4RS)-3-Hydroxy-4-[4-(3-phenoxy-propoxy)-phenyl]-piperidin-1-carbonsäure tert-butylesters [Beispiel 123 (j)] mit 2-Chlormethyl-1,4-dimethoxy-naphthalin der (3RS,4RS)-3-(1,4-Dimethoxynaphthalin-2-ylmethoxy)-4-[4-(3-phenoxy-propoxy)-phenyl]-piperidin-1-carbonsäure tert-butylester als viskoses gelbes Öl erhalten; MS : 628 $(M+H)^+$.

(aa) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit 1-(3-Brom-propoxy)-2-methoxy-benzol der (3RS,4RS)-3-Hydroxy-4-{4-[3-(2-methoxy-phenoxy)-propoxy]-phenyl}-piperidin-1-carbonsäure tert-butylester herhalten, dessen weitere Alkylierung mit 2-Chlormethyl-1,4-dimethoxynaphthalin den (3RS,4RS)-3-(1,4-Dimethoxy-naphthalin-2-ylmethoxy)-4-{4-[3-(2-methoxy-phenoxy)-propoxy]-phenyl}-piperidin-1-carbonsäure tert-butylesterals hellgelben Festkörper ergab; MS : 657 $(M)^+$.

Das als Alkylierungsmittel eingesetzte 1-(3-Brom-propoxy)-2-methoxybenzol wurde, analog Beispiel 44 (e), durch Alkylierung von 2-Methoxyphenol mit 1,3-Dibrom-propan als farblose Flüssigkeit erhalten; MS : 244, 246 $(M)^+$.

(bb) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit 1-(3-Brom-propoxy)-3-methoxy-benzol der (3RS,4RS)-3-Hydroxy-4-{4-[3-(3-methoxy-phenoxy)-propoxy]-phenyl}-piperidin-1-carbonsäure tert-butylester erhalten, dessen weitere Alkylierung mit 2-Chlormethyl-1,4-dimethoxynaphthalin den (3RS,4RS)-3-(1,4-Dimethoxy-naphthalin-2-ylmethoxy)-4-{4-[3-(3-methoxy-phenoxy)-propoxy]-phenyl}-piperidin-1-carbonsäure tert-butylester als hellgelben Festkörper ergab; MS : 658 $(M+H)^+$.

Das als Alkylierungsmittel eingesetzte 1-(3-Brom-propoxy)-3-methoxybenzol wurde, analog Beispiel 44 (e), durch Alkylierung von 3-Methoxyphenol mit 1,3-Dibrom-propan als farblose Flüssigkeit erhalten; MS : 244, 246 $(M)^+$.

(cc) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit 1-(3-Brom-propoxy)-2-chlorbenzol der (3RS,4RS)-4-{4-[3-(2-Chlor-phenoxy)-propoxy]-phenyl}-3-hydroxy-piperidin-1-carbonsäure tert-butylester erhalten, dessen weitere Alkylierung mit 2-Chlormethyl-1,4-dimethoxy-naphthalin den (3RS,4RS)-4-{4-[3-(2-Chlor-phenoxy)-propoxy]-phenyl}-3-(1,4-dimethoxynaphthalin-2-ylmethoxy)-piperidin-1-carbonsäure tert-butylester als farbloses viskoses Öl ergab; MS : 662 $(M+H)^+$.

Das als Alkylierungsmittel eingesetzte 1-(3-Brom-propoxy)-2-chlorbenzol wurde, analog Beispiel 44 (e), durch Alkylierung von 2-Chlorphenol mit 1,3-Dibrom-propan als farblose Flüssigkeit erhalten; MS : 248 $(M)^+$.

(dd) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Methansulfonsäure 3-(2-methoxy-phenylsulfanyl)-propylester der (3RS,4RS)-3-Hydroxy-4-{4-[3-(2-methoxy-phenylsulfanyl)-propoxy]-phenyl}-piperidin-1-carbonsäure tert-butylester erhalten, dessen weitere Alkylierung mit 2-Chlormethyl-1,4-dimethoxy-naphthalin den (3RS,4RS)-3-(1,4-Dimethoxy-naphthalin-2-ylmethoxy)-4-{4-[3-(2-methoxy-phenylsulfanyl)-propoxy]-phenyl}piperidin-1-carbonsäure tert-butylester als hellgelbes viskoses Öl ergab; MS : 674 $(M+H)^+$.

Der als Alkylierungsmittel eingesetzte Methansulfonsäure 3-(2-methoxy-phenylsulfanyl)-propylester wurde wie folgt hergestellt:

($\alpha$) In analoger Weise wie in Beispiel 44 e) beschrieben, wurde durch Alkylierung des 2-Methoxy-thiophenols mit (RS)-2-(3-Brom-propoxy)-tetrahydro-pyran das (RS)-2-[3-(2-Methoxy-phenylsulfanyl)-propoxy]-tetrahydro-pyran erhalten.

($\beta$) Eine Lösung von 9.5 g (RS)-2-[3-(2-Methoxy-phenylsulfanyl)-propoxy]-tetrahydro-pyran (33.6 mMol) und 1.0 g (4 mMol) Pyridinium-(toluol-4-sulfonat) in 100 ml Methanol wurde 2 Stunden lang zum Rückfluß erhitzt. Zur Aufarbeitung wurde das Lösungsmittel unter vermindertem Druck abdestilliert, dann der Rückstand zwischen Essigester und gesättigter Natriumhydrogencarbonat-Lösung verteilt. Die organische Phase wurde anschließend über Natriumsulfat getrocknet und unter vermindertem Druck eingedampft. Das 3-(2-Methoxy-phenylsulfanyl)-propan-1-ol wurde als gelbliche Flüssigkeit in quantitativer Ausbeute erhalten; MS : 198 $(M)^+$.

Die nach allgemein bekanntem Verfahren erfolgende Umsetzung mit Mesylchlorid lieferte den Methansulfonsäure 3-(2-methoxyphenylsulfanyl)-propylester als hellgelbe Flüssigkeit; MS : 276 $(M)^+$.

(ee) In analoger Weise wie in Beispiel 1 (g) beschrieben, wurde durch Alkylierung des (3RS,4RS)-4-[4-(3-Benzylsulfanyl-propoxy)-phenyl]-3-hydroxy-piperidin-1-carbonsäure tert-butylesters [Beispiel 123 d)] mit 3-Chlormethyl-1,5-dimethoxy-naphthalin [Beispiel 123 r)] der (3RS,4RS)-4-[4-(3-Benzylsulfanyl-propoxy)-phenyl]-3-(4,8-dimethoxy-naphthalin-2-ylmethoxy)-piperidin-1-carbonsäure tert-butylester als gelbes viskoses Öl erhalten; MS : 658 $(M+H)^+$.

(ff) In analoger Weise wie in Beispiel 1 (g) beschrieben, wurde durch Alkylierung des (3RS,4RS)-3-Hydroxy-4-[4-[3-(2-methoxy-benzyloxy)-propoxy]-phenyl]-piperidin-1-carbonsäure tert-butylesters mit 2-Chlormethyl-1,4-dimethoxy-naphthalin der (3RS,4RS)-3-(1,4-Dimethoxynaphthalin-2-ylmethoxy)-4-{4-[3-(2-methoxy-benzyloxy)-propoxy]-phenyl}-piperidin-1-carbonsäure tert-butylester als hellgelber Sirup erhalten; MS : 689 $(M+NH_4)^+$.

(gg) In analoger Weise wie in Beispiel 1 (g) beschrieben, wurde durch Alkylierung des (3RS,4RS)-3-(7-Hydroxy-naphthalin-2-ylmethoxy)-4-{4-[3-(thiophen-2-ylmethoxy)-propoxy]-phenyl}-piperidin-1-carbonsäure tert-butylesters [Beispiel 123 (s)] mit Toluol-4-sulfonsäure (R)-oxiranylmethylester das 1:1-Gemisch des (3R,4R)- und (3S,4S)-3-[(R)-7-Oxiranylmethoxy-naphthalin-2-ylmethoxy]-4-{4-[3-(thiophen-2-ylmethoxy)-propoxy]-phenyl}-piperidin-1-carbonsäure tert-butylesters, MS : 660 $(M+H)^+$, als gelbliches viskoses Öl erhalten. Die darauffolgende Epoxidöffnung, analog Beispiel 71 (a), mit Morpholin lieferte das 1:1-Gemisch des (3R,4R)- und (3S,4S)-3-[7-[(R)-2-Hydroxy-3-morpholin-4-yl-propoxy]-naphthalin-2-ylmethoxy]-4-{4-[3-(thiophen-2-ylmethoxy)-propoxy]-phenyl}-piperidin-1-carbonsäure tert-butyl esters als farblosen Festkörper; MS : 747 $(M+H)^+$.

<u>Beispiel 124</u>

Durch Abspaltung der BOC-Gruppe wurden die folgenden Verbindungen erhalten:

1) - Aus (3RS,4RS)-4-{4-[3-(2-Methoxy-phenyl)-[1,2,4]oxadiazol-5-ylmethoxy]-phenyl}-3-(4-methylsulfanyl-benzyloxy)-piperidin-1-carbonsäure tert-butylester mittels Zinkbromid in Methylenchlorid das (3RS,4RS)-4-[4-[3-(2-Methoxy-phenyl)-[1,2,4]oxadiazol-5-ylmethoxy]-phenyl]-3-(4-methylsulfanyl-benzyloxy)-piperidin als farbloses Öl; MS : 518 $(M+H)^+$;

2) - aus dem 3-(4-Methansulfonyl-benzyloxy)-4-{4-[3-(2-methoxyphenyl)-[1,2,4]oxadiazol-5-ylmethoxy]-phenyl}-piperidin-1-carbonsäure tert-butylester mittels Zinkbromid in Methylenchlorid das (3RS,4RS)-4-[4-[3-(2-Methoxy-phenyl)-[1,2,4]oxadiazol-5-ylmethoxy]-phenyl]-3-(4-methylsulfonyl-benzyloxy)-piperidin als beiger Festkörper; MS : 550 $(M+H)^+$;

3) - aus dem Gemisch des (3RS,4RS)-3-[4-[(RS)- und -[(SR)-2,3-Dimethoxy-propoxy]-8-methoxy-naphthalin-2-ylmethoxy]-4-{4-[3-(2-methoxy-phenyl)-[1,2,4]oxadiazol-5-ylmethoxy]-phenyl}-piperidin-1-carbonsäure tert-butylesters mittels Zinkbromid in Methylenchlorid das Gemisch des (3RS,4RS)-3-[4-[(RS)- und -[(SR)-2,3-dimethoxy-propoxy]-8-methoxy-naphthalin-2-ylmethoxy]-4-[4-[3-(2-methoxy-phenyl)-[1,2,4]oxadiazol-5-ylmethoxy]-phenyl]-piperidins als beiger Festkörper; MS : 670 $(M+H)^+$;

4) - aus dem (3RS,4RS)-3-(1,4-Dimethoxy-naphthalin-2-ylmethoxy)-4-[4-[3-(2-methoxy-phenyl)- [1,2,4]oxadiazol-5-ylmethoxy]-phenyl]-piperidin-1-carbonsäure tert-butylester mittels Zinkbromid in Methylenchlorid das (3RS, 4RS)-3-(1,4-Dimethoxy-naphthalin-2-ylmethoxy)-4-[4-[3-(2-methoxy-phenyl)-[1,2,4]oxadiazol-5-ylmethoxy]-phenyl]-piperidin als beiger Festkörper; MS : 582 $(M+H)^+$;

5) - aus dem (3RS,4RS)-3-(1,4-Dimethoxy-naphthalin-2-ylmethoxy)-4-{4-[3-(3,4,5-trimethoxy-phenyl)-[1,2,4] oxadiazol-5-ylmethoxy]-phenyl}piperidin-1-carbonsäure tert-butylester mittels Zinkbromid in Methylenchlorid das (3RS,4RS)-3-(1,4-Dimethoxy-naphthalin-2-ylmethoxy)-4-[4-[3-(3,4,5-trimethoxy-phenyl)-[ 1,2, 4]oxadiazol-5-ylmethoxy]-phenyl]-piperidin als beiger Festkörper; MS : 642 $(M+H)^+$;

6) - aus dem (3RS,4RS)-3-(4-Methylsulfanyl-benzyloxy)-4-[4-(3-thiophen-2-yl-[1,2,4]oxadiazol-5-ylmethoxy)-phenyl]-piperidin-1-carbonsäure tert-butylester mittels Zinkbromid in Methylenchlorid das (3RS,4RS)-3-(4-Methylsulfanyl-benzyloxy)-4-[4-(3-thiophen-2-yl-[1,2,4]oxadiazol-5-ylmethoxy)-phenyl]-piperidin als hellgelbes, viskoses Öl; MS : 494 $(M+H)^+$;

7) - aus dem (3RS,4RS)-3-(1,4-Dimethoxy-naphthalin-2-ylmethoxy)-4-[4-(3-thiophen-2-yl-[1,2,4) oxadiazol-5-ylmethoxy)-phenyl]-piperidin-1-carbonsäure tert-butylester mittels Zinkbromid in Methylenchlorid das (3RS,4RS)-3-(1,4-Dimethoxy-naphthalin-2-ylmethoxy)-4-[4-(3-thiophen-2-yl-[1,2,4]oxadiazol-5-ylmethoxy)-phenyl]-piperidin als beiger Festkörper; MS : 558 $(M+H)^+$;

8) - aus dem 3-[1-(2-Methoxy-äthoxymethoxy)-naphthalin-2-ylmethoxy]-4-[4-(3-thiophen-2-yl-[1,2,4] oxadiazol-5-ylmethoxy)-phenyl]-piperidin-1-carbonsäure tert-butylester mittels Zinkbromid in Methylenchlorid unter gleichzeitiger Abspaltung der MEM-Gruppe das (3R5,4RS)-2-[4-[4-(3-Thiophen-2-yl-[1,2,4]oxadiazol-5-ylmethoxy)-phenyl]-piperidin-3-yloxymethyl]-naphthalin-1-ol als brauner Festkörper; MS : 514 $(M+H)^+$;

9) - aus dem (3RS,4RS)-3-[1-(2-Methoxy-äthoxy)-naphthalin-2-ylmethoxy]-4-[4-(3-thiophen-2-yl-[1,2,4]oxadiazol-5-ylmethoxy)-phenyl]-piperidin-1-carbonsäure tert-butylester mittels Zinkbromid in Methylenchlorid das (3RS, 4RS)-3-[1-(2-Methoxy-äthoxy)-naphthalin-2-ylmethoxy]-4-[4-(3-thiophen-2-yl-[1,2,4]oxadiazol-5-ylmethoxy)-phenyl]-piperidin Hydrobromid als farbloser Festkörper; MS : 572 $(M+H)^+$;

10) - aus dem (3RS,4RS)-3-(Naphthalin-2-ylmethoxy)-4-[4-(3-thiophen-2-yl-[1,2,4]oxadiazol-5-ylmethoxy)-phenyl]-piperidin 1-carbonsäure tert-butylester mittels Trifluoressigsäure in Methylenchlorid das (3RS,4RS)-3-(Naphthalin-2-ylmethoxy)-4-[4-(3-thiophen-2-yl-[1,2,4]oxadiazol-5-ylmethoxy)-phenyl]-piperidin Trifluoracetat als farbloser Festkörper; MS : 498 $(M+H)^+$;

11) - aus dem (3RS,4RS)-4-[4-(3-Furan-2-yl-[1,2,4]oxadiazol-5-ylmethoxy)-phenyl]-3-(naphthalin-2-ylmethoxy)-piperidin-1-carbonsäure tert-butylester mittels Trifluoressigsäure in Methylenchlorid das (3RS,4RS)-4-[4-(3-Furan-2-yl-[1,2,4]oxadiazol-5-ylmethoxy)-phenyl]-3-(naphthalin-2-ylmethoxy)-piperidin Trifluoracetat als farbloser Festkörper; MS : 482 $(M+H)^+$;

12) - aus dem (3RS,4RS)-4-[4-[3-(2-Chlor-phenyl)-[1,2,4]oxadiazol-5-ylmethoxy]-phenyl]-3-(naphthalin-2-ylmethoxy)-piperidin-1-carbonsäure tert-butylester mittels Trifluoressigsäure in Methylenchlorid das (3RS,4RS)-4-[4-[3-(2-Chlor-phenyl)-[1,2,4]oxadiazol-5-ylmethoxy]-phenyl]-3-(naphthalin-2-ylmethoxy)-piperidin Trifluoracetat als farbloser Festkörper; MS : 526 $(M+H)^+$;

13) - aus dem (3RS,4RS)-3-(1,4-Dimethoxy-naphthalin-2-ylmethoxy)-4-[4-(2-phenyl-oxazol-5-ylmethoxy)-phenyl]-piperidin-1-carbonsäure tert-butylester mittels Zinkbromid in Methylenchlorid das (3RS,4RS)-3-(1,4-Dimethoxy-naphthalin-2-ylmethoxy)-4-[4-(2-phenyl-oxazol-5-ylmethoxy)-phenyl]-piperidin als farbloser Festkörper; MS : 551 $(M+H)^+$;

14) - aus dem (3RS,4RS)-3-(1,4-Dimethoxy-naphthalin-2-ylmethoxy)-4-[4-(2-phenyl-thiazol-5-ylmethoxy)-phenyl]-piperidin-1-carbonsäure tert-butylester mittels Zinkbromid in Methylenchlorid das {3RS,4RS}-3-(1,4-Dimethoxy-naphthalin-2-ylmethoxy)-4-[4-(2-phenyl-thiazol-5-ylmethoxy)-phenyl]-piperidin als beiger Festkörper; MS : 567 $(M+H)^+$;

15) - aus dem (3RS,4RS)-3-[1-(2-Methoxy-äthoxy)-naphthalin-2-ylmethoxy]-4-[4-(3-phenyl-isoxazol-5-ylmethoxy)-phenyl]-piperidin-1-carbonsäure tert-butylester mittels Zinkbromid in Methylenchlorid das (3RS,4RS)-3-[1-(2-Methoxy-äthoxy)-naphthalin-2-ylmethoxy]-4-[4-(3-phenyl-isoxazol-5-ylmethoxy)-phenyl-piperidin als farbloser Festkörper; MS : 565 $(M+H)^+$;

16) - aus dem (3RS,4RS)-3-(1,4-Dimethoxy-naphthalin-2-ylmethoxy)-4-[4-(3-phenyl-isoxazol-5-ylmethoxy)-phenyl]-piperidin-1-carbonsäure tert-butylester mittels Zinkbromid in Methylenchlorid das (3RS,4RS)-3-(1,4-Dimethoxy-naphthalin-2-ylmethoxy)-4-[4-(3-phenyl-isoxazol-5-ylmethoxy)-phenyl]-piperidin als farbloser Festkörper; MS : 551 $(M+H)^+$;

17) - aus dem (3RS,4RS)-3-(1-Methoxy-naphthalin-2-ylmethoxy)-4-[4-(3-phenyl-isoxazol-5-ylmethoxy)-phenyl]-piperidin-1-carbonsäure tert-butylester mittels Trifluoressigsäure in Methylenchlorid das (3RS,4RS)-3-(1-Methoxy-naphthalin-2-ylmethoxy)-4-[4-(3-phenyl-isoxazol-5-ylmethoxy)-phenyl]-piperidin Trifluoracetat als farbloser Festkörper; MS : 521 $(M+H)^+$.

Die als Ausgangssubstanzen eingesetzten BOC-Derivate wurden wie folgt hergestellt:

(a) In analoger Weise wie in Beispiel 1 (g) beschrieben, wurde durch Alkylierung des (3RS,4RS)-4-(4-Allyloxy-phenyl)-3-hydroxy-piperidin-1-carbonsäure tert-butylesters [Beispiel 86 (b)] mit 4-Methylthio-benzylchlorid [J.Org. Chem. (1988), 53(3), 561-569] der (3RS,4RS)-4-(4-Allyloxy-phenyl)-3-(4-methylsulfanyl-benzyloxy)-piperidin-1-carbonsäure tert-butylester erhalten, aus dem durch Abspaltung der Allyl-Gruppe mittels Bis-(triphenyl-phosphin)-palladium(II)-diacetat, analog Beispiel 152 (e), der (3RS,4RS)-4-(4-Hydroxy-phenyl)-3-(4-methylsulfanyl-benzyloxy)-piperidin-1-carbonsäure tert-butylester erhalten wurde. Die darauffolgende Alkylierung mit 5-Brommethyl-3-(2-methoxy-phenyl)-[1,2,4]oxadiazol, analog Beispiel 44 (e), ergab den (3RS,4RS)-4-{4-[3-(2-Methoxy-phenyl)-[1,2,4]oxadiazol-5-ylmethoxy]-phenyl}-3-(4-methylsulfanyl-benzyloxy)-piperidin-1-carbonsäure tert-butylester als hellgelbes, viskoses Öl; MS : 618 $(M+H)^+$.

(b) In analoger Weise wie in Beispiel 152 (c) beschrieben, wurde durch Oxidation des (3RS,4RS)-4-{4-[3-(2-Methoxy-phenyl)-[1,2,4Joxadiazol-5-ylmethoxy]-phenyl}-3-(4-methylsulfanyl-benzyloxy)-piperidin-1-carbonsäure tert-butylesters mit m-Chlorperbenzoesäure der (3RS,4RS)-3-(4-Methansulfonyl-benzyloxy)-4-{4-[3-(2-methoxy-phenyl)-[1,2,4]oxadiazol-5-ylmethoxy]-phenyl}-piperidin-1-carbonsäure tert-butylester als beiger Festkörper erhalten; MS : 650 $(M+H)^+$.

(c) In analoger Weise wie in Beispiel 1 (g) beschrieben, wurde durch Alkylierung des (3RS,4RS)-4-(4-Allyloxy-phenyl)-3-hydroxy-piperidin-1-carbonsäure tert-butylesters [Beispiel 86 (b)] mit (RS)-3-Chlormethyl-1-(2,3-dimethoxy-propoxy)-5-methoxy-naphthalin das Gemisch des (3RS,4RS)-4-(4-Allyloxy-phenyl)-3-[4-[(RS)- und [(SR)-2,3-dimethoxypropoxy] -8-methoxy-naphthalin-2-ylmethoxy]-piperidin-1-carbonsäure tert-butylesters erhalten, aus dem durch Abspaltung der Allyl-Gruppe mittels Bis-(triphenylphosphin)-palladium(II)-diacetat, analog Beispiel 152 (e), das Gemisch des (3RS,4RS)-3-[4-[(RS)- und [(SR)-2,3-Dimethoxypropoxy]-8-methoxy-naphthalin-2-ylmethoxy]-4-(4-hydroxy-phenyl)-piperidin-1-carbonsäure tert-butylesters erhalten wurde. Die darauffolgende Alkylierung mit 5-Brommethyl-3-(2-methoxy-phenyl)-[1,2,4]oxadiazol, analog Beispiel 44 (e), ergab das Gemisch des (3RS,4RS)-3-[4-[(RS)- und [(SR)-2,3-Dimethoxy-propoxy]-8-methoxynaphthalin-2-ylmethoxy)-4-{4-[3-(2-methoxy-phenyl)-[1,2,4]oxadiazol-5-ylmethoxy]-phenyl}-piperidin-1-carbonsäure tert-butylesters als beigen Festkörper; MS : 770 $(M+H)^+$.

Das als Alkylierungsmittel eingesetzte (RS)-3-Chlormethyl-1-(2,3-dimethoxy-propoxy)-5-methoxy-naphthalin wurde wie folgt hergestellt:

Der 4-Acetoxy-8-methoxy-naphthalin-2-carbonsäure äthylester [Chem.Pharm.Bull.19 (6), 1245-1256 (1971)] wurde mittels wäßrigem Kaliumcarbonat in Äthanol zum 4-Hydroxy-8-methoxy-naphthalin-2-carbonsäure äthylester verseift, der, in Analogie zu Beispiel 44 (e), in Gegenwart von Kaliumcarbonat mit Methansulfonsäure (RS)-2,3-dimethoxy-propylester, das nach literaturbekanntem Verfahren aus (RS)-2,3-Dimethoxy-propan-1-ol [J.Chem.Soc. (1931), 450] erhalten worden war, zum (RS)-4-(2,3-Dimethoxy-propoxy)-8-methoxynaphthalin-2-carbonsäure äthylester alkyliert wurde. Die darauffolgende Reduktion mittels Lithiumaluminiumdydrid, analog Beispiel 5 (b), ergab das (RS) -[4-(2,3-Dimethoxy-propoxy)-8-methoxynaphthalin-2-yl]-methanol, das in Analogie zu Beispiel 5 (c) in das (RS)-3-Chlormethyl-1-(2,3-dimethoxy-propoxy)-5-methoxy-naphthalin überführt wurde; MS : 324 $(M)^+$.

(d) Die Alkylierung des (3RS,4RS)-3-(1,4-Dimethoxy-naphthalin-2-ylmethoxy)-4-(4-hydroxy-phenyl)-piperidin-1-carbonsäure tert-butylesters [Beispiel 152 (e)] mit 5-Brommethyl-3-(2-methoxy-phenyl)-[1,2,4]oxadiazol, analog Beispiel 44 (e), ergab den (3RS,4RS)-3-(1,4-Dimethoxy-naphthalin-2-ylmethoxy)-4-[4-[3-(2-methoxy-phenyl)

-[1,2,4]oxadiazol-5-ylmethoxy]-phenyl]-piperidin-1-carbonsäure tert-butylester als farblosen Festkörper; MS : 682 $(M+H)^+$.

(e) Die Alkylierung des (3RS,4RS)-3-(1,4-Dimethoxy-naphthalin-2-ylmethoxy)-4-(4-hydroxy-phenyl)-piperidin-1-carbonsäure tert-butylesters [Beispiel 152 (e)] mit 5-Brommethyl-3-(3,4,5-trimethoxyphenyl)-[1,2,4]oxadiazol, analog Beispiel 44 (e), ergab den (3RS,4RS)-3-(1,4-Dimethoxy-naphthalin-2-ylmethoxy)-4-{4-[3-(3,4,5-trimethoxy-phenyl)- [1,2,4] oxadiazol-5-ylmethoxy]-phenyl]-piperidin-1-carbonsäure tert-butylester als farblosen Festkörper; MS : 742 $(M+H)^+$.

(f) Die Alkylierung des (3RS,4RS)-4-(4-Hydroxy-phenyl)-3-(naphthalin-2-ylmethoxy)-piperidin-1-carbonsäure tert-butylesters [Beispiel 61 (c)] mit 5-Brommethyl-3-(2-chlor-phenyl)-[1,2,4]oxadiazol, analog Beispiel 44 (e), ergab den (3RS,4RS)-4-[4-[3-(2-Chlor-phenyl)-[1,2,4]oxadiazol-5-ylmethoxy]-phenyl]-3-(naphthalin-2-ylmethoxy)-piperidin-1-carbonsäure tert-butylester als gelben Festkörper; MS : 626 $(M+H)^+$.

(g) Die Alkylierung des (3RS,4RS)-4-(4-Hydroxy-phenyl)-3-(4-methylsulfanyl-benzyloxy)-piperidin- 1-carbonsäure tert-butylesters mit 5-Brommethyl-3-thiophen-2-yl-[1,2,4]oxadiazol, analog Beispiel 44 (e), ergab den (3RS,4RS)-3-(4-Methylsulfanyl-benzyloxy)-4-[4-(3-thiophen-2-yl-[1,2,4]oxadiazol-5-ylmethoxy)-phenyl]-piperidin-1-carbonsäure tert-butylester als farblosen Festkörper; MS : 594 $(M+H)^+$.

(h) Die Alkylierung des (3RS,4RS)-3-(1,4-Dimethoxy-naphthalin-2-ylmethoxy)-4-(4-hydroxy-phenyl)-piperidin-1-carbonsäure tert-butylesters [Beispiel 152 (e)] mit 5-Brommethyl-3-thiophen-2-yl-[1,2,4]oxadiazol, analog Beispiel 44 (e), ergab den (3RS,4RS)-3-(1,4-Dimethoxy-naphthalin-2-ylmethoxy)-4-[4-(3-thiophen-2-yl-[1,2,4]oxadiazol-5-ylmethoxy)-phenyl]-piperidin-1-carbonsäure tert-butylester als farbloses, viskoses Öl; MS : 658 $(M+H)^+$.

(i) In analoger Weise wie in Beispiel 1 (g) beschrieben, wurde durch Alkylierung des (3RS,4RS)-4-(4-Allyloxy-phenyl)-3-hydroxy-piperidin-1-carbonsäure tert-butylesters [Beispiel 86 (b)] mit 2-Chlormethyl-l-(2-methoxy-äthoxymethoxy)-naphthalin der (3RS,4RS)-4-(4-Allyloxyphenyl)-3- [1-(2-methoxy-äthoxymethoxy)-naphthalin-2-ylmethoxy)-piperidin-1-carbonsäure tert-butylester erhalten, aus dem durch Abspaltung der Allyl-Gruppe mittels Bis-(triphenylphosphin)-palladium(II)-diacetat, analog Beispiel 152 (e), der (3RS,4RS)-4-(4-Hydroxy-phenyl)-3-[1-(2-methoxy-äthoxymethoxy)-naphthalin-2-ylmethoxyl-piperidin-1-carbonsäure tert-butylester erhalten wurde. Die darauffolgende Alkylierung mit 5-Brommethyl-3-thiophen-2-yl-[1,2,4]oxadiazol, analog Beispiel 44 (e), ergab den (3RS,4RS)-3-[1-(2-Methoxy-äthoxymethoxy)-naphthalin-2-ylmethoxy]-4-[4-(3-thiophen-2-yl-[1,2,4]oxadiazol-5-ylmethoxy)-phenyl]-piperidin-1-carbonsäure tert-butylester als farbloses, viskoses Öl; MS : 702 $(M+H)^+$.

Das als Alkylierungsmittel eingesetzte 2-Chlormethyl-1-(2-methoxy-äthoxymethoxy)-naphthalin wurde wie folgt hergestellt:

($\alpha$) Eine Lösung von 2.3 g (11.4 mMol) 1-Hydroxy-naphthalin-2-carbonsäure methylester in 15 ml trockenem Tetrahydrofuran wurde mit 0.51 g (17 mMol) Natriumhydrid (80 %ig) versetzt und anschließend unter Eiskühlung 2.13 g 2-Methoxy-äthoxymethylchlorid zugetropft. Nach 3 Stunden bei Raumtemperatur wurde die Lösung mit wäßriger Natriumhydrogencarbonat-Lösung ausgeschüttelt, die organische Phase abgetrennt, über Natriumsulfat getrocknet und unter vermindertem Druck eingedampft. Zur Reinigung des Rohprodukts wurde an Kieselgel unter Verwendung eines 9:1-Gemisches von Methylenchlorid und Äther als Eluierungsmittel chromastographiert. Es wurden neben 1.55 g Ausgangsmaterial 1.2 g 1-(2-Methoxy-äthoxymethoxy)-naphthalin-2-carbonsäure methyl ester erhalten; MS : 290 $(M)^+$.

($\beta$) In analoger Weise wie in Beispiel 5 (b)-(c) beschrieben, wurde aus dem 1-(2-Methoxy-äthoxymethoxy)-naphthalin-2-carbonsäure methylester durch Reduktion mittels Lithiumaluminiumhydrid das [1-(2-Methoxy-äthoxymethoxy)-naphthalin-2-yl]-methanol erhalten, das dann in das 2-Chlormethyl-1-(2-methoxy-äthoxymethoxy)-naphthalin überführt wurde; MS : 280 $(M)^+$.

(j) Die Alkylierung des (3RS,4RS)-4-(4-Hydroxy-phenyl)-3-[1-(2-methoxy-äthoxy)-naphthalin-2-ylmethoxy]-piperidin-1-carbonsäure tert-butylesters [Beispiel 122 (h)] mit 5-Brommethyl-3-thiophen-2-yl-[1,2,4]oxadiazol, analog Beispiel 44 (e), ergab den (3RS,4RS)-3-[1-(2-Methoxy-äthoxy)-naphthalin-2-ylmethoxy]-4-[4-(3-thiophen-2-yl-(1,2,4)oxadiazol-5-ylmethoxy)-phenyl]-piperidin-1-carbonsäure tert-butyl als farbloses, viskoses Öl; MS : 672 $(M+H)^+$.

(k) Die Alkylierung des (3RS,4RS)-4-(4-Hydroxy-phenyl)-3-(naphthalin-2-ylmethoxy)-piperidin-1-carbonsäure tert-butylesters [Beispiel 61 (c)] mit 5-Brommethyl-3-thiophen-2-yl-[1,2,4]oxadiazol, analog Beispiel 44 (e), ergab den (3RS,4RS)-3-(Naphthalin-2-ylmethoxy)-4-[4-(3-thiophen-2-yl-[1,2,4]oxadiazol-5-ylmethoxy)-phenyl]-piperidin-1-carbonsäure tert-butylester als farbloses, viskoses Öl; MS : 597 $(M+H)^+$.

(l) Die Alkylierung des (3RS,4RS)-4-(4-Hydroxy-phenyl)-3-(naphthalin-2-ylmethoxy)-piperidin-1-carbonsäure tert-butylesters [Beispiel 61 (c)] mit 5-Brommethyl-3-furan-2-yl-[1,2,4]oxadiazol, analog Beispiel 44 (e), ergab den (3RS,4RS)-4-[4-(3-Furan-2-yl-[1,2,4]oxadiazol-5-ylmethoxy)-phenyl]-3-(naphthalin-2-ylmethoxy)-piperidin-1-carbonsäure tert-butylester als farblosen Festkörper; MS : 582 $(M+H)^+$.

(m) Die Alkylierung des (3RS,4RS)-3-(1,4-Dimethoxy-naphthalin-2-ylmethoxy)-4-(4-hydroxy-phenyl)-piperidin-1-carbonsäure tert-butylesters [Beispiel 152 (e)] mit 4-Chlormethyl-2-phenyl-oxazol [Arch.Pharmazie (1971), 425], analog Beispiel 44 (e), ergab den (3RS ,4RS )-3-( 1 ,4-Dimethoxy-naphthalin-2-ylmethoxy)-4-[4-(2-phenyloxazol-5-ylmethoxy)-phenyl]-piperidin-1-carbonsäure tert-butylester als farblosen Festkörper; MS : 651 $(M+H)^+$.

(n) Die Alkylierung des (3RS,4RS)-3-(1,4-Dimethoxy-naphthalin-2-ylmethoxy)-4-(4-hydroxy-phenyl)-piperidin-1-carbonsäure tert-butylesters [Beispiel 152 (e)] mit 4-Chlormethyl-2-phenyl-thiazol [Chem.Ber. (1961), 2887], analog Beispiel 44 (e), ergab den (3RS,4RS)-3-(1,4-Dimethoxy-naphthalin-2-ylmethoxy)-4-[4-(2-phenyl-thiazol-5-ylmethoxy)-phenyl]-piperidin-1-carbonsäure tert-butylester als hellgelben Festkörper; MS : 667 $(M+H)^+$.

(o) In analoger Weise wie in Beispiel 1 (g) beschrieben, wurde durch Alkylierung des (3RS,4RS)-4-(4-Allyloxy-phenyl)-3-hydroxy-piperidin-1-carbonsäure tert-butylesters [Beispiel 86 (b)] mit 2-Chlormethyl-1-(2-methoxy-äthoxy)-naphthalin [Beispiel 122 (h)] der (3RS,4RS)-4-(4-Allyloxy-phenyl)-3- [1-(2-methoxy-äthoxy)-naphthalin-2-ylmethoxy]-piperidin-1-carbonsäure tert-butylester erhalten, aus dem durch Abspaltung der Allyl-Gruppe mittels Bis-(triphenylphosphin)-palladium(II)-diacetat, analog Beispiel 152 (e), der (3RS,4RS)-4-(4-Hydroxy-phenyl)-3-[1-(2-methoxy-äthoxy)-naphthalin-2-ylmethoxy]-piperidin-1-carbonsäure tert-butylester erhalten wurde. Die darauffolgende Alkylierung mit Methansulfonsäure 3-phenyl-isoxazol-5-ylmethylester, analog Beispiel 44 (e), ergab den (3RS,4RS)-3-[1-(2-Methoxy-äthoxy)-naphthalin-2-ylmethoxy]-4-[4-(3-phenyl-isoxazol-5-ylmethoxy)-phenyl]-piperidin-1-carbonsäure tert-butylester als farblosen Festkörper; MS : 665 $(M+H)^+$.

(p) In analoger Weise wie in Beispiel 44 (e) beschrieben, wurde durch Alkylierung des (3RS,4RS)-3-Hydroxy-4-(4-hydroxy-phenyl)-piperidin-1-carbonsäure tert-butylesters [Beispiel 46 (b)] mit Methansulfonsäure 3-phenyl-isoxazol-5-ylmethylester, der (3RS,4RS)-3-Hydroxy-4-[4-(3-phenyl-isoxazol-5-ylmethoxy)-phenyl]-piperidin-1-carbonsäure tert-butylester erhalten, dessen weitere Alkylierung mit 2-Chlormethyl-1,4-dimethoxy-naphthalin [J.Org.Chem. (1983), 48(19),3265-3268)], analog Beispiel 1 (g), den (3RS,4RS)-3-(1,4-Dimethoxy-naphthalin-2-ylmethoxy)-4-[4-(3-phenyl-isoxazol-5-ylmethoxy)-phenyl]-piperidin-1-carbonsäure tert-butylester als farblosen Festkörper lieferte; MS : 651 $(M+H)^+$.

(q) Die Alkylierung des (3RS,4RS)-3-Hydroxy-4-[4-(3-phenyl-isoxazol-5-ylmethoxy)-phenyl]-piperidin-1-carbonsäure tert-butylesters mit 1-Methoxy-2-brommethyl-naphthalin [Beispiel 7 (f)], analog Beispiel 1 (g), lieferte den (3RS,4RS)-3-(1-Methoxy-naphthalin-2-ylmethoxy)-4-[4-(3-phenyl-isoxazol-5-ylmethoxy)-phenyl]-piperidin-1-carbonsäure tert-butylester als farblosen Festkörper; MS : 621 $(M+H)^+$.

Die Herstellung der als Alkylierungsmittel eingesetzten substituierten 5-Brommethyl-[1,2,4]oxadiazole:

- 5-Brommethyl-3-(2-methoxy-phenyl)-[1,2,4]oxadiazol,
- 5-Brommethyl-3-(3,4,5-trimethoxy-phenyl)-[1,2,4]oxadiazol,
- 5-Brommethyl-3-(2-chlor-phenyl)-[1,2,4]oxadiazol,
- 5-Brommethyl-3-thiophen-2-yl-[1,2,4]oxadiazol und
- 5-Brommethyl-3-furan-2-yl-[1,2,4]oxadiazol

erfolgte analog den im J.Med.Chem. 1986, 26, 2174-2183 beschriebenen Verfahren.

Der als Alkylierungsreagenz eingesetzte Methansulfonsäure 3-phenylisoxazol-5-ylmethylester wurde wie folgt synthetisiert:

(α) Zu einer Lösung von 1.21 g Benzaldehydoxim in 10 ml Methylenchlorid wurden bei -30 °C 1.47 g (11 mMol) N-Chlorsuccinimid gegeben. Nach 2 Stunden wurde eine Lösung von 1.0 g Triäthylamin und 1.4 g (RS)-Tetrahydro-2-(2-propynyloxy)-2H-pyran in 5 ml Methylenchlorid zugetropft. Das Reaktionsgemisch wurde 5 Stunden lang bei Raumtemperatur gerührt, danach wurde das Lösungsmittel abdestilliert und das Rohprodukt zur Reinigung an Kieselgel unter Verwendung von Methylenchlorid als Eluierungsmittel chromatographiert. Man erhielt 1.8 g (RS)-3-Phenyl-5-(tetrahydropyran-2-yloxymethyl)-isoxazol als farblose Flüssigkeit; MS : 259 $(M)^+$.

(β) Die anschließende Abspaltung der THP-Gruppe erfolgte analog Beispiel 53 (c). Das so erhaltene (3-Phenyl-isoxazol-5-yl)-methanol wurde nach literaturbekanntem Verfahren in den Methansulfonsäure 3-Phenyl-isoxazol-5-ylmethylester umgewandelt und dabei als farbloser Festkörper erhalten; MS : 253 $(M)^+$.

Beispiel 125

(a) Eine Suspension von 13.32 g (0.1 Mol) (E)-3-(4-Pyridyl)-2-propenal [Tetrahedron Letters 26, 6447 (1985)] und 19.92 g (0.1 Mol) 2-(Phenylsulfonyl)-acetamid [Synthesis 1987, 56] in 300 ml Äthanol wurde unter Rühren bei Raumtemperatur während 15 Minuten tropfenweise mit 20 ml Triton B Lösung (40% in Methanol) versetzt und anschliessend während 16 Stunden bei Raumtemperatur und während 90 Minuten unter Rückfluss gerührt. Nach dem Abkühlen wurde mit 100 ml Eisessig versetzt und anschliessend 2.5 Stunden unter Rückfluss erwärmt. Dann wurde in Wasserstrahlvakuum eingeengt, mit 200 ml Wasser gefolgt von 16.4 g (0.2 Mol) Natriumacetat versetz und erneut eingeengt. Der Rückstand wurde in Methylenchlorid aufgenommen, filtriert, das Filtrat eingeengt und der so erhaltene Rückstand aus Isopropanol umkristallisiert. Dabei resultierten 3.9 g (23% d. Th.) 1H-[4,4']Bipyridin-2-on in Form schwach gelber Kristalle; Smp: 263 - 265° C.

(b) Zu einer Suspension von 9.0 g (52.3 mMol) 1H-[4,4']Bipyridin-2-on in 150 ml N,N-Dimethylformamid wurden 15 ml Methyliodid zugegeben und das Reaktionsgemisch während 20 Stunden bei Raumtemperatur gerührt. Anschliessend wurden 300 ml Äther zugetropft und der dabei gebildete Niederschlag abfiltriert, mit Äther gewaschen und im Hochvakuum getrocknet. Dabei resultierten 15.8 g (96% d. Th.) 1-Methyl-4-(2-oxo-1,2-dihydro-pyridin-4-yl)-pridinium iodid in Form schwach gelber Kristalle; Smp: 264 - 266° C.

(c) 5.3 g (16.9 mMol) 1-Methyl-4-(2-oxo-1,2-dihydro-pyridin-4-yl)-pridinium iodid wurden in 100 ml Methanol aufgeschlämmt und unter Argon bei Raumtemperatur portionenweise mit 1.1 g (29 mMol) Natriumborhydrid versetzt. Hierauf wurde das Reaktionsgemisch während 5 Stunden unter Rückfluss erwärmt und anschliessend am Wasserstrahlvakuum eingeengt. Der dabei erhaltene Rückstand wurde zwischen gesättigter Kochsalzlösung und Methylenchlorid/Methanol (9:1) verteilt, die vereinigten Methylenchloridphasen über Magnesiumsulfat getrocknet und eingeengt. Umkristallisation des Rückstandes aus Methanol/Essigester lieferte schliesslich 2.7 g (84% d. Th.) 1'-Methyl-1',2',3',6'-tetrahydra-1H-[4,4']bipyridin-2-an in Form schwach gelber Kristalle; Smp: 250 - 252° C.

(d) 0.88 g (4.6 mMol) 1'-Methyl-1',2',3',6'-tetrahydro-1H-[4,4']bipyridin-2-on, 180 mg (2.4 mMol) Lithiumcarbonat und 2 g Molekularsieb (4Å) wurden in 20 ml 1,2-Dichloräthan suspendiert, mit 1.1 ml (10 mMol) Chlorameisensäure-1-chloräthylester versetzt und während 18 Stunden unter Rückfluss erwärmt. Hierauf wurde das Reaktionsgemisch im Wasserstrahlvakuum eingeengt und mit 2.2 g (10 mMol) Di-tert-butyl dicarbonat und 2 g (24 mMol) Natriumhydrogencarbonat in 60 ml Dioxan/Wasser (2:1) während 18 Stunden bei Raumtemperatur gerührt. Anschliessend wurde erneut im Wasserstrahlvakuum eingeengt und der so erhaltene Rückstand zwischen Methylenchlorid und 0.1 N Salzsäure verteilt. Die vereinigten Methylenchloridphasen wurden über Magnesiumsulfat getrocknet und aus Äther umkristallisiert. Dabei resultierten 0.24 g (19% d. Th.) 2'-Oxo-1',2',3,6-tetrahydro-2H-[4,4']bipyridin-1-carbonsäure tert-butylester in Form eines gelblichen Festkörpers; MS: 277 $(M+H)^+$.

(e) 0.50 g (1.8 mMol) 2'-Oxo-1',2',3,6-tetrahydro-2H-[4,4']bipyridin-1-carbonsäure tert-butylester, 0.50 g (2.5 mMol) (2-Brom-äthoxy)-benzol und 0.35 g (2.5 mMol) Kaliumcarbonat wurden in 6 ml Acetonitril während 20 Stunden bei 75° C erwärmt. Hierauf wurde das Reaktionsgemisch im Wasserstrahlvakuum eingeengt und zwischen Wasser und Methylenchlorid verteilt, die vereinigten Methylenchloridphasen über Magnesiumsulfat getrocknet, eingeengt und der so erhaltene Rückstand an Kieselgel mit Methylenchlorid/Essigester (1:1) chromatographiert. Dabei resultierten 0.41 g (58% d. Th.) 2'-Oxo-1'-(2-phenoxy-äthyl)-1',2',3,6-tetrahydro-2H-[4,4']bipyridin-1-carbonsäure tert-butylester in Form eines amorphen gelblichen Festkörpers; MS: 397 $(M+H)^+$.

(f) 0.20 g (0.5 mMol) 2'-Oxo-1'-(2-phenoxy-äthyl)-1',2',3,6-tetrahydro-2H-[4,4']bipyridin-1-carbonsäure tert-butylester wurden in 5 ml 1,2-Dimethoxyäthan gelöst und mit 1.5 ml 1 molarer Boran-Tetrahydrofuran-Lösung versetzt und während 48 Stunden bei Raumtemperatur gerührt. Hierauf wurden weitere 1.0 ml 1 molare Boran-Tetrahydrofuran-Lösung hinzugefügt und weitere 60 Stunden bei Raumtemperatur gerührt. Anschliessend wurden unter Eiskühlung 2.5 ml 50 %ige KOH-Lösung in Wasser gefolgt von 2.5 ml 30 %iger Wasserstoffperoxid-Lösung in Wasser zugesetzt und das Reaktionsgemisch während 2 Stunden unter Rückfluss erwärmt. Nun wurde die Reaktionslösung zwischen Wasser und Methylenchlorid verteilt, die vereinigten Methylenchloridphasen über Magnesiumsulfat getrocknet, eingeengt und der so erhaltene Rückstand an Kieselgel mit Methylenchlorid/Methanol (95:5) chromatographiert. Dabei resultierten 23 mg (11% d. Th.) (3RS,4RS)-3-Hydroxy-2'oxo-1'-(2-phenoxy-äthyl)-3,4,5,6,1',2'-hexahydro-2H-[4,4']-bipyridin-1-carbonsäure tert-butylester in Form eines amorphen gelblichen Festkörpers; MS: 414 $(M)^+$.

(g) Eine Lösung von 21 mg (0.051 mMol) (3RS,4RS)-3-Hydroxy-2'oxo-1'-(2-phenoxy-äthyl)-3,4,5,6,1',2'-hexahydro-2H-[4,4']-bipyridin-1-carbonsäure tert-butylester und 15 mg (0.068 mMol) 2-Brommethylnaphthalin in 0.5 ml Dimethylformamid wurde mit 4.0 mg (0.083 mMol) Natriumhydrid (50%ige Dispersion in Weißöl) versetzt und das Gemisch 0.5 Stunden lang bei Raumtemperatur gerührt. Anschließend wurde das Reaktionsgemisch zwischen Äther und Wasser verteilt, die vereinigten Ätherphasen über Magnesiumsulfat getrocknet und im Wasserstrahlvakuum eingeengt. Das Rohprodukt wurde an Kieselgel mit Methylenchlorid/Methanol (95:5) chromatographiert. Dabei resultierten 20 mg (71% d.Th.) (3'RS,4'RS)-3'-(Naphthalin-2-ylmethoxy)-1-(2-phenoxy-äthyl)-1',2',3',4',5', 6'hexahydro-1H-[4,4']bipyridin-2-on-1'-carbonsäure tert-butylester in Form eines amorphen gelblichen Festkörpers; MS: 555 $(M+H)^+$.

(h) 20 mg (0.036 mMol) (3'RS,4'RS)-3'-(Naphthalin-2-ylmethoxy)-1-(2-phenoxy-äthyl)-1',2',3',4',5',6'-hexahydro-1H-[4,4']bipyridin-2-on-1'carbonsäure tert-butylester wurden in 3 ml Methylenchlorid gelöst, mit 40 mg (0.18 mMol) wasserfreiem Zinkbromid versetzt und während 3 Stunden bei Raumtemperatur gerührt. Hierauf wurde das Reaktionsgemisch auf wässerige Natriumcarbonatlösung gegossen und diese mit Methylenchlorid extrahiert. Die vereinigten Methylenchloridphasen wurden über Magnesiumsulfat getrocknet, eingeengt und der dabei erhaltene Rückstand an Kieselgel mit einem 9:1- Gemisch von Methylenchlorid und Methanol als Eluierungsmittel chromatographiert. Daraus resultierten 5.8 mg (35% d. Th.) (3'RS,4'RS)-3'-(Naphthalin-2-ylmethoxy)-1-(2-phenoxy-äthyl)-1',2',3',4',5',6'-hexahydro-1H-[4,4']bipyridin-2-on in Form eines amorphen farblosen Festkörpers; MS: 455 $(M+H)^+$.

Beispiel 126

(a) 41 g (0.173 Mol) 2,5-Dibrompyridin und 20.1 g (0.173 Mol) 3-Phenyl-1-propyn wurden unter Argon und Feuchtigkeitsausschluss in 450 ml Triäthylamin gelöst, unter Eiskühlung mit 740 mg (3.88 mMol) Kupfer(I)iodid und 2.7 g (3.88 mMol) Bis-(triphenylphosphin)-palladium-dichlorid versetzt und 1 Stunde bei 0 - 5° C und 1 Stunde bei Raumtemperatur gerührt. Nun wurde die Reaktionslösung auf Eiswasser gegossen und mit Methylenchlorid extrahiert, die vereinigten Methylenchloridphasen über Magnesiumsulfat getrocknet, eingeengt und der so erhaltene Rückstand an Kieselgel einmal mit Methylenchlorid und einmal mit Hexan/Essigsäureäthylester (9:1) chromatographiert. Dabei resultierten 27 g (57% d. Th.) 5-Brom-2-(3-phenyl-prop-1-ynyl)-pyridin als farbloser Festkörper; MS: 271, 273 $(M)^+$.

(b) 17 g (0.062 Mol) 5-Brom-2-(3-phenyl-prop-1-ynyl)-pyridin wurden in 300 ml Äthanol gelöst, mit 150 mg Platinoxid versetzt und in einer Wasserstoff-atmosphäre während 1 Stunde hydriert. Anschliessend wurde das Reaktionsgemisch über einen 0.8 µ Cellulosefilter filtriert und im Wasserstrahlvakuum eingeengt. Der so erhaltene Rückstand wurde an Kieselgel mit Methylenchlorid chromatographiert. Dabei resultierten 5.2 g (30% d. Th.) 5-Brom-2-(3-phenyl-propyl)-pyridin in Form eines gelblichen Öls; MS: 171, 173 $(M\text{-}Vinylbenzol)^+$.

(c) Zu 21.5 ml (0.152 Mol) Diisopropylamin, gelöst in 145 ml Tetrahydrofuran wurden unter Argon und Feuchtigkeitsauschluss 100 ml 1.6 molare n-Butyllithium Lösung in Hexan (ca. 0.16 Mol) so zugetropft, dass die Temperatur nicht über -70° C anstieg. Hierauf wurden 29 g (0.145 Mol) 4-Piperidon-1-carbonsäure tert-butylester, gelöst in 145 ml Tetrahydrofuran während 45 Minuten zugetropft und dabei die Temperatur unter -70° C gehalten. Nach 10 Minuten Rühren bei eben dieser Temperatur wurde eine Lösung von 56 g (0.157 Mol) N-Phenyl-bis-(trifluoromethansulfonamid) in 145 ml Tetrahydrofuran innert 30 Minuten so zugetropft, dass die Temperatur nicht über -70° C anstieg. Hierauf liess man das Reaktionsgemisch auf 0° C aufwärmen und rührte bei dieser Temperatur noch 3 Stunden nach. Anschiessend wurde die Reaktionslösung bei 40° C im Wasserstrahlvakuum eingeengt und der so erhaltene Rückstand an basischem Alox mit Hexan/Essigsäureäthylester (9:1) chromatographiert. Dabei resultierten 41 g (85% d. Th.) 4-Trifluormethylsulfonyloxy-3,6-dihydro-2H-pyridin-1-carbonsäure tert-butylester in Form eines farblosen Öls; MS: 332 $(M+H)^+$.

(d) 1.05 g (3.8 mMol) 5-Brom-2-(3-phenyl-propyl)-pyridin, 1.12 ml (5.4 mMol) Hexamethyldistannan, 100 mg (0.086 mMol) Tetrakis-(triphenylphosphin)-palladium, 3 g Molekularsieb (4Ä) und einige Kristalle 2,6-Di-tert-butyl-p-kresol wurden in 15 ml Dioxan suspendiert und das Reaktionsgemisch unter Argon während 3 Stunden bei 100° C gerührt. Hierauf wurde das Reaktionsgemisch filtriert, im Wasserstrahlvakuum eingeengt und der Rückstand mit Hexan/Essigester (3:1) an Kieselgel chromatographiert. Dabei resultierten 0.93 g (68% d. Th.) 2-(3-Phenyl-propyl)-5-trimethylstannylpyridin in Form eines gelblichen Öls; MS: 362 $(M+H)^+$.

(e) 0.93 g (2.6 mMol) 2-(3-Phenyl-propyl)-5-trimethylstannyl-pyridin, 0.9 g (2.7 mMol) 4-Trifluormethylsulfonyloxy-3,6-dihydro-2H-pyridin-1-carbonsäure tert-butylester, 0.345 g (8.0 mMol) Lithiumchlorid, 100 mg (0.086 mMol) Tetrakis-(triphenylphosphin)-palladium, 3 g Molekularsieb (4Å) und einige Kristalle 2,6-Di-tert-butyl-p-kresol wurden in 40 ml 1,2-Dimethoxyäthan suspendiert und das Reaktionsgemisch unter Argon während 8 Stunden unter Rückfluss gerührt. Hierauf wurde filtriert, im Wasserstrahlvakuum eingeengt und der Rückstand mit Methylenchlorid/Äther (3:2) an Kieselgel chromatographiert. Dabei resultierten 0.411 g (42% d. Th.) 6-(3-Phenylpropyl)-3',6'-dihydro-2'H-[3,4']bipyridin-1'-carbonsäure tert-butylester in Form eines gelblichen Öls; MS: 379 $(M+H)^+$.

(f) 0.587 g (1.55 mMol) 6-(3-Phenyl-propyl)-3',6'-dihydro-2'H-[3,4']bipyridin-1'-carbonsäure tert-butylester wurden in 8 ml 1,2-Dimethoxyäthan gelöst, mit 6 ml 1 molarer Boran-Tetrahydrofuranlösung versetzt und in einem mit einem Teflon Stopfen verschlossenen Kolben während 4 Stunden bei 60° bis 65° C gerührt. Danach wurden weitere 3 ml 1 molare Boran-Tetrahydrofuranlösung zugesetzt und nach 24 Stunden weitere 2.2 ml 1 molare Boran-Tetrahydrofuranlösung und insgesamt 48 Stunden bei 60° bis 65° C gerührt. Anschliessend wurden unter Eiskühlung 7.0 ml 50 %ige KOH-Lösung in Wasser gefolgt von 6.0 ml 30 %iger Wasserstoffperoxid-Lösung in Wasser zugesetzt und das Reaktionsgemisch während 2 Stunden unter Rückfluss erwärmt. Nun wurde die Reaktionslösung zwischen Wasser und Methylenchlorid verteilt, die vereinigten Methylenchloridphasen über Magnesiumsulfat getrocknet, eingeengt und der so erhaltene Rückstand an Kieselgel mit Äther/Methanol (99:1) chromatographiert. Dabei resultierten 211 mg (34% d. Th.) (3'RS,4'RS)-3'Hydroxy-6-(3-phenyl-propyl)-3',4' ,5',6'-tetrahydro-2'H-[3,4'] bipyridin-1'-carbonsäure tert-butylester in Form eines amorphen, farblosen Festkörpers; MS: 397 $(M+H)^+$.

(g) In analoger Weise wie in Beispiel 125 (g) beschrieben wurde aus (3'RS,4'RS)-3'Hydroxy-6-(3-phenyl-propyl)-3',4',5',6'-tetrahydro-2'H-[3,4']bipyridin-1'-carbonsäure tert-butylester und 2-Brommethyl-naphthalin (3'RS,4'RS)-3'-(Naphthalin-2-ylmethoxy)-6-(3-phenylpropyl)-3',4',5',6'-tetrahydro-2'H-[3,4']bipyridin-1'-carbonsäure tert-butylester in Form eines amorphen, farblosen Festkörpers erhalten; MS: 537 $(M+H)^+$.

(h) In analoger Weise wie in Beispiel 125 (h) beschrieben, wurde aus (3'RS,4'RS)-3'-(Naphthalin-2-ylmethoxy)-6-(3-phenyl-propyl)-3',4',5',6'tetrahydro-2'H-[3,4']bipyridin-1'-carbonsäure tert-butylester durch Abspaltung der BOC-Gruppe mittels wasserfreiem Zinkbromid (3'RS,4'RS)-3'-(Naphthalin-2-ylmethoxy)-6-(3-phenyl-propyl)-1',2',3',4',5',6'-hexahydro-[3,4']bipyridin in Form eines amorphen, farblosen Schaumes erhalten; MS: 437 $(M+H)^+$.

Beispiel 127

(a) 1.24 g (5.7 mMol) 5-Brom-2-(3-hydroxy-propyl)-pyridin [J. Org. Chem. 53, 386 (1988)] wurden in 4 ml N,N-Dimethylformamid gelöst, mit 0.7 ml (5.9 mMol) Benzylbromid gefolgt von 285 mg (ca. 5.9 mMol) Natriumhydriddispersion (ca. 50% in Mineralöl) versetzt und während 90 Minuten bei Raumtemperatur unter Argon gerührt. Nun wurde die Reaktionslösung zwischen Wasser und Methylenchlorid verteilt, die vereinigten Methylenchloridphasen über Magnesiumsulfat getrocknet, filtriert und im Wasserstrahlvakuum eingeengt. Der so erhaltene Rückstand wurde an Kieselgel mit Äther/Methylenchlorid (5:95) chromatographiert. Dabei resultierten 1.57 g (90% d. Th.) 2-(3-Benzyloxy-propyl)-5-brom-pyridin in Form eines gelblichen Öls.

(b) 1.57 g (5.10 mMol) 2-(3-Benzyloxy-propyl)-5-brom-pyridin, 1.6 ml (7.5 mMol) Hexamethyldistannan, 150 mg (0.129 mMol) Tetrakis-(triphenylphosphin)-palladium, 3 g Molekularsieb (4Å) und einige Kristalle 2,6-Di-tert-butyl-p-kresol wurden in 18 ml Dioxan suspendiert und das Reaktionsgemisch unter Argon während 2.5 Stunden bei 100° C gerührt. Hierauf wurde das Reaktionsgemisch filtriert, im Wasserstrahlvakuum eingeengt und der Rückstand mit Hexan/Essigester (3:1) an Kieselgel chromatographiert. Dabei resultierten 1.43 g (72% d. Th.) 2-(3-Benzyloxy-propyl)-5-trimethylstannanyl-pyridin in Form eines gelblichen Öls; MS: 392 $(M+H)^+$.

(c) 1.43 g (3.66 mMol) 2-(3-Benzyloxy-propyl)-5-trimethylstannanylpyridin, 1.32 g (4.00 mMol) 4-Trifluormethylsulfonyloxy-3,6-dihydro-2Hpyridin-1-carbonsäure tert-butylester (Beispiel 126 (c)), 0.477 g (11.3 mMol) Lithiumchlorid, 150 mg (0.129 mMol) Tetrakis-(triphenylphosphin)-palladium, 3 g Molekularsieb (4Å) und einige Kristalle 2,6-Di-tert-butyl-p-kresol wurden in 40 ml 1,2-Dimethoxyäthan suspendiert und das Reaktionsgemisch unter Argon während 8 Stunden unter Rückfluss gerührt. Hierauf wurde filtriert, im Wasserstrahlvakuum eingeengt und der Rückstand mit Methylenchlorid/Äther (1:1) an Kieselgel chromatographiert. Dabei resultierten 0.903 g (60% d. Th.) 6-(3-Benzyloxy-propyl)-3',6'-dihydro-2'H-[3,4']bipyridinyl-1'-carbonsäure tert-butylester in Form eines gelblichen Öls; MS: 409 $(M+H)^+$.

(d) 0.115 g (0.28 mMol) 6-(3-Benzyloxy-propyl)-3',6'-dihydro-2'H-[3,4']bipyridinyl-1'-carbonsäure tert-butylester wurden in 1 ml 1,2-Dimethoxyäthan gelöst, mit 1 ml 1 molarer Boran-Tetrahydrofuranlösung versetzt und während 96 Stunden bei Raumtemperatur gerührt. Anschliessend wurden unter Eiskühlung 1.0 ml 50 %ige KOH-Lösung in Wasser gefolgt von 1.0 ml 30 %iger Wasserstoffperoxid-Lösung in Wasser zugesetzt und das Reaktionsgemisch während 2 Stunden unter Rückfluss erwärmt. Nun wurde die Reaktionslösung zwischen Wasser und Methylenchlorid verteilt, die vereinigten Methylenchloridphasen über Magnesiumsulfat getrocknet und eingeengt. Dabei resultierten 105 mg (88% d. Th.) (3'RS,4'RS)-6-(3-Benzyloxy-propyl)-3'-hydroxy-3',4',5',6'tetrahydro-2'H-[3,4']bipyridin-1'-carbonsäure tert-butylester in Form eines gelblichen Öls, welches direkt in die nächste Stufe eingesetzt wurde.

(e) In analoger Weise wie in Beispiel 125 (g) beschrieben wurde aus (3'RS,4'RS)-6-(3-Benzyloxy-propyl)-3'-hydroxy-3',4',5',6'-tetrahydro-2'H-[3,4']bipyridin-1'-carbonsäure tert-butylester und 2-Brommethyl-naphthalin (3'RS, 4'RS)-6-(3-Benzyloxy-propyl)-3'-(naphthalin-2-ylmethoxy)-3',4',5',6'-tetrahydro-2'H-[3,4']bipyridin-1'-carbonsäure tert-butylester in Form eines farblosen Öls erhalten; MS: 568 $(M+H)^+$.

(f) In analoger Weise wie in Beispiel 125 (h) beschrieben, wurde aus (3'RS,4'RS)-6-(3-Benzyloxy-propyl)-3'-(naphthalin-2-ylmethoxy)-3',4',5',6'-tetrahydro-2'H-[3,4']bipyridin-1'-carbonsäure tert-butylester durch Abspaltung der BOC-Gruppe mittels wasserfreiem Zinkbromid (3'RS,4'RS)-6-(3-Benzyloxy-propyl)-3'-(naphthalin-2-ylmethoxy)-1',2',3',4',5',6'-hexahydro-[3,4']bipyridin in Form eines farblosen Öls erhalten; MS: 467 $(M+H)^+$.

Beispiel 128

(a) Zu 104 ml (1.0 Mol) Benzylalkohol gelöst in 175 ml N,N-Dimethylformamid wurden bei maximal 30° C 19 g Natriumhydriddispersion (50% in Öl, 0.38 Mol) portionenweise eingetragen und während 2 Stunden bei Raumtemperatur nachgerührt. Hierauf wurden 46.4 g (0.183 Mol) 5-Bromo-2-(2-trimethylsilyl)äthynyl-pyridin [J. Org. Chem. 53, 386 (1988)] in 50 ml N,N-Dimethylformamid innert 10 Minuten zugetropft und während 2 Stunden bei Raumtemperatur gerührt. Anschliessend wurde das Reaktionsgemisch auf 1000 ml gesättigte Natriumhydrogencarbonat-Lösung gegossen und mit Äther extrahiert. Die vereinigten Ätherphasen wurden mit gesättigter Kochsalz-Lösung gewaschen, über Magnesiumsulfat getrocknet, filtriert und im Wasserstrahlvakuum eingeengt. Der so erhaltene Rückstand wurde an Kieselgel mit Methylenchlorid/Äther (99:1) chromatographiert. Dabei resultierten 19.5 g (37% d. Th.) (E)-2-(2-Benzyloxy-vinyl)-5-brom-pyridin in Form eines gelblichen Festkörpers.

(b) 17.5 g (0.0603 Mol) (E)-2-(2-Benzyloxy-vinyl)-5-brom-pyridin wurden in 650 ml Toluol gelöst, mit ca. 3 g Raney Nickel versetzt (feucht, mit Methanol und Toluol gewaschen) und während 72 Stunden in einer Wasserstoffatmosphäre bei Raumtemperatur gerührt. In diesem Zeitraum wurde noch dreimal dieselbe Menge Raney Nickel zugesetzt. Hierauf wurde über ein Dicalitpolster filtriert, im Wasserstrahlvakuum eingeengt und der Rückstand an Kieselgel mit Methylenchlorid/Äther (95:5) chromato-graphiert. Dabei resultierten 13.2 g (75.4% d. Th.) 2-(2-Benzyloxy-äthyl)-5-brom-pyridin in Form eines rötlichen Schaumes; MS: 292,294 $(M+H)^+$.

(c) In Analogie zu Beispiel 127 (b) - (d) wurde aus 2-(2-Benzyloxy-äthyl)-5-brom-pyridin via 2-(2-Benzyloxy-äthyl)-5-trimethylstannanyl-pyridin [gelbliches Öl, MS: 362 $(M-CH_3)^+$] sowie 4-Trifluormethylsulfonyloxy-3,6-dihydro-2H-pyridin-1-carbonsäure tert-butylester, und 6-(2-Benzyloxy-äthyl)-3',6'-dihydro-2'H-[3,4']bipyridin-1'-carbonsäure tert-butylester [farbloses Öl, MS: 395 $(M+H)^+$], (3'RS,4'RS)-6-(2-Benzyloxy-äthyl)-3'-hydroxy-3',4',5',6'-tetrahydro-2'H- [3,4']-bipyridin-1'carbonsäure tert-butylester in Form eines farblosen Festkörpers erhalten; MS: 413 $(M+H)^+$.

(d) In analoger Weise wie in Beispiel 125 (g) beschrieben wurde aus (3'RS,4'RS)-6-(2-Benzyloxy-äthyl)-3'-hydroxy-3',4',5',6'-tetrahydro-2'H-[3,4']-bipyridin-1'-carbonsäure tert-butylester und 2-Brommethyl-naphthalin (3'RS,4'RS)-6-(2-Benzyloxy-äthyl)-3'-(naphthalin-2-ylmethoxy)-3',4',5',6'-tetrahydro-2'H-[3,4']bipyridin-1'-carbonsäure tert-butylester in Form eines amorphen, farblosen Festkörpers erhalten; MS : 553 (M+H)+.

(e) In analoger Weise wie in Beispiel 125 (h) beschrieben wurde aus (3'RS,4'RS)-6-(2-Benzyloxy-äthyl)-3'-(naphthalin-2-ylmethoxy)-3',4',5',6'tetrahydro-2'H-[3,4']bipyridin-1'-carbonsäure tert-butylester durch Abspaltung der BOC-Gruppe mittels wasserfreiem Zinkbromid (3'RS,4'RS)-6-(2-Benzyloxy-äthyl)-3'-(naphthalin-2-ylmethoxy)-1',2',3',4',5',6'-hexahydro-[3,4']bipyridin in Form eines beigen Gummis erhalten; MS: 453 (M+H)+.

Beispiel 129

(a) In Analogie zu Beispiel 127 (b) - (d) wurde aus 5-Brom-2-methylsulfanyl-pyrimidin [J. Chem. Soc. 1953, 3129] via 2-Methylsulfanyl-5-trimethyl-stannanyl-pyrimidin (gelbliches Öl) sowie 4-Trifluormethylsulfonyloxy-3,6-dihydro-2H-pyridin-1-carbonsäure tert-butylester, und 4-(2-Methylsulfanyl-pyrimidin-5-yl)-3,6-dihydro-2Hpyridin-1-carbonsäure tert-butylester (gelblicher Festkörper, MS: 308 (M+H)+), (3RS,4RS)-3-Hydroxy-4-(2-methylsulfanyl-pyrimidin-5-yl)-piperidin-1-carbonsäure tert-butylester in Form eines gelblichen, amorphen Festkörpers erhalten; MS: 326 (M+H)+.

(b) In analoger Weise wie in Beispiel 125 (g) beschrieben wurde aus (3RS,4RS)-3-Hydroxy-4-(2-methylsulfanyl-pyrimidin-5-yl)-piperidin-1-carbonsäure tert-butylester und 2-Brommethylnaphthalin (3RS,4RS)-4-(2-Methylsulfanyl-pyrimidin-5-yl)-3-(naphthalin-2-ylmethoxy)-piperidin-1-carbonsäure tert-butylester in Form eines amorphen, gelblichen Festkörpers erhalten; MS: 466 (M+H)+.

(c) 0.138 g (0.296 mMol) (3RS,4RS)-4-(2-Methylsulfanyl-pyrimidin-5-yl)-3-(naphthalin-2-ylmethoxy)-piperidin-1-carbonsäure tert-butylester wurden in 5 ml Methylenchlorid gelöst, mit 0.113 g (ca. 0.46 mMol) m-Chlorperbenzoesäure (ca. 70%ig) versetzt, 3 Stunden bei Raumtemperatur gerührt, mit weiteren 0.050 g (ca. 0.20 mMol) m-Chlorperbenzoesäure versetzt und weitere 16 Stunden bei Raumtemperatur gerührt. Hierauf wurde das Reaktionsgemisch zwischen Methylenchlorid und gesättigter Sodalösung verteilt, die vereinigten Methylenchloridphasen über Magnesiumsulfat getrocknet, filtriert und im Wasserstrahlvakuum eingeengt. Der so erhaltene Rückstand wurde aus Äther kristallisiert. Dabei resultierten 0.102 g (69% d. Th.) (3RS,4RS)-4-(2-Methylsulfonyl-pyrimidin-5-yl)-3-(naphthalin-2-ylmethoxy)-piperidin-1-carbonsäure tert-butylester in Form eines farblosen Festkörpers; MS: 498 (M+H)+.

(d) 0.027 g (0.24 mMol) Kalium tert-butylat wurden in 1 ml Tetrahydrofuran vorgelegt und bei 0° C 0.038 g (0.22 mMol) 3-Benzyloxypropanol gelöst in 0.5 ml Tetrahydrofuran zugetropft. Nach 15 Minuten Rühren bei 0° C wurden 0.098 g (0.20 mMol) (3RS,4RS)-4-(2-Methylsulfonyl-pyrimidin-5-yl)-3-(naphthalin-2-ylmethoxy)-piperidin-1-carbonsäure tert-butylester gelöst in 1 ml Tetrahydrofuran bei ebendieser Temperatur zugetropft und das Reaktionsgemisch während 18 Stunden bei Raumtemperatur gerührt. Hierauf wurde dieses auf Eiswasser gegossen und mit Äther extrahiert. Die vereinigten Ätherphasen wurden mit gesättigter Kochsalz-Lösung gewaschen, büber Magnesiumsulfat getrocknet, filtriert und im Wasserstrahlvakuum eingeengt. Der so erhaltene Rückstand wurde an Kieselgel mit Methylenchlorid/Methanol (95:5) chromatographiert. Dabei resultierten 0.076 g (66% d. Th.) (3RS,4RS)-4-[2-(3-Benzyloxy-propoxy)-pyrimidin-5-yl]-3-(naphthalin-2-ylmethoxy)-piperidin-1-carbonsäure tert-butylester in Form eines farblosen Festkörpers; MS: 585 (M+H)+.

(e) In analoger Weise wie in Beispiel 125 (h) beschrieben, wurde aus (3RS,4RS)-4-[2-(3-Benzyloxy-propoxy)-pyrimidin-5-yl]-3-(naphthalin-2-ylmethoxy)-piperidin-1-carbonsäure tert-butylester durch Abspaltung der BOC-Gruppe mittels wasserfreiem Zinkbromid 2-(3-Benzyloxypropoxy)-5-[(3RS,4RS)-3-(naphthalin-2-ylmethoxy)-piperidin-4-yl]-pyrimidin in Form eines gelblichen Gummis erhalten; MS: 485 (M+H)+.

Beispiel 130

(a) 0.90 g (2.18 mMol) (3'RS,4'RS)-6-(2-Benzyloxy-äthyl)-3'-hydroxy-3',4',5',6'-tetrahydro-2'H-[3,4']-bipyridin-1'-carbonsäure tert-butylester [Beispiel 128 (c)], gelöst in 15 ml Tetrahydrofuran wurden mit 0.3 ml Essigsäure und 250 mg Palladium auf Kohle (10%) versetzt und das Reaktionsgemisch in einer Wasserstoffatmosphäre während 14 Tagen gerührt. Anschliessend wurde über einen 0.8 μ Cellulosefilter filtriert und im Wasserstrahlvakuum eingeengt. Der so erhaltene Rückstand wurde zwischen Methylenchlorid und gesättigter Sodalösung verteilt, die vereinigten Methylenchloridphasen über Magnesiumsulfat getrocknet, filtriert und im Wasserstrahlvakuum eingeengt. Das Rohprodukt wurde anschliessend an Kieselgel mit Methylenchlorid/Methanol (95:5) chromatographiert. Dabei resultierten 0.555 g (80% d. Th.) (3'RS,4'RS)-3'-Hydroxy-6-(2-hydroxy-äthyl)-3',4',5',6'tetrahydro-2'H-[3,4'] bipyridin-1'-carbonsäure tert-butylester in Form eines farblosen Gummis; MS: 323 $(M+H)^+$.

(b) 0.46 g (1.43 mMol) (3'RS,4'RS)-3'-Hydroxy-6-(2-hydroxy-äthyl)-3',4',5',6'-tetrahydro-2'H-[3,4']bipyridin-1'-carbonsäure tert-butylester, 0.18 g (1.5 mMol) 4-Dimethylamino-pyridin und 0.32 ml (2.2 mMol) Triäthylamin wurden in 5 ml Methylenchlorid vorgelegt und mit 0.55 g (1.7 mMol) Bromtriphenylmethan versetzt. Nach 16 Stunden Rühren bei Raumtemperatur wurden weitere und 0.32 ml (2.2 mMol) Triäthylamin und 0.50 g (1.5 mMol) Bromtriphenylmethan hinzugefügt und noch eine weitere Stunde bei Raumtemperatur gerührt. Darauf wurde das Reaktionsgemisch zwischen Methylenchlorid und gesättigter Sodalösung verteilt, die vereinigten Methylenchloridphasen über Magnesiumsulfat getrocknet, filtriert und im Wasserstrahlvakuum eingeengt. Der so erhaltene Rückstand wurde an Kieselgel mit Methylenchlorid/Methanol (95:5) chromatographiert. Dabei resultierten 0.67 g (83% d. Th.) (3'RS,4'RS)-3'-Hydroxy-6-(2-trityloxy-äthyl)-3',4',5',6'-tetrahydro-2'H-[3,4']bipyridin-1'-carbonsäure tert-butylester in Form eines farblosen Schaumes; MS: 566 $(M+H)^+$.

(c) In analoger Weise wie in Beispiel 125 (g) beschrieben wurde aus (3'RS,4'RS)-3'-Hydroxy-6-(2-trityloxy-äthyl)-3',4',5',6'-tetrahydro-2'H-[3,4']bipyridin-1'-carbonsäure tert-butylester und 2-Brommethyl-naphthalin (3'RS,4'RS)-3'-(Naphthalin-2-ylmethoxy)-6-(2-trityloxy-äthyl)-3',4',5',6'-tetrahydro-2'H-[3,4']bipyridin-1'-carbonsäure tert-butylester in Form eines amorphen, farblosen Festkörpers erhalten; MS: 706 $(M+H)^+$.

(d) 0.35 g (0.50 mMol) (3'RS,4'RS)-3'-(Naphthalin-2-ylmethoxy)-6-(2-trityloxy-äthyl)-3',4',5',6'-tetrahydro-2'H-[3,4'] bipyridin-1'-carbonsäure tert-butylester wurden in 8 ml Methylenchlorid gelöst und bei Raumtemperatur rasch mit einer Lösung von 240 mg Trifluoressigsäure und 440 mg Trifluoressigsäureanhydrid in 2 ml Methylenchlorid versetzt und das Reaktionsgemisch während 50 Sekunden gerührt. Hierauf wurde unter Eiskühlung 2.2 ml Triäthylamin zugesetzt, gefolgt von 3 ml Methanol und 10 Minuten ohne Kühlung gerührt. Darauf wurde das Reaktionsgemisch zwischen Methylenchlorid und gesättigter Sodalösung verteilt, die vereinigten Methylenchloridphasen über Magnesiumsulfat getrocknet, filtriert und im Wasserstrahlvakuum eingeengt. Der so erhaltene Rückstand wurde an Kieselgel mit Methylenchlorid/Methanol (95:5) chromatographiert. Dabei resultierten 0.189 g (82% d. Th.)(3'RS,4'RS)-6-(2-Hydroxy-äthyl)-3'-(naphthalin-2-ylmethoxy)-3',4',5',6'-tetrahydro-2'H-[3,4']bipyridin-1'-carbonsäure tert-butylester in Form eines amorphen, orangen Festkörpers; MS: 463 $(M+H)^+$.

(e) 0.060 g (0.129 mMol) 6-(2-Hydroxy-äthyl)-3'-(naphthalin-2-ylmethoxy)-3',4',5',6'-tetrahydro-2'H-[3,4']bipyridin-1'-carbonsäure tert-butylester und 0.022 g (0.129 mMol) 2-Chlor-benzothiazol wurden in 0.5 ml N,N-Dimethylformamid gelöst und mit 0.008 g (ca. 50%ig in Mineralöl, ca. 0.17 mMol) Natriumhydrid versetzt und das Reaktionsgemisch während 4.5 Stunden bei Raumtemperatur gerührt. Hierauf wurde mit Eiswasser versetzt und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Methylenchlorid/Äther (1:1) chromatographiert. Dabei resultierten 0.053 g (70% d. Th.) (3'RS,4'RS)-3'-(Naphthalin-2-ylmethoxy)-6-vinyl-3',4',5',6'-tetrahydro-2'H-[3,4']bipyridin-1'-carbonsäure tert-butylester in Form eines amorphen, farblosen Festkörpers; MS: 446 $(M+H)^+$.

(f) 0.041 g (0.091 mMol) (3'RS,4'RS)-3'-(Naphthalin-2-ylmethoxy)-6-vinyl-3',4',5',6'-tetrahydro-2'H-[3,4')bipyridin-1'-carbonsäure tert-butylester und 0.054 g (0.32 mMol) 2-Mercaptobenzothiazol wurden in 0.5 ml Acetonitril gelöst, mit 0.2 ml (0.12 mMol) 0.6 M Natriummethylat-Lösung in Methanol versetzt und während 3.5 Stunden bei 80° C gerührt. Hierauf wurde das Reaktionsgemisch eingeengt und der Rückstand an Kieselgel mit Essigsäureäthylester/Hexan (3:2) chromatographiert. Dabei resultierten 0.040 g (72% d. Th.) (3'RS,4'RS)-3'-(Naphthalin-2-ylmethoxy)-6-[2-(2-thioxo-benzothiazol-3-yl)-äthyl]-3',4',5',6'-tetrahydro-2'H-[3,4']bipyridin-1'-carbonsäure tert-butylester in Form eines amorphen, gelblichen Festkörpers; MS: 612 $(M+H)^+$.

(g) In analoger Weise wie in Beispiel 125 (h) beschrieben, wurde aus (3'RS,4'RS)-3'-(Naphthalin-2-ylmethoxy)-6-[2-(2-thioxo-benzothiazol-3-yl)-äthyl]-3',4',5',6'-tetrahydro-2'H-[3,4']bipyridin-1'-carbonsäure tert-butylester durch Abspaltung der BOC-Gruppe mittels wasserfreiem Zinkbromid 3-[2-[(3'RS,4'RS)-3'-(Naphthalin-2-ylmethoxy)-1',2',3',4',5',6'-hexahydro-[3,4']bipyridin-6-yl]-äthyl]-3H-benzothiazol-2-thion in Form eines amorphen, farblosen Festkörpers erhalten; MS: 512 $(M+H)^+$.

Beispiel 131

(a) 0.15 g (0.56 mMol) 5-Brommethyl-3-(2-chloro-phenyl)-[1,2,4]oxadiazol [Beispiel 124], 0.21 g Kaliumcarbonat und 0.15 g Natriumhydrogencarbonat wurden in einem Gemisch von 4.5 ml Tetrahydrofuran und 1.0 ml Wasser während 42 Stunden bei 65° C gerührt. Hierauf wurde das Reaktionsgemisch abgekühlt, mit Wasser verdünnt und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Methylenchlorid/Äther (9:1) chromatographiert. Dabei resultierten 0.041 g (35% d. Th.) [3-(2-Chlor-phenyl)-[1,2,4]oxadiazol-5-yl]-methanol in Form eines farblosen Festkörpers; MS: 210 $(M)^+$.

(b) 0.040 g (0.19 mMol) [3-(2-Chlor-phenyl)-[1,2,4]oxadiazol-5-yllmethanol und 0.094 g (0.19 mMol) (3RS,4RS)-4-(2-Methylsulfonylpyrimidin-5-yl)-3-(naphthalin-2-ylmethoxy)-piperidin-1-carbonsäure tert-butylester [Beispiel 129 (c)] wurden in 0.5 ml N,N-Dimethylformamid gelöst, unter Eiskühlung mit 0.0095 g (ca. 50%ig in Mineralöl, ca. 0.20 mMol) Natriumhydrid versetzt und das Reaktionsgemisch während 1 Stunde bei Raumtemperatur gerührt. Hierauf wurde mit Eiswasser versetzt und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Äther chromatographiert. Dabei resultierten 0.112 g (94% d. Th.) (3RS,4RS)-4-[2-[3-(2-Chlor-phenyl) -[1,2,4] oxadiazol-5-ylmethoxy]-pyrimidin-5-yl]-3-(naphthalin-2-ylmethoxy)-piperidin-1-carbonsäure tert-butylester in Form eines amorphen, rötlichen Gummis; MS: 628 $(M+H)^+$.

(c) In analoger Weise wie in Beispiel 125 (h) beschrieben, wurde aus (3RS,4RS)-4-[2-[3-(2-Chlor-phenyl)-[1,2,4] oxadiazol-5-ylmethoxy]-pyrimidin-5-yl]-3-(naphthalin-2-ylmethoxy)-piperidin-1-carbonsäure tert-butylester durch Abspaltung der BOC-Gruppe mittels wasserfreiem Zinkbromid 2-[3-(2-Chloro-phenyl)- [1,2,4]oxadiazol-5-ylmethoxy]-5-[(3RS,4RS)-3-(naphthalin-2-ylmethoxy)-piperidin-4-yl]-pyrimidin in Form eines amorphen, gelblichen Festkörpers erhalten; MS: 528 $(M+H)^+$.

Beispiel 132

(a) 2.3 g (10 mMol) 3-Benzyloxy-1-brom-propan und 0.76 g (10 mMol) Thioharnstoff wurden in 5.0 ml Äthanol während 3.5 Stunden unter Rückfluss erwärmt. Hierauf wurde auf Raumtemperatur gekühlt, mit 0.6 g (15 mMol) Natriumhydroxid in 6.0 ml Wasser versetzt und weitere 3 Stunden unter Argon gerührt. Hierauf wurde mit verdünnter Salzsäure angesäuert und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Hexan/Methylenchlorid (1:1) chromatographiert. Dabei resultierten 1.5 g (83% d. Th.) 3-Benzyloxy-propan-1-thiol als farblose Flüssigkeit.

(b) In analoger Weise wie in Beispiel 131 (b) beschrieben, wurde aus 3-Benzyloxy-propan-1-thiol und (3RS,4RS)-4-(2-Methylsulfonyl-pyrimidin-5-yl)-3-(naphthalin-2-ylmethoxy)-piperidin-1-carbonsäure tert-butylester [Beispiel 129 (c)] (3RS,4RS)-4-[2-(3-Benzyloxy-propylsulfanyl)-pyrimidin-5-yl]-3-(naphthalin-2-ylmethoxy)-piperidin-1-carbonsäure tert-butylester in Form eines amorphen, rosaroten Festkörpers erhalten; MS: 600 $(M+H)^+$.

(c) In analoger Weise wie in Beispiel 125 (h) beschrieben, wurde aus (3RS,4RS)-4-[2-(3-Benzyloxy-propylsulfanyl)-pyrimidin-5-yl]-3-(naphthalin-2-ylmethoxy)-piperidin-1-carbonsäure tert-butylester durch Abspaltung der BOC-Gruppe mittels wasserfreiem Zinkbromid 2-(3-Benzyloxy-propylsulfanyl)-5-[(3RS,4RS)-3-(naphthalin-2-ylmethoxy)-piperidin-4-yl]-pyrimidin in Form eines amorphen, farblosen Festkörpers erhalten; MS: 500 $(M+H)^+$.

Beispiel 133

(a) 0.50 g (2.2 mMol) 3-Benzyloxy-1-brom-propan wurden mit 2.5 ml 30 prozentiger Methylamin-Lösung in Äthanol versetzt und im geschlossenen Gefäss während 10 Stunden bei 60° C gerührt. Hierauf wurde mit Eiswasser versetzt und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Methylenchlorid/Methanol

(9:1) chromatographiert. Dabei resultierten 0.16 g (41% d. Th.) (3-Benzyloxy-propyl)-methyl-amin als farbloses Öl.

(b) 0.15 g (0.84 mMol) (3-Benzyloxy-propyl)-methyl-amin und 0.060 g (0.12 mMol) (3RS,4RS)-4-(2-Methylsulfonyl-pyrimidin-5-yl)-3-(naphthalin-2-ylmethoxy)-piperidin-1-carbonsäure tert-butylester [Beispiel 129 (c)] wurden in 1.5 ml Triäthylamin unter Argon während 18 Stunden bei 80° C gerührt. Hierauf wurde mit Eiswasser versetzt und mit Äther extrahiert. Die vereinigten Ätherphasen wurden über Magnesiumsulfat getrocknet, filtriert und im Wasserstrahlvakuum eingeengt. Der so erhaltene Rückstand wurde an Kieselgel mit Methylenchlorid/Essigsäureäthylester (1:1) chromatographiert. Dabei resultierten 0.070 g (97% d. Th.) (3RS,4RS)-4-[2-[(3-Benzyloxy-propyl)-methyl-amino]-pyrimidin-5-yl]-3-(naphthalin-2-ylmethoxy)-piperidin-1-carbonsäure tert-butylester in Form eines bräunlichen Gummis; MS: 598 $(M+H)^+$.

(c) In analoger Weise wie in Beispiel 125 (h) beschrieben, wurde aus (3RS,4RS)-4-[2-[(3-Benzyloxy-propyl)-methyl-amino]-pyrimidin-5-yl]-3-(naphthalin-2-ylmethoxy)-piperidin-1-carbonsäure tert-butylester durch Abspaltung der BOC-Gruppe mittels wasserfreiem Zinkbromid (3-Benzyloxy-propyl)-methyl-[5-[(3RS,4RS)-3-(naphthalin-2-ylmethoxy)-piperidin-4-yl]-pyrimidin-2-yl]-amin in Form eines amorphen, farblosen Festkörpers erhalten; MS: 498 $(M+H)^+$.

Beispiel 134

(a) 1.15 g (5.0 mMol) 3-Benzyloxy-1-brom-propan und 1.02 g (5.5 mMol) Kalium-phthalimid wurden in 10 ml N, N-Dimethylformamid während 2 Stunden bei 70 - 80° C gerührt. Hierauf wurde mit Eiswasser versetzt und der dabei gebildete Niederschlag abfiltriert, mit Wasser gewaschen und über Phosphorpentoxid im Wasserstrahlvakuum getrocknet. Dabei resultierten 1.4 g (95% d. Th.) 2-(3-Benzyloxy-propyl)-isoindol-1,3-dion als farbloser Festkörper.

(b) 1.4 g (4.7 mMol) 2-(3-Benzyloxy-propyl)-isoindol-1,3-dion und 0.9 ml Hydrazinmonohydrat wurden in 10 ml absolutem Äthanol während 2 Stunden unter Argon bei 100° C gerührt. Nach dem Abkühlen wurde mit Äther versetzt, filtriert und das Filtrat eingeengt. Dabei resultierten 0.75 g (96% d. Th.) 3-Benzyloxy-propylamin als leicht gelbliches Öl.

(c) In analoger Weise wie in Beispiel 133 (b) beschrieben, wurde aus 3-Benzyloxy-propylamin und (3RS,4RS)-4-(2-Methylsulfonyl-pyrimidin-5-yl)-3-(naphthalin-2-ylmethoxy)-piperidin-1-carbonsäure tert-butylester [Beispiel 129 (c)] (3RS,4RS)-4-[2-(3-Benzyloxy-propylamino)-pyrimidin-5-yl]-3-(naphthalin-2-ylmethoxy)-piperidin-1-carbonsäure tert-butylester in Form eines bräunlichen Gummis erhalten; MS: 583 $(M+H)^+$.

(d) In analoger Weise wie in Beispiel 125 (h) beschrieben, wurde aus (3RS,4RS)-4-[2-(3-Benzyloxy-propylamino)-pyrimidin-5-yl]-3-(naphthalin-2-ylmethoxy)-piperidin- 1-carbonsäure tert-butylester durch Abspaltung der BOC-Gruppe mittels wasserfreiem Zinkbromid (3-Benzyloxy-propyl)-{5-[(3RS,4RS)-3-(naphthalin-2-ylmethoxy)-piperidin-4-yl]-pyrimidin-2-yl}-amin in Form eines bräunlichen Gummis erhalten; MS : 483 $(M+H)^+$.

Beispiel 135

In analoger Weise wie in Beispiel 125 (h) beschrieben, wurden durch Abspaltung der BOC-Gruppe mittels wasserfreiem Zinkbromid die folgenden Verbindungen erhalten:

1) - Aus (3RS,4RS)-4-(2-Methylsulfanyl-pyrimidin-5-yl)-3-(naphthalin-2-ylmethoxy)-piperidin-1-carbonsäure tert-butylester [Beispiel 129 (b)] 2-Methylsulfanyl-5-[(3RS,4RS)-3-(naphthalin-2-ylmethoxy)-piperidin-4-yl]-pyrimidin in Form eines amorphen, gelblichen Festkörpers; MS: 366 $(M+H)^+$;

2) - aus (3'RS,4'RS)-6-(3-Benzyloxy-propyl)-3'-(naphthalin-2-ylmethoxy)-1-oxy-3',4',5',6'-tetrahydro-2'H-[3,4']bipyridin-1'carbonsäure tert-butylester (3'RS,4'RS)-6-(3-Benzyloxy-propyl)-3'-(naphthalin-2-ylmethoxy)-1',2',3',4',5',6'-hexahydro-[3,4']bipyridin-1-oxid in Form eines farblosen Öls; MS: 483 $(M+H)^+$;

3) - aus (3'RS,4'RS)-3'-(Naphthalin-2-ylmethoxy)-6-(3-phenyl-propyl)-1-oxy-3',4',5',6'-tetrahydro-2'H-[3,4']bipyridin-1'-carbonsäure tert-butylester (3'RS,4'RS)-3'-(Naphthalin-2-ylmethoxy)-6-(3-phenyl-propyl)-1',2',3',4',5',6'-hexahydro-[3,4']bipyridin- 1-oxid in Form eines amorphen, gelblichen Festkörpers; MS: 453 $(M+H)^+$;

4) - aus (3RS,4RS)-3-(Naphthalin-2-ylmethoxy)-4-[2-(4-phenylbutylamino)-pyrimidin-5-yl]-piperidin-1-carbonsäure tert-butylester [5-[(3RS,4RS)-3-(Naphthalin-2-ylmethoxy)-piperidin-4-yl]pyrimidin-2-yl]-(4-phenyl-butyl)-amin in Form eines amorphen, rötlichen Festkörpers; MS: 467 $(M+H)^+$;

5) - aus (3RS,4RS)-4-{2-[Methyl-(4-phenyl-butyl)-amino]-pyrimidin-5-yl}-3-(naphthalin-2-ylmethoxy)-piperidin-1-carbonsäure tert-butylester Methyl-(5-[(3RS,4RS)-3-(naphthalin-2-ylmethoxy)-piperidin-4-yl]-pyrimidin-2-yl}-(4-phenyl-butyl)-amin in Form eines amorphen, farblosen Festkörpers; MS: 481 $(M+H)^+$.

Die als Ausgangssubstanzen eingesetzten BOC-Derivate wurden wie folgt hergestellt:

(b) 0.074 g (0.13 mMol) (3'RS,4'RS)-6-(3-Benzyloxy-propyl)-3'-(naphthalin-2-ylmethoxy)-3',4',5',6'-tetrahydro-2'H-[3,4']bipyridin-1'carbonsäure tert-butylester [Beispiel 127 (e)] wurden in 1.5 ml Methylenchlorid gelöst und mit 0.046 g (ca. 0.19 mMol) m-Chlorperbenzoesäure (ca. 70%ig) versetzt und 30 Minuten bei Raumtemperatur gerührt. Hierauf wurde das Reaktionsgemisch zwischen Methylenchlorid und gesättigter Sodalösung verteilt, die vereinigten Methylenchloridphasen über Magnesiumsulfat getrocknet, filtriert und im Wasserstrahlvakuum eingeengt. Der so erhaltene Rückstand wurde an Kieselgel mit Methylenchlorid/Methanol (95:5) chromatographiert. Dabei resultierten 0.032 g (42% d. Th.) (3'RS,4'RS)-6-(3-Benzyloxy-propyl)-3'-(naphthalin-2-ylmethoxy)-1-oxy-3',4',5',6'tetrahydro-2'H-[3,4']bipyridin-1'-carbonsäure tert-butylester in Form eines amorphen, farblosen Festkörpers; MS: 584 $(M+H)^+$.

(c) In analoger Weise wie in Beispiel 135 (b) beschrieben, wurde aus (3'RS,4'RS)-3'-(Naphthalin-2-ylmethoxy)-6-(3-phenyl-propyl)-3',4',5',6'tetrahydro-2'H-[3,4']bipyridin-1'-carbonsäure tert-butylester [Beispiel 126 (g)] (3'RS, 4'RS)-3'-(Naphthalin-2-ylmethoxy)-6-(3-phenyl-propyl)-1-oxy-3',4',5',6'-tetrahydro-2'H-[3,4']bipyridin-1'-carbonsäure tert-butylester in Form eines farblosen Öls erhalten; MS: 553 $(M+H)^+$.

(d) In analoger Weise wie in Beispiel 133 (b) beschrieben, wurde aus (3RS,4RS)-4-(2-Methylsulfonyl-pyrimidin-5-yl)-3-(naphthalin-2-ylmethoxy)-piperidin-1-carbonsäure tert-butylester und 4-Phenylbutylamin (3RS,4RS)-3-(Naphthalin-2-ylmethoxy)-4-[2-(4-phenylbutylamino)-pyrimidin-5-yl]-piperidin-1-carbonsäure tert-butylester in Form eines amorphen, farblosen Festkörpers erhalten; MS: 568 $(M+H)^+$.

(e) 0.058 g (0.10 mMol) (3RS,4RS)-3-(Naphthalin-2-ylmethoxy)-4-[2-(4-phenyl-butylamino)-pyrimidin-5-yl]-piperidin-1-carbonsäure tert-butylester wurden in 1.0 ml N,N-Dimethylformamid gelöst und bei 0° C mit 0.08 ml (1.3 mMol) Methyliodid gefolgt von 0.010 g (ca. 0.2 mMol) Natriumhydriddispersion (ca. 50% in Mineralöl) versetzt und während 90 Minuten bei Raumtemperatur unter Argon gerührt. Nun wurde die Reaktionslösung zwischen Wasser und Äther verteilt, die vereinigten Ätherphasen über Magnesiumsulfat getrocknet, filtriert und im Wasserstrahlvakuum eingeengt. Der so erhaltene Rückstand wurde an Kieselgel mit Hexan/Essigsäureäthylester (1:1) chromatographiert. Dabei resultierten 0.021 g (35% d. Th.) (3RS,4RS)-4-{2-[Methyl-(4-phenylbutyl)-amino]-pyrimidin-5-yl}-3-(naphthalin-2-ylmethoxy)-piperidin-1-carbonsäure tert-butylester in Form eines amorphen, farblosen Festkörpers; MS: 582 $(M+H)^+$.

Beispiel 136

(a) Eine Lösung von 40 mg (0.080 mMol) (3RS,4RS)-4-(2-Methylsulfonylpyrimidin-5-yl)-3-(naphthalin-2-ylmethoxy)-piperidin-1-carbonsäure tert-butylester [Beispiel 129 (c)] in 1.1 ml 2 M Chlorwasserstoff in Methanol wurde 5 Stunden lang bei Raumtemperatur gerührt. Anschließend wurde die Reaktionslösung zwischen gesättigter Natriumcarbonatlösung und Methylenchlorid verteilt, die vereinigten Methylenchloridphasen über Magnesiumsulfat getrocknet und eingeengt. Dabei resultieren (33 mg, 95% d.Th.) 2-Methylsulfonyl-5-[(3RS,4RS)-3-(naphthalin-2-ylmethoxy)-piperidin-4-yl]-pyrimidin Hydrochlorid in Form eines farblosen Festkörpers; MS: 398 $(M+H)^+$.

(b) Durch Umsetzung von (3RS,4RS)-2-Methylsulfonyl-5-[(3RS,4RS)-3-(naphthalin-2-ylmethoxy)-piperidin-4-yl]-pyrimidin Hydrochlorid mit Alkoholen in analoger Weise wie in Beispiel 131 (b) beschrieben jedoch unter Verwendung von 2 Aequivalenten Natriumhydrid wurden die folgenden Verbindungen erhalten:

1) - Mit (E)-3-Phenyl-2-propen-1-ol (E)-5-[(3RS,4RS)-3-(Naphthalin-2-ylmethoxy)-piperidin-4-yl]-2-(3-phenylallyloxy)-pyrimidin in Form eines amorphen gelblichen Festkörpers; MS: 452 $(M+H)^+$;

2) - mit (E)-2-Methyl-3-phenyl-2-propen-1-ol (E)-2-(2-Methyl-3-phenylallyloxy)-5-[(3RS,4RS)-3-(naphthalin-2-ylmethoxy)-piperidin-4-yl]-pyrimidin in Form eines amorphen gelblichen Festkörpers; MS: 466 (M+H)+.

Beispiel 137

Durch Umsetzung von (3RS,4RS)-4-(2-Methylsulfonyl-pyrimidin-5-yl)-3-(naphthalin-2-ylmethoxy)-piperidin-1-carbonsäure tert-butylester [Beispiel 129 (c)] mit Alkoholen und Phenolen in analoger Weise wie in Beispiel 131 (b) beschrieben und anschliessende Abspaltung der BOC-Gruppe mittels 2 M Chlorwasserstoff in Methanol wie in Beispiel 136 (a) beschrieben wurden die folgenden Verbindungen erhalten:

1) - Mit 3-Hydroxy-biphenyl 2-(Biphenyl-3-yloxy)-5- [(3RS,4RS)-3-(naphthalin-2-ylmethoxy)-piperidin-4-yl]-pyrimidin in Form eines amorphen, farblosen Festkörpers; MS: 488 $(M+H)^+$;

2) - mit 3-Phenoxy-benzyl-alkohol 5-[(3RS,4RS)-3-(Naphthalin-2-ylmethoxy)-piperidin-4-yl]-2-(3-phenoxy-benzyloxy)-pyrimidin in Form eines amorphen, bräunlichen Festkörpers; MS: 519 $(M+H)^+$;

3) - mit 4-Phenoxy-phenol 5-[(3RS,4RS)-3-(Naphthalin-2-ylmethoxy)-piperidin-4-yl]-2-(4-phenoxy-phenoxy)-pyrimidin in Form eines amorphen, bräunlichen Festkörpers; MS: 504 $(M+H)^+$ sowie 2-Methoxy-5-[(3RS,4RS)-3-(naphthalin-2-ylmethoxy)-piperidin-4-yl]-pyrimidin in Form eines amorphen, bräunlichen Gummis; MS: 349 $(M+H)^+$;

4) - mit 4-Hydroxy-biphenyl 2-(Biphenyl-4-yloxy)-5-[(3RS,4RS)-3-(naphthalin-2-ylmethoxy)-piperidin-4-yl]-pyrimidin in Form eines amorphen, farblosen Festkörpers; MS: 488 $(M+H)^+$.

5) - mit 3-Phenyl-2-proyn-1-ol 5-[(3RS,4RS)-3-(Naphthalin-2-ylmethoxy)-piperidin-4-yl]-2-(3-phenyl-prop-2-ynyloxy)-pyrimidin in Form eines amorphen, bräunlichen Festkörpers; MS: 451 $(M+H)^+$;

6) - mit 2-Hydroxymethyl-2,3-dihydro-benzo[1,4]dioxin 2-(2RS und 2SR)-(2,3-Dihydro-benzo[1,4]dioxin-2-ylmethoxy)-5-[(3RS,4RS)-3-(naphthalin-2-ylmethoxy)-piperidin-4-yl]-pyrimidin in Form eines farblosen Festkörpers; MS: 484 $(M+H)^+$;

7) - mit 4-Biphenyl-äthanol [Chemische Berichte 85, 897 (1952)] 2-(2-Biphenyl-4-yl-äthoxy)-5-[(3RS,4RS)-3-(naphthalin-2-ylmethoxy)-piperidin-4-yl]-pyrimidin Form eines amorphen, gelblichen Gummis; MS: 517 $(M+H)^+$;

8) - mit 4-Phenoxy-benzylalkohol 5-[(3RS,4RS)-3-(Naphthalin-2-ylmethoxy)-piperidin-4-yl]-1-(4-phenoxy-benzyl)-1H-pyrimidin-2-on in Form eines amorphen, farblosen Festkörpers; MS: 518 $(M+H)^+$;

9) - mit 4-Biphenyl-methanol 2-(Biphenyl-4-ylmethoxy)-5-[(3RS,4RS)-3-(naphthalin-2-ylmethoxy)-piperidin-4-yl]-pyrimidin in Form eines gelblichen Gummis; MS: 503 $(M+H)^+$;

10) - mit [1-(4-Chlor-phenyl)-cyclopentyl]-methanol 2-[[1-(4-Chlorphenyl)-cyclopentyl]-methoxy]-5-[(3RS,4RS)-3-(naphthalin-2-ylmethoxy)-piperidin-4-yl]-pyrimidin in Form eines farblosen Festkörpers; MS: 528 $(M+H)^+$;

11) - mit 2-Naphthalin-methanol 2-(Naphthalin-2-ylmethoxy)-5-[(3RS,4RS)-3-(naphthalin-2-ylmethoxy)-piperidin-4-yl]-pyrimidin in Form eines amorphen, gelblichen Festkörpers; MS: 476 $(M+H)^+$;

12) - mit 2-Naphthalin-äthanol 2-(2-Naphthalin-2-yl-äthoxy)-5-[(3RS,4RS)-3-(naphthalin-2-ylmethoxy)-piperidin-4-yl]-pyrimidin in Form eines amorphen, bräunlichen Festkörpers; MS: 490 $(M+H)^+$;

13) - mit 2-(4-Bromphenyl)-äthanol 2-[2-(4-Bromo-phenyl)-äthoxy]-5-[(3RS,4RS)-3-(naphthalin-2-ylmethoxy)-piperidin-4-yl]-pyrimidin in Form eines amorphen, bräunlichen Festkörpers; MS: 518, 520 $(M+H)^+$;

14) - mit 2-(2-Chloro-phenoxy)-äthanol 2-[2-(2-Chloro-phenoxy)-äthoxy]-5-[(3RS,4RS)-3-(naphthalin-2-ylmethoxy)-piperidin-4-yl]-pyrimidin in Form eines amorphen, bräunlichen Festkörpers; MS: 490 $(M+H)^+$;

15) - mit 2-Benyzloxy-äthanol 2-(2-Benzyloxy-äthoxy)-5-[(3RS,4RS)-3-(naphthalin-2-ylmethoxy)-piperidin-4-yl]-pyrimidin in Form eines amorphen, gelblichen Festkörpers; MS: 470 $(M+H)^+$;

16) - mit 3-Cyclohexyl-propanol 2-(3-Cyclohexyl-propoxy)-5-[(3RS,4RS)-3-(naphthalin-2-ylmethoxy)-piperidin-4-yl]-pyrimidin in Form eines amorphen, bräunlichen Festkörpers; MS: 461 $(M+H)^+$;

17) - mit 3-(6-Methyl-pyridin-2-yl)-propanol 2-[3-(6-Methyl-pyridin-2-yl)-propoxy]-5-[(3RS,4RS)-3-(naphthalin-2-ylmethoxy)-piperidin-4-yl]-pyrimidin in Form eines amorphen, bräunlichen Festkörpers; MS: 469 $(M+H)^+$;

18) - mit 2-Cyclohexyloxy-äthanol 2-(2-Cyclohexyloxy-äthoxy)-5-[(3RS,4RS)-3-(naphthalin-2-ylmethoxy)-piperidin-4-yl]-pyrimidin in Form eines amorphen, gelblichen Festkörpers; MS: 462 $(M+H)^+$;

19) - mit 2-(Phenylthio)-äthanol 5-[(3RS,4RS)-3-(Naphthalin-2-ylmethoxy)-piperidin-4-yl]-2-(2-phenylsulfanyl-äthoxy)-pyrimidin in Form eines amorphen, gelblichen Festkörpers; MS: 472 $(M+H)^+$;

20) - mit 2-(5-Methyl-2-phenyloxazol-4-yl)-äthanol (erworben von der Maybridge Chemical Company) 2-[2-(5-Methyl-2-phenyl-oxazol-4-yl)-äthoxy]-5-[(3RS,4RS)-3-(naphthalin-2-ylmethoxy)-piperidin-4-yl]-pyrimidin in Form eines amorphen, bräunlichen Festkörpers; MS: 522 $(M+H)^+$;

21) - mit 2-Cyclohexyl-äthanol 2-(2-Cyclohexyl-äthoxy)-5-[(3RS,4RS)-3-(naphthalin-2-ylmethoxy)-piperidin-4-yl]-pyrimidin in Form eines amorphen, farblosen Festkörpers; MS: 447 $(M+H)^+$;

22) - mit (RS)-4-(2-Hydroxy-äthyl)-5-methyl-2-phenyl-2,4-dihydropyrazol-3-on (erworben von der Maybridge Chemical Company) ein Gemisch von (RS)- und (SR)-5-Methyl-4-[2-[5-[(3RS,4RS)-3-(naphthalin-2-ylmethoxy)-piperidin-4-yl]-pyrimidin-2-yloxy]-äthyl]-2-phenyl-2,4-dihydro-pyrazol-3-on in Form eines amorphen, farblosen Festkörpers; MS: 536 $(M+H)^+$.

Beispiel 138

0.045 g (0.082 mMol) (3'RS,4'RS)-3'-(Naphthalin-2-ylmethoxy)-6-(3-phenyl-propyl)-1-oxy-3',4',5',6'-tetrahydro-2'H-[3,4']bipyridin-1'carbonsäure tert-butylester [Beispiel 135 (c)] wurden in 1.5 ml N,N-Dimethylformamid gelöst, mit 0.10 ml (0.8 mMol) Trifluoressigsäureanhydrid versetzt und während 2 Stunden bei Raumtemperatur gerührt. Hierauf wurde das Reaktionsgemisch zwischen Methylenchlorid und gesättigter Bicarbonatlösung verteilt, die vereinigten Methylenchloridphasen über Magnesiumsulfat getrocknet, filtriert und im Wasserstrahlvakuum eingeengt. Der so erhaltene Rückstand wurde an Kieselgel mit Methylenchlorid/Methanol (95:5) chromatographiert. Dabei resultierten 0.019 g (52% d. Th.) (3'RS,4'RS)-3'-(Naphthalin-2-ylmethoxy)-6-(3-phenyl-propyl)-1',2',3',4',5',6'hexahydro-[3,4']bipyridin-4-ol in Form eines amorphen, farblosen Festkörpers; MS: 453 $(M+H)^+$.

Beispiel 139

Durch Umsetzung von (3RS,4RS)-3-(1,4-Dimethoxy-naphthalin-2-ylmethoxy)-4-(2-methylsulfonyl-pyrimidin-5-yl)-piperidin-1-carbonsäure tert-butylester mit Alkoholen in analoger Weise wie in Beispiel 131 (b) beschrieben und anschliessende Abspaltung der BOC-Gruppe mittels wasserfreiem Zinkbromid in Methylenchlorid wie in Beispiel 125 (h) beschrieben, wurden die folgenden Verbindungen erhalten:

1) - Mit 3-Phenyl-2-propyn-1-ol 5-[(3RS,4RS)-3-(1,4-Dimethoxynaphthalin-2-ylmethoxy)-piperidin-4-yl]-2-(3-phenyl-prop-2-ynyloxy)-pyrimidin in Form eines amorphen, gelblichen Festkörpers; MS: 510 $(M+H)^+$;

2) - mit 3-Cyclohexyl-propanol 2-(3-Cyclohexyl-propoxy)-5-[(3RS,4RS)-3-(1,4-dimethoxy-naphthalin-2-ylmethoxy)-piperidin-4-yl]-pyrimidin in Form eines amorphen, bräunlichen Festkörpers; MS: 521 $(M+H)^+$;

3) - mit 4-Cyclohexyl-butanol 2-(4-Cyclohexyl-butoxy)-5-[(3RS,4RS)-3-(1,4-dimethoxy-naphthalin-2-ylmethoxy)-piperidin-4-yl]-pyrimidin in Form eines amorphen, gelblichen Festkörpers; MS: 535 $(M+H)^+$;

4) - mit 2-Indan-2-yl-äthanol [J. Am. Chem. Soc. 87, 1297 (1965)] 5-[(3RS,4RS)-3-(1,4-Dimethoxy-naphthalin-2-ylmethoxy)-piperidin-4-yl]-2-(2-indan-2-yl-äthoxy)-pyrimidin in Form eines amorphen, gelblichen Festkörpers; MS: 540 $(M+H)^+$;

5) - mit 3-(2-Methoxy-benzyloxy)-propan-1-ol (hergestellt durch Alkylierung von Propylenglycol in grossem Überschuss mit 2-Methoxybenzylchlorid unter Verwendung von Natriumhydrid in N,N-Dimethylformamid) 5-[(3RS,4RS)-3-(1,4-Dimethoxy-naphthalin-2-ylmethoxy)-piperidin-4-yl]-2-[3-(2-methoxy-benzyloxy)propoxy]-pyrimidin in Form eines amorphen, farblosen Festkörpers; MS: 574 $(M+H)^+$;

6) - mit (E)-4-Phenyl-but-3-en-1-ol [Beispiel 122 (β)] 5-[(3RS,4RS)-3-(1,4-Dimethoxy-naphthalin-2-ylmethoxy)-piperidin-4-yl]-2-[(E)-4-phenyl-but-3-enyloxy]-pyrimidin in Form eines amorphen, farblosen Festkörpers; MS: 526 $(M+H)^+$;

7) - mit 2-(5-Phenyl-pyridin-2-yl)-äthanol [Beispiel 139 (β)] 5-[(3RS,4RS)-3-(1,4-Dimethoxy-naphthalin-2-ylmethoxy)-piperidin-4-yl]-2-[2-(5-phenyl-pyridin-2-yl)-äthoxy]-pyrimidin in Form eines amorphen, farblosen Festkörpers; MS: 576 $(M)^+$;

8) - mit 5-Phenyl-4-pentyn-1-ol 5-[(3RS,4RS)-3-(1,4-Dimethoxynaphthalin-2-ylmethoxy)-piperidin-4-yl]-2-(5-phenyl-pent-4-ynyloxy)-pyrimidin in Form eines amorphen, orangen Festkörpers; MS: 537 $(M)^+$.

Durch Umsetzung von (3RS,4RS)-3-(1,4-Dimethoxy-naphthalin-2-ylmethoxy)-4-(2-methylsulfonyl-pyrimidin-5-yl)-piperidin-1-carbonsäure tert-butylester mit Aminen in analoger Weise wie in Beispiel 133 (b) beschrieben und anschliessende Abspaltung der BOC-Gruppe mittels wasserfreiem Zinkbromid in Methylenchlorid wie in Beispiel 125 (h) beschrieben, wurden die folgenden Verbindungen erhalten:

9) - Mit 4-Phenylbutylamin {5-[(3RS,4RS)-3-(1,4-Dimethoxynaphthalin-2-ylmethoxy)-piperidin-4-yl]-pyrimidin-2-yl}-(4-phenylbutyl)-amin in Form eines amorphen, orangen Festkörpers; MS: 527 $(M+H)^+$;

10) - mit 2-(5-Phenyl-pyridin-2-yl)-äthylamin [Beispiel 139 (δ)] {5-[(3RS,4RS)-3-(1,4-Dimethoxy-naphthalin-2-ylmethoxy)-piperidin-4-yl]-pyrimidin-2-yl}-[2-(5-phenyl-pyridin-2-yl)-äthyl]-amin in Form eines amorphen, farblosen Festkörpers; MS: 576 $(M+H)^+$;

11) - mit 3-Methoxy-benzylamin {5-[(3RS,4RS)-3-(1,4-Dimethoxynaphthalin-2-ylmethoxy)-piperidin-4-yl]-pyrimidin-2-yl}-(3-methoxybenzyl)-amin in Form eines amorphen, farblosen Festkörpers; MS: 514 $(M)^+$;

12) - mit 4-Methoxy-benzylamin {5-[(3RS,4RS)-3-(1,4-Dimethoxynaphthalin-2-ylmethoxy)-piperidin-4-yl]-pyrimidin-2-yl}-(4-methoxybenzyl)-amin in Form eines amorphen, gelblichen Festkörpers; MS: 514 $(M)^+$;

13) - mit 3-Brom-benzylamin (3-Bromo-benzyl)-{5-[(3RS,4RS)-3-(1,4-dimethoxy-naphthalin-2-ylmethoxy)-piperidin-4-yl]-pyrimidin-2-yl}amin in Form eines amorphen, farblosen Festkörpers; MS: 562, 564 $(M)^+$;

14) - mit 3-Methyl-benzylamin {5-[(3RS,4RS)-3-(1,4-Dimethoxynaphthalin-2-ylmethoxy)-piperidin-4-yl] -pyrimidin-2-yl}-(3-methylbenzyl)-amin in Form eines amorphen, farblosen Festkörpers; MS: 499 $(M+H)^+$;

15) - mit 4-Brom-benzylamin (4-Bromo-benzyl)-{5-[(3RS,4RS)-3-(1,4-dimethoxy-naphthalin-2-ylmethoxy)-piperidin-4-yl]-pyrimidin-2-yl}amin in Form eines amorphen, farblosen Festkörpers; MS: 563, 565 $(M+H)^+$.

Der als Ausgangsmaterial benötigte (3RS,4RS)-3-(1,4-Dimethoxynaphthalin-2-ylmethoxy)-4-(2-methylsulfonyl-pyrimidin-5-yl)-piperidin-1- carbonsäure tert-butylester wurde wie folgt hergestellt:

(a) In analoger Weise wie in Beispie 125 (g) beschrieben, wurde aus dem (3R5 ,4RS )-3-Hydroxy-4-(2-methylsulfanyl-pyrimidin-5-yl)-piperidin- 1-carbonsäure tert-butylester [Beispiel 129 (a)] und 2-Chlormethyl-1,4-dimethoxy-naphthalin [J. Amer. Chem. Soc. 64, 2657 (1942)] der (3RS,4RS)-3-(1,4-Dimethoxy-naphthalin-2-ylmethoxy)-4-(2-methylsulfanyl-pyrimidin-5-yl)-piperidin-1-carbonsäure tert-butylester in Form eines amorphen farblosen Feskörpers erhalten; MS: 527 $(M+H)^+$.

(b) In analoger Weise wie in Beispiel 129 (c) beschrieben, wurde aus dem (3RS,4RS)-3-(1,4-Dimethoxy-naphthalin-2-ylmethoxy)-4-(2-methylsulfanyl-pyrimidin-5-yl)-piperidin-1-carbonsäure tert-butylester durch Oxidation mit m-Chlorperbenzoesäure der (3RS,4RS)-3-(1,4-Dimethoxy-naphthalin-2-ylmethoxy)-4-(2-methylsulfonyl-pyrimidin-5-yl)-piperidin-1- carbonsäure tert-butylester in Form eines farblosen Festkörpers erhalten; MS: 558 $(M+H)^+$.

Die als Kondensationsreagenzien verwendeten 2-(5-Phenyl-pyridin-2-yl)-äthanol und 2-(5-Phenyl-pyridin-2-yl)-äthylamin wurden wie folgt hergestellt:

(α) 1.16 g (4 mMol) 2-(2-Benzyloxy-äthyl)-5-brom-pyridin [Beispiel 128 (b)], , 139 mg (1.13 mMol) Tetrakis-(triphenylphosphin)-palladium und 537 mg (8.2 mMol) Phenylborsäure wurden in wenig Äthanol gelöst und zu 80 ml Toluol auf einmal zugegeben. Anschliessend wurden 1.87 g (17.6 mMol) Natriumcarbonat in 4.4 ml Wasser zugegeben und das Reaktionsgemisch während 7.5 Stunden unter Argon und unter Rückfluss erwärmt. Nach dem Abkühlen wurde die Reaktionslösung im Wasserstrahlvakuum eingeengt und zwischen Methylenchlorid und Wasser verteilt. Die vereinigten Methylenchloridphasen wurden über Magnesiumsulfat getrocknet, filtriert und im Wasserstrahlvakuum eingeengt. Der so erhaltene Rückstand wurde hierauf an Kieselgel mit Methylenchlorid/Äther (93:7) chromatographiert. Dabei wurden 1.005 g (87% d. Th.) 2-(2-Benzyloxy-äthyl)-5-phenyl-pyridin als farbloser Festkörper erhalten; $R_f$: 0.08 ($SiO_2$, Methylenchlorid:Äther=93:7).

(β) 1.0 g (3.5 mMol) 2-(2-Benzyloxy-äthyl)-5-phenyl-pyridin wurden in 1 ml Eisessig gelöst, mit 1.8 ml (6.7 mMol) Bromwasserstoffsäure in Eisessig (30%) versetzt und das Reaktionsgemisch während 18 Stunden bei Raumtemperatur gerührt. Hierauf wurde Eiswasser zugegeben, zweimal mit Hexan extrahiert, die Hexanphasen verworfen, die wässrige Phase mit Natriumcarbonat-Lösung alkalisch gestellt und dreimal mit Äther extrahiert. Die vereinigten Ätherphasen wurden über Magnesiumsulfat getrocknet, filtriert und im Wasserstrahlvakuum eingeengt. Der so erhaltene rohe Essigsäure 2-(5-phenylpyridin-2-yl)-äthylester wurde hierauf in 12 ml Acetonitril aufgenommen und mit 5 ml Wasser und 4 ml 2N Natronlauge versetzt und 2.5 Stunden bei Raumtemperatur gerührt. Hierauf wurde wässrige Ammoniumchloridlösung zugesetzt und mit Methylenchlorid extrahiert. Die vereinigten Methylenchloridphasen wurden über Magnesiumsulfat getrocknet, filtriert und im Wasserstrahlvakuum eingeengt. Der so erhaltene Rückstand wurde hierauf an Kieselgel mit Methylenchlorid/Äther (1:1) chromatographiert. Dabei wurden 0. 656 g (94% d. Th.) 2-(5-Phenyl-pyridin-2-yl)-äthanol als farbloser Festkörper erhalten; $R_f$: 0.09 ($SiO_2$, Methylenchlorid: Äther=1:1).

(γ) 0.473 g (2.4 mMol) 2-(5-Phenyl-pyridin-2-yl)-äthanol, 0.420 g (2.85 mMol) Phthalimid und 0.747 g (2.85 mMol) Triphenylphosphin wurden in 10 ml Tetrahydrofuran gelöst und das Reaktionsgemisch hierauf unter Argon bei -5 °C mit 0.47 ml (3.0 mMol) Azodicarbonsäure diäthylester versetzt, weitere 20 Minuten bei -5 °C und weitere 18 Stunden bei Raumtemperatur gerührt. Hierauf wurde das Reaktionsgemisch im Wasserstrahlvakuum eingeengt und der Rückstand an Kieselgel mit Methylenchlorid/Methanol (98:2) chromatographiert. Dabei wurden 0. 657 g (83% d. Th.) 2-[2-(5-Phenylpyridin-2-yl)-äthyl]-isoindol-1,3-dion als farbloser Festkörper erhalten; $R_f$: 0.38 ($SiO_2$, Hexan:Essigsäureäthylester=1:1).

(8) 0.657 g (2.0 mMol) 2-[2-(5-Phenyl-pyridin-2-yl)-äthyl]-isoindol-1,3-dion, 0.5 ml Hydrazin-Hydrat und 5 ml Äthanol wurden unter Argon während 3.5 Stunden unter Rückfluss erwärmt. Hierauf wurde mit 5 ml Äthanol und 20 ml Äther verdünnt, filtriert und das Filtrat im Wasserstrahlvakuum eingeengt. Der so erhaltene Rückstand wurde anschliessend an Kieselgel mit Methylenchlorid/Methanol/konz. aq. Ammoniak (89:10:1) chromatographiert. Dabei wurden 0. 250 g (63% d. Th.) 2-(5-Phenyl-pyridin-2-yl)-äthylamin als gelblicher, amorpher Festkörper erhalten; $R_f$: 0.17 ($SiO_2$, Methylenchlorid:Methanol:konz. aq. Ammoniak=89:10:1).

Beispiel 140

Durch Umsetzung von (3'RS,4'RS)-3'-(1,4-Dimethoxy-naphthalin-2-ylmethoxy)-6-methylsulfonyl-3',4',5',6'-tetrahydro-2'H-[3,4']bipyridin-1'-carbonsäure tert-butylester mit Alkoholen in analoger Weise wie in Beispiel 129 (d) beschrieben und anschliessende Abspaltung der BOC-Gruppe mittels wasserfreiem Zinkbromid in Methylenchlorid wie in Beispiel 125 (h) beschrieben, wurden die folgenden Verbindungen erhalten:

1) - Mit 3-Cyclohexyl-propanol (3'RS,4'RS)-6-(3-Cyclohexyl-propoxy)-3'-(1,4-dimethoxy-naphthalin-2-ylmethoxy)-1',2',3',4',5',6'-hexahydro-[3,4']bipyridin in Form eines farblosen Festkörpers; MS: 519 $(M+H)^+$.

2) - Mit 3-(2-Methoxy-benzyloxy)-propan-1-ol (hergestellt durch Alkylierung von Propylenglycol in grossem Überschuss mit 2-Methoxybenzylchlorid unter Verwendung von Natriumhydrid in N,N-Dimethylformamid) (3'RS,4'RS)-3'-(1,4-Dimethoxy-naphthalin-2-ylmethoxy)-6-[3-(2-methoxybenzyloxy)-propoxy]-1',2',3',4',5',6'hexahydro-[3,4'] bipyridin in Form eines amorphen, farblosen Festkörpers; MS: 573 $(M+H)^+$.

3) - Mit 4-Cyclohexyl-butanol (3'RS,4'RS)-6-(4-Cyclohexyl-butoxy)-3'-(1,4-dimethoxy-naphthalin-2-ylmethoxy)- 1', 2',3',4',5',6'-hexahydro-[3,4']bipyridin in Form eines amorphen, farblosen Öles; MS: 533 $(M+H)^+$.

Der als Ausgangsmaterial eingesetzte (3'RS,4'RS)-3'-(1,4-Dimethoxynaphthalin-2-ylmethoxy)-6-methylsulfonyl-3',4',5',6'-tetrahydro-2'H-[3,4']bipyridin-1'-carbonsäure tert-butylester wurde wiefolgt hergestellt:

(a) In analoger Weise wie in Beispiel 127 (b) - (c) beschrieben, wurde aus 2-Methylsulfanyl-5-bromo-pyridin [Tetrahedron 41, 1373 (1985)] via 2-Methylsulfanyl-5-trimethylstannanyl-pyridin [farbloses Öl, MS: 289 $(M)^+$] sowie

4-Trifluormethylsulfonyloxy-3,6-dihydro-2H-pyridin-1-carbonsäure tert-butylester, 6-Methylsulfanyl-3',6'-dihydro-2'H-[3,4']bipyridin-1'-carbonsäure tert-butylester in Form eines gelblichen Festkörpers erhalten: MS: 307 $(M+H)^+$.

(b) 1.5 g (4.9 mMol) 6-Methylsulfanyl-3',6'-dihydro-2'H-[3,4']bipyridin-1'carbonsäure tert-butylester wurden in 15 ml 1,2-Dimethoxyäthan gelöst, bei 3 - 4° C mit 8.8 ml 1 molarer Boran-Tetrahydrofuranlösung versetzt und anschliessend während 4 Stunden bei Raumtemperatur gerührt. Hierauf wurden unter Eiskühlung 15 ml Wasser und anschliessend portionenweise 3.5 g (22.3 mMol) festes Natriumpercarbonat zugesetzt und das Reaktionsgemisch während 1 Stunde auf 50° C erwärmt. Nun wurde die Reaktionslösung zwischen Wasser und Methylenchlorid verteilt, die vereinigten Methylenchloridphasen mit Natriumpyrosulfit-Lösung und Wasser gewaschen, über Magnesiumsulfat getrocknet und eingeengt. Der so erhaltene Rückstand wurde anschliessend an Kieselgel mit Hexan/Essigsäureäthylester (1:1) chromatographiert. Dabei resultierten 330 mg (21% d. Th.) (3'RS,4'RS)-3'-Hydroxy-6-methylsulfanyl-3',4',5',6'-tetrahydro-2'H-[3,4']bipyridin-1'-carbonsäure tert-butylester in Form eines farblosen, amorphen Festkörpers: MS: 324 $(M)^+$.

(c) In analoger Weise wie in Beispiel 125 (g) beschrieben, wurde aus (3'RS,4'RS)-3'-Hydroxy-6-methylsulfanyl-3',4',5',6'-tetrahydro-2'H-[3,4']bipyridin-1'-carbonsäure tert-butylester durch Alkylierung mit 2-Chlormethyl-1,4-dimethoxy-naphthalin [J. Amer. Chem. Soc. 64, 2657 (1942)] (3'RS,4'RS)-3'-(1,4-Dimethoxy-naphthalin-2-ylmethoxy)-6-methylsulfanyl-3',4',5',6'-tetrahydro-2'H-[3,4']bipyridin-1'-carbonsäure tert-butylester in Form eines farblosen Festkörpers erhalten; MS: 526 $(M+H)^+$.

(d) In analoger Weise wie in Beispiel 129 (c) beschrieben, wurde aus (3'RS,4'RS)-3'-(1,4-Dimethoxy-naphthalin-2-ylmethoxy)-6-methylsulfanyl-3',4',5',6'-tetrahydro-2'H-[3,4']bipyridin-1'-carbonsäure tert-butylester durch Oxidation mit m-Chlorperbenzoesäure (3'RS,4'RS)-3'-(1,4-Dimethoxy-naphthalin-2-ylmethoxy)-6-methylsulfonyl-3',4',5',6'-tetrahydro-2'H-[3,4']bipyridin-1'-carbonsäure tert-butylester in Form eines amorphen, farblosen Festkörpers erhalten; MS: 557 $(M+H)^+$.

Beispiel 141

(a) 50.0 g (0.6 Mol) 1,2,5,6-Tetrahydropyridin und 135.3 g (0.6 Mol) Ditert-butyl-dicarbonat wurden unter Zusatz von 166.0 g (1.2 Mol) Kaliumcarbonat (wasserfrei) in 1250 ml Wasser (deionisiert) / Dioxan (3:2) während 3 Stunden bei Raumtemperatur gerührt. Der Ansatz wurde auf Eiswasser gegossen, das Produkt 3 mal mit je 300 ml Essigester extrahiert, die organischen Phasen wurden 2 mal mit je 500 ml destilliertem Wasser gewaschen, dann über Magnesiumsulfat getrocknet, filtriert und im Wasserstrahlvakuum eingeengt. Das so erhaltene Rohprodukt wurde an Kieselgel mit Hexan und Essigester chromatographiert. Nach 3 Stunden Trocknen im Hochvakuum bei Raumtemperatur wurden so 109.4 g (99% d. Th.) 3,6-Dihydro-2Hpyridin-1-carbonsäure tert-butylester als hellgelbes Öl erhalten; MS: 183 $(M)^+$.

(b) 127.0 g (0.6 Mol) m-Chlorperbenzoesäure wurden in 1.5 l Methylenchlorid unter Argon gelöst, dann eine Lösung von 108 g (0.59 Mol) 3,6-Dihydro-2H-pyridin-1-carbonsäure tert-butylester in 500 ml Methylenchlorid bei 5° C innert 1 Stunde zugetropft und anschliessend über Nacht bei Raumtemperatur gerührt. Der Ansatz wurde auf Eiswasser gegossen, mit Kaliumcarbonatlösung auf pH >8 gestellt und nach Phasentrennung wurde 2 mal mit je 500 ml Methylenchlorid nachextrahiert; die organischen Phasen wurden 2 mal mit Wasser neutral gewaschen, dann über Magnsiumsulfat getrocknet, filtriert und das Lösungsmittel im Wasserstrahlvakuum abdestilliert. Das Rohprodukt wurde an Kieselgel mit Hexan und Essigester chromatographiert. Dabei erhielt man 86.64 g (74% d. Th.) (1RS,6SR)-7-Oxa-3-aza-bicyclo[4.1.0]heptan-3-carbonsäure tert-butylester als hellgelbes Öl; MS: 199 $(M)^+$.

(c) 19.9 g (100 mMol) (1RS,6SR)-7-Oxa-3-aza-bicyclo[4.1.0]heptan-3-carbonsäure tert-butylester und 32.5 g (500 mMol) Natriumazid wurden unter Zusatz von 39.1 g (250 mMol) Magnesiumsulfat Dihydrat in 500 ml Methanol abs. während 3 Stunden bei Rückfluss gerührt. Der Ansatz wurde hierauf auf 10° C abgekühlt, filtriert und das Lösungsmittel im Wasserstrahlvakuum abdestilliert. Der Rückstand wurde in 300 ml Methylenchlorid aufgenommen, nochmals filtriert und das Lösungsmittel im Wasserstrahlvakuum abdestilliert. Das so erhaltene Rohprodukt wurde an Kieselgel mit n-Hexan und Essigester chromatographiert. So erhielt man 15.86 g (66% d. Th.) (3RS, 4RS)-4-Azido-3-hydroxy-piperidin-1-carbonsäure tert-butylester in Form farbloser Kristalle; MS: 242 $(M)^+$.

(d) 15.4 g (63.5 mMol) (3RS,4RS)-4-Azido-3-hydroxy-piperidin-1-carbonsäure tert-butylester und 15.5 g (69.9 mMol) 2-Brom-methylnaphthalin wurden unter Argon in 200 ml Dimethylformamid bei 5° C vorgelegt. Hierauf wurden auf einmal 3.33 g (76.2 mMol) Natriumhydrid-Dispersion (55% in Mineralöl) unter externer Kühlung zugegeben, dann über Nacht bei Raumtemperatur gerührt. Der Ansatz wurde auf Eiswasser gegossen, das Produkt 3 mal mit je 200 ml Essigester extrahiert, die organischen Phasen wurden 2 mal mit je 300 ml Wasser gewaschen, dann über Magnesiumsulfat getrocknet, filtriert und im Wasserstrahlvakuum eingeengt. Das so erhaltene Rohprodukt wurde an Kieselgel mit n-Hexan und Essigester chromatographiert. So erhielt man 23.18 g (95% d. Th.) (3RS, 4RS)-4-Azido-3-(naphthalin-2-ylmethoxy)-piperidin-1-carbonsäure tert-butylester als hellgelbes Öl; MS: 383 $(M+H)^+$.

(e) 3.20 g (8.37 mMol) (3RS,4RS)-4-Azido-3-(naphthalin-2-ylmethoxy)-piperidin-1-carbonsäure tert-butylester und 9.87 ml (176.3 mMol) Propargylalkohol wurden in 80 ml Toluol während 5 Stunden bei Rückfluss gerührt. Nach dem Abdestillieren des Lösungsmittels im Wasserstrahlvakuum wurde das Rohprodukt an Kieselgel mit Methylenchlorid und Methanol chromatographiert. So erhielt man 1.47 g (40% d. Th.) (3RS,4RS)-4-(4-Hydroxymethyl-[1,2,3]triazol-1-yl)-3-(naphthalin-2-ylmethoxy)-4-piperidin-1-carbonsäure tert-butylester in Form farbloser Kristalle [MS: 439 $(M+H)^+$] und 0.81 g (22% d. Th.) (3RS,4RS)-4-(5-Hydroxymethyl-[1,2,3]triazol-1-yl)-3-(naphthalin-2-ylmethoxy)-4-piperidin-1-carbonsäure tert-butylester in Form farbloser Kristalle; MS: 439 $(M+H)^+$.

(f) 0.22 g (0.5 mMol) (3RS,4RS)-4-(4-Hydroxymethyl-[1,2,3)triazol-1-yl)-3-(naphthalin-2-ylmethoxy)-4-piperidin-1-carbonsäure tert-butylester und 0.064 ml (0.5 mMol) ortho-Chlor-benzoylchlorid wurden in 10 ml Methylenchlorid unter Argon bei Raumtemperatur vorgelegt. Hierauf wurden zuerst unter Rühren 0.41 ml (3 mMol) Triäthylamin, dann 0.025 g (0.2 mMol) 4-Dimethylamino-pyridin zugegeben und 18 Stunden bei Raumtemperatur gerührt. Nach dem Abdestillieren des Lösungsmittels im Wasserstrahlvakuum wurde das Rohprodukt an Kieselgel mit Methylenchlorid und Methanol chromatographiert. So erhielt man 0.14 g (49% d. Th.) (3RS,4RS)-4-[4-(2-Chlorbenzoyloxymethyl)-[1,2,3]triazol-1-yl]-3-(naphthalin-2-ylmethoxy)-piperidin-1-carbonsäure tert-butylester als farbloses Harz; MS: 577 $(M+H)^+$.

(g) In analoger Weise wie in Beispiel 136 (a) beschrieben, wurde aus (3RS,4RS)-4-[4-(2-Chlor-benzoyloxymethyl) -[1,2,3]triazol-1-yl]-3-(naphthalin-2-ylmethoxy)-piperidin-1-carbonsäure tert-butylester durch Abspaltung der BOC-Gruppe mit Chlorwasserstoff in Methanol 2-Chlor-benzoesäure 1-[(3RS,4RS)-3-(Naphthalin-2-ylmethoxy)-piperidin-4-yl]-1H-[1,2,3]triazol-4-ylmethyl ester Hydrochlorid (1:1 ) in Form farbloser Kristalle erhalten; MS: 477 $(M+H)^+$.

Beispiel 142

In analoger Weise wie in Beispiel 136 (a) beschrieben, wurden durch Abspaltung der BOC-Gruppe mittels Chlorwasserstoff in Methanol die folgenden Verbindungen erhalten:

1) - Aus (3RS,4RS)-4-(5-Benzyloxymethyl-[1,2,3)triazol-1-yl)-3-(naphthalin-2-ylmethoxy)-piperidin-1-carbonsäure tert-butylester (3RS,4RS)-4-(5-Benzyloxymethyl-[1,2,3]triazol-1-yl)-3-(naphthalin-2-ylmethoxy)-piperidin als hellgelbes Öl; MS: 429 $(M+H)^+$;

2) - aus (3RS,4RS)-4-(4-Benzyloxymethyl-[1,2,3]triazol-1-yl)-3-(naphthalin-2-ylmethoxy)-piperidin-1-carbonsäure tert-butylester (3RS,4RS)-4-(4-Benzyloxymethyl-[1,2,3]triazol-1-yl)-3-(naphthalin-2-ylmethoxy)-piperidin Hydrochlorid (1:1) in Form farbloser Kristalle; MS: 429 $(M+H)^+$;

3) - aus (3RS,4RS)-4-[5-(3-Benzyloxy-propoxymethyl)-[1,2,3]triazol-1-yl]-3-(naphthalin-2-ylmethoxy)-piperidin-1-carbonsäure tert-butylester (3RS,4RS)-4-[5-(3-Benzyloxy-propoxymethyl)-[1,2,3)triazol-1-yl]-3-(naphthalin-2-ylmethoxy)-piperidin als farbloses Öl; MS: 487 $(M+H)^+$;

4) - aus (3RS,4RS)-4-[4-(3-Benzyloxy-propoxymethyl)-[1,2,3]triazol-1-yl]-3-(naphthalin-2-ylmethoxy)-piperidin-1-carbonsäure tert-butylester (3RS,4RS)-4-[4-(3-Benzyloxy-propoxymethyl)-[1,2,3)triazol-1-yl)-3-(naphthalin-2-ylmethoxy)-piperidin als farbloses Öl; MS: 487 $(M+H)^+$.

Die als Ausgangssubstanzen eingesetzten BOC-Derivate wurden wie folgt hergestellt:

(a) 0.22 g (0.5 mMol) (3RS,4RS)-4-(5-Hydroxymethyl-[1,2,3] triazol-1-yl)-3-(naphthalin-2-ylmethoxy)-4-piperidin-1-carbonsäure tert-butylester [Beispiel 141 (e)] und 0.09 ml (0.75 mMol) Benzylbromid wurden unter Argon in 5 ml Dimethylformamid bei 5° C vorgelegt, dann 0.044 g (1 mMol) Natriumhydrid-Dispersion (55% in Mineralöl) auf einmal zugegeben und während 18 Stunden bei Raumtemperatur gerührt. Der Ansatz wurde hierauf auf Eiswasser

gegossen, das Produkt 3 mal mit je 30 ml Essigester extrahiert, die organischen Phasen wurden 2 mal mit je 25 ml destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. So erhielt man 0.24 g (91% d. Th.) (3RS,4RS)-4-(5-Benzyloxymethyl-[1,2,3]triazol-1-yl)-3-(naphthalin-2-ylmethoxy)-piperidin-1-carbonsäure tert-butylester als hellgelbes Öl; MS: 529 $(M+H)^+$.

(b) In analoger Weise wie in Beispiel 142 (a) beschrieben, wurde aus dem (3RS,4RS)-4-(4-Hydroxymethyl-[1,2,3] triazol-1-yl)-3-(naphthalin-2-ylmethoxy)-4-piperidin-1-carbonsäure tert-butylester [Beispiel 141 (e)] der (3RS, 4RS)-4-(4-Benzyloxymethyl-[1,2,3]triazol-1-yl)-3-(naphthalin-2-ylmethoxy)-piperidin-1-carbonsäure tert-butylester als gelbes Öl erhalten; MS: 529 $(M+H)^+$.

(c) 0.22 g (0.5 mMol) (3RS,4RS)-4-(5-Hydroxymethyl-[1,2,3] triazol-1-yl)-3-(naphthalin-2-ylmethoxy)-4-piperidin-1-carbonsäure tert-butylester [Beispiel 141 (e)] und 0.13 ml (0.75 mMol) 3-Benzyloxy-propylbromid wurden unter Argon in 5 ml Dimethylformamid bei 5° C vorgelegt, dann 0.17 g (1 mMol) Kaliumiodid gefolgt von 0.044 g (1 mMol) Natriumhydrid-Dispersion (55% in Mineralöl) auf einmal zugegeben und danach während 72 Stunden bei Raumtemperatur gerührt. Der Ansatz wurde hierauf auf Eiswasser gegossen, das Produkt 3 mal mit je 30 ml Essigester extrahiert, die organischen Phasen wurden 2 mal mit je 25 ml destilliertem Wasser gewaschen, über Magnesiumsulfat getrocknet, filtriert und im Wasserstrahlvakuum eingeengt. Das so erhaltene Rohprodukt wurde an Kieselgel mit Methylenchlorid und Methanol chromatographiert. So erhielt man 0.27 g (92% d. Th.) (3RS,4RS)-4-[5-(3-Benzyloxy-propoxymethyl)-[1,2,3]triazol-1-yl]-3-(naphthalin-2-ylmethoxy)-piperidin-1-carbonsäure tert-butylester als hellgelbes Öl; MS: 586 $(M)^+$.

(d) In analoger Weise wie in Beispiel 142 (c) beschrieben, wurde aus dem (3RS,4RS)-4-(4-Hydroxymethyl [1,2,3] triazol-1-yl)-3-(naphthalin-2-ylmethoxy)-4-piperidin-1-carbonsäure tert-butylester [Beispiel 141 (e)] der (3RS, 4RS)-4-[4-(3-Benzyloxy-propoxymethyl)-[1,2,3]triazol-1-yl]-3-(naphthalin-2-ylmethoxy)-piperidin-1-carbonsäure tert-butylester als hellgelbes Öl erhalten; MS: 586 $(M)^+$.

Beispiel 143

In analoger Weise wie in Beispiel 136 (a) beschrieben, wurden durch Abspaltung der BOC-Gruppe mittels Chlorwasserstoff in Methanol die folgenden Verbindungen erhalten:

1) - Aus dem (3'RS,4'RS)-3'-(Naphthalin-2-ylmethoxy)-3',4',5',6'tetrahydro-2'H-[2,4']-bipyridin-1-carbonsäure tert-butylester das (3'RS,4'RS)-3'-(Naphthalin-2-ylmethoxy)-1',2',3',4',5',6'-hexahydro-[2,4']-bipyridin als leicht gelbes Öl; MS : 319 $(M+H)^+$;

2) - aus dem (3'RS,4'RS)-3'-(Naphthalin-2-ylmethoxy)-3',4',5',6'tetrahydro-2'H-[3,4']-bipyridin-1-carbonsäure tert-butylester das (3'RS,4'RS)-3'-(Naphthalin-2-ylmethoxy)-1',2',3',4',5',6'-hexahydro-[3,4']bipyridin als gelbes Öl; MS : 319 $(M+H)^+$.

Die als Ausgangsstoffe eingesetzten BOC-Derivate wurden wie folgt hergestellt:

(a) (α) Eine Lösung von 662 mg (2.0 mMol) 4-Trifluormethyl-sulfonyloxy-3,6-dihydro-2H-pyridin-1-carbonsäure tert-butylester [Beispiel 126 (c)], 884 mg (2.4 mMol) 2-Tributylstannylpyridin (obtained from the Maybridge Chemical Company) und 254 mg (6.0 mMol) wasserfreiem Lithiumchlorid in 30 ml absolutem DMF wurde mit Argon gespült, danach mit 115 mg (0.1 mMol) Tetrakis(triphenylphosphin)-palladium(IV) versetzt und dann 3 Stunden lang unter Argon zum Rückfluß erhitzt. Zur Aufarbeitung wurde das Reaktionsgemisch in 25 ml 10%ige Ammoniaklösung gegossen und schließlich 5 Minuten lang intensiv gerührt. Die leicht gelbe Lösung wurde mit 100 ml Methylenchorid versetzt und 5 Minuten nachgerührt. Die organische Phase wurde abgetrennt und die wäßrige Phase dreimal mit je 25 ml Methylenchlorid extrahiert. Die vereinigten organischen Phasen wurden zweimal mit je 25 ml Wasser gewaschen, über Magnesiumsulfat getrocknet und schließlich das Lösungsmittel unter verminderten Druck abdestilliert. Das Rohprodukt wurde mittels Flash-Chromatographie an Kieselgel mit einem 2:1-Gemisch von Hexan und Essigester als Eluierungsmittel gereinigt. Es wurden 354 mg (68 % d.Th) 3',6'-Dihydro-2'H-[2,4']bipyridin-1'-carbonsäure tert-butyl ester als gelbes Öl erhalten; MS : 261 $(M+H)^+$.

(β) Eine Lösung von 1.30 g (5.0 mMol) 3',6'-Dihydro-2'H-[2,4']bipyridin-1'-carbonsäure tert-butylester in 15 ml absolutem Tetrahydrofuran wurde bei 0 °C unter Argon tropfenweise mit 1.0 ml (801 mg, 10.0 mMol) Boran-Dimethylsulfidkomplex (95%ig) in Dimethylsulfid versetzt. Das Gemisch wurde zum Sieden erhitzt, so daß ein langsames Abdestillieren des Lösungmittels stattfand (ca. ein Tropfen pro Minute). Nach 3 Stunden wurden bei 0 °C tropfenweise 3 ml 2N Natriumhydroxid-Lösung und 2 ml Wasserstoffperoxidlösung (30 %ig) zugegeben. Das Gemisch wurde 6 Stunden lang bei 50 °C gerührt, dann auf Raumtemperatur abgekühlt und zur Aufarbeitung unter starkem Rühren auf ein Gemisch von 200 ml Äther, 200 ml Wasser und 25 ml Natriumpyrosulfitlösung (10%ig) gegossen. Die organische Phase wurde abgetrennt und die wäßrige Phase dreimal mit je 50 ml Äther extrahiert. Die vereinigten organischen Phasen wurden zweimal mit je 25 ml Wasser gewaschen, über Magnesiumsulfat getrocknet und schließlich das Lösungsmittel unter vermindertem Druck abdestilliert. Das Rohprodukt wurde mittels Flash-Chromatographie an Kieselgel mit einem 1:1-Gemisch von Hexan und Essigester als Eluierungsmittel gereinigt. Es wurden 354 mg (24 % d.Th) (3'RS,4'RS)-3'-Hydroxy-3',4',5',6'-tetrahydro-2'H-[2,4']bipyridin-1'-carbonsäure tert-butylester als gelbes Öl erhalten; MS : 279 $(M+H)^+$.

(γ) In analoger Weise wie im Beispiel 125 (g) beschrieben, wurde durch Alkylierung des (3'RS,4'RS)-3'-Hydroxy-3',4',5',6'-tetrahydro-2'H-[2,4']bipyridin-1'-carbonsäure tert-butylesters mit 2-Brommethyl-naphthalin der (3'RS, 4'RS)-3'-(Naphthalin-2-ylmethoxy)-3',4',5',6'tetrahydro-2'H-[2,4']-bipyridin-1-carbonsäure tert-butylester als farbloses Harz erhalten; MS : 419 $(M+H)^+$.

(b) (α) Eine Lösung von 16.7 g (106 mMol) 3-Brom-pyridin in 200 ml tert-Butylmethyläther wurde auf -75° C abgekühlt. Dazu wurde innerhalb von 45 Minuten eine Lösung von 66 ml (106 mMol) n-Butyllithium (1.6 M in Hexan) getropft und eine Stunde lang bei -75° C nachgerührt. Anschließend wurde eine Lösung von 10.0 g (52.8 mMol) 1-Benzyl-4-piperidon in 50 ml tert-Butylmethyläther bei -70° C bis -75° C tropfenweise zugegeben und danach 2 Stunden gerührt. Anschließend ließ man das Gemisch auf Raumtemperatur erwärmen. Danach wurde mit 50 ml Wasser hydrolysiert und mit 100 ml Essigester extrahiert. Die organische Phase wurde über Magnesiumsulfat getrocknet und schließlich das Lösungsmittel unter verminderten Druck eingeengt, wobei das Produkt auszufallen begann. Aus der eingedampften Mutterlauge wurden durch Kristallisation aus einem Gemisch von Essigester und Hexan weitere 1.9 g isoliert, so daß insgesamt 8.4 g (60 % d.Th.) 1'-Benzyl-2',3',5',6'-tetrahydro-1'H-[3,4']bipyridinyl-4'-ol als farbloser Festkörper erhalten wurden; MS : 268 $(M+H)^+$.

(β) Eine Dispersion von 4.52 g (16.8 mMol) 1'-Benzyl-2',3',5',6'tetrahydro-1'H-[3,4']bipyridinyl-4'-ol und 18 g (71 mMol) Kaliumdisulfat in 35 ml Dekalin wurde 30 Minuten lang bei 190 °C gerührt. Nach dem Abkühlen auf Raumtemperatur wurde das Reaktionsgemisch in Wasser gelöst und zweimal mit je 50 ml Toluol extrahiert. Anschließend wurde die wäßrige Phase mit Natronlauge alkalisch gestellt und mit Essigester extrahiert. Die Essigesterphase wurde danach über Natriumsulfat getrocknet und unter vermindertem Druck eingedampft. Zur Reinigung wurde das Rohprodukt an Kieselgel unter Verwendung eines 98:2:0.1-Gemisches von Methylenchlorid, Methanol und Ammoniak als Eluierungsmittel chromatographiert. Es wurden 4.03 g (61 % d.Th.) 1'-Benzyl-1',2',3',6'-tetrahydro-3,4'-bipyridin als gelbliches Öl erhalten; MS : 250 (M)+.

(γ) In analoger Weise wie in Beispiel 126 (f) beschrieben, wurde durch Hydroborierung des 1'-Benzyl-1',2',3',6'-tetrahydro-3,4'-bipyridins das (3'RS,4'RS)-1'-Benzyl-1',2',3',4',5',6'-hexahydro-[3,4']bipyridinyl-3'-ol als gelbliches Öl erhalten; MS : 268 (M)+. Die sich anschließende Abspaltung der Benzylgruppe mittels katalytischer Hydrierung in Gegenwart von Palladium-Kohle (10%) bei Raumtemperatur unter Normaldruck in Methanol während 18 Stunden lieferte das (3'RS,4'RS)-1',2',3',4',5',6'-Hexahydro-[3,4']bipyridin-3'-ol, das nach Umsetzung mit Di-tert-butyldicarbonat, analog Beispiel 141 (a), den (3'RS,4'RS)-3'-Hydroxy-3',4',5',6'-tetrahydro-2'H-[3,4']bipyridin-1'-carbonsäure tert-butylester als gelbliches Öl lieferte; MS : 279 $(M+H)^+$. Die darauffolgende Alkylierung mit 2-Brommethyl-naphthalin, analog Beispiel 125 (g), lieferte den (3'RS,4'RS)-3'-(Naphthalin-2-ylmethoxy)-3',4',5',6'-tetrahydro-2'H-[3,4']-bipyridin-1-carbonsäure tert-butylester, der als Rohprodukt in die Abspaltungsreaktion der BOC-Gruppe mittels Chlorwasserstoff in Methanol eingesetzt wurde.

Beispiel 144

Durch Abspaltung der BOC-Gruppe wurden die folgenden Verbindungen erhalten:

1) - Aus dem (3'RS,4'RS)-6-(3-Benzyloxy-propoxy)-3'-(naphthalin-2-ylmethoxy)-3',4',5',6'-tetrahydro-2'H-[3,4']bipyridin-1'-carbonsäure tert-butylesters mittels Zinkbromid in Methylenchlorid, analog Beispiel 125 (h), das (3'RS, 4'RS)-6-(3-Benzyloxy-propoxy)-3'-(naphthalin-2-ylmethoxy)-1',2',3',4',5',6'-hexahydro-[3,4']bipyridin als farbloser Festkörper; MS : 483 $(M+H)^+$;

2) - aus dem (3'RS,4'RS)-5-(2-Benzyloxy-äthoxymethyl)-3'-(naphthalin-2-ylmethoxy)-3',4',5',6'-tetrahydro-2'H-[2,4']bipyridin-1'carbonsäure tert-butylester mittels Chlorwasserstoff in Methanol, analog Beispiel 136 (a), das

(3'RS,4'RS)-5-(2-Benzyloxy-äthoxymethyl)-3'-(naphthalin-2-ylmethoxy)-1',2',3',4',5',6'-hexahydro-[2,4']bipyridin als leicht gelbes Öl; MS : 483 $(M+H)^+$;

Die als Ausgangsstoffe eingesetzten BOC-Derivate wurden wie folgt hergestellt:

(a) (α) Eine Lösung von 10.62 g (40.2 mMol) 2-Benzyloxy-5-brompyridin [J.Org.Chem. 60, 1408(1995)] und 10.0 ml (15.8 g, 48.2 mMol) Hexamethyldistannan 100 ml absolutem Dioxan wurde mit Argon gespült und mit 2.32 g (2.0 mMol) Tetrakis(triphenylphosphin)-palladium(IV) versetzt. Das Gemisch wurde 15 Stunden lang unter Rückfluss gekocht. Die dunkle Lösung wurde zur Aufarbeitung über Speedex filtriert und das Lösungsmittel unter vermindertem Druck abdestilliert. Der Rückstand wurde in 300 ml Methylenchlorid gelöst, zweimal mit je 100 ml Wasser gewaschen, über Magnesiumsulfat getrocknet und schließlich das Lösungsmittel unter vermindertem Druck abdestilliert. Das Rohprodukt wurde mittels Flash-Chromatographie an Kieselgel mit einem 3:1-Gemisch von Hexan und Methylenchlorid als Eluierungsmittel gereinigt. Es wurden 10.4 g (74 % d.Th) 2-Benzyloxy-5-trimethylstannyl-pyridin als farbloses Öl erhalten; MS : 349 $(M+H)^+$.

(β) In analoger Weise wie oben beschrieben wurde durch eine Palladium katalysierte Kopplung des 2-Benzyloxy-5-trimethylstannylpyridins mit 4-Trifluormethyl-sulfonyloxy-3,6-dihydro-2H-pyridin-1-carbonsäure tert-butylester [Beispiel 126 (c)] der 6-Benzyloxy-3',6'dihydro-2H-[3,4']bipyridin-1'-carbonsäure tert-butylester als farbloser Festkörper erhalten; MS : 367 $(M+H)^+$.

(γ) Eine Lösung von 0.75 g (2.04 mMol) 6-Benzyloxy-3',6'-dihydro-2H-[3,4']bipyridin-1'-carbonsäure tert-butylester in 3 ml absolutem Tetrahydrofuran wurde bei 0 °C unter Argon tropfenweise mit 0.42 ml (4.20 mMol) Boran-Dimethylsulfidkomplex (95%ig) in Dimethylsulfid versetzt. Das Gemisch wurde auf 60 °C erhitzt und gleichzeitig ein leichter Argonstrom durchgeleitet. Nach 1.5 Stunden wurden bei 0 °C tropfenweise 3 ml Tetrahydrofuran und 2 ml Wasser zugetropft. Anschließend wurden portionsweise 740 mg (4.71 mMol) Natriumpercarbonat zugegeben, das Gemisch auf Raumtemperatur erwärmt und anschließend 1 Stunde lang bei 60 °C erhitzt. Danach wurde auf Raumtemperatur abgekühlt und zur Aufarbeitung unter starkem Rühren auf ein Gemisch von 100 ml Äther, 100 ml Wasser und 10 ml 10%iger Natriumpyrosulfitlösung gegossen. Die organische Phase wurde abgetrennt und die wäßrige Phase dreimal mit je 25 ml Äther extrahiert. Die vereinigten organischen Phasen wurden zweimal mit je 10 ml Wasser gewaschen, über Magnesiumsulfat getrocknet und schließlich das Lösungsmittel unter vermindertem Druck abdestilliert. Das Rohprodukt (0.9 g) wurde mittels Flash-Chromatographie an Kieselgel mit einem 4:1-Gemisch von Methylenchlorid und Essigester als Eluierungsmittel gereinigt. Es wurden 0.630 g (80 % d.Th) eines 4:1 Gemisches des (3'RS,4'RS)-6-Benzyloxy-3'-hydroxy-3',4',5',6'tetrahydro- 2'H-[3,4']bipyridin-1'-carbonsäure tert-butylesters und 6-Benzyloxy-4'-hydroxy-3',4',5',6'-tetrahydro-2'H-[3,4']bipyridin-1'-carbonsäure tert-butylesters als farbloser Festkörper erhalten; MS : 385 $(M+H)^+$.

(δ) Eine Lösung von 256 mg (0.67 mMol) eines 4:1 Gemisches des (3'RS,4'RS)-6-Benzyloxy-3'-hydroxy-3',4',5', 6'-tetrahydro-2'H-[3,4']bipyridin-1'-carbonsäure tert-butylesters und 6-Benzyloxy-4'hydroxy-3',4',5',6'-tetrahydro-2'H-[3,4']bipyridin-1'-carbonsäure tert-butylesters in 6 ml absolutem Methanol wurde mit 40 mg Palladium-Kohle (10%ig) versetzt und 6 Stunden lang bei Normaldruck hydriert. Nach dem Abfiltrieren des Katalysators und anschließendem Abdestillieren des Lösungsmittels unter vermindertem Druck wurde das 4:1 Gemisch des (3'RS, 4'RS)-3'-Hydroxy-6-oxo-1,6,3',4',5',6'hexahydro-2'H-[3,4']bipyridin-1'-carbonsäure tert-butylesters und 4'-Hydroxy-6-oxo-1,6,3',4',5',6'-hexahydro-2'H-[3,4']bipyridin-1'carbonsäure tert-butylesters als farbloser Festkörper erhalten; MS : 295 $(M+H)^+$.

(ε) Eine Lösung von 74 mg (0.25 mMol) eines 4:1 Gemisches des (3'RS,4'RS)-3'-Hydroxy-6-oxo-1,6,3',4',5',6'-hexahydro-2'H-[3,4']bipyridin-1'-carbonsäure tert-butylesters und 4'-Hydroxy-6-oxo-1,6,3',4',5',6'-hexahydro-2'H-[3,4']bipyridin-1'-carbonsäure tert-butylesters, 99 mg (0.375 mMol) Triphenylphosphin und 52 mg (0.312 mMol) 3-Benzyloxy-1-propanol in 5 ml absolutem Tetrahydrofuran wurde portionsweise mit 69 mg (0.30 mMol) Azodicarbonsäure-di-tertbutylester versetzt und 6 Stunden bei Raumtemperatur gerührt. Anschließend wurde das Reaktionsgemisch mit 0.25 ml Methanol versetzt und zur Aufarbeitung unter starkem Rühren in 10 ml Methylenchlorid und 10 ml Wasser eingegossen. Die organische Phase wurde abgetrennt und die wässrige Phase dreimal mit je 10 ml Methylenchlorid extrahiert. Die vereinigten organischen Phasen wurden zweimal mit je 10 ml Wasser gewaschen, über Magnesiumsulfat getrocknet und schließlich das Lösungsmittel unter verminderten Druck abdestilliert. Das Rohprodukt (280 mg) wurde mittels Flash-Chromatographie an Kieselgel mit einem 4:1-Gemisch von Methylenchlorid und Essigester als Eluierungsmittel gereinigt. Es wurden 37 mg (34 % d.Th) eines 4:1 Gemisch des (3'RS, 4'RS)-6-(3-Benzyloxy-propoxy)-3'-hydroxy-3',4',5',6'-tetrahydro-2'H-[3,4']bipyridin-1'-carbonsäure tert-butylesters und 6-(3-Benzyloxy-propoxy)-4'-hydroxy-3',4',5',6'-tetrahydro-2'H-[3,4']bipyridin-1'-carbonsäure tert-butylesters als farbloses Öl erhalten; MS : 443 $(M+H)^+$.

(ζ) In analoger Weise wie im Beispiel 125 (g) beschrieben, wurde durch Alkylierung des 4:1 Gemisches des (3'RS, 4'RS)-6-(3-Benzyloxypropoxy)-3'-hydroxy-3',4',5',6'-tetrahydro-2'H-(3,4')bipyridin-1'carbonsäure tert-butylesters und 6-(3-Benzyloxy-propoxy)-4'-hydroxy-3',4',5',6'-tetrahydro-2'H-[3,4']bipyridin-1'-carbonsäure tert-butylesters mit 2-Brommethynaphthalin und anschließende Trennung der Isomeren der (3'RS,4'RS)-6-(3-Benzyloxy-propoxy)-3'-(naphthalin-2-ylmethoxy)-3',4',5',6'-tetrahydro-2'H-[3,4']bipyridin-1'-carbonsäure tert-butylester als farbloses Öl erhalten; MS : 583 $(M+H)^+$.

(b) (α) Zunächst wurde eine Lösung von 54 ml (86 mMol) Methyllithium (1.6 M in Äther) bei 0 °C in 100 absolutem Tetrahydrofuran hergestellt. Danach wurde tropfenweise innerhalb von 30 Minuten bei 0°C eine Lösung von 20 ml (96.8 mMol) Hexamethyldistannan in 100 ml absolutem Tetrahydrofuran zugegeben und 30 Minuten lang bei 0 °C nachgerührt. Die hellgelbe Lösung wurde auf -78 °C abgekühlt. Innerhalb von 45 Minuten wurde dazu bei -78 °C tropfenweise eine Lösung von 14.2 g (71.3 mMol) N-tert-butoxycarbonyl-4-piperidon in 80 ml absolutem Tetrahydrofuran gegeben. Nach 4 Stunden bei -78 °C wurden 60 ml gesättigte Kaliumnatriumtartrat-Lösung tropfenweise zugegeben und das Gemisch auf Raumtemperatur erwärmt. Die organische Phase wurde abgetrennt und die wäßrige Phase dreimal mit je 200 ml Äther extrahiert. Die vereinigten organischen Phasen wurden zweimal mit je 100 ml gesättigter Ammoniumchloridlösung und zweimal mit je 100 ml gesättigter Kochsalzlösung gewaschen, über Magnesiumsulfat getrocknet und schließlich das Lösungsmittel unter vermindertem Druck abdestilliert. Das erhaltene gelbe Öl (23.1 g), wurde in 250 ml Methylenchlorid gelöst, mit 25.6 ml (183.9 mMol) Triäthylamin versetzt und auf 0 °C abgekühlt. Es wurde innerhalb 1 Stunde bei 0 °C eine Lösung von 9.82 ml (18.4 mMol) Methansulfochlorid in 90 ml Methylenchlorid tropfenweise zugegeben und dann 1 Stunde bei 0 °C gerührt. Anschließend wurden bei 0 °C 28.3 ml (190.3 mMol) 1,8-Diazabicyclo[5.4.0]undec-7-en (DBU) innerhalb von 30 Minuten tropfenweise zugegeben. Die rote Lösung wurde 15 Stunden bei Raumtemperatur gerührt. Zur Aufarbeitung wurde das Gemisch mit 200 ml Wasser unter starkem Rühren versetzt. Die organische Phase wurde abgetrennt und die wäßrige Phase dreimal mit je 100 ml Methylenchlorid extrahiert. Die vereinigten organischen Phasen wurden zweimal mit je 100 ml gesättigter Kochsalzlösung gewaschen, über Magnesiumsulfat getrocknet und schließlich das Lösungsmittel unter verminderten Druck abdestilliert. Das Rohprodukt (21.4 g) wurde mittels Flash-Chromatographie an Kieselgel mit Methylenchlorid als Eluierungsmittel gereinigt. Es wurden 12.9 g (37.2 mMol, 52 % d.Th.) 4-Trimethylstannyl-3,6-2H-pyridin-1-carbonsäure tert-butylester als gelbes Öl erhalten; MS : 348 $(M+H)^+$.

(β) In analoger Weise wie oben beschrieben, wurde durch eine Palladium-katalysierte Kopplung des 4-Trimethylstannyl-3,6-2Hpyridin-1-carbonsäure tert-butylesters mit (rac)-2-Chlor-5-[(tetrahydro-2H-pyran-2-yloxy)methyl]-pyridin [EP 475 273] der (RS)-5-(Tetrahydropyran-2-yloxymethyl)-3',6'-dihydro-2'H-[2,4']bipyridin-1'-carbonsäure tert-butylester als gelbes Öl erhalten; MS : 375 $(M+H)^+$.

(γ) Eine Lösung von 2.56 g (6.84 mMol) (RS)-5-(Tetrahydro-pyran-2-yloxymethyl)-3',6'-dihydro-2'H-[2,4']bipyridin-1'-carbonsäure tert-butylester in 10 ml absolutem Tetrahydrofuran wurde unter Argon bei 0 °C tropfenweise mit 1.40 ml (1.12 g, 14.0 mMol) Boran-Dimethylsulfidkomplex (95%ig) in Dimethylsulfid versetzt. Das Gemisch wurde auf 50 °C erhitzt und gleichzeitig ein leichter Argonstrom durchgeleitet. Nach 45 Minuten wurden 10 ml Tetrahydrofuran zugegeben, auf 0 °C abgekühlt und portionsweise 1.58 g (21.0 mMol) Trimethylamin-N-Oxyd in fester Form zugegeben, wobei die Temperatur bei 5-10 °C gehalten wurde. Das Gemisch wurde auf Raumtemperatur erwärmt, 1 Stunde unter Rückfluß erhitzt, mit 10 ml Methanol versetzt und anschließend eine weitere Stunde unter Rückfluß erhitzt. Das Gemisch wurde auf Raumtemperatur abgekühlt und zur Aufarbeitung unter starkem Rühren auf ein Gemisch von 200 ml Methylenchlorid, 200 ml Wasser, und 10 ml 2N Natriumhydroxidlösung gegossen. Die organische Phase wurde abgetrennt und die wässrige Phase dreimal mit je 50 ml Methylenchlorid extrahiert. Die vereinigten organischen Phasen wurden zweimal mit je 50 ml Natriumpyrosulfitlösung (10%ig) (wobei der pH mittels Zugabe von 2N Natriumhydroxidlösung auf ca. 9 gestellt wurde) und zweimal mit je 25 ml Wasser gewaschen, danach über Magnesiumsulfat getrocknet und schließlich unter verminderte Druck eingedampft. Das Rohprodukt (2.59 g) wurde mittels Flash-Chromatographie an Kieselgel mit einem 1:1-Gemisch von Methylenchlorid und Essigester als Eluierungsmittel gereinigt. Es wurden 0.280 g (10 % d.Th.) eines 1:1-Gemisches des (3'RS, 4'RS)-3'-Hydroxy-5-[(RS)- und [(SR)-tetrahydro-pyran-2-yloxymethyl]-3',4',5',6'tetrahydro-2'H-[2,4']bipyridin-1'-carbonsäure tert-butylesters als leicht gelbes Öl erhalten; MS : 393 $(M+H)^+$.

(δ) In analoger Weise wie im Beispiel 125 (g) beschrieben, wurde durch Alkylierung des 1:1-Gemisches des (3'RS, 4'RS)-3'-Hydroxy-5-[(RS)- und -[(SR)-tetrahydro-pyran-2-yloxymethyl]-3',4',5',6'-tetrahydro-2'H-[2,4']bipyridin-1'-carbonsäure tert-butylesters mit 2-Brommethyl - naphthalin das 1:1 Gemisch des (3'RS,4'RS)-3'-(Naphthalin-2-yl-

methoxy)-5-[(RS)- und -[(SR)-tetrahydro-pyran-2-yloxymethyl]-3',4',5',6'-tetrahydro-2'H-[2,4']bipyridin-1-carbonsäure tert-butylesters als gelbes Öl erhalten; MS : 533 $(M+H)^+$.

(ε) Eine Lösung von 102 mg (0.19 mMol) eines 1:1 Gemisches des (3'RS,4'RS)-3'-(Naphthalin-2-ylmethoxy)-5-[(RS)- und -[(SR)-tetrahydropyran-2-yloxymethyl]-3',4',5',6'-tetrahydro-2'H-[2,4']bipyridin-1-carbonsäure tert-butylesters wurde in 2 ml Methanol gelöst und auf -15 °C abgekühlt. Es wurden innerhalb von 2 Minuten bei -10 bis -15 °C 2 ml einer 2 N Lösung von Chlorwasserstoff in Methanol tropfenweise zugegeben. Das Gemisch wurde auf Raumtemperatur erwärmt und 30 Minuten lang nachgerührt, dann zur Aufarbeitung zwischen 25 ml Essigester und 25 ml wäßriger 5%iger Natriumhydrogencarbonat-Lösung verteilt. Die organische Phase wurde abgetrennt und die wäßrige Phase dreimal mit je 10 ml Essigester extrahiert. Die vereinigten Essigesterphasen wurden über Magnesiumsulfat getrocknet und schließlich unter verminderten Druck eingedampft. Das Rohprodukt (96 mg) wurde durch Chromatographie an Kieselgel mit einem 1:1-Gemisch von Essigester und Methylenchlorid als Eluierungsmittel gereinigt. Es wurden 78 mg (92% d.Th.) (3'RS,4'RS)-5-Hydroxymethyl-3'-(naphthalin-2-ylmethoxy)-3', 4',5',6'-tetrahydro-2'H-[2,4']bipyridin-1'-carbonsäure tert-butylester als leicht gelbes Öl erhalten; MS: 449 $(M+H)^+$.

(ζ) Eine Lösung von 70 mg (0.156 mMol) (3'RS,4'RS)-5-Hydroxymethyl-3'-(naphthalin-2-ylmethoxy)-3',4' ,5',6'-tetrahydro-2'H- [2,4'] bipyridin-1'carbonsäure tert-butylester in 2 ml absolutem DMF wurde auf -10 °C abgekühlt und mit 26 µl (19 mg, 0.187 mMol) Triäthylamin versetzt. Bei -10 bis -15 °C wurden 13µl (20 mg, 0.172 mMol) Methansulfochlorid und 2 mg N,N-Dimethylaminopyridin (DMAP) zugegeben und anschließend 1 Stunde lang bei 0 °C gerührt. Zur Aufarbeitung wurde das Gemisch zwischen 25 ml Essigester und 25 ml wäßriger 5%iger Ammoniumchlorid-Lösung verteilt und die organische Phase abgetrennt. Die wäßrige Phase wurde dreimal mit je 10 ml Essigester extrahiert. Die vereinigten Essigesterphasen wurden über Magnesiumsulfat getrocknet und schließlich unter verminderten Druck eingedampft. Das Rohprodukt wurde durch Chromatographie an Kieselgel mit einem 1:1-Gemisch von Essigester und Hexan als Eluierungsmittel gereinigt. Es wurden 22 mg (35% d.Th.) (3'RS,4'RS)-5-Chlormethyl-3'-(naphthalin-2-ylmethoxy)-3',4',5',6'-tetrahydro-2'H-[2,4']bipyridin-1'carbonsäure tert-butylester als leicht gelbes Öl erhalten; MS: 467, 469 $(M+H)^+$.

(η) Eine Lösung von 22 mg (0.047 mMol) (3'RS,4'RS)-5-Chlormethyl-3'-(naphthalin-2-ylmethoxy)-3',4',5',6'-tetrahydro-2'H-[2,4']bipyridin-1'carbonsäure tert-butylester in 0.5 ml DMF wurde mit 67 µl (72 mg, 0.47 mMol) 2-Benzyloxyäthanol und 19 mg (0.47 mMol) Natriumhydrid (60%ige Dispersion in Öl) versetzt und anschließend 2 Stunden bei Raumtemperatur gerührt. Das Gemisch wurde zur Aufarbeitung zwischen 15 ml Essigester und 15 ml wäßriger 5%iger Ammoniumchlorid-Lösung verteilt, dann die organische Phase abgetrennt. Die wäßrige Phase wurde dreimal mit je 5 ml Essigester extrahiert. Die vereinigten Essigesterphasen wurden über Magnesiumsulfat getrocknet und schließlich das Lösungsmittel unter verminderten Druck abdestilliert. Der rohe (3'RS,4'RS)-5-(2-Benzyloxy-äthoxymethyl)-3'-(naphthalin-2-ylmethoxy)-3',4',5',6'-tetrahydro-2'H-[2,4']bipyridin-1'carbonsäure tert-butylester wurde ohne weitere Reinigung und Charakterisierung in der nächsten Stufe eingesetzt.

Beispiel 145

(a) Eine Lösung von 10.8 g (54.3 mMol) (1RS,6SR)-7-Oxa-3-azabicyclo[4.1.0]heptan-3-carbonsäure tert-butylester [Beispiel 141 (b)] in 250 ml Acetonitril wurde mit 7.98 g (162.9 mMol) pulverisiertem Natriumcyanid und 17.3 g (162.9 mMol) Lithiumperchlorat versetzt und das Reaktionsgemisch unter Argon 24 Stunden lang bei 95° C gerührt. Zur Aufarbeitung wurde die bräunliche Lösung abgekühlt, mit 150 ml Essigester versetzt und über Decalit filtriert. Das Filtrat wurde mit 100 ml Wasser gewaschen und die Phasen getrennt. Die wäßrige Phase wurde auf pH 5 gestellt und dreimal mit je 60 ml Essigester ausgeschüttelt. Die vereinigten organischen Phasen wurden über Natriumsulfat getrocknet und unter vermindertem Druck eingedampft. Der Rückstand wurde zur Reinigung an Kieselgel unter Verwendung eines 4:1-Gemisches von Hexan und Essigester als Eluierungsmittel chromatographiert. Man erhielt 9.9 g (80.5% d.Th.) eines 4:1-Gemisches von (3RS,4RS)-4-Cyano-3-hydroxy-piperidin-1-carbonsäure tert-butylester und (3RS,4SR)-3-Cyano-4-hydroxypiperidin-1-carbonsäure tert-butylester in Form farbloser Kristalle; MS : 227 $(M+H)^+$.

(b) Eine Lösung von 5.8 g (25.6 mMol) eines 4:1-Gemisches von (3RS,4RS)-4-Cyano-3-hydroxy-piperidin-1-carbonsäure tert-butylester und (3RS,4SR)-3-Cyano-4-hydroxy-piperidin-1-carbonsäure tert-butylester in 50 ml N, N-Dimethylformamid wurde mit 1.8 g (38.4 mMol) Natriumhydrogensulfid Monohydrat und 2.05 g (38.4 mMol) Ammoniumchlorid 4 Stunden lang bei Raumtemperatur unter Argon gerührt. Zur Aufarbeitung wurde das Reaktionsgemisch unter vermindertem Druck eingedampft und der Rückstand in 150 ml Methylenchlorid aufgenommen.

Die erhaltene Lösung wurde zweimal mit je 10 ml Wasser gewaschen, die organische Phase abgetrennt, über Natriumsulfat getrocknet und dann unter vermindertem Druck eingedampft. Der Rückstand wurde zur Reinigung und Trennung des Isomerengemisches an Kieselgel unter Verwendung eines 95:5:0.1-Gemisches von Methylenchlorid, Methanol und Ammoniak als Eluierungsmittel chromatographiert. Man erhielt 3.48 g (3RS,4SR)-3-Hydroxy-4-thiocarbamoyl-piperidin-1-carbonsäure tert-butylester (52 % d.Th.), (Rf: 0.37, Kieselgel; Methylenchlorid:Methanol:Ammoniak = 90:10:0.1 v/v/v), MS: 260 $(M)^+$, sowie 1.2 g eines Gemisches von (3RS,4SR)-3-Hydroxy-4-thiocarbamoyl-piperidin-1-carbonsäure tert-butylester und (3RS,4RS)-4-Hydroxy-3-thiocarbamoyl-piperidin-1-carbonsäure tert-butylester jeweils als farbloses Öl.

(c) Zu einer Lösung von 1.88g (7.22 mMol) (3RS,4SR)-3-Hydroxy-4-thiocarbamoyl-piperidin-1-carbonsäure tert-butylester in 5 ml Aceton wurden bei Raumtemperatur 4 ml Methyljodid gegeben. Nach 14 Stunden Rühren bei Raumtemperatur war das Produkt ausgefallen. Es wurden 10 ml Äther zugegeben und das Reaktionsgemisch 30 Minuten lang nachgerührt. Nach dem Abfiltrieren und Trocknen wurden 2.76 g (3RS,4SR)-[Amino-(1-tert-butoxycarbonyl-3-hydroxy-piperidin-4-yl)-methylen]-methyl-sulfonium Iodid (98% d.Th.) als farblose Kristalle erhalten; Rf : 0.22 (Kieselgel; Methylenchlorid:Methanol:Ammoniak = 95:5:0.1 v/v/v).

(d) Eine Lösung von 2.76 g (7.09 mMol) (3RS,4SR)-[Amino-(1-tertbutoxycarbonyl-3-hydroxy-piperidin-4-yl)-methylen]-methyl-sulfonium Iodid in 15 ml Methanol wurde mit 0.41 g (3.55 mMol) Ammoniumcarbonat versetzt und 18 Stunden lang bei Raumtemperatur gerührt. Zur Aufarbeitung wurde das Reaktionsgemisch unter vermindertem Druck eingedampft. Es wurden 2.5 g (95 % d.Th.) (3RS,4RS)-4-Carbamimidoyl-3-hydroxy-piperidin-1-carbonsäure tert-butylester Iodid als farbloser Schaum erhalten.

Eine Lösung von 715 mg (1.92 mMol) (3RS,4RS)-4-Carbamimidoyl-3-hydroxy-piperidin-1-carbonsäure tert-butylester Iodid in 20 ml Methanol wurde mit 321 mg (1.92 mMol) Silberacetat bei Raumtemperatur eine Stunde lang gerührt. Das ausgefallene Silberjodid wurde abfiltriert und mit 20 ml Methanol gewaschen. Das erhaltene leichtgelbe Filtrat wurde unter vermindertem Druck eingedampft. Man erhielt 538 mg (92% d.Th.) (3RS,4RS)-4-Carbamimidoyl-3-hydroxy-piperidin-1-carbonsäure tert-butylester Acetat als farblosen Schaum.

(e) Eine Lösung von 372 mg (1 mMol) (3RS,4RS)-4-Carbamimidoyl-3-hydroxy-piperidin-1-carbonsäure tert-butylester Acetat in 10 ml Methanol wurde mit 1 ml 1N Natriummethylat-Lösung versetzt und bei Raumtemperatur gerührt. Anschließend wurden 205 mg (1 mMol) 2-Benzyloxy-3-dimethylamino-acrolein (EPA 0477 901) dazu gegeben und die Lösung 18 Stunden lang zum Rückfluß erhitzt. Zur Aufarbeitung wurde das Reaktionsgemisch unter vermindertem Druck eingedampft, der Rückstand in 20 ml Methylenchlorid aufgenommen und mit 5 ml Wasser gewaschen. Die organische Phase wurde abgetrennt, über Natriumsulfat getrocknet und schließlich unter vermindertem Druck eingedampft. Der Rückstand wurde zur Reinigung an Kieselgel unter Verwendung eines 1:4-Gemisches von Methylenchlorid und Essigester als Eluierungsmittel chromatographiert. Man erhielt 106 mg (27 % d. Th.) (3RS,4RS)-4-(5-Benzyloxy-pyrimidin-2-yl)-3-hydroxy-piperidin-1-carbonsäure tert-butylester als farbloses Öl; MS : 386 $(M+H)^+$.

(f) Eine Lösung von 110mg (0.29 mMol) (3RS,4RS)-4-(5-Benzyloxypyrimidin-2-yl)-3-hydroxy-piperidin-1-carbonsäure tert-butylester in 5 ml Methanol wurde mit 20 mg Palladium(5 %ig)-Kohle versetzt und bei Raumtemperatur während 12 Stunden hydriert. Zur Aufarbeitung wurde der Katalysator abfiltriert und mit 20 ml Methanol gewaschen. Die Methanollösung wurde unter vermindertem Druck eingedampft und das resultierende Öl durch Zugabe von Äther zum Kristallisieren gebracht. Man erhielt 80 mg (93% d.Th.) (3RS,4RS)-3-Hydroxy-4-(5-hydroxy-pyrimidin-2-yl)-piperidin-1-carbonsäure tert-butylester in Form farbloser, leicht zerfließender Kristalle; MS : 296 $(M+H)^+$.

(g) Ein Gemisch von 65 mg (0.22 mMol) (3RS,4RS)-3-Hydroxy-4-(5-hydroxy-pyrimidin-2-yl)-piperidin-1-carbonsäure tert-butylester, 90mg (0.66 mMol) Kaliumcarbonat und 151.2 mg (0.66 mMol) (3-Brompropoxymethyl)-benzol in 10 ml Methyläthylketon wurde bei 80 °C 48 Stunden lang unter Argon gerührt. Zur Aufarbeitung wurde das Reaktionsgemisch unter vermindertem Druck eingedampft, der Rückstand in Methylenchlorid aufgenommen und die Lösung direkt an Kieselgel unter Verwendung eines 1 : 4-Gemisches von Methylenchlorid und Essigester als Eluierungsmittel chromatographiert. Man erhielt 40 mg (41% d.Th.) (3RS,4RS)-4-[5-(3-Benzyloxy-propoxy)-pyrimidin-2-yl]-3-hydroxy-piperidin-1-carbonsäure tert-butylester als Schaum; Rf: 0.43 (Kieselgel; Methylenchlorid:Essigester = 1:4 v/v).

h) Eine Lösung von 40 mg (0.09 mMol) (3RS,4RS)-4-[5-(3-Benzyloxypropoxy)-pyrimidin-2-yl]-3-hydroxy-piperidin-1-carbonsäure tert-butylester und 23 mg (0.1 mMol) 2-Brommethyl-naphthalin in 5 ml N,N-Dimethylformamid wurde mit 5 mg (0.1mMol) Natriumhyrid (50 %ige Dispersion in Öl) versetzt und 18 Stunden bei Raumtemperatur gerührt. Zur Aufarbeitung wurde das Reaktiongemisch unter vermindertem Druck eingedampft, der erhaltene Rückstand in 3 ml Methylenchlorid aufgenommen und die Lösung direkt an Kieselgel unter Verwendung eines 2: 1-Gemisches von Hexan und Essigester als Eluierungsmittel chromatographiert. Man erhielt 40 mg (76% d.Th.) (3RS,4RS)-4-[5-(3-Benzyloxy-propoxy)-pyrimidin-2-yl]-3-(naphthalin-2-ylmethoxy)-piperidin-1-carbonsäure tert-butylester als Öl; MS : 584 $(M+H)^+$.

(i) Eine Lösung von 40 mg (0.07 mMol) (3RS,4RS)-4-[5-(3-Benzyloxypropoxy)-pyrimidin-2-yl]-3-(naphthalin-2-ylmethoxy)-piperidin-1-carbonsäure tert-butylester und 0.1 ml Trifluoressigsäure in 1 ml Methylenchlorid wurde 2 Stunden lang bei Raumtemperatur gerührt. Danach wurde die Lösung eingedampft, der Rückstand in 1 ml Essigester aufgenommen und durch Zugabe von Hexan zum Kristallisieren gebracht. Man erhielt 25 mg (60% d.Th.) (3RS,4RS)-5-(3-Benzyloxy-propoxy)-2-[3-(naphthalin-2-ylmethoxy)-piperidin-4-yl]-pyrimidin Trifluoracetat in Form farbloser Kristalle; MS: 484 $(M+H)^+$.

Beispiel 146

(a) In analoger Weise wie in Beispiel 145 (a) beschrieben, wurde durch Epoxidöffnung mittels 4-Benzyloxy-2(1H)-pyridon [Chem. Pharm. Bull. 22, 763 - 770 (1974)] aus dem (1RS,6SR)-7-Oxa-3-aza-bicyclo[4.1.0]heptan-3-carbonsäure tert-butylester [Beispiel 141 (b)] das 1:1-Gemisch des (3'RS,4'RS)-4-Benzyloxy-4'-hydroxy-2-oxo-3',4',5',6'-tetrahydro-2H-2'H-[1,3']bipyridin-1'-carbonsäure tert-butylesters und (3'RS,4'RS)-4-Benzyloxy-3'-hydroxy-2-oxo-3',4',5',6'-tetrahydro-2H-2'H-[1,4']bipyridin-1'-carbonsäure tert-butylesters als farblose Kristalle erhalten; MS : 401 $(M+H)^+$.

(b) In analoger Weise wie in Beispiel 145 (f) beschrieben, wurde aus dem 1:1-Gemisch des (3'RS,4'RS)-4-Benzyloxy-4'-hydroxy-2-oxo-3',4',5',6'tetrahydro-2H-2'H-[1,3']bipyridin-1'-carbonsäure tert-butylesters und (3'RS, 4'RS)-4-Benzyloxy-3'-hydroxy-2-oxo-3',4',5',6'-tetrahydro-2H-2'H-[1,4']bipyridin-1'-carbonsäure tert-butylesters mittels katalytischer Hydrierung das 1:1-Gemisch des (3'RS,4'RS)-4,3'-Dihydroxy-2-oxo-3',4',5',6'-tetrahydro-2H, 2'H-(1,4']bipyridin-1'-carbonsäure tert-butylesters und (3'RS,4'RS)-4,4'-Dihydroxy-2-oxo-3',4',5',6'-tetrahydro-2H, 2'H-[1,3']bipyridin-1'-carbonsäure tert-butylesters als farbloser Schaum erhalten; MS: 311 $(M+H)^+$.

(c) In analoger Weise wie in Beispiel 145 (g) beschrieben, wurde aus dem 1:1-Gemisch des (3'RS,4'RS)-4,3'-Dihydroxy-2-oxo-3',4',5',6'tetrahydro-2H,2'H-[1,4']bipyridin-1'-carbonsäure tert-butylesters und (3'RS,4'RS)-4,4'-Dihydroxy-2-oxo-3',4',5',6'-tetrahydro-2H,2'H-[1,3']bipyridin-1'-carbonsäure tert-butylesters durch Umsetzung mit (3-Brom-propoxymethyl)-benzol in Gegenwart von Kaliumcarbonat das Gemisch des (3'RS,4'RS)-4-(3-Benzyloxy-propoxy)-3'-hydroxy-2-oxo-3',4',5',6'-tetrahydro-2H,2'H-[1,4']bipyridin-1'-carbonsäure tert-butylesters und (3'RS, 4'RS)-4-(3-Benzyloxy-propoxy)-4'-hydroxy-2-oxo-3',4',5',6'-tetrahydro-2H,2'H-[1,3']bipyridin-1'-carbonsäure tert-butylesters als farbloses Öl erhalten; MS : 459 $(M+H)^+$.

(d) In analoger Weise wie in Beispiel 145 (h) beschrieben, wurden aus dem Gemisch des (3'RS,4'RS)-4-(3-Benzyloxy-propoxy)-3'-hydroxy-2-oxo-3',4',5',6'-tetrahydro-2H,2'H-[1,4']bipyridin-1'-carbonsäure tert-butylesters und (3'RS,4'RS)-4-(3-Benzyloxy-propoxy)-4'-hydroxy-2-oxo-3',4',5',6'-tetrahydro-2H,2'H-[1,3']bipyridin-1'-carbonsäure tert-butylesters mittels Alkylierung durch 2-Brommethyl-naphthalin und nach chromatographischer Trennung der beiden Isomeren an Kieselgel unter Verwendung eines 1:4-Gemisches von Methylenchlorid und Essigester als Eluierungsmittel der (3'RS,4'RS)-4-(3-Benzyloxypropoxy)-4'-(naphthalin-2-ylmethoxy)-2-oxo-3',4',5',6'-tetrahydro-2H,2'H-[1,3']bipyridin-1'-carbonsäure tert-butylester, (Rf: 0.64, $SiO_2$; Methylenchlorid:Essigester = 1:4 v/v), und der (3'RS,4'RS)-4-(3-Benzyloxy-propoxy)-3'-(naphthalin-2-ylmethoxy)-2-oxo-3',4',5',6'tetrahydro-2H, 2'H-[1,4']bipyridin-1'-carbonsäure tert-butylester MS: 599 $(M+H)^+$, (Rf: 0.44, $SiO_2$; Methylenchlorid:Essigester = 1:4 v/v) jeweils als gelbliches Öl erhalten.(e) In analoger Weise wie in Beispiel 145 (i) beschrieben, wurde aus dem (3'RS,4'RS)-4-(3-Benzyloxy-propoxy)-3'-(naphthalin-2-ylmethoxy)-2-oxo-3',4',5',6'-tetrahydro-2H,2'H-[1,4'] bipyridin-1'-carbonsäure tert-butylester durch Abspaltung der BOC-Gruppe mittels Trifluoressigsäure das (3'RS, 4'RS)-4-(3-Benzyloxypropoxy)-3'-(naphthalin-2-ylmethoxy)-1',2',3',4',5',6'-hexahydro-[1,4']bipyridin-2-on Trifluoracetat als farbloser Festkörper erhalten; MS: 499 $(M+H)^+$.

Beispiel 147

In analoger Weise wie in Beispiel 22 (l) beschrieben, wurden durch Abspaltung der BOC-Gruppe mittels

Chlorwasserstoff in Methanol die folgenden Verbindungen erhalten:

1) - Aus dem (1RS,2RS,3R5,5SR)-3-[4-(2-Benzyloxy-äthoxymethyl)-phenyl] -2-(naphthalin-2-ylmethoxy)-8-aza-bicyclo (3.2.1] octan-8-carbonsäure tert-butylester das (1RS,2RS,3RS,5SR)-3-[4-(2-Benzyloxy-äthoxymethyl)-phenyl]-2-(naphthalin-2-yl-methoxy)-8-aza-bicyclo[3.2.1]-octan als farbloses Öl; $R_f$ : 0.15 (Kieselgel; Methylenchlorid:Methanol: Ammoniak=95:5:0.1);

2) - aus dem (1RS,2RS,3RS,5SR)-2-(Naphthalin-2-ylmethoxy)-3-[4-(2-phenoxy-äthoxymethyl)-phenyl]-8-aza-bicyclo[3.2.1]octan-8-carbonsäure tert-butylester das (1RS,2RS,3RS,5SR)-2-(Naphthalin-2-ylmethoxy)-3-[4-(2-phenoxy-äthoxymethyl)-phenyl]-8-aza-bicyclo[3.2.1]octan als gelbes Öl; $R_f$: 0.21 (Kieselgel; Methylenchlorid:Methanol:Ammoniak=95:5:0.1);

3) - aus dem (1RS,2RS,3RS,5SR)-3-(4-Benzyloxymethyl-phenyl)-2-(naphthalin-2-ylmethoxy)-8-aza-bicyclo [3.2.1]octan-8-carbonsäure tert-butylester +BOC das (1RS,2RS,3RS,5SR)-3-(4-Benzyloxymethyl-phenyl)-2-(naphthalin-2-ylmethoxy)-8-aza-bicyclo[3.2.1]octan Hydrochlorid als farbloses Öl; MS : 464 $(M+H)^+$;

4) - aus dem (1RS,2RS,3RS,5SR)-2-(Naphthalin-2-ylmethoxy)-3-(4-phenylsulfanylmethyl-phenyl)-8-aza-bicyclo [3.2.1]octan-8-carbonsäure tert-butylester das (1RS,2RS,3RS,5SR)-2-(Naphthalin-2-ylmethoxy)-3-(4-phenylsulfanylmethyl-phenyl)-8-aza-bicyclo[3.2.1]octan Hydrochlorid als farbloses Öl; MS : 566 $(M+H)^+$;

5) - aus dem (1RS,2RS,3RS,5SR)-3-[4-(2-Chlor-benzoyloxymethyl)-phenyl] -2-(naphthalin-2-ylmethoxy)-8-aza-bicyclo [3.2.1] octan-8-carbonsäure tert-butylester +BOC der 2-Chlor-benzoesäure (1RS,2RS,3RS,5SR)-4-[2-(naphthalin-2-ylmethoxy)-8-azabicyclo[3.2.1]oct-3-yl]-benzylester als gelblicher Schaum; MS : 512 $(M+H)^+$;

Die als Ausgangsverbindungen eingesetzten BOC-Derivate wurden wie folgt hergestellt:

(a) In analoger Weise wie in Beispiel 1 (g) beschrieben, wurde durch Alkylierung des (1RS,2RS,3RS,5SR)-3-(4-Hydroxymethyl-phenyl)-2-(naphthalin-2-ylmethoxy)-8-aza-bicyclo[3.2.1]octan-8-carbonsäure tert-butylesters [Beispiel 86 (eee)] mit 2-(Benzyloxy)-äthyliodid [Helv.Chim.Acta Vol.71, (1988), 2039] der (1RS,2RS,3RS,5SR)-3-[4-(2-Benzyloxy-äthoxymethyl)-phenyl]-2-(naphthalin-2-ylmethoxy)-8-azabicyclo[3.2.1]octan-8-carbonsäure tert-butylester als farbloses Öl erhalten; MS : 625 $(M+NH4)^+$.

(b) In analoger Weise wie in Beispiel 1 (g) beschrieben, wurde durch Alkylierung des (1RS,2RS,3RS,5SR)-3-(4-Hydroxymethyl-phenyl)-2-(naphthalin-2-ylmethoxy)-8-aza-bicyclo[3.2.1]octan-8-carbonsäure tert-butylesters [Beispiel 86 (eee)] mit Phenoxy-äthylbromid der (1RS,2RS,3RS,5SR)-2-(Naphthalin-2-ylmethoxy)-3-[4-(2-phenoxy-äthoxymethyl)-phenyl]-8-aza-bicyclo[3.2.1]octan-8-carbonsäure tert-butylester als farbloses Öl erhalten; MS : 611 $(M+NH_4)^+$.

(c) In analoger Weise wie in Beispiel 1 (g) beschrieben, wurde durch Alkylierung des (1RS,2RS,3RS,5SR)-3-(4-Hydroxymethyl-phenyl)-2-(naphthalin-2-ylmethoxy)-8-aza-bicyclo[3.2.1]octan-8-carbonsäure tert-butylesters [Beispiel 86 (eee)] mit Benzylbromid der (1RS,2RS,3RS,5SR)-3-(4-Benzyloxymethyl-phenyl)-2-(naphthalin-2-ylmethoxy)-8-azabicyclo[3.2.1]octan-8-carbonsäure tert-butylester +BOC erhalten, der als Rohprodukt in die Reaktion zur BOC-Abspaltung eingesetzt wurde.

(d) In analoger Weise wie in Beispiel 33 (a) beschrieben, wurde durch Umsetzung des (1RS,2RS,3RS,5SR)-3-(4-Hydroxymethyl-phenyl)-2-(naphthalin-2-ylmethoxy)-8-aza-bicyclo[3.2.1]octan-8-carbonsäure tert-butylesters [Beispiel 86 (eee)] mit Diphenyldisulfid in Gegenwart von Tributylphosphin der (1RS,2RS,3RS,5SR)-2-(Naphthalin-2-ylmethoxy)-3-(4-phenylsulfanylmethyl-phenyl)-8-aza-bicyclo[3.2.1]octan-8-carbonsäure tert-butylester als farbloses Öl erhalten; MS : 566 $(M+H)^+$.

(e) In analoger Weise wie in Beispiel 22 (k) beschrieben, wurde durch Veresterung des (1RS,2RS,3RS,5SR)-3-(4-Hydroxymethyl-phenyl)-2-(naphthalin-2-ylmethoxy)-8-aza-bicyclo[3.2.1]octan-8-carbonsäure tert-butylesters [Beispiel 86 (eee)] mit 2-Chlor-benzoylchlorid der 2-Chlor-benzoesäure (1RS,2RS,3RS,5SR)-4-[2-(naphthalin-2-ylmethoxy)-8-azabicyclo[3.2.1]oct-3-yl]-benzylester als farbloses Ö erhalten, der als Rohprodukt in die Reaktion zur BOC-Abspaltung eingesetzt wurde.

Beispiel 148

In analoger Weise wie in Beispiel 22 (l) beschrieben, wurde aus dem (3SR,4RS,5RS)-4-[4-(3-Benzyloxy-propoxy)-phenyl]-3-methoxymethyl-5-(naphthalin-2-ylmethoxy)-piperidin-1-carbonsäure tert-butylester durch Abspaltung der BOC-Gruppe mittels Chlorwasserstoff in Methanol das (3SR,4RS,5RS)-4-[4-(3-Benzyloxy-propoxy)-phenyl]-3-methoxymethyl-5-(naphthalin-2-ylmethoxy)-piperidin als amorpher, farbloser Festkörper erhalten; MS : 526 $(M+H)^+$.

Der als Ausgangsstoff eingesetzte (3SR,4RS,5RS)-4-[4-(3-Benzyloxypropoxy)-phenyl]-3-methoxymethyl-5-(naphthalin-2-ylmethoxy)-piperidin-1-carbonsäure tert-butylester wurde wie folgt synthetisiert:

(a) In analoger Weise wie in Beispiel 68 (a)-(b) beschrieben, wurde zunächst die primäre Hydroxyfunktion des (3RS,4RS)- und (3SR,4RS)-1-Benzyl-3-hydroxymethyl-piperidin-4-ols [E.Jaeger und J.H.Biel, J.Org.Chem. 30 (3), 740-744 (1965)] geschützt, indem an Stelle des tert-Butyldiphenylchlorsilans mit Triphenylchlormethan, analog Beispiel 22 (h), in Pyridin eingesetzt wurde und so das (3RS,4RS)- und (3SR,4RS)-1-Benzyl-3-trityloxymethyl-piperidin-4-ol erhalten wurde. Die darauffolgende Oxidation mit Oxalylchlorid in Dimethylsulfoxid lieferte das (RS)-1-Benzyl-3-trityloxymethyl-piperidin-4-on als farblosen Schaum; MS : 462 $(M+H)^+$. Die sich anschließende Umsetzung mit 4-Iodanisol, analog Beispiel 62 (b), lieferte das Gemisch des (3RS,4RS)-und (3RS,4SR)-1-Benzyl-4-(4-methoxy-phenyl)-3-trityloxymethylpiperidin-4-ols als farblosen Festkörper; MS : 570 $(M+H)^+$.

(b) Zu einer Lösung von 8.36 g (14.6 mMol) des Gemisches des (3RS,4RS)- und (3RS,4SR)-1-Benzyl-4-(4-methoxy-phenyl)-3-trityloxymethyl-piperidin-4-ols in 20 ml trockenem Pyridin wurde innerhalb von 20 Minuten eine Lösung von 5.5 ml (58.7 mMol) Phosphoroxychlorid in 20 ml trockenem Pyridin getropft. Das Reaktionsgemisch wurde 20 Stunden lang bei 60 °C gerührt. Das dunkelrote Reaktionsgemisch wurde abgekühlt und unter vermindertem Druck eingedampft. Der Rückstand wurde mit Methylenchlorid aufgenommen und mit gesättigter Natriumcarbonat-Lösung versetzt. Die organische Phase wurde abgetrennt, über Natriumsulfat getrocknet und unter vermindertem Druck eingedampft. Der Rückstand wurde an Kieselgel unter Verwendung eines 20:1-Gemisches von Toluol und Essigester als Eluierungsmittel chromatographiert. Es wurden 6.1 g (75 % d.Th.) (RS)-1-Benzyl-4-(4-methoxy-phenyl)-3-trityloxymethyl-1,2,3,6-tetrahydro-pyridin als farbloser Festkörper erhalten; MS : 552 $(M+H)^+$.

(c) In analoger Weise wie in Beispiel 62 (d) beschrieben, wurde durch Hydroborierung des (RS)-1-Benzyl-4-(4-methoxy-phenyl)-3-trityloxymethyl-1,2,3,6-tetrahydro-pyridins mit Boran-Tetrahydrofuran und anschließende Oxidation mittels Natriumpercarbonat das (3RS,4RS,5SR)-1-Benzyl-4-(4-methoxy-phenyl)-5-trityloxymethylpiperidin-3-ol als farbloser Schaum erhalten; MS : 570 $(M+H)^+$.

(d) In analoger Weise wie in Beispiel 44 (d) beschrieben, wurde durch gleichzeitige Spaltung der Methoxy- und Trityloxygruppe mittels Bortribromid in Methylenchlorid aus dem (3RS,4RS,5SR)-1-Benzyl-4-(4-methoxy-phenyl)-5-trityloxymethyl-piperidin-3-ol das (3RS,4RS,5SR)-1-Benzyl-5-hydroxymethyl-4-(4-hydroxy-phenyl)-piperidin-3-ol Hydrobromid als weißer Festkörper erhalten; MS : 314 $(M+H)^+$.

(e) In analoger Weise wie in Beispiel 2 (e) beschrieben, wurde durch katalytische Hydrierung bei Atmosphärendruck unter Verwendung eines Palladium(10%ig)-Kohle-Katalysators in Methanol aus dem (3RS,4RS,5SR)-1-Benzyl-5-hydroxymethyl-4-(4-hydroxy-phenyl)-piperidin-3-ol Hydrobromid das (3RS,4RS,5SR)-5-Hydroxymethyl-4-(4-hydroxy-phenyl)-piperidin-3-ol Hydrobromid als farbloser Schaum erhalten; MS : 224 $(M+H)^+$.

(f) In analoger Weise wie in Beispiel 1 (f) beschrieben, wurde durch Umsetzung des (3RS,4RS,5SR)-5-Hydroxymethyl-4-(4-hydroxy-phenyl)-piperidin-3-ol Hydrobromids mit Di-tert-butyl-dicarbonat der (3RS,4RS,5SR)-3-Hydroxy-5-hydroxymethyl-4-(4-hydroxy-phenyl)-piperidin-1-carbonsäure tert-butylester als farbloser Schaum erhalten.

(g) In analoger Weise wie in Beispiel 44 (e) beschrieben, wurde durch Alkylierung des (3RS,4RS,5SR)-3-Hydroxy-5-hydroxymethyl-4-(4-hydroxy-phenyl)-piperidin-1-carbobsäure tert-butylesters mit Benzyl 3-brompropyläther in Gegenwart von Kaliumcarbonat in Butan-2-on der (3RS,4RS,5SR)-4-[4-(3-Benzyloxy-propoxy)-phenyl]-3-hydroxy-5-hydroxymethyl-piperidin-1-carbonsäure tert-butylester als farbloses Öl erhalten; MS : 472 $(M+H)^+$.

(h) In analoger Weise wie in Beispiel 22 (h) beschrieben, wurde durch Umsetzung des (3RS,4RS,5SR)-4-[4-(3-Benzyloxy-propoxy)-phenyl]-3-hydroxy-5-hydroxymethyl-piperidin-1-carbonsäure tert-butylesters mit Triphenylchlormethan in Pyridin der (3RS,4RS,5SR)-4-[4-(3-Benzyloxypropoxy)-phenyl]-3-hydroxy-5-trityloxymethyl-piperidin-1-carbonsäure tert-butylester [MS : 731 $(M+NH_4)^+$], als farbloser Schaum erhalten, dessen Alkylierung mit 2-Brommethyl-naphthalin, analog Beispiel 62 (h), den (3RS,4RS,5SR)-4-[4-(3-Benzyloxy-propoxy)-phenyl]-3-(naphthalin-2-ylmethoxy)-5-trityloxymethyl-piperidin-1-carbonsäure tert-butylester [MS : 871(M+$NH_4$)$^+$], als farblosen Schaum ergab. Die sich anschließende selektive Abspaltung der Trityl-Gruppe mittels eines Gemisches aus Trifluoressigsäure und Trifluoressigsäureanhydrid in Methylenchlorid, analog Beispiel 86 (u), lieferte den (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylester als farblosen Festkörper; MS : 612 (M+H)$^+$. Die darauffolgende Alkylierung mit Methyliodid, analog Beispiel 62 (h) ergab den (3SR,4RS,5RS)-4-[4-(3-Benzyloxy-propoxy)-phenyl]-3-methoxymethyl-5-(naphthalin-2-ylmethoxy)-piperidin-1-carbonsäure tert-butylester als farblosen Festkörper; MS : 626 (M+H)$^+$.

Beispiel 149

In analoger Weise wie in Beispiel 22 (l) beschrieben, wurden durch Abspaltung der BOC-Gruppe mittels Chlorwasserstoff in Methanol die folgenden Verbindungen erhalten:

1) - Aus dem (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylester0 [Beispiel 148 (h)] das (3SR,4RS,5RS)-[4-[4-(3-Benzyloxypropoxy)-phenyl]-5-(naphthalin-2-ylmethoxy)-piperidin-3-yl]-methanol Hydrochlorid als farbloser Festkörper; MS : 512 (M+H)$^+$;

2) - aus dem (3RS,4RS,5SR)-4-[4-(3-Benzyloxy-propoxy)-phenyl]-3-(naphthalin-2-ylmethoxy)-5-(pyridin-4-ylsulfanylmethyl)-piperidin-1-carbonsäure tert-butylester das (3SR,4RS,5RS)-4-[4-[4-(3-Benzyloxypropoxy)-phenyl]-5-(naphthalin-2-ylmethoxy)-piperidin-3-ylmethylsulfanyl]-pyridin Hydrochlorid als farbloser Festkörper; MS : 605 (M+H)$^+$;

3) - aus dem (3RS,4RS,5SR)-4-[4-(3-Benzyloxy-propoxy)-phenyl]-3-(naphthalin-2-ylmethoxy)-5-phenylsulfanylmethyl-piperidin-1-carbonsäure tert-butylester das (3RS,4RS,5SR)-4-[4-(3-Benzyloxypropoxy)-phenyl]-3-(naphthalin-2-ylmethoxy)-5-phenylsulfanylmethylpiperidin Hydrochlorid als farbloses Öl; MS : 604 (M+H)$^+$;

4) - aus dem (3SR,4RS,5RS)-4-[4-(3-Benzyloxy-propoxy)-phenyl]-3-(2-morpholin-4-yl-äthoxymethyl)-5-(naphthalin-2-ylmethoxy)-piperidin-1-carbonsäure tert-butylester das (3SR,4RS,5RS)-4-[2-[4-[4-(3-Benzyloxypropoxy)-phenyl]-5-(naphthalin-2-ylmethoxy)-piperidin-3-ylmethoxy]-äthyl]-morpholin als farbloses Öl; MS : 625 (M+H)$^+$;

5) - aus dem (3RS,4RS,5RS)-4-[4-(3-Benzyloxy-propoxy)-phenyl]-3-diethylaminomethyl-5-(naphthalin-2-ylmethoxy)-piperidin-1-carbonsäure tert-butylester das (3SR,4RS,5RS)-[4-[4-(3-Benzyloxypropoxy)-phenyl]-5-(naphthalin-2-ylmethoxy)-piperidin-3-ylmethyl]-diethyl-amin als gelbliches Öl; MS : 567 (M+H)$^+$;

6) - aus dem (3RS,4RS,5RS)-4-[4-(3-Benzyloxy-propoxy)-phenyl]-3-[[(2-dimethylamino-äthyl)-methyl-amino]-methyl)-5-(naphthalin-2-ylmethoxy)-piperidin-1-carbonsäure tert-butylester das (3SR,4RS,5RS)-N-[4-[4-(3-Benzyloxy-propoxy)-phenyl]-5-(naphthalin-2-ylmethoxy)-piperidin-3-ylmethyl]-N,N',N'-trimethyl-äthan-1,2-diamin als gelbliches Öl; MS : 596 (M+H)$^+$;

7) - aus dem (3RS,4RS,5SR)-4-[4-(3-Benzyloxy-propoxy)-phenyl]-3-(naphthalin-2-ylmethoxy)-5-[1,2,4]triazol-1-ylmethyl-piperidin-1-carbonsäure tert-butylester das (3RS,4RS,5SR)-4-[4-(3-Benzyloxypropoxy)-phenyl]-3-(naphthalin-2-ylmethoxy)-5-[1,2,4]triazol-1-ylmethylpiperidin Hydrochlorid als farbloser Festkörper; MS : 563 (M+H)$^+$;

8) - aus dem (3RS,4RS,5RS)-4-[4-(3-Benzyloxy-propoxy)-phenyl]-3-(naphthalin-2-ylmethoxy)-5-(2-oxo-imidazolidin-1-ylmethyl)-piperidin-1-carbonsäure tert-butylester das (3SR,4RS,5RS)-1-[4-[4-(3-Benzyloxypropoxy)-phenyl]-5-(naphthalin-2-ylmethoxy)-piperidin-3-ylmethyl]-imidazolidin-2-on als farbloser Festkörper; MS : 580 (M+H)$^+$;

9) aus dem (3SR,4RS,5RS)-4-[4-(3-Benzyloxy-propoxy)-phenyl]-3-(naphthalin-2-ylmethoxy)-5-sulfooxymethyl-piperidin-1-carbonsäure tert-butylester Trimethylammonium-Salz der Schwefelsäure mono-(3SR,4RS,5RS) -[4-[4-(3-Benzyloxy-propoxy)-phenyl]-5-(naphthalin-2-ylmethoxy)-piperidin-3-ylmethyl] ester als farbloser Festkörper; MS : 590 (M-H)-.

Die als Ausgangsverbindungen eingesetzten BOC-Derivate wurden wie folgt hergestellt:

(a) In analoger Weise wie in Beispiel 33 (a) beschrieben, wurde durch Umsetzung des (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylesters [Beispiel 148 (h)] mit 4,4'-Dithiopyridin in Gegenwart von Tributylphosphin der (3RS,4RS,5SR)-4-[4-(3-Benzyloxy-propoxy)-phenyl]-3-(naphthalin-2-ylmethoxy)-5-(pyridin-4-ylsulfanylmethyl)-piperidin-1-carbonsäure tert-butylester als gelbe, halbfeste Substanz erhalten; MS : 705 $(M+H)^+$.

(b) In analoger Weise wie in Beispiel 33 (a) beschrieben, wurde durch Umsetzung des (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylesters [Beispiel 148 (h)] mit Diphenyldisulfid in Gegenwart von Tributylphosphin der (3RS,4RS,5SR)-4-[4-(3-Benzyloxy-propoxy)-phenyl]-3-(naphthalin-2-ylmethoxy)-5-phenylsulfanylmethyl-piperidin-1-carbonsäure tert-butylester als farbloses Öl erhalten; MS : 721 $(M+NH_4)^+$.

(c) In analoger Weise wie in Beispiel 1 (g) beschrieben, wurde durch Alkylierung des (3SR,4RS,5RS)-4-[4-(3-Benzyloxy-propoxy)-phenyl]-3-hydroxymethyl-5-(naphthalin-2-ylmethoxy)-piperidin- 1-carbonsäure tert-butyleste rs [Beispiel 148 (h)] mit 4-(2-Chloräthyl)-morpholin der (3SR,4RS,5RS)-4-[4-(3-Benzyloxy-propoxy)-phenyl]-3-(2-morpholin-4-yl-äthoxymethyl)-5-(naphthalin-2-ylmethoxy)-piperidin-1-carbonsäure tert-butylester als farbloses Öl erhalten; MS : 725 $(M+H)^+$.

(d) Nach literaturbekannter Methode wurde durch Umsetzung des (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylesters [Beispiel 148 (h)] mit Mesylchlorid der (3SR,4RS,5RS)-4-[4-(3-Benzyloxypropoxy)-phenyl]-3-methanesulfonyloxymethyl-5-(naphthalin-2-ylmethoxy)-piperidin-1-carbonsäure tert-butylester [MS : 707 $(M+NH_4)^+$] als farbloser Festkörper erhalten. Die weitere Umsetzung mit Diäthylamin in Acetonitril bei 50 °C, analog Beispiel 34, ergab den (3RS,4RS,5RS)-4-[4-(3-Benzyloxy-propoxy)-phenyl]-3-diäthylaminomethyl-5-(naphthalin-2-ylmethoxy)-piperidin-1-carbonsäure tert-butylester als braunen Schaum; MS : 667 $(M+H)^+$.

(e) Die Umsetzung des (3SR,4RS,5RS)-4-[4-(3-Benzyloxy-propoxy)-phenyl]-3-methanesulfonyloxymethyl-5-(naphthalin-2-ylmethoxy)-piperidin-1-carbonsäure tert-butylesters mit N,N,N'-Trimethyl-äthylendiamin in Dimethylformamid bei 100 °C, analog Beispiel 34, lieferte den (3RS,4RS,5RS)-4-[4-(3-Benzyloxy-propoxy)-phenyl]-3-[[(2-dimethylamino-äthyl)-methyl-amino]-methyl]-5-(naphthalin-2-ylmethoxy)-piperidin-1-carbonsäure tert-butylester als farbloses Öl; MS : 696 $(M+H)^+$.

(f) Eine Lösung von 22 mg (0.32 mMol) 1,2,4-Triazol in 5 ml DMF wurde auf 0 °C abgekühlt und mit 15 mg (0.29 mMol) Natriumhydrid (50 %ige Dispersion in Weißöl) versetzt. Anschließend ließ man auf Raumtemperatur erwärmen und rührte 1 Stunde lang weiter. Zu dieser Lösung wurden 70 mg (0.105 mMol) (3SR,4RS,5RS)-4-[4-(3-Benzyloxy-propoxy)-phenyl]-3-methanesulfonyloxymethyl-5-(naphthalin-2-ylmethoxy)-piperidin-1-carbonsäure tert-butylesters gegeben. Das Reaktionsgemisch wurde während 24 Stunden auf 100 °C erhitzt. Zur Aufarbeitung wurde das Reaktionsgemisch unter vermindertem Druck eingedampft, der Rückstand zwischen Wasser und Methylenchlorid verteilt, die organische Phase abgetrennt, über Natriumsulfat getrocknet und eingedampft. Zur Reinigung des Rohprodukts (70 mg) wurde an Kieselgel unter Verwendung eines 98:2-Gemisches von Methylenchlorid und Methanol als Eluierungsmittel chromatographiert. Es wurden 52 mg (77 % d.Th.) (3RS,4RS,5SR)-4-[4-(3-Benzyloxy-propoxy)-phenyl]-3-(naphthalin-2-ylmethoxy)-5-[1,2,4]triazol-1-ylmethyl-piperidin-1-carbonsäure tert-butylester als farbloser Festkörper erhalten; MS : 663 $(M+H)^+$.

(g) In analoger Weise wie in Beispiel 149 (f) beschrieben, wurde durch Umsetzung des (3SR,4RS,5RS)-4-[4-(3-Benzyloxy-propoxy)-phenyl]-3-methanesulfonyloxymethyl-5-(naphthalin-2-ylmethoxy)-piperidin-1-carbonsäure tert-butylesters mit Imidazolidin-2-on der (3RS,4RS,5RS)-4-[4-(3-Benzyloxy-propoxy)-phenyl]-3-(naphthalin-2-ylmethoxy)-5-(2-oxoimidazolidin-1-ylmethyl)-piperidin-1-carbonsäure tert-butylester als gelbliches Öl erhalten.

(h) Eine Lösung von 100 mg (0.136 mMol) (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylester [Beispiel 148 (h)] in 10 ml trockenem Pyridin wurde mit 78 mg (0.43 mMol) Schwefeltrioxid-Trimethylamin-Komplex versetzt und bei Raumtemperatur 36 Stunden lang gerührt. Zur Aufarbeitung wurde das Reaktionsgemisch unter vermindertem Druck eingedampft und das Rohprodukt direkt mittels Flash-Chromatographie unter Verwendung eines 9:1-Gemisches von Methylenchlorid und Methanol als Eluierungsmittel gereinigt. es wurden 102 mg (3SR,4RS,5RS)-4-[4-(3-Benzyloxy-propoxy)-phenyl]-3-(naphthalin-2-ylmethoxy)-5-sulfooxymethyl-piperidin-1-carbonsäure tert-butylester Trimethylammonium-Salz als farbloser Festkörper erhalten; MS : 690 $(M-H)^-$.

Beispiel 150

In analoger Weise wie in Beispiel 10 (b) beschrieben, wurden durch Abspaltung der BOC-Gruppe mittels

Zinkbromid in Methylenchlorid die folgenden Verbindungen erhalten:

1) - Aus dem (3SR,4RS,5RS)-4-[4-(3-Benzyloxy-propoxy)-phenyl]-3-[3-(4-methyl-piperazin-1-yl)-propylcarbamoyloxymethyl]-5-(naphthalin-2-ylmethoxy)-piperidin-1-carbonsäure tert-butylester der [3-(4-Methylpiperazin-1-yl)-propyl]-carbaminsäure (3SR,4RS,5RS)-4-[4-(3-benzyloxypropoxy)-phenyl]-5-(naphthalin-2-ylmethoxy)-piperidin-3-ylmethylester als farbloser Festkörper; MS : 695 $(M+H)^+$.

2) - Aus dem (3SR,4RS,5RS)-4-[4-(3-Benzyloxy-propoxy)-phenyl]-3-(2-morpholin-4-yl-äthylcarbamoyloxymethyl)-5-(naphthalin-2-ylmethoxy)-piperidin-1-carbonsäure tert-butylester der (2-Morpholin-4-yl-äthyl)-carbaminsäure (3SR,4RS,5RS)-4-[4-(3-benzyloxypropoxy)-phenyl]-5-(naphthalin-2-ylmethoxy)-piperidin-3-ylmethylester als farbloses Öl; MS: 668 $(M+H)^+$.

3) - Aus dem (3SR,4RS,5RS)-4-[4-(3-Benzyloxy-propoxy)-phenyl]-3-carbamoyloxymethyl-5-(naphthalin-2-ylmethoxy)-piperidin-1-carbonsäure tert-butylester der Carbaminsäure (3SR,4RS,5RS)-4-[4-(3-benzyloxy-propoxy)-phenyl]-5-(naphthalin-2-ylmethoxy)-piperidin-3-ylmethylester als farbloser Festkörper; MS : 555 $(M+H)^+$.

Die als Ausgangsverbindungen eingesetzten BOC-Derivate wurden wie folgt hergestellt:

(a) Ein Gemisch von 90 mg (0.15 mMol) (3SR,4RS,5RS)-4-[4-(3-Benzyloxypropoxy)-phenyl]-3-hydroxymethyl-5-(naphthalin-2-ylmethoxy)-piperidin-1-carbonsäure tert-butylester [Beispiel 148 (h)] und 60 mg (0.75 mMol) Lithiumcarbonat in 10 ml trockenem Tetrahydrofuran wurde auf 0 °C abgekühlt. Dazu wurde tropfenweise unter Eiskühlung eine Lösung von 0.90 ml (1.6 mMol) Phosgen in Toluol (1.93 N) gegeben. Zur Vervollständigung der Reaktion wurde über Nacht bei Raumtemperatur gerührt. Zur Entfernung des überschüssigen Phosgens wurde das Reaktionsgemisch unter vermindertem Druck eigedampft und der erhaltene rohe Chlorameisensäureester in 10 ml Tetrahydrofuran aufgenommen. Dieses Gemisch wurde mit 58 mg (0.38 mMol) 1-(3-Aminopropyl)-4-methylpiperazin versetzt und 3 Stunden lang bei Raumtemperatur nachgerührt. Zur Aufarbeitung wurde das Reaktiongemisch mit 40 ml Methylenchlorid verdünnt, dann mit 20 ml Wasser extrahiert. Die organische Phase wurde über Natriumsulfat getrocknet und anschließend unter vermindertem Druck eingedampft. Der Rückstand wurde zur Reinigung an Kieselgel unter Verwendung eines 95:5:0.1-Gemisches von Methylenchlorid, Methanol und Ammoniak als Eluierungsmittel chromatographiert. Es wurden 85 mg (73 % d.Th.) (3SR,4RS,5RS)-4-[4-(3-Benzyloxy-propoxy)-phenyl]-3-[3-(4-methyl-piperazin-1-yl)-propylcarbamoyloxymethyl]-5-(naphthalin-2-ylmethoxy)-piperidin-1-carbonsäure tert-butylester als farbloser Festkörper erhalten; MS : 795 $(M+H)^+$.

(b) In analoger Weise wie in Beispiel 150 (a) beschrieben wurde aus dem (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylester [Beispiel 148 (h)] der entsprechende Chlorameisensäureester synthetisiert, dessen Umsetzung mit 4-(2-Aminoäthyl)-morpholin den (3SR,4RS,5RS)-4-[4-(3-Benzyloxy-propoxy)-phenyl]-3-(2-morpholin-4-yl-äthylcarbamoyloxymethyl)-5-(naphthalin-2-ylmethoxy)-piperidin-1-carbonsäure tert-butylester als farblosen Festkörper ergab; MS : 768 $(M+H)^+$.

(c) In analoger Weise wie in Beispiel 150 (a) beschrieben wurde aus dem (3SR,4RS,5RS)-4-[4-(3-Benzyloxy-propoxy)-phenyl]-3-hydroxymethyl-5-(naphthalin-2-ylmethoxy)-piperidin-1-carbonsäure tert-butylester [Beispiel 148 (h)] der entsprechende Chlorameisensäureester synthetisiert, dessen Umsetzung mit einer Lösung von Ammoniak in Tetrahydrofuran den (3SR,4RS,5RS)-4-[4-(3-Benzyloxy-propoxy)-phenyl]-3-carbamoyloxymethyl-5-(naphthalin-2-ylmethoxy)-piperidin-1-carbonsäure tert-butylester als farblosen Festkörper ergab; MS : 672 $(M+NH_4)^+$.

Beispiel 151

In analoger Weise wie in Beispiel 22 (l) beschrieben, wurden durch Abspaltung der BOC-Gruppe mittels Chlorwasserstoff in Methanol die folgenden Verbindungen erhalten:

1) - Aus dem (3RS,4RS)-4-[4-(2-Benzyloxy-äthoxymethyl)-phenyl]-3-(naphthalin-2-ylmethoxy)-piperidin-1-carbonsäure tert-butylester das (3RS,4RS)-4-[4-(2-Benzyloxy-äthoxymethyl)-phenyl]-3-(naphthalin-2-ylmethoxy)-piperidin als farbloser Schaum; MS : 482 $(M+H)^+$;

2) - aus dem (3SR,4RS,5RS)-4-[4-(2-Benzyloxy-äthoxymethyl)-phenyl]-3-hydroxymethyl-5-(naphthalin-2-ylmethoxy)-piperidin-1-carbonsäure tert-butylester das (3SR,4RS,5RS)-[4-[4-(2-Benzyloxy-äthoxymethyl)-phenyl]-5-(naphthalin-2-ylmethoxy)-piperidin-3-yl]-methanol als farbloses Öl; MS : 512 $(M+H)^+$;

3) - aus dem (3SR,4RS,5RS)-4-[4-(2-Benzyloxy-äthoxymethyl)-phenyl]-3-methoxymethyl-5-(naphthalin-2-ylmethoxy)-piperidin-1-carbonsäure tert-butylester das (3SR,4RS,5RS)-4-[4-(2-Benzyloxy-äthoxymethyl)-phenyl]-3-methoxymethyl-5-(naphthalin-2-ylmethoxy)-piperidin als farbloses Öl; MS : 526 $(M+H)^+$;

4) - aus dem (3RS,4RS,5SR)-4-[4-(2-Benzyloxy-äthoxymethyl)-phenyl]-3-(naphthalin-2-ylmethoxy)-5-(pyridin-3-ylmethoxymethyl)-piperidin-1-carbonsäure tert-butylester das (3SR,4RS,5RS)-3-[4-[4-(2-Benzyloxy-äthoxymethyl)-phenyl]-5-(naphthalin-2-ylmethoxy)-piperidin-3-ylmethoxymethyl]-pyridin als farbloses Öl; MS : 603 $(M+H)^+$;

5) - aus dem (3SR,4RS,5RS)-4-[4-(2-Benzyloxy-äthoxymethyl)-phenyl]-3-(2-methoxy-äthoxymethyl)-5-(naphthalin-2-ylmethoxy)-piperidin-1-carbonsäure tert-butylester das (3SR,4RS,5RS)-4-[4-(2-Benzyloxy-äthoxymethyl)-phenyl]-3-(2-methoxy-äthoxymethyl)-5-(naphthalin-2-ylmethoxy)-piperidin als farbloses Öl; MS : 570 $(M+H)^+$;

6) - aus dem Gemisch des (3RS,4RS,5SR)- und (3SR,4SR,5RS)-4-[4-(2-Benzyloxy-äthoxymethyl)-phenyl]-3-(naphthalin-2-ylmethoxy)-5-[3-[(RS)-tetrahydro-pyran-2-yloxy]-propoxymethyl]-piperidin-1-carbonsäure tert-butylesters unter gleichzeitiger Abspaltung der THP-Gruppe das (3SR,4RS,5RS)-3-[4-[4-(2-Benzyloxy-äthoxymethyl)-phenyl]-5-(naphthalin-2-ylmethoxy)-piperidin-3-ylmethoxy]-propan-1-ol als farbloses Öl; MS : 570 $(M+H)^+$;

7) - aus dem (3SR,4RS,5RS)-3-Methoxymethyl-4-{4-[(methyl-phenylcarbamoyloxy)-methyl]-phenyl}-5-(naphthalin-2-ylmethoxy)-piperidin-1-carbonsäure tert-butylester der Methyl-phenyl-carbaminsäure (3SR,4RS,5RS)-4-[3-methoxymethyl-5-(naphthalin-2-ylmethoxy)-piperidin-4-yl]-benzylester als farbloses Öl; MS : 525 $(M+H)^+$;

8) - aus dem (3SR,4RS,5RS)-4-[4-[(Benzyl-methyl-carbamoyloxy)-methyl]-phenyl]-3-methoxymethyl-5-(naphthalin-2-ylmethoxy)-piperidin-1-carbonsäure tert-butylester den Benzyl-methylcarbaminsäure (3SR,4RS,5RS)-4-[3-methoxymethyl-5-(naphthalin-2-ylmethoxy)-piperidin-4-yl]-benzylester als farbloses Öl; MS : 539 $(M+H)^+$.

Die als Ausgangsstoffe eingesetzten BOC-Derivate wurden wie folgt erhalten:

(a) In analoger Weise wie in Beispiel 1 (g) beschrieben, wurde durch Alkylierung des (3RS,4RS)-4-(4-Hydroxymethyl-phenyl)-3-(naphthalin-2-ylmethoxy)-piperidin-1-carbonsäure tert-butylesters [Beispiel 22 (j)] mit 2-(Benzyloxy)-äthyliodid (Helv.Chim.Acta Vol.71, (1988), 2039] der (3RS,4RS)-4-[4-(2-Benzyloxy-äthoxymethyl)-phenyl]-3-(naphthalin-2-ylmethoxy)-piperidin-1-carbonsäure tert-butylester als farbloser Schaum erhalten; MS : 599 $(M+NH_4)^+$.

(b) In analoger Weise wie in Beispiel 22 (d) beschrieben, wurde aus dem (3RS,4RS,5SR)-1-Benzyl-4-(4-bromphenyl)-5-hydroxymethyl-piperidin-3-ol [Beispiel 68 (e)] durch eine Palladium-katalysierte Carbonylierung mit Kohlenmonoxid in Methanol der (3RS,4RS,5SR)-4-(1-Benzyl-3-hydroxy-5-hydroxymethyl-piperidin-4-yl)-benzoesäure methylester erhalten, dessen Hydrogenolyse in Gegenwart von Palladium(5 %ig)-Kohle bei Atmosphärendruck in Methanol, analog Beispiel 2 (e), den 4-(3-Hydroxy-5-hydroxymethyl-piperidin-4-yl)-benzoesäure methylester ergab. Die darauffolgende Einführung der BOC-Gruppe, analog Beispiel 1 (f), lieferte den (3RS,4RS,5SR)-3-Hydroxy-5-hydroxymethyl-4-(4-methoxycarbonyl-phenyl)-piperidin-1-carbonsäure tert-butylester, aus dem durch Umsetzung mit Triphenylchlormethan, analog Beispiel 68 (i), der (3RS,4RS,5SR)-3-Hydroxy-4-(4-methoxycarbonyl-phenyl)-5-trityloxymethyl-piperidin-1-carbonsäure tert-butylester erhalten wurde. Die sich anschließende Alkylierung mittels 2-Brommethyl-naphthalin, analog Beispiel 1 (g), lieferte den (3RS,4RS,5SR)-4-(4-Methoxycarbonyl-phenyl)-3-(naphthalin-2-ylmethoxy)-5-trityloxymethyl-piperidin-1-carbonsäure tert-butylester , dessen Reduktion mit Lithiumborhydrid, analog zu Beispiel 22 (e), den (3RS,4RS,5SR)-4-(4-Hydroxymethylphenyl)-3-(naphthalin-2-ylmethoxy)-5-trityloxymethyl-piperidin-1-carbonsäure tert-butylester als farblosen Schaum lieferte; MS : 737 $(M+NH_4)^+$. Die weitere Alkylierung mit 2-(Benzyloxy)-äthyliodid [Helv.Chim.Acta Vol.71, (1988), 2039], analog Beispiel 1 (g), ergab den (3RS,4RS,5SR)-4-[4-(2-Benzyloxy-äthoxymethyl)-phenyl]-3-(naphthalin-2-ylmethoxy)-5-trityloxymethyl-piperidin-1-carbonsäure tert-butylester, aus dem nach Abspaltung der Trityl-Gruppe mittels eines Gemisches aus Trifluoressigsäure und Trifluoressigsäureanhydrid in Methylenchlorid, analog Beispiel 86 (u), der (3SR,4RS,5RS)-4-[4-(2-Benzyloxy-äthoxymethyl)-phenyl]-3-hydroxymethyl-5-(naphthalin-2-ylmethoxy)-piperidin-1-carbonsäure tert-butylester als farbloses Öl erhalten wurde; MS : 629 $(M+NH_4)^+$.

(c) In analoger Weise wie in Beispiel 1 (g) beschrieben, wurde durch Alkylierung des (3SR,4RS,5RS)-4-[4-(2-Benzyloxy-äthoxymethyl)-phenyl]-3-hydroxymethyl-5-(naphthalin-2-ylmethoxy)-piperidin-1-carbonsäure tert-butylesters mit Methyliodid der (3SR,4RS,5RS)-4-[4-(2-Benzyloxy-äthoxymethyl)-phenyl]-3-methoxymethyl-5-(naphthalin-2-ylmethoxy)-piperidin-1-carbonsäure tert-butylester als farbloses Öl erhalten; MS : 643 $(M+NH_4)^+$.

(d) In analoger Weise wie in Beispiel 1 (g) beschrieben, wurde durch Alkylierung des (3SR,4RS,5RS-4-[4-(2-Benzyloxy-äthoxymethyl)-phenyl]-3-hydroxymethyl-5-(naphthalin-2-ylmethoxy)-piperidin-1--carbonsäure tert-butylesters mit 3-(Chlormethyl)-pyridin der (3RS,4RS,5SR)-4-[4-(2-Benzyloxy-äthoxymethyl)-phenyl]-3-(naphthalin-2-ylmethoxy)-5-(pyridin-3-ylmethoxymethyl)-piperidin-1-carbonsäure tert-butylester als farbloses Öl erhalten; MS : 703 $(M+H)^+$.

(e) In analoger Weise wie in Beispiel 1 (g) beschrieben, wurde durch Alkylierung des (3SR,4RS,5RS)-4-[4-(2-Benzyloxy-äthoxymethyl)-phenyl]-3-hydroxymethyl-5-(naphthalin-2-ylmethoxy)-piperidin-1-carbonsäure tert-butylesters mit 2-Methoxy-äthylbromid der (3SR,4RS,5RS)-4-[4-(2-Benzyloxy-äthoxymethyl)-phenyl]-3-(2-methoxy-äthoxymethyl)-5-(naphthalin-2-ylmethoxy)-piperidin-1-carbonsäure tert-butylester als farbloses Öl erhalten; MS : 687 $(M+NH_4)^+$.

(f) In analoger Weise wie in Beispiel 1 (g) beschrieben, wurde durch Alkylierung des (3SR,4RS,5RS)-4-[4-(2-Benzyloxy-äthoxymethyl)-phenyl]-3-hydroxymethyl-5-(naphthalin-2-ylmethoxy)-piperidin-1-carbonsäure tert-butylesters mit rac-2-(3-Brompropoxy)-tetrahydro-2Hpyran [J.Org.Chem. 53, (1988), 25, 5903-5908] das Gemisch des (3RS,4RS,5SR)- und (3SR,4SR,5RS)-4-[4-(2-Benzyloxy-äthoxymethyl)-phenyl]-3-(naphthalin-2-ylmethoxy)-5-[3-[(RS)-tetrahydro-pyran-2-yloxy]-propoxymethyl]-piperidin-1-carbonsäure tert-butylesters als farbloses Öl erhalten; MS : 771 $(M+NH_4)^+$.

(g) In analoger Weise wie in Beispiel 24 (m) beschrieben, ergab die Umsetzung des (3RS,4RS,5SR)-4-(4-Hydroxymethyl-phenyl)-3-(naphthalin-2-ylmethoxy)-5-trityloxymethyl-piperidin-1-carbonsäure tert-butylesters mit Phenylisocyanat den (3RS,4RS,5SR)-3-(Naphthalin-2-ylmethoxy)-4-(4-phenylcarbamoyloxymethyl-phenyl)-5-trityloxymethylpiperidin-1-carbonsäure tert-butylester, aus dem durch Abspaltung der Tritylgruppe mittels eines Gemisches aus Trifluoressigsäure und Trifluoressigsäureanhydrid in Methylenchlorid, analog Beispiel 86 (u), der (3SR, 4RS,5RS)-3-Hydroxymethyl-5-(naphthalin-2-ylmethoxy)-4-(4-phenylcarbamoyloxymethyl-phenyl)-piperidin-1-carbonsäure tert-butylester erhalten wurde. Die sich anschließende Alkylierung mit Methyliodid, analog Beispiel 1 (g), lieferte den (3SR,4RS,5RS)-3-Methoxymethyl-4-{4-[(methyl-phenyl-carbamoyloxy)-methyl]-phenyl}-5-(naphthalin-2-ylmethoxy)-piperidin-1-carbonsäure tert-butylester als farbloses Öl; MS : 642 $(M+NH_4)^+$.

(h) In analoger Weise wie in Beispiel 24 (m) beschrieben, ergab die Umsetzung des (3RS,4RS,5SR)-4-(4-Hydroxymethyl-phenyl)-3-(naphthalin-2-ylmethoxy)-5-trityloxymethyl-piperidin-1-carbonsäure tert-butylesters mit Benzylisocyanat den (3RS,4RS,5SR)-4-(4-Benzylcarbamoyloxymethyl-phenyl)-3-(naphthalin-2-ylmethoxy)-5-trityloxymethyl-piperidin-1-carbonsäure tert-butylester, aus dem durch Abspaltung der Tritylgruppe mittels eines Gemisches aus Trifluoressigsäure und Trifluoressigsäureanhydrid in Methylenchlorid, analog Beispiel 86 (u), der (3SR, 4RS,5RS)-4-(4-Benzylcarbamoyloxymethyl-phenyl)-3-hydroxymethyl-5-(naphthalin-2-ylmethoxy)-piperidin-1-carbonsäure tert-butylester erhalten wurde. Die sich anschließende Alkylierung mit Methyliodid, analog Beispiel 1 (g), lieferte den (3SR,4RS,5RS)-4-[4-[(Benzyl-methyl-carbamoyloxy)-methyl]-phenyl]-3-methoxymethyl-5-(naphthalin-2-ylmethoxy)-piperidin-1-carbonsäure tert-butylester als farbloses Öl; MS : 656 $(M+NH_4)^+$.

Beispiel 152

Durch Abspaltung der BOC-Gruppe mittels Zinkbromid in Methylenchlorid wurden die folgenden Verbindungen erhalten:

1) - Aus dem (3RS,4RS)-4-(4-Fluor-phenyl)-3-(4-methylsulfanylbenzyloxy)-piperidin-1-carbonsäure tert-butylester das (3RS,4RS)-4-(4-Fluor-phenyl)-3-(4-methylsulfanyl-benzyloxy)-piperidin als gelbliches Öl; MS : 332 $(M+H)^+$;

2) - aus dem Gemisch des (3RS,4RS)- und (3SR,4SR)-4-(4-Fluorphenyl)-3-[(RS)-4-methansulfinyl-benzyloxy)-piperidin-1-carbonsäure tert-butylester das Gemisch des (3RS,4RS)- und (3SR,4SR)-4-(4-Fluorphenyl)-3-[(RS)-4-methylsulfinyl-benzyloxy]-piperidins als gelbliches Öl; MS : 348 $(M+H)^+$;

3) - aus dem (3RS,4RS)-4-(4-Fluor-phenyl)-3-(4-methylsulfonylbenzyloxy)-piperidin-1-carbonsäure tert-butylester das (3RS,4RS)-4-(4-Fluor-phenyl)-3-(4-methylsulfonyl-benzyloxy)-piperidin Hydrobromid als gelblicher Festkörper; MS : 364 $(M+H)^+$;

4) - aus dem (3RS,4R5)-3-(1,4-Dimethoxy-naphthalin-2-ylmethoxy)-4-(4-hydroxy-phenyl)-piperidin-1-carbonsäure tert-butylester das (3RS,4RS)-4-[3-(1,4-Dimethoxy-naphthalin-2-ylmethoxy)-piperidin-4-yl]-phenol als farbloser Festkörper; MS : 394 $(M+H)^+$;

5) - aus dem (3RS,4RS)-4-[4-(3-Cyano-benzyloxy)-phenyl]-3-(1,4-dimethoxy-naphthalin-2-ylmethoxy)-piperidin-1-carbonsäure tert-butylester mittels Zinkbromid das (3RS,4RS)-3-[4-[3-(1,4-Dimethoxynaphthalin-2-ylmethoxy)-piperidin-4-yl]-phenoxymethyl]-benzonitril als farbloser Festkörper; MS : 509 $(M+H)^+$.

Die als Ausgangsverbindungen eingesetzten BOC-Derivate wurden wie folgt hergestellt:

(a) In analoger Weise wie in Beispiel 1 (g) beschrieben, wurde durch Alkylierung des (3RS,4RS)-4-(4-Fluor-phenyl)-3-hydroxy-piperidin-1-carbonsäure tert-butylesters [Beispiel 3 (b)] mit 4-Methylthio-benzylchlorid [J.Org. Chem. (1988), 53(3), 561-569] der (3RS,4RS)-4-(4-Fluor-phenyl)-3-(4-methylsulfanyl-benzyloxy)-piperidin-1-carbonsäure tert-butylester als farbloses Öl erhalten; MS : 432 $(M+H)^+$.

(b) In analoger Weise wie in Beispiel 58 (i) beschrieben, wurde durch Oxidation des (3RS,4RS)-4-(4-Fluor-phenyl)-3-(4-methylsulfanylbenzyloxy)-piperidin-1-carbonsäure tert-butylesters mittels Natriummetaperjodat das Gemisch des (3RS,4RS)- und (3SR,4SR)-4-(4-Fluor-phenyl)-3-[(RS)-4-methansulfinyl-benzyloxy)-piperidin-1-carbonsäure tert-butylesters als gelbliches Öl erhalten; MS : 448 $(M+H)^+$.

(c) Zu einer Lösung von 115 mg (0.27 mMol) (3RS,4RS)-4-(4-Fluorphenyl)-3-(4-methylsulfanyl-benzyloxy)-piperidin-1-carbonsäure tert-butylester in 2 ml Methylenchlorid wurden 126 mg 0.586 mMol) m-Chlorperbenzoesäure (80 %ig) gegeben. Die Reaktionslösung wurde während 2 Stunden bei Raumtemperatur gerührt und danach mit Kaliumcarbonat in Methanol neutralisiert. Anschließend wurde die Lösung mit Methylenchlorid verdünnt. Danach wurde in üblicher Weise aufgearbeitet und das erhaltene Rohprodukt zur Reinigung an Kieselgel unter Verwendung eines 4:1-Gemisches von Methylenchlorid und Äther als Eluierungsmittel chromatographgiert. Es wurden 105 mg (85 % d.Th.) (3RS,4RS)-4-(4-Fluor-phenyl)-3-(4-methylsulfonylbenzyloxy)-piperidin-1-carbonsäure tert-butylester als farbloses, viskoses Öl erhalten; MS : 464 $(M+H)^+$.

(d) (a) In analoger Weise wie in Beispiel 1 (g) beschrieben, wurde durch Alkylierung des (3RS,4RS)-4-(4-Allyioxy-phenyl)-3-hydroxy-piperidin-1-carbonsäure tert-butylesters [Beispiel 86 (b)] mit 2-Chlormethyl-1,4-dimethoxy-naphthalin [J.Org.Chem. (1983), 48(19),3265-3268) der (3RS,4RS)-4-(4-Allyloxy-phenyl)-3-(1,4-dimethoxy-naphthalin-2-ylmethoxy)-piperidin-1-carbonsäure tert-butylester als hellgelber Festkörper erhalten; MS : 534 $(M+H)^+$.

(β) Eine Lösung von 315 mg (0.59 mMol) (3RS,4RS)-4-(4-Allyloxy-phenyl)-3-(1,4-dimethoxy-naphthalin-2-ylmethoxy)-piperidin-1-carbonsäure tert-butylester, 44 mg (0.059 mMol) Bis-(triphenylphosphin)-palladium(II)-diacetat und 132 mg (1.18 mMol) 1,4-Diaza-bicyclo[2.2.2]octan in 10 ml 95 %igem Äthanol wurde während 2 Stunden unter Argon zum Rückfluß erhitzt. Anschließend wurde das Reaktionsgemisch auf 0-5 °C abgekühlt und mit 0.6 ml 1 N Salzsäure versetzt. Danach wurde das Lösungsmittel unter vermindertem Druck abdestilliert und der Rückstand zwischen Essigester und Wasser verteilt. Nach der Extraktion und dem Trocknen der organischen Phase über Natriumsulfat wurde unter vermindertem Druck eingedampft. Der Rückstand wurde an Kieselgel unter Verwendung eines 9:1-Gemisches von Methylenchlorid und Äther als Eluierungsmittel gereinigt. Man erhielt 265 mg (91 % d. Th.) (3RS,4RS)-3-(1,4-Dimethoxy-naphthalin-2-ylmethoxy)-4-(4-hydroxy-phenyl)-piperidin-1-carbonsäure tert-butylester als gelblichen Schaum;MS : 494 $(M+H)^+$.

(e) In analoger Weise wie in Beispiel 44 e) beschrieben, wurde durch Alkylierung des (3RS,4RS)-3-(1,4-Dimethoxy-naphthalin-2-ylmethoxy)-4-(4-hydroxy-phenyl)-piperidin-1-carbonsäure tert-butylesters mit 3-Brommethyl-benzonitril der (3RS,4RS)-4-[4-(3-Cyano-benzyloxy)-phenyl]-3-( 1,4-dimethoxy-naphthalin-2-ylmethoxy)-piperidin-1-carbonsäure tert-butylester als beiger Festkörper erhalten; MS : 609 $(M+H)^+$.

<u>Beispiel 153</u>

Eine Lösung von 736 mg (1.28 mMol) (3R,4R)-4-(4-Bromphenyl)-3-(naphthalin-2-ylmethoxy)-1-[(1S,4R)-4,7,7-trimethyl-3-oxo-2-oxabicyclo[2.2.1]heptan-1-carbonyl]-piperidin in 32 ml absolutem Tetrahydrofuran wurde auf -70 °C abgekühlt. Dazu wurde eine Lösung von 1.86 ml (6.5 mMol) Natrium dihydrido-bis-(2-methoxyäthoxy)-aluminat (70%ig in Toluol, ca. 3.5 M) in 32 ml Tetrahydrofuran getropft. Das Gemisch wurde auf -40 °C erwärmt, 8 Stunden lang bei -40 °C nachgerührt, danach auf -78 °C abgekühlt und mit einer Lösung von 0.5 ml Eisessig in 10 ml Tetrahydrofuran tropfenweise versetzt. Zur Aufarbeitung wurde das Reaktionsgemisch zwischen 200 ml Essigester und 200 ml wäßriger 5%iger Natriumhydrogencarbonat-Lösung verteilt. Die organische Phase wurde abgetrennt und die wäßrige Phase

dreimal mit je 50 ml Essigester nachextrahiert. Die vereinigten organischen Phasen wurden über Magnesiumsulfat getrocknet und schließlich das Lösungsmittel unter verminderten Druck abdestilliert. Das Rohprodukt (800 mg) wurde durch Chromatographie an Kieselgel mit einem 9:1-Gemisch von Methylenchlorid und Methanol als Eluierungsmittel gereinigt. Es wurden 344 mg (68% d.Th.) (3R,4R)-4-(4-Bromphenyl)-3-(naphthalin-2-ylmethoxy)-piperidin als farbloser Festkörper erhalten; MS: 396, 398 $(M+H)^+$.

Das als Ausgangssubstanz eingesetzte Camphansäure-Derivate wurde wie folgt hergestellt:

Eine Lösung von 1.98 g (5.00 mMol) (3RS,4RS)-4-(4-Bromphenyl)-3-(naphthalin-2-ylmethoxy)-piperidin (Beispiel 2.6) in 20 ml Methylenchlorid wurde auf 0 °C abgekühlt und mit 0.84 ml (0.61 g, 6.0 mMol) Triäthylamin versetzt. Dazu wurde unter Argon bei 0 °C eine Lösung von 1.19 g (5.5 mMol) (-)-(1S,4R)-Camphansäurechlorid in 20 ml Methylenchlorid getropft, anschließend 30 Minuten lang bei 0 °C und weitere 2 Stunden bei Raumtemperatur gerührt. Zur Aufarbeitung wurde das Reaktionsgemisch zwischen 200 ml Methylenchlorid und 200 ml Eiswasser verteilt. Die organische Phase wurde abgetrennt und die wäßrige Phase dreimal mit je 50 ml Essigester extrahiert. Die vereinigten organischen Phasen wurden über Magnesiumsulfat getrocknet und schließlich das Lösungsmittel unter verminderten Druck abdestilliert. Das rohe Isomerengemisch (2.89 g) wurde durch Chromatographie an Kieselgel mit einem 2:3-Gemisch von Essigester und Hexan als Eluierungsmittel aufgetrennt. Es wurden 1.14 g (40% d.Th.) (3R,4R)-4-(4-Bromphenyl)-3-(naphthalin-2-ylmethoxy)-1-[(1S,4R)-4,7,7-trimethyl-3-oxo-2-oxabicyclo[2.2.1]heptan-1-carbonyl]-piperidin, MS: 575, 577 $(M)^+$, und 1.01 g (35% d.Th.) (3S,4S)-4-(4-Bromphenyl)-3-(naphthalin-2-ylmethoxy)-1-[(1S,4R)-4,7,7-trimethyl-3-oxo-2-oxabicyclo[2.2.1]heptan-1-carbonyl]-piperidin, MS: 575, 577 $(M)^+$, jeweils als farbloser Festkörper erhalten.

<u>Beispiel 154</u>

In analoger Weise wie im vorangehenden Beispiel beschrieben, wurden durch reduktive Abspaltung des Camphanyl-Restes die folgenden Verbindungen erhalten:

1) - Aus dem(3S,4S)-4-(4-Bromphenyl)-3-(naphthalin-2-ylmethoxy)-1-[(1S,4R)-4,7,7-trimethyl-3-oxo-2-oxabicyclo [2.2.1]heptan-1-carbonyl]-piperidin das (3S,4S)-4-(4-Bromphenyl)-3-(naphthalin-2-ylmethoxy)-piperidin als farbloser Festkörper; MS: 396, 398 $(M+H)^+$;

2) - aus dem (3R,4R)-4-(4-Chlorphenyl)-3-(4-methoxy-benzyloxy)-1-((1S,4R)-4,7,7-trimethyl-3-oxo-2-oxabicyclo [2.2.1]heptan-1-carbonyl]-piperidin das (3R,4R)-4-(4-Chlorphenyl)-3-(4-methoxy-benzyloxy)-piperidin als farbloser Festkörper; MS: 332, 334 $(M+H)^+$;

3) - aus dem (3S,4S)-4-(4-Chlorphenyl)-3-(4-methoxy-benzyloxy)-1-[(1S,4R)-4,7,7-trimethyl-3-oxo-2-oxabicyclo [2.2.1]heptan-1-carbonyl]-piperidin das (3S,4S)-4-(4-Chlorphenyl)-3-(4-methoxy-benzyloxy)-piperidin als farbloser Festkörper; MS: 332, 334 $(M+H)^+$;

4) - aus dem (3R,4R)-4-Naphthalin-1-yl-3-(naphthalin-2-ylmethoxy)-1-[(1S,4R)-4,7,7-trimethyl-3-oxo-2-oxabicyclo [2.2.1]heptan-1-carbonyl)-piperidin das (3R,4R)-4-Naphthalin-1-yl-3-(naphthalin-2-ylmethoxy)-piperidin als farbloser Festkörper; MS: 368 $(M+H)^+$;

5) - aus dem (3S,4S)-4-Naphthalin-1-yl-3-(naphthalin-2-ylmethoxy)-1-[(1S,4R)-4,7,7-trimethyl-3-oxo-2-oxabicyclo [2.2.1]heptan-1-carbonyl]-piperidin wurde das (3S,4S)-4-Naphthalin-1-yl-3-(naphthalin-2-ylmethoxy)-piperidin als farbloser Festkörper; MS: 368 $(M+H)^+$;

6) - aus dem (3R,4R)-3-(4-Benzyloxy-naphthalin-2-ylmethoxy)-4-(4-fluor-phenyl)-1-[(1S,4R)-4,7,7-trimethyl-3-oxo-2-oxabicyclo[2.2.1]heptan-1-carbonyl]-piperidin das (3R,4R)-3-(4-Benzyloxy-naphthalin-2-ylmethoxy)-4-(4-fluor-phenyl)-piperidin als farbloser Festkörper; MS: 441 (M)+;

7) - aus dem (3S,4S)-3-(4-Benzyloxy-naphthalin-2-ylmethoxy)-4-(4-fluor-phenyl)-1-[(1S,4R)-4,7,7-trimethyl-3-oxo-2-oxabicyclo[2.2.1]heptan-1-carbonyl]-piperidin das (3S,4S)-3-(4-Benzyloxy-naphthalin-2-ylmethoxy)-4-(4-fluor-phenyl) piperidin als farbloser Festkörper; MS: 441 $(M)^+$.

In analoger Weise wie in dem vorangehenden Beispiel beschrieben, wurden durch Acylierung mit (-)-Camphansäurechlorid die folgenden Derivate hergestellt:

(a) - Aus dem (3RS,4RS)-4-(4-Chlorphenyl)-3-(4-methoxy-benzyloxy)-piperidin [hergestellt wie in den Beispielen 1 und 2 für (3RS,4RS)-3-(4-Methoxy-benzyloxy)-4-p-tolyl-piperidin (Beispiel 2.4) beschrieben] das (3R,4R)-4-(4-Chlorphenyl)-3-(4-methoxy-benzyloxy)-1-[( 1S,4R)-4,7,7-trimethyl-3-oxo-2-oxabicyclo [2.2.1] heptan-1-carbonyl]-piperidin, MS: 511, 513 $(M)^+$, und das (3S,4S)-4-(4-Chlorphenyl)-3-(4-methoxybenzyloxy)-1-[(1S,4R)-4,7,7-trimethyl-3-oxo-2-oxabicyclo[2.2.1]heptan-1-carbonyl]-piperidin, MS: 511, 513 $(M)^+$, jeweils als farbloser Festkörper;

(b) - aus dem (3RS,4RS)-4-Naphthalin-1-yl-3-(naphthalin-2-ylmethoxy)-piperidin (Beispiel 2.12) das (3R,4R)-4-Naphthalin-1-yl-3-(naphthalin-2-ylmethoxy)-1-[(1S,4R)-4,7,7-trimethyl-3-oxo-2-oxabicyclo[2.2.1]heptan-1-carbonyl]-piperidin, MS: 547 $(M)^+$, und das (3S,4S)-4-Naphthalin-1-yl-3-(naphthalin-2-ylmethoxy)-1-[(1S,4R)-4,7,7-trimethyl-3-oxo-2-oxabicyclo[2.2.1]heptan-1-carbonyl]-piperidin, MS: 547 $(M)^+$, jeweils als farbloser Schaum;

(c) - aus dem (3RS,4RS)-3-(4-Benzyloxy-naphthalin-2-ylmethoxy)-4-(4-fluor-phenyl)-piperidin (Beispiel 14.13) das (3R,4R)-3-(4-Benzyloxynaphthalin-2-ylmethoxy)-4-(4-fluor-phenyl)-1-[(1S,4R)-4,7,7-trimethyl-3-oxo-2-oxabicyclo[2.2.1]heptan-1-carbonyl]-piperidin, MS: 621 $(M)^+$, und das (3S,4S)-3-(4-Benzyloxy-naphthalin-2-ylmethoxy)-4-(4-fluor-phenyl)-1-[(1S,4R)-4,7,7-trimethyl-3-oxo-2-oxabicyclo[2.2.1]heptan-1-carbonyl]-piperidin, MS: 621 $(M)^+$, jeweils als farbloser Schaum.

Beispiel A

Orale, wässrige Suspension

Zusammensetzung:

| | |
|---|---|
| Verbindung der Formel I, z.B.(3RS,4RS)-4-[4-(3-Benzyloxy-propoxy)-phenyl]-3-(naphthalin-2-ylmethoxy)-piperidin, mikronisiert | 5.0 g |
| Polysorbat 80 | 0.3 g |
| Hydroxypropylmethylcellulose | 1.0 g |
| Geschmackstoff | q.s. |
| Methylparaben | 0.2 g |
| Propylenparaben | 0.04 g |
| Wasser | ad 100.0 ml |

Beispiel B

Tabletten

Zusammensetzung:

| | |
|---|---|
| 1) Verbindung der Formel I, z.B. (3RS,4RS)-4-[4-(3-Benzyloxy-propoxy)-phenyl]-3-(naphthalin-2-ylmethoxy)-piperidin | 200 mg |
| 2) Milchzucker wasserfrei | 160 mg |
| 3) Hydroxypropylmethylcellulose | 18 mg |
| 4) Natrium-carboxymethylcellulose | 20 mg |
| 5) Magnesiumstearat | 2 mg |
| Tablettengewicht | 400 mg |

Herstellung: 1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt und zu Tabletten geeigneter Größe verpreßt.

Beispiel C

Kapseln

Zusammensetzung:

| | |
|---|---|
| 1) Verbindung der Formel I, z.B. (3RS,4RS)-4-[4-(3-Benzyloxy-propoxy)-phenyl]-3-(naphthalin-2-ylmethoxy)-piperidin | 200 mg |
| 2) Milchzucker wasserfrei | 160 mg |
| 3) Hydroxypropylmethylcellulose | 18 mg |
| 4) Natrium-carboxymethylcellulose | 20 mg |
| 5) Magnesiumstearat | 2 mg |
| Kapselfüllgewicht | 400 mg |

Herstellung: 1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt, das Gemisch in Kapseln geeigneter Größe abgefüllt.

Beispiel D

Injektionslösung

Zusammensetzung:

| | |
|---|---|
| Verbindung der Formel I, z.B. 4-[2-[7-[(3RS,4RS)-4-[4-(3-Benzyloxy-propoxy)-phenyl]-piperidin-3-yloxymethyl]-naphthalin-2-yloxy]-äthyl]-morpholin Hydrochlorid (1:2) | 3 mg |
| D-Mannit pyrogenfrei | 10 mg |
| Wasser zu Injektionszwecken | ad 1.0 ml |

Herstellung: Der Wirkstoff und das Mannit werden in Stickstoffbegastem Wasser gelöst und anschließend nach einem üblichen Verfahren sterilisiert.

Beispiel E

Injektionslösung in Form einer Mischmizell-Lösung

Zusammensetzung:

| | |
|---|---|
| Verbindung der Formel I, z.B. (3RS,4RS)-4-[4-(3-Benzyloxy-propoxy)-phenyl]-3-(naphthalin-2-ylmethoxy)- piperidin | 2.0 mg |
| Natrium-Glycocholat | 98.5 mg |
| Soya-Lecithin | 158.2 mg |
| Na-dihydrogenphosphat | 1.8 mg |

(fortgesetzt)

| | |
|---|---|
| Di-Na-hydrogenphosphat | 9.5 mg |
| Wasser zu Injektionszwecken | ad 1.0 ml |

Herstellung: Verbindung der Formel I, Na-Glycocholat sowie Soya-Lecithin werden in der erforderlichen Menge Äthanol (oder einem adäquaten flüchtigen Lösungsmittel) gelöst. Unter vermindertem Druck und leichter Erwärmung wird das Lösungsmittel evaporiert. Der verbleibende Rückstand wird mit der gepufferten wäßrigen Phase in Lösung gebracht. Die Lösung wird nach üblichen Verfahren weiterbehandelt.

## Patentansprüche

**1.** Piperidinderivate der allgemeinen Formel I

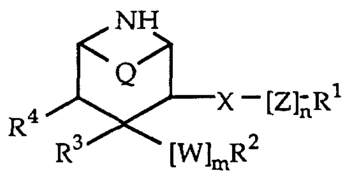

I

worin

$R^1$ Phenyl oder durch $C_{1-6}$-Alkyl, $C_{1-6}$-Alkenyl, $C_{1-6}$-Alkoxy, $C_{1-6}$-Alkylthio, Halogen, Hydroxy, Hydroxy-$C_{1-6}$-alkoxy, $C_{1-6}$-Alkoxy-$C_{1-6}$-alkoxy, $C_{1-6}$-Alkylsulfinyl, $C_{1-6}$-Alkylsulfonyl, Cyano, Trifluormethyl, Trifluormethoxy, Carboxy, Cyclobutylmethoxy-$C_{1-6}$-alkyl, $C_{1-6}$-Alkylendioxy, Phenyl, Phenoxy, $C_{1-6}$-Alkoxycarbonylphenyl, Hydroxy-$C_{1-6}$-alkylphenyl, 2,3-Dihydroxypropylaminocarbonylphenyl, Benzyloxy, Benzoyl, Pyridyl-$C_{1-6}$-alkoxy-$C_{1-6}$-alkyl oder Nicotinoylamino-Ci-6-alkyl substituiertes Phenyl ist; oder auch Naphthyl oder durch Hydroxy, Oxo, $C_{1-6}$-Alkoxy, $C_{1-6}$-Alkenyloxy, $C_{1-6}$-Alkoxy-$C_{1-6}$-alkoxy, Di-$C_{1-6}$-Alkylamino, 2,3-Dihydroxypropoxy, 2,3-Dihydroxypropoxy-$C_{1-6}$-alkoxy, 2,3-Dimethoxypropoxy, $C_{1-6}$-Alkoxycarbonyl-$C_{1-6}$-alkoxy, Carbamoyl-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridyl-carbamoyloxy-$C_{1-6}$-alkoxy, Morpholino-$C_{1-6}$-alkoxy, 3-Morpholino-2-hydroxypropoxy, N-Methylpiperazino-N-$C_{1-6}$-alkoxy, Benzoyloxy-$C_{1-6}$-alkoxy oder Picolyloxy substituiertes Naphthyl ist; oder auch Tetrahydronaphthyl oder durch Methyl substituiertes Tetrahydronaphthyl oder Indanyl ist; oder auch Pyridyl, Benzimidazolyl, Di-$C_{1-6}$-alkoxypyrimidinyl oder 2- und 5-Benzo[b]thienyl, 6- und 7-Chinolyl, 6- und 7-Isochinolyl, 6- und 7-Tetrahydrochinolyl, 6- und 7- Tetrahydroisochinolinyl, 6-Chinoxalinyl, 6- und 7-Chinazolinyl oder durch Hydroxy, Oxo, $C_{1-6}$-Alkoxy, $C_{1-6}$-Alkenyloxy, $C_{1-6}$-Alkoxy-$C_{1-6}$-alkoxy, Di-$C_{1-6}$-Alkylamino, 2,3-Dihydroxypropoxy, 2,3-Dihydroxypropoxy-$C_{1-6}$-alkoxy, 2,3-Dimethoxypropoxy, $C_{1-6}$-Alkoxycarbonyl-$C_{1-6}$-alkoxy, Carbamoyl-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridyl-carbamoyloxy-$C_{1-6}$-alkoxy, Morpholino-$C_{1-6}$-alkoxy, 3-Morpholino-2-hydroxypropoxy, N-Methylpiperazino-N-$C_{1-6}$-alkoxy, Benzoyloxy-$C_{1-6}$-alkoxy oder Picolyloxy substituiertes 6- und 7-Chinolyl, 6- und 7-Isochinolyl, 6- und 7-Tetrahydrochinolyl, 6- und 7-Tetrahydroisochinolinyl, 6-Chinoxalinyl oder 6- und 7- Chinazolinyl ist;

$R^2$ Phenyl, Naphthyl, Acenaphthyl, Cyclohexyl,Pyridyl, Pyrimidinyl, Pyrazinyl, Oxo-pyridinyl, Diazinyl, Triazolyl, Thienyl, Oxazolyl, Oxadiazolyl, Thiazolyl, Pyrrolyl oder Furyl, welche Reste durch 1-3 Halogen, Hydroxy, Cyano, Trifluormethyl, $C_{1-6}$-Alkyl, Halo-$C_{1-6}$-alkyl, Hydroxy-$C_{1-6}$-alkyl, $C_{1-6}$-Alkoxy-$C_{1-6}$-alkyl, Cyano-$C_{1-6}$-alkyl, Carboxy-$C_{1-6}$-alkyl, $C_{1-6}$-Alka-

noyloxy-$C_{1-6}$-alkyl, $C_{1-6}$-Alkoxycarbonyloxy-$C_{1-6}$-alkyl, $C_{1-6}$-Alkoxycarbonyl, oder $C_{1-6}$-Alkoxygruppen, oder eine $C_{1-6}$-Alkylendioxygruppe, und/oder durch einen Rest $L^1$-$T^1$-$L^2$-$T^2$-$L^3$-$T^3$-$L^4$-$T^4$-$L^5$-U substituiert sein können, darstellen;

| | |
|---|---|
| $L^1$, $L^2$, $L^3$, $L^4$ und $L^5$ | unabhängig voneinander eine Bindung, $C_{1-8}$-Alkylen, $C_{2-8}$-Alkenylen oder $C_{2-8}$- Alkinylen darstellen, oder abwesend sind; |
| $T^1$, $T^2$, $T^3$ und $T^4$ | unabhängig voneinander (a) eine Bindung darstellen, oder abwesend sind, oder eine der Gruppen (b) -CH(OH)-; (c) -CH($OR^6$)-; (d) -CH($NR^5R^6$)-; (e) -CO-; (f) -$CR^7R^8$-; (g) -O- oder -$NR^6$-, (h) -$S(O)_{0-2}$-; (i) -$SO_2NR^6$-; (j) -$NR^6$ $SO_2$-; (k) -$CONR^6$-; (l) -$NR^6$ CO-; (m) -O-CO-; (n) -CO-O-; (o) -O-CO-O-; (p) -O-CO-$NR^6$-; (q) -N($R^6$)-CO-N($R^6$)- oder (r) -N($R^6$)-CO-O- darstellen, wobei die von (b), (d), (e) und (g)-(r) ausgehenden Bindungen zu einem C-Atom der benachbarten Gruppe führen und dieses C-Atom gesättigt ist, falls die Bindung von einem Heteroatom ausgeht, und wobei nicht mehr als zwei Gruppen (b)-(f), drei Gruppen (g)-(h) und eine Gruppe (i)-(p) anwesend sind; |
| $R^3$ | Wasserstoff, Hydroxy, $C_{1-6}$-Alkoxy oder $C_{1-6}$-Alkenyloxy; |
| $R^4$ | Wasserstoff, $C_{1-6}$-Alkyl, $C_{1-6}$-Alkenyl, $C_{1-6}$-Alkoxy, Hydroxy-$C_{1-6}$-alkyl, $C_{1-6}$-Alkoxy-$C_{1-6}$alkyl, Benzyl, Oxo, oder eine Gruppe $R^{4a}$- $Z^1$ -$X^1$ - darstellen, wobei |
| $R^{4a}$ | eine Gruppe (a) H-; (b) $C_{1-6}$-Alkyl-; (c) $C_{1-6}$-Alkenyl-; (d) Hydroxy-$C_{1-6}$-Alkyl; (e) Polyhydroxy-$C_{1-6}$-alkyl-; (f) $C_{1-6}$-Alkyl-O-$C_{1-6}$-alkyl-; (g) Aryl-; (h) Heterocyclyl-; (i) Aryl-$C_{1-6}$-alkyl-; (j) Heterocyclyl-$C_{1-6}$-alkyl-; (k) Aryloxy-$C_{1-6}$-alkyl-, (l) Heterocyclyloxyl-$C_{1-6}$-alkyl-; (m) ($R^5$, $R^6$)-N-$(CH_2)_{1-3}$- ; (n) ($R^5$, $R^6$)-N-; (o) $C_{1-6}$-Alkyl-$S(O)_{0-2}$-; (p) Aryl-$S(O)_{0-2}$-; (q) Heterocyclyl-$S(O)_{0-2}$-; (r) HO-$SO_3$- bzw. deren Salze; (s) $H_2N$-C(NH)-NH-; oder (t) NC-darstellt und die von (n) - (t) ausgehenden Bindungen zu einem C-Atom der benachbarten Gruppe führen und dieses C-Atom gesättigt ist, falls die Bindung von einem Heteroatom ausgeht; |
| $Z^1$ | (a) eine Bindung darstellt, abwesend ist, oder eine der Gruppen(b) $C_{1-6}$-Alkylen-; (c) $C_{1-6}$-Alkenylen-; (d) -O-, -N($R^{11}$)-, -$S(O)_{0-2}$-; (e) -CO-; (f) - O-CO-; (g) -O-CO-O-; (h) -O-CO-N($R^{11}$)-; (i) - N($R^{11}$)-CO-O-; (j) - CO-N($R^{11}$)-; (k) -N($R^{11}$)-CO-; (l) - N($R^{11}$)-CO-N ($R^{11}$)- oder (m) -CH($OR^9$)- darstellt und die von (d) und (f) - (m) ausgehenden Bindungen zu einem C-Atom der benachbarten Gruppe führen und dieses C-Atom gesättigt ist, falls die Bindung von einem Heteroatom ausgeht; |
| $X^1$ | (a) eine Bindung darstellt, abwesend ist, oder eine der Gruppen (b) -O-; (c) -N($R^{11}$) -; (d) -$S(O)_{0-2}$-; oder (e) - $(CH_2)_{1-3}$; darstellt; oder |
| $R^3$ und $R^4$ | zusammen eine Bindung darstellen; |
| $R^5$ und $R^6$ | Wasserstoff, $C_{1-6}$-Alkyl, $C_{1-6}$-Alkenyl, Aryl-$C_{1-6}$-alkyl oder Acyl bedeuten, oder gemeinsam mit dem N-Atom, an das sie gebunden sind, einen 5- oder 6- gliedrigen heterocyclischen Ring bedeuten, der ein zusätzliches N-, O- oder S- Atom oder eine -SO- oder -$SO_2$- Gruppe enthalten kann, darstellen, wobei das zusätzliche N-Atom gegebenenfalls durch $C_{1-6}$-Alkyl-Reste substituiert sein kann; |
| $R^7$ und $R^8$ | gemeinsam mit dem C-Atom, an das sie gebunden sind, einen 3-7-gliedrigen Ring darstellen, der ein oder zwei -O- oder -S-Atome oder -SO- oder -$SO_2$- Gruppen enthalten kann; |
| $R^9$ | Wasserstoff, $C_{1-6}$-Alkyl, Acyl oder Aryl-$C_{1-6}$-alkyl ist; |
| $R^{10}$ | Carboxy-$C_{1-6}$-alkyl, $C_{1-6}$-Alkoxycarbonyl-$C_{1-6}$-alkyl, $C_{1-6}$-Alkyl oder Wasserstoff ist; |
| $R^{11}$ | Wasserstoff oder $C_{1-6}$-Alkyl ist; |
| U | Wasserstoff, $C_{1-6}$-Alkyl, $C_{3-6}$-Cycloalkyl, Cyano, gegebenenfalls substituiertes $C_{3-6}$-Cycloalkyl, Aryl, oder Heterocyclyl darstellt: |

| | |
|---|---|
| Q | Aethylen darstellt oder abwesend ist; |
| X | eine Bindung, Sauerstoff, Schwefel oder eine Gruppe -CHOR$^9$- ist, -O-CO, -CO- oder C=NOR$^{10}$- darstellt, wobei die von einem Sauerstoff- oder Schwefelatom ausgehende Bindung zu einem gesättigten C-Atom der Gruppe Z oder zu R$^1$ führt; |
| W | Sauerstoff oder Schwefel darstellt; |
| Z | $C_{1-6}$-Alkylen, $C_{1-6}$-Alkenylen, Hydroxy-$C_{1-6}$-alkyliden-, -Alk-O- oder -Alk-S- ist, wobei Alk $C_{1-6}$-Alkylen bezeichnet; und wobei, falls X eine Bindung darstellt, Z $C_{1-6}$-Alkenylen darstellt, |
| n | 1 ist oder, wenn X -O-CO- ist, 0 oder 1 ist; |
| m | 0 oder 1 ist; |

und pharmazeutisch anwendbare Salze davon;
mit Ausnahme der Verbindungen 4-(4-Fluorphenyl)-3-(3,4-methylendioxybenzyloxy)piperidin, dessen Hydrochlorid und trans-3-(3- Chlorphenoxy)-4-(3, 4-dimethyl-phenoxy) piperidin sowie dessen pharmazeutisch verträgliche Salze; und wobei

"Acyl" $C_{1-6}$-Alkanoylreste oder Benzoyl darstellt;

"Aryl" allein oder in Zusammensetzungen Phenyl, Naphthyl oder Tetrahydronaphthyl darstellt, welche Gruppen ein- oder mehrfach durch $C_{1-6}$-Alkyl, Trifluormethyl, Nitro, Amino, $C_{1-6}$-Alkenyl, $C_{1-6}$-Alkoxy, $C_{1-6}$-Alkylcarbonyloxy, Hydroxy, Halogen, Cyano, Carbamoyl, $C_{1-6}$-Alkylendioxy, gegebenenfalls durch Halogen, $C_{1-6}$-Alkyl, $C_{1-6}$-Alkoxy oder Dihydroxy-$C_{1-6}$-alkylaminocarbonyl substituiertes Phenyl, Phenoxy, Phenylthio, Phenyl-$C_{1-6}$-alkyl, Phenyl-$C_{1-6}$-alkoxy, $C_{1-6}$-Alkoxycarbonylphenyl, Hydroxy-$C_{1-6}$-alkylphenyl, Benzyloxy, Pyridylcarbonylamino-$C_{1-6}$-alkyl, $C_{1-6}$-Alkenyloxy, $C_{1-6}$-Alkoxy-$C_{1-6}$-alkoxy, Methoxybenzyloxy, Hydroxybenzyloxy, Phenäthyloxy, Methylendioxybenzyloxy, Dioxolanyl-$C_{1-6}$-alkoxy, Cyclopropyl-$C_{1-6}$-alkoxy, Hydroxy-$C_{1-6}$-alkoxy, Carbamoyloxy-$C_{1-6}$-alkoxy, Pyridyl-carbamoyloxy-$C_{1-6}$-alkoxy, Benzoyloxy-$C_{1-6}$-alkoxy; sowie gegebenenfalls durch Halogen, $C_{1-6}$-Alkyl, $C_{1-6}$-Alkoxy oder Dihydroxy-$C_{1-6}$-alkylaminocarbonyl substituiertes Pyridyl, Pyridyloxy, Pyridylthio, Pyridylamino, Pyridyl-$C_{1-6}$-alkyl, Pyridyl-$C_{1-6}$-alkoxy, Pyrimidinyl, Pyrimidinyloxy, Pyrimidinylthio, Pyrimidinylamino, Pyrimidinyl-$C_{1-6}$-alkyl, Pyrimidinyl-$C_{1-6}$-alkoxy, Thienyl, Thienyl-$C_{1-6}$-alkyl, Thienyl-$C_{1-6}$-alkoxy, Furyl, Furyl-$C_{1-6}$-alkyl und/oder Furyl-$C_{1-6}$-alkoxy substituiert sein können;

"Heterocyclischer Ring" bzw. "Heterocyclyl" allein oder in Zusammensetzungen mono- oder bicyclische, gesättigte und ungesättigte heterocyclische Reste mit 1 bis 4 Stickstoff- und/oder 1 oder 2 Schwefel- oder Sauerstoffatomen bezeichnet, die ein- oder mehrfach, insbesondere durch (im Falle ungesättigter Heterocyclylreste) $C_{1-6}$-Alkyl, Hydroxy, $C_{1-6}$-Alkoxy, Nitro oder Halogen oder durch Substituenten, wie oben für "Aryl" definiert, substituiert sein können oder (im Falle gesättigter Heterocyclylreste) durch $C_{1-6}$-Alkyl oder $C_{1-6}$-Alkoxy substituiert sein können.

**2.** Verbindung gemäss Anspruch 1 der allgemeinen Formel

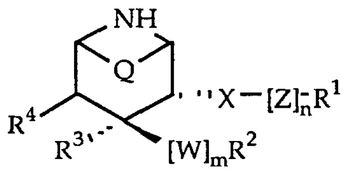

I A

worin R$^1$ - R$^4$, Q, W, X und Z, n und m die in Anspruch 1 angegebene Bedeutung haben, mit Ausnahme der Verbindungen 4-(4-Fluorphenyl)-3-(3,4-methylendioxy-benzyloxy) piperidin, und dessen Hydrochlorid und trans-3-(3-Chlorphenoxy)-4-(3,4-imethyl-phenoxy) piperidin sowie dessen pharmazeutisch verträgliche Salze

**3.** Verbindungen gemäss Anspruch 1 der allgemeinen Formel I

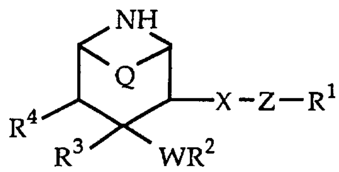

I - 1

worin

| | |
|---|---|
| $R^1$ | die in Anspruch 1 gegebene Bedeutung hat; |
| $R^2$ | Phenyl, Cyclohexyl, durch Halogen, Hydroxy, Cyano, Trifluormethyl, $C_{1-6}$-Alkyl, Halo-$C_{1-6}$-alkyl, Hydroxy-$C_{1-6}$-alkyl, $C_{1-6}$-Alkoxy-$C_{1-6}$-alllyl, Cyano-$C_{1-6}$-alkyl, Carboxy-$C_{1-6}$-alkyl, $C_{1-6}$-Alkanoyloxy-$C_{1-6}$-alkyl, $C_{1-6}$-Alkoxycarbonyloxy-$C_{1-6}$-alkyl, $C_{1-6}$-Alkoxycarbonyl, $C_{1-6}$-Alkoxy, $C_{1-6}$-Alkylendioxy, oder durch einen Rest $L^1$-$T^1$-$L^2$-$T^2$-$L^3$-$T^3$-$L^4$-$T^4$-$L^5$-U substituiertes Phenyl oder Cyclohexyl; oder Naphthyl oder Acenaphthyl; |
| $L^1$, $L^2$, $L^3$, $L^4$ und $L^5$ | unabhängig voneinander eine Bindung, $C_{1-8}$-Alkylen, $C_{2-8}$-Alkenylen oder $C_{2-8}$- Alkinylen, oder abwesend sind; |
| $T^1$, $T^2$, $T^3$ und $T^4$ | unabhängig voneinander (a) eine Bindung darstellen oder abwesend sind, oder eine der Gruppen (b) -CH(OH)-; (c) -CH($OR^6$)-; (d) -CH($NR^5R^6$)-; (e) -CO-; (f) -$CR^7R^8$-; (g) -O- oder -$NR^6$-, (h) -$S(O)_{0-2}$-; (i) -$SO_2NR^6$-; (j) -$NR^6SO_2$-; (k) -$CONR^6$-; (l) -$NR^6CO$-; (m) -O-CO-; (n) -CO-O-; (o) -O-CO-O- oder (p) -O-CO-$NR^6$- darstellen, wobei die von (b), (d), (e) und (g)-(p) ausgehenden Bindungen zu einem C-Atom der benachbarten Gruppe führen und dieses C-Atom gesättigt ist, falls die Bindung von einem Heteroatom ausgeht, und wobei nicht mehr als zwei Gruppen (b)-(f), drei Gruppen (g)-(h) und eine Gruppe (i)-(p) anwesend sind; |
| $R^3$ | Wasserstoff, Hydroxy, $C_{1-6}$-Alkoxy oder $C_{1-6}$-Alkenyloxy; |
| $R^4$ | Wasserstoff, $C_{1-6}$-Alkyl, $C_{1-6}$-Alkenyl, $C_{1-6}$-Alkoxy, Hydroxy-$C_{1-6}$-alkyl, $C_{1-6}$-Alkoxy-$C_{1-6}$alkyl oder Benzyl ist; |
| $R^5$ und $R^6$ | Wasserstoff, $C_{1-6}$-Alkyl oder Acyl, oder gemeinsam mit dem N-Atom, an das sie gebunden sind, einen 5- oder 6-gliedrigen heterocyclischen Ring, der ein zusätzliches N-, O- oder S-Atom enthalten kann; |
| $R^7$ und $R^8$ | gemeinsam mit dem C-Atom, an das sie gebunden sind, einen 3-7-gliedrigen Ring bedeuten, der ein oder zwei Sauerstoff- oder Schwefelatome enthalten kann; |
| U | Wasserstoff, $C_{1-6}$-Alkyl, $C_{3-6}$-Cycloalkyl, Cyano, Aryl oder Heterocyclyl; |
| Q | Aethylen oder abwesend ist; |
| X | Sauerstoff, Schwefel oder eine Gruppe, -$CHOR^9$-, oder -OCO- und $R^9$ Wasserstoff, $C_{1-6}$-Alkyl, Acyl oder Aryl-$C_{1-6}$-alkyl ist; |
| W | abwesend ist; oder Sauerstoff oder Schwefel darstellen kann, wenn $R^3$ Wasserstoff ist; |
| Z | $C_{1-6}$-Alkylen oder abwesend ist. |

**4.** Verbindungen gemäss Anspruch 1 -3, in denen W abwesend ist.

**5.** Verbindungen gemäss Anspruch 1-4, in denen Q abwesend ist.

**6.** Verbindungen gemäss Anspruch 1-5, in denen X Sauerstoff, Schwefel oder -CO- ist.

**7.** Verbindungen gemäss Anspruch 1-6, in denen Z Methylen ist.

**8.** Verbindungen gemäss Anspruch 1-7, in denen $R^2$ Phenyl, durch Halogen, Hydroxy, Cyano, Trifluormethyl, $C_{1-6}$-Alkyl, Halo-$C_{1-6}$-alkyl, Hydroxy-$C_{1-6}$-alkyl, $C_{1-6}$-Alkoxy-$C_{1-6}$-alkyl, Cyano-$C_{1-6}$-alkyl, Carboxy-$C_{1-6}$-alkyl,$C_{1-6}$-Alkanoyloxy-$C_{1-6}$-alkyl, $C_{1-6}$-Alkoxycarbonyloxy-$C_{1-6}$-alkyl, $C_{1-6}$-Alkoxycarbonyl,$C_{1-6}$-Alkoxy oder $C_{1-6}$-Alkylendioxy substituiertes Phenyl ist.

**9.** Verbindungen gemäss Anspruch 1-7, in denen $R^2$ durch einen Rest $L^1$-$T^1$-$L^2$-$T^2$-$L^3$-$T^3$-$L^4$-$T^4$-$L^5$-U substituiertes Phenyl bedeutet, wobei $L^1$ und $L^2$ abwesend oder $C_{1-8}$-Alkylen sind und $L^3$ abwesend ist und U Wasserstoff, $C_{1-6}$-Alkyl, $C_{3-6}$-Cycloalkyl, Phenyl, durch $C_{1-6}$-Alkyl, $C_{1-6}$-Alkoxy, $C_{1-6}$-Alkylthio, $C_{1-6}$-Alkylsulfinyl, $C_{1-6}$-Alkylendioxy, Halogen, Benzoyl-$C_{1-6}$-alkyl, Halogen-$C_{1-6}$alkyl, $C_{1-6}$-Alkanoyloxy oder Hydroxy substituiertes Phenyl; oder Naphthyl; oder Pyridyl, Thienyl, Pyrazinyl, Triazolyl, Imidazolyl, Phenyl-oxadiazolyl, Thienyl-oxadiazolyl, Furyl-oxadiazolyl, Phenyl-oxazolyl, Benzthiazolyl, Furyl, Pyrimidinyl, Nitrobenzthiazolyl Phenyltetrazolyl oder Morpholinyl darstellt.

**10.** Verbindungen gemäss den Ansprüchen 1-7, in denen $R^2$ Phenyl oder Phenyl substituiert durch 2-Benzothiazolylthio-$C_{1-6}$-alkyl, 2-Benzyloxy-3-methoxypropoxy, 2 Benzoyloxy-3-methoxypropoxy, 2,3-Dihydroxypropoxy, 2-Hydroxy-3--benzylamino-propoxy, 2-Hydroxy-3-phenoxypropoxy, 2-Hydroxy-3-phenylthiopropoxy, 2-Methoxy-3-phenoxypropoxy, 2-Methoxy-3-benzyloxypropoxy, 2-Methyl-3-fluor-phenylbutyryloxy-$C_{1-6}$-alkoxy, 2-$C_{2-6}$-Alkenyloxy-4-phenylbutyl, 3,4,5-Trimethoxyphenyl-oxadiazolyl-$C_{1-6}$-alkoxy, 6-Nitro-2-benzothiazolyl-thio-$C_{1-6}$-alkyl, Benzamido-$C_{1-6}$-alkoxy, Benzamido-$C_{1-6}$-alkyl, Benzoyl-$C_{1-6}$-alkoxy und Ketale davon, Benzoyl-$C_{1-6}$-alkyl und Ketale davon, Benzoyl-$C_{1-6}$-alkyl-aminocarbonyl-$C_{1-6}$-alkyl, Benzoyl$C_{1-6}$alkoxycarbonyl-$C_{1-6}$-alkyl, Benzoyl-$C_{1-6}$-alkylaminocarbonyl, Benzoyloxy, Benzoyloxy-$C_{1-6}$-alkyl-benzoyloxy-$C_{1-6}$-alkoxy, Benzoyloxy-$C_{1-6}$-alkoxy, Benzoyloxy-$C_{1-6}$-alkyl, Benzthiazolylhio-$C_{1-6}$-alkoxy, Benzthiazolylthio-$C_{1-6}$-alkyl, Benzylcarbamoyl-$C_{1-6}$-alkoxy, Benzyloxy-$C_{1-6}$ alkylcarbonyloxy-$C_{1-6}$-alkyl, Benzyloxy-$C_{1-6}$-alkoxy, Benzylthio-$C_{1-6}$-alkoxy, Carbamoyloxy-$C_{1-6}$-alkoxy, Carbamoyloxy-$C_{1-6}$-alkyl, Carboxy-$C_{1-6}$-alkoxy, Carboxy-$C_{1-6}$-alkyl, Cyano, Cyano-$C_{1-6}$-alkoxy, Cyano-$C_{1-6}$-alkyl, Cyanophenyl-$C_{1-6}$-alkoxy, Cyclohexylcarbonyloxy-$C_{1-6}$-alkyl, Cyclopropylcarbonyloxy-$C_{1-6}$-alkyl, Cyclopropyloxy-benzyloxy-$C_{1-6}$-alkoxy, Dioxolanyl-$C_{1-6}$-alkoxy, Furyl-oxadiazolyl-$C_{1-6}$-alkoxy, Furoyloxy-$C_{1-6}$-alkoxy, Halophenoxy-$C_{1-6}$-alkyl, Halobenzoyl-$C_{1-6}$-alkoxy, Halobenzoyloxy-$C_{1-6}$-alkyl, Halobenzoyloxy-$C_{1-6}$-alkoxy, Halobenzyloxy-$C_{1-6}$-alkoxy, Halogen, Halogen-$C_{1-6}$-alkyl, Halophenoxy, Halophenyl-oxadiazolyl-$C_{1-6}$-alkoxy, Hydroxy, Hydroxy-benzoyloxy-$C_{1-6}$-alkyl, Hydroxy-benzyloxy-$C_{1-6}$-alkoxy, Hydroxy-$C_{1-6}$-alkoxy, Hydroxy-$C_{1-6}$-alkyl, Imidazolylcarbonyloxy-$C_{1-6}$-alkyl, Methoxybenzoyl-$C_{1-6}$-alkyl, Methoxybenzyl-oxy-$C_{1-6}$-alkoxy, Methylendioxybenzoyl-$C_{1-6}$-alkoxy, Morpholino-$C_{1-6}$-alkoxy, Morpholinocarbonyloxy-$C_{1-6}$-alkoxy, Morpholinocarbonyloxy-$C_{1-6}$-alkyl, N-Methylaminophenyl-carbonyloxy-$C_{1-6}$-alkyl, N-Methyl-benzylamino-$C_{1-6}$-alkoxy, N-Methylpyrrolylcarbonyloxy-$C_{1-6}$-alkoxy, N-$C_{1-6}$-Alkylbenzamido-$C_{1-6}$-alkyl, Naphthyl-$C_{1-6}$-alkoxy, Nicotinoyloxy-$C_{1-6}$-alkoxy, Nicotinoyloxy-$C_{1-6}$-alkyl, $C_{1-6}$-Alkanoylbenzoyloxy-$C_{1-6}$-alkyl, $C_{1-6}$-alkanoyloxy-$C_{1-6}$-alkoxy, $C_{1-6}$-Alkanoyloxy- $C_{1-6}$-alkyl, $C_{1-6}$-Alkenyl-benzyloxy-$C_{1-6}$-alkoxy, $C_{1-6}$-Alkenyloxy, $C_{1-6}$-Alkenyloxybenzyloxy-$C_{1-6}$-alkoxy,$C_{1-6}$-Alkoxy, $C_{1-6}$-Alkoxy-benzoyloxy-$C_{1-6}$-alkyl,$C_{1-6}$-Alkoxy-carbonyl, $C_{1-6}$-Alkoxy-$C_{1-6}$-alkyl, $C_{1-6}$-Alkoxybenzoylamino-$C_{1-6}$-alkyl, $C_{1-6}$-Alkoxybenzylcarbonyloxy-$C_{1-6}$-alkyl, $C_{1-6}$-Alkoxy-benzyloxy-$C_{1-6}$-alkoxy, $C_{1-6}$-Alkoxy-benzylthio-$C_{1-6}$-alkoxy, $C_{1-6}$-Alkoxycarbonyl, $C_{1-6}$-Alkoxycarbonyl-$C_{1-6}$-alkoxy, $C_{1-6}$-Alkoxycarbonyl-$C_{1-6}$-alkyl,$C_{1-6}$-Alkoxyphenyl-oxadiazolyl-$C_{1-6}$-alkoxy, $C_{1-6}$-Alkyl, $C_{1-6}$-Alkylbenzyloxy-$C_{1-6}$-alkoxy, $C_{1-6}$-Alkylendioxy, $C_{1-6}$-Alkylendioxybenzyloxy-$C_{1-6}$-alkoxy, $C_{1-6}$-Alkylsulfonylbenzoyl-$C_{1-6}$-alkoxy, $C_{1-6}$-Alkylthiobenzoyloxy-$C_{1-6}$-alkoxy, $C_{1-6}$ Alkylthio-benzyloxy-$C_{1-6}$-alkoxy, Benzoyloxybenzyl-$C_{1-6}$-alkoxy, Hydroxybenzyl-$C_{1-6}$-alkoxy, $C_{1-6}$-Alkoxybenzyl-$C_{1-6}$-alkoxy, $C_{1-6}$-Alkoxybenzylcarbonyloxy-alkoxy, Phenoxy-benzyloxy-$C_{1-6}$-alkoxy, Phenoxycarbonyl-$C_{1-6}$-alkyl, Phenoxy-$C_{1-6}$-alkenyloxy, Phenoxy-$C_{1-6}$-alkinyloxy, Phenyl-$C_{1-6}$-alkanoylamino-$C_{1-6}$-alkyl, Phenyl-$C_{1-6}$-alkenyloxy, Phenyl-$C_{1-6}$-alkoxy, Phenoxy-$C_{1-6}$-alkyl, Phenyl-$C_{1-6}$-alkylaminocarbonyl, Phenoxy-$C_{1-6}$-alkylcarbonyl-$C_{1-6}$-alkoxy, Phenyl-$C_{1-6}$-alkylaminocarbonyl-$C_{1-6}$-alkyl, Phenylaminocarbonyloxy-$C_{1-6}$-alkoxy, Phenylaminocarbonyloxy-$C_{1-6}$-alkyl, Phenyl-hydroxy-$C_{1-6}$-alkyl, Phenyl-oxadiazolyl-$C_{1-6}$-alkoxy, Phenyl-oxadiazolyl-$C_{1-6}$-alkoxy, Phenyl-oxadiazolyl-$C_{1-6}$-alkyl, Phenyl-oxazolyl-$C_{1-6}$-alkoxy, Phenyloxy-$C_{1-6}$-alkoxy, Phenylsulfamoyl-$C_{1-6}$-alkyl, Phenylsulfinyl-$C_{1-6}$-alkyl, Phenylsulfonyl-$C_{1-6}$-alkoxy, Phenylsulfonyl-$C_{1-6}$alkyl, Phenyltetrazolyl-thio-$C_{1-6}$-alkyl, Phenylthio-$C_{1-6}$-alkoxy, Phenylthio-$C_{1-6}$-alkyl, Pyrazinylcarbonyloxy-$C_{1-6}$-alkyl, Pyridylaminocarbonyloxy-$C_{1-6}$-alkoxy, Pyridylaminocarbonyloxy-$C_{1-6}$-alkyl, Pyridylcarbamoyloxy, Pyridyl-$C_{1-6}$-alkoxy-$C_{1-6}$-alkoxy, Pyridyl-$C_{1-6}$-alkoxy-$C_{1-6}$-alkyl, Pyridyl-oxadiazolyl-$C_{1-6}$-alkoxy, Pyridylthio-$C_{1-6}$-alkyl, Pyrimidinyloxy-$C_{1-6}$-alkoxy, Pyrimidinylthio-$C_{1-6}$-alkyl, Thenoyloxy-$C_{1-6}$-alkoxy, Thenoyloxy-$C_{1-6}$-alkyl, Thienyl-oxadiazolyl-$C_{1-6}$-alkoxy, Triazolyl-$C_{1-6}$-alkoxy, Trifluormethylbenzyloxy-$C_{1-6}$-alkoxy oder Trifluormethyl ist.

**11.** Verbindungen gemäss den Ansprüchen 1-7, in denen $R^2$ Cyclohexyl oder Benzoyloxycyclohexyl ist.

**12.** Verbindungen gemäss den Ansprüchen 1-7, in denen $R^2$ Naphthyl, Tetrahydronaphthyl oder Acenaphthyl ist.

**13.** Verbindungen gemäss den Ansprüchen 1-7, in denen $R^2$ Pyridyl oder Oxopyridyl, oder durch 3-H-2-Thioxo-benzthiazolyl, $C_{1-6}$-Alkoxyphenyl-$C_{1-6}$-alkoxy-$C_{1-6}$-alkoxy, Phenyl-$C_{1-6}$-alkoxy-$C_{1-6}$-alkoxy, Phenyl-$C_{1-6}$-alkyl, Cyclohexyl-$C_{1-6}$-alkoxy, Phenoxy-$C_{1-6}$-alkyl oder Phenyl-$C_{1-6}$-alkoxy-$C_{1-6}$-alkyl substituiertes Pyridyl oder Oxopyridyl ist.

**14.** Verbindungen gemäss den Ansprüchen 1-7, in denen $R^2$ Pyrimidinyl oder durch Benzodioxanyl-$C_{1-6}$-alkoxy, Biphenylyloxy, Biphenylyl-$C_{1-6}$-alkoxy, Cyclohexyl-$C_{1-6}$-alkoxy, Cyclohexyloxy-$C_{1-6}$-alkoxy, Halophenyl-$C_{1-6}$-alkoxy, Halophenyloxadiazolyl-$C_{1-6}$-alkoxy, Indanyl-$C_{1-6}$-alkoxy, Naphthyl-$C_{1-6}$-alkoxy, Phenyl-$C_{1-6}$-alkyl, N-$C_{1-6}$-Alkylphenyl-$C_{1-6}$-alkoxy-$C_{1-6}$-alkylamino, N-$C_{1-6}$-Alkyl-phenyl-$C_{1-6}$-alkylamino, $C_{1-6}$-Alkythio, $C_{1-6}$-Alkoxy, $C_{1-6}$-Alkoxyphenyl-$C_{1-6}$-alkoxy-$C_{1-6}$-alkoxy, $C_{1-6}$-Alkoxyphenyl-$C_{1-6}$-alkylamino, $C_{1-6}$-Alkylphenyl-$C_{1-6}$-alkylamino, Halophenyl-$C_{1-6}$-alkylamino, Halophenoxy-$C_{1-6}$-alkoxy, $C_{1-6}$-Alkylpyridyl-$C_{1-6}$-alkoxy, Phenyl-$C_{1-6}$-alkoxy-$C_{1-6}$-alkoxy, Phenyl-$C_{1-6}$-alkoxy-$C_{1-6}$-alkylthio, Phenyl-$C_{1-6}$-alkoxy-$C_{1-6}$-alkylamino, Phenoxy-phenyl-$C_{1-6}$-alkoxy, Phenoxyphenoxy, Phenyl-$C_{1-6}$-alkinyloxy, Phenyl-$C_{1-6}$-alkoxy-$C_{1-6}$-alkoxy, Phenylthio-$C_{1-6}$-alkoxy, Phenyl-oxazolyl-$C_{1-6}$-alkoxy, Phenyl-$C_{1-6}$-alkinyloxy, Phenyl-$C_{1-6}$-alkenyloxy, Phenyl-$C_{1-6}$-alkylamino, Phenyl-pyridyl-$C_{1-6}$-alkoxy oder Phenylpyridyl-$C_{1-6}$-alkylaminosubstituiertes Pyrimidinyl ist.

**15.** Verbindungen gemäss den Ansprüchen 1-7, in denen $R^2$ Halobenzoyl-$C_{1-6}$-alkyl-triazolyl, Phenyl-$C_{1-6}$-alkoxy-$C_{1-6}$-alkyl-triazolyl oder Phenyl-$C_{1-6}$-alkoxy-$C_{1-6}$-alkoxy-$C_{1-6}$-alkyl-triazolyl ist.

**16.** Verbindungen gemäss den Ansprüchen 1-7, in denen $R^4$ 2-Oxo-imidazolidin-1-yl-$C_{1-6}$-alkyl, 4-Hydroxy-piperidin-1-yl-$C_{1-6}$-alkoxy, 4-Hydroxy-piperidin-1-yl-$C_{1-6}$-alkoxy-$C_{1-6}$-alkyl, 4-Methyl-piperazin-1-yl-$C_{1-6}$-alkoxy, 4-Methylpiperazin-1-yl-$C_{1-6}$-alkoxy-$C_{1-6}$-alkyl, 4-Methyl-piperazin-1-yl-$C_{1-6}$-alkyl-carbamoyloxy-$C_{1-6}$-alkyl, 1,2,4-Triazolyl-$C_{1-6}$-alkyl, Amino, Amino-$C_{1-6}$-alkyl, Amino-$C_{1-6}$-alkyl-amino, Amino-$C_{1-6}$-alkyl-amino-$C_{1-6}$-alkyl, Amino-$C_{1-6}$-alkoxyl, Amino-$C_{1-6}$-alkoxyl-$C_{1-6}$-alkyl, Aminocarbonyloxy-$C_{1-6}$-alkyl, Benzyloxy oder durch $C_{1-6}$-Alkyl, $C_{1-6}$-Alkenyl, $C_{1-6}$-Alkoxy, Trifluormethoxy, $C_{1-6}$-Alkylthio, Hydroxy oder Halogen substituiertes Benzyloxy, Benzyloxy-$C_{1-6}$-alkyl oder durch $C_{1-6}$-Alkyl, $C_{1-6}$-Alkenyl, $C_{1-6}$-Alkoxy oder Halogen substituiertes Benzyloxy, Carbamoyloxy-$C_{1-6}$-alkyl, Cyano-$C_{1-6}$-alkyl, Di-$C_{1-6}$-Alkyl-amino, Di-$C_{1-6}$-Alkyl-amino-$C_{1-6}$-alkyl, Di-$C_{1-6}$-Alkyl-amino-$C_{1-6}$-alkyl-(N-$C_{1-6}$-alkyl)-amino-$C_{1-6}$-alkyl, Di-$C_{1-6}$-Alkyl-amino-$C_{1-6}$-alkyl-amino Di-$C_{1-6}$-Alkyl-amino-$C_{1-6}$-alkyl-amino-$C_{1-6}$-alkyl, Di-$C_{1-6}$-Alkyl-ammo-$C_{1-6}$-alkoxyl Di-$C_{1-6}$-Alkyl-amino-$C_{1-6}$-alkoxyl-$C_{1-6}$-alkyl, Dihydroxy-$C_{1-6}$-alkoxy, Dihydroxy-$C_{1-6}$- alkoxy-$C_{1-6}$-alkyl, Dihydroxy-$C_{1-6}$-alkyl-amino, Dihydroxy-$C_{1-6}$-alkyl-amino-$C_{1-6}$-alkyl, Guanidinyl-$C_{1-6}$-alkoxy-$C_{1-6}$-alkyl, Guanidinyl-$C_{1-6}$-alkyl, Hydroxy, Hydroxy-$C_{1-6}$-alkyl, Sulfooxy-$C_{1-6}$-alkyl,Hydroxy-$C_{1-6}$-alkoxyl, Hydroxy-$C_{1-6}$-alkoxyl-$C_{1-6}$-alkyl, Morpholin-4-yl-$C_{1-6}$-alkoxy, Morpholin-4-yl-$C_{1-6}$-alkoxy-$C_{1-6}$-alkyl, Morpholin-4-yl-$C_{1-6}$-alkyl-carbamoyloxy-$C_{1-6}$-alkyl, Naphthyl-alkoxy oder durch $C_{1-6}$-Alkoxy substituiertes Naphthyl-alkoxy, $C_{1-6}$-Alkoxy, $C_{1-6}$-Alkoxy-$C_{1-6}$-alkoxy $C_{1-6}$-Alkoxy-$C_{1-6}$-alkoxy-$C_{1-6}$-alkyl, $C_{1-6}$-Alkoxy-$C_{1-6}$-alkyl, $C_{1-6}$-Alkyl, $C_{1-6}$-Alkylsulfonylamino-$C_{1-6}$-alkyl, Phenoxy-$C_{1-6}$-alkyl oder durch $C_{1-6}$-Alkyl, $C_{1-6}$-Alkoxy substituiertes Phenoxy-$C_{1-6}$-alkyl, Phenyl-thio-$C_{1-6}$-alkyl oder durch $C_{1-6}$-Alkyl, $C_{1-6}$-Alkoxy substituiertes Phenyl-thio-$C_{1-6}$-alkyl, Piperazin-4-yl-$C_{1-6}$-alkoxy, Piperazin-4-yl-$C_{1-6}$-alkoxy-$C_{1-6}$-alkyl, Piperidin-1-yl-$C_{1-6}$-alkyl-carbamoyloxy-$C_{1-6}$-alkyl, Piperidin-4-yl-$C_{1-6}$-alkoxy, Piperidin-4-yl-$C_{1-6}$-alkoxy-$C_{1-6}$-alkyl, Pyridyl-$C_{1-6}$-alkoxyl, Pyridyl-$C_{1-6}$-alkoxy-$C_{1-6}$-alkyl, Pyridylthio-$C_{1-6}$-alkyl, Pyrimidinyloxy-$C_{1-6}$-alkyl oder durch $C_{1-6}$-Alkoxy substituiertes Pyrimidinyloxy-$C_{1-6}$-alkyl, Tetrazolyl-$C_{1-6}$-alkyl, Trifluormethylsulfonylamino-$C_{1-6}$-alkyl oder Wasserstoffist.

**17.** Die Verbindungen gemäss Anspruch 1-16,

4-[2-[7-[(3R,4R)-4-[4-(3-Benzyloxy-propoxy)-phenyl]-piperidin-3-yloxymethyl]-naphthalin-2-yloxy]-äthyl]-morpholin,
(R)-3-[7-[(3R,4R)-4-[4-(3-Benzyloxy-propoxy)-phenyl]-piperidin-3-yloxymethyl]-naphthalin-2-yloxy]-propan-1,2-diol,
(S)-3-[7-[(3R,4R)-4-[4-(3-Benzyloxy-propoxy)-phenyl]-piperidin-3-yloxymethyl]-naphthalin-2-yloxyl-propan-1,2-diol,
(R)-3-[2-[7-[(3R,4R)-4-[4-(3-Benzyloxy-propoxy)-phenyl]-piperidin-3-yloxymethyl]-naphthalin-2-yloxy]-äthoxy]-propan-1,2-diol,
(S)-3-[2-[7-[(3R,4R)-4-[4-(3-Benzyloxy-propoxy)-phenyl]-piperidin-3-yloxymethyl]-naphthalin-2-yloxy]-äthoxy]-propan-1,2-diol,
1-[2-[7-[(3R,4R)-4-[4-(3-Benzyloxy-propoxy)-phenyl]-piperidin-3-yloxymethyl]-naphthalin-2-yloxy]-äthyl]-4-methyl-piperazin,
1-[(3R,4S-4-[4-(3-Benzyloxy-propoxy)-phenyl]-piperidin-3-yl]-2-naphthalin-2-yl-äthanon,
(3R,4S,5S)-4-[4-(3-Benzyloxy-propoxy)-phenyl]-3-(1,4-dimethoxy-naphthalin-2-ylmethoxy)-piperidin-5-ol,

3R,4R)-4-[4-(3-Benzyloxy-propoxy)-phenyl]-3-[7-[(R)-2,3-dihydroxypropoxymethyl]-naphthalin-2-ylmethoxy]-piperidin,
(3R,4R)-4-[4-(3-Benzyloxy-propoxy)-phenyl]-3-[7-[ (S)-2,3-dihydroxypropoxymethyl]-naphthalin-2-ylmethoxy]-piperidin,
(3R,4R)-4-[4-(3-Benzyloxy-propoxy)-phenyl]-3-[6-[(R)-2,3-dihydroxypropoxymethyl]-naphthalin-2-ylmethoxy]-piperidin,
(3R,4R)-4-[4-(3-Benzyloxy-propoxy)-phenyl]-3-[6-[(S)-2,3-dihydroxypropoxymethyl]-naphthalin-2-ylmethoxy]-piperidin,
4-[(3R,4S,5S)-4-[4-(3-Benzyloxy-propoxy)-phenyl]-3-(naphthalin-2-ylmethoxy)-piperidin-5-yloxy] -butan-1-ol,
3-[(3R,4S,5S)-4-[4-(3-Benzyloxy-propoxy)-phenyl]-3-(naphthalin-2-ylmethoxy)-piperidin-5-yloxy]-propan-1-ol,
1-{2-[(3R,4R,5S)-4-[4-(3-Benzyloxy-propoxy)-phenyl]-3-(naphthalin-2-ylmethoxy)-piperidin-5-yloxy]-äthyl}-4-methyl-piperazin,
(3R,4R,5R)-4-[2-[4-[4(3-Benzyloxy-propoxy)-phenyl]-5-(naphthalin-2-ylmethoxy)-piperidin-3-ylmethoxy]-äthyl}-morpholin,
(3R,4S,5S)-4-[4-(3-Benzyloxy-propoxy)-phenyl]-3-(4-methoxy-benzyloxy)-piperidin-5-ol,
(3R,4S,5S)-4-[4-(3-Benzyloxy-propoxy)-phenyl]-3,5-bis-(4-methoxy-benzyloxy)-piperidin,
(3S,4R,5R)-4-[2-[4-[4-(3-Benzyloxy-propoxy)-phenyl)-5-(naphthalin-2-ylmethoxy)-piperidin-3-ylmethoxy]-äthyl]-morpholin,
(3S,4R,5R)-4-[4-(3-Benzyloxy-propoxy)-phenyl]-3-methoxymethyl-5-(naphthalin-2-ylmethoxy)-piperidin,
[3-(4-Methyl-piperazin-1-yl)-propyl]-carbaminsäure (3S,4R,5R)-4-[4-(3-benzyloxypropoxy)-phenyl]-5-(naphthalin-2-ylmethoxy)-piperidin-3-ylmethylester,
(3S,4R,5R)-4-[4-[4-(3-Benzyloxy-propoxy)-phenyl]-5-(naphthalin-2-ylmethoxy)-piperidin-3-ylmethylsulfanyl] -pyridin,
2-(4-Cyclohexyl-butoxy)-5-[(3R,4R)-3-(1,4-dimethoxy-naphthalin-2-ylmethoxy)-piperidin-4-yl]-pyrimidin,
(3 R,4'R)-6-(3-Cyclohexyl-propoxy)-3'-(1,4-dimethoxy-naphthalin-2-ylmethoxy)-1',2',3',4',5',6'-hexahydro-[3,4']bipyridin,
( 3S,4R,5R)-[4-[4-(3-Benzyloxy-propoxy)-phenyl]-5-(naphthalin-2-ylmethoxy)-piperidin-3-yl]-methanol,
(3S,4R,5R)-N-[4-[4-(3-Benzyloxy-propoxy)-phenyl]-5-(naphthalin-2-ylmethoxy)-piperidin-3-ylmethyl]-N,N',N'-trimethyl-äthan-1,2-diamin,
(3S,4R,5R)-[4-[4-(3-Benzyloxy-propoxy)-phenyl]-5-(naphthalin-2-ylmethoxy)-piperidin-3-ylmethyl]-diäthylamin,
1-[(3R,4S,5S)-4-[4-(3-Benzyloxy-propoxy)-phenyl]-5-(2-morpholin-4-yl-äthoxymethyl)-piperidin-3-yl]-2-naphthalin-2-yl-äthanon,
(3R,4R)-3-(1,4-Dimethoxy-naphthalin-2-ylmethoxy)-4-[4-[3-(2-methoxyphenoxy)-propoxy]-phenyl]-piperidin,
(3R,4s,5S)-4-[4-(3-Benzyloxy-propoxy)-phenyl]-3,5-bis-(3,4,5-trimethoxybenzyloxy)-piperidin,
(3R,4R,5S)-4-[4-(3-Benzyloxy-propoxy)-phenyl]-3-(naphthalin-2-ylmethoxy)-5-[1,2,4]triazol-1-ylmethyl-piperidin,
(3R,4R)-4-[4-[3-(2-Methoxy-benzyloxy)-propoxy]-phenyl]-3-(quinolin-7-ylmethoxy)-piperidin,
2-(7-{(3R,4R)-4-[4-(3-Benzyloxy-propoxy)-phenyl]-piperidin-3-yloxymethyl}naphthalin-2-ylmethoxy)-äthanol,
7-{(3R,4R)-4-[4-(3-Benzyloxy-propoxy)-phenyl]-piperidin-3-yloxymethyl}-naphthalin-2-ylmethyl)-dimethylamin,
(3R,4R)-3-(4-Benzyloxy-naphthalin-2-ylmethoxy)-4-(4-fluor-phenyl)-piperidin
(3'R,4'R)-3'-(1,4-Dimethoxy-naphthalin-2-ylmethoxy)-6-[3-(2-methoxybenzyloxy)-propoxy]-1',2',3',4',5',6'-hexahydro-[3,4']bipyridin,
(3R,4R)-3-(1,4-Dimethoxy-naphthalin-2-ylmethoxy)-4-[4-[3-(2-methoxybenzyloxy)-propoxy]-phenyl]-piperidin,
(3S,4R,5R)-1-[4-[4-(3-Benzyloxy-propoxy)-phenyl]-5-(naphthalin-2-ylmethoxy)-piperidin-3-ylmethyl]-imidazolidin-2-on,
(3R,4R)-4-[4-[3-(2-Methoxy-benzyloxy)-propoxy]-phenyl]-3-(2-oxo-1,2-dihydrochinolin-7-ylmethoxy)-piperidin,
(3R,4R)-3-(Isochinolin-7-ylmethoxy)-4-[4-[3-(2-methoxy-benzyloxy)-propoxy]-phenyl]-piperidin,
(3R,4R)-4-[4-[3-(2-Methoxy-benzyloxy)-propoxy]-phenyl]-3-(1,2,3,4-tetrahydrochinolin-7-ylmethoxy)-piperidin,
1-[2-[7-[(3R,4R)-4-[4-[3-(2-Methoxy-benzyloxy)-propoxy]-piperidin-3-yloxymethyl]-naphthalin-2-yloxy]-äthyl]-4-methyl-piperazin,
1-[2-[7-[(3R,4S,5S)-5-Hydroxy-4-[4-[-3-(2-methoxy-benzyloxy)-propoxy]-piperidin-3-yloxymethyl]-naphthalin-2-yloxy]-äthyl]-4-methyl-piperazin,

(3R,4S,5S)-3-(1,4-Dimethoxy-naphthalin-2-ylmethoxy)-4-[4-[3-(2-methoxybenzyloxy)-propoxy]-phenyl]-piperidin-5-ol und
(3R,4R,5S)-3-(1,4-Dimethoxy-naphthalin-2-ylmethoxy)-4-[4-[3-(2-methoxybenzyloxy)-propoxy]-phenyl}-5-(1H-tetrazol-5-ylmethyl)-piperidin.

**18.** Verbindungen der Formel II

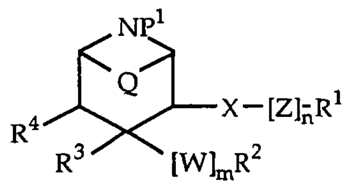

II

in der $P^1$ eine Schutzgruppe darstellt und die übrigen Symbole die in Anspruch 1 angegebene Bedeutung haben und in $R^1$, $R^2$ und $R^4$ gegebenenfalls enthaltene Hydroxygruppen in geschützter Form vorliegen können.

**19.** Arzneimittel, insbesondere zur Bekämpfung bzw. Verhütung von Bluthochdruck, Herz- und Niereninsuffizienz, enthaltend eine Verbindung der Formel I von Anspruch 1 und ein therapeutisch inertes Excipiens.

**20.** Verfahren zur Herstellung einer Verbindung gemäss einem der Ansprüche 1-17, **dadurch gekennzeichnet, dass** man aus einer Verbindung der Formel II

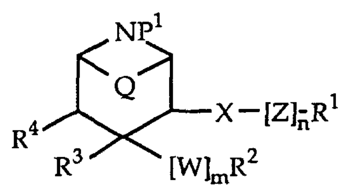

II

in der $P^1$ eine Schutzgruppe darstellt und die übrigen Symbole die in Anspruch 1 angegebene Bedeutung haben und in $R^1$, $R^2$ und $R^4$ gegebenenfalls enthaltene Hydroxygruppen in geschützter Form vorliegen können, die Schutzgruppe(n) abspaltet, gewünschtenfalls in der so erhaltenen Verbindung der Formel I reaktionsfähige Gruppe funktionell abwandelt und/oder die Verbindung der Formel I in ein pharmazeutisch anwendbares Salz überführt.

**21.** Verbindungen gemäss einem der Ansprüche 1-20 zur Anwendung als therapeutische Wirkstoffe.

**22.** Verwendung von Verbindungen der Formel I von Anspruch 1 bei der Herstellung von Arzneimitteln zur Behandlung von Bluthochdruck, Herz- und Niereninsuffizienz.

## Claims

**1.** Piperidine derivatives having the general formula I

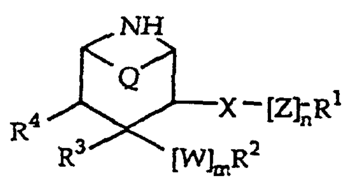

I

wherein

$R^1$ denotes phenyl or phenyl substituted by $C_{1-6}$-alkyl, $C_{1-6}$-alkenyl, $C_{1-6}$-alkoxy, $C_{1-6}$-alkylthio, halogen, hy-

droxy, hydroxy-$C_{1-6}$-alkoxy, $C_{1-6}$-alkoxy-$C_{1-6}$-alkoxy, $C_{1-6}$-alkylsulfinyl, $C_{1-6}$-alkylsulfonyl, cyano, trifluoromethyl, trifluoromethoxy, carboxy, cyclobutylmethoxy-$C_{1-6}$-alkyl, $C_{1-6}$-alkylenedioxy, phenyl, phenoxy, $C_{1-6}$-alkoxycarbonylphenyl, hydroxy-$C_{1-6}$-alkylphenyl, 2,3-dihydroxypropylaminocarbonylphenyl, benzyloxy, benzoyl, pyridyl-$C_{1-6}$-alkoxy-$C_{1-6}$-alkyl or nicotinoylamino-$C_{1-6}$-alkyl; or naphthyl or naphthyl substituted by hydroxy, oxo, $C_{1-6}$-alkoxyl $C_{1-6}$-alkenyloxy, $C_{1-6}$-alkoxy-$C_{1-6}$-alkoxy, di-$C_{1-6}$-alkylamino, 2, 3-dihydroxypropoxy, 2,3-dihydroxypropoxy-$C_{1-6}$-alkoxy, 2,3-dimethoxypropoxy, $C_{1-6}$-alkoxycarbonyl-$C_{1-6}$-alkoxy, carbamoyl-$C_{1-6}$-alkoxy, methoxybenzyloxy, hydroxybenzyloxy, phenethyloxy, methylenedioxybenzyloxy, dioxolanyl-$C_{1-6}$-alkoxy, cyclopropyl-$C_{1-6}$-alkoxy, hydroxy-$C_{1-6}$-alkoxy, carbamoyloxy-$C_{1-6}$-alkoxy, pyridyl-carbamoyloxy-$C_{1-6}$-alkoxy, morpholino-$C_{1-6}$-alkoxy, 3-morpholino-2-hydroxypropoxy, N-methylpiperazino-N-$C_{1-6}$-alkoxy, benzoyloxy-$C_{1-6}$-alkoxy or picolyloxy; or tetrahydronaphthyl or indanyl subtituted by methyl; or pyridyl, benzimidazolyl, Di-$C_{1-6}$-alkoxypyrimidinyl or 2- or 5-benzo[b]thienyl, 6- or 7-quinolyl, 6- or 7-isoquinolyl, 6- or 7-tetrahydroquinolyl, 6- or 7-tetrahydroisoquinolinyl, 6-quinoxalinyl, 6- or 7-quinazolinyl or 6- or 7-quinolinyl, 6- or 7-isoquinolinyl, 6- or 7-tetrahydroquinolinyl, 6- or 7-tetrahydroisoquinolinyl, 6-quinoxalinyl or 6- or 7-quinazolinyl substituted by hydroxy, oxo, $C_{1-6}$-alkoxy, $C_{1-6}$-alkenyloxy, $C_{1-6}$-alkoxy-$C_{1-6}$-alkoxy, di-$C_{1-6}$-alkylamino, 2, 3-dihydroxypropoxy, 2,3-dihydroxypropoxy-$C_{1-6}$-alkoxy, 2,3-dimethoxypropoxy, $C_{1-6}$-alkoxycarbonyl-$C_{1-6}$-alkoxy, carbamoyl-$C_{1-6}$-alkoxy, methoxybenzyloxy, hydroxybenzyloxy, phenethyloxy, methylenedioxybenzyloxy, dioxolanyl-$C_{1-6}$-alkoxy, cyclopropyl-$C_{1-6}$-alkoxy, hydroxy-$C_{1-6}$-alkoxy, carbamoyloxy-$C_{1-6}$-alkoxy, pyridyl-carbamoyloxy-$C_{1-6}$-alkoxy, morpholino-$C_{1-6}$-alkoxy, 3-morpholino-2-hydroxypropoxy, N-methylpiperazino-N-$C_{1-6}$-alkoxy, benzoyloxy-$C_{1-6}$-alkoxy or picolyloxy;

$R^2$ denotes phenyl, naphthyl, acenaphthyl, cyclohexyl, pyridyl, pyrimidinyl, pyrazinyl, oxo-pyridinyl, diazinyl, triazolyl, thienyl, oxazolyl, oxadiazolyl, thiazolyl, pyrrolyl or furyl, the said radicals optionally being substituted by 1-3 halogen, hydroxy, cyano, trifluoromethyl, $C_{1-6}$-alkyl, halo-$C_{1-6}$-alkyl, hydroxy-$C_{1-6}$-alkyl, $C_{1-6}$-alkoxy-$C_{1-6}$-alkyl, cyano-$C_{1-6}$-alkyl, carboxy-$C_{1-6}$-alkyl, $C_{1-6}$-alkanoyloxy-$C_{1-6}$-alkyl, $C_{1-6}$-alkoxycarbonyloxy-$C_{1-6}$-alkyl, $C_{1-6}$-alkoxycarbonyl or $C_{1-6}$-alkoxy groups, or a $C_{1-6}$-alkylenedioxy group and/or by a radical $L^1$-$T^1$-$L^2$-$T^2$-$L^3$-$T^3$-$L^4$-$T^4$-$L^5$-U;

$L^1$, $L^2$, $L^3$, $L^4$ and $L^5$ independently of one another denote a bond, $C_{1-8}$-alkylene, $C_{2-8}$-alkenylene or $C_{2-8}$ alkinylene, or are absent;

$T^1$, $T^2$, $T^3$ and $T^4$ independently of one another denote (a) a bond, or are absent, or denote one of the groups (b) -CH(OH)-; (c) -CR($OR^6$)-; (d) -CH($NR^5R^6$)-; (e) -CO-; (f) -$CR^7R^8$-; (g) -O- or -$NR^6$-, (h) -$S(O)_{0-2}$-; (i) -$SO_2NR^6$-; (j) -$NR^6SO_2$-; (k) -$CONR^6$-; (l) -$NR^6$CO-; (m) -O-CO-; (n) -CO-O-; (o) -O-CO-O-; (p) -O-CO-$NR^6$-; (q) -N($R^6$)-CO-N($R^6$)- or (r) -N($R^6$)-CO-O-, wherein the bonds starting from (b), (d), (e) and (g) - (r) lead to a C atom in the neighbouring group and the said C atom is saturated if the bond starts from a heteroatom, and wherein not more than two groups (b)-(f), three groups (g)-(h) and one group (i)-(p) are present;

$R^3$ denotes hydrogen, hydroxy, $C_{1-6}$-alkoxy or $C_{1-6}$-alkenyloxy;

$R^4$ denotes hydrogen, $C_{1-6}$-alkyl, $C_{1-6}$-alkenyl, $C_{1-6}$-alkoxy, hydroxy-$C_{1-6}$-alkyl, $C_{1-6}$-alkoxy-$C_{1-6}$-alkyl, benzyl, oxo or a group $R^{4a}$ - $Z^1$-$X^1$, wherein

$R^{4a}$ denotes a group (a) H-; (b) $C_{1-6}$-alkyl-; (c) $C_{1-6}$-alkenyl-; (d) hydroxy-$C_{1-6}$-alkyl; (e) polyhydroxy-$C_{1-6}$-alkyl-; (f) $C_{1-6}$-alkyl-O-$C_{1-6}$-alkyl-; (g) aryl-; (h) heterocyclyl-; (i) aryl-$C_{1-6}$-alkyl-; (j) heterocyclyl-$C_{1-6}$-alkyl-; (k) aryloxy-$C_{1-6}$-alkyl-; (l) heterocyclyloxyl-$C_{1-6}$-alkyl-; (m) ($R^5$, $R^6$) -N- $(CH_2)_{1-3}$-; (n) ($R^5$, $R^6$)-N-; (o) $C_{1-6}$-alkyl-S $(O)_{0-2}$-; (p) aryl-$S(O)_{0-2}$-; (q) heterocyclyl-$S(O)_{0-2}$-; (r) HO-$SO_3$- or salts thereof; (s) $H_2$N-C(NR)-NH-; or (t) NC- and the bonds starting from (n) - (t) lead to a C atom in the neighbouring group and the said C atom is saturated if the bond starts from a heteroatom;

$Z^1$ denotes (a) a bond, is absent, or denotes one of the groups (b) $C_{1-6}$-alkylene-; (c) $C_{1-6}$-alkenylene-; (d)-O-, -N($R^{11}$)-, -$S(O)_{0-2}$-; (e) -CO-; (f) -O-CO-; (g) -O-CO-O-; (h) -O-CO-N($R^{11}$)-; (i) -N($R^{11}$)-CO-O-; (j) -CO-N($R^{11}$)-; (k) -N($R^{11}$ )-CO-; (l) -N($R^{11}$)-CO-N($R^{11}$)- or (m) -CH($OR^9$)- and the bonds starting from (d) and (f) - (m) lead to a C atom in the neighbouring group and the said C atom is saturated if the bond starts from a heteroatom;

$X^1$ (a) denotes a bond or is absent or denotes one of the groups (b) -O-; (c) -N($R^{11}$ )-; (d) -$S(O)_{0-2}$-; or (e) $-CH_2)_{1-3}$; or

$R^3$ and $R^4$ together denote a bond;

$R^5$ and $R^6$ denote hydrogen, $C_{1-6}$-alkyl, $C_{1-6}$-alkenyl, aryl-$C_{1-6}$-alkyl or acyl, or together with the N atom to which they are bonded denote a 5- or 6-member heterocyclic ring which can contain an additional N-, O- or S- atom or an -SO- or -$SO_2$- group, wherein the additional N atom can if required be substituted by $C_{1-6}$-alkyl radicals;

$R^7$ and $R^8$ together with the C atom to which they are bonded denote a 3-7 member ring which can contain one or more -O- or -S- atoms or -SO- or -$SO_2$- groups;

$R^9$ denotes hydrogen, $C_{1-6}$-alkyl, acyl or aryl-$C_{1-6}$-alkyl;

$R^{10}$ denotes carboxy-$C_{1-6}$-alkyl, $C_{1-6}$-alkoxycarbonyl-$C_{1-6}$-alkyl, $C_{1-6}$-alkyl or hydrogen;

$R^{11}$ denotes hydrogen or $C_{1-6}$-alkyl;

U denotes hydrogen, $C_{1-6}$-alkyl, $C_{3-6}$-cycloalkyl, cyano, optionally substituted $C_{3-6}$-cycloalkyl, aryl or heterocyclyl;

Q denotes ethylene or is absent;

X denotes a bond, oxygen, sulphur or a group -CHOR$^9$-, -O-CO, -CO- or C=NOR$^{10}$-, wherein the bond starting from an oxygen or sulphur atom leads to a saturated C atom in the group Z or to $R^1$;

W denotes oxygen or sulphur;

Z denotes $C_{1-6}$-alkylene, $C_{1-6}$-alkenylene, hydroxy-$C_{1-6}$-alkylidene, -alk-O- or -alk-S-, wherein alk denotes $C_{1-6}$-alkylene; and wherein if X is a bond, Z denotes $C_{1-6}$-alkenylene;

n is 1 or if X denotes -O-CO-, is 0 or 1;

m is 0 or 1;

and pharmaceutically usable salts thereof
except for the compounds 4-(4-fluorophenyl)-3-(3,4-methylenedioxybenzyloxy)piperidine, the hydrochloride thereof and trans-3-(3-chlorophenoxy)-4-(3,4-dimethyl-phenoxy)piperidine or pharmaceutically compatible salts thereof and
"acyl" denotes $C_{1-6}$-alkanoyl radicals or benzoyl;
"aryl" alone or in compounds denotes phenyl, naphthyl or tetrahydronaphthyl, these groups being substitutable if required one or more times by $C_{1-6}$-alkyl, trifuloromethyl, nitro, amino, $C_{1-6}$-alkenyl, $C_{1-6}$-alkoxy, $C_{1-6}$-alkylcarbonyloxy, hydroxy, halogen, cyano, carbamoyl, $C_{1-6}$-alkylenedioxy, phenyl substituted if required by halogen, $C_{1-6}$-alkyl, $C_{1-6}$-alkoxy or dihydroxy-$C_{1-6}$-alkylaminocarbonyl, or by similarly substituted phenoxy, phenylthio, phenyl-$C_{1-6}$-alkyl, phenyl-$C_{1-6}$-alkoxy, $C_{1-6}$-alkoxycarbonylphenyl, hydroxy-$C_{1-6}$-alkylphenyl, benzyloxy, pyridylcarbonylamino-$C_{1-6}$-alkyl, $C_{1-6}$-alkenyloxy, $C_{1-6}$-alkoxy-$C_{1-6}$-alkoxy, methoxybenzyloxy, hydroxybenzyloxy, phenylethyloxy, metylenedioxybenzyloxy, dioxolanyl-$C_{1-6}$-alkoxy, cyclopropyl-$C_{1-6}$-alkoxy, hydroxy-$C_{1-6}$-alkoxy, carbamoyloxy-$C_{1-6}$-alkoxy, pyridyl-carbamoyloxy-$C_{1-6}$-alkoxy, benzoyloxy-$C_{1-6}$-alkoxy; or by pyridyl, pyridyloxy, pyridylthio, pyridylamino, pyridyl-$C_{1-6}$-alkyl, pyridyl-$C_{1-6}$-alkoxy, pyrimidinyl, pyrimidinyloxy, pyrimidinylthio, pyrimidinylamino, pyrimidinyl-$C_{1-6}$-alkyl, pyrimidinyl-$C_{1-6}$-alkoxy, thienyl, thienyl-$C_{1-6}$-alkyl, thienyl-$C_{1-6}$-alkoxy, furyl, furyl-$C_{1-6}$-alkyl and/or furyl-$C_{1-6}$-alkoxy, substituted if required by halogen, $C_{1-6}$-alkyl, $C_{1-6}$-alkoxy or dihydroxy-$C_{1-6}$-alkylaminocarbonyl; and
"heterocyclic ring" or "hetercyclyl" alone or in compounds denotes monocyclic or bicyclic, saturated or unsaturated heterocyclic radicals with 1 to 4 carbon and/or 1 or 2 sulphur or oxygen atoms substituted one or more times, more particularly (in the case of unsaturated heterocyclyl radicals) by $C_{1-6}$-alkyl, hydroxy, $C_{1-6}$--alkoxy, nitro or halogen or by substituents as defined hereinbefore for "aryl" or, in the case of saturated heterocyclyl radicals) can be substituted by $C_{1-6}$-alkyl or $C_{1-6}$-alkoxy.

**2.** A compound according to claim 1 having the general formula:

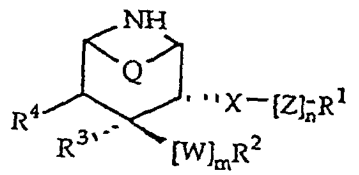

I A

wherein $R^1$ - $R^4$, Q, W, X and Z, n and m have the meanings given in claim 1 except for the compounds 4-(4-fluorophenyl)-3-(3,4-metylenedioxy-benzyloxy) piperidine, the hydrochloride thereof and trans-3-(3-chlorophenoxy)-4-(3,4-imethyl-phenoxy)piperidine or pharmaceutically acceptable salts thereof.

**3.** Compounds according to claim 1 having the general formula I

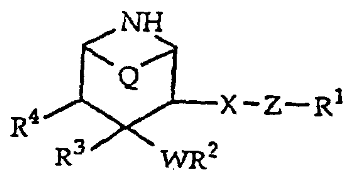

I - 1

wherein

$R^1$ has the meanings given in claim 1;

$R^2$ denotes phenyl, cyclohexyl, phenyl or cyclohexyl substituted by halogen, hydroxy, cyano, trifluoromethyl, $C_{1-6}$-alkyl, halo-$C_{1-6}$-alkyl, hydroxy-$C_{1-6}$-alkyl, $C_{1-6}$-alkoxy-$C_{1-6}$-alkyl, cyano-$C_{1-6}$-alkyl, carboxy-$C_{1-6}$-alkyl, $C_{1-6}$-alkanoyloxy-$C_{1-6}$-alkyl, $C_{1-6}$-alkoxycarbonyloxy-$C_{1-6}$-alkyl, $C_{1-6}$-alkoxycarbonyl, $C_{1-6}$-alkoxy, $C_{1-6}$-alkylenedioxy or by a radical $L^1$-$T^1$-$L^2$-$T^2$-$L^3$-$T^3$-$L^4$ -$T^4$-$L^5$-U or naphthyl or acenaphthyl;

$L^1$, $L^2$, $L^3$, $L^4$ and $L^5$ independently of one another denote a bond, $C_{1-8}$-alkylene, $C_{2-8}$-alkenylene or $C_{2-8}$-alkinylene, or are absent;

$T^1$, $T^2$, $T^3$ and $T^4$ independently of one another denote (a) a bond, or are absent, or denote one of the groups (b) -CH (OH) -; (c) -CH($OR^6$)-; (d) -CH ($NR5R^6$)-; (e) -CO-; (f) -$CR^7R^8$-; (g) -O- or -$NR^6$-, (h) -$S(O)_{0-2}$-; (i) -$SO_2NR^6$-; (j) -$NR^6SO_2$-; (k) -$CONR^6$-; (l) -$NR^6CO$-; (m) -O-CO-; (n) -CO-O-; (o) -O-CO-O-; or (p) -O-CO-$NR^6$-; wherein the bonds starting from (b), (d), (e) and (g) - (p) lead to a C atom in the neighbouring group and the said C atom is saturated if the bond starts from a heteroatom and wherein not more than two groups (b) - (f), three groups (g) - (h) and one group (i) - (p) are present;

$R^3$ denotes hydrogen, hydroxy, $C_{1-6}$-alkoxy or $C_{1-6}$-alkenyloxy;

$R^4$ denotes hydrogen, $C_{1-6}$-alkyl, $C_{1-6}$-alkenyl, $C_{1-6}$-alkoxy, hydroxy-$C_{1-6}$-alkyl, $C_{1-6}$-alkoxy-$C_{1-6}$-alkyl or benzyl;

$R^5$ and $R^6$ denote hydrogen, $C_{1-6}$-alkyl or acyl or together with the N atom to which they are bonded denote a 5- or 6-member heterocyclic ring which can contain an additional N-, O- or S-atom;

$R^7$ and $R^8$ together with the C atom to which they are bonded denote a 3-7-member ring which can contain one or more oxygen or sulphur atoms;

U denotes hydrogen, $C_{1-6}$-alkyl, $C_{3-6}$-cycloalkyl, cyano, aryl or heterocyclyl;

Q denotes ethylene or is absent;

X denotes oxygen, sulphur or a group -$CHOR^9$-, or -OCO- and $R^9$ denotes hydrogen, $C_{1-6}$-alkyl, acyl or aryl-$C_{1-6}$-alkyl;

W is absent or can denote oxygen or sulphur if $R^3$ is hydrogen;

Z denotes $C_{1-6}$-alkylene or is absent.

**4.** Compounds according to claims 1 - 3 in which W is absent.

**5.** Compounds according to claims 1 - 4 in which Q is absent.

**6.** Compounds according to claims 1 - 5 in which X is oxygen, sulphur or -CO-.

**7.** Compounds according to claims 1 - 6 in which Z is methylene.

**8.** Compounds according to claims 1 - 7 in which $R^2$ denotes phenyl, or phenyl substituted by halogen, hydroxy, cyano, trifluoromethyl, $C_{1-6}$-alkyl, halo-$C_{1-6}$-alkyl, hydroxy-$C_{1-6}$-alkyl, $C_{1-6}$-alkoxy-$C_{1-6}$-alkyl, cyano-$C_{1-6}$-alkyl, carboxy-$C_{1-6}$-alkyl, $C_{1-6}$-alkanoyloxy-$C_{1-6}$-alkyl, $C_{1-6}$-alkoxycarbonyloxy-$C_{1-6}$-alkyl, $C_{1-6}$-alkoxycarbonyl, $C_{1-6}$-alkoxy or $C_{1-6}$-alkylenedioxy.

**9.** Compounds according to claims 1 - 7, in which $R^2$ denotes phenyl substituted by a radical $L^1$-$T^1$-$L^2$-$T^2$-$L^3$-$T^3$-$L^4$-$T^4$-$L^5$-U wherein $L^1$ and $L^2$ are absent or denote $C_{1-8}$-alkylene and $L^3$ is absent and U denotes hydrogen, $C_{1-6}$-alkyl, $C_{3-6}$-cycloalkyl, phenyl, phenyl substituted by $C_{1-6}$-alkyl, $C_{1-6}$-alkoxy, $C_{1-6}$-alkylthio, $C_{1-6}$-alkylsulfinyl, $C_{1-6}$-alkylenedioxy, halogen, benzoyl-$C_{1-6}$-alkyl, halogen-$C_{1-6}$-alkyl, $C_{1-6}$-alkanoyloxy or hydroxy; or naphthyl; or pyridyl, thienyl, pyrazinyl,triazolyl, imidazolyl, phenyl-oxadiazolyl, thienyl-oxadiazolyl, furyl-oxadiazolyl, phenyl-oxazolyl, benzothiazolyl, furyl, pyrimidinyl, nitrobenzothiazolyl, phenyltetrazolyl or morpholinyl.

**10.** Compounds according to claims 1 - 7 in which $R^2$ denotes phenyl or phenyl substituted by 2-benzothiazolyl-thio-$C_{1-6}$-alkyl, 2-benzyloxy-3-metoxypropoxy, 2-benzoyloxy-3-methoxypropoxy, 2,3-dihydroxypropoxy, 2-hydroxy-3-benzylamino-propoxy, 2-hydroxy-3-phenoxypropoxy, 2-hydroxy-3-phenylthiopropoxy, 2-methoxy-3-phenoxypropoxy, 2-methoxy-3-benzyloxypropoxy, 2-methyl-3-fluoro-phenylbutyryloxy-$C_{1-6}$-alkoxy, 2-$C_{2-6}$-alkenyloxy-4-phenylbutyl, 3,4,5-trimethoxyphenyl-oxadiazolyl-$C_{1-6}$-alkoxy, 6-nitro-2-benzothiazolyl-thio-$C_{1-6}$-alkyl, benzamido-$C_{1-6}$-alkoxy, benzamido-$C_{1-6}$-alkyl, benzoyl-$C_{1-6}$-akoxy or ketyls thereof, benzoyl-$C_{1-6}$-alkyl or ketyls thereof, benzoyl-$C_{1-6}$-alkyl-aminocarbonyl-$C_{1-6}$-alkyl, benzoyl-$C_{1-6}$--alkoxycarbonyl-$C_{1-6}$-alkyl, benzoyl-$C_{1-6}$-alkylaminocarbonyl, benzoyloxy, benzoyloxy-$C_{1-6}$-alkyl benzoyloxy-$C_{1-6}$-alkoxy, benzoyloxy-$C_{1-6}$-alkoxy, benzoyloxy-$C_{1-6}$-alkyl. benzthiazolylthio-$C_{1-6}$-alkoxy, benzthiazolylthio-$C_{1-6}$-alkyl, benzylcarbamoyl-$C_{1-6}$-alkoxy, benzyloxy-$C_{1-6}$-alkylcarbonyloxy-$C_{1-6}$-alkyl, benzyloxy-$C_{1-6}$-alkoxy, benzylthio-$C_{1-6}$-alkoxy, carbamoyloxy-$C_{1-6}$-alkoxy, carbamoyloxy-$C_{1-6}$-alkyl, carboxy-$C_{1-6}$-alkoxy, carboxy-$C_{1-6}$-alkyl, cyano, cyano-$C_{1-6}$-alkoxy, cyano-$C_{1-6}$-alkyl, cyanophenyl-$C_{1-6}$-alkoxy, cyclohexylcarbonyloxy-$C_{1-6}$-alkyl, cyclopropylcarbonyloxy-$C_{1-6}$-alkyl, cyclopropyloxy-benzyloxy-$C_{1-6}$-alkoxy, dioxolanyl-$C_{1-6}$-alkoxy, furyl-oxadiazolyl-$C_{1-6}$-alkoxy, furyloxy-$C_{1-6}$-alkoxy, halo-phenoxy-$C_{1-6}$-alkyl, halobenzoyl-$C_{1-6}$-alkoxy, halobenzoyloxy-$C_{1-6}$-alkyl, halobenzyloxy-$C_{1-6}$-alkoxy, halobenzyloxy-$C_{1-6}$-alkoxy, halogen, halogen-$C_{1-6}$-alkyl, halophenoxy, halophenyl-oxadiaziolyl-$C_{1-6}$-alkoxy, hydroxy, hydroxy-benzoyloxy-$C_{1-6}$-alkyl, hydroxy-benzyloxy-$C_{1-6}$-alkoxy, hydroxy-$C_{1-6}$-alkoxy, hydroxy-$C_{1-6}$-alkyl, imidazolylcarbonyloxy-$C_{1-6}$-alkyl, methoxybenzoyl-$C_{1-6}$-alkyl, methoxybenzyl-oxy-$C_{1-6}$-alkoxy, methylenedioxybenzoyl-$C_{1-6}$-alkoxy, morpholino-$C_{1-6}$-alkoxy, morpholinocarbonyloxy-$C_{1-6}$-alkoxy, morpholinocarbonyloxy-$C_{1-6}$-alkyl, N-methylaminophenyl-carbonyloxy-$C_{1-6}$-alkyl, N-methyl-benzylamino-$C_{1-6}$-alkoxy, N-methylpyrrolylcarbonyloxy-$C_{1-6}$-alkoxy, N-$C_{1-6}$-alkylbenzamido-$C_{1-6}$-alkyl, naphthyl-$C_{1-6}$-alkoxy, nicotinoyloxy-$C_{1-6}$-alkoxy, nicotinoyloxy-$C_{1-6}$-alkyl, $C_{1-6}$-alkanoylbenzoyloxy-$C_{1-6}$-alkyl, $C_{1-6}$-alkanoyloxy-$C_{1-6}$-alkoxy, $C_{1-6}$-alkanoyloxy-$C_{1-6}$-alkyl, $C_{1-6}$-alkenyl-benzyloxy-$C_{1-6}$-alkoxy, $C_{1-6}$-alkenyloxy, $C_{1-6}$-alkenyloxy-benzyloxy-$C_{1-6}$-alkoxy, $C_{1-6}$-alkoxy, $C_{1-6}$-alkoxy-benzoyloxy-$C_{1-6}$-alkyl, $C_{1-6}$-alkoxy-carbonyl, $C_{1-6}$-alkoxy-$C_{1-6}$-alkyl, $C_{1-6}$-alkoxybenzoylamino-$C_{1-6}$-alkyl, $C_{1-6}$-alkoxybenzylcarbonyloxy-$C_{1-6}$-, $C_{1-6}$-alkoxy-benzyloxy-$C_{1-6}$-alkoxy, $C_{1-6}$-alkoxy-benzylthio-$C_{1-6}$-alkoxy, $C_{1-6}$-alkoxycarbonyl, $C_{1-6}$-alkoxycarboyl-$C_{1-6}$-alkoxy, $C_{1-6}$-alkoxycarbonyl-$C_{1-6}$-alkyl, $C_{1-6}$-alkoxyphenyl-oxadiazolyl-$C_{1-6}$-alkoxy, $C_{1-6}$-alkyl, $C_{1-6}$-alkylbenzyloxy-$C_{1-6}$-alkoxy, $C_{1-6}$-alkylenedioxy, $C_{1-6}$-alkylenedioxybenzyloxy-$C_{1-6}$-alkoxy, $C_{1-6}$-alkylsulfonylbenzoyl-$C_{1-6}$-alkoxy, $C_{1-6}$-alkylthiobenzoyloxy-$C_{1-6}$-alkoxy, $C_{1-6}$-alkylthiobenzyloxy-$C_{1-6}$-alkoxy, benzoylpxybenzyl-$C_{1-6}$-alkoxy, hydroxybenzyl-$C_{1-6}$-alkoxy, $C_{1-6}$-alkoxybenzyl-$C_{1-6}$-alkoxy, $C_{1-6}$-alkoxybenzylcarbonyloxy-alkoxy, phenoxy-benzyloxy-$C_{1-6}$-alkoxy, phenoxycarbonyl-$C_{1-6}$-alkyl, phenoxy-$C_{1-6}$-alkenyloxy, phenoxy-$C_{1-6}$-alkinyloxy, phenyl-$C_{1-6}$-alkanoylamino-$C_{1-6}$-alkyl, phenyl-$C_{1-6}$-alkenyloxy, phenyl-$C_{1-6}$-alkoxy, phenoxy-$C_{1-6}$-alkyl, phenyl-$C_{1-6}$-alkylaminocarbonyl, phenoxy-$C_{1-6}$-alkylcarbonyl-$C_{1-6}$-alkoxy, phenyl-$C_{1-6}$-alkylaminocarbonyl-$C_{1-6}$-alkyl, phenylaminocarbonyloxy-$C_{1-6}$-alkoxy, phenylaminocarbonyloxy-$C_{1-6}$-alkyl, phenyl-hydroxy-$C_{1-6}$-alkyl, phenyl-oxadiazolyl-$C_{1-6}$-aloxy, phenyl-oxadiazolyl-$C_{1-6}$-alkoxy, phenyl-oxadiazolyl-$C_{1-6}$-alkyl, phenyl-oxazolyl-$C_{1-6}$-alkoxy, phenyloxy-$C_{1-6}$-alkoxy,1 phenylsulfamoyl-$C_{1-6}$-alkyl, phenylsulfinyl-$C_{1-6}$-alkyl, phe-

nylsulfonyl-$C_{1-6}$-alkoxy, phenylsulfonyl-$C_{1-6}$-alkyl, phenyltetrazolyl-thio-$C_{1-6}$-alkyl, phenylthio-$C_{1-6}$-alkoxy, phenylthio-$C_{1-6}$-alkyl, pyrazinylcarbonyloxy-$C_{1-6}$-alkyl, pyridylaminocarbonyloxy-$C_{1-6}$-alkoxy, pyridylaminocarbonyloxy-$C_{1-6}$-alkyl, pyridylcarbamoyloxy, pyridyl-$C_{1-6}$-alkoxy-$C_{1-6}$-alkoxy, pyridyl-$C_{1-6}$-alkoxy-$C_{1-6}$-alkyl, pyridyl-oxadiazolyl-$C_{1-6}$-alkoxy, pyridylthio-$C_{1-6}$-alkyl, pyrimidinyloxy-$C_{1-6}$-alkoxy, pyrimidinylthio-$C_{1-6}$-alkyl, thenoyloxy-$C_{1-6}$-alkoxy, thenoyloxy-$C_{1-6}$-alkyl, thienyl-oxadiazolyl-$C_{1-6}$-alkoxy, triazolyl-$C_{1-6}$-alkoxy, trifluoromethylbenzyloxy-$C_{1-6}$-alkoxy or trifluoromethyl.

**11.** Compounds according to claims 1 - 7 in which $R^2$ denotes cyclohexyl or benzoyloxycyclohexyl.

**12.** Compounds according to claims 1 - 7 in which $R^2$ denotes naphthyl, tetrahydronaphthyl or acenaphthyl.

**13.** Compounds according to claims 1 - 7 in which $R^2$ denotes pyridyl or oxopyridyl, or pyridyl or oxopyridyl substituted by 3-H-2 thioxo-benzthiazolyl, $C_{1-6}$-alkoxypehnyl-$C_{1-6}$-alkoxy-$C_{1-6}$-alkoxy, phenyl-$C_{1-6}$-alkoxy-$C_{1-6}$-alkoxy, phenyl-$C_{1-6}$-alkyl, cyclohexyl-$C_{1-6}$-alkoxy, phenoxy-$C_{1-6}$-alkyl or phenyl-$C_{1-6}$-alkoxy-$C_{1-6}$-alkyl.

**14.** Compounds according to claims 1 - 7, in which $R^2$ denotes pyrimidinyl or pyrimidinyl substituted by benzodioxanyl-$C_{1-6}$-alkoxy, biphenylyloxy, biphenylyl-$C_{1-6}$-alkoxy, cyclohexyl-$C_{1-6}$-alkoxy, cyclohexyloxy-$C_{1-6}$-alkoxy, halophenyl-$C_{1-6}$-alkoxy, halophenyl-oxadiazolyl-$C_{1-6}$-alkoxy, indanyl-$C_{1-6}$-alkoxy, naphthyl-$C_{1-6}$-alkoxy, phenyl-$C_{1-6}$-alkyl, N-$C_{1-6}$-alkylphenyl-$C_{1-6}$-alkoxy-$C_{1-6}$-alkylamino, N-$C_{1-6}$-alkylphenyl-$C_{1-6}$-alkylamino, $C_{1-6}$-alkythio, $C_{1-6}$-alkoxy, $C_{1-6}$-alkoxyphenyl-$C_{1-6}$-alkoxy-$C_{1-6}$-alkoxy, $C_{1-6}$-alkoxyphenyl-$C_{1-6}$-alkylamino, $C_{1-6}$-alkylpheyl-$C_{1-6}$-alkylamino, halophenyl-$C_{1-6}$-alkylamino, halophenoxy-$C_{1-6}$-alkoxy, $C_{1-6}$-alkylpyridyl-$C_{1-6}$-alkoxy, phenylalkoxy-$C_{1-6}$-alkoxy, phenyl-$C_{1-6}$-alkoxy-$C_{1-6}$-alkylthio, phenyl-$C_{1-6}$-alkoxy-$C_{1-6}$-alkylamino, phenoxy-phenyl-$C_{1-6}$-alkoxy, phenoxy-phenoxy, phenyl-$C_{1-6}$-alkinyloxy, phenyl-$C_{1-6}$-alkoxy-$C_{1-6}$-alkoxy, phenylthio-$C_{1-6}$-alkoxy, phenyloxazolyl-$C_{1-6}$-alkoxy, phenyl-$C_{1-6}$-alkinyloxy, phenyl-$C_{1-6}$-alkenyloxy, phenyl-$C_{1-6}$-alkylamino, phenyl-pyridyl-$C_{1-6}$-alkoxy or phenylpyridyl-$C_{1-6}$-alkylamino.

**15.** Compounds according to claims 1 - 7 in which $R^2$ is halobenzoyl-$C_{1-6}$-alkyl-triazolyl, phenyl-$C_{1-6}$-alkoxy-$C_{1-6}$-alkyl-triazolyl or phenyl-$C_{1-6}$-alkoxy-$C_{1-6}$-alkoxy-$C_{1-6}$-alkyl-triazolyl.

**16.** Compounds according to claims 1 - 7 in which $R^4$ denotes 2-oxo-imidazolidin-1-yl-$C_{1-6}$-alkyl, 4-hydroxy-piperidin-1-yl-$C_{1-6}$-alkoxy, 4-hydroxy-piperidin-1-yl-$C_{1-6}$-alkoxy-$C_{1-6}$-alkyl, 4-methyl-piperazin-1-yl-$C_{1-6}$-alkoxy, 4-methyl-piperazin-1-yl-$C_{1-6}$-alkoxy-$C_{1-6}$-alkyl, 4-methyl-piperazin-1-yl-$C_{1-6}$-alkyl-carbamoyloxy-$C_{1-6}$-alkyl, 1, 2, 4-triazolyl-$C_{1-6}$-alkyl, amino, amino-$C_{1-6}$-alkyl, amino-$C_{1-6}$-alkyl-amino, amino-$C_{1-6}$-alkyl-amino-$C_{1-6}$-alkyl, amino-$C_{1-6}$-alkoxyl, amino-$C_{1-6}$-alkoxyl-$C_{1-6}$-alkyl, aminocarbonyloxy-$C_{1-6}$-alkyl, benzyloxy, benzyloxy substituted by $C_{1-6}$-alkyl, $C_{1-6}$-alkenyl, $C_{1-6}$-alkoxy, trifluoromethoxy, $C_{1-6}$-alkylthio, hydroxy or halogen, benzyloxy, benzyloxy-$C_{1-6}$-alkyl or by $C_{1-6}$-alkyl $C_{1-6}$-alkenyl, $C_{1-6}$-alkoxy or halogen, carbamoyloxy-$C_{1-6}$-alkyl, cyano-$C_{1-6}$-alkyl, di-$C_{1-6}$-alkyl-amino, di-$C_{1-6}$-alkyl-amino-$C_{1-6}$-alkyl, di-$C_{1-6}$-alkyl-amino-$C_{1-6}$-alkyl-(N-$C_{1-6}$-alkyl) -amino-$C_{1-6}$-alkyl, di-$C_{1-6}$-alkyl-amino-$C_{1-6}$-alkyl-amino di-$C_{1-6}$-alkyl-amino-$C_{1-6}$-alkyl-amino-$C_{1-6}$-alkyl, di-$C_{1-6}$-alkyl-amino-$C_{1-6}$-alkoxyl di-$C_{1-6}$-alkyl-amino-$C_{1-6}$-alkoxyl-$C_{1-6}$-alkyl, dihydroxy-$C_{1-6}$-alkoxy, dihydroxy-$C_{1-6}$-alkoxy-$C_{1-6}$-alkyl, dihydroxy-$C_{1-6}$-alkyl-amino, dihydroxy-$C_{1-6}$-alkyl-amino-$C_{1-6}$-alkyl, guanidinyl-$C_{1-6}$-alkoxy-$C_{1-6}$-alkyl, guanidinyl-$C_{1-6}$-alkyl, hydroxy, hydroxy-$C_{1-6}$-alkyl, sulfooxy-$C_{1-6}$-alkyl, hydroxy-$C_{1-6}$-alkoxyl, hydroxy-$C_{1-6}$-alkoxyl-$C_{1-6}$-alkyl, morpholin-4-yl-$C_{1-6}$-alkoxy, morpholin-4-yl-$C_{1-6}$-alkoxy-$C_{1-6}$-alkyl, morpholin-4-yl-$C_{1-6}$-alkyl-carbamjoyloxy-$C_{1-6}$-alkyl, naphthyl-alkoxy or naphthyl-alkoxy, $C_{1-6}$-alkoxy, $C_{1-6}$-alkoxy-$C_{1-6}$-alkoxy-$C_{1-6}$-alkoxy-$C_{1-6}$-alkoxy-$C_{1-6}$-alkyl, $C_{1-6}$-alkoxy-$C_{1-6}$-alkyl, $C_{1-6}$-alkyl, $C_{1-6}$-alkylsulfonylamino-$C_{1-6}$-alkyl, phenoxy-$C_{1-6}$-alkyl substituted by $C_{1-6}$-alkoxy or by phenoxy-$C_{1-6}$-alkyl, phenyl-thio-$C_{1-6}$-alkyl substituted by $C_{1-6}$-alkyl, $C_{1-6}$-alkoxy or by phenyl-thio-$C_{1-6}$-alkyl, piperazin-4-yl-$C_{1-6}$-alkoxy, piperazin-4-yl-$C_{1-6}$-alkoxy-$C_{1-6}$-alkyl, piperidin-1-yl-$C_{1-6}$-alkyl-carbamoyloxy-$C_{1-6}$-alkyl, piperidin-4-yl-$C_{1-6}$-alkoxy, piperidin-4-yl-$C_{1-6}$-alkoxy-$C_{1-6}$-alkyl, pyridyl-$C_{1-6}$-alkoxyl, pyridyl-$C_{1-6}$-alkoxy-$C_{1-6}$-alkyl, pyridylthio-$C_{1-6}$-alkyl, pyrimidinyloxy-$C_{1-6}$-alkyl substituted by $C_{1-6}$-alkoxy or by pyrimidinyloxy-$C_{1-6}$-alkyl, tetrazolyl-$C_{1-6}$-alkyl, trifluoromethylsulfonylamino-$C_{1-6}$-alkyl substituted by $C_{1-6}$-alkoxy or hydrogen.

**17.** The compounds according to claims 1 - 16,

4-[2-[7-[(3R,4R)-4-[4-(3-benzyloxy-propoxy)-phenyl]-piperidin-3-yloxymethyl]-napthalin-2-yloxy]-ethyl]-morpholine,

(R)-3[7-[(3R,4R)-4-[4-(3-benzyloxy-propoxy)-phenyl]-piperidin-3-yloxymethyl]-napthalin-2-yloxy]-propane-1,2-diol,

(S)-3-[7-[(3R,4R)-4-[4-(3-benzyloxy-propoxy)-phenyl]-piperidin-3-yloxymethyl]-napthalin-2-yloxy]-propane-1,2-diol,

(R)-3-[2-[7-[(3R,4R)-4-[4-(3-benzyloxy-propoxy)-phenyl]-piperidin-3-yloxymethyl]-napthalin-2-yloxy]-ethoxy]-propane-1,2-diol,

(S)-3-[2-[7-[(3R,4R)-4-[4-(3-benzyloxy-propoxy)-phenyl]-piperidin-3-yloxymethyl]-napthalin-2-yloxy]-ethoxy]-propane-1,2-diol,

1-[2-[7-[(3R,4R)-4-[4-(3-benzyloxy-propoxy)-phenyl]-piperidin-3-yloxymethyl]-naphthalin-2-yloxy]-ethyl]-4-methyl-piperazine,

1-[3R,4S-4-[4-(3-benzyloxy-propoxy)-phenyl]-piperidin-3-yl]-2-naphthalin-2-yl-ethanone,

(3R,4S,5S)-4-[4-(3-benzyloxy-propoxy)-phenyl-3-(1,4-dimethoxy-naphthalin-2-ylmethoxy)-piperidin-5-ol,

3R,4R)-4-[4-(3-benzyloxy-propoxy)-phenyl]-3-[7-[(R)-2,3-dihydroxy-propxymethyl]-naphthalin-2-ylmethoxy]-piperidine,

3R,4R)-4-[4-(3-benzyloxy-propoxy)-phenyl]-3-[7-[(S)-2,3-dihydroxy-propxymethyl]-naphthalin-2-ylmethoxy]-piperidine,

3R,4R)-4-[4-(3-benzyloxy-propoxy)-phenyl]-3-[6-[(R)-2,3-dihydroxy-propoxymethyl]-naphthalin-2-ylmethoxy]-piperidine,

3R,4R)-4-[4-(3-benzyloxy-propoxy)-phenyl]-3-[6-[(S)-2,3-dihydroxy-propoxymethyl]-naphthalin-2-ylmethoxy]-piperidine,

4-[(3R,4S,5S))-4-[4-(3-benzyloxy-propoxy)-phenyl]-3-naphthalin-2-ylmethoxy]-piperidine-5-yloxy]-butan-1-ol,

3-[(3R,4S,5S))-4-[4-(3-benzyloxy-propoxy)-phenyl]-3-naphthalin-2-ylmethoxy]-piperidine-5-yloxy]-propan-1-ol,

1-{2-[(3R,4R,5S)-4-[4-(3-benzyloxy-propoxy)-phenyl]-3-naphthalin-2-ylmethoxy]-piperidine-5-yloxy]-ethyl}-4-methyl-piperazine,

(3R,4R,5R)-4-[2-[4-[4(3-benzyloxy-propoxy)-phenyl]-5-(naphthalin-2-ylmethoxy)-piperidin-3-ylmethoxy]-ethyl}-morpholine,

(3R,4S,5S)-4-[4-(3-benzyloxy-propoxy)-phenyl]-3-(4-methoxy-benzyloxy)-piperidine-5-ol,

(3R,4S,5S)-4-[4-(3-benzyloxy-propoxy)-phenyl]-3,5-bis-(4-methoxy-benzyloxy) -piperidine,

(3S,4R,5R)-4-[2-[4-[4-(3-benzyloxy-propoxy)-phenyl]-5-(naphthalin-2-ylmethoxy)-piperidin-3-ylmethoxy]-ethyl-morpholine,

(33,4R,SR)-4-[4-(3-benzyloxy-propoxy)-phenyl]-3-methoxymethyl-5-(naphthalin-2-ylmethoxy)-piperidine,

[3-(4-methyl-piperazin-1-yl)-propyl]-carbamic acid (3S,4R,5R)-4-[4-(3-benzyloxy-propoxy)-phenyl]-5-(naphthalin-2-ylmethoxy)-piperidine-3-ylmethylester,

(3S,4R,SR)-4-[4-[4-(3-benzyloxy-propoxy)-phenyl]-5-(naphthalin-2-ylmethoxy)-piperidine-3-ylmethylsulfanyl]-pyridine,

2-(4-cyclohexyl-butoxy)-5-[(3R,4R)-3-(1,4-dimethoxy-naphthalin-2-ylmethoxy)-piperidin-4-yl]-pyrimidine,

(3'R,4'R)-6-(3-cyclohexyl-propoxy)-3'-(1,4-dimethoxy-naphthalin-2-ylmethoxy)-1',2',3',4',5',6'-hexahydro-[3,4']bipyridine,

(3S,4R,5R)-[4-[4-)3-benzyloxy-propoxy)-phenyl] -5-(naphthalin-2-ylmethoxy)-piperidin-3-yl]-methanol,

(3S,4R,5R)-N-[4-[4-)3-benzyloxy-propoxy)-phenyl]-5-(naphthalin-2-ylmethoxy)-piperidin-3-ylmethyl]-N,N'N'-trimethyl-ethane-1,2-diamine,

(3S,4R,5R)-[4-[4-(3-benzyloxy-propoxy)-phenyl]-5-(naphthalin-2-ylmethoxy)-piperidin-3-ylmethyl]-diethyl-amine,

1-[)3R,4S,5S)-4-[4-(3-benzyloxy-propoxy)-phenyl]-5-(2-moprholin-4-yl-ethoxymethyl)-piperidin-3-yl]-2-naphthalin-2-yl-ethanone,

(3R,4R)-3-(1,4-dimethoxy-naphthalin-2-ylmethoxy)-4-[4-[3-(2-methoxy-phenoxy)-propoxy]-phenyl]-piperidine,

(3R,4S,5S)-4-[4-(3-benzyloxy-propoxy)-phenyl]-3,5-bis-(3,4,5-trimethoxy-benzyloxy)-piperidine,

(3R,4R,5S)-4-[4-(3-benzyloxy-propoxy)-phenyl]-3-(naphthalin-2-ylmethoxy)-5-[1,2,4]triazol-1-ylmethyl-piperidine,

(3R,4R)-4-[4-[3-(2-methoxy-benzyloxy)-propoxy]-phenyl]-3-(quinolin-7-ylmethoxy)-piperidine,

2-(7-{(3R,4R)-4-[4-(3-benzyloxy-propoxy)-phenyl]-piperidin-3-yloxymethyl}-naphthalin-2-ylmethoxy)-ethanol,

7-{3R,4R)-4-[4-(3-benzyloxy-propoxy)-phenyl]-piperidin-3-yloxymethyl}-naphthalin-2-ylmethyl)-dimethyl-amine,

(3R,4R)-3-(4-benzyloxy-naphthalin-2-ylmethoxy)-4-(4-fluoro-phenyl)-piperidine(3'R,4'R)-3'-(1,4-dimethoxy-naphthalin-2-ylmethoxy)-6-[3-(2-methoxybenzyloxy)-propoxy]-1',2',3',4',5',6'-hexahydro-[3,4']bipyridine,

(3R,4R)-3-(1,4-dimethoxy-naphthalin-2-ylmethoxy)-4-[4-[3-(2-methoxy-benzyloxy)-propoxy]-phenyl]-piperidine,

(3S,4R,5R)-1-[4-[4-(3-benzyloxy-propoxy)-phenyl]-5-(naphthalin-2-ylmethoxy)-piperidin-3-ylmethyl]-imidazolidin-2-one,

(3R,4R)-4-[4-[3-(2-methoxy-benzyloxy)-propoxy]-phenyl]-3-(2-oxo-1,2-dihydro-quinoline-7-ylmethoxy)-piperidine,

(3R,4R)-3-(isoquinoline-7-ylmethoxy)-4-[4-[3-(2-methoxy-benzyloxy)-propoxy]-phenyl]-piperidine,

(3R,4R)-4-[4-[3-(2-methoxy-benzyloxy)-propoxy]-phenyl]-3-(1,2,3,4-tetrahydroquinolin-7-ylmethoxy)-piperidine,

1-[2-[7-[(3R,4R)-4-[4-[3-(2-methoxy-benzyloxy)-propoxy]-piperidin-3-yloxymethyl]-naphthalin-2-yloxy]-ethyl]-4-methyl-piperazine,

1-[2-[7-[(3R,4S,5S)-5-hydroxy-4-[4-[-3-(2-methoxy-benzyloxy)-propoxy]-piperidine-3-yloxymethyl]-naphthalin-2-yloxy]-ethyl]-4-methyl-piperazine,

(3R,4S,5S)-3-(1,4-dimethoxy-naphthalin-2-ylmethoxy)-4-[4-[3-(2-methoxy-benzyloxy)-propoxy]-phenyl]-piperidine-5-ol and

(3R,4R,5S)-3-(1,4-dimethoxy-naphthalin-2-ylmethoxy)-4-{4-[3-(2-methoxy-benzyloxy)-propoxy]-phenyl}-5-(1H-tetrazol-5-ylmethyl)-piperidine.

**18.** Compounds having the formula II

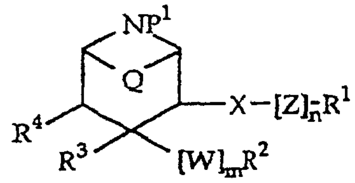

II

in which $P^1$ denotes a protective group and the other symbols have the meanings given in claim 1 and any hydroxy groups in $R^1$, $R^2$ and $R^4$ can be in protected form.

**19.** A drug, more particularly for treatment or prevention of high blood pressure and heart or kidney insufficiency, containing a formula I compound according to claim 1 and a therapeutically inert excipient.

**20.** A method of producing a compound according to any of claims 1 to 17, **characterised in that** the protective group or groups are split off from a formula II compound

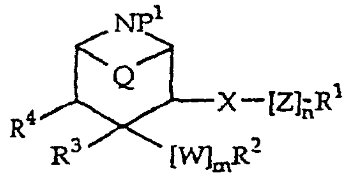

II

in which $P^1$ denotes a protective group and the other symbols have the meaning given in claim 1 and any hydroxy groups in $R^1$, $R^2$ and $R^4$ can be in protected form, the protective group or groups are split off, any reactive groups in the thus-obtained formula I compound are functionally modified if required, and/or the formula I compound is converted into a pharmaceutically useful salt.

**21.** Compounds according to any of claims 1 - 20 for use as therapeutic active substances.

**22.** Use of the formula I compounds according to claim 1 in the production of drugs for treatment of high blood pressure and heart or kidney insufficiency.

**Revendications**

**1.** Dérivés de pipéridine de formule I

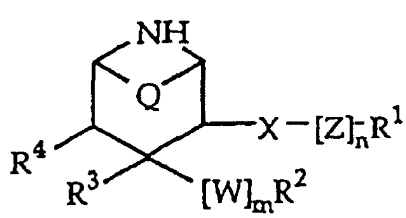

I

dans laquelle

$R^1$ représente un groupe phényle ou un groupe phényle substitué par un alkyle en $C_{1-6}$, alcènyle en $C_{1-6}$, alcoxy en $C_{1-6}$, alkylthio en $C_{1-6}$, halogène, hydroxy- $C_{1-6}$-alcoxy, $C_{1-6}$-alcoxy-$C_{1-6}$-alcoxy, alkylsulfinyle en $C_{1-6}$, alkylsulfonyl en $C_{1-6}$, cyano, trifluorométhyle, trifluorométhoxy, carboxy, cyclobutylméthoxy-$C_{1-6}$-alkyle, alkylènedioxy en $C_{1-6}$, phényle, phénoxy, alcoxycarbonylphényle en $C_{1-6}$, hydroxy-$C_{1-6}$-alkylphényle, 2,3-dihydroxypropylaminocarbonylphényle, benzyloxy, benzoyle, pyridyl-$C_{1-6}$-alcoxy-$C_{1-6}$-alkyle ou nicotinoyl-amino-$C_{1-6}$-alkyle ; ou représente un groupe naphtyle ou un groupe naphtyle substitué par un hydroxy, oxo, alcoxy en $C_{1-6}$, alcényloxy en $C_{1-6}$, $C_{1-6}$-alcoxy-$C_{1-6}$-alcoxy, di-$C_{1-6}$-alkylamino, 2, 3-dihydroxypropoxy, 2, 3-dihy-

droxypropoxy-$C_{1-6}$-alcoxy, 2,3-diméthoxypropoxy, $C_{1-6}$-alcoxycarbonyl-$C_{1-6}$-alcoxy, carbamoyl-$C_{1-6}$-alcoxy, méthoxybenzyloxy, hydroxybenzyloxy, phénéthyloxy, méthylènedioxybenzyloxy, dioxolanyl-$C_{1-6}$-alcoxy, cyclopropyl-$C_{1-6}$-alcoxy, hydroxy-$C_{1-6}$-alcoxy, carbamoyloxy-$C_{1-6}$-alcoxy, pyridyl-carbamoyloxy-$C_{1-6}$-alcoxy, morpholino-$C_{1-6}$-alcoxy, 3-morpholino-2-hydroxypropoxy, N-méthyl-pipérazino-N-$C_{1-6}$-alcoxy, benzoyloxy-$C_{1-6}$-alcoxy ou picoyloxy ; ou représente un groupe tétrahydronaphtyle ou un groupe tétrahydronaphtyle substitué par un méthyle ou un groupe indanyle ; ou représente aussi un groupe pyridyle, benzimidazolyle, di- $C_{1-6}$-alcoxypyrimidinyle ou 2- et 5-benzo[b]thiényle, 6- et 7-quinolyle, 6- et 7-isoquinolyle, 6- et 7-tétrahydroquinolyle, 6- et 7-tétrahydroisoquinolinyle, 6-quinoxalinyle, 6- et 7-quinazolinyle ou un groupe 6-et 7-quinolyle, 6- et 7-isoquinolyle, 6- et 7-tétrahydroquinolyle, 6- et 7-tétrahydroisoquinolinyle, 6-quinoxalinyle, 6- et 7-quinazolinyle substitué par un hydroxy, oxo, alcoxy en $C_{1-6}$, alcényloxy en $C_{1-6}$, $C_{1-6}$-alcoxy-$C_{1-6}$-alcoxy, di-$C_{1-6}$-alkylamino, 2,3-dihydroxypropoxy, 2, 3-dihydroxypropoxy-$C_{1-6}$-alcoxy, 2,3-diméthoxypropoxy, $C_{1-6}$-alcoxycarbonyl-$C_{1-6}$-alcoxy, carbamoyl-$C_{1-6}$-alcoxy, méthoxybenzyloxy, hydroxybenzyloxy, phénéthyloxy, méthylènedioxybenzyloxy, dioxolanyl-$C_{1-6}$-alcoxy, cyclopropyl-$C_{1-6}$-alcoxy, hydroxy-$C_{1-6}$-alcoxy, carbamoyloxy-$C_{1-6}$-alcoxy, pyridyl-carbamoyloxy-$C_{1-6}$-alcoxy, morpholino-$C_{1-6}$-alcoxy, 3-morpholino-2-hydroxypropoxy, N-méthylpipérazino-N-$C_{1-6}$-alcoxy, benzoyloxy-$C_{1-6}$-alcoxy ou picoyloxy ;

$R^2$ représente un groupe phényle, naphtyle, acénaphtyle, cyclohexyle, pyridyle, pyrimidinyle, pyrazinyle, oxopyrdinyle, diazinyle, triazolyle, thiényle, oxazolyle, oxadiazolyle, thiazolyle, pyrrolyle ou furyle, lesdits radicaux pouvant être substitués par des groupes 1-3-halogène, hydroxy, cyano, trifluorométhyle, alkyle en $C_{1-6}$, halo-$C_{1-6}$-alkyle, hydroxy-$C_{1-6}$-alkyle, $C_{1-6}$-alcoxy-$C_{1-6}$-alkyle, cyano-$C_{1-6}$-alkyle, carboxy-$C_{1-6}$-alkyle, $C_{1-6}$-alkanoyloxy-$C_{1-6}$-alkyle, $C_{1-6}$-alcoxycarbonyloxy-$C_{1-6}$-alkyle, alcoxy-carbonyle en $C_{1-6}$ ou alcoxy en $C_{1-6}$ ou $C_{1-6}$-alkylendioxy et/ou par un radical $L^1$-T-$L^2$-$T^2$-$L^3$-$T^3$-$L^4$-$T^4$-$L^5$-U

$L^1$, $L^2$, $L^3$, $L^4$ et $L^5$, indépendamment les uns des autres, représentent une liaison, un alkylène en $C_{1-8}$, alcénylène en $C_{2-8}$ ou alkinylène en $C_{2-8}$, ou sont absents ;

$T^1$, $T^2$, $T^3$ et $T^4$, indépendamment les uns des autres, représentent (a) une liaison ou sont absents, ou l'un des groupes (b) -CH(OH)- ; (c) -CH($OR^6$)- ; (d) -CH($NR^5R^6$)- ; (e) -CO- ; (f) -$CR^7R^8$- ; (g) -O- ou -$NR^6$-, (h) -S$(O)_{0-2}$ ; (i) -$SO_2NR^6$- ; (j) -$NR^6SO_2$- ; (k) -$CONR^6$- ; (l) -$NR^6$CO- ; (m) -O-CO- ; (n) -CO-O- ; (o) -O-CO-O- ; (p) -O-CO-$NR^6$- ; (q) -N($R^6$)-CO-N($R^6$)- ou (r)-N($R^6$)-CO-O-, les liaisons partant de (b), (d), (e) et (g)-(r) conduisant à un atome de C du groupe adjacent et cet atome de C étant saturé, si la liaison part d'un hétéroatome, et pas plus de deux groupes (b)-(f), trois groupes (g)-(h) et un groupe (i)-(p) étant présents ;

$R^3$ représente l'hydrogène, un hydroxy, un alcoxy en $C_{1-6}$ ou alcènyloxy en $C_{1-6}$ ;

$R^4$ représente l'hydrogène, un alkyle en $C_{1-6}$, alcènyle en $C_{1-6}$, alcoxy en $C_{1-6}$, hydroxy-$C_{1-6}$-alkyle, $C_{1-6}$-alcoxy-$C_{1-6}$-alkyle, benzyle, oxo ou un groupe $R^{4a}$-$Z^1$-$X^1$-,

$R^{4a}$ étant un groupe (a) H- ; (b) $C_{1-6}$-alkyl- ; (c) $C_{1-6}$-alcènyl-; (d) hydroxy-$C_{1-6}$-alkyl-; (e) polyhydroxy-$C_{1-6}$-alkyl- ; (f) $C_{1-6}$-alkyl-O-$C_{1-6}$-alkyl-; (g) aryl- ; (h) hétérocyclyl- ; (i) aryl-$C_{1-6}$-alkyl- ; (j) hétérocyclyl-$C_{1-6}$-alkyl-; (k) aryloxy- $C_{1-6}$-alkyl- ; (l) hétérocyclyloxyl-$C_{1-6}$-alkyl-; (m) ($R^5$,$R^6$)-N-$(CH_2)_{1-3}$ ; (n) ($R^5$, $R^6$) -N ; (o) $C_{1-6}$-alkyl-$S(O)_{0-2}$ ; (p) aryl-S-$(O)_{0-2}$- ; (q) hétérocyclyl-$S(O)_{0-2}$-; (r) HO-$SO_3$- ou leurs sels ; (s) $H_2N$-C(NH)-NH- ou (t)NC- et les liaisons partant de (n)-(t) conduisant à un atome de C du groupe adjacent et cet atome étant saturé, si la liaison part d'un hétéroatome ;

$Z^1$ représente une liaison, est absent ou est l'un des groupes (b) $C_{1-6}$-alkylène- ; (c) $C_{1-6}$-alcényle ; (d) -O-, -N($R^{11}$) , -$S(O)_{0-2}$-; (e) -CO- ; (f) -O-CO- ; (g) -O-CO-O- ; (h) -O-CO-N($R^{11}$)- ; (i) -N($R^{11}$)-CO-O- ; (j) -CO-N($R^{11}$)- ; (k) -N($R^{11}$ )-CO- ; (l) -N($R^{11}$)-CO-N($R^{11}$)- ou (m) -CH($OR^9$)- et les liaisons partant de (d) et (f) - (m) conduisant à un atome de C du groupe adjacent et cet atome étant saturé, si la liaison part d'un hétéroatome ;

$X^1$ représente (a) une liaison, est absent ou est l'un des groupes (b) -O- ; (c) -N($R^{11}$)- ; (d) -$S(O)_{0-2}$- ou (e) -$(CH_2)_{1-3}$ ; ou

$R^3$ et $R^4$, ensemble, représentent une liaison ;

$R^5$ et $R^6$ représentent l'hydrogène, un groupe alkyle en $C_{1-6}$, alcènyle en $C_{1-6}$, aryl-$C_{1-6}$-alkyle ou acyle, ou ensemble avec l'atome de N, auquel ils sont liés, représentent un radical hétérocycle de 5 à 6 éléments qui peut contenir un atome de N-, O- ou S-supplémentaire ou un groupe -SO- ou -$SO_2$-, l'atome de N supplémentaire pouvant être éventuellement substitué par des radicaux alkyle en $C_{1-6}$ ;

$R^7$ et $R^8$, ensemble avec l'atome de C auquel ils sont liés, représentent un radical à 3 à 7 éléments qui peut contenir un ou deux atomes de -O- ou -S- ou un groupe -SO- ou -$SO_2$-;

$R^9$ représente un hydrogène, un groupe alkyle en $C_{1-6}$, acyle ou aryl-$C_{1-6}$-alkyle ;

$R^{10}$ représente un groupe carboxy-$C_{1-6}$-alkyle, $C_{1-6}$-alcoxycarbonyl-$C_{1-6}$-alkyle, alkyle en $C_{1-6}$ ou hydrogène ;

$R^{11}$ représente l'hydrogène ou un alkyle en $C_{1-6}$

U représente l'hydrogène, un alkyle en $C_{1-6}$, cycloalkyle en $C_{3-6}$, cyano, cycloalkyle en $C_{3-6}$ éventuellement substitué, aryle ou hétérocyclyle ;

Q représente l'éthylène ou est absent ;

X représente une liaison, l'oxygène, le soufre ou un groupe -$CHOR^9$-, -O-CO, -CO- ou C=$NOR^{10}$-, la liaison

partant d'un atome d'oxygène ou de soufre conduisant à un atome de C saturé du groupe Z ou à $R^1$ ;
W représente l'oxygène ou le soufre ;
Z représente un groupe alkylène en $C_{1-6}$, alcénylène en $C_{1-6}$, hydroxy-$C_{1-6}$-alkylidène-, alk-O- ou alk-S-, Alk désignant un alkylène en $C_{1-6}$ ; et si X représente une liaison, Z représente un alcènylène en $C_{1-6}$,
n est égal à 1 ou si X représente -O-CO-, est égal à 0 ou 1 ;
m est égal à 0 ou 1 ;

et les sels pharmaceutiquement utilisables de ceux-ci ;

à l'exception des composés 4-(4-fluorophényl)-3-(3,4-méthylènedioxybenzyloxy)pipéridine, son chlorhydrate et la trans-3-(3-chlorophénoxy)-4-(3,4-diméthylphénoxy)pipéridine ainsi que ses sels pharmaceutiquement acceptables ; et où

"acyle" représente des radicaux alcanoyl en $C_{1-6}$ ou benzoyl ;

"aryle" représente seul ou dans les compositions le phényle, napthyle ou le tétrahydronaphtyle, lesdits groupes pouvant être substitués une ou plusieurs fois par un groupe alkyle en $C_{1-6}$, trifluorométhyle, nitro, amino, alcènyle en $C_{1-6}$, alcoxy en $C_{1-6}$, alkylcarbonyloxy en $C_{1-6}$, hydroxy, halogène, cyano, carbamoyl, alkylènedioxy en $C_{1-6}$, un groupe phényle, phénoxy, phénylthio, phényl-$C_{1-6}$-alkyle, phényl-$C_{1-6}$-alcoxy, alcoxycarbonylphényle en $C_{1-6}$, hydroxy-$C_{1-6}$-alkylphényle, benzyloxy, pyridylcarbonylamino-$C_{1-6}$-alkyle, alcényloxy en $C_{1-6}$, $C_{1-6}$-alcoxy-$C_{1-6}$-alcoxy, méthoxybenzyloxy, hydroxybenzyloxy, phénéthyloxy, méthylènedioxybenzyloxy, dioxolanyl-$C_{1-6}$-alcoxy, cyclopropyl-$C_{1-6}$-alcoxy, hydroxy-$C_{1-6}$-alcoxy, carbamoyloxy-$C_{1-6}$-alcoxy, pyridyl-carbamoyloxy-$C_{1-6}$-alcoxy, benzoyloxy-$C_{1-6}$-alcoxy éventuellement substitué par un groupe halogène, alkyle en $C_{1-6}$, alcoxy en $C_{1-6}$ ou dihydroxy-$C_{1-6}$-alkylaminocarbonyle ainsi que par un groupe pyridyle, pyridyloxy, pyridylthio, pyridylamino, pyridyl-$C_{1-6}$-alkyle, pyridyl-$C_{1-6}$-alcoxy, pyrimidinyl, pyrimidinyloxy, pyrimidinylthio, pyrimidinylamino, pyrimidinyl-$C_{1-6}$-alkyle, pyrimidinyl-$C_{1-6}$-alcoxy, thiényle, thiényl-$C_{1-6}$-alkyle, thiényl-$C_{1-6}$-alcoxy, furyle, furyl-$C_{1-6}$-alkyle et/ou furyl-$C_{1-6}$-alcoxy éventuellement substitué par un groupe halogène, alkyle en $C_{1-6}$, alcoxy en $C_{1-6}$ ou dihydroxy-$C_{1-6}$-alkylaminocarbonyle;

"radical hétérocyclique" ou "hétérocyclyle", seul ou dans des compositions, désigne des radicaux hétérocycliques mono- ou bicycliques, saturés et insaturés ayant de 1 à 4 atomes d'azote et/ou 1 à 2 atomes de soufre ou d'oxygène, qui peuvent être substitués une ou plusieurs fois, en particulier par un groupe (dans le cas des radicaux hétérocyclyle insaturés) alkyle en $C_{1-6}$, hydroxy, alcoxy en $C_{1-6}$, nitro ou halogène ou par des substituants, comme ceux définis précédemment pour "aryle", ou (dans le cas des radicaux hétérocyclyles saturés) par un groupe alkyle en $C_{1-6}$ ou alcoxy en $C_{1-6}$.

**2.** Composé selon la revendication 1 de formule générale

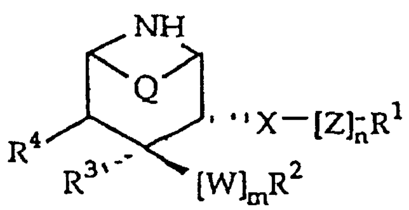

I A

dans laquelle $R^1$ à $R^4$, Q, W, X et Z, n et m ont les significations indiquées dans la revendication 1, à l'exception des composés 4-(4-fluorophényl)-3-(3,4-méthylènedioxy-benzyloxy)pipéridine et son chlorhydrate et la trans-3-(3-chlorophénoxy)-4-(3,4-diméthylphénoxy)pipéridine ainsi que ses sels pharmaceutiquement acceptables.

**3.** Composés selon la revendication 1 de formule générale

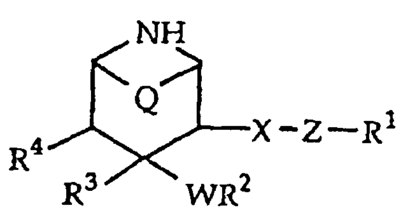

I - 1

dans laquelle

$R^1$ a la signification indiquée dans la revendication 1 ;
$R^2$ représente un groupe phényle, cyclohexyle, un groupe phényle ou cyclohexyle substitué par un groupe halogène, hydroxy, cyano, trifluorométhyle, alkyle en $C_{1-6}$, halo-$C_{1-6}$-alkyle, hydroxy-$C_{1-6}$-alkyle, $C_{1-6}$-alcoxy-$C_{1-6}$-alkyle, cyano-$C_{1-6}$-alkyle, carboxy-$C_{1-6}$-alkyle, $C_{1-6}$-alcanoyloxy-$C_{1-6}$-alkyle, $C_{1-6}$-alcoxycarbonyloxy-$C_{1-6}$-alkyle, alcoxy-carbonyle en $C_{1-6}$, un alcoxy en $C_{1-6}$, un alkylènedioxy en $C_{1-6}$ ou par un radical $L^1$-$T^1$-$L^2$-$T^2$-$L^3$-$T^3$-$L^4$-$T^4$-$L^5$-U ; ou un groupe naphtyle ou acénaphtyle ;
$L^1$, $L^2$, $L^3$, $L^4$ et $L^5$, indépendamment les uns des autres, représentent une liaison, un alkylène en $C_{1-8}$, alcénylène en $C_{2-8}$ ou alkinylène en $C_{2-8}$, ou sont absents ;
$T^1$, $T^2$, $T^3$ **et** $T^4$, indépendamment les uns des autres, représentent (a) une liaison ou sont absents, ou l'un des groupes (b) -CH(OH)-; **(c)** -CH($OR^6$)-; (d) -CH($NR^5R^6$)- ; (e) -CO- ; (f) -$CR^7R^8$- ; (g) -O- ou -$NR^6$-, (h) -S(O)$_{0-2}$ ; (i) -$S_2NR^6$- ; (j) -$NR^6SO_2$- ; (k) -$CONR^6$- ; (l) -$NR^6CO$- ; (m) -O-CO- ; (n) -CO-O- ; (o) -O-CO-O- ou (p) -O-CO-$NR^6$-, les liaisons partant de (b), (d), (e) et (g)-(p) conduisant à un atome de C du groupe adjacent et cet atome de C étant saturé, si la liaison part d'un hétéroatome, et pas plus de deux groupes (b)-(f), trois groupes (g)-(h) et un groupe (i)-(p) étant présents ;
$R^3$ représente l'hydrogène, un hydroxy, un alcoxy en $C_{1-6}$ ou alcènyloxy en $C_{1-6}$ ;
$R^4$ représente l'hydrogène, un alkyle en $C_{1-6}$, alcènyle en $C_{1-6,}$ alcoxy en $C_{1-6}$, hydroxy-$C_{1-6}$-alkyle, $C_{1-6}$-alcoxy-$C_{1-6}$-alkyle ou benzyle ;
$R^5$ et $R^6$ représentent l'hydrogène, un groupe alkyle en $C_{1-6}$, ou acyle, ou ensemble avec l'atome de N, auquel ils sont liés, un radical hétérocycle de 5 à 6 éléments qui peut contenir un atome de N-, O- ou S-supplémentaire ;
$R^7$ et $R^8$, ensemble avec l'atome de C auquel ils sont liés, représentent un radical à 3 à 7 éléments qui peut contenir un ou deux atomes d'oxygène ou de soufre ;
U représente l'hydrogène, un alkyle en $C_{1-6}$, cycloalkyle en $C_{3-6}$, cyano, aryle ou hétérocyclyle ;
Q représente l'éthylène ou est absent ;
X représente l'oxygène, le soufre ou un groupe
-$CHOR^9$- ou -OCO- et $R^9$ représente un hydrogène, un groupe alkyle en $C_{1-6}$, acyle ou aryl-$C_{1-6}$-alkyle ;
W est absent ou représente l'oxygène ou le soufre ; si $R^3$ est hydrogène ;
Z représente un groupe alkylène en $C_{1-6}$, ou est absent.

**4.** Composés selon la revendication 1 à 3, dans lesquels W est absent.

**5.** Composés selon la revendication 1 à 4, dans lesquels Q est absent.

**6.** Composés selon la revendication 1 à 5, dans lesquels X est l'oxygène, le soufre ou -CO-,

**7.** Composés selon la revendication 1 à 6, dans lesquels Z est le méthylène.

**8.** Composés selon la revendication 1 à 7, dans lesquels $R^2$ est un groupe phényle ou un groupe phényle substitué par un groupe halogène, hydroxy, cyano, trifluorométhyle, alkyle en $C_{1-6}$, halo-$C_{1-6}$-alkyle, hydroxy-$C_{1-6}$-alkyle, $C_{1-6}$-alcoxy-$C_{1-6}$-alkyle, cyano-$C_{1-6}$-alkyle, carboxy-$C_{1-6}$-alkyle, $C_{1-6}$-alcanoyloxy-$C_{1-6}$-alkyle, $C_{1-6}$-alcoxycarbonyloxy-$C_{1-6}$-alkyle, alcoxycarbonyle en $C_{1-6}$, alcoxy en $C_{1-6}$, alkylènedioxy en $C_{1-6}$.

**9.** Composés selon la revendication 1 à à 7, dans lesquels $R^2$ représente un groupe phényle substitué par un radical $L^1$-$T^1$-$L^2$-$T^2$-$L^3$-$T^3$-$L^4$-$T^4$-$L^5$-U, $L^1$ et $L^2$ étant absents ou étant un groupe alkylène en $C_{1-8}$ et $L^3$ étant absent et U étant l'hydrogène, un alkyle en $C_{1-6}$, cycloalkyle en $C_{3-6}$, cycloalkyle en $C_{3-6}$, un groupe phényle, un groupe phényle substitué par un groupe alkyle en $C_{1-6}$, alcoxy en $C_{1-6}$, alkylthio en $C_{1-6}$, alkylsulfinyle en $C_{1-6}$, alkylènedioxy en $C_{1-6}$, halogène, benzoyl-$C_{1-6}$-alkyle, halogène-$C_{1-6}$-alkyle, alcanoyloxy en $C_{1-6}$ ou hydroxy ; ou un groupe naphtyle ; ou un groupe pyridyle, thiényle, pyrazinyle, triazolyle, imidazolyle, phényl-oxadiazolyle, thiényl-oxadiazolyle, furyl-oxadiazolyle, phényl-oxazolyle, benzothiazolyle, furyle, pyrimidinyle, nitrobenzothiazolyle, phényltétrazolyle ou morpholinyle.

**10.** Composés selon les revendications 1 à 7, dans lesquels $R^2$ est un groupe phényle ou un groupe phényle substitué par un groupe 2-benzothiazolyl-thio-$C_{1-6}$-alkyle, 2-benzyloxy-3-méthoxypropoxy, 2-benzoyloxy-3-méthoxypropoxy, 2,3-dihydroxypropoxy, 2-hydroxy-3-benzylamino-propoxy, 2-hydroxy-3-phénoxypropoxy, 2-hydroxy-3-phénylthiopropoxy, 2-méthoxy-3-phénoxy-propoxy, 2-méthoxy-3-benzyloxypropoxy, 2-méthyl-3-fluorophénylbutyryloxy-$C_{1-6}$-alcoxy, 2-$C_{2-6}$-alcènyloxy-4-phénylbutyl, 3,4, 5-triméthoxyphényl-oxadiazolyl-$C_{1-6}$-alcoxy, 6-nitro-

2-benzothiazolyl-thio-$C_{1-6}$-alkyle, benzamido-$C_{1-6}$-alcoxy, benzamido-$C_{1-6}$-alkyle, benzoyl-$C_{1-6}$-alcoxy et les cétals de ceux-ci, benzoyl-$C_{1-6}$-alkyle et les cétals de ceux-ci, benzoyl-$C_{1-6}$-alkylaminocarbonyl-$C_{1-6}$-alkyle, benzoyl-$C_{1-6}$-alcoxycarbonyl-$C_{1-6}$-alkyle, benzoyl-$C_{1-6}$-alkylaminocarbonyle, benzoyloxy, benzoyloxy-$C_{1-6}$-alkyl-benzoyloxy-$C_{1-6}$-alcoxy, benzoyloxy-$C_{1-6}$-alcoxy, benzoyloxy-$C_{1-6}$-alkyle, benzothiazolylthio- $C_{1-6}$-alcoxy, benzo-thiazolylthio-$C_{1-6}$-alkyle, benzylcarbamoyl-$C_{1-6}$-alcoxy, benzyloxy-$C_{1-6}$-alkylcarbonyloxy-$C_{1-6}$-alkyle, benzyloxy-$C_{1-6}$-alcoxy, benzylthio-$C_{1-6}$-alcoxy, carbamoyloxy-$C_{1-6}$-alcoxy, carbamoyloxy-$C_{1-6}$-alkyle, carboxy-$C_{1-6}$-alcoxy, carboxy-$C_{1-6}$-alkyle, cyano, cyano-$C_{1-6}$-alcoxy, cyano-$C_{1-6}$-alkyle, cyanophényl-$C_{1-6}$-alcoxy, cyclohexylcarbonyloxy-$C_{1-6}$-alkyle, cyclopropylcarbonyloxy-$C_{1-6}$-alkyle, cyclo-propyloxy-benzyloxy-$C_{1-6}$-alcoxy, dioxolanyl-$C_{1-6}$-alcoxy, furyl-oxadiazolyl-$C_{1-6}$-alcoxy, furoyloxy-$C_{1-6}$-alcoxy, halo-phénoxy-$C_{1-6}$-alkyle, halobenzoyl-$C_{1-6}$-alcoxy, halobenzoyloxy-$C_{1-6}$-alkyle, halobenzoyloxy-$C_{1-6}$-alcoxy, halobenzyloxy-$C_{1-6}$-alcoxy, halogène, halogène-$C_{1-6}$-alkyle, halophénoxy, halophényl-oxadiazolyl-$C_{1-6}$-alcoxy, hydroxy, hydroxy-benzoyloxy-$C_{1-6}$-alkyle, hydroxy-benzyloxy-$C_{1-6}$-alcoxy, hydroxy-$C_{1-6}$-alcoxy, hydroxy-$C_{1-6}$-alkyle, imidazolylcarbonyloxy-$C_{1-6}$-alkyle, méthoxy-benzoyl-$C_{1-6}$-alkyle, méthoxybenzyl-oxy-$C_{1-6}$-alcoxy, méthylènedioxybenzoyloxy-$C_{1-6}$-alcoxy, morpholino-$C_{1-6}$-alcoxy, morpholinocarbonyloxy-$C_{1-6}$-alcoxy, morpholinocarbonyloxy-$C_{1-6}$-alkyle, N-méthylaminophényl-carbonyloxy-$C_{1-6}$-alkyle, N-méthylbenzylamino-$C_{1-6}$-alcoxy, N-méthylpyrrolylcarbonyloxy-$C_{1-6}$-alcoxy, N-$C_{1-6}$-alkylbenzamido-$C_{1-6}$-alkyle, naphtyl-$C_{1-6}$-alcoxy, nicotinoyloxy-$C_{1-6}$-alcoxy, nicotinoyl-oxy-$C_{1-6}$-alkyle, $C_{1-6}$-alcanoylbenzoyloxy-$C_{1-6}$-alkyle, $C_{1-6}$-alcanoyloxy-$C_{1-6}$-alcoxy, $C_{1-6}$-alcanoyloxy-$C_{1-6}$-alkyle, $C_{1-6}$-alcènyl-benzyloxy-$C_{1-6}$-alcoxy, alcènyloxy en $C_{1-6}$, $C_{1-6}$-alcènylox-benzyloxy-$C_{1-6}$-alcoxy, alcoxy en $C_{1-6}$, $C_{1-6}$-alcoxy-benzoyloxy-$C_{1-6}$-alkyle, alcoxycarbonyle en $C_{1-6}$, $C_{1-6}$-alcoxy-$C_{1-6}$-alkyle, $C_{1-6}$-alcoxy-benzoylamino-$C_{1-6}$-alkyle, $C_{1-6}$-alcoxy-benzylcarbonyloxy-$C_{1-6}$-alkyle, $C_{1-6}$-alcoxy-benzyloxy-$C_{1-6}$-alcoxy, $C_{1-6}$-alcoxy-benzylthio-$C_{1-6}$-alcoxy, alcoxycarbonyle en $C_{1-6}$, $C_{1-6}$-alcoxycarbonyl- $C_{1-6}$-alcoxy, $C_{1-6}$-alcoxycarbonyl-$C_{1-6}$-alkyle, $C_{1-6}$-alcoxy-phényl oxadiazolyl-$C_{1-6}$-alcoxy, alkyle en $C_{1-6}$, $C_{1-6}$-alkylbenzyloxy-$C_{1-6}$-alcoxy, alkylènedioxy en $C_{1-6}$, $C_{1-6}$-alkylènedioxybenzyloxy-$C_{1-6}$-alcoxy, $C_{1-6}$-alkylsulfonyl-benzoyl-$C_{1-6}$-alcoxy, $C_{1-6}$-alkylthiobenzoyloxy-$C_{1-6}$-alcoxy, $C_{1-6}$-alkylthio-benzyloxy-$C_{1-6}$-alcoxy, benzoyloxybenzyl-$C_{1-6}$-alcoxy, hydroxybenzyl-$C_{1-6}$-alcoxy, $C_{1-6}$-alcoxybenzyl-$C_{1-6}$-alcoxy, $C_{1-6}$-alcoxybenzylcarbonyloxy-alcoxy, phénoxy-benzyloxy-$C_{1-6}$-alcoxy, phénoxy-carbonyl-$C_{1-6}$-alkyle, phénoxy-$C_{1-6}$-alcènyloxy, phénoxy-$C_{1-6}$-alkinyloxy, phényl-$C_{1-6}$-alcanoylamino-$C_{1-6}$-alkyle, phényl-$C_{1-6}$-alcènyloxy, phényl-$C_{1-6}$-alcoxy, phénoxy-$C_{1-6}$-alkyle, phényl-$C_{1-6}$-alkylaminocarbonyle, phénoxy-$C_{1-6}$-alkylcarbonyl-$C_{1-6}$-alcoxy, phényl-$C_{1-6}$-alkylaminocarbonyl-$C_{1-6}$-alkyle, phénylaminocarbonyloxy-$C_{1-6}$-alcoxy, phénylaminocarbonyloxy-$C_{1-6}$-alkyle, phénylhydroxy-$C_{1-6}$-alkyle, phényloxadiazolyl-$C_{1-6}$-alcoxy, phényloxadiazolyl-$C_{1-6}$-alcoxy, phényloxadiazolyl-$C_{1-6}$-alkyle, phényloxazolyl-$C_{1-6}$-alcoxy, phényloxy-$C_{1-6}$-alcoxy, phénylsulfamoyl-$C_{1-6}$-alkyle, phénylsulfinyl-$C_{1-6}$-alkyle, phénylsulfonyl-$C_{1-6}$-alcoxy, phénylsulfonyl-$C_{1-6}$-alkyle, phényltétrazolylthio-$C_{1-6}$-alkyle, phénylthio-$C_{1-6}$-alcoxy, phénylthio-$C_{1-6}$-alkyle, pyrazinylcarbonyloxy-$C_{1-6}$-alkyle, pyridylaminocarbonyloxy-$C_{1-6}$-alcoxy, pyridylaminocarbonyloxy-$C_{1-6}$-alkyle, pyridylcarbamoyloxy, pyridyl-$C_{1-6}$-alcoxy-$C_{1-6}$-alcoxy, pyridyl-$C_{1-6}$-alcoxy-$C_{1-6}$-alkyle, pyridyl-oxadiazolyl-$C_{1-6}$-alcoxy, pyridylthio-$C_{1-6}$-alkyle, pyrimidinyloxy-$C_{1-6}$-alcoxy, pyrimidinylthio-$C_{1-6}$-alkyle, thénoyloxy-$C_{1-6}$-alcoxy, thénoyloxy-$C_{1-6}$-alkyle, thiényl-oxadiazolyl-$C_{1-6}$-alcoxy, triazolyl-$C_{1-6}$-alcoxy, trifluorométhylbenzyloxy-$C_{1-6}$-alcoxy ou trifluorométhyle.

**11.** Composés selon les revendications 1 à 7, dans lesquels $R^2$ est un groupe cyclohexyle ou benzoyloxycyclohexyle.

**12.** Composés selon les revendications 1 à 7, dans lesquels $R^2$ est un groupe naphtyle, tétrahydronaphtyle ou acénaphtyle.

**13.** Composés selon les revendications 1 à 7, dans lesquels $R^2$ est un groupe pyridyle ou oxopyridyle, ou un groupe pyridyle ou oxopyridyle substitué par un groupe 3-H-2-thioxo-benzothiazolyle, $C_{1-6}$-alcoxyphényl-$C_{1-6}$-alcoxy-$C_{1-6}$-alcoxy, phényl-$C_{1-6}$-alcoxy-$C_{1-6}$-alcoxy, phényl-$C_{1-6}$-alkyle, cyclohexyl-$C_{1-6}$-alcoxy, phénoxy-$C_{1-6}$-alkyle ou phényl-$C_{1-6}$-alcoxy-$C_{1-6}$-alkyle.

**14.** Composés selon les revendications 1 à 7, dans lesquels $R^2$ est un groupe pyrimidinyle ou un groupe pyrimidinyle substitué par un groupe benzodioxanyl-$C_{1-6}$-alcoxy, biphénylyloxy, biphénylyl-$C_{1-6}$-alcoxy, cyclohexyl-$C_{1-6}$-alcoxy, cyclohexyloxy-$C_{1-6}$-alcoxy, halophényl-$C_{1-6}$-alcoxy, halophényl-oxadiazolyl-$C_{1-6}$-alcoxy, indanyl-$C_{1-6}$-alcoxy, naphtyl-$C_{1-6}$-alcoxy, phényl-$C_{1-6}$-alkyle, N-$C_{1-6}$-alkylphényl-$C_{1-6}$-alcoxy-$C_{1-6}$-alkylamino, N-$C_{1-6}$-alkylphényl-$C_{1-6}$-alkylamino, alkylthio en $C_{1-6}$, alcoxy en $C_{1-6}$, $C_{1-6}$-alcoxyphényl-$C_{1-6}$-alcoxy-$C_{1-6}$-alcoxy, $C_{1-6}$-alcoxyphényl-$C_{1-6}$-alkylamino, $C_{1-6}$-alkylphényl-$C_{1-6}$-alkylamino, halophényl-$C_{1-6}$-alkylamino, halophénoxy-$C_{1-6}$-alcoxy, $C_{1-6}$-alkylpyridyl-$C_{1-6}$-alcoxy, phényl-$C_{1-6}$-alcoxy-$C_{1-6}$-alcoxy, phényl-$C_{1-6}$-alcoxy-$C_{1-6}$-alkylthio, phényl-$C_{1-6}$-alcoxy-$C_{1-6}$-alkylamino, phénoxyphényl-$C_{1-6}$-alcoxy, phénoxy-phénoxy, phényl-$C_{1-6}$-alkinyloxy, phényl-$C_{1-6}$-alcoxy-$C_{1-6}$-alcoxy, phénylthio-$C_{1-6}$-alcoxy, phényl-oxazolyl-$C_{1-6}$-alcoxy, phényl-$C_{1-6}$-alkinyloxy, phényl-$C_{1-6}$-alcènyloxy, phényl-$C_{1-6}$-alkylamino, phénylpyridyl-$C_{1-6}$-alcoxy ou phénylpyridyl-$C_{1-6}$-alkylamino.

**15.** Composés selon les revendications 1 à 7, dans lesquels $R^2$ représente un groupe halobenzoyl-$C_{1-6}$-alkyl-triazolyl, phényl-$C_{1-6}$-alcoxy-$C_{1-6}$-alkyl-triazolyl ou phényl-$C_{1-6}$-alcoxy-$C_{1-6}$-alcoxy-$C_{1-6}$-alkyl-triazolyl.

**16.** Composés selon les revendications 1 à 7, dans lesquels $R^4$ est un groupe 2-oxo-imidazolidin-1-yl-$C_{1-6}$-alkyle, 4-hydroxy-pipéridin-1-yl-$C_{1-6}$-alcoxy, 4-hydroxy-pipéridin-1-yl-$C_{1-6}$-alcoxy-$C_{1-6}$-alkyle, 4-méthyl-pipéridin-1-yl-$C_{1-6}$-alcoxy, 4-méthyl-pipérazin-1-yl-$C_{1-6}$-alcoxy-$C_{1-6}$-alkyle, 4-méthyl-pipérazin-1-yl-$C_{1-6}$-alkyl-carbamoyloxy-$C_{1-6}$-alkyle, 1,2,4-triazolyl-$C_{1-6}$-alkyle, amino, amino-$C_{1-6}$-alkyle, amino-$C_{1-6}$-alkylamino, amino-$C_{1-6}$-alkylamino-$C_{1-6}$-alkyle, amino-$C_{1-6}$-alcoxyl, amino-$C_{1-6}$-alcoxyl-$C_{1-6}$-alkyle, aminocarbonyloxy-$C_{1-6}$-alkyle, benzyloxy ou un groupe benzyloxy, benzyloxy-$C_{1-6}$-alkyle substitué par un groupe alkyle en $C_{1-6}$, alcènyle en C1-6, alcoxy en $C_{1-6}$, trifluorométhoxy, alkylthio en $C_{1-6,}$ hydroxy ou halogène ou un groupe benzyloxy, carbamoyloxy-$C_{1-6}$-alkyle, cyano-$C_{1-6}$-alkyle, di-$C_{1-6}$-alkylamino, di-$C_{1-6}$-alkylamino-$C_{1-6}$-alkyle, di-$C_{1-6}$-alkylamino-$C_{1-6}$-alkyl-(N-$C_{1-6}$-alkyl)-amino-$C_{1-6}$-alkyle, di-$C_{1-6}$-alkyl-amino-$C_{1-6}$-alkylamino, di-$C_{1-6}$-alkylamino-$C_{1-6}$-alkylamino-$C_{1-6}$-alkyle, di-$C_{1-6}$-alkylamino-$C_{1-6}$-alcoxyle, di-$C_{1-6}$-alkylamino-$C_{1-6}$-alcoxyl-$C_{1-6}$-alkyle, dihydroxy-$C_{1-6}$-alcoxy, dihydroxy-$C_{1-6}$-alcoxy-$C_{1-6}$-alkyle, dihydroxy-$C_{1-6}$-alkylamino, dihydroxy-$C_{1-6}$-alkylamino-$C_{1-6}$-alkyle, guanidinyl-$C_{1-6}$-alcoxy-$C_{1-6}$-alkyle, guanidinyl-$C_{1-6}$-alkyle, hydroxy, hydroxy-$C_{1-6}$-alkyle, sulfooxy-$C_{1-6}$-alkyle, hydroxy-$C_{1-6}$-alcoxy, hydroxy-$C_{1-6}$-alcoxy-$C_{1-6}$-alkyle, morpholin-4-yl-$C_{1-6}$-alcoxy, morpholin-4-yl-$C_{1-6}$-alcoxy-$C_{1-6}$-alkyle, morpholin-4-yl-$C_{1-6}$-alkyl-carbamoyloxy-$C_{1-6}$-alkyle, naphtyl-alcoxy ou un groupe naphtyl-alcoxy, substitué par alcoxy en $C_{1-6}$, alcoxy en $C_{1-6}$, $C_{1-6}$-alcoxy-$C_{1-6}$-alcoxy-$C_{1-6}$-alcoxy-$C_{1-6}$-alcoxy-$C_{1-6}$-alkyle, $C_{1-6}$-alcoxy-$C_{1-6}$-alkyle, alkyle en $C_{1-6}$, $C_{1-6}$-alkylsulfonylamino-$C_{1-6}$-alkyle, phénoxy-$C_{1-6}$-alkyle ou phénoxyalkyle en $C_{1-6}$ substitué par un groupe alkyle en $C_{1-6}$, alcoxy en $C_{1-6}$, phénylthio-$C_{1-6}$-alkyle substitué par un groupe alkyle en $C_{1-6}$, alcoxy en $C_{1-6}$, ou un groupe phénylthio-$C_{1-6}$-alkyle, pipérazin-4-yl-$C_{1-6}$-alcoxy, pipérazin-4-yl-$C_{1-6}$-alcoxy-$C_{1-6}$-alkyle, pipéridin-1-yl-$C_{1-6}$-alkyl-carbamoyloxy-$C_{1-6}$-alkyle, pipéridin-4-yl-$C_{1-6}$-alcoxy, pipéridin-4-yl-$C_{1-6}$-alcoxy-$C_{1-6}$-alkyle, pyridyl-$C_{1-6}$-alcoxyle, pyridyl-$C_{1-6}$-alcoxy-$C_{1-6}$-alkyle, pyridylthio-$C_{1-6}$-alkyle, pyrimidinyloxy-$C_{1-6}$-alkyle ou un groupe pyrimidinyloxy-$C_{1-6}$-alkyle, substitué par un groupe alcoxy en $C_{1-6}$, tétrazolyl-$C_{1-6}$-alkyle, trifluorométhylsulfonylamino-$C_{1-6}$-alkyle ou hydrogène.

**17.** Composés selon les revendications 1 à 16,

4-[2-[7-[(3R,4R)-4-[4-(3-benzyloxy-propoxy)-phényl]-pipéridin-3-yloxyméthyl]-naphtalin-2-yloxy]-éthyl]-morpholine,
(R)-3-[7-[(3R,4R)-4-[4-(3-benzyloxy-propoxy)-phényl]-pipéridin-3-yloxyméthyl]-naphtalin-2-yloxy]-propane-1,2-diol,
(S)-3-[7-[(3R,4R)-4-[4-(3-benzyloxy-propoxy)-phényl]-pipéridin-3-yloxyméthyl]-naphtalin-2-yloxy]-propane-1,2-diol,
(R)-3-[2-[7-[(3R,4R)-4-[4-(3-benzyloxy-propoxy)-phényl]-pipéridin-3-yloxyméthyl]-naphtalin-2-yloxy]-éthoxy]-propane-1,2-diol,
(S)-3-[2-[7-[(3R,4R)-4-[4-(3-benzyloxy-propoxy)-phényl]-pipéridin-3-yloxyméthyl]-naphtalin-2-yloxy]-éthoxy]-propane-1,2-diol,
1-[2-[7-[(3R,4R)-4-[4-(3-benzyloxy-propoxy)-phényl]-pipéridin-3-yloxyméthyl]-naphtalin-2-yloxy]-éthyl]-4-méthyl-pipérazine,
1-[(3R,4S)-4-[4-(3-benzyloxy-propoxy)-phényl]-pipéridin-3-yl]-2-naphtalin-2-yl]-éthanone,
(3R,4S,5S)-4-[4-(3-benzyloxy-propoxy)-phényl] -3-(1,4-diméthoxy-naphtalin-2-ylméthoxy]-pipéridin-5-ol,
(3R,4R)-4-[4-(3-benzyloxy-propoxy)-phényl]-3-[7-[(R)-2,3-dihydroxy-propoxyméthyl]-naphtalin-2-ylméthoxy]-pipéridine,
(3R,4R)-4-[4-(3-benzyloxy-propoxy)-phényl]-3-[7-[(S)-2,3-dihydroxy-propoxyméthyl]-naphtalin-2-ylméthoxy]-pipéridine,
(3R,4R)-4-[4-(3-benzyloxy-propoxy)-phényl]-3-[6-[(R)-2,3-dihydroxy-propoxyméthyl]-naphtalin-2-ylméthoxy]-pipéridine,
(3R,4R)-4-[4-(3-benzyloxy-propoxy)-phényl]-3-[6-[(S)-2,3-dihydroxy-propoxyméthyl]-naphtalin-2-ylméthoxy]-pipéridine,
4-[(3R,4S,5S)-4-[4-(3-benzyloxy-propoxy)-phényl]-3-(naphtalin-2-ylméthoxy)-pipéridin-5-yloxy]-butan-1-ol,
3-[(3R,4S,5S)-4-[4-(3-benzyloxy-propoxy)-phényl]-3-(naphtalin-2-ylméthoxy)-pipéridin-5-yloxy]-butan-1-ol,
1-{2-[(3R,4R,5S)-4-[4-(3-benzyloxy-propoxy)-phényl]-3-(naphtalin-2-ylméthoxy)-pipéridin-5-yloxy]-éthyl}-4-méthyl-pipérazine,
{(3R,4R,5R)-4-[2-[4-[4-(3-benzyloxy-propoxy)-phényl]-5-(naphtalin-2-ylméthoxy)-pipéridin-3-yloxy]-éthyl}-morpholine,
(3R,4S,5S)-4-[4-(3-benzyloxy-propoxy)-phényl]-3-(méthoxybenzyloxy)-pipéridin-5-ol,
(3R,4S,5S)-4-[4-(3-benzyloxy-propoxy)-phényl] -3,5-bis-(4-méthoxybenzyloxy)-pipéridine,

(3S, 4R, 5R)-4-[2-[4-[4-(3-benzyloxy-propoxy)-phényl]-5-(naphtalin-2-ylméthoxy)-pipéridin-3-yloxy]-éthyl)-morpholine,
(3S,4R,5R)-4-[4-(3-benzyloxy-propoxy)-phényl]-3-méthoxyméthyl-5-(naphtalin-2-yl-méthoxy)-pipéridine,
ester méthylique de (3S,4R,5R)-4-[4-(3-benzyloxy-propoxy)-phényl]-5-(naphtalin-2-yl-méthoxy)-pipéridin-3-yl d'acide [3-(4-méthyl-pipérazin-1-yl)-propyl]carbaminique
(3S,4R,5R)-4-[4-[4-(3-benzyloxy-propoxy)-phényl]-5-(naphtalin-2-yl-méthoxy)-pipéridin-3-ylméthylsulfanyl]-pyridine,
2-(4-cyclohexyl-butoxy)-5-[(3R,4R)-3-(1,4-diméthoxy-naphtalin-2-ylméthoxy)-pipéridin-4-yl]-pyrimidine,
(3'R,4'R)-6-(3-cyclohexyl-propoxy)-3'-(1,4-diméthoxy-naphtalin-2-ylméthoxy)-1',2',3',4',5',6'-hexahydro-[3,4'] bipypiridine,
(3S,4R,5R)-[4-[4-(3-benzyloxy-propoxy)-phényl] -5-(naphtalin-2-yl-méthoxy)-pipéridin-3-yl]-méthanol,
(3S,4R,5R)-N-[4-[4-(3-benzyloxy-propoxy)-phényl]-5-(naphtalin-2-yl-méthoxy)-pipéridin-3-ylméthyl]-N,N',N'-triméthyl-éthane-1,2-diamine,
(3S,4R,5R)-[4-[4-(3-benzyloxy-propoxy)-phényl]-5-(naphtalin-2-yl-méthoxy)-pipéridin-3-ylméthyl]-diéthylamine,
1-[(3R,4S,5S)-4-[4-(3-benzyloxy-propoxy)-phényl]-5- (2-morpholin-4-yl-éthoxyméthyl)-pipéridin-3-yl]-2-naphtalin-2-yl]-éthanone,
(3R,4R)-3-(1,4-diméthoxy-naphtalin-2-ylméthoxy)-4-[4-[3-(2-méthoxyphénoxy)-propoxy-phényl]-pipéridine,
(3R,4S,5S)-4-[4-(3-benzyloxy-propoxy)-phényl] -3,5-bis-(3,4,5-triméthoxy-benzyloxy)-pipéridine,
(3R,4R,5S)-4-[4-(3-benzyloxy-propoxy)-phényl]-3-(naphtalin-2-ylméthoxy)-5-[1,2,4]triazol-1-ylméthyl-pipéridine,
(3R,4R)-4-[4-[3-(2-méthoxybenzyloxy)-propoxy]-phényl]-3-(quinolin-2-ylméthoxy)-pipéridine,
2-(7-{(3R,4R)-4-[4-(3-benzyloxy-propoxy)-phényl]-pyridin-3-yloxyméthyl}-(naphtalin-2-ylméthoxy)-éthanol,
7-{(3R,4R)-4-[4-(3-benzyloxy-propoxy)-phényl]-pyridin-3-yloxyméthyl}-(naphtalin-2-ylméthoxy)-diméthylamine,
(3R,4R)-3-(4-benzyloxy-naphtalin-2-ylméthoxy)-4-(4-fluorophényl)-pipéridine,
(3'R,4'R)-3'-(1,4-diméthoxy-naphtalin-2-ylméthoxy)-6-[3-(2-méthoxybenzyloxy)-propoxy]-1',2',3',4',5',6'-hexahydro-[3,4']bipypiridine,
(3R,4R)-3-(1,4-diméthoxy-naphtalin-2-ylméthoxy)-4-[4-[3-(2-méthoxybenzyloxy)-propoxy]-phényl]-pipéridine,
[3S,4R,5R)-1-[4-[4-(3-benzyloxy-propoxy)-phényl]-5-(naphtalin-2-yl-méthoxy)-pipéridin-3-ylméthyl]-imidazolidin-2-one,
(3R,4R)-4-[4-[3-(2-méthoxy-benzyloxy)-propoxy]-phényl]-3-(2-oxo-1,2-dihydroquinolin-7-yl-méthoxy)-pipéridine,
(3R,4R)-3-(isoquinolin-7-ylméthoxy)-4-[4-[3-(2-méthoxy-benzyloxy)-propoxy]-phényl] -pipéridine,
(3R,4R)-4-[4-[3-(2-méthoxy-benzyloxy)-propoxy]-phényl]-3-(1,2,3,4-tétrahydroquinolin-7-ylméthoxy)-pipéridine,
1-[2-[7-[(3R,4R)-4-[4-[3-(2-méthoxybenzyloxy)-propoxy]-pipéridin-3-yloxyméthyl]-naphtalin-2-yloxy]-éthyl]-4-méthyl-pipérazine,
1-[2-[7-[(3R,4S,5S)-5-hydroxy-4-[4-[3-(2-méthoxy-benzyloxy)-propoxy]-pipéridin-3-yloxyméthyl]-naphtalin-2-yloxy]-éthyl]-4-méthyl-pipérazine,
(3R,4S,5S)-3-(1,4-diméthoxy-naphtalin-2-ylméthoxy)-4-[4-[3-(2-méthoxybenzyloxy)-propoxy]-phényl] -pipéridin-5-ol et
(3R,4R,5S)-3-(1,4-diméthoxy-naphtalin-2-yl-méthoxy)-4-{4-[3-(2-méthoxybenzyloxy)-propoxy]-phényl}-5-(1H-tétrazol-5-ylméthyl)-pipéridine.

**18.** Composés de formule II

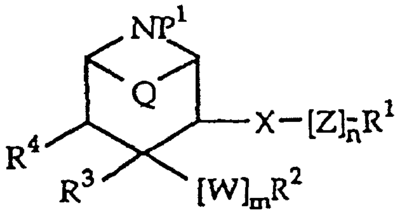

II

où $P^1$ représente un groupe protecteur et les autres symboles ont la même signification que celle indiquée dans la revendication 1 et des groupes hydroxy éventuellement présents dans $R^1$, $R^2$ et $R^4$ peuvent se présenter sous forme protégée.

**19.** Médicament, en particulier pour lutter ou prévenir l'hypertension artérielle, l'insuffisance cardiaque et l'insuffisance rénale, contenant un composé de formule I de la revendication 1 et un excipient thérapeutiquement inerte.

**20.** Procédé de préparation d'un composé selon l'une quelconque des revendications 1 à 17, **caractérisé en ce que** l'on élimine les groupes protecteurs d'un composé de formule II :

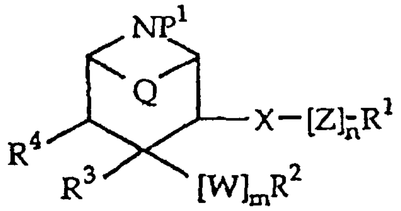

II

où $P^1$ représente un groupe protecteur et les autres symboles ont la même signification que celle indiquée dans la revendication 1 et des groupes hydroxy éventuellement présents dans $R^1$, $R^2$ et $R^4$ peuvent se présenter sous forme protégée, l'on transforme des groupes susceptibles de réagir fonctionnellement si désiré dans le composé de formule I ainsi obtenu et/ou l'on transforme le composé de formule I en un sel pharmaceutiquement utilisable.

**21.** Composés selon l'une quelconque des revendications 1 à 20 pour l'application comme actif thérapeutique.

**22.** Utilisation de composés de formule I de la revendication 1 pour la préparation de médicaments pour le traitement de l'hypertension artérielle, de l'insuffisance cardiaque et rénale.